# EUROPÄISCHE PATENTSCHRIFT

(11) **EP 2 007 794 B1**
(45) Veröffentlichungstag und Bekanntmachung des Hinweises auf die Patenterteilung: **14.09.2011**
(21) Anmeldenummer: 07724241.0
(22) Anmeldetag: 13.04.2007
(51) Int. Cl.: C07K 7/56, A61K 38/12, C07K 1/00, C07K 7/60

(54) **LYSOBACTINAMIDE**
LYSOBACTIN AMIDES
LYSOBACTINAMIDE

(30) Priorität: 13.04.2006 DE 102006018080
(43) Veröffentlichungstag der Anmeldung: 31.12.2008
(73) Patentinhaber: AiCuris GmbH & Co. KG, 42117 Wuppertal (DE)
(72) Erfinder: VON NUSSBAUM, Franz, 40219 Düsseldorf (DE); BECK, Hartmut, 50733 Köln (DE); BRUNNER, Nina, 45279 Essen (DE); ENDERMANN, Rainer, 42113 Wuppertal (DE); KOEBBERLING, Johannes, 41466 Neuss (DE); RAGOT, Jacques, 40625 Düsseldorf (DE); TELSER, Joachim, 42115 Wuppertal (DE); SCHUHMACHER, Joachim, 42113 Wuppertal (DE); ANLAUF, Sonja, 42929 Wermelskirchen (DE); CANCHO-GRANDE, Yolanda, 51373 Leverkusen (DE); GRESCHAT, Susanne, 49419 Wagenfeld (DE); MILITZER, Hans-Christian, 51519 Odenthal (DE); SCHIFFER, Guido, 42111 Wuppertal (DE)
(74) Vertreter: Scheuermann, Erik
(86) Internationale Anmeldenummer: PCT/EP2007/003303
(87) Internationale Veröffentlichungsnummer: WO 2007/118691

(56) Entgegenhaltungen:
- WO-A-2004/099239
- WO-A-2006/048156
- US-A- 4 754 018
- US-A1- 2005 272 646
- US-B1- 6 380 156
- MURAKAMI N ET AL: "Synthesis of Amide Analogs of Arenastatin A" TETRAHEDRON, ELSEVIER SCIENCE PUBLISHERS, AMSTERDAM, NL, Bd. 56, Nr. 46, 10. November 2000 (2000-11-10), Seiten 9121-9128, XP004238570 ISSN: 0040-4020
- BARRETT D ET AL: "An expedient synthesis of the amide analog of the potent antifungal lipopeptidolactone FR901469" TETRAHEDRON LETTERS, ELSEVIER, AMSTERDAM, NL, Bd. 42, Nr. 4, 22. Januar 2001 (2001-01-22), Seiten 703-705, XP004314770 ISSN: 0040-4039
- NORMAN B H ET AL: "TOTAL SYNTHESIS OF CRYPTOPHYCIN ANALOGUES. ISOSTERIC REPLACEMENT OF THE C-D ESTER" JOURNAL OF ORGANIC CHEMISTRY, AMERICAN CHEMICAL SOCIETY. EASTON, US, Bd. 63, Nr. 15, 1998, Seiten 5288-5294, XP000872483 ISSN: 0022-3263
- SHEMYAKIN M M ET AL: "Mutual replaceability of amide and ester groups in biologically active peptides and depsipeptides." EXPERIENTIA 15 AUG 1966, Bd. 22, Nr. 8, 15. August 1966 (1966-08-15), Seiten 535-536, XP009085077 ISSN: 0014-4754
- SRIRAM DHARMARAJAN ET AL: "Camptothecin and its analogues: a review on their chemotherapeutic potential.", NATURAL PRODUCT RESEARCH JUN 2005, vol. 19, no. 4, June 2005 (2005-06), pages 393-412, ISSN: 1478-6419
- HERTZBERG R P ET AL: "Modification of the hydroxy lactone ring of camptothecin: Inhibition of mammalian topoisomerase I and biological activity", JOURNAL OF MEDICINAL CHEMISTRY, AMERICAN CHEMICAL SOCIETY, WASHINGTON, US, vol. 32, no. 3, 1 January 1989 (1989-01-01), pages 715-720, XP002014409, ISSN: 0022-2623
- KRAVCHENKO VLADIMIR V ET AL: "N-(3-oxo-acyl)homoserine lactones signal cell activation through a mechanism distinct from the canonical pathogen-associated molecular pattern recognition receptor pathways.", THE JOURNAL OF BIOLOGICAL CHEMISTRY 29 SEP 2006, vol. 281, no. 39, 29 September 2006 (2006-09-29), pages 28822-28830, ISSN: 0021-9258
- CHEN S-H ET AL: "SYNTHESIS AND BIOLOGICL EVALUATION OF C-13 AMIDE-LINKED PACLITAXEL (TAXOL) ANALOGS", JOURNAL OF ORGANIC CHEMISTRY, AMERICAN CHEMICAL SOCIETY, EASTON.; US, vol. 61, no. 6, 22 March 1996 (1996-03-22) , pages 2065-2070, XP000556979, ISSN: 0022-3263
- ZHANG ET AL: "beta-Lactam congeners of orlistat as inhibitors of fatty acid synthase", BIOORGANIC & MEDICINAL CHEMISTRY LETTERS, PERGAMON, ELSEVIER SCIENCE, GB, vol. 18, no. 7, 19 February 2008 (2008-02-19), pages 2491-2494, XP022574988, ISSN: 0960-894X
- BORZILLERI R M ET AL: "A novel application of a Pd(0)-catalyzed nucleophilic substitution reaction to the regio- and stereoselective synthesis of lactam analogues of epothilone natural products", JOURNAL OF THE AMERICAN CHEMICAL SOCIETY, AMERICAN CHEMICAL SOCIETY, NEW YORK, USA, vol. 122, 1 January 2000 (2000-01-01), pages 8890-8897, XP002421787, ISSN: 0002-7863
- MOORE S ET AL: "Synthesis and some properties and antitumor effects of the actinomycin lactam analog, (di(1-L-alpha, beta-diaminopropionic))actinomycin D1.", JOURNAL OF MEDICINAL CHEMISTRY JUN 1976, vol. 19, no. 6, June 1976 (1976-06), pages 766-772, ISSN: 0022-2623
- SRIRAM DHARMARAJAN ET AL: "Camptothecin and its analogues: a review on their chemotherapeutic potential." NATURAL PRODUCT RESEARCH JUN 2005, vol. 19, no. 4, June 2005 (2005-06), pages 393-412, ISSN: 1478-6419
- HERTZBERG R P ET AL: "Modification of the hydroxy lactone ring of camptothecin: Inhibition of mammalian topoisomerase I and biological activity" JOURNAL OF MEDICINAL CHEMISTRY, AMERICAN CHEMICAL SOCIETY, WASHINGTON, US, vol. 32, no. 3, 1 January 1989 (1989-01-01), pages 715-720, XP002014409 ISSN: 0022-2623
- KRAVCHENKO VLADIMIR V ET AL: "N-(3-oxo-acyl)homoserine lactones signal cell activation through a mechanism distinct from the canonical pathogen-associated molecular pattern recognition receptor pathways." THE JOURNAL OF BIOLOGICAL CHEMISTRY 29 SEP 2006, vol. 281, no. 39, 29 September 2006 (2006-09-29), pages 28822-28830, ISSN: 0021-9258
- CHEN S-H ET AL: "SYNTHESIS AND BIOLOGICL EVALUATION OF C-13 AMIDE-LINKED PACLITAXEL (TAXOL) ANALOGS" JOURNAL OF ORGANIC CHEMISTRY, AMERICAN CHEMICAL SOCIETY, EASTON.; US, vol. 61, no. 6, 22 March 1996 (1996-03-22) , pages 2065-2070, XP000556979 ISSN: 0022-3263
- ZHANG ET AL: "beta-Lactam congeners of orlistat as inhibitors of fatty acid synthase" BIOORGANIC & MEDICINAL CHEMISTRY LETTERS, PERGAMON, ELSEVIER SCIENCE, GB, vol. 18, no. 7, 19 February 2008 (2008-02-19), pages 2491-2494, XP022574988 ISSN: 0960-894X
- BORZILLERI R M ET AL: "A novel application of a Pd(0)-catalyzed nucleophilic substitution reaction to the regio- and stereoselective synthesis of lactam analogues of epothilone natural products" JOURNAL OF THE AMERICAN CHEMICAL SOCIETY, AMERICAN CHEMICAL SOCIETY, NEW YORK, USA, vol. 122, 1 January 2000 (2000-01-01), pages 8890-8897, XP002421787 ISSN: 0002-7863
- MOORE S ET AL: "Synthesis and some properties and antitumor effects of the actinomycin lactam analog, (di(1-L-alpha, beta-diaminopropionic))actinomycin D1." JOURNAL OF MEDICINAL CHEMISTRY JUN 1976, vol. 19, no. 6, June 1976 (1976-06), pages 766-772, ISSN: 0022-2623

## Beschreibung

Die Erfindung betrifft Lysobactinamide und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W.Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Gram-positiven Erregern eingesetzt. Durch das wachsende Auftreten von resistenten Keimen, z.B. Methicillin-resistenten Staphylokokken, Penicillin-resistenten Pneumokokken und Vancomycin-resistenten Enterokokken (F. Baquero, Gram-positive resistance: challenge for the development of new antibiotics, J. Antimicrob. Chemother., 1997, 39, Suppl A:1-6; A.P. Johnson, D.M. Livermore, G.S. Tillotson, Antimicrobial susceptibility of Gram-positive bacteria: what's current, what's anticipated?, J. Hosp. Infect., 2001, (49), Suppl A: 3-11) sowie jüngst auch erstmals Vancomycin-resistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen.

Der Naturstoff Lysobactin und einige Derivate sind als antibakteriell wirksam beschrieben in US 4,754,018. Die Isolierung und antibakterielle Wirksamkeit von Lysobactin ist auch in EP-A-196 042 und JP 01132600 beschrieben. WO04/099239 beschreibt Derivate des Lysobactins mit antibakterieller Wirksamkeit.

Die antibakterielle Wirkung von Lysobactin und Katanosin A wird weiterhin beschrieben in O'Sullivan, J. et al., J. Antibiot. 1988, 41, 1740 - 1744, Bonner, D. P. et al., J. Antibiot. 1988, 41, 1745 - 1751, Shoji, J. et al., J. Antibiot. 1988, 41, 713 - 718 und Tymiak, A. A. et al., J. Org. Chem. 1989, 54, 1149 - 1157.

US 6,380,156 B1 offenbart ein synthetisches Verfahren zur Herstellung von Analoga von Didemnin A insbesondere des Amino-Hip-Analogons von Didemnin A, das auch als "AIP Didemnin A" bekannt ist.

Barrett et al., Tetrahedron Letters 42(2001), 703-705, beschreiben eine Synthese für ein Lactam-Analog des antimykotisch wirksamen Antibiotikums FR901469.

Moore et al., J. Med. Chem., 1976, Band 19, Nr. 6, 766-772 beschreiben die Synthese und einige Eigenschaften eines Lactam-Analogons von Actinomycin D, welches als potenzielles chemotherapeutisches Mittel in der Antitumortherapie vorgesehen ist.

Die Stabilität eines Wirkstoffs ist ein wichtiger Parameter für dessen Eignung als Arzneimittel. Die Stabilität spielt u.a. eine Rolle bei der Lagerung und der Darreichung von Arzneimitteln. Viele Naturstoffe zeigen eine für Arzneimittel unzureichende Stabilität.

Das antibakteriell wirksame Depsipeptid Lysobactin hydrolysiert im wässrigen neutralen bis basischen Milieu (pH > 7) innerhalb von Tagen. Hierbei entsteht das - antibakteriell unwirksame - am Lacton geöffnete "*open*-Lysobactin". Wünschenswert sind deshalb wirksame Analoga des Lysobactins mit einer höheren Ringstabilität.

Eine Aufgabe der vorliegenden Erfindung ist es, alternative Verbindungen zu Lysobactin mit vergleichbarer oder verbesserter antibakterieller Wirkung, besserer Verträglichkeit, z.B. geringerer Nephrotoxizität, und verbesserter Stabilität in wässrigem neutralen bis basischen Milieu zur Behandlung von bakteriellen Erkrankungen bei Menschen und Tieren zur Verfügung zu stellen.

Im Rahmen dieser Erfindung wurde überraschenderweise gefunden, daß Lysobactinamide (cyclische Nonapeptidamide) eine analoge antibakterielle Wirkung haben wie Lysobactin und hydrolysestabil in wässrigem neutralen bis basischen Milieu sind. Lysobactinamide sind bisher nicht beschriebene Aza-Analoga des Lysobactins, bei denen die zentrale Lactonfunktionalität durch eine Lactamfunktionalität ersetzt ist.

Gegenstand der Erfindung sind Verbindungen der Formel 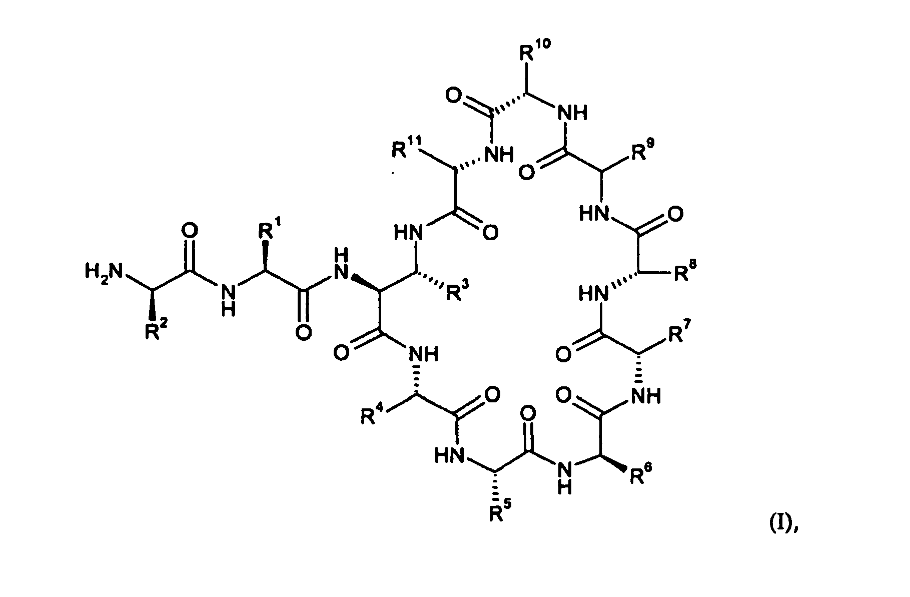 in welcher
- R¹: 2-Methytprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
- R²: 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
- R³: Wasserstoff, C₁-C₄-Alkyl, C₃-C₇-Cycloalkyl, C₃-C₇-Cycloalkylmethyl, Phenyl, Benzyl, 5- oder 6-gliedriges Heteroaryl oder 5- oder 6-gliedriges Heteroarylmethyl bedeutet,
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl, C₁-C₄-Alkylaminocarbonyl und über Stickstoff gebundenes 5- oder 6-gliedriges Heterocyclyl,
- R⁴: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R⁵: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R⁶: C₁-C₆-Alkyl bedeutet,
wobei Alkyl substituiert ist mit einem Substituenten ausgewählt aus der Gruppe bestehend aus Amino, 1,4,5,6-Tetrahydropyrimidin-2-ylamino, [Amino(imino)methyl]amino, 2-Pyridyl, 3-Pyridyl und 4-Pyridyl,
- R⁷: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl oder Trimethylsilylmethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R⁸: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R⁹: Wasserstoff, C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R¹⁰: Wasserstoff, C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R¹¹: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
und ihre Salze, ihre Solvate und die Solvate ihrer Salze,
ausgenommen die Verbindungen der Formel 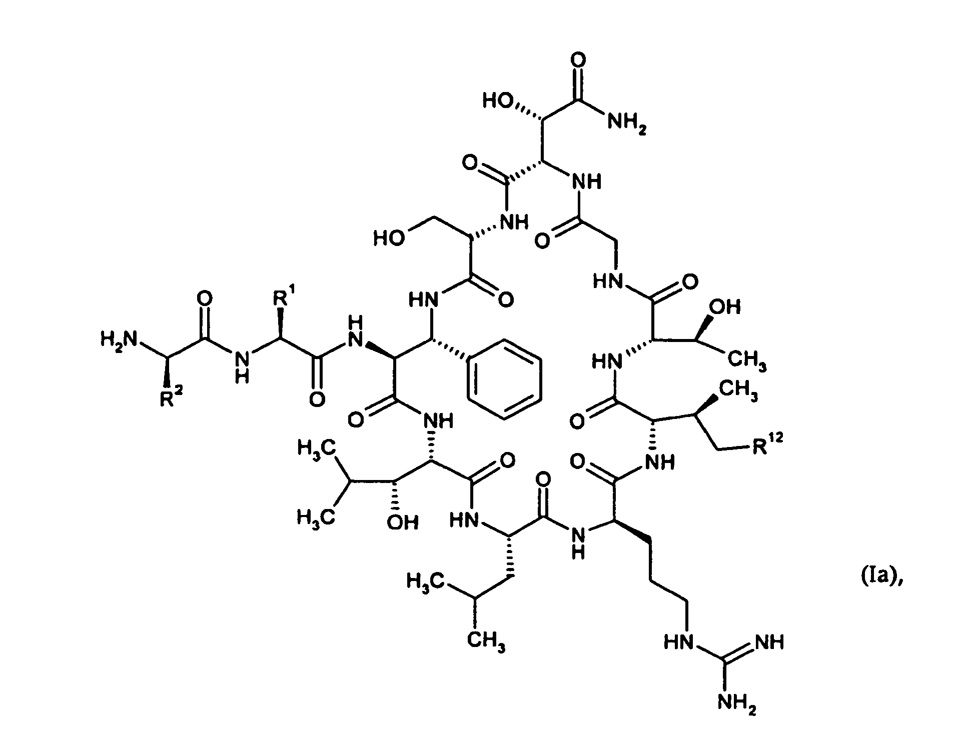 in welcher
- R¹: 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
- R²: 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
und
- R¹²: Wasserstoff oder Methyl bedeutet,
und ihre Salze, ihre Solvate und die Solvate ihrer Salze.

Erfindungsgemäße Verbindungen sind die Verbindungen der Formel (I) und deren Salze, Solvate, Solvate der Salze und Prodrugs, die von Formel (I) umfassten Verbindungen der nachfolgend genannten Formeln und deren Salze, Solvate, Solvate der Salze und Prodrugs sowie die von Formel (I) umfassten, nachfolgend als Ausführungsbeispiele genannten Verbindungen und deren Salze, Solvate, Solvate der Salze und Prodrugs, soweit es sich bei den von Formel (I) umfassten, nachfolgend genannten Verbindungen nicht bereits um Salze, Solvate, Solvate der Salze und Prodrugs handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können oder Mischsalze. Unter Mischsalz wird im Rahmen der vorliegenden Erfindung ein Additionssalz verstanden, das zwei oder mehrere unterschiedliche Säuren bzw. Basen enthält, wie z. B. ein Trifluoracetat-Mesylat-Salz.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkoxycarbonyl und Alkylaminocarbonyl steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, 2,2-Dimethylprop-1-yl, 2,2-Dimethylbut-1-yl, n-Pentyl und n-Hexyl.
Alkoxy steht beispielhaft und vorzugsweise für Methoxy, Ethoxy, n-Propoxy, Isopropoxy, *tert*-Butoxy, n-Pentoxy und n-Hexoxy.
Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N*,*N*-Dimethylamino, *N*,*N*-Diethylamino, *N*-Ethyl-*N*-methylamino, *N-*Methyl-*N*-n-propylamino, *N*-Isopropyl-*N*-n-propylamino, *N*-*tert*-Butyl-*N*-methylamino, *N*-Ethyl-*N*-n-pentylamino und N-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-*tert*-Butyl-*N*-methylaminocarbonyl, *N*-Ethyl-*N*-n-pentylaminocarbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylamino-carbonylrest mit jeweils 1 bis 3 Kohlenstoffatomen pro Alkylsubstituent.
Cycloalkyl steht für eine Cycloalkylgruppe mit in der Regel 3 bis 7 Kohlenstoffatomen, beispielhaft und vorzugsweise für Cyclopropyl, Cyclobutyl, Cyclopentyl, Cyclohexyl und Cycloheptyl.
Heterocyclyl steht für einen monocyclischen, heterocyclischen Rest mit 5 oder 6 Ringatomen und bis zu 3 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein und stehen beispielhaft und vorzugsweise für Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl.
Heteroaryl steht für einen aromatischen monocyclischen Rest mit 5 oder 6 Ringatomen und bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl und Pyridazinyl.
Halogen steht für Fluor, Chlor, Brom und Jod, bevorzugt Fluor und Chlor.

Bevorzugt sind Verbindungen der Formel (I), in welcher
- R¹: 2-Methylprop-1-yl, 2,2-Dimethylprop-1-yl, 2,2-Dimethylbut-1-yl, Trimethylsilylmethyl, Benzyl, 2-Pyridylmethyl oder 3-Pyridylmethyl bedeutet,
wobei Benzyl, 2-Pyridylmethyl und 3-Pyridylmethyl substituiert sein können mit 1 oder 2 Substituenten unabhängig voneinander ausgewählt aus der Gruppe bestehend aus Halogen, Trifluormethyl und Methyl,
- R²: 2-Methylprop-1-yl, 2,2-Dimethylprop-1-yl, 2,2-Dimethylbut-1-yl, Trimethylsilylmethyl, Benzyl, 2-Pyridylmethyl oder 3-Pyridylmethyl bedeutet,
wobei Benzyl, 2-Pyridylmethyl und 3-Pyridylmethyl substituiert sein können mit 1 oder 2 Substituenten unabhängig voneinander ausgewählt aus der Gruppe bestehend aus Halogen, Trifluormethyl und Methyl,
- R³: C₁-C₄-Alkyl, Phenyl, Benzyl, 2-Pyridyl, 3-Pyridyl oder 4-Pyridyl bedeutet,
wobei Phenyl, Benzyl, 2-Pyridyl, 3-Pyridyl und 4-Pyridyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
- R⁴: C₁-C₆-Alkyl, C₃-C₇-Cycloalkylmethyl, Benzyl, 5- oder 6-gliedriges Heteroarylmethyl oder Trimethylsilylmethyl bedeutet,
wobei Alkyl substituiert sein kann mit einem Substituenten Hydroxy,
und
wobei Cycloalkylmethyl, Benzyl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄- Alkylamino, Hydroxycarbonyl, C₁-C₄-Alkoxycarbonyl, Aminocarbonyl und C₁-C₄-Alkylaminocarbonyl,
- R⁵: C₁-C₆-Alkyl, C₃-C₇-Cycloalkylmethyl, Phenyl, Benzyl, 5- oder 6-gliedriges Heteroarylmethyl oder Trimethylsilylmethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl] amino,
und
wobei Cycloalkylmethyl, Phenyl, Benzyl und Heteroarylmethyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
- R⁶: C₁-C₆-Alkyl bedeutet,
wobei Alkyl substituiert ist mit einem Substituenten ausgewählt aus der Gruppe bestehend aus Amino, 1,4,5,6-Tetrahydropyrimidin-2-ylamino, [Amino(imino)methyl]amino, 2-Pyridyl, 3-Pyridyl und 4-Pyridyl,
- R⁷: C₁-C₆-Alkyl, C₃-C₇-Cycloalkylmethyl, Phenyl, Benzyl, 5- oder 6-gliedriges Heteroarylmethyl oder Trimethylsilylmethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkylmethyl, Phenyl, Benzyl und Heteroarylmethyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
- R⁸: C₁-C₆-Alkyl, C₃-C₇-Cycloalkylmethyl, Benzyl, 5- oder 6-gliedriges Heteroarylmethyl oder Trimethylsilylmethyl bedeutet,
wobei Alkyl substituiert sein kann mit einem Substituenten Hydroxy,
und
wobei Cycloalkylmethyl, Benzyl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
- R⁹: Wasserstoff, C₁-C₆-Alkyl, C₃-C₇-Cycloalkyl, Phenyl, Trimethylsilylmethyl oder 2-Amino-2-oxoethyl bedeutet,
wobei Methyl substituiert sein kann mit einem Substituenten ausgewählt aus der Gruppe bestehend aus Hydroxy, Amino und Mercapto,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino,
- R¹⁰: Wasserstoff, C₁-C₆-Alkyl, C₃-C₇-Cycloalkyl, Phenyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino,
- R¹¹: Methyl oder Ethyl bedeutet,
wobei Methyl und Ethyl substituiert sein können mit einem Substituenten ausgewählt aus der Gruppe bestehend aus Hydroxy, Amino, Mercapto, 1,4,5,6-Tetrahydropyrimidin-2-ylamino und [Amino(imino)methyl]amino,
und ihre Salze, ihre Solvate und die Solvate ihrer Salze.

Bevorzugt sind auch Verbindungen der Formel (I), in welcher
- R¹: 2,2-Dimethylprop-1-yl, 2,2-Dimethylbut-1-yl, Trimethylsilylmethyl oder 3-Pyridylmethyl bedeutet,
- R²: 2,2-Dimethylprop-1-yl, 2,2-Dimethylbut-1-yl, Trimethylsilylmethyl oder 3-Pyridylmethyl bedeutet,
- R³: C₁-C₄-Alkyl, 3-Pyridyl oder Phenyl bedeutet,
wobei 3-Pyridyl oder Phenyl substituiert sein können mit einem Substituenten ausgewählt aus der Gruppe bestehend aus Halogen, Cyano, Methyl, Methoxy, Dimethylamino und Diethylamino,
- R⁴: -CH(OH)-C₁-C₅-Alkyl oder -CH(OH)Phenyl bedeutet,
wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino,
- R⁵: C₁-C₆-Alkyl, C₃-C₇-Cycloalkylmethyl, Benzyl oder Trimethylsilylmethyl bedeutet,
- R⁶: linerares C₂-C₄-Alkyl bedeutet,
wobei Alkyl substituiert ist mit einem Substituenten ausgewählt aus der Gruppe bestehend aus Amino, 1,4,5,6-Tetrahydropyrimidin-2-ylamino und [Amino(imino)methyl]amino,
- R⁷: C₁-C₆-Alkyl, C₃-C₇-Cycloalkylmethyl, Benzyl oder Trimethylsilylmethyl bedeutet,
- R⁸: C₁-C₄-Alkyl, -CH₂-OH, -CH(OH)-C₁-C₅-Alkyl oder -CH(OH)Phenyl bedeutet, wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino,
- R⁹: Wasserstoff, C₁-C₆-Alkyl, Hydroxymethyl oder 2-Amino-2-oxoethyl bedeutet,
- R¹⁰: Wasserstoff, C₁-C₄-Alkyl, 2-Amino-2-oxoethyl oder 2-Amino-1-hydroxy-2-oxoethyl bedeutet,
wobei C₁-C₄-Alkyl substituiert sein können mit einem Substituenten ausgewählt aus der Gruppe bestehend aus Amino und Hydroxy, mit der Ausnahme, dass R¹⁰ nicht 4-Aminobut-1-yl bedeutet,
- R¹¹: Methyl bedeutet, wobei Methyl substituiert ist mit einem Substituenten ausgewählt aus der Gruppe bestehend aus Hydroxy und Amino,
und ihre Salze, ihre Solvate und die Solvate ihrer Salze.

Bevorzugt sind auch Verbindungen der Formel (I), in denen
- R¹: 2-Methylprop-1-yl, 2,2-Dimethylprop-1-yl, 2,2-Dimethylbut-1-yl, Trimethylsilylmethyl oder Benzyl bedeutet,
wobei Benzyl substituiert sein kann mit 1 oder 2 Substituenten unabhängig voneinander ausgewählt aus der Gruppe bestehend aus Halogen, Trifluormethyl und Methyl,
oder eines ihrer Salze, ihrer Solvate oder der Solvate ihrer Salze.

Bevorzugt sind auch Verbindungen der Formel (I), in denen
- R¹: 2,2-Dimethylprop-1-yl, 2,2-Dimethylbut-1-yl oder Trimethylsilylmethyl bedeutet,
oder eines ihrer Salze, ihrer Solvate oder der Solvate ihrer Salze.

Bevorzugt sind auch Verbindungen der Formel (I), in denen
- R²: 2,2-Dimethylprop-1-yl oder 3-Pyridylmethyl bedeutet,
oder eines ihrer Salze, ihrer Solvate oder der Solvate ihrer Salze.

Bevorzugt sind auch Verbindungen der Formel (I), in denen
- R³: Phenyl, Benzyl, 2-Pyridyl, 3-Pyridyl oder 4-Pyridyl bedeutet,
wobei Phenyl, Benzyl, 2-Pyridyl, 3-Pyridyl und 4-Pyridyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
oder eines ihrer Salze, ihrer Solvate oder der Solvate ihrer Salze.

Bevorzugt sind auch Verbindungen der Formel (I), in denen
- R³: Phenyl bedeutet,
wobei Phenyl substituiert sein kann mit einem Substituenten ausgewählt aus der Gruppe bestehend aus Halogen, Cyano, Methyl, Methoxy, Dimethylamino und Diethylamino,
oder eines ihrer Salze, ihrer Solvate oder der Solvate ihrer Salze.

Bevorzugt sind auch Verbindungen der Formel (I), in denen
- R⁸: -CH(OH)-C₁-C₅-Alkyl oder -CH(OH)Phenyl bedeutet,
wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino, und
oder eines ihrer Salze, ihrer Solvate oder der Solvate ihrer Salze.

Bevorzugt sind auch Verbindungen der Formel (I), in denen
- R¹⁰: C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
oder eines ihrer Salze, ihrer Solvate oder der Solvate ihrer Salze.

Bevorzugt sind auch Verbindungen der Formel (I), in denen
- R¹⁰: C₁-C₆-Alkyl, C₃-C₇-Cycloalkyl, Phenyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino,
oder eines ihrer Salze, ihrer Solvate oder der Solvate ihrer Salze.

Bevorzugt sind auch Verbindungen der Formel (I), in denen
- R¹⁰: Methyl, Ethyl, 2-Amino-2-oxoethyl oder 2-Amino-1-hydroxy-2-oxoethyl bedeutet,
wobei Methyl und Ethyl substituiert sein können mit einem Substituenten Hydroxy,
oder eines ihrer Salze, ihrer Solvate oder der Solvate ihrer Salze.

Gegenstand der Erfindung ist weiterhin ein Verfahren zur Herstellung der Verbindungen der Formeln (I), wobei
[A] Verbindungen der Formel 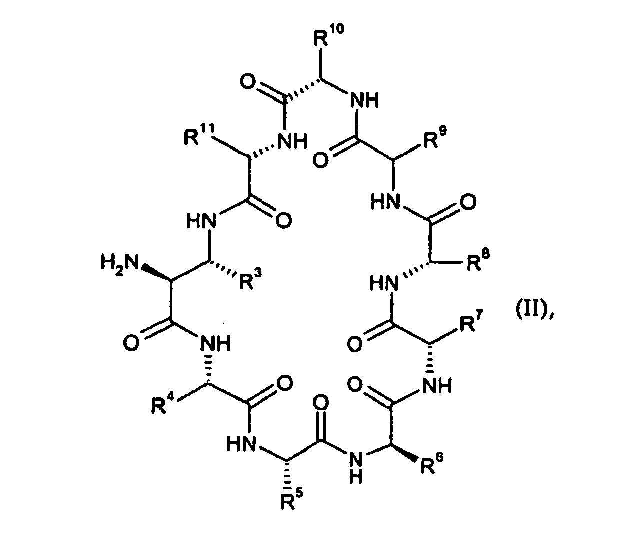 in welcher
   R³, R⁴, R⁵, R⁶, R⁷, R⁸, R⁹, R¹⁰ und R¹¹ die oben angegebene Bedeutung haben,
   zunächst mit Verbindungen der Formel 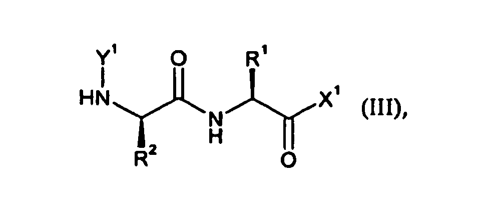 in welcher
   - R¹ und R²: die oben angegebene Bedeutung haben,
   - Y¹: *tert*-Butoxycarbonyl oder Benzyloxycarbonyl bedeutet, und
   - X¹: Halogen, bevorzugt Brom, Chlor oder Fluor, oder Hydroxy bedeutet,
   und anschließend mit einer Säure und/oder durch Hydrogenolyse umgesetzt werden,
   oder
[B] Verbindungen der Formel 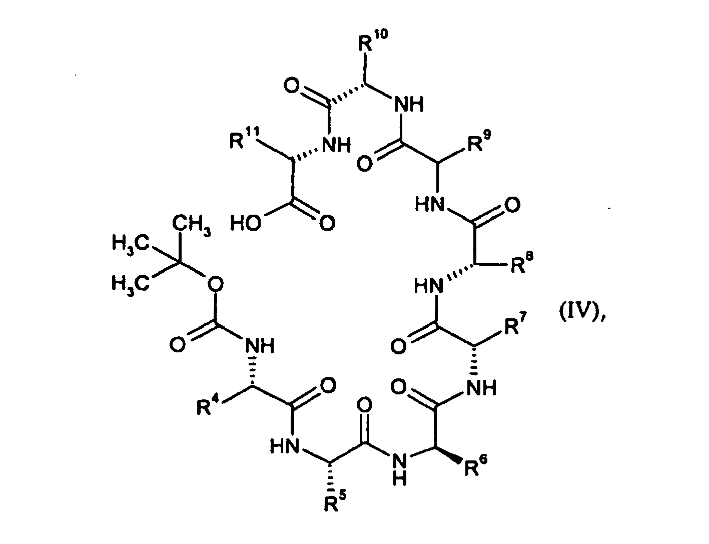 in welcher
   R⁴, R⁵, R⁶, R⁷, R⁸, R⁹, R¹⁰ und R¹¹ die oben angegebene Bedeutung haben,
   zunächst mit Verbindungen der Formel 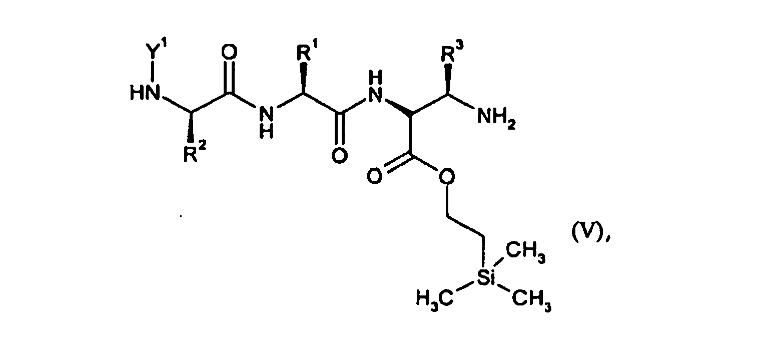 in welcher
   - R¹, R² und R³: die oben angegebene Bedeutung haben, und
   - Y¹: *tert*-Butoxycarbonyl oder Benzyloxycarbonyl bedeutet,
   und anschließend in einer 4-stufigen Synthese
   a) mit einem Fluorid-Reagenz wie Tetrabutylammoniumfluorid,
   b) mit einer Säure,
   c) mit einem Dehydratisierungsreagenz, gegebenenfalls in Gegenwart einer Base, und
   d) durch Hydrogenolyse
umgesetzt werden.

### Verfahren [A]:

Falls X¹ für Halogen steht, erfolgt die Umsetzung der ersten Stufe im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan, Dimethylacetamid, *N*-Methylpyrrolidin oder Dimethylformamid, bevorzugt ist Pyridin oder Dimethylformamid.

Basen sind beispielsweise Triethylamin, Diisopropylethylamin oder *N*-Methylmorpholin, bevorzugt ist Düsopropylethylamin.

Falls X¹ für Hydroxy steht, erfolgt die Umsetzung der ersten Stufe im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoffe wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N*,*N*'-Diethyl-, *N*,*N*'-Dipropyl-, *N*,*N*'-Diisopropyl-, *N*,*N*'-Dicyclohexylcarbodiimid, *N*-(3-Dimethylaminoisopropyl)-N'-ethylcarbodiimid-hydrochlorid (EDC), *N*-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert* Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N*,*N*,*N*',*N*'-tetramethyluronium-hexafluorophosphat (HBTU), 2-(2-Oxo-1-(2H)-pyridyl)-1,1,3,3-tetramethyluroniumtetrafluoroborat (TPTU), *O-*(1H-6-Chlorbenzotriazol-1-yl)-1,1,3,3-tetramethyluronium-Tetrafluoroborat (TCTU) oder *O-*(7-Azabenzotriazol-1-yl)-*N*,*N*,*N*',*N*'-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Umsetzung mit einer Säure in der zweiten Stufe des Verfahrens erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Hydrogenolyse in der zweiten Stufe des Verfahrens erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Essigsäure oder wässriger Salzsäure, bevorzugt ist ein Gemisch aus Ethanol, Wasser und Essigsäure oder ein Gemisch aus iso-Propanol und wässriger Salzsäure.

### Verfahren [B]:

Die Umsetzung der Verbindungen der Formel (IV) mit Verbindungen der Formel (V) erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N*'-Diethyl-, *N*,*N*'-Dipropyl-, *N*,*N*'-Diisopropyl-, *N*,*N*'-Dicyclohexylcarbodiimid, *N*-(3-Dimethylaminoisopropyl)-*N*'-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O-*(Benzotriazol-1-yl)-*N*,*N*,*N*',*N*'-tetramethyluronium-hexafluorophosphat (HBTU), 2-(2-Oxo-1-(2H)-pyridyl)-1,1,3,3-tetramethyluroniumtetrafluoroborat (TPTU), *O-*(1H-6-Chlorbenzotriazol-1-yl)-1,1,3,3-tetramethyluronium-Tetrafluoroborat (TCTU) oder *O-*(7-Azabenzotriazol-1-yl)-*N*,*N*,*N*',*N*'-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N-*Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N-*Methylmorpholin, *N-*Methylpiperidin, 4-Dimethylaminopyridin oder Diisopropylethylamin.

Vorzugsweise wird die Kondensation mit HATU oder mit einer Mischung aus EDC und HOBt durchgeführt.

Die Umsetzung mit Tetrabutylammoniumfluorid in der ersten Stufe (a) des weiteren Verfahrens erfolgt im Allgemeinen in inerten Lösungsmitteln, bevorzugt in einem Temperaturbereich von 0°C bis 40°C bei Normaldruck.

Inerte Lösungsmittel sind beispielsweise Halogenkohlenwasserstoffe wie Dichlormethan oder Trichlormethan, oder Ether wie Tetrahydrofuran oder Dioxan. Besonders bevorzugt ist Tetrahydrofuran.

Die Umsetzung mit einer Säure in der zweiten Stufe (b) des Verfahrens erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Umsetzung in der dritten Stufe (c) des Verfahrens erfolgt analog zu der Umsetzung von Verbindungen der Formel (IV) mit Verbindungen der Formel (V).

Die Hydrogenolyse in der vierten Stufe (d) des Verfahrens erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Essigsäure oder wässriger Salzsäure, bevorzugt ist ein Gemisch aus Ethanol, Wasser und Essigsäure oder ein Gemisch aus iso-Propanol und wässriger Salzsäure.

Die Verbindungen der Formel (II) sind bekannt oder und können hergestellt werden, indem Verbindungen der Formel 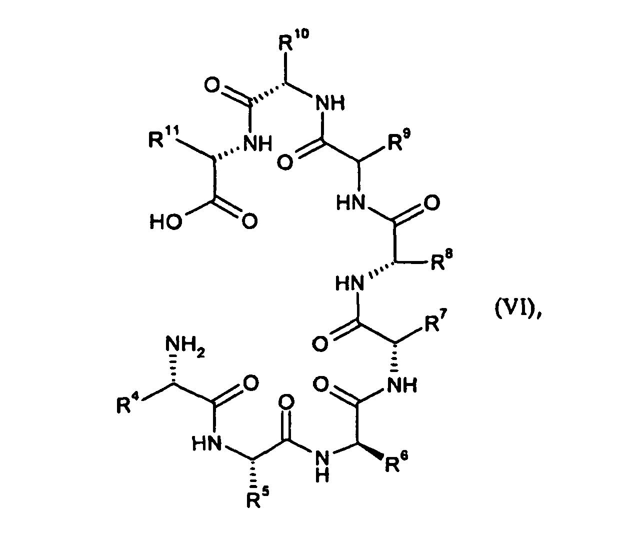 in welcher
R⁴, R⁵, R⁶, R⁷, R⁸, R⁹, R¹⁰ und R¹¹ die oben angegebene Bedeutung haben,
zunächst mit Verbindungen der Formel 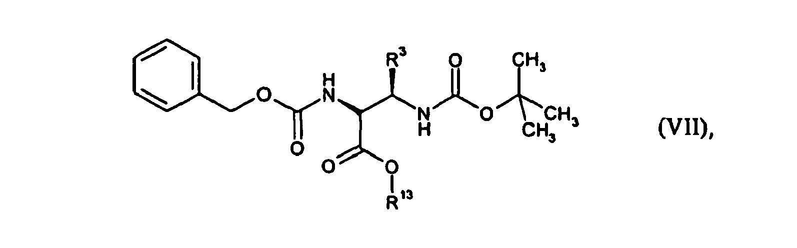 in welcher
- R³: die oben angegebene Bedeutung hat, und
- R¹³: Cyanomethyl, p-Nitrophenyl, o-Nitrophenyl, 2,4-Dinitrophenyl, 2,4,5-Trichlorphenyl, Pentachlorphenyl, Pentafluorphenyl (Pfp), N-Hydroxyphtalimidyl, N-Hydroxysuccinimidyl (O-Su), 1-Hydroxypiperidinyl oder 5-Chlor-8-hydroxychinolinyl bedeutet,
und anschließend in einer 3-stufigen Synthese
a) mit einer Säure,
b) mit einem Dehydratisierungsreagenz, gegebenenfalls in Gegenwart einer Base, und
c) durch Hydrogenolyse
umgesetzt werden.

Die Umsetzung der Verbindungen der Formel (VI) mit Verbindungen der Formel (VII) erfolgt im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan, Dimethylacetamid, *N-*Methylpyrrolidin oder Dimethylformamid, bevorzugt ist Pyridin oder Dimethylformamid.

Die Umsetzung mit einer Säure in der ersten Stufe (a) des Verfahrens erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Umsetzung in der zweiten Stufe (b) des Verfahrens erfolgt analog zu der Umsetzung von Verbindungen der Formel (IV) mit Verbindungen der Formel (V).

Die Hydrogenolyse in der dritten Stufe (c) des Verfahrens erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Essigsäure oder wässriger Salzsäure, bevorzugt ist ein Gemisch aus Ethanol, Wasser und Essigsäure oder ein Gemisch aus iso-Propanol und wässriger Salzsäure.

Die Verbindungen der Formeln (III), (V) und (VII) sind bekannt oder lassen sich nach bekannten oder nach den unten beschriebenen Verfahren aus den entsprechenden Edukten synthetisieren.

Die Verbindungen der Formeln (IV) und (VI) sind bekannt oder lassen sich in Lösung unter Verwendung entsprechender Schutzgruppen oder an der Festen Phase nach bekannten Peptidsyntheseverfahren oder nach den unten beschriebenen Verfahren aus den entsprechenden Edukten synthetisieren.

Die Herstellung der erfindungsgemäßen Verbindungen kann durch folgende Syntheseschemata verdeutlicht werden.

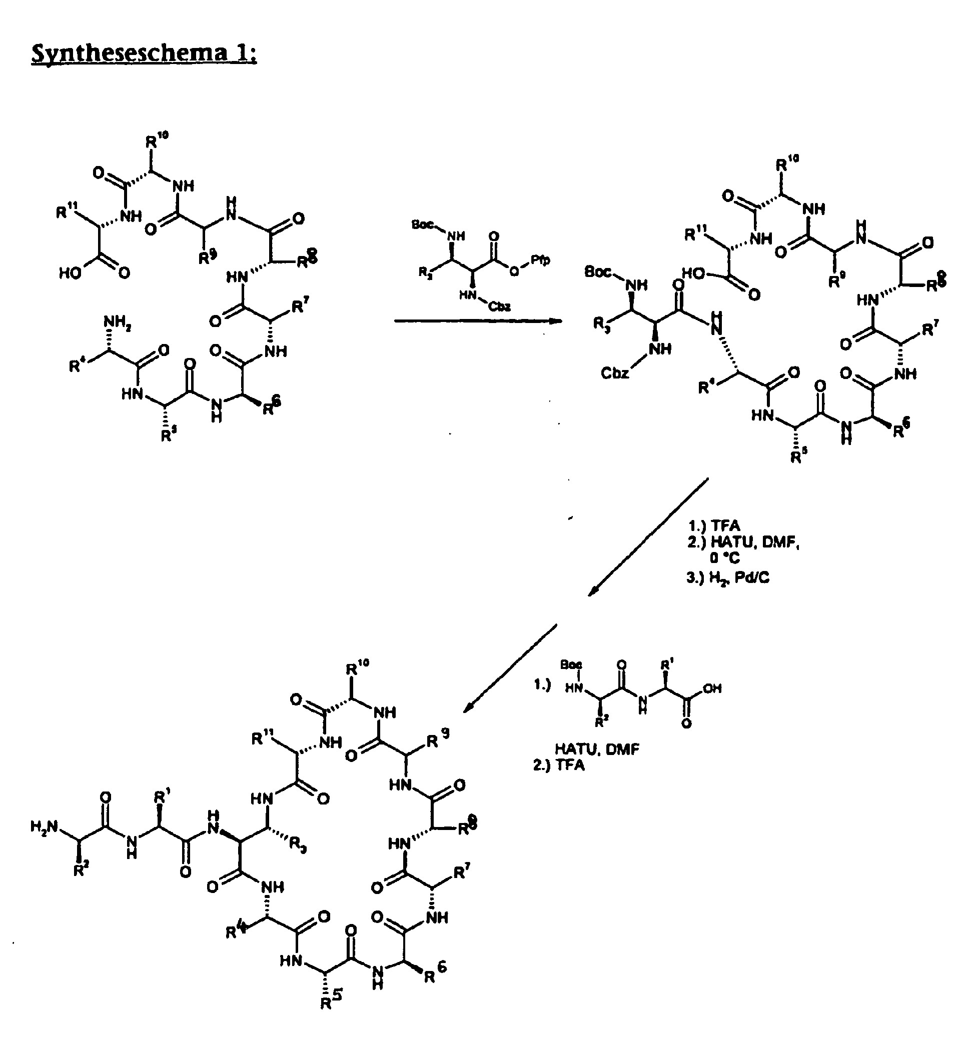

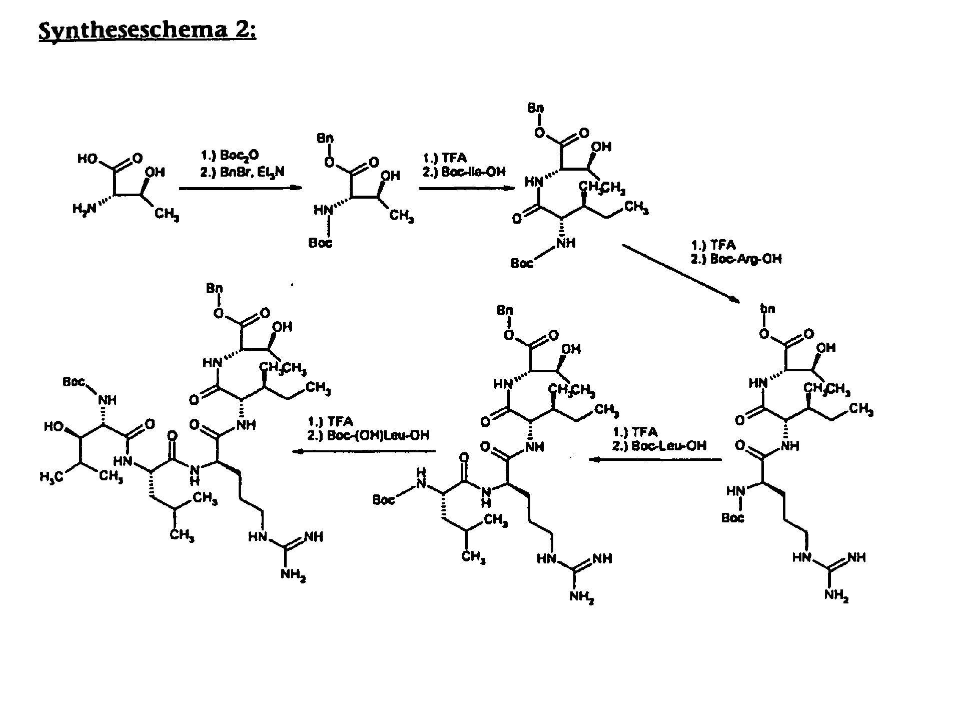

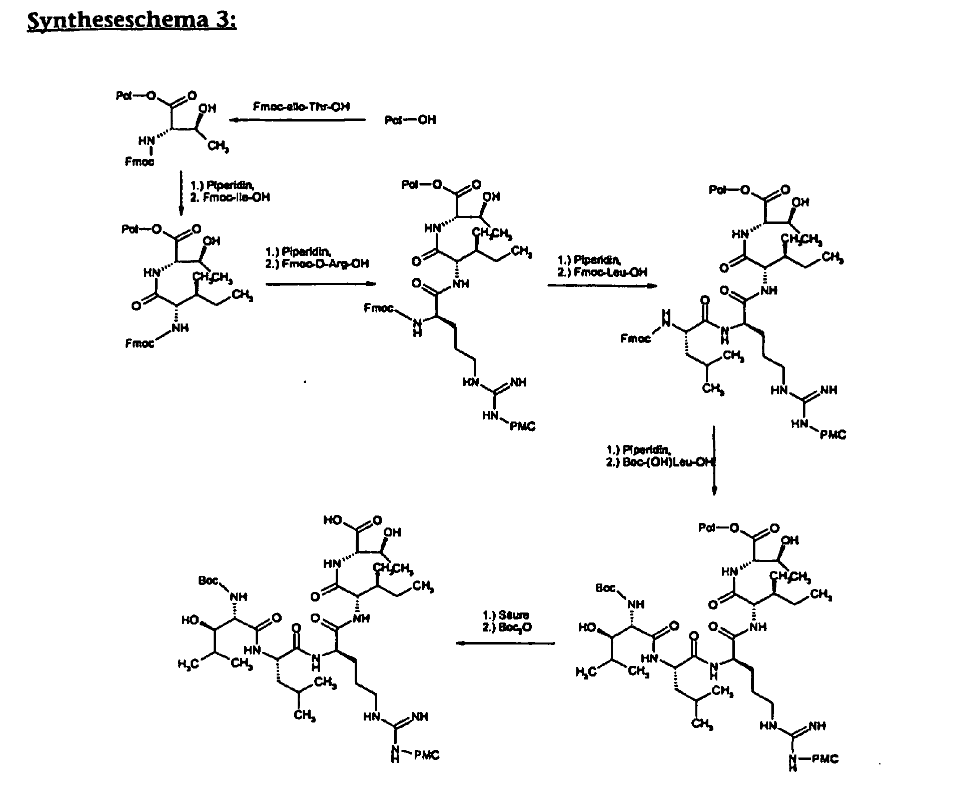

Die erfindungsgemäßen Verbindungen zeigen ein nicht vorhersehbares, wertvolles pharmakologisches Wirkspektrum. Sie zeigen eine antibakterielle Wirkung.

Sie eignen sich daher zur Verwendung als Arzneimittel zur Behandlung und/oder Prophylaxe von Krankheiten bei Menschen und Tieren.

Die erfindungsgemäßen Verbindungen zeichnen sich durch eine geringere Nephrotoxizität gegenüber Lysobactin aus.

Die erfindungsgemäßen Verbindungen zeichnen sich durch eine verbesserte Stabilität in wässrigem neutralen bis basischen Milieu aus. Diese Eigenschaft verbessert die Lagerung der erfindungsgemäßen Verbindungen und die Darreichung als Arzneimittel.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakterielle Zellwand (Murein sacculus) bzw. den dazugehörigen Enzymsystemen sind, beispielsweise gegen die folgenden Erreger oder gegen Mischungen der folgenden Erreger:
Gram-negative Kokken (*Neisseria gonorrhoeae*) sowie Gram-negative Stäbchen wie Enterobakteriaceen, z.B. *Escherichia coli, Hämophilus influenzae,* Pseudomonas, Klebsiella, Citrobacter (*C. freundii*, *C. divernis*), Salmonella und Shigella; ferner Enterobacter (E. *aerogenes*, *E. agglomerans*), Hafnia, Serratia (*S. marcescens*), Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis*, Vertreter der Gattung Peptococcus, Peptostreptococcus sowie die Gattung Clostridium; ferner Mykobakterien, z.B. *M. tuberculosus.* Besonders ausgeprägte Wirkung zeigen die erfindungsgemäßen Verbindungen gegen Gram-positive Kokken, z.B. Staphylokokken (*S. aureus*, *S*. *epidermidis*, *S*. *haemolyticus*, *S*. *carnosus*), Enterokokken (*E. faecalis*, *E. faecium*) und Streptokokken (*S*. *agalactiae*, *S*. *pneumoniae*, *S*. *pyogenes*).

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:
Infektionskrankheiten beim Menschen wie z.B. unkomplizierte und komplizierte Harnwegsinfekte, unkomplizierte Haut- und Oberflächeninfektionen, komplizierte Haut- und Weichteilinfektionen, im Hospital und ambulant erworbene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, desweiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:
Schwein: Diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): *E. coli*-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Weiterer Gegenstand der vorliegenden Erfindung ist der Einsatz der erfindungsgemäßen Verbindungen zur Behandlung und/oder Prophylaxe von Erkrankungen, insbesondere von bakteriellen Infektionskrankheiten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von bakteriellen Erkrankungen geeignet sind.

Weiterer Gegenstand der vorliegenden Erfindung ist die Verwendung einer antibakteriell wirksamen Menge der erfindungsgemäßen Verbindungen in einem Verfahren zur Behandlung und/oder Prophylaxe von Erkrankungen, insbesondere der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die beiliegenden Zeichnungen zeigen Stabilitätsdiagramme von ausgewählten Beispielverbindungen sowie Lysobactin als Vergleichsbeispiel. Es zeigen:
- Fig. 1:: Eine Kurve für den Konzentrationsverlauf für Lysobactin sowie eines Abbauproduktes von Lysobactin über die Zeit in wässriger, leicht alkalischer Lösung.
- Fig. 2: Eine Kurve für den Konzentrationsverlauf für die Verbindung von Beispiel 19 über die Zeit in wässriger, leicht alkalischer Lösung.
- Fig. 3: Eine Kurve für den Konzentrationsverlauf für die Verbindung von Beispiel 23 über die Zeit in wässriger, leicht alkalischer Lösung.
- Fig. 4: Eine Kurve für den Konzentrationsverlauf für die Verbindung von Beispiel 25 über die Zeit in wässriger, leicht alkalischer Lösung.
- Fig. 5: Eine Kurve für den Konzentrationsverlauf für die Verbindung von Beispiel 40 über die Zeit in wässriger, leicht alkalischer Lösung.

### A. Beispiele

### Abkürzungen

- abs.: Absolut
- Äq.: Äquivalent(en)
- BHI: Brain Heart Infusion
- Boc: *tert*-Butoxycarbonyl
- Bsp.: Beispiel
- DCC: Dicyclohexylcarbodümid
- DCI: direkte chemische Ionisation (bei MS)
- DCM: Dichlormethan
- DIEA: *N*,*N-*Diisopropytethylamin
- DMAP: 4-*N*,*N*-Dimethylaminopyridin
- DMSO: Dimethylsulfoxid
- DMF: *N*,*N*-Dimethylformamid
- d. Th.: der Theorie
- EDC: 1-Ethyl-3-(3-dimethylaminopropyl)carbodiimid (auch EDCI)
- EDCxHCl: 1-Ethyl-3-(3-dimethylaminopropyl)carbodiimidhydrochlorid
- EDTA: Ethylendiamintetraessigsäure (Ethylene Diamine Tetraacetic Acid)
- EE: Ethylacetat (Essigsäureethylester)
- EI: Elektronenstoß-Ionisation (bei MS)
- ESI: Elektrospray-Ionisation (bei MS)
- Fa.: Firma
- ges.: gesättigt(er)
- h: Stunde(n)
- HATU: *O-*(7-Azabenzotriazol-1-yl)-*N*,*N*,*N*',*N*'-tetramethyluroniumhexafluorphosphat
- HOBt: 1-Hydroxybenzotriazol
- HPLC: Hochdruck-, Hochleistungsflüssigchromatographie
- konz.: Konzentriert
- LC-MS: Flüssigchromatographie-gekoppelte Massenspektroskopie
- LDA: Lithium-Diisopropylamid
- LHMDS: Lithiumhexamethyldisilazid
- LLA(3-cyclohexyl): D-Leu-Leu-(3-cyclohexyl)Ala
- LLF: D-Leu-Leu-Phe
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- MTBE: Methyl-*tert*-butylether
- NCCLS: National Committee for Clinical Laboratory Standards
- neg.: negativ
- NMM: *N*-Methylmorpholin
- NMR: Kernresonanzspektroskopie (nuclear magnetic resonance)
- org.: organische(n)
- p.a.: pro analysi
- Pd: Palladium
- Pd-C: Palladium auf Kohle
- pos.: positiv
- proz.: prozentig
- PTFE: Polytetrafluorethylen
- quant.: quantitativ
- RP-HPLC: Reverse Phase HPLC
- RT: Raumtemperatur
- R_{f}: Retentionsfaktor (bei Dünnschichtchromatographie)
- Rₜ: Retentionszeit (bei HPLC)
- TBAF: Tetrabutylammoniumfluorid
- TBTU: *O-*(Benzotriazol-1-yl)-*N*,*N*,*N*',*N*'-tetramethyluronium-Tetrafluoroborat
- TCTU: *O-*(1H-6-Chlorbenzotriazol-1-yl)-1,1,3,3-tetramethyluronium-Tetrafluoroborat
- TFA: Trifluoressigsäure
- TFE: 2,2,2-Triftuorethanol
- THF: Tetrahydrofuran
- TMG: *N*,*N*,*N*,*N*-Tetramethylguanidin
- TMSE: 2-(Trimethylsilyl)ethyl
- TOF: Flugzeit (time of flight)
- UV: Ultraviolett
- VRSA: Vancomycin-resistenter *Stapylococcus aureus*
- wässr.: wässrig(e)
- XPHOS: Dicyclohexyl(2',4',6'-trüsopropylbiphenyl-2-yl)phosphin
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical journal 1984, 219, 345-373. Sowie zitierte Literatur.

### Material und Methoden

### Analytisch Methoden

Methode 1: Gerätetyp HPLC: HP 1100 Series; UV DAD; Säule: Phenomenex Synergi 2 µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 2: Gerätetyp HPLC: HP 1050 Series; UV DAD; Säule: Phenomenex Synergi 2 µ Max-RP Mercury 20 mm x 4 mm; Eluent A: 1 1 Wasser + 0.05% TFA, Eluent B: 1 1 Acetonitril; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 3: Gerätetyp HPLC: HP 1050/1100 Series; UV DAD 1100 Series; Säule: Kromasil C₁₈, 60 x 2.1 mm, 3.5 µm; Eluent A: Wasser/0.5% Perchlorsäure, Eluent B: Acetonitril; Gradient: 0-0.5 min 2%B, 0.5-4.5 min 2-90%B, 4.5-6.5 min 90%B, 6.5-6.7 min 90-2%B, 6.7-7.5 min 2%B; Fluss: 0.75 ml/min, Ofen: 30°C, UV-Detektion 210 nm.

Methode 4: Instrument: HP 1100 mit DAD-Detektion; Säule: Kromasil 100 RP-18, 60 mm x 2,1 mm, 3.5µm; Eluent A: 5 ml Perchlorsäure (70%ig) /1 Wasser, Eluent B: Acetonitril; Gradient: 0 min 2%B, 0.5 min 2%B, 4.5 min 90%B, 9 min 90%B, 9.2 min. 2%B, 10 Min 2%B; Fluß: 0.75 ml/min; SäulenTemp.: 30°C; Detektion: UV 210 nm

Methode 5: Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.05% 70% Perchlorsäure in Wasser; Eluent B: Acetonitril; Fluss: 2.00 ml/min; Gradient: 0-1 min 10% B, Rampe, 4-5 min 90% B, Rampe, 5.5 min 10% B.

Methode 6: Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.01% TFA in Wasser; Eluent B: 0.01% TFA in Acetonitril; Fluss: 2.00 ml/min; Gradient: 0-1 min 10% B, Rampe, 4-5 min 90% B, Rampe, 5.5 min 10% B.

Methode 7: Agilent 1100 mit DAD (G1315B), Binary Pump (G1312A), Autosampler (G1313A), Entgaser (G1379A) und Säulenthermostat (G1316A); Säule: Synergi 4 Hydro-RP 80A, 4.6 x 150 x 5 mm; Ofentemperatur: 30°C; Eluens A: Wasser + 0.05% 70% Perchlorsäure; Eluens B: Acetonitril; Fluss: 2.00 ml/min; Gradient: 0-1 min 10% B, Rampe, 4-5 min 90% B, Rampe, 5.5 min 10% B.

Methode 8: Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Eclipse XDB-C8 4.6 x 150 x 5 mm; Säulentemperatur: 40°C; Eluent A: 0.05% 70% Perchlorsäure in Wasser; Eluent B: Methanol; Fluss: 2.00 ml/min; Isokratisch: 0-7 min 55% B.

Methode 9: Gerätetyp HPLC: HP 1050 Series; UV DAD; Säule: Zorbax 300 mSB-C18 3.5 µ, 4.6 mm x 150 mm; Eluent A: 11 Wasser + 0.1% TFA, Eluent B: 400 ml Acetonitril/600 ml Wasser + 0.1% TFA; Gradient: 0.0 min 100%A, 1.3 min 10%B, 18.0 min 80%B, 20.0 min 80%B, 21.0 min 100%B, 25.0 min 100%B, 26.0 min 0%B, 30.0 min 0%B. Fluss: 1 ml/min; Ofen: 40°C; UV-Detektion: 210 nm.

Methode 10: Gerätetyp HPLC: HP 1050 Series; UV DAD; Säule: Zorbax 300 mSB-C18 3.5 µ, 4.6 mm x 150 mm; Eluent A: 11 Wasser + 0.1% TFA, Eluent B: Acetonitril; Gradient: 0.0 min 100%A, 15.0 min 100%B, 17.0 min 100%B, 18.0 min 100%A. Fluss: 1 ml/min; Ofen: 40°C; UV-Detektion: 210 nm.

Methode 11: Gerätetyp HPLC: HP 1050 Series; UV DAD; Säule: Zorbax 300 mSB-C18 3.5 µ, 4.6 mm x 150 mm; Eluent A: 11 Wasser + 0.1% TFA, Eluent B: 400 ml Acetonitril/600 ml Wasser + 0.1% TFA; Gradient: 0.0 min 100%A, 2.0 min 10%B, 50.0 min 80%B, 52.0 min 100%B, 55.0 min 100%A, 60.0 min 100%A. Fluss: 1 ml/min; Ofen: 40°C; UV-Detektion: 210 nm.

Methode 12: Gerätetyp HPLC: HP 1050 Series; Säule SymmetryPrep^{™}C₁₈, Fa. Waters, 50 x 2.1 mm, 3.5 µm; Eluent A: Wasser/0.1% TFA, Eluent B: Acetonitril; Gradient: 0-3 min 0%B, 3.01-9 min 100%B, 9.01-11 min 100%B, 11.01-12 min 0-100%A ; Ofen: 40°C; Fluss: 0.4 ml/min; UV-Detektion: 210 nm.

Methode 13: Gerätetyp HPLC: HP 1100 Series; UV DAD; Säule: Chromolith Speed ROD RP18; Eluent A: 11 Wasser + 0.05% TFA, Eluent B: 11 Acetonitril; Gradient: 0.0-0.5 min 95%A, 0.51-3.0 min 5-95%B, 3.01-3.80 min 95%A; Fluss: 5 ml/min; Ofen: 40°C; UV-Detektion: 210 nm.

Methode 14: Säule (Stahlsäule, Dimension 250 x 4.6 mm); stationäre Phase (chirales Kieselsäure/Polyamid Komposit KBD 5326, basierend auf dem Selektor Poly(*N-*methacryolyl-L-leucin-dicyclopropylmethylamid); Eluent Essigsäureethylester, isokratisch; Fluss: 1 ml/min; Temperatur: 25°C; UV-Detektion: 220 nm; Probe: Injektion von 10 µl.

Methode 15: Stationäre Phase (Chiralcel OD); Eluent i-Hexan/i-PrOH : 9/1, isokratisch; Fluss: 2 ml/min; Temperatur: 25°C; UV-Detektion: 254 nm.

Methode 16: Gerätetyp HPLC: HP 1100 Series; Säule: Chiralpack AD-H 250 mm x 4.6 mm; Eluent A: iso-Hexan, Eluent B: Ethanol + 0.2%TFA + 1% H2O; Isokratisch; Fluss: 1 ml/min; Ofen: 40°C; UV-Detektion: 230 nm.

Methode 17: Daicel Chiralpak AD-H 5 µm 250mm x 4.6 mm, Hexan/ 2-Propanol/Methanol 85/6/9, Fluss: 2.0 ml/min, UV-Detektion bei 254 nm.

Methode 18: Gerätetyp MS: Micromass ZQ; Gerätetyp HPLC: Waters Alliance 2795/HP 1100; Säule: Phenomenex Synergi 2 µ Hydro-RP Mercury 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 19: Gerätetyp MS: Micromass ZQ; Gerätetyp HPLC: Waters Alliance 2795/HP 1100; Säule: Phenomenex Gemini 3 µ C-18 100 Å, 30 mm x 3 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 20: UV-Detektion: 210 nm. Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 21: Instrument: Micromass Quattro LCZ mit HPLC Agilent Serie 1100; Säule: Phenomenex Synergi 2 µ Hydro-RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

Methode 22: Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0min 100%A → 0.2min 100%A → 2.9min 30%A → 3.1min 10%A →5.5min 10%A; Ofen: 50°C; Fluss: 0.8 ml/min; UV-Detektion: 210 nm.

Methode 23: Gerätetyp MS: Micromass LCT (ESI pos./neg.); Gerätetyp HPLC: HP 1100 Series; UV DAD 1100 Series; Säule SymmetryPrep™C₁₈, Fa. Waters, 50 x 2.1 mm, 3.5 µm; Eluent A: Wasser/0.1% Ameisensäure, Eluent B: Acetonitril/0.1% Ameisensäure; Gradient: 0-1 min 0%B, 1-6 min 0-90%B, 6-8 min 90-100%B, 8-10 min 100%B, 10-10.1 min 100-0%B, 10.1-12 min 0%B, anschließend Regeneration der Chromatographiesäule. Ofen: 40°C, Fluss: 0.5 ml/min (bei 10.1 min kurzfristig auf 1 ml/min), UV-Detektion: 210 nm.

Methode 24: *TOF-HR-MS-ESI*+ Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Fa. Harvard Apparatus) für die Probenzuführung verwendet. Als Standard dient Leucin-Enkephalin (Tyr-Gly-Gly-Phe-Leu).

Methode 25: Die *MALDI-MS*/*MS-Untersuchungen* werden auf einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.

### Präparative Chromatographie

Methode 26: Gerät: Gilson Abimed HPLC; binäres Pumpensystem; Säule: Nucleodur C₁₈ Gravity, Fa. Macherey-Nagel, 5 µm; 250 x 21 mm; Eluent A: Wasser/0.05%-0.1% TFA, Eluent B: Acetonitril; Gradient: 0-8 min 5%B, 8-40 min 5-60%B, 40-60 min 60%B, 60-75 min 60-100%B, 75-80 min 100%B, anschließend Regeneration der Chromatographiesäule; Fluss: 7-15 ml/min; UV Detektor 210 nm.

Methode 27: Gerät: Gilson Abimed HPLC; binäres Pumpensystem; Säule: Nucleodur C₁₈ Gravity, Fa. Macherey-Nagel, 5 µm; 250 x 21 mm; Eluent A: Wasser/0.05%-0.1% TFA, Eluent B: Acetonitril; Gradient: 0-8 min 5%B, 8-40 min 5-60%B, 40-60 min 60%B, 60-75 min 60-100%B, 75-80 min 100%B, anschließend Regeneration der Chromatographiesäule; Fluss: 7-15 ml/min; UV Detektor 210 nm.

Methode 28: Gerät: Gilson Abimed HPLC; binäres Pumpensystem; Säule: Nucleodur C₁₈ Gravity, Fa. Macherey-Nagel, 5 µm; 250 × 40 mm; Eluent A: Wasser/0.05-0.1% TFA, Eluent B: Acetonitril/ 0.05-0.1% TFA; Gradient: 0-10 min 10%B, 10-24 min 10-30%B, 24-28 min 30-50%B, 28-35 min 50%B, 35-45 min 50-60%B, 45-53 min 60-70%B, 53-60 min 60-90%B, 60-70 min 100%B, anschließend Regeneration der Chromatographiesäule; Fluss: 15-45 ml/min; UV Detektor 210 nm.

Methode 29: Gerät: Gilson Abimed HPLC; binäres Pumpensystem; Säule: Nucleodur C₁₈ Gravity, Fa. Macherey-Nagel, 5 µm; 250 x 21 mm; Eluent A: Wasser/0.2% Essigsäure, Eluent B: Acetonitril/0.2%Essigsäure; Gradient: 0-10 min 10%B, 10-24 min 10-30%B, 24-28 min 30-50%B, 28-35 min 50%B, 35-45 min 50-60%B, 45-53 min 60-70%B, 53-60 min 60-90%B, 60-70 min 100%B, anschließend Regeneration der Chromatographiesäule; Fluss: 7-15 ml/min; UV Detektor 210 nm.

Methode 30: Gerät: Gilson Abimed HPLC; binäres Pumpensystem; Säule: Nucleodur C₁₈ Gravity, Fa. Macherey-Nagel, 5 µm; 250 × 40 mm; Eluent A: Wasser/0.2% Essigsäure, Eluent B: Acetonitril/0.2%Essigsäure; Gradient: 0-10 min 10%B, 10-24 min 10-30%B, 24-28 min 30-50%B, 28-35 min 50%B, 35-45 min 50-60%B, 45-53 min 60-70%B, 53-60 min 60-90%B, 60-70 min 100%B, anschließend Regeneration der Chromatographiesäule; Fluss: 15-45 ml/min; UV Detektor 210 nm.

Methode 31: Gerät: Gilson Abimed HPLC; binäres Pumpensystem; Säule: Kromasil-100A C₁₈, 5 µm; 250 x 30 mm; Eluent A: Wasser/0.25% Essigsäure, Eluent B: Acetonitril; Gradient: 0-3 min 5%B, 3-30 min 5-100%B, 30-38 min 100%B, anschließend Regeneration der Chromatographiesäule; Fluss: 25 ml/min; UV Detektor 210 nm.

Methode 32: Gerät: Gilson Abimed HPLC; binäres Pumpensystem; Säule: Kromasil-100A C₁₈, 5 µm; 250 x 30 mm; Eluent A: Wasser/0.05-0.5% TFA, Eluent B: Acetonitril; Gradient: 0-5 min 5%B, 5.01-10 min 10%B, 10.01-20 min 40%B, 20.01-27 min 50%B, 27.01-40 min 60%B, 40.01-45 min 90%B, 45.01-60 min 100%B; Fluss: 15-60 ml/min; UV Detektor 210 nm.

Methode 33: Gilson Abimed HPLC; UV-Detector 210 nm; Säule: Kromasil RP-18 5 µm, 100 Å, 250 x 20 mm; Eluens A: Wasser + 0.05% TFA, Eluens B: Acetonitril + 0.05% TFA: Fluss: 10 ml/min; 0-3 min 10% B, Rampe, 30-38 min 90% B, 38-45 min 10% B.

Methode 34: Gilson Abimed HPLC; UV-Detector 210 nm; Säule: Gromsil ODS-4HE 10 µm, 250 x 40 mm; Eluens A: Wasser + 0.05% TFA, Eluens B: Acetonitril + 0.05% TFA: Fluss: 20 ml/min; 0-3 min 10% B, Rampe, 30-35 min 90% B, 35-40 min 90% B.

Methode 35: Gerät: Gilson Abimed HPLC; binäres Pumpensystem; Säule: Waters Symmetry-Prep^{™} C₁₈, 7 µm, 300 x 19 mm; Eluent A: Wasser/0.2% Eisessig, Eluent B: Acetonitril; Gradient: 0-8 min 5%B, 8-25 min 5-100%B, 25.01-40 min 100%B, anschließend Regeneration der Chromatographiesäule; Fluss: 15 ml/min; UV Detektor 210 nm.

Methode 36: Gerät: Gilson Abimed HPLC; binäres Pumpensystem; Säule: Waters Symmetry-Prep^{™} C₁₈, 7 µm, 300 x 19 mm; Eluent A: Wasser/0.1% TFA, Eluent B: Acetonitril; Gradient: 0-5 min 5%B, 5.01-32 min 5-100%B, 32.01-35 min 100%B, anschließende Regeneration der Chromatographiesäule; Fluss: 15 ml/min; UV Detektor 210 nm.

Methode 37: Säule: Kromasil-100 C₁₈, 5 µm; 250 x 20 mm; Eluent: 25% Wasser + 0.2 TFA/ 75% Acetonitril; isokratisch; 0-8.5 min; Fluss: 25 ml/min; UV Detektor 220 nm; Temperatur 30°C.

Methode 38: Säule (Stahlsäule, Dimension 250 x 30 mm); stationäre Phase (chirales Kieselsäure/ Polyamid Komposit KBD 5326, basierend auf dem Selektor Poly(*N-*methacryolyl-L-leucin-dicyclopropylmethylamid); Eluent Essigsäureethylester, isokratisch; Fluss: 25 ml/min; Temperatur: 24°C; UV-Detektion: 225 nm; Probe: Repetitive Injektion von 2000 µl.

Methode 39: Säule (Stahlsäule, Dimension 430 x 75 mm); stationäre Phase (chirales Kieselsäure/ Polyamid basierend auf dem Selektor Poly(*N*-methacryloyl-D-leucindicyclopropylmethylamid); Eluent iso-Hexan (80%) / THF (20%), isokratisch; Fluss: 200 ml/min; Temperatur: 24°C; UV-Detektion: 254 nm; Probe: Repetitive Injektion von 10000 µl.

Methode 40: Säule (Stahlsäule, Dimension 250 x 20 mm); stationäre Phase (Daicel Chiralpak AD-H, 5 µm); Eluent: iso-Hexan (70%)/ 2-Propanol + 0.2% Essigsäure + 1% Wasser (30%), isokratisch; Fluss: 15 ml/min; Temperatur: 29°C; UV-Detektion: 220 nm; Probe: Repetitive Injektion von 750 µl.

Methode 41: Gilson Abimed HPLC; Säule: Daicel Chiralpak AD-H 5 µm; 250 x 20 mm; Eluent A: iso-Hexan, Eluent B: 0.2% Essigsäure/ 1% Wasser/ 2-Propanol; isokratisch; Fluss: 15 ml/min; UV-Detektor 212 nm

Methode 42: Säule (Stahlsäule, Dimension 500x40 mm); stationäre Phase (Daicel Chiralpak AD 20 µm), iso-Hexan/ Isopropanol/ Methanol 95/2/3, Fluss: 100 ml/min, UV-Detektion: 230 nm.

Methode 43: Gerät: Gilson Abimed HPLC; UV Detektor 210 nm; binäres Pumpensystem; Säule: SymmetryPrep^{™}C₁₈, Firma Waters, 7 µm; 300 mm x 19 mm; Fluß: 25 ml/min; Eluent A: Wasser/0.2% TFA, Eluent B: Acetonitril; Gradient: 0-10 min 15-65%B, anschließend Regeneration der Chromatographiesäule.

Methode 44: Gilson Abimed HPLC; UV-Detektor 210 nm; Säule: Waters Symmetry-Prep^{™} C-18, 7 µm, 300 x 19 mm; Eluens A: Wasser + 0.05% TFA, Eluens B: Acetonitril + 0.05% TFA: Fluss: 10 ml/min; 0-3 min 10% B, Rampe, 30.38 min 90% B, 38-45 min 10% B.

Methode 45: Gelchromatographie wird ohne Druck an Sephadex LH-20 (Fa. Pharmacia) durchgeführt. Es wird nach UV-Aktivität (UV Detektor für 254 nm, Fa. Knauer) fraktioniert (Fraktionssammler ISCO Foxy 200). Säulendimensionen: 32 x 7 cm (1000-100 µmol Maßstab); 30 x 4 cm (100-10 µmol Maßstab); 25 x 2 cm (10-1 µmol Maßstab). Als Eluent wird Methanol verwendet.

Methode 46: Gilson Abimed HPLC; UV-Detektor 210 nm; Säule: Biotage Flash40 RP-18 oder kompatibles Modul Varian Metaflash C18 40M, 35 µm, 150 x 40 mm; Eluens A: Wasser + 0.05% TFA, Eluens B: Acetonitril + 0.05% TFA: Fluss: 40 ml/min; 0-3 min 10% B, Rampe, 30-38 min 90% B, 38-45 min 10% B.

Methode 47: Präparative Trennung der Chymotrypsinspaltung der Fragmente 1-3 und 4-11 des Dihydrolysobactins über Reversed Phase-Chromatographie. Säule: Source 15-RPC (GE-HealthCare), Durchmesser 5 cm, Länge 12 cm, Volumen 235 ml. Eluent A: 0.1% Essigsäure in Wasser. Eluent B: 0.1% Essigsäure in Methanol. Flussrate: 30 ml/min. UV-Detektion: 215 nm, 280 nm. Beladung: ca. 6-7 mg Gesamtprotein/ml Harz. Probenaufgabe: Die Säule mit Eluent A equilibrieren, die 0.2 µm filtrierte Spaltansatz-Lösung aufgeben und mit Eluent A nachwaschen. Elution: 0-45% in ca. 18 Säulenvolumen, Nachwaschen mit ca. 1.3 Säulenvolumen 100%B. Fraktionierung: Ab Start Elution Fraktionen à 50 ml sammeln. CIP: Säule bei reduzierter Flussrate (5 ml/min) mit 2 Säulenvolumen 1 N Natronlauge waschen.

### Spezielle Methoden zur Analyse von Aminosäuren und Peptiden

Methode 48: Die Aminosäureanalysen werden mit einem Aminosäureanalysator LC 3000 von Eppendorf/Biotronik durchgeführt. Ein leicht modifiziertes Standardtrennprogramm von Eppendorf/Biotronik wird benutzt. Die Trennprogramme und die Funktion des Analysators sind ausführlich im Handbuch des Gerätes beschrieben (Handbuch des Aminosäureanalysators LC 3000, Wissenschaftliche Geräte GmbH Biotronik, Maintal, 1996).

Methode 49: Die Kapillarelektrophorese erlaubt die Trennung von Peptiden und Proteinen aufgrund ihrer Ladung im elektrischen Feld. Die Güte der Trennung hängt dabei vom Puffer, dem pH-Wert, der Temperatur und den verwendeten Additiven ab. Als Kapillaren werden sogenannte "fused silica" Säulen mit einem Innendurchmesser von 50-100 µm eingesetzt. Diese Methode ist ein sehr gutes Kriterium für die Beurteilung der Reinheit eines Peptids und zur Kontrolle der Entstehung von enzymatischen Spaltprodukten. Die Peptide Dihydro-Lysobactin und Octahydro-Lysobactin eluieren bei ca. 21 min, das Fragment 4-11 bei ca. 18 min, 1-3 (LLF) bei ca. 24 min, 1-3 (LLA(3-cyclohexyl)) bei ca. 22 min, die deamidierten Formen als Doppelpeak bei ca. 30 min (1-11) und 24 min (4-11) von der Kapillarsäule. Man sieht deutlich die starke Zunahme der deamidierten Produkte nach 24 h im Puffer. Ca. 4 ng der enzymatischen Spaltprodukte bzw. der Ausgangsverbindungen Dihydro-Lysobactin und Octahydro-Lysobactin oder des Gemisch werden mittels Kapillarelektrophorese an einer Glassäule (Länge 72 cm, Innendurchmesser 50 µm) untersucht. Bedingungen: Stromstärke 90 µA, Säulentemperatur 25°C, 100 mM Phosphatpuffer pH 3.0, UV-Detektion 210 nm, Aufgabe unter Druck 3 Sekunden.

Methode 50: 3 nmol in 60% Acetonitril/0.1% TFA gelöste Fragmente werden auf ein Sequenzer-sheet, das mit Polybren® vorinkubiert ist, geladen. Die Proteine werden mit dem normalen Sequenzerzyklus sequenziert. Die PTH-Aminosäuren werden mittels Online-HPLC mit Hilfe eines 40 pmol PTH-Standards identifiziert. Die nicht proteinogenen Aminosäuren werden durch ihre relative Lage zu den Standardaminosäuren identifiziert. Die Reinheit der Peptide wird über die Aminosäure des 1.PTH-Zyklus abgeschätzt. Die verschiedenen Peptide werden über 4 bis 12 Stufen sequenziert.

### Weitere analytische und präparative Methoden

Methode 51: Instrument: Micromass Quattro LCZ mit HPLC Agilent Serie 1100; Säule: Phenomenex Onyx Monolithic C18, 100 mm x 3 mm. Eluent A: 1 l Wasser + 0.5 mL 50%ige Ameisensäure, Eluent B: 1 1 Acetonitril + 0.5 mL 50%ige Ameisensäure; Gradient: 0.0 min 90%A → 2 min 65%A → 4.5 min 5%A → 6 min 5%A; Fluss: 2 mL/min; Ofen: 40°C; UV-Detektion: 208- 400 nm.

Methode 52: Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 1 l Wasser + 0.5 mL 50%ige Ameisensäure, Eluent B: 1 l Acetonitril + 0.5 mL 50%ige Ameisensäure; Gradient: 0.0 min 90%A → 0.1 min 90%A → 3.0 min 5%A → 4.0 min 5%A → 4.01 min 90%A; Fluss: 2 mL/min; Ofen: 50°C; UV-Detektion: 210 nm.

Methode 53: Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.01% TFA in Wasser; Eluent B: 0.01% TFA in Acetonitril; Fluss: 2.00 mL/min; Gradient: 0-1 min 10% B, Rampe, 10-14 min 90%, Rekonditionierung.

Methode 54: Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.01% TFA in Wasser; Eluent B: 0.01% TFA in Acetonitril; Fluss: 1.40 mL/min; Gradient: 0 min 10% B, Rampe, 2.5-5 min 90%, Rekonditionierung.

Methode 55: Säule (Stahlsäule, Dimension 250 x 20 mm); stationäre Phase (Daicel Chiralpak AD-H, 5 µm); Eluent: iso-Hexan (90%)/ Ethanol + 0.2% AcOH + 1% H2O (10%), isokratisch; Fluss: 15 mL/min; Temperatur: 30°C; UV-Detektion: 220 nm; Probe: Repetitive Injektion von 500 µL.

Methode 56: Säule (Stahlsäule, Dimension 250 x 4.6 mm); stationäre Phase (Daicel Chiralpak AD-H, 5 µm); Eluent: iso-Hexan (90%)/ Ethanol + 0.2% AcOH + 1% H2O (10%), isokratisch; Fluss: 1.0 mL/min; Temperatur: 30°C; UV-Detektion: 220 nm; Probe: Injektion von 10 µL.

Methode 57: Säule: Säule: Kromasil 100 C18, 60x2,1mm 3,5µm, Säulenofen 30°C, Fluss: 0,75 mL/min, Detektor: 210nm, Laufzeit: 15 min; Eluent A: Wasser mit 5mL HClO₄ / Liter Wasser), Eluent B: Acetonitril; Gradient: 0-1 min 2%B, Rampe, 9-13 min, 98% B, Rekonditionierung.

Methode 58: (Chirale präparative HPLC, Chiralpak AD-H 40-60); Säule (Stahlsäule, Dimension 250 x 20 mm); stationäre Phase (Daicel Chiralpak AD-H, 5 µm); Eluent: iso-Hexan (40%)/ Ethanol + 0.2% AcOH + 1% H2O (60%), isokratisch; Fluss: 15 mL/min; Temperatur: 30°C; UV-Detektion: 220 nm; Probe: Repetitive Injektion von 500 µL.

Methode 59: (Chirale analytische HPLC, Chiralpak AD-H 40-60); Säule (Stahlsäule, Dimension 250 x 4.6 mm); stationäre Phase (Daicel Chiralpak AD-H, 5 µm); Eluent: iso-Hexan (40%)/ Ethanol + 0.2% AcOH + 1% H2O (60%), isokratisch; Fluss: 1.0 mL/min; Temperatur: 30°C; UV-Detektion: 220 nm; Probe: Injektion von 10 µL.

### Allgemeine Arbeitsvorschriften

Arbeitsvorschrift 1 (Abspaltung der Boc-Schutzgruppen mit TFA): Die Boc-geschützte Verbindung wird in Dichlormethan suspendiert (1/5-1/10 der Reaktionslösung) und anschließend unter Argonschutzgasatmosphäre mit 30% TFA in Dichlormethan (ca 1 ml/10 mg Edukt im 100-1 Millimol-Maßstab, ca. 1 ml/l mg im 100-1 Mikromol-Maßstab) versetzt und so lange bei RT gerührt bis das HPLC-Chromatogramm vollständigen Umsatz zeigt (z. B. Methode 1). Dann wird das Lösungsmittel im Vakuum abdestilliert, wobei die Badtemperatur 30°C nicht übersteigen soll. Das Rohprodukt wird in Toluol suspendiert, erneut am Rotationsverdampfer eingeengt und im Hochvakuum getrocknet. Diese Prozedur wird mehrfach wiederholt (zweimal bis fünfmal). Bei ungenügender Reinheit des Produktes wird gegebenenfalls chromatographisch, z.B. über die präparative HPLC oder über die Gelchromatographie, gereinigt.

Arbeitsvorschrift 2 (Abspaltung der Boc-Schutzgruppe mit 30% TFA in Dichlormethan); Das Ausgangsmaterial wird in 30% TFA (Lösung in Dichlormethan) aufgenommen und 30 min bei Raumtemperatur gerührt. Dann wird das Lösungsmittel im Vakuum abdestilliert, wobei die Badtemperatur 30°C nicht übersteigen soll. Das Produkt wird danach im Ölpumpenvakuum bis zur Gewichtskonstanz getrocknet.

Arbeitsvorschrift 3 (Abspaltung der Boc-Schutzgruppe, mit 4 N Salzsäure in Dioxan): Die *N*-(*tert*-Butoxycarbonyl)-geschützte Verbindung (1 mmol) wird in Dioxan (2-3 ml) unter Argonschutzgas-atmosphäre vorgelegt. Bei RT oder Eiskühlung und unter starkem Rühren wird 4 N Salzsäure in Dioxan (30 ml) zugetropft. Man rührt, bis die analytische HPLC (Methode 1) vollständigen Umsatz anzeigt (ca. 30 min bis 2 h). Das Reaktionsgemisch wird bei RT im Vakuum eingedampft. Das Rohprodukt wird in wenig Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert oder nach Hochvakuumtrocknung direkt weiter umgesetzt. Bei ungenügender Reinheit des Produktes wird gegebenenfalls chromatographisch, z.B. über die präparative HPLC oder über die Gelchromatographie, gereinigt.

Arbeitsvorschrift 4 (Hydrogenolytische Esterspaltung 1): Der peptidische Benzylester wird in Methanol oder Dioxan (ca. 3.10⁻⁴ - 2.10⁻³ mol/l) gelöst, anschließend wässrige 0.1%ige TFA oder 0.1N Salzsäure (6-10 Äq.) zugegeben und unter Argonschutzgasatmosphäre mit 10 proz. Palladium-Kohle (10 mol%) versetzt. Bei RT und Normaldruck wird so lange hydriert bis die analytische HPLC (z. B. Methode 1) vollständigen Umsatz anzeigt. Das Reaktionsgemisch wird filtriert (z. B. über Kieselgur, Celite^{®}), im Vakuum eingeengt und am Hochvakuum getrocknet. Bei ungenügender Reinheit des Produktes wird gegebenenfalls chromatographisch, z.B. über die präparative HPLC oder über die Gelchromatographie, gereinigt.

Arbeitsvorschrift 5 (Hydrogenolytische Esterspaltung 2): Der peptidische Benzylester wird in Methanol (ca. 3.10⁻⁴ - 2.10⁻³ mol/l) gelöst und unter Argonschutzgasatmosphäre mit 10%iger Palladium-Kohle (2-10 mol%) versetzt. Bei RT und Normaldruck wird so lange hydriert bis die analytische HPLC (z.B. Methode 1) vollständigen Umsatz anzeigt. Das Reaktionsgemisch wird filtriert (z. B. über Kieselgur, Celite^{®}), im Vakuum eingeengt und am Hochvakuum getrocknet. Bei ungenügender Reinheit des Produktes wird gegebenenfalls chromatographisch, z.B. über die präparative HPLC oder über die Gelchromatographie, gereinigt.

Arbeitsvorschrift 6 (hydrolytische Esterspaltung, Verseifung): Der Carbonsäureester (3 mmol) wird unter Argonschutzgasatmosphäre in THF/Wasser/DMF 200/100/2.5 (20 ml) vorgelegt. Bei 0°C wird unter strikter Temperaturkontrolle gepulvertes Lithiumhydroxid (3.6 mmol, 1.2 Äq.) portionsweise in die stark gerührte Lösung gegeben. Wird nach 2 h mittels analytischer HPLC (Methode 13) kein vollständiger Umsatz beobachtet, so wird erneut festes Lithiumhydroxid zugesetzt (3.3 mmol, 1.1 Äq.). Dieser Vorgang wird bis zu vollständigem Umsatz wiederholt, woraufhin man das Reaktionsgemisch bei 0°C mit 0.1 N wässriger Salzsäure auf pH 3-4 einstellt im Vakuum einengt und anschließend gefriertrocknet. Das Rohprodukt kann jetzt gelchromatographiert werden (z.B. Methode 45) und/oder mittels präparativer HPLC (z.B. Methode 31) feingereinigt werden.

Arbeitsvorschrift 7 (Peptidkupplung am Harz): Die an Chlortrityl-Harz gebundene Fmoc-geschützte Aminosäure wird mit 20%iger Piperidinlösung in DMF versetzt und 30 min geschüttelt. Anschließend wird die Lösung über eine Fritte abgesaugt, nochmals Piperidinlösung zugegeben und weitere 30 min geschüttelt. Man saugt die Lösung ab und wäscht das Harz mit DMF, Methanol, THF und Dichlormethan.

Das entschützte Polymer wird in DMF (pro 100 mg Harz 1 ml Lösungsmittel) suspendiert und mit der Fmoc-geschützten Aminosäure (1.3-2.0 Äq.), mit DIEA (2.0-3.0 Äq.) und mit TBTU (1.5-2.0 Äq.) in DMF gelöst versetzt. Das Harz wird über Nacht bei RT geschüttelt. Zur Aufarbeitung wird das Polymer über eine Fritte abgesaugt, anschließend mit DMF, THF und Dichlormethan gewaschen.

Nach der Probeabspaltung in Essigsäure/ Trifluorethanol/ Dichlormethan 1:1:3 kann das erhaltene Peptid durch die Analytik bestätigt werden.

Arbeitsvorschrift 8 (automatisierte Festphasensynthese am Chemspeed Roboter):

### Gerät:

Chemspeed ASW 2000, ausgerüstet mit 75 mL Reaktoren und direkter Absaugung in den Lösemittelabfall. Mischung über Vortexierung.

### Reagenzien:

Eingesetzt werden Aminosäuren mit einer *N*-[(9H-fluoren-9-ylmethoxy)carbonyl]-Schutzgruppe (Fmoc-Gruppe) an der *N*²-Position.

Bei folgenden Aminosäuren wird zusätzlich zur Fmoc-Schutzgruppe an der *N*²-Position eine weitere Schutzgruppe in der Seitenkette verwendet: Arginin und D-Arginin tragen jeweils eine [(2,2,5,7,8-pentamethyl-3,4-dihydro-2H-chromen-6-yl)sulfonylgruppe (PMC-Gruppe) an der Guanidinfunktion; Serin und Threonin sind als O³-*tert*Butylserin bzw -threonin seitenkettengeschützt. Die Aminogruppen in den Seitenketten von Lysin, Ornithin, 2,4-Diaminobuttersäure und 2,3-Diaminobuttersäure sind mit Benzyloxycarbonylgruppen (Cbz-Gruppen) geschützt.

### Durchführung:

Pro Reaktor wurden 1,7 g 2-Chlortritylchlorid-Harz (Iris Biotech, CAS No 42074-68-0) (vorbeladen mit einer -Fmoc-geschützten Aminosäure, 0.85 mmol/g) trocken vorgelegt, 17 mL DMF werden zugegeben und 30 min geschüttelt. Das DMF wird abgesaugt.

Zur Synthese der beschriebenen Octapeptide werden 7 Entschützungs-Kupplungszyklen durchgeführt, die jeweils aus den folgenden Schritten bestehen:

### 1.) Fmoc-Abspaltung

- 12,75 mL DMF und 4,25 mL Piperidin werden zugegeben.
- Es wird 30 min geschüttelt.
- Das DMF/Piperidingemisch wird abgesaugt.
- 12,75 mL DMF und 4,25 mL Piperidin werden zugegeben.
- Es wird 10 min geschüttelt.
- Das DMF/Piperidingemisch wird abgesaugt.

### 2.) Waschvorgang (5-fache Wiederholung)

- 17 mL DMF werden zugegeben
- Es wird 30 min geschüttelt.
- Das DMF wird abgesaugt.

### 3.) Aminosäure-Kupplung

- 10,2 mL der entsprechenden Fmoc-Aminosäure (1,5 Äquivalente.) gelöst in DMF werden zugegeben.
- 6,8 mL TBTU (2 Äquivalente) gelöst in DMF werden zugegeben.
- Diisopropylethylamin (3 Äquivalente) wird zugegeben.
- Es wird 2 h geschüttelt, dann wird das Reagenzgemisch abgesaugt.

### 4.) Waschvorgang (3-fache Wiederholung)

- 17 mL DMF werden zugegeben
- Es wird 30 min geschüttelt.
- Das DMF wird abgesaugt.

Nach dem letzten Reaktionsschritt werden die absaugten Produktharze mit 10 mL Dichlormethan auf ein Filter überführt, abfiltriert und zweimal mit je 5 mL Dichlormethan gewaschen.

Arbeitsvorschrift 9 (Abspaltung unter vollständiger Entschützung): Das Harz mit dem Peptid wird in einem Reaktionsgefäß mit Frittenboden mit 50% TFA in DCM (20 mL der Lösung für einen Ansatz der in Arbeitsvorschrift 8 beschriebenen Größe) versetzt, Trüsopropylsilan (100 µL) und Wasser (100 µL) werden zugesetzt und über Nacht bei Raumtemperatur geschüttelt. Dann saugt man die dunkle Lösung ab, wäscht das Harz noch mit 4 Portionen DCM, schüttelt dabei jeweils 2 min. und saugt abermals ab. Die Filtrate werden vereinigt und das Lösemittel abdestilliert. Der Rückstand wird mit MTBE verrührt, abgesaugt und in Methanol aufgenommen. Schließlich wird das Methanol im Vakuum abdestilliert.

Arbeitsvorschrift 10 (Kupplung mit Brückenkopf-Aktivester): Das Octapeptid und der Aktivester (Verbindung 17A oder Verbindung 277A (1.1 Äquivalente) werden bei 0°C in DMF (20-40 µL/µmol Octapeptid) gelöst und mit DIEA (4-5 Äq.) versetzt. Die Reaktionsmischung wird zwischen 90 min und 2 h bei Raumtemperatur weitergerührt. Der Ansatz wird in Acetonitril aufgenommen und chromatographisch gereinigt. Produkthaltige Fraktionen werden vereinigt und lyophilisiert.

Arbeitsvorschrift 11 (Cyclisierung): Die Ausgangsverbindung wird in DMF (ca. 50 µL/µmol) gelöst und die Lösung auf 0 °C gekühlt. 4-Methylmorpholin (6 Äquivalente) und HATU (3 Äquivalente) werden zugegeben und der Ansatz 2 h bei 0 °C gerührt. Die Aufarbeitung erfolgt direkt durch Aufgabe der gesamten Reaktionsmischung und Trennung nach Methode 45 oder einer der angeführten HPLC-Methoden. Produkthaltige Fraktionen werden vereinigt und lyophilisiert oder am Rotationsverdampfer eingeengt.

Arbeitsvorschrift 12 (Abspaltung der Fmoc-Gruppe mit Piperidin): Das Peptid wird bei 0 °C mit einer Mischung aus THF und Piperidin (4+1, ca. 20 µL/µmol) versetzt. Anschließend lässt man 1h bei dieser Temperatur rühren. Das Reaktionsgemisch wird direkt nach Methode 45 gereinigt, produkthaltige Fraktionen werden vereinigt und eingeengt.

Arbeitsvorschrift 13 (Peptidkupplung mit HATU in der flüssigen Phase): Ein cyclisches Peptid mit ungeschütztem *N*-Terminus (z.B. Beispielverbindung 407, 408, 409, u.s.w.) und ein Dipeptid (beispielsweise Verbindung 8A, 10A, u.s.w.) (1.5 Äquivalente) werden in DMF (ca. 30-80 µL/µmol Cyclopeptid) gelöst und die Lösung auf 0 °C gekühlt. 4-Methylmorpholin (4 Äquivalente) und HATU (1.6 Äquivalente) werden zugegeben und der Ansatz 2 h bei Raumtemperatur gerührt. Dann wird mit 3 mL Methanol abgestoppt und nach Methode 45 chromatographisch gereinigt. Produkthaltige Fraktionen werden vereinigt und eingeengt.

### Ausgangsverbindungen

### Beispiel 1A

D-Leucyl-L-leucyl-L-phenylalanyl-[(3*R*)-leucyl]-L-leucyl-D-arginyl-L-isoleucyl-allothreonyl-glycyl-[(3*S*)-3-hydroxy-asparaginyl]-serin-trifluoracetat (Dihydro-Lysobactin)
und

### Beispiel 2A

D-Leucyl-L-leucyl-[3-cyclohexyl-L-alanyl]-[(3*R*)-leucyl]-L-leucyl-D-arginyl-L-isoleucyl-allothreonyl-glycyl-[(3*S*)-3-hydroxy-asparaginyl]-serin-trifluoracetat (Octahydro-Lysobactin)

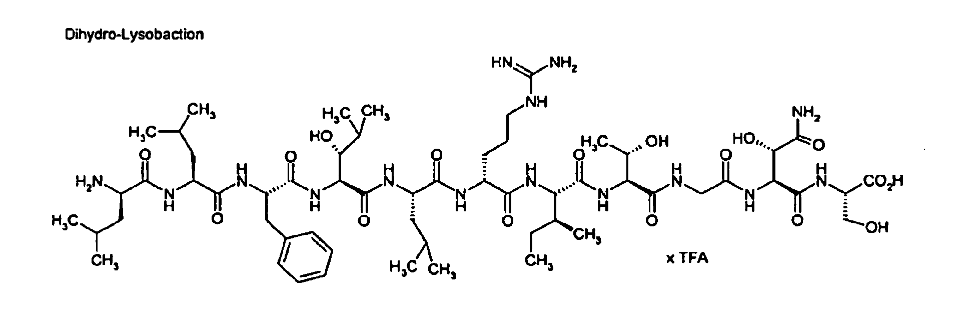

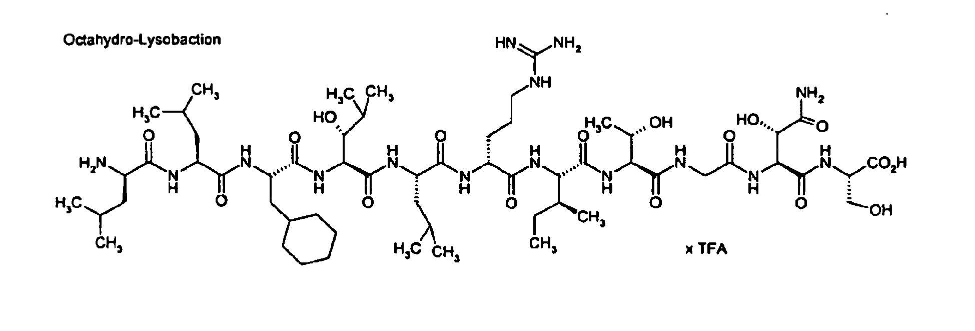

Die Darstellung von Lysobactin-bis-trifluoracetat durch Fermentation und seine Isolierung ist in WO 2004/099239 beschrieben. Lysobactin-bis-trifluoracetat (20 g, 13.29 mmol) wird in zwei Portionen Isopropanol/Wasser 9:2 (jeweils 110 ml) gelöst. Unter Argonschutzgasatmosphäre wird Palladium auf Kohle (10%ig; jeweils 5 g) zugegeben. Das Reaktionsgemisch wird (nach Entgasung) in einem Druckautoklaven bei 80-70 bar Wasserstoffdruck und 40°C für 12 h gerührt. Zur Reaktion wird erneut Palladium auf Kohle (10%ig; 5 g) gegeben. Das Reaktionsgemisch wird (nach Entgasung) erneut in einem Druckautoklaven bei 80-70 bar Wasserstoffdruck und 40°C für 12 h gerührt. Das Reaktionsgemisch wird (nach Entgasung) wieder einmal in einem Druckautoklaven bei 80-70 bar Wasserstoffdruck und 40°C für 12 h gerührt. Nun ist mittels analytischer HPLC (Methode 10) kein Lysobactin mehr detektierbar. Das Reaktionsgemisch wird über Kieselgur filtriert, im Vakuum eingeengt und im Hochvakuum getrocknet. Man erhält 20.10 g (99% d. Theorie) Produkt (60% Dihydro-lysobactin, 40% Octahydrolysobactin).

### Beispiel 3A

### [(3R)-Leucyl]-L-leucyl-D-arginyl-L-isoleucyl-allothreonyl-glycyl-[(3S)-3-hydroxyasparaginyl]-serin-triffuoracetat

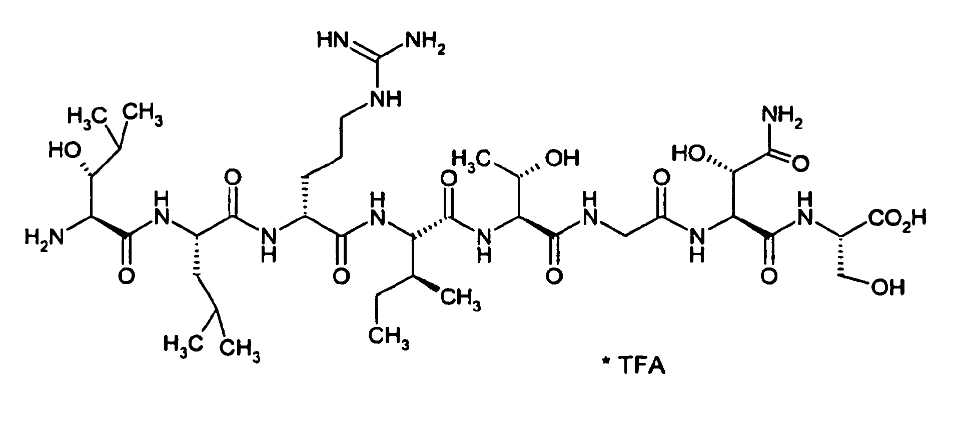

### Präparative Chymotrypsinspaltung vom Gemisch Dihydro- / Octahydro-Lysobactin Substratkonzentration 5 mg/ml

20 g Dihydro- (ca. 40%) und Octahydro-Lysobactin (ca. 60%) werden in 400 ml Methanol gelöst und dann mit 3400 ml Spaltpuffer (0.1 M Ammoniumhydrogencarbonat / 0.5 M Harnstoff pH 8) versetzt. Vor der Enzymzugabe wird die Lösung im Trockenschrank auf 37°C erwärmt. 800 mg Chymotrypsin (100 ml Chymotrypsinlösung Wasser / Ethylenglykol 1:1, 4 mg/ml; 1:25; 37°C vorgewärmt) werden hinzugegeben und die Reaktion bei 37°C durchgeführt. Es werden 200 µl Aliquots nach 0.5, 1 h entnommen und die Enzymspaltung mit 200 µl 30% Acetonitril / 0.1% TFA abgestoppt. Die Proben werden mit der HPLC parallel zur Enzymspaltung innerhalb von 15 min analysiert (Retentionszeit Fragment 4-11 ca. 3.6 min, Fragment 1-3 (LLF) ca. 9.6 min, Fragment 1-3 (LLA(3-cyclohexyl)) ca. 11.3 min) (Solvens A 0.1% TFA, Solvens B 60% Acetonitril/0.1% TFA, Gradient 0 min 30% B, 10 min 80% B, 11 min 100% B, 12 min 30% B, 15 min 30% B; Fluss: 0.7 ml/min, Temperatur: 40°C, UV-Detektion 210 nm). Die Enzymreaktion wird nach 60 min mit 150 ml Acetonitril und ca. 30 ml TFA abgestoppt. Der pH-Wert der Lösung soll zwischen 1 und 2 liegen. Bis zur präparativen Trennung kann die Lösung bei -20°C gelagert werden.

Die Aktivität der eingesetzten Chymotrypsincharge (70U/mg) wird durch eine Kontrollspaltung mit dem Protein Interleukin-4-Doppelmutein Arg(121) → Asp(121) /Tyr(124) - Asp(124) (Bayer Healthcare AG, D-Wuppertal) überprüft.

Präparative Reinigung: Jeweils 500 ml einer Lösung des oben beschriebenen Spaltansatzes werden zunächst mit Vakuum über eine Schicht Celite und dann über eine Druckfilternutsche (450 µm mit Vorfilter) filtriert. Das Filtrat wird auf eine Varian Metaflash C-18 40M Kartusche aufgegeben. Anschließend wird mit folgendem Programm eluiert: Lösungsmittel A: 0.05% TFA in Wasser, Lösungsmittel B: 0.05% TFA in Acetonitril. Flussrate: 50 ml/min; 0-3 min 5% B; Rampe; 30 min 90% B, 33 min. 90% B. Produkthaltige Fraktionen werden vereinigt und lyophilisiert. Aus den vereinigten produkthaltigen Fraktionen erhält man 11.8 g (87% d. Th.) der Titelverbindung.

### Beispiel 4A

### Methyt-(Z)-2-[(tert-butoxycarbonyl)amino]-4,4-dimethylpent-2-enoat

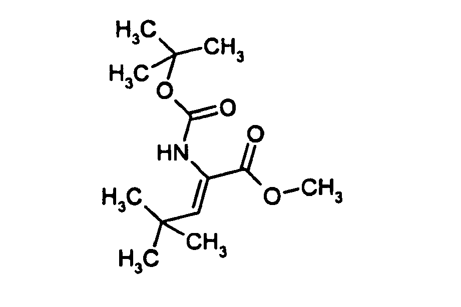

Pivalaldehyd (303.2 g, 3.41 mol, 10 Äq.) und Methyl-{[(*tert*-butoxy)carbonyl]amino}-(dimethoxyphosphoryl)acetat (101.5 g, 0.341 mol, 1.0 Äq.) werden in THF (800 ml) gelöst und auf -70°C gekühlt. Bei -70°C wird TMG (78.7 g, 0.683 mmol, 6.95 ml, 2.0 Äq.) langsam zugetropft und dann wird 4 h bei -70°C gerührt, anschließend wird 4 Tage bei RT gerührt. Das Reaktionsgemisch wird eingeengt, dann wird mit Essigsäureethylester (zweimal 500 ml) gegen Wasser ausgeschüttelt, die vereinigten org. Phasen mit ges. Natriumchloridlösung (100 ml) gewaschen und über Natriumsulfat getrocknet. Nach dem Einengen wird das Rohprodukt chromatographiert (1.5 kg Kieselgel, Eluent: Cyclohexan/Essigsäureethylester 5:1). Es werden 67 g (76% d. Th.) der Titelverbindung erhalten.

Alternativ kann das Rohprodukt nach der wässriger Aufarbeitung durch Kristallisation aus Cyclohexan/Essigsäureethylester gereinigt werden.
R*_{f}* (Kieselgel, Cyclohexan/Essigsäureethylester 4:1) = 0.5
LC-MS (Methode 18): Rₜ = 2.5 min; MS (ESIpos.): m/z (%) = 158 (100) [M - C₄H₈ - CO₂ + H]⁺, 280 (5) [M + Na]⁺.
¹H NMR (400 MHz, *d*₆-DMSO): δ 1.08 (s, 9H, C(CH₃)₃), 1.38 (s, 9H, OC(CH₃)₃), 3.61 (s, 3H, CO₂CH₃), 6.40 (s, 1 H, H^{β}), 8.14 (s, 1H, NH).
¹³C NMR (126 MHz, *d*₆-DMSO): δ 28.46 (3C), 29.53 (3C), 33.28, 52.22, 78.89, 125.62, 147.05, 154.67, 166.25.
HR-TOF-MS (Methode 24): C₁₃H₂₃NO₄ [M+H]⁺ gef. 258.1696, ber. 258.1700.

### Beispiel 5A

### N²-(tert-Butoxycarbonyl)-3-tert-butyl-D-alanin-methylester

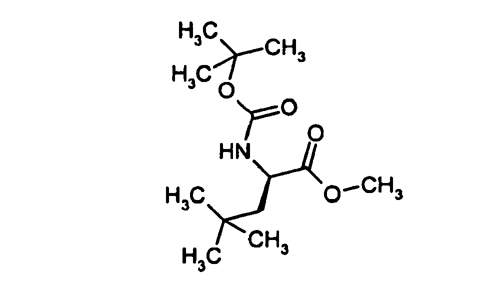

Methyl-(2Z)-2-[(*tert*-butoxycarbonyl)amino]-4,4-dimethylpent-2-enoat (Beispiel 4A, 60 g, 233.2 mmol) wird in Ethanol p.a./Dioxan 3:1 (1000 ml) gelöst. Mit einer Kanüle wird Argonschutzgasatmosphäre durchgeleitet (-10 min). Die Lösung wird ins Ultraschallbad gestellt (ca. 5 min), und es wird (+)-1,2-Bis-[(2*R*,5*R*)diethylphospholano]benzen(cyclooctadien)rhodium(I)-Triflat (600 mg, 1 Gew.-%) zugegeben. Es wird für 3 Tage bei 3.5 bar Wasserstoffdruck und RT hydriert. Das Reaktionsgemisch wird über Kieselgur filtriert und das Eluat eingeengt. Das Rohprodukt wird chromatographiert (Kieselgel, Eluent: Cyclohexan/Essigsäureethylester 4:1). Es werden 60 g (99% d. Th.) der Titelverbindung erhalten.
[α]²⁰_{Na} = +5° (*c* = 0.33 in CHCl₃).
DCI-MS (NH₃): *m*/*z* (%) = 221 (100), 260 (40) [M+H]⁺, 277 (100) [M + NH₄]⁺.
¹H NMR (400 MHz, CDCl₃): δ 0.93 (s, 9H, C(CH₃)₃), 1.40 (m, 10H, OC(CH₃)₃), 1.68 ("d", *J* = 14.5 Hz, 2H, H^{β}), 3.68 (s, 3H, OCH₃), 4.30 (t, *J* = 7.9 Hz, 1H, H^{α}), 4.81 (d, br, *J* = 7.7 Hz, 1H, NH).
¹³C NMR (126 MHz, CDCl₃): δ 28.30 (3C, CH₂C(CH₃)₃), 29.52 (3C, OC(CH₃)₃), 30.61 (CH₂C(CH₃)₃), 46.27 (C^{β}H₂), 51.19 (H^{α}), 52.17 (OCH₃), 79.79 (OC(CH₃)₃), 155.11 (NHCO₂), 174.39 (CO₂CH₃).
HR-TOF-MS (Methode 24): C₂₆H₅₁N₂O₈ ber. 519.3640, gef. 519.3634 [M+H]⁺.

### Beispiel 6A

### N²-(tert-Butoxycarbonyl)-3-tert-butyl-D-alanin

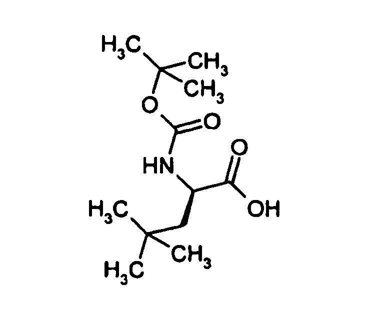

*N*-*tert-*Butoxycarbonyl-3-*tert*-butyl-D-alanin-methylester (Beispiel 5A, 60 g, 231 mmol) wird in THF p.a. (463 ml) gelöst. Bei RT wird langsam eine Lösung von Lithiumhydroxid-monohydrat (19.4 g, 462.7 mmol) in Wasser (463 ml) zugetropft. Wenn das HPLC-Chromatogramm (Methode 1) vollständigen Umsatz zeigt (ca. 20 h) wird das Reaktionsgemisch unter Eiskühlung vorsichtig mit 1 N wässr. Salzsäure auf pH 3-4 eingestellt. Dem Reaktionsgemisch wird festes Natriumchlorid (150 g) zugefügt, um es anschließend zweimal mit Essigsäureethylester (500 ml) zu extrahieren. Die vereinigten org. Phasen werden mit ges. Natriumchloridlösung gewaschen, dann mit Natriumsulfat getrocknet und filtriert. Das Filtrat wird am Rotationsverdampfer eingeengt und am Hochvakuum getrocknet. Man erhält 55.4 g (98% d. Th.) der Titelverbindung.
HPLC/UV-Vis (Methode 3): Rₜ = 4.2 min.
DCI-MS (NH₃): *m*/*z* (%) = 263 (100) [M + NH₄]⁺, 280 (5) [M + N₂H₇]⁺.
¹H NMR (400 MHz, CDCl₃): δ = 0.96 (s, 9H, C(CH₃)₃), 1.42 (m, 10H, OC(CH₃)₃, H^{β}), 1.79 ("d", *J* = 14.4 Hz, 2H, H^{β}), 4.31 (t, *J* = 8.0 Hz, 1H, H^{α}), 4.82 (d, *J* = 8.4 Hz, 1H, NH).
¹³C NMR (126 MHz, EDCl₃): δ = 28.32 (3C), 29.54 (3C), 30.74, 45.92, 51.24, 80.19, 155.41, 178.93.
HR-TOF-MS (Methode 24): C₂₄H₄₇N₂O₈ ber. 491.3327, gef. 491.3328 [2M+H]⁺.

### Beispiel 7A

### [N²-(tert-Butoxycarbonyl)-(3-tert-butyl-D-alanyl)]-(3-tert-butyl-L-alanin)-methylester

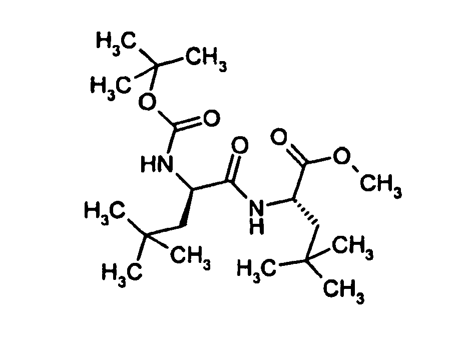

Zu einer Lösung von 3-*tert*-Butyl-L-alanin-methylester-hydrochlorid (1.1 Äq., 21 g, 107 mmol) in Dichlormethan p.a. (1.4 1) werden bei -20°C nacheinander HOBt (3 Äq., 39.4 g, 292 mmol), NMM (3 Äq., 32.1 ml, 291.8 mmol), *N*-*tert*-Butoxycarbonyl-3-*tert*-butyl-D-alanin (Beispiel 6A, 1.0 Äq., 97.3 mmol), EDC (2 Äq., 37.3 g, 194.6 mmol) und erneut NMM (2 Äq., 21.4 ml, 194.5 mmol) gegeben. Das Reaktionsgemisch erwärmt sich langsam (ca. 12 h) auf RT, wobei vollständiger Umsatz der Amin-Komponente mittels HPLC beobachtet wird. Das Reaktionsgemisch wird anschließend mit ges. wässr. Natriumhydrogencarbonatlösung (300 ml), mit 5%iger wässr. Zitronensäure (500 ml), ges. wässr. Natriumhydrogencarbonatlösung (500 ml) und ges. Natriumchloridlösung gewaschen. Man trocknet über Natriumsulfat und filtriert. Im Vakuum wird bis zur Trockne eingedampft und anschließend im Hochvakuum nachgetrocknet. Man erhält 36 g (96% d. Th.) der Titelverbindung, die ohne weitere Reinigung umgesetzt wird.
[α]²⁰_{Na} = +24° (c = 0.10 in CH₂Cl₂).
DCI-MS (NH₃): *m*/*z* (%) = 387 (40) [M+H]⁺, 404 (100) [M + NH₄]⁺.
LC-MS (Methode 20): Rₜ = 2.6 min; MS (ESIpos.): *m*/*z* (%) = 287 (100) [M - C₅O₂H₈ + H]⁺, 387 (60) [M+H]⁺.
¹H NMR (400 MHz, *d*₆-DMSO): δ 0.95 (s, br, 18H, tBu), 1.35 (dd, 1H), 1.45 (s, 9H, Ot-Bu), 1.50 (dd, 1H), 1.65 (dd, 1H), 1.95 (dd, br, 1H), 3.70 (s, 3H, OCH₃), 4.15 (" t", br, 1H), 4.55 ("t", br, 1H), 4.80 (d, 1H), 6.65 (d, br, 1H).
¹³C NMR (126 MHz, *d*₆-DMSO): δ 28.39 (3C, C(CH₃)₃), 29.49 (3C, C(CH₃)₃), 29.67 (3C, C(CH₃)₃), 30.40 (C(CH₃)₃), 30.44 (C(CH₃)₃), 44.25, 45.12, 49.56, 52.06, 52.21, 78.17, 154.93, 173.00, 173.35.
HR-TOF-MS (Methode 24): C₂₀H₃₉N₂O₅ [M+H]⁺ gef. 387.2860, ber. 387.2859.

### Beispiel 8A

### [N²-(tert-Butoxycarbonyl)-3-tert-butyl-D-alanyl]-3-tert-butyl-L-alanin

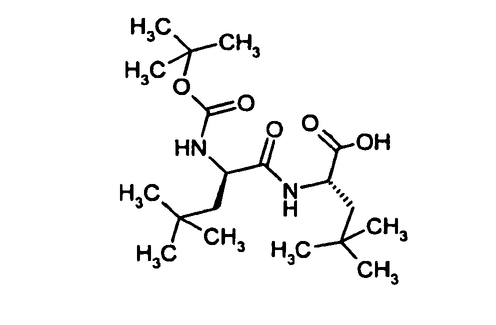

Die Verbindung aus Beispiel 7A (36 g, 93.1 mmol) wird in THF p.a. (279 ml) gelöst. Bei ca. 10°C wird langsam eine Lösung von Lithiumhydroxid-monohydrat (7.82 g, 186.3 mmol, 2 Äq.) in Wasser (187 ml) zugetropft. Wenn das HPLC-Chromatogramm (Methode 1) vollständigen Umsatz zeigt (ca. 20 h) wird das Reaktionsgemisch bei 200 mbar vom THF befreit und dann mit MTBE (200 ml) extrahiert. Die org. Phase wird mit Essigsäureethylester (500 ml) verdünnt, dann mit Wasser versetzt und anschließend vorsichtig mit 1 N wässr. Salzsäure auf pH 3-4 eingestellt. Die vereinigten org. Phasen werde mit ges. Kochsalzlösung gewaschen, über Natriumsulfat getrocknet, filtriert, im Vakuum eingedampft und im Hochvakuum getrocknet. Man erhält 97.4 g (97% d. Th.) der Titelverbindung.
LC-MS (Methode 18): Rₜ = 2.26 min; MS (ESIpos.): m/z (%) = 373 (100) [M+H]⁺.
¹H NMR (400 MHz, d₆-DMSO): δ 0.83 (s, br, 18H, tBu), 1.31 (s, 9H, OtBu), 1.40 (m, 2H, β-CH₂), 1.48 (dd, *J* = 14.1, 9.4 Hz, 1H, β-CH), 1.59 (dd, *J*= 14.1, 2.7 Hz, 1H, α-CH'), 3.98 (m, 1H, α-CH), 4.12 (m, 1H, α-CH), 6.73 (d, *J*= 9.1 Hz, 1H, NH), 7.72 (d, *J* = 7.9 Hz, 1H, NH), 12.42 (s, br, 1H, CO₂H).
¹³C NMR (126 MHz, *d*₆-DMSO): δ 28.49 (3C), 29.66 (3C), 29.78 (3C), 30.52, 30.58, 44.63 (β-CH₂), 45.24 (β-CH₂), 49.67 (α-CH), 52.40 (α-CH), 78.29, 155.05, 172.97, 174.61.
HR-TOF-MS (Methode 24): C₁₉H₃₇N₂O₅ ber. 373.2702, gef. 373.2717 [M+H]⁺.

### Beispiel 9A

### (3-tert-Butyl-D-alanyl)-3-tert-butyl-L-alanin-hydrochlorid

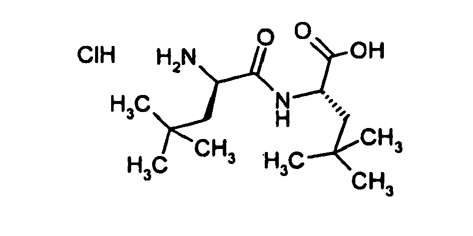

Die Verbindung aus Beispiel 8A (4.5 g, 12.1 mmol) wird in Dioxan (3 ml) vorgelöst. Bei RT wir eine 4 N Lösung von Salzsäure in Dioxan (30.2 mmol, 120 mmol, 10 Äq.) zugetropft. Das Reaktionsgemisch wird für 30 min gerührt, im Vakuum eingedampft und im Hochvakuum getrocknet. Man erhält die Titelverbindung als farblosen Feststoff (3.5 g, 99% d. Th.).
LC-MS (Methode 18): Rₜ = 1.52 min; MS (ESIpos.): *m*/*z* (%) = 273.6 (100) [M+H]⁺; ESIneg: *m*/*z* = 271.5 (100) [M - H]⁻.
¹H NMR (500 MHz, d₆-DMSO): δ 0.85 (s, 9H, tBu), 0.86 (s, 9H, tBu), 1.49 (dd, *J*= 14.3, 1.6 Hz, 1H, β-CH), 1.50 (d, *J* = 13.8 Hz, 1H, β-CH), 1.64 (dd, *J* = 14.3, 4.0 Hz, 1H, β-CH), 1.71 (dd, *J* = 14.3, 6.9 Hz, 1H, β-CH), 3.77 ("t", *J* = 6.5 Hz, 1H, α-CH), 4.14 (m, 1H, α-CH), 8.27 (s, br, 3H), 8.94 (d, *J* = 8.2 Hz, 1H, NH), 12.58 (s, br, 1H, CO₂H).
¹³C NMR (126 MHz, d₆-DMSO): δ 28.41 (3C), 29.50 (3C), 30.08, 30.38, 44.46, 44.80, 50.03, 50.30, 169.10, 173.98.
HR-TOF-MS (Methode 24): C₁₄H₂₉N₂O₃ ber. 273.2173, gef. 273.2167 [M+H]⁺.

### Beispiel 10A

### [N²-(Benzyloxycarbonyl)-3-tert-butyl-D-alanyl]-3-tert-butyl-L-alanin

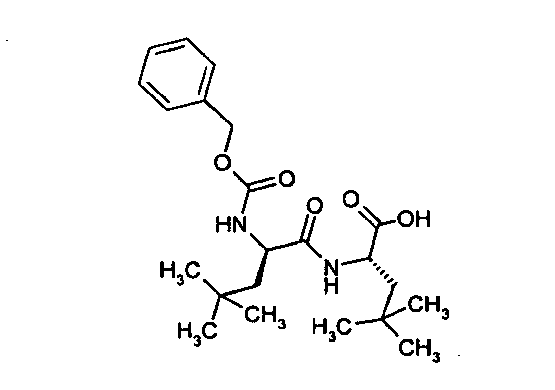

Das Dipeptid (Beispiel 9A, 3.73 g, 12.1 mmol) wird unter Argonschutzgasatmosphäre in THF (170 ml) gelöst. Nach Zugabe von Wasser (170 ml), Benzyloxycarbonyloxysuccinimidester (4.52 g, 18.1 mmol, 1.5 Äq.) und NMM (4.28 g, 4.23 mmol, 3.5 Äq.) bei 0°C wird bei RT kräftig gerührt, bis alles Edukt umgesetzt ist (mehrere Stunden, HPLC Kontrolle, Methode 1). Der Ansatz wird mit Eisessig gequecht. Im Vakuum wird das THF abgezogen. Die zurückbleibende wässr. Phase wird mit Essigsäureethylester überschichtet mit 4 N Salzsäure auf pH < 3 angesäuert und dann mehrfach mit Essigsäureethylester extrahiert. Die org. Phasen werden mit ges. Natriumchloridlösung gewaschen über Natriumsulfat getrocknet, filtriert und eingeengt. Der erhaltene Schaum wird mit Acetonitril verrührt, wobei sich ein farbloser Feststoff bildet, der abfiltriert wird und dann mit wenig Acetonitril gewaschen wird. Dieser Prozeß kann mit dem Filtrat, nachdem es eingeengt wurde, noch mehrfach wiederholt werden. Die vereinigten Feststoffe werden am Hochvakuum getrocknet, wobei man die Titelverbindung als Feststoff erhält. Die zurückgebliebene Mutterlauge wird eingeengt und mittels präparativer HPLC (Methode 26) aufgereinigt. Man erhält die Titelverbindung (vereinigte Feststoffe und Reinigungsprodukt aus der HPLC Trennung) als farblosen Feststoff (3.49 g, 71% d. Th.).
HPLC/UV-Vis (Methode 1): Rₜ = 2.6 min.
LC-MS (Methode 21): Rₜ = 2.46 min; MS (ESIpos.): m/z (%) = 363 (60), 407 (100) [M+H]⁺. ESIneg: *m*/*z* = 297 (100), 405.5 (40) [M - H]⁻.
¹H NMR (500 MHz, *d*₆-DMSO): δ 0.81 (s, 9H, tBu), 0.83 (s, 9H, tBu), 1.40-1.44 (m, 2H, β-CH₂), 1.49 (dd, *J* = 14.3, 9.7 Hz, 1H, β-CH), 1.58 (dd, *J* = 13.5, 1.4 Hz, 1H, β-CH), 4.07 (m, 1H, α-CH), 4.13 (m, 1H, α-CH), 4.94 (d, *J* = 12.3 Hz, 1H, *CH*HPh), 4.99 (d, *J* = 12.5 Hz, 1H, *CH*HPh), 7.25-7.32 (m, 5H, ArH), 7.92 (d, *J* = 8.5 Hz, 1H, NH).
¹³C NMR (126 MHz, *d*₆-DMSO): δ 29.49 (3C), 29.75 (3C), 30.41, 30.46, 44.52, 45.11, 49.55, 52.73, 65.49, 127.70 (2C), 127.91, 128.48 (2C), 137.31, 155.59, 172.52, 174.50.
HR-TOF-MS (Methode 24): C₂₂H₃₄N₂O₅ ber. 407.2541, gef. 407.2531 [M+H]⁺.

### Beispiel 11A

### Methyl-(2R*,3R*)-N²-[(benzyloxy)carbonyl]-N²-[(benzyloxy)carbonylamino]-3-[(tert-butoxycarbonyl)amino]-phenylalaninat

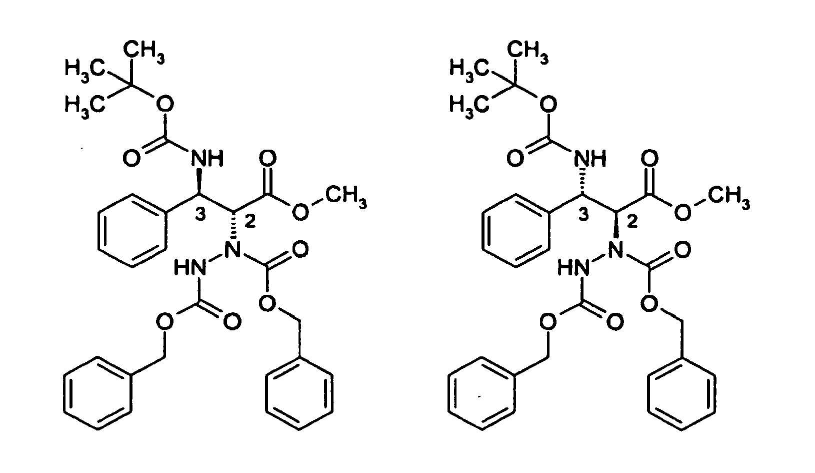

Unter Argonschutzgasatmosphäre wird eine 1 N Lösung von Lithiumhexamethyldisilazid (157.5 mmol, 157.5 ml, 2.2 Äq.) in THF in das Reaktionslösungsmittel THF (300 ml) vorgelegt. Bei -78°C wird eine Lösung von (*rac*)-3-[(*tert*-Butoxycarbonyl)-amino]-3-phenylpropansäuremethylester (A. V. Rao Rama, A. K. Singh, Ch. V. N. S. Varaprasad, Tetrahedron Lett., 1991, 32, 4393-4396) (20 g, 71.2 mmol) langsam zugetropft. Man rührt 10 min bei -25°C und kühlt dann erneut auf -78°C herunter. Dibenzylazadicarboxylat (34.2 g, 114.6 mmol, 1.6 Äq.) wird in einer Portion zum Reaktionsgemisch gegeben. Es wird 3 h bei -60 bis -45°C gerührt. Um die Reaktion zu stoppen, wird erneut auf -78°C heruntergekühlt und mit Essigsäure (20.5 ml, 358 mmol, 5 Äq.) versetzt, und dann auf 0°C und schließlich RT erwärmt. Das Reaktionsgemisch wird im Vakuum eingedampft und in Essigsäureethylester (1000 ml) aufgenommen. Die Suspension wird zweimal mit ges. wässr. Natriumhydrogencarbonatlösung, einmal mit Wasser, zweimal mit 5%iger wässr. Zitronensäure und einmal mit ges. wässr. Natriumchloridlösung gewaschen. Alle wässr. Phasen werden einzeln mit Essigsäureethylester reextrahiert. Alle org. Phasen werden im Vakuum eingedampft und erneut in Dichlormethan (2000 ml) aufgenommen, filtriert, über Natriumsulfat getrocknet, erneut filtriert, im Vakuum eingedampft und im Hochvakuum getrocknet. Man erhält 7.2 g (18% d. Th.) der Titelverbindung als Feststoff. Das Filtrat der Dichlormethanphase (*vide supra*) wird eingeengt und dann wird erneut aus Methanol umkristallisiert, wobei man 13.2 g (26% d. Th.) der Titelverbindung erhält.
LC-MS (Methode 23): Rₜ = 6.8 min;
MS (ESIpos.): *m*/*z* (%) = 578 (40) [M+H]⁺, 1156 (100) [2M+H]⁺.
MS (ESIneg.): *m*/*z* (%) = 576 (100) [M - H]⁻.

### Beispiel 12A

### Methyl-(2S*,3R*)-N²-[(benzyloxy)carbonyl]-N²-[(benzyloxy)carbonylamino]-3-[(tert-butoxycarbonyl)amino]-phenylalaninat

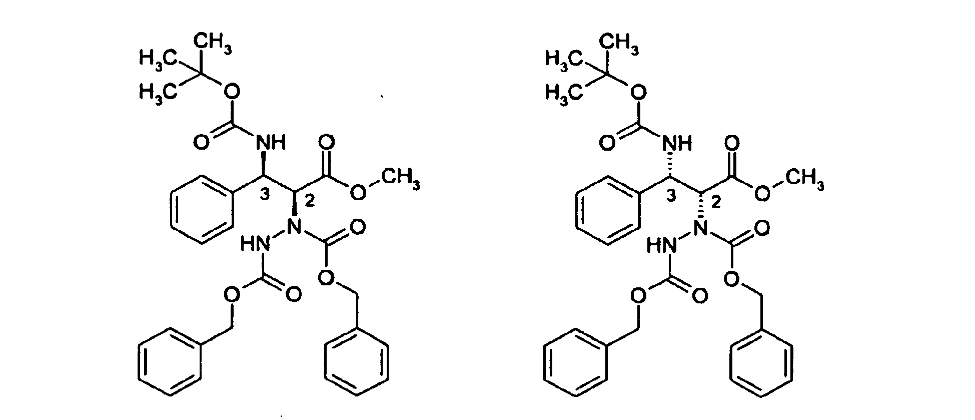

Unter Argonschutzgasatmosphäre wird eine Lösung von Methyl-(2*R**,3*R**)-*N*²-[(benzyloxy)carbonyl]-*N*²-[(benzyloxy)carbonylamino]-3-[(*tert*-butoxycarbonyl)-amino]-phenylalaninat (Beispiel 11A, 20.5 g, 35.5 mmol) in trockenem DMF p.a. (750 ml) bei 0°C mit TMG (50 ml, 399 mmol) versetzt. Man lässt das Reaktionsgemisch auftauen und rührt bis das HPLC-Chromatogramm (Methode 1) vollständigen Umsatz (ca. 60% Produkt) anzeigt (ca. 12 h), um die Reaktion dann durch Zugabe von Essigsäure (pH 4-6) zu stoppen. Das Reaktionsgemisch wird im Vakuum bei RT eingedampft und in Essigsäureethylester aufgenommen. Die org. Phase wird zweimal mit Wasser, zweimal mit 5%iger Zitronensäure, einmal mit Wasser, zweimal mit ges. wässr. Natriumhydrogencarbonatlösung, einmal mit ges. wässr. Natriumchloridlösung gewaschen, über Natriumsulfat getrocknet, filtriert, im Vakuum eingedampft, im Hochvakuum getrocknet. Das Rohprodukt wird mittels präparativer HPLC (Methode 30) oder Flashchromatographie (Kieselgel, Cyclohexan/Essigsäureethylester 3:1) gereinigt. Man erhält 7.7 g (37% d. Th.) der Titelverbindung und 5 g der Ausgangsverbindung (25% d. Th.).
HPLC/UV-Vis (Methode 1): Rₜ = 3.0 min.
LC-MS (Methode 10): Rₜ = 3.0 min;
MS (ESIpos.): *m*/*z* (%) = 478 (100) [M -Boc + H]⁺, 578 (30) [M+H]⁺.
LC-MS (Methode 23): Rₜ = 7.0 min;
MS (ESIpos.): *m*/*z* (%) = 578 (40) [M+H]⁺, 1156 (100) [2M+H]⁺.
MS (ESIneg.): *m*/*z* (%) = 576 (100) [M - H]⁻.

### Beispiel 13A

### Methyl-(2S*,3R*)-3-[(tert-butoxycarbonyl)amino]phenylalaninat

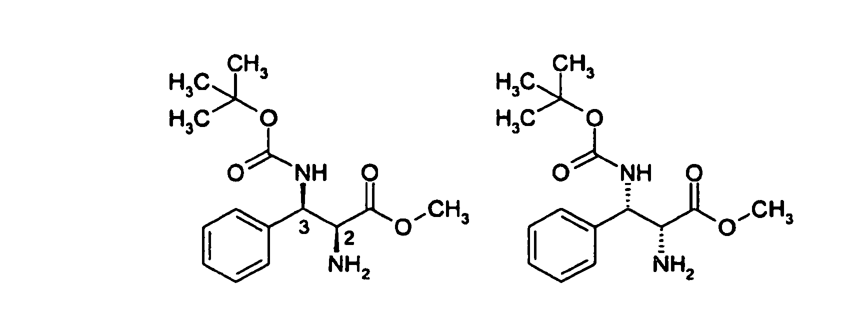

Unter Argonschutzgasatmosphäre wird zu einer Lösung von Methyl-(2*S**,3*R**)-*N*²-[(benzyloxy)carbonyl]-*N*²-[(benzyloxy)carbonylamino]-3-[(*tert*-butoxycarbonyl)-amino]phenylalaninat (Beispiel 12A, 705 mg, 1.22 mmol) in Methanol/Dichlormethan 1:1 (42 ml) Raney-Nickel (61 mg, ca. 10 mol%) gegeben. Das Reaktionsgemisch wird in einem Druck-Autoklaven bei 80 bar Wasserstoffdruck und RT hydriert (40 h). Das HPLC-Chromatogramm zeigt vollständigen Umsatz. Das Reaktionsgemisch wird unter Argonschutzgasatmosphäre über einer Glasfritte filtriert, die Glasfritte wird mehrfach mit Methanol/Wasser/0.2% Essigsäure gewaschen. Das Filtrat wird im Vakuum eingedampft und am Hochvakuum getrocknet. Man erhält einen Feststoff (ca. 3 g) der dann im Ultraschallbad in Essigsäureethylester suspendiert wird. Die Suspension wird mit einer Lösung von EDTA (400 mg) in 7%iger wässr. Natriumhydrogencarbonatlösung (400 ml) versetzt. Die wässr. Phase wird mit Essigsäureethylester (100 ml, dreimal) extrahiert. Die vereinigten org. Phasen werden dann mit einmal ges. wässr. Natriumhydrogencarbonatlösung, und zweimal mit ges. wässr. Natriumchloridlösung gewaschen. Alle wässr. Phasen werden einzeln mit Essigsäureethylester reextrahiert. Die vereinigten org. Phasen werden dann über Natriumsulfat getrocknet, filtriert und am Hochvakuum getrocknet. Als Produkt erhält man einen Feststoff (1.26 g, quantitativ) der ohne Feinreinigung weiter umgesetzt wird.
HPLC/UV-Vis (Methode 2): Rₜ = 1.7 min.
LC-MS (Methode 23): Rₜ = 4.1 min;
MS (ESIpos.): *m*/*z* (%) = 239 (100), 295 (80) [M+H]⁺.

### Beispiel 14A

### Methyl-(2S*,3R*)-N²-[(benzyloxy)carbonyl]-3-[(tert-butoxycarbonyl)amino]phenylalaninat

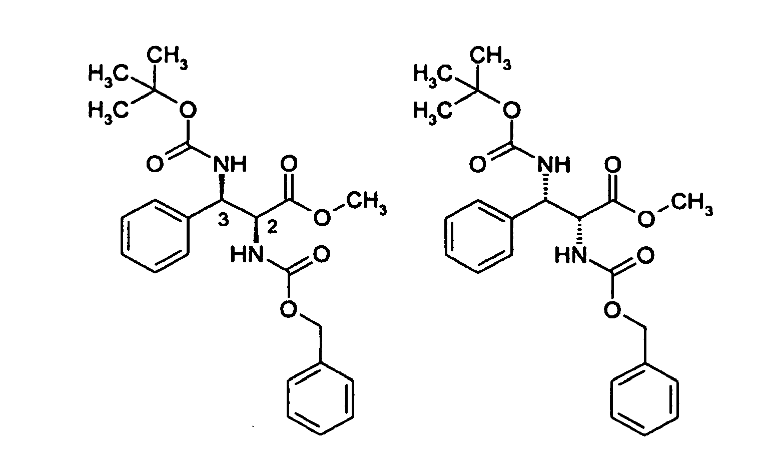

Unter Argonschutzgasatmosphäre wird zu einer Lösung von Methyl-(2S*,3R*)-3-[(*tert-*butoxycarbonyl)amino]phenylalaninat (Beispiel 13A, 360 mg, 1.2 mmol) und *N*-Benzyloxycarbonyloxysuccinimidester (610 mg, 2.44 mmol, 2 Äq.) in THF (25 ml) bei 0°C NMM (260 mg, 2.6 mmol, 2.1 Äq.) zugegeben. Das Reaktionsgemisch wärmt sich langsam auf (12 h), wobei vollständiger Umsatz mittels HPLC (Methode 2) beobachtet wird. Anschließend wird Essigsäure (0.7 ml) zugegeben, im Vakuum eingeengt und mittels präparativer HPLC (Methode 31) gereinigt. Man erhält 396 mg (76% d. Th.) der Titelverbindung.
HPLC/UV-Vis (Methode 2): Rₜ = 2.7 min.
LC-MS (Methode 23): Rₜ = 6.4 min;
MS (ESIpos.): *m*/*z* (%) = 329 (100) [M - C₄H₈ - CO₂ + H]⁺, 429 (80) [M+H]⁺, 858 (60) [2M+H]⁺.

### Beispiel 15A

### (2S*,3R*)-N²-[(Benzyloxy)carbonyl]-3-[(tert-butoxycarbonyl)amino]-phenylalanin

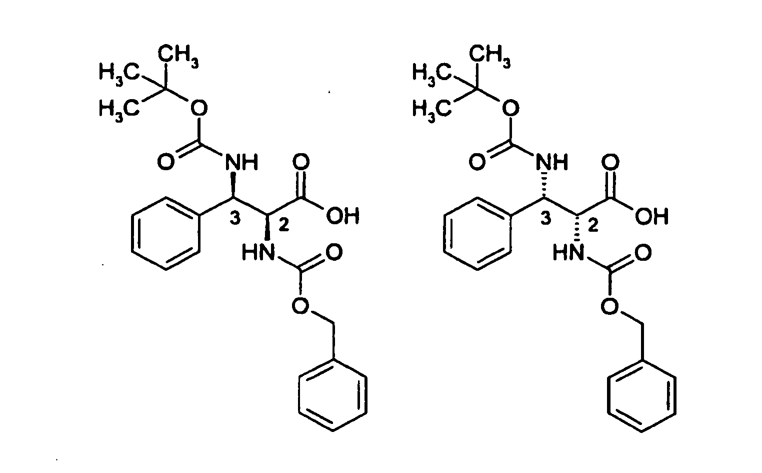

Unter Argonschutzgasatmosphäre wird eine Lösung von Methyl-(2*S**,3*R**)-*N-*[(benzyloxy)carbonyl]-3-[(*tert*-butoxycarbonyl)amino]phenylalaninat (Beispiel 15A, 755 mg, 1.76 mmol) in THF/Wasser 2:1 (30 ml) vorgelegt. Bei 0°C wird unter starkem Rühren eine entgasten 1%ige wässr. Lösung von Lithiumhydroxid-Monohydrat (86.5 mg, 3.6 mmol, 2 Äq.) langsam zugetropft. Man rührt bei RT so lange nach, bis das HPLC-Chromatogramm (Methode 1) vollständigen Umsatz anzeigt (ca. 1 h). Anschließend versetzt man mit Essigsäure (0.5 ml), konzentriert das Reaktionsgemisch im Vakuum auf und überschichtet mit Essigsäureethylester (100 ml). Die wässr. Phase wird nun mit 5%iger Zitronensäure angesäuert (pH 2-3) und dann dreimal mit Essigsäureethylester (50 ml) extrahiert. Die vereinigten org. Phasen werden zweimal mit ges. wässr. Natriumchloridlösung (20 ml) gewaschen, über Natriumsulfat getrocknet, filtriert, im Vakuum eingeengt und im Hochvakuum getrocknet. Man erhält 750 mg (quantitativ) Rohprodukt der Titelverbindung, welches mittels präparativer HPLC feingereinigt wird (Methode 31).
HPLC/UV-Vis (Methode 1): Rₜ = 2.4 min.
LC-MS (Methode 23): Rₜ = 6.1 min;
MS (ESIpos.): *m*/*z* (%) = 359 (100), 415 (60) [M+H]⁺, 829 (60) [2M+H]⁺.
MS (ESIneg.): *m*/*z* (%) = 413 (100) [M - H]⁻.

### Beispiel 16A

### (3R)-N²-[(Benzyloxy)carbonyl]-3-[(tert-butoxycarbonyl)amino]-L-phenylalanin

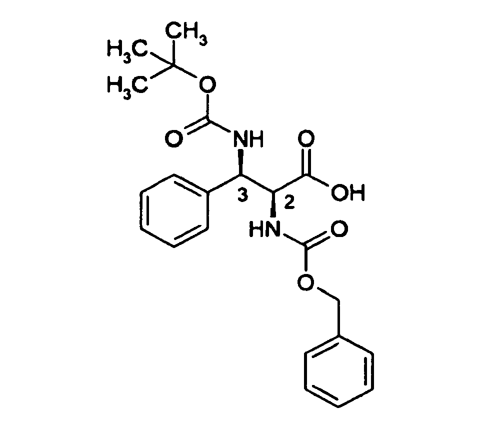

Das Enantiomerengemisch von (2*S**,3*R**)-*N*-[(Benzyloxy)carbonyl]-3-[(*tert*-butoxycarbonyl)amino]phenylalanin (Beispiel 15A, 750 mg, 1.8 mmol) wird mittels präparativer HPLC (Methode 38) getrennt. Man erhält 334 mg (98% ee, 45% d. Th.) (2*S*,3*R*)-*N*-[(Benzyloxy)carbonyl]-3-[(*tert*-butoxycarbonyl)amino]phenylalanin (Titelverbindung) und 275 mg (98% ee, 37% d. Th.) (2*R*,3*S*)-*N*-[(Benzyloxy)carbonyl]-3-[(*tert-*butoxycarbonyl)amino]phenylalanin (weiteres Enantiomer).
Enantiomerenbestimmung nach Methode 14.
[α]²⁰_{Na} = +22° (*c* = 0.50 in Chloroform) (Titelverbindung).
[α]²⁰_{Na} = -20° (*c* = 0.49 in Chloroform) (weiteres Enantiomer).

### Beispiel 17A

### Pentafluorphenyl (3R)-N²-[(benzyloxy)carbonyl]-3-[(tert-butoxycarbonyl)amino]-L-phenylalaninat

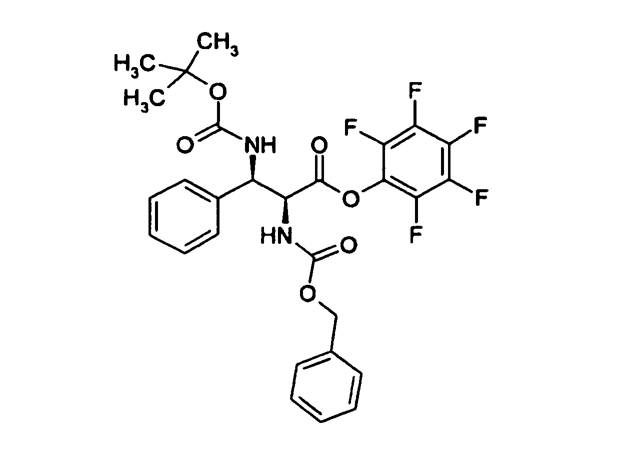

Die Verbindung aus Beispiel 16A (2.0 g, 4.83 mmol) wird in Dichlormethan (30 ml) vorgelegt und mit Pentafluorphenol (4.4 g, 24.13 mmol, 5 Äq.) und EDC (1.4 g, 7.24 mmol, 1.5 Äq.) bei 0°C versetzt. Die Reaktion wird langsam auf RT gebracht und bei dieser Temperatur über Nacht gerührt. Zur Aufarbeitung wird das Lösungsmittel am Rotationsverdampfer bei maximal 30°C Badtemperatur vollständig entfernt und nach der Feinreinigung des Rückstandes über die präparative HPLC (Methode 28) werden 2.3 g (83.3% d. Th.) Produkt isoliert.

HPLC (Methode 1) Rₜ = 3.44 min.
LC-MS (Methode 18): Rₜ = 3.23 min; MS (ESIpos): m/z (%) = 581 (13) [M+H]⁺, 525 (100) [M - C₄H₈ + H]⁺.
¹H NMR (500 MHz, *d₆*-DMSO): δ = 1.36 (s, 9H, 3 CH₃), 4.97 (d, *J* = 7.1 Hz, 2H, PhCH₂O), 5.15 (dd, *J* = 9.7, 4.8 Hz, 1H), 5.49 (dd, *J* = 10.2, 4.6 Hz, 1H), 7.18 (d, *J* = 10.4 Hz, 1H, ArH), 7.25-7.42 (m, 8H, ArH), 7.68 (d, *J* = 10.4 Hz, 1H, NH), 7.90 (d, *J* = 9.8 Hz, 1H, NH).
¹³C NMR (126 MHz, *d₆*-DMSO): δ = 27.97, 54.33, 58.72, 65.75, 78.65, 124.10, 126.48, 127.37 127.46, 127.82, 128.23 (2C), 136.40/138.36 (C-F), 136.54, 138.49/140.27 (C-F), 139.34/141.40 (C-F), 154.72, 156.04, 167.10.
HR-TOF-MS (Methode 24): C₂₈H₂₆N₂O₆ [M+H]⁺ gef. 581.1719, ber. 581.1706.

### Beispiel 18A

### 2,5-Dioxopyrrolidin-1-yl (3R)-N²-[(benzyloxy)carbonyl]-3-[(tert-butoxycarbonyl)-amino]-L-phenylalaninat

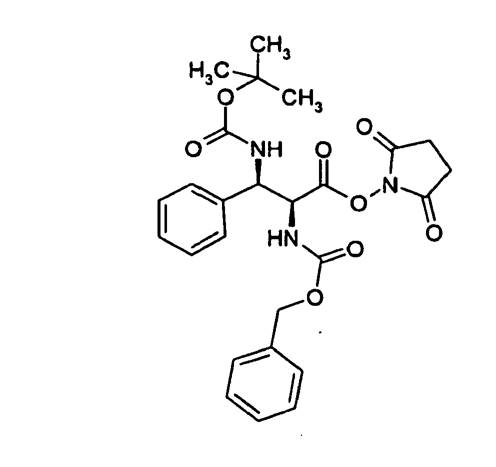

Unter Argonschutzgasatmosphäre Atmosphäre wird (3*R*)-*N*-[(Benzyloxy)carbonyl]-3-[(*tert*-butoxycarbonyl)amino]-L-phenylalanin (Beispiel 16A, 100.0 mg, 241.28 µmol) in Methylenchlorid (10 ml) vorgelegt, die Lösung auf 0°C gekühlt, mit *N-*Hydroxysuccinimid (33.3 mg, 289.53 µmol, 1.2 Äq.) und EDC (55.5 mg, 289.53 µmol, 1.2 Äq.) versetzt und über Nacht bei 0°C nachgerührt. Zur Aufarbeitung wird das Lösungsmittel vollständig am Rotationsverdampfer bei 30°C Badtemperatur entfernt und nach der Feinreinigung über die präparative HPLC (Methode 32) 96.0 mg (77.8% d. Th.) Produkt isoliert.
HPLC (Methode 1) Rₜ = 2.53 min.
LC-MS (Methode 18): Rₜ = 2.58 min; MS (ESIpos): *m*/*z* (%) = 529 (28) [M + NH₄]⁺, 412 (100).

### Beispiel 19A

### Methyl [N²-[(benzyloxy)carbonyl]-3-tert-butyl-D-alanyl]-[3-tert-butyl-L-alanyl]-[(3R)-3-[(tert-butoxycarbonyl)amino]-L-phenylalaninat]

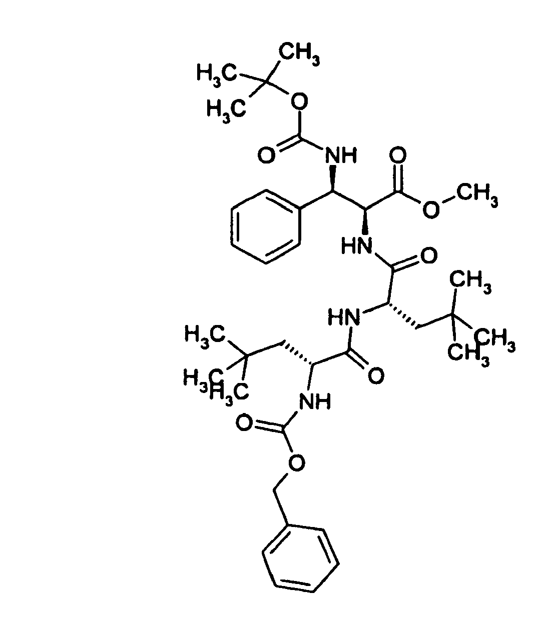

Unter Argonschutzgasatmosphäre wird N-Benzyloxycarbonyl-geschütztes Dipeptid (Beispiel 10A, 58.4 mg, 0.14 mmol) in Methylenchlorid (5 ml) vorgelegt, anschließend die Lösung auf -10°C abgekühlt, nacheinander mit HOBt (77.7 mg, 0.57 mmol, 4 Äq.), mit NMM (47 µl, 43.7 mg, 0.43 mmol, 3 Äq.) und mit EDC (55.1 mg, 0.29 mmol, 2 Äq.) versetzt und abschließend die Beispielverbindung 13A (94.0 mg, 0.14 mmol, 1 Äq.), und NMM 32 µl, 29.1 mg, 0.29 mmol, 2 Äq.) zugegeben. Die Reaktion wird langsam auf RT erwärmt und bei dieser über Nacht nachgerührt. Zur Aufarbeitung wird der Niederschlag abfiltriert, das Filtrat am Rotationsverdampfer bei 30°C Badtemperatur eingeengt und nach der Feinreinigung des Rückstandes über die präparative HPLC (Methode 31) wird das Produkt mit 77.9 mg (79.4% d. Th.) erhalten.
HPLC (Methode 1) Rₜ = 3.12 min.
LC-MS (Methode 23): Rₜ = 7.05 min; MS (ESIpos): *m*/*z* (%) = 683 (100) [M+H]⁺, 1366 (75) [2M + 2H]²⁺.
¹H NMR (300 MHz, CDCl₃): δ = 0.89 (d, *J* = 4.6, 9H, CH₂C(CH₃)₃), 0.96 (d, *J* = 2.9, 9H, CH₂C(CH₃)₃), 1.23-1.49 (m, 11H: darin 1.39 (s, 9H, OC(CH₃)₃), 1.85 (m, 2H, CH₂C(CH₃)₃), 3.59/3.68 (2s, 3H, OCH₃), 4.04 (m, 1H, ^{α}CH), 4.41 (m, 1H, ^{α}CH), 4.88-5.42 (m, 6H), 5.99 (m, 1H, NH), 6.22 (m, 1H, NH), 7.13-7.42 (m, 10H, ArH).
HR-TOF-MS (Methode 24): C₃₇H₅₅N₄O₈ ber. 683.4015, gef. 683.4033 [M+H]⁺.

### Beispiel 20A

### [N²-(Benzyloxycarbonyl)-3-tert-butyl-D-alanyl]-[3-tert-butyl-L-alanyl]-[(3R)-3-{(tert-butoxycarbonyl)-amino}-L-phenylalanin]

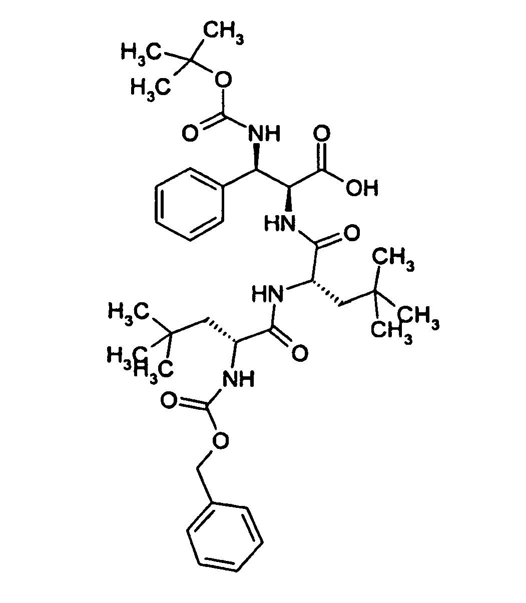

Der Ester (Beispiel 19A, 1.0 g, 1.46 mmol) wird in einer einer Mischung aus THF (100 ml) und Wasser (50 ml) vorgelegt und bei 0°C wird eine 0.5%ige Lithiumhydroxid-Lösung (12 ml, 59.6 mg, 2.49 mmol, 1.7 Äq.) zugetropft. Die Lösung wird 4 h bei 0°C bis zum vollständigen Umsatz gerührt. Zur Aufarbeitung wird die Reaktion mit Kaliumdihydrogenphosphat (996.4 mg, 7.32 mmol) versetzt, das Lösungsmittel bei 30°C Badtemperatur am Rotationsverdampfer vollständig entfernt und der Rückstand über die präparative HPLC (Methode 28) gereinigt. Man erhält 737.0 mg (75.3% d. Th.) Produkt.
HPLC (Methode 1) Rₜ = 2.86 min.
LC-MS (Methode 21): Rₜ = 2.89 min; MS (ESIpos): *m*/*z* (%) = 669 (20) [M+H]⁺, 569 (100) [M - C₄H₈ -CO₂ + H]⁺.
HR-TOF-MS (Methode 24): C₃₆H₅₃N₄O₈ [M+H]⁺ gef. 669.3881, ber. 669.3858.

### Beispiel 21A

### 2,5-Dioxopyrrolidin-1-yl [N²-(benzyloxycarbonyl)-3-tert-butyl-D-alanyl]-[3-tert-butyl-L-alanyl]-[(3R)-3-{(tert-butoxycarbonyl)-amino}-L-phenylalaninat]

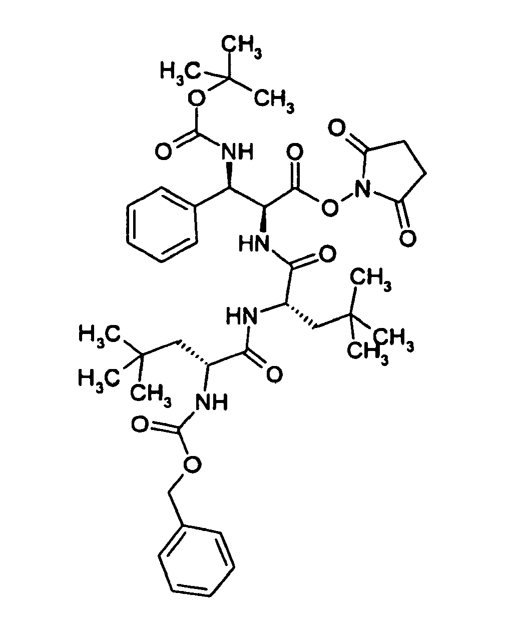

Unter Argonschutzgasatmosphäre wird die Beispielverbindung 20A (30.0 mg, 44.85 µmol) in Dichlormethan (6 ml) gelöst, die Lösung auf 0°C gekühlt, mit *N-*Hydroxysuccinimid (6.2 mg, 53.83 µmmol, 1.2 Äq.), EDC (10.3 mg, 53.83 µmol, 1.2 Äq.) und Molsieb 4Å versetzt und über Nacht bei 0°C nachgerührt. Das Lösungsmittel wird bei maximal 30°C Badtemperatur am Rotationsverdampfer entfernt, der Rückstand mit Essigsäureethylester aufgenommen, nacheinander zweimal mit 5%iger Zitronensäure-Lösung und zweimal mit ges. Natriumchloridlösung gewaschen, die org. Phase über Natriumsulfat getrocknet, filtriert, das Lösungsmittel bei 30°C Badtemperatur am Rotationsverdampfer eingeengt und aus dem Rückstand werden nach der Feinreinigung über eine RP-Kartusche (Eluent: Essigsäureethylester) 34.0 mg (98.7% d. Th.) Produkt isoliert.
HPLC (Methode 1) Rₜ = 2.99 min.
LC-MS (Methode 20): Rₜ = 2.88 min; MS (ESIpos): *m*/*z* (%) = 766 (57) [M+H]⁺, 666 (100) [M+H-C₄H₈-CO₂]⁺.

### Beispiel 22A

### N²-(tert-Butoxycarbonyl)-L-allothreonin

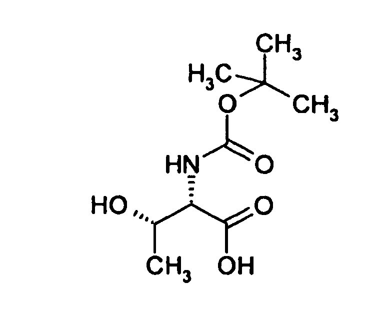

L-*allo*-Threonin (3.15 g, 26.44 mmol) wird in Wasser-Dioxan (1+2, 75 ml) gelöst, Di-*tert*-butyldicarbonat (6.35 g, 29.09 mmol, 1.1 Äquivalente) und Triethylamin (4.79 ml, 34.38 mmol, 1.3 Äquivalente) werden zugegeben und die Mischung wird über Nacht bei Raumtemperatur gerührt. Dann wird das Lösungsmittel im Vakuum abgezogen. Der Rückstand wird in Ethylacetat aufgenommen und 1 M Zitronensäure ausgeschüttelt. Die wässrige Phase wird noch mehrmals mit Ethylacetat extrahiert, bis sich darin kein Produkt mehr nachweisen lässt (HPLC, Methode 5). Dann werden die vereinigten organischen Extrakte über Natriumsulfat getrocknet, eingeengt und im Ölpumpenvakuum bis zur Gewichtskonstanz getrocknet. Das Produkt wird ohne weitere Reinigung weiter umgesetzt. Ausbeute: 6.5 g Rohprodukt.
HPLC (Methode 5): Rₜ = 3.23 min.
LC-MS (Methode 22): Rₜ = 2.51 min, MS (ESIneg): m/z (%) = 217.8 (100) [M-H]⁻.
¹H NMR (400 MHz, *d₆*-DMSO) δ (ppm) = 1.08 (d, *J* = 5.4 Hz, 3H), 1.38 (s, 9H), 3.72-3.84 (m, 2H), 6.77 (d, *J* = 7.4 Hz, 1H).

### Beispiel 23A

### Benzyl N²-(tert-Butoxycarbonyl)-L-allothreoninat

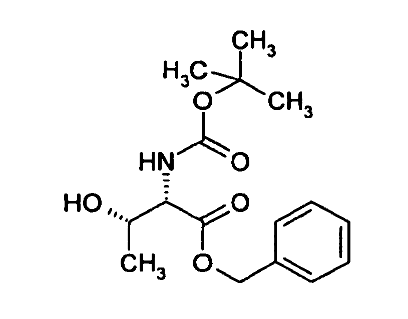

Die Methode wurde in Analogie zu folgender Literatur durchgeführt: S. B. Cohen, R. Halcomb, J. Am. Chem. Soc 2004, 124, 2534-2543. W. Jiang, J. Wanner, R. J. Lee, P.-Y. Bounaud, D. L. Boger, J. Am. Chem. Soc 2003, 125, 1877-1887.

Die Verbindung aus Beispiel 22A (6.8 g Rohprodukt, 26.44 mmol), wird in Methanol (177 ml) aufgenommen, Caesiumcarbonat (5.56 g, 17.06 mmol, 0.63 Äquivalente) wird hinzugefügt und bis zur vollständigen Auflösung gerührt. Dann entfernt man das Lösemittel destillativ, setzt DMF (42 ml) und dann Benzylbromid (4.06 ml, 34.12 mmol, 1.26 Äquivalente) zu. Man lässt die Mischung 16 h rühren, dann wird das DMF weitgehend im Vakuum abgezogen. Der Rückstand wird in Wasser aufgenommen und mit 3 Portionen Dichlormethan extrahiert. Die vereinigten org. Phasen werden über Natriumsulfat getrocknet, filtriert und im Vakuum eingeengt. Das Rohprodukt wird an Biotage RP18-Flash (Wasser-Acetonitril-Gradient: 0-5 min. 10% Acetonitril, 3-30 min. 10 - 90% Acetonitril, 30-35 min. 90% Acetonitril; Flow: 20ml/min.) aufgereinigt. Ausbeute: 5.00 g (16.16 mmol, 52% d. Th.).
HPLC (Methode 5): Rₜ = 4.36 min.
LC-MS (Methode 18): Rₜ = 2.39 min, MS (ESIpos): m/z (%) = 332.6 (25) [M+H]⁺.
¹H NMR (400 MHz, *d₆*-DMSO) δ (ppm) = 1.09 (d, *J* = 6.4, 3H), 1.37 (s, 9H), 3.82 (m, 1H), 3.95 (dd, *J* = 6.4, *J* = 8.1 Hz), 4.98 (d, *J* = 5.4 Hz, 1H), 5.09 (d, *J* = 12.7 Hz, 1H), 5.16 (d, *J* = 12.7 Hz, 1H), 7.10 (d, *J* = 8.1 Hz, 1H), 7.31-7.37 (m, 5H).

### Beispiel 24A

### Benzyl L-Allothreoninat-trifluoracetat

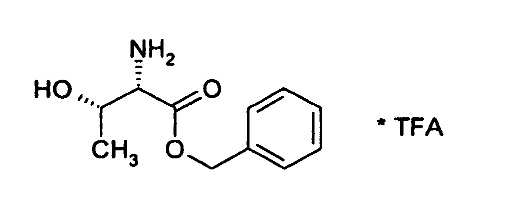

530 mg der Verbindung aus Beispiel 23A werden nach Arbeitsvorschrift 2 mit 8.0 ml der TFA-Lösung umgesetzt. Das Rohprodukt (589 mg, quant.) wird ohne weitere Reinigung weiter umgesetzt.
HPLC (Methode 5): Rₜ = 3.18 min.
LC-MS (Methode 22): Rₜ = 2.24 min, MS (ESIpos): m/z (%) = 210.0 (100) [M+H]⁺.
¹H NMR (400 MHz, *d₆*-DMSO) δ (ppm) = 1.15 (d, *J* = 6.6 Hz, 3H), 4.09-4.10 (m, 2H), 5.26 (s, 2H), 7.36-7.44 (m, 5H), 8.34 (br. S, 2H).

### Beispiel 25A

### Benzyl [N²-(tert-Butoxycarbonyl)-L-isoleucyl]-L-allothreoninat

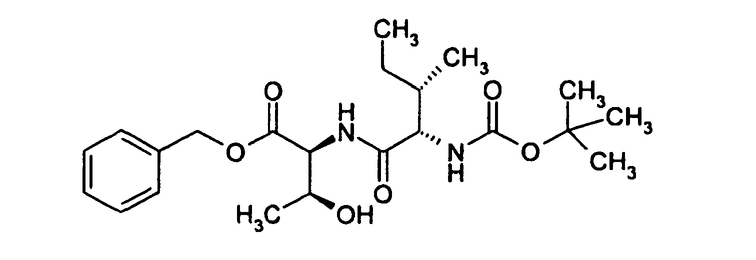

Die Verbindung aus Beispiel 24A (2.30 g 7.12 mmol) und N-(*tert*-Butoxycarbonyl)-L-isoleucin (2.14 g, 9.25 mmol, 1.3 Äquivalente) werden in DMF (21.0 ml) gelöst. 4-Methylmorpholin (1.3 ml, 12.02 mmol, 1.7 Äquivalente) und HATU (3.52 g, 9.25 mmol, 1.3 Äquivalente) werden zugegeben und der Ansatz 16 h bei Raumtemperatur gerührt. Dann wird der gesamte Ansatz zunächst nach Methode 45 und anschließend nach Methode 46 chromatographisch gereinigt. Ausbeute: 1.75 g (4.14 mmol, 58% d. Th.) als hellbeigefarbener amorpher Feststoff.
HPLC (Methode 5): Rₜ = 4.59 min.
LC-MS (Methode 18): Rₜ = 2.56 min, MS (ESIpos): m/z (%) = 423.8 (70) [M+H]⁺.
¹H NMR (400 MHz, *d₆*-DMSO) δ (ppm) = 0.74-0.78 (m, 6H), 1.01-1.07 (m, 2H), 1.10 (d, *J* = 6.3 Hz, 3H), 1.37 (s, 9H), 1.64-1.66 (m, 1H), 3.86-3.94 (m, 1H), 4.28 (dd, *J* = 7.3, *J* = 7.3 Hz, 1H), 5.05 (d, *J* = 5.6 Hz), 5.09 (d, *J* = 12.7 Hz, 1H), 5.13 (d, *J*= 12.7 Hz 1H), 6.70 (d, *J*= 9.0 Hz, 1H), 7.31-7.36 (m, 5H), 8.11 (d, *J* = 8.1 Hz).
HR-TOF-MS (Methode 24): C₂₂H₃₅N₂O₆ ber. 423.2490, gef. 423.2489 [M+H]⁺.

### Beispiel 26A

### Benzyl L-Isoleucyl-L-allothreoninat-trifluoracetat

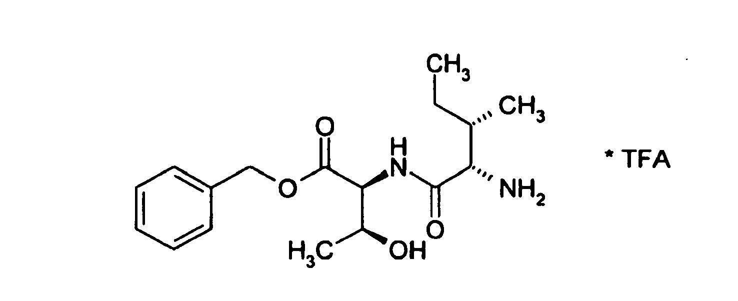

Die Verbindung aus Beispiel 25A (224 mg, 0.53 mmol) wird nach Arbeitsvorschrift 2 mit 8.0 ml der TFA Lösung behandelt. Man erhält 253 mg rohes Produkt Beispiel 26A (ca 91% rein, 0.53 mmol, quant.), das ohne weitere Reinigung umgesetzt wird.
HPLC (Methode 5): Rₜ = 3.51 min.
LC-MS (Methode 18): Rₜ = 1.58 min, MS (ESIpos): m/z (%) = 323.6 (100) [M+H]⁺.
¹H NMR (400 MHz, *d₆*-DMSO) δ (ppm) = 0.77-0.86 (m, 6H), 1.02 (m, 1H), 1.15 (d, *J* = 6.4 Hz, 3H), 1.45 (m, 1H), 1.77 (m, 1H), 3.97 (m, 1H), 4.34 (m, 1H), 5.11 (d, J = 12.5 Hz, 1H), 5.16 (d, *J*= 12.5 Hz, 1H), 7.37-7.39 (m, 5H), 7.47 (m, 1H), 8.07-8.08 (m, 3H), 8.69 (d, *J* = 7.3 Hz, 1H).

### Beispiel 27A

### Benzyl [N²-(tert-Butoxycarbonyl)-D-arginyl]-L-isoleucyl-L-allothreoninat

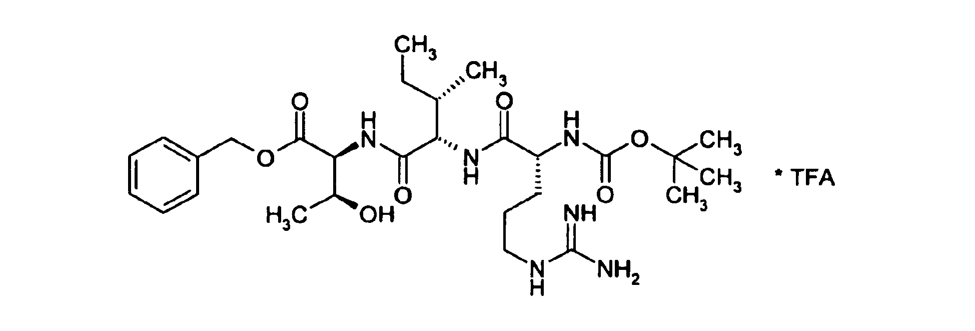

Die Verbindung aus Beispiel 26A (253 mg 91% rein, 0.53 mmol) und *N²*-(*tert* Butoxycarbonyl)-D-arginin (145 mg, 0.53 mmol, 1 Äquivalent) werden in DMF (3.0 ml) gelöst. 4-Methylmorpholin (76 µl, 0.70 mmol, 1.3 Äquivalente) und HATU (221 mg, 0.58 mmol, 1.1 Äquivalente) werden zugegeben und der Ansatz 16 h bei Raumtemperatur gerührt. Dann wird der gesamte Ansatz auf eine HPLC-Säule gegeben und chromatographisch gereinigt (Methode 34). Ausbeute: 364 mg (0.53 mmol, 99% d. Th.).
HPLC (Methode 5): Rₜ = 3.91 min.
LC-MS (Methode 18): Rₜ = 2.04 min, MS (ESIpos): m/z (%) = 579.9 (100) [M+H]⁺.
¹H NMR (400 MHz, d₆-DMSO) δ (ppm) = 0.72-1.16 (m, 8 H), 1.37 (s, 9H), 1.46 (m, 2H), 1.60 (m, 1H), 1.69 (m, 1H), 3.06 (m, 2H), 3.93-4.01 (m, 2H), 4.25 (m, 1H), 4.33 (m, 1H), 5.07-5.14 (m, 2H), 6.96 (d, *J* = 7.8, 1H), 7.35 (m, 5H), 7.45 (m, 1H), 7.66 (d, *J* = 8.8), 8.33 (m, 1H).

### Beispiel 28A

### Benzyl D-Arginyl-L-isoleucyl-L-allothreoninat-bis-trifluoracetat

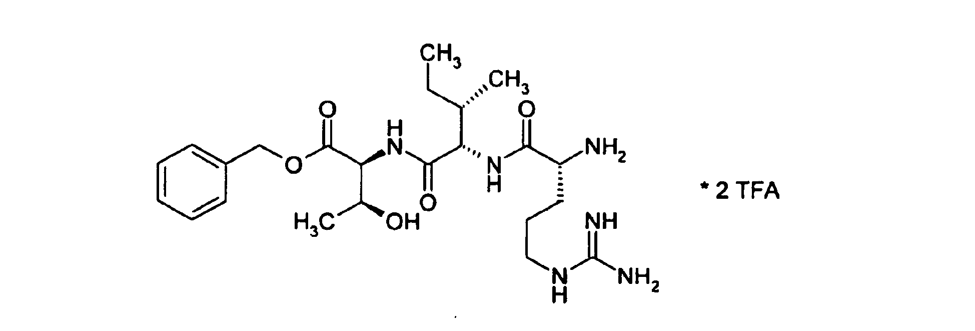

Die Verbindung aus Beispiel 27A (237 mg, 0.34 mmol) wird nach Arbeitsvorschrift 2 mit 2.0 ml der TFA Lösung behandelt. Man erhält 255 mg rohes Produkt Beispiel 28A (94% rein, 0.34 mmol, quant.), das ohne weitere Reinigung umgesetzt wird.
HPLC (Methode 5): Rₜ = 3.42 min.
LC-MS (Methode 22): Rₜ = 2.42 min, MS (ESIpos): m/z (%) = 479.3 (50) [M+H]⁺.
¹H NMR (400 MHz, *d₆*-DMSO) δ (ppm) = 0.73-0.81 (m, 5H), 1.11-1.19 (m, 5H), 1.33-1.49 (m, 3H), 1.74 (m, 3H), 3.10 (m, 2H), 3.88-3.95 (m, 2H), 4.25 (dd, *J* = 6.8, *J* = 7.1 Hz, 1H), 4.46 (dd, *J* = 7.3, *J* = 8.8 Hz, 1H), 5.09 (d, *J* = 12.5 Hz, 1H), 5.15 (dd, *J* = 12.5 Hz), 7.36 (m, 5H), 7.61 (m, 1H), 8.10 (m, 2H), 8.51 (d, *J* = 7.6 Hz, 1H), 8.57 (d, *J* = 9.0 Hz, 1H).

### Beispiel 29A

### Benzyl [N²-(tert-Butoxycarbonyl)-L-leucyl]-D-arginyl-L-isoleucyl-L-allothreoninat-trifluoracetat

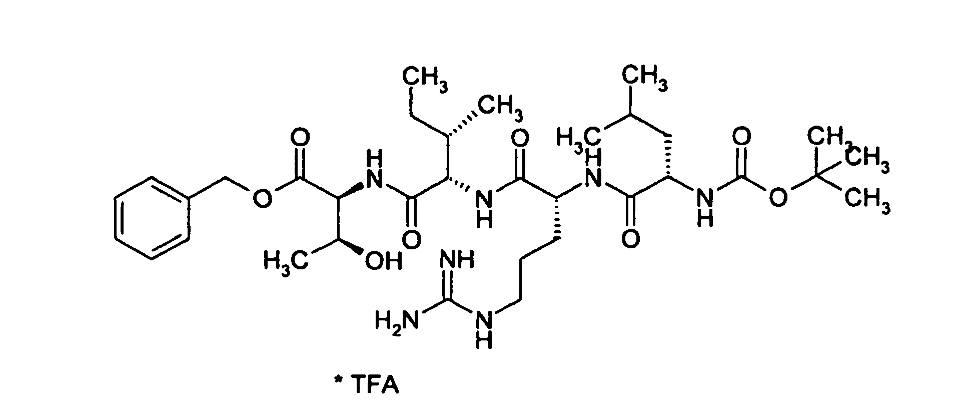

Die Verbindung aus Beispiel 28A (240 mg, 0.34 mmol) und *N*-(*tert*-Butoxycarbonyl)-L-leucin (79 mg, 0.34 mmol, 1 Äquivalent) werden in Dichlormethan-DMF (5+1, 6 ml) gelöst. Diisopropylethylamin (296 µl, 1.70 mmol, 5 Äquivalente) und HATU (194 mg, 0.51 mmol, 1.5 Äquivalente) werden zugegeben und der Ansatz 24 h bei Raumtemperatur gerührt. Dann wird der gesamte Ansatz auf eine Gelchromatographie-Säule gegeben und chromatographisch gereinigt (Methode 45, Eluens ist Methanol). Ausbeute: 146 mg (0.18 mmol, 53% d. Th.).
HPLC (Methode 5): Rₜ = 4.15 min.
LC-MS (Methode 18): Rₜ = 1.92 min, MS (ESIpos): m/z (%) = 692.8 (100), [M+H]⁺.
¹H NMR (400 MHz, *d₆*-DMSO) δ (ppm) = 0.72-1.23 (m, 22H), 1.37 (s, 9H), 1.38-1.71 (m, 3H), 3.08 (m, 2H), 3.91-4.00 (m, 2H), 4.26 (m, 1H), 4.33-4.42 (m, 2H), 5.07-5.15 (m, 2H), 6.92 (d, *J* = 7.8 Hz, 1H), 7.35 (m, 5H), 7.47 (m, 1H), 7.88 (d, *J* = 8.1 Hz, 1H), 7.93 (d, *J* = 9.0 Hz, 1H), 8.35 (d, *J* = 7.3 Hz, 1H).

### Beispiel 30A

### Benzyl L-Leucyl-D-arginyl-L-isoleucyl-L-allothreoninat-bis-trifluoracetat

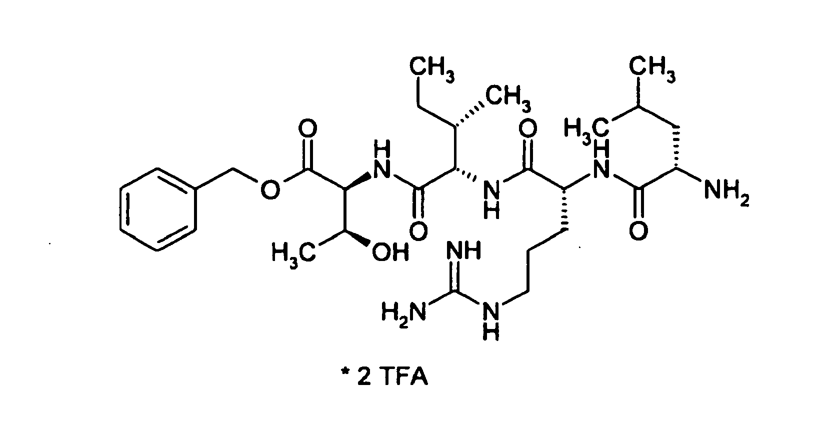

Die Verbindung aus Beispiel 29A (220 mg, 0.27 mmol) wird nach Arbeitsvorschrift 2 mit 2.0 ml der TFA Lösung behandelt. Man erhält 223 mg rohes Produkt Beispiel 28A (0.27 mmol, quant.), das ohne weitere Reinigung umgesetzt wird.
HPLC (Methode 4): Rₜ = 3.80.
LC-MS (Methode 22): Rₜ = 2.54 min, MS (ESIpos): m/z (%) = 592.4 (2) [M+H]⁺.
¹H NMR (400 MHz, *d₆*-DMSO) δ (ppm) = 0.73-1.11 (m, 13H), 1.22-1.74 (m, 12H), 3.11 (m, 4H), 3.60 (m, 2H), 3.87 (m, 1H), 3.95 (m, 1H), 4.25 (m, 1H), 4.38 (dd, *J* = 7.8, *J* = 8.6 Hz, 1H), 4.64 (dd, *J* = 7.8, *J* = 13.7 Hz, 1H), 5.09 (d, *J* = 12.7 Hz, 1H), 5.13 (d, *J* = 12.7 Hz, 1H), 7.35 (m, 5H), 7.58 (m, 1H), 8.07 (m, 2H), 8.25 (d, *J* = 8.8 Hz, 1H), 8.39 (d, *J* = 7.6 Hz, 1H), 8.77 (d, *J* = 8.3 Hz, 1H).

### Beispiel 31A

### Benzyl [(3R)-N²-(tert-Butoxycarbonyl)-3-hydroxy-L-leucyl]-L-leucyl-D-arginyl-L-isoleucyl-L-allothreoninat-trifluoracetat

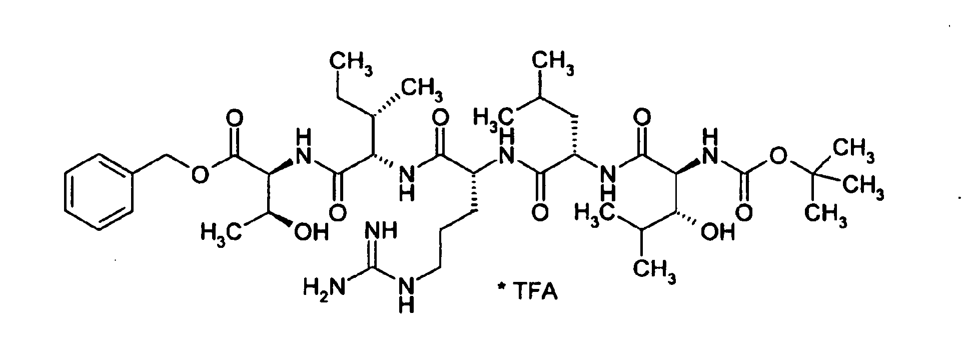

Die Verbindung aus Beispiel 30A (223 mg, 0.27 mmol) und *N*-(*tert*-Butoxycarbonyl)-(3*R*)-3-hydroxy-L-leucin (89 mg, 0.33 mmol, 1.22 Äquivalente) werden in DMF (6 ml) gelöst und die Lösung auf -20 °C gekühlt. 4-Methylmorpholin (150 µl, 1.36 mmol, 5 Äquivalente) und HATU (165 mg, 0.44 mmol, 1.6 Äquivalente) werden zugegeben und der Ansatz 16 h bei Raumtemperatur gerührt. Dann wird der gesamte Ansatz auf eine Gelchromatographie-Säule gegeben und chromatographisch gereinigt (Methode 45, Eluens ist Methanol). Ausbeute: 188 mg (0.20 mmol, 74% d. Th.).
HPLC (Methode 5): Rₜ = 4.24 min.
LC-MS (Methode 19): Rₜ = 1.99 min, MS (ESIpos): m/z (%) = 821.9 (100) [M+H]⁺.
¹H NMR (400 MHz, *d₆*-DMSO) δ (ppm) = 0.71-0.90 (m, 15H), 1.00 (m, 1H), 1.10 (d, *J*= 6.4 Hz, 3H), 1.24-1.26 (m, 3H), 1.38 (s, 9H), 1.42-1.71 (m, 6H), 3.06-3.17 (m, 3H), 3.45 (m, 1H), 3.61 (m, 1H), 3.93 (m, 1H), 4.05 (m, 1H), 4.26 (m, 1H), 4.35 (m, 2H), 4.54 (d, *J* = 7.8 Hz, 1H), 5.07-5.15 (m, 2H), 5.45 (d, *J* = 9.0 Hz, 1H), 7.35 (m, 5H), 7.46 (m, 1H), 7.85 (d, *J* = 7.8 Hz, 1H), 7.89 (d, *J* = 8.8 Hz, 1H), 7.97 (d, *J* = 8.1 Hz, 1H), 8.35 (d, *J* = 7.6 Hz, 1H).

### Beispiel 32A

### [(3R)-N²-(tert-Butoxycarbonyl)-3-hydroxy-L-leucyl]-L-leucyl-D-arginyl-L-isoleucyl-L-allothreonin-trifluoracetat

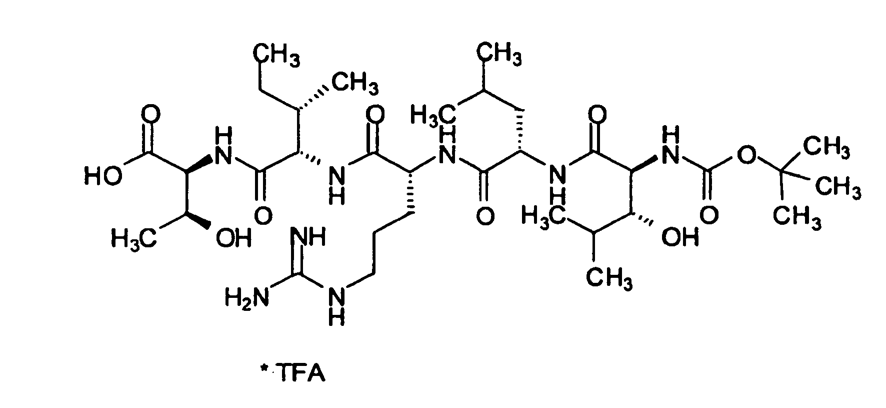

Die Verbindung aus Beispiel 31A (100 mg, 0.11 mmol) wird in Eisessig (4.3 ml) gelöst, 10% Palladium auf Aktivkohle (22 mg) wird hinzugefügt und der Ansatz 2 h bei Raumtemperatur unter Normaldruck hydriert. Man filtriert vom Katalysator ab und lyophilisiert das Filtrat. Das Rohprodukt wird chromatographisch gereinigt (Methode 33). Man erhält 58 mg (60 µmol, 55% d. Th.) der Titelverbindung.
HPLC (Methode 5): Rₜ = 3.75 min.
LC-MS (Methode 1Methode 19): Rₜ = 1.80 min, MS (ESIpos): m/z (%) = 731.8 (100) [M+H]⁺.

### Beispiel 33A

### [N²-(tert-Butoxycarbonyl)-glycyl]-(3S)-3-hydroxy-O⁴-methyl-L-aspartat

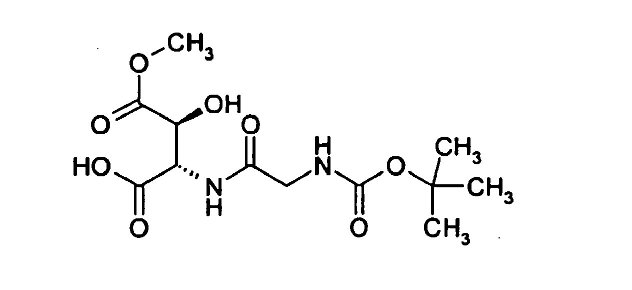

(3S)-3-Hydroxyasparaginsäure wird nach der Vorschrift G. Cardillo, L. Gentilucci, A. Tolomelli, C. Tomasini, Synlett 1999, 1727-1730, dargestellt und analog P. G. Mattingly, M. J. Miller, J. Org. Chem. 1983, 48, 3556-3559, unter Verwendung von Mikrowellenstrahlung in einem geschlossenen Reaktor zu (2*S*,3*S*)-2-Amino-3-hydroxy-4-methoxy-4-oxobuttersäure Hydrochlorid umgesetzt. (2*S*,3*S*)-2-Amino-3-hydroxy-4-methoxy-4-oxobuttersäure Hydrochlorid (447 mg, 2.24 mmol) wird in DMF (9 ml) gelöst. Die Lösung wird auf 0 °C gekühlt, Boc-glycin-*N*-hydroxysuccinimidester (763 mg, 2.91 mmol, 1.3 Äquivalente), DMAP (14 mg, 0.11 mmol, 0.05 Äquivalente) und schließlich DIPEA (1170 µl, 6.72 mmol, 3 Äquivalente) werden hinzugefügt. Die Mischung lässt man langsam auf Raumtemperatur erwärmen und dann noch 2 h rühren. Der Ansatz wird mit Eisessig angesäuert, mit Acetonitril versetzt und an Sephadex LH 20 (Methode 45) chromatographiert. Produkthaltige Fraktionen werden vereinigt, eingeengt und erneut chromatographiert (Methode 46). Das erhaltene Produkt (761 mg, quant.) wird ohne weitere Reinigung weiter umgesetzt. Für analytische Zwecke wird eine reine Probe durch HPLC (Methode 44) erhalten.
HPLC (Methode 5): Rₜ = 3.15 min
LC-MS (Methode 1): Rₜ = 1.17 min, MS (ESIpos) = 321.2 [M+H]⁺.
[α]²⁰_{Na} = + 39° (c = 0.55, MeOH).
¹H NMR (300 MHz, d₆-DMSO) δ (ppm) = 1.40 (s, 9 H), 3.49 - 3.60 (m, 2 H), 3.61 (s, 3 H), 4.29 (m, 1H), 4.73 (d, *J* = 6.6 Hz, 1H), 7.01 (m, 1H), 7.49 (d, *J* = 6.99 Hz, 1H).
¹³C-NMR (d₆-Aceton, 126 MHz, DEPT) δ (ppm) = 28.5 (CH₃), 42.2 (CH₂), 51.8 (CH₃), 53.7 (CH), 56.0 (CH), 79.2 (quat), 169.6 (quat), 169.7 (quat), 172.8 (quat), 173.8 (quat).
HR-TOF-MS (Methode 24): C₁₂H₂₂N₂O₈ [M+H]⁺ ber.: 321.1298, gef.: 321.1299.

### Beispiel 34A

### Benzyl [N²-(tert-Butoxycarbonyl)-glycyl]-[(3S)-3-hydroxy-O⁴-methyl-L-aspartyl]-O³-(tert-butyl)-L-serinat

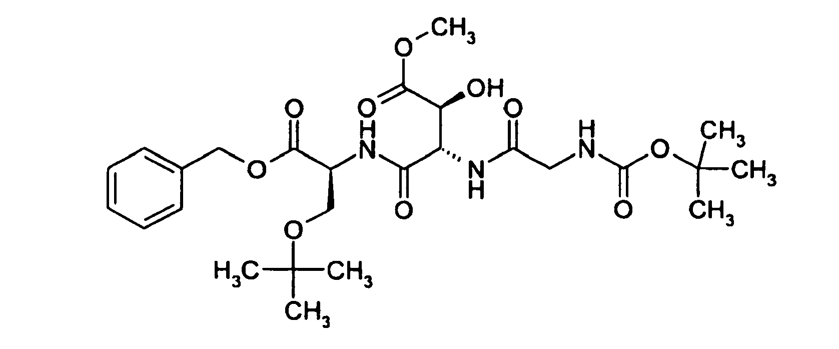

Die Verbindung aus Beispiel 33A (390 mg, 1.22 mmol) und Benzyl *O*-(*tert*-Butyl)-L-serinat (445.14 mg, 1.22 mmol, 1 Äquivalent) werden in DMF (9 ml) gelöst. Die Lösung wird auf 0°C gekühlt, dann werden 2.44 ml (2 Äquivalente) einer 1 M Lösung von 4-Methylmorpholin in DMF hinzugefügt gefolgt von HATU (925 mg, 2.44 mmol, 2 Äquivalente). Die Mischung wird ca 15 Minuten bei 0°C gerührt, weitere 2.44 ml (2 Äquivalente) der 4-Methylmorpholinlösung werden zugesetzt und 2 h bei Raumtemperatur gerührt. Dann wird Wasser zugegeben und mit 2 Portionen Ethylacetat extrahiert. Die vereinigten organischen Extrakte werden mit 1 M Zitronensäure, konz. Natriumhydrogencarbonat und konz. Kochsalzlösung gewaschen. Die organische Phase wird dann über Natriumsulfat getrocknet, das Lösemittel wird abdestilliert und der Rückstand wird chromatographiert (Methode 46). Ausbeute: 413 mg (61% d. Th.) als Feststoff.
HPLC: Rₜ = 4.46 min.
LC-MS: Rₜ = 2.37 min. MS (ESIpos): 554.4 [M+H]⁺.
[α]²⁰_{Na} = -1.7 (c = 0.57, CH₃CN).
¹H NMR (400 MHz, d₆-DMSO) δ (ppm) = 1.09 (s, 9 H), 1.45 (s, 9 H), 3.17 (br s, 1H), 3.50 - 3.56 (dd, *J* = 2.85 Hz, *J* = 8.8 Hz, 1H), 3.59 (s, 3 H), 3.61 - 3.67 (m, 3 H), 4.66 (s, 1H), 4.70 - 4.72 (m, 1H), 4.97 (dd, *J* = 2.7 Hz, *J* = 8.65 Hz, 1H), 5.14 (d, *J* = 12.3 Hz, 1H), 5.19 (br s, 1H), 5.23 (d, *J* = 12.3 Hz, 1H), 7.11 (d, J = 7.45 Hz, 1H), 7.19 (d, *J* = 8.5 Hz, 1H), 7.46 (m, 5 H).
¹³C-NMR-DEPT (126 MHz, d₆-DMSO) δ (ppm) = 28.5 (CH₃), 29.6 (CH₃), 45.5 (CH₂), 54.4 (CH₃), 54.5 (CH), 55.9 (CH), 62.8 (CH₂), 68.6 (CH₂), 71.7 (CH), 75.0 (quat), 81.8 (quat), 129.6 (CH), 129.7 (CH), 129.9 (CH), 136.7 (quat), 157.3 (quat), 169.9 (quat), 171.2 (quat), 173.4 (quat).
HR-TOF-MS: C₂₆H₄₀N₃O₁₀ [M+H]⁺ ber.: 554.2714, gef.: 554.2707.

### Beispiel 35A

### Benzyl [N²-(tert-Butoxycarbonyl)glycyl]-[(3S)-3-hydroxy-L-asparaginyl]-O³-(tert-butyl)-L-serinat

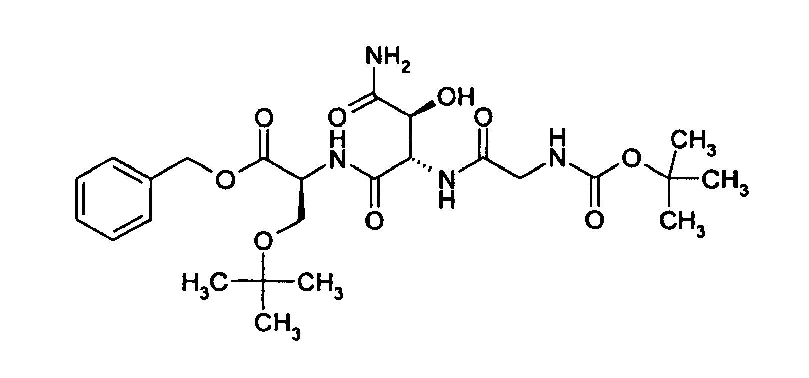

Verbindung 34A (430 mg, 0.78 mmol) wird in Acetonitril (30 ml) gelöst und auf 0°C gekühlt. Ammoniak konz. (15 ml) wird hinzugegeben und der Ansatz ca 15 min gerührt. Sobald die Umsetzung vollständig ist (Nachweis mit HPLC, Methode 5) wird durch Zugabe von Essigsäure sauer gestellt und mit Wasser verdünnt. Man extrahiert mit 2 Portionen Ethylacetat. Die vereinigten organischen Extrakte werden mit konz. Kochsalzlösung gewaschen, über Natriumsulfat getrocknet, eingeengt und mit HPLC gereinigt. Ausbeute: 158 mg (38% d. Th.) als Feststoff.
[α]²⁰_{Na} = - 16° (c = 0.53, MeOH).
HPLC: Rₜ = 4.18 min. LC-MS: Rₜ = 2.14 min, MS (ESIpos) *m*/*z* = 539.4 [M+H]⁺.
HR-TOF-MS: C₂₅H₃₉N₄O₉ [M+H]⁺ calcd: 539.2717, found: 539.2709.
¹H NMR (300 MHz, d₆DMSO) δ (ppm) = 1.05 (s, 9 H), 1.38 (s, 9 H), 3.50 - 3.56 (m, 2 H), 3.61 - 3.67 (m, 2 H), 4.33 (s, 1H), 4.48 (m, 1H), , 4.71 (m, 1H, 5.10 (d, *J* = 12.7 Hz, 1 H), 5.19 (d, *J* = 12.7 Hz, 1H), 7.07 (m, 1H), 7.17 (br s, 1H), 7.33 - 7.46 (m, 6 H), 7.65 (d, *J* = 8.8 Hz, 1H), 7.83 (d, *J* = 7.7 Hz, 1H).
¹³C-NMR-DEPT (126 MHz, d₆-Aceton) δ (ppm) = 27.54 (CH₃), 28.58 (CH₃), 44.73 (CH₂), 54.06 (CH), 55.67 (CH), 62.61 (CH₂), 67.08 (CH₂), 71.64 (CH), 73.90 (quat), 79.70 (quat), 128.75 (CH), 128.82 (CH), 129.25 (quat), 137.20 (quat), 157.04 (quat), 170.56, 170.62 (quat), 170.72 (quat), 174.13 (quat).

### Beispiel 36A

### Benzyl Glycyl-[(3S)-3-hydroxy-L-asparaginyl]-O³-(tert-butyl)-L-serina-trifluoracetat

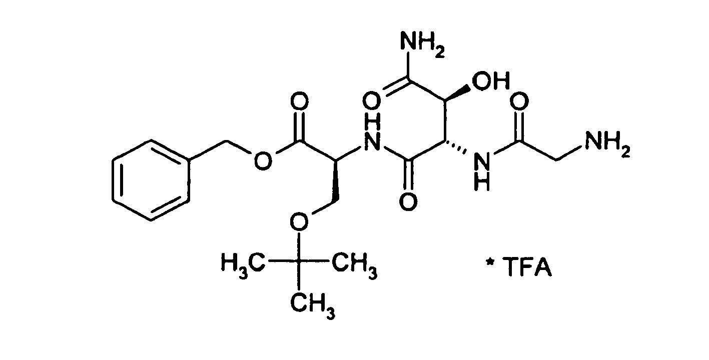

55 mg (100 µmol) der Beispielverbindung 35A werden nach Arbeitsvorschrift 2 in 2 ml der Reagenzlösung umgesetzt. Das Produkt wird ohne weitere Reinigung umgesetzt. Ausbeute: 50 mg (quant.).
HPLC (Methode 3): Rₜ = 3.05 min.
LC-MS (Methode 22): Rₜ = 2.22 min; MS (ESIpos) *m*/*z* (%) = 383.0 (100) [M+H]⁺.

### Beispiel 37A

### {(3R)-N²-[(Benzyloxy)carbonyl]-3-[(tert-butoxycarbonyl)amino]-L-phenylalanyl}-[(3R)-3-hydroxy-L-leucyl]-L-leucyl-D-arginyl-L-isoleucyl-L-allothreonyl-glycyl-[(3S)-3-hydroxy-L-asparaginyl]-L-serin-trifluoracetat

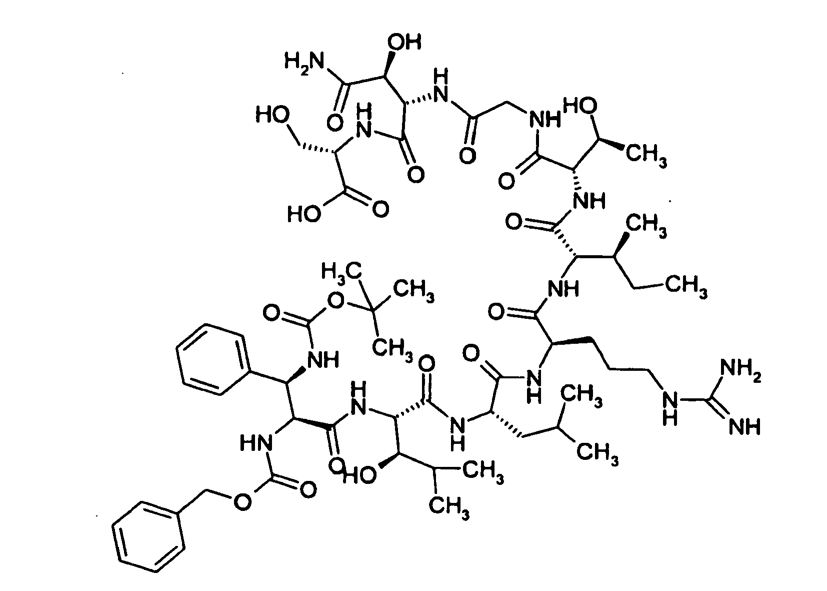

Das Abbauprodukt aus Beispiel 3A (2.8 g, 2.53 mmol) und die Verbindung aus Beispiel 17A (1.5 g, 2.53 mmol, 1 Äq.) werden in DMF (70 ml) vorgelegt. Die Lösung wird auf 0°C gekühlt, mit DIEA (2.64 ml, 2.0 g, 15.14 mmol, 6 Äq.) versetzt, die Reaktionsmischung langsam auf RT erwärmt und 3 h bei dieser Temperatur gerührt. Das Lösungsmittel wird teilweise am Rotationsverdampfer entfernt, wobei die Badtemperatur 30°C nicht überschreiten sollte. Nach der Feinreinigung über die präparative HPLC (Methode 28) werden 2.2 g (61.4% d. Th.) Produkt isoliert.
HPLC (Methode 9) Rₜ = 17.03 min.
LC-MS (Methode 20): Rₜ = 1.79 min; MS (ESIpos): *m*/*z* (%) = 1301 (28) [M+H]⁺, 601 (100) [M - C₄H₈ - CO₂ + 2H]²⁺; MS (ESIneg.): *m*/*z* (%) = 1300 (100) [M - H]⁻.

### Beispiel 38A

### {(3R)-N2-[(Benzyloxy)carbonyl]-3-amino-L-phenylalanyl}-[(3R)-3-hydroxy-L-leucyl]-L-leucyl-D-arginyl-L-isoleucyl-L-allothreonyl-glycyl-[(3S)-3-hydroxy-L-asparaginyl]-L-serin-trifluoracetat

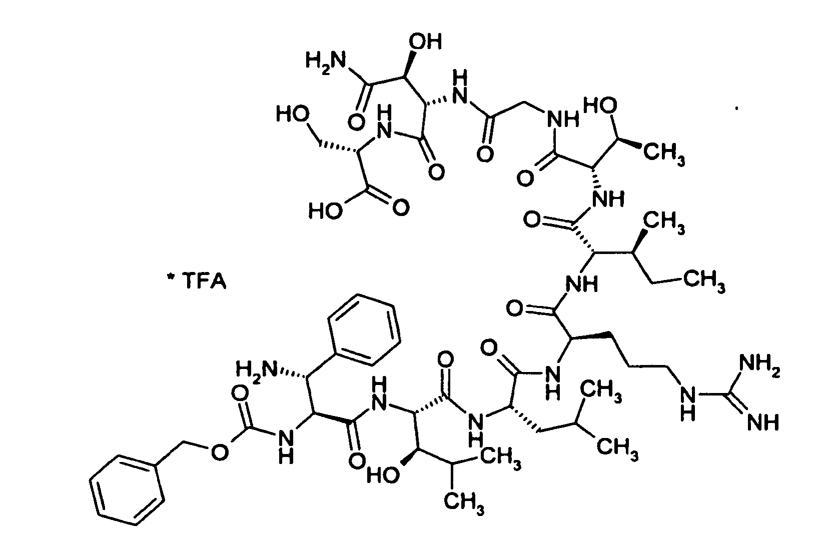

Von der Verbindung aus Beispiel 37A (2.2 g, 1.55 mmol) wird nach der Arbeitsvorschrift 1 die Boc-Schutzgruppe abgespalten. Nach dem Chromatographieren (Methode 28) erhält man 1.8 g (87.1% d. Th.) Produkt.
HPLC (Methode 9) Rₜ = 12.37 min.
LC-MS (Methode 20): Rₜ = 1.17 min; MS (ESIpos): *m*/*z* (%) = 1201 (8) [M+H]⁺, 601 (100) [M + 2H]²⁺; MS (ESIneg.): *m*/*z* (%) = 1199 (100) [M - H]⁻.

### Beispiel 39A

### {(3R)-N²-[(Benzyloxy)carbonyl]-3-amino-L-phenylalanyl}-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-trifluoracetat

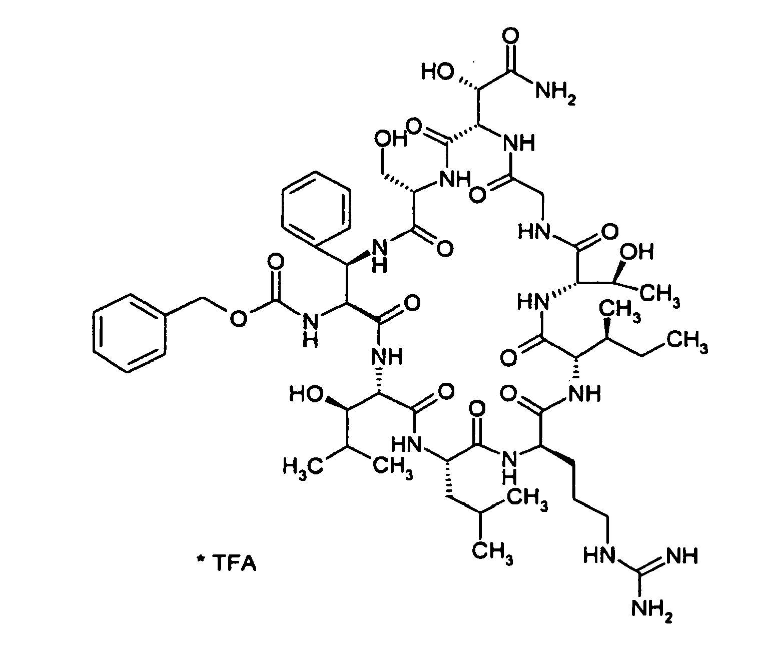

In einer Argonschutzgasatmosphäre wird die Verbindung aus Beispiel 38A (1.3 g, 0.89 mmol) in DMF (700 ml) vorgelegt, die Lösung auf 0°C gekühlt, anschließend mit HATU (1.0 g, 2.68 mmol, 3 Äq.) und NMM (0.8 ml, 0.7 g, 7.14 mmol, 8 Äq.) versetzt. Nach 3 h Rühren bei 0°C unter leichtem Argonstrom ist der Umsatz vollständig erfolgt. Zur Aufarbeitung wird die Reaktion mit Methanol (30 ml) versetzt, das Lösungsmittel am Rotationsverdampfer bei 30°C Badtemperatur entfernt, der Rückstand mit Methanol (30 ml) im Ultraschallbad suspendiert und der ausgefallene Niederschlag wird durch Filtrieren abgetrennt. Das Filtrat wird am Rotationsverdampfer bei maximal 30°C Badtemperatur eingeengt und nach der Feinreinigung über die präparative HPLC (Methode 28) erhält man 905.6 mg (78.3% d. Th.) der Zielverbindung.
HPLC (Methode 9) Rₜ = 15.09 min.
LC-MS (Methode 18): Rₜ = 1.75 min; MS (ESIpos): m/z (%) = 1183 (100) [M+H]⁺.

### Beispiel 40A

### Pentafluorophenyl {N2-[(benzyloxy)carbonyl]-3-tert-butyl-D-alanyl}-{3-tert-butyl-L-alanyl}-[(3R)-3-[(tert-butoxycarbonyl)amino]-L-phenylalaninat]

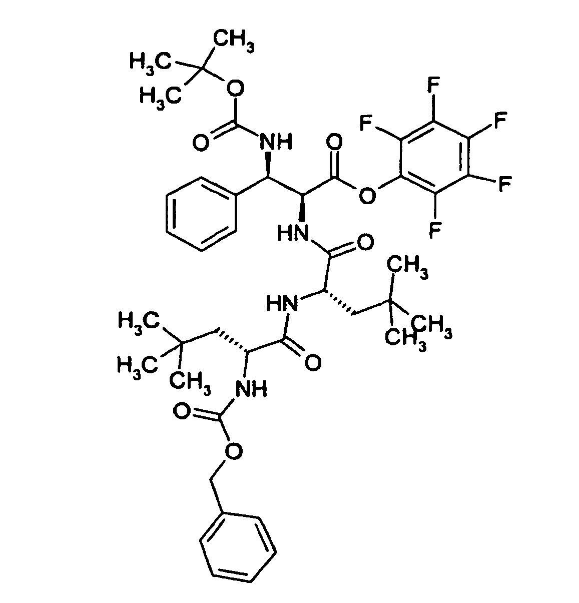

Unter Argonschutzgasatmosphäre wird die Säure (Beispiel 20A, 170.0 mg, 0.25 mmol) in Methylenchlorid (5 ml) vorgelegt, auf 0°C gekühlt, anschließend Pentafluorphenyl Diphenylphosphinat (146,5 mg, 0.38 mmol, 1.5 Äq.) und NMM (140 µl, 128.5 mg, 1.27 mmol, 5 Äq.) zugegeben. Die Reaktion wird langsam auf RT erwärmt und über Nacht bei dieser Temperatur nachgerührt. Zur Aufarbeitung wird das Lösungsmittel bei 30°C Badtemperatur am Rotationsverdampfer entfernt, der Rückstand über die präparative HPLC (Methode 32) gereinigt, die eingeengten Produktfraktionen nochmals mit Toluol und Dichlormethan versetzt, anschließend am Rotationsverdampfer bei 30°C Badtemperatur die Lösungsmittel wieder vollständig entfernt, den Rückstand 2 h lyophilisiert und das Produkt bei -25°C gelagert. Man erhält 130.8 mg (61.6% d. Th.) Produkt.
HPLC (Methode 1) Rₜ = 3.30 min.
LC-MS (Methode 18): Rₜ = 3.42 min; MS (ESIpos): *m*/*z* (%) = 835 (100) [M+H]⁺, 735 (54) [M - C₄H₈ - CO₂ + H]⁺, MS (ESIneg.): *m*/*z* (%) = 833 (60) [M - H]⁻, 183 (100).

### Beispiel 41A

### Chlortrityl-Harz gebundenes O3-(tert-Butyl)-N2-[(9H-fluoren-9-ylmethoxy)carbonyl]-L-serin

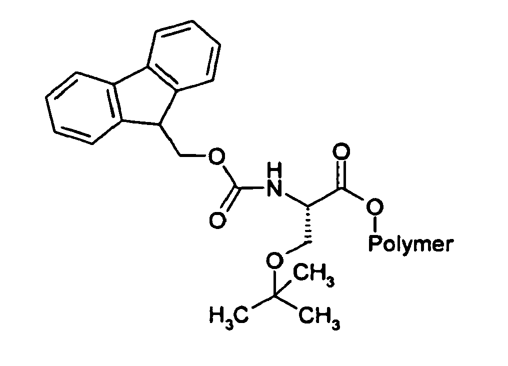

Chlortritylchlorid-Harz (4.0 g, 5.96 mmol) wird in Dichlormethan (40 ml) vorgelegt und anschließend mit *O*-(*tert*-Butyl)-*N*-[(*9H*-fluoren-9-ylmethoxy)carbonyl]-L-serin (8.0 g, 20.86 mmol, 3.5 Äq.) und DIEA (10.3 ml, 7.7 g, 59.60 mol, 10 Äq.) versetzt. Nach 20 h Schütteln bei RT wird das Polymer über eine Fritte abgesaugt und anschließend nacheinander dreimal mit Dichlormethan/ Methanol/ DIEA 17/2/1, dreimal mit Dichlormethan, zweimal mit DMF und dreimal mit Dichlormethan nachgewaschen.

Eine Probeabspaltung mit Essigsäure/ Trifluorethanol/ Dichlormethan 1:1:3 ergibt die korrespondierende fmoc-geschützte Aminosäure.
HPLC (Methode 13) Rₜ = 1.95 min.
LC-MS (Methode 20): Rₜ = 2.38 min; MS (ESIpos): *m*/*z* (%) = 384 (35) [M+H]⁺, 767 (10) [2M+H]⁺, 105 (100); MS (ESIpos): *m*/*z* (%) = 382 (25) [M - H]⁻, 765 (10) [2M - H]⁻, 160 (100).

### Beispiel 42A

### Chlortrityl-Harz gebundenes {N²-[(9H-Fluoren-9-ylmethoxy)carbonyl]-N⁴-trityl-L-asparaginyl}-[O³-(tert-butyl)-L-serin]

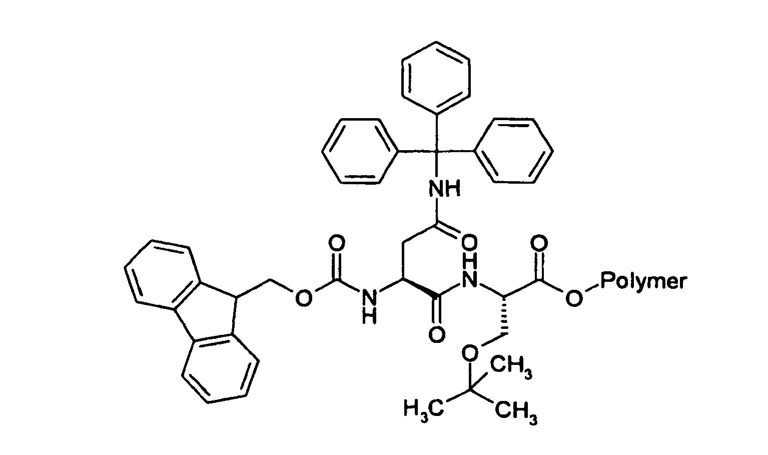

Die Fmoc-Schutzgruppe wird von dem Polymer (Beispiel 41A, 2.0 g, 2.98 mmol), wie in Arbeitsvorschrift 7 beschrieben ist, abgespalten. Anschließend wird die am Harz gebundene entschützte Aminosäure mit *N*²-[(9*H*-Fluoren-9-ylmethoxy)carbonyl]-*N-*trityl-L-asparagin (3.6 g, 5.96 mmol, 2 Äq.), DIEA (1.5 ml, 1.2 g, 8.94 mmol, 3 Äq.) und TBTU (1.9 g, 5.96 mmol) über Nacht zum F-moc geschützten Dipeptid umgesetzt. Analog der Arbeitsvorschrift 7 erfolgt die Aufarbeitung des Polymeres. Es wird das korrespondierende seitenkettengeschützte Dipeptid nach der Probeabspaltung bestätigt.
HPLC (Methode 13) Rₜ = 2.43 min.
LC-MS (Methode 18): Rₜ = 3.10 min; MS (ESIpos): *m*/*z* (%) = 740 (5) [M+H]⁺, 243 (100); MS (ESIpos): *m*/*z* (%) = 738 (40) [M - H]⁻, 516 (85) [M -Fmoc- H]⁻, 542 (100).
HR-TOF-MS (Methode 24): C₄₅H₄₆N₃O₇ [M+H]⁺ gef. 740.3342, ber. 740.3331.

### Beispiel 43A

### Chlortrityl-Harz gebundenes {N²-[(9H-fluoren-9-ylmethoxy)carbonyl]glycyl}-(N⁴-trityl-L-asparaginyl)-[O³-(tert-butyl)-L-serin]

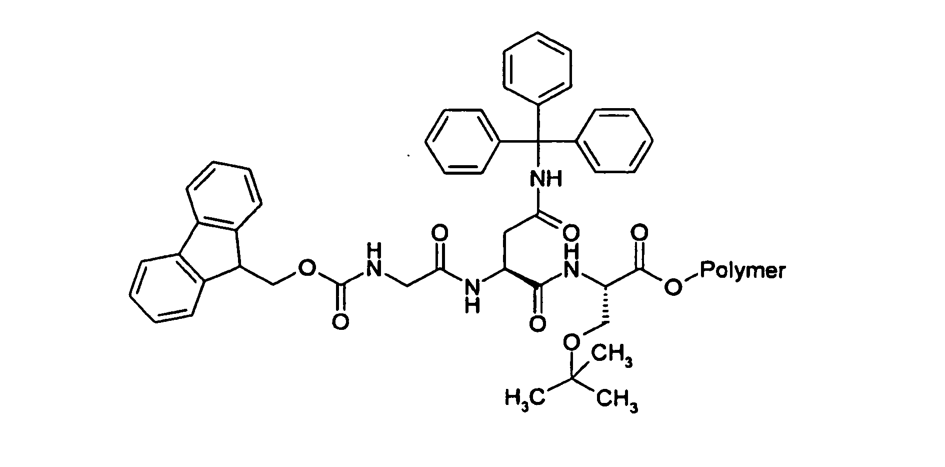

Die Fmoc- Schutzgruppe wird von dem Polymer (Beispiel 42A, 2.0 g, 2.98 mmol), wie in Arbeitsvorschrift 7 beschrieben ist, abgespalten. Anschließend wird das am Harz gebundene entschützte Dipeptid mit *N*-[(9*H*-Fluoren-9-ylmethoxy)carbonyl]glycin (1.8 g, 5.96 mmol, 2 Äq.), DIEA (1.5 ml, 1.2 g, 8.94 mmol, 3 Äq.) und TBTU (1.9 g, 5.96 mmol, 2 Äq.) über Nacht zum F-moc geschützten Tripeptid umgesetzt. Analog der Arbeitsvorschrift 7 erfolgt die Aufarbeitung des Polymeres. Es wird das korrespondierende seitenkettengeschützte Tripeptid nach der Probeabspaltung bestätigt.
HPLC (Methode 13) Rₜ = 2.33 min.
LC-MS (Methode 21): Rₜ = 2.99 min; MS (ESIpos): *m*/*z* (%) = 797 (5) [M+H]⁺, 243 (100); MS (ESIpos): *m*/*z* (%) = 795 (40) [M - H]⁻, 573 (100) [M - Fmoc - H]⁻.
HR-TOF-MS (Methode 24): C₄₇H₄₉N₄O₈ [M+H]⁺ gef. 797.3556, ber. 797.3545.

### Beispiel 44A

### Chlortrityl-Harz gebundenes {O³-(tert-Butyl)-N²-[(9H-fluoren-9-ylmethoxy)carbonyl]-allothreonyl}-glycyl-(N⁴-trityl-L-asparaginyl)-[O³-(tert-butyl)-L-serin]

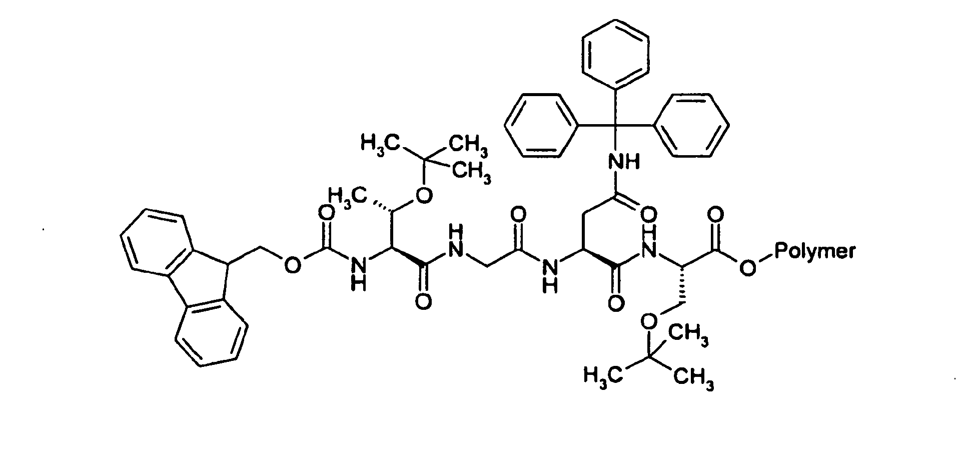

Die Fmoc-Schutzgruppe wird von dem Polymer (Beispiel 43A, 1000.0 mg, 1.49 mmol), wie in Arbeitsvorschrift 7 beschrieben ist, abgespalten. Anschließend wird das am Harz gebundene entschützte Tripeptid mit *O*-(*tert*-Butyl)-*N*-[(9*H*-fluoren-9-ylmethoxy)carbonyl]-allothreonin (1184.5 mg, 2.98 mmol, 2 Äq.), DIEA (772 µl, 577.7 mg, 3 Äq.) und TBTU (956.6 mg, 2.98 mmol, 2 Äq.) über Nacht zum F-moc geschützten Tetrapeptid umgesetzt. Analog der Arbeitsvorschrift 7 erfolgt die Aufarbeitung des Polymeres. Es wird das korrespondierende seitenkettengeschützte Tetrapeptid nach der Probeabspaltung bestätigt.
HPLC (Methode 13) Rₜ = 2.48 min.
LC-MS (Methode 20): Rₜ = 3.02 min; MS (ESIpos): *m*/*z* (%) = 954 (100) [M+H]⁺; MS (ESIpos): *m*/*z* (%) = 752 (100) [M - H]⁻.
HR-TOF-MS (Methode 24): C₅₅H₆₄N₅O₁₀ [M+H]⁺ gef. 954.4663, ber. 954.4648.

### Beispiel 45A

### Chlortrityl-Harz gebundenes {N²-[(9H-Fluoren-9-ylmethoxy)carbonyl]}-L-isoleucyl-[O³-(tert-butyl)]-L-allothreonyl-glycyl-(N³-trityl-L-asparaginyl)-[O³-(tert-butyl)-L-serin]

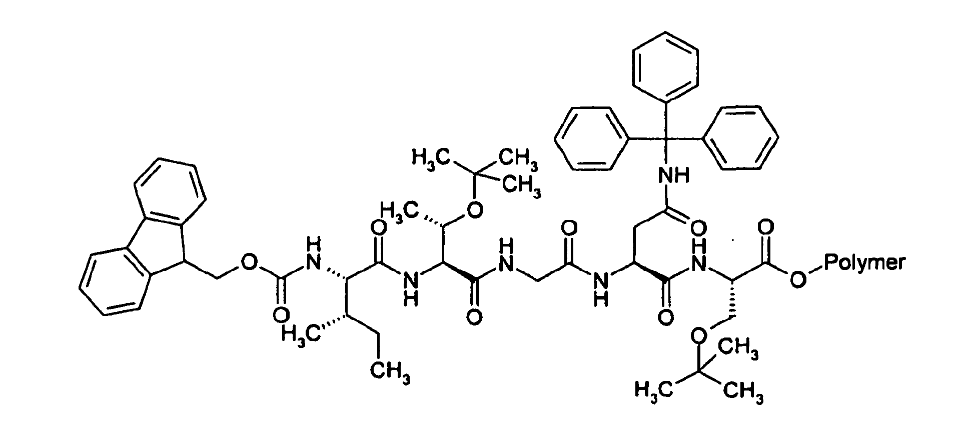

Die Fmoc-Schutzgruppe wird von dem Polymer (Beispiel 44A, 1000.0 mg, 1.49 mmol), wie in Arbeitsvorschrift 7 beschrieben ist, abgespalten. Anschließend wird das am Harz gebundene entschützte Tetrapeptid mit *N*-[(9*H*-Fluoren-9-ylmethoxy)carbonyl]-L-isoleucin (1053.2 mg, 2.98 mmol, 2 Äq.), DIEA (772 µl, 577.7 mg, 4.47 mmol, 3 Äq.) und TBTU (956.6 mg, 2.98 mmol, 2 Äq.) über Nacht zum F-moc geschützten Pentapeptid umgesetzt. Analog der Arbeitsvorschrift 7 erfolgt die Aufarbeitung des Polymeres. Es wird das korrespondierende seitenkettengeschützte Pentapeptid nach der Probeabspaltung bestätigt.
HPLC (Methode 13) Rₜ = 2.57 min.
LC-MS (Methode 21): Rₜ = 3.23 min; MS (ESIpos): *m*/*z* (%) = 1067 (13) [M+H]⁺, 243 (100).
HR-TOF-MS (Methode 24): C₆₁H₇₅N₆O₁₁ [M+H]⁺ gef. 1067.5488, ber. 1067.5489.

### Beispiel 46A

### N⁵-[(Benzyloxy)carbonyl]-{N⁵-[{[(benzyloxy)carbonyl]amino}(imino)methyl]-N²-[(9H-fluoren-9-ylmethoxy)carbonyl]-D-ornithin}

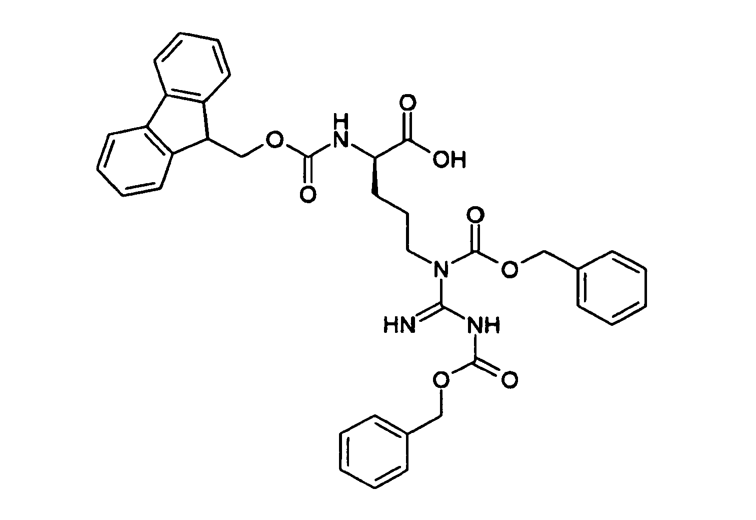

*N*⁵-[(Benzyloxy)carbonyl]-*N*⁵-[{[(benzyloxy)carbonyl]amino}(imino)methyl]-*N*²-(*tert-*butoxycarbonyl)-D-ornithin (4.90 g, 9.03 mmol) [M. Jetten et al., Tetrahedron Lett. 1991, 32, 6025-6028] wird über Nacht bei RT in 4 M Salzsäure in Dioxan (150 ml) und Dichlormethan (150 ml) gerührt. Das Lösungsmittel wird vollständig abgedampft und der Rückstand im Vakuum getrocknet. Anschließend wird das entschützte Amin in Dichlormethan (180 ml) vorgelegt, mit DIEA (4.5 ml, 3.5 g, 27.09 mmol, 3 Äq.) und Chlortrimethylsilan (2.3 ml, 2.0 g, 18.061 mmol, 2 Äq.) versetzt und über Nacht unter Rückfluß gerührt. Zur abgekühlten Lösung (0°C) wird DIEA (3.0 ml, 2.3 g, 18.06 mmol, 2 Äq.) und Chlorameisensäure-(9-fluorenylmethyl)-ester (2.3 g, 9.03 mmol, 1 Äq.), auf RT erwärmt und bei dieser Temperatur über Nacht nachgerührt. Zur Aufarbeitung wird die Reaktion mit Dichlormethan verdünnt, mit 10%iger wässr. Zitronensäurelösung gewaschen, die organische Phase über Natriumsulfat getrocknet, das Lösungsmittel am Rotationsverdampfer entfernt und im Vakuum getrocknet. Nach der Feinreinigung (Methode 37) des Rohproduktes wird die Titelverbindung mit 2.0 g (32.4% d. Th.) isoliert.
LC-MS (Methode 21): Rₜ = 2.99 min; MS (ESIpos): *m*/*z* (%) = 665 (100) [M+H]⁺ ;MS (ESIneg): *m*/*z* (%) = 663 (30) [M - H]⁻, 333 (100).

### Beispiel 47A

### Chlortrityl-Harz gebundenes [N⁵-(Benzyloxycarbonyl)-N⁵-({[benzyloxycarbonyl]amino}{imino}methyl)-N²-({9H-fluoren-9-ylmethoxy}carbonyl)-D-ornithyl]-L-isoleucyl-[O³-(tert-butyl)-L-allothreonyl]-glycyl-(N³-trityl-L-asparaginyl)-[O³-(tert-butyl)-L-serin]

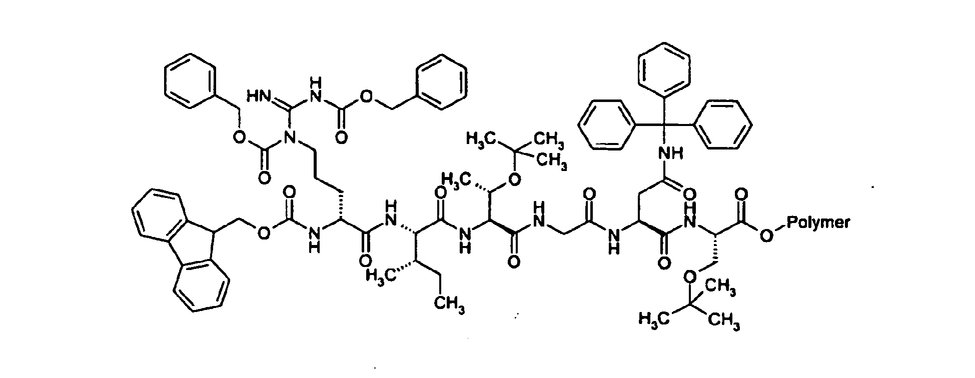

Die Fmoc-Schutzgruppe wird von dem Polymer (Beispiel 45A, 1000.0 mg, 1.49 mmol), wie in Arbeitsvorschrift 7 beschrieben ist, abgespalten. Anschließend wird das am Harz gebundene entschützte Pentapeptid mit der Fmoc-geschützten Aminosäure (Beispiel 46A, 1980.9 mg, 2.98 mmol, 2 Äq.), DIEA (772 µl, 577.7 mg, 4.47 mmol, 3 Äq.) und TBTU (956.6 mg, 2.98 mmol, 2 Äq.) über Nacht zum F-mocgeschützten Hexapeptid umgesetzt. Analog der Arbeitsvorschrift 7 erfolgt die Aufarbeitung des Polymeres. Das polymergebundene Hexapeptid wird ohne Bestätigung der Analytik gleich weiter verarbeitet.

### Beispiel 48A

### Chlortrityl-Harz gebundenes {N²-[(9H-Fluoren-9-ylmethoxy)carbonyl]-L-leucyl)-[N⁵-(Benzyloxycarbonyl)-N⁵-({[benzyloxycarbonyl]amino}{imino}methyl)-D-ornithyl]-L-isoleucyl-[O³-(tert-butyl)-L-allothreonyl]-glycyl-(N³-trityl-L-asparaginyl)-[O³-(tert-butyl)-L-serin]

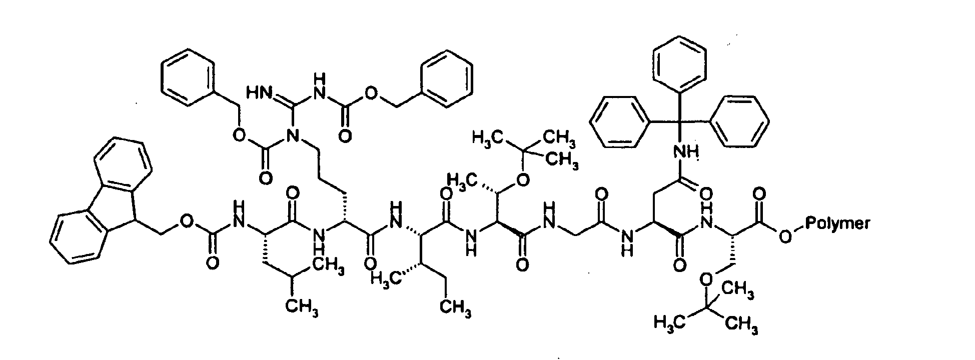

Die Fmoc-Schutzgruppe wird von dem Polymer (Beispiel 47A, 1000.0 mg, 1.49 mmol), wie in Arbeitsvorschrift 7 beschrieben ist, abgespalten. Anschließend wird das am Harz gebundene entschützte Hexapeptid mit *N*-[(9*H*-Fluoren-9-ylmethoxy)carbonyl]-L-leucin (1053.2 mg, 2.98 mmol, 2 Äq.), DIEA (779 µl, 577.7 mg, 4.47 mmol, 3 Äq.) und TBTU (956.7 mg, 2.98 mmol, 2 Äq.) über Nacht zum F-moc geschützten Heptapeptid umgesetzt. Analog der Arbeitsvorschrift 7 erfolgt die Aufarbeitung des Polymeres. Es wird das korrespondierende seitenkettengeschützte Heptapeptid nach der Probeabspaltung bestätigt.
LC-MS (Methode 18): Rₜ = 3.51 min; MS (ESIpos): *m*/*z* (%) = 1605 (100) [M+H]⁺.

### Beispiel 49A

### {N²-[tert-Butoxycarbonyl]-[(3R)-3-hydroxy-L-leucyl]}-L-leucyl}-[N⁵-(Benzyloxycarbonyl)-N⁵-({[benzyloxycarbonyl]amino}{imino}methyl)-D-ornithyl]-L-isoleucyl-[O³-(tert-butyl)-L-allothreonyl]-glycyl-(N³-trityl-L-asparaginyl)-[O³-(tert-butyl)-L-serin]

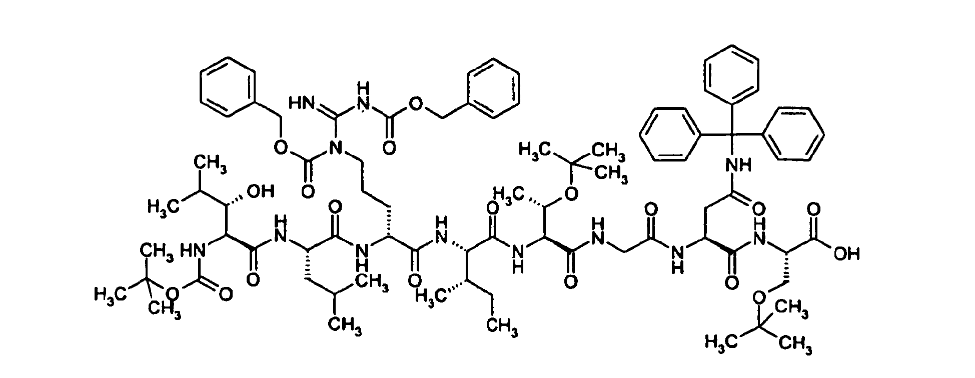

Die Fmoc-Schutzgruppe wird von dem Polymer (Beispiel 48A, 1000.0 mg, 1.49 mmol), wie in Arbeitsvorschrift 7 beschrieben ist, abgespalten. Anschließend wird das am Harz gebundene entschützte Heptapeptid mit (3*R*)-3-Hydroxy-*N*-(*tert-*butoxycarbonyl)-L-leucin (552.7 mg, 2.24 mmol, 1.5 Äq.), DIEA (597 µl, 442.9 mg, 3.43 mmol, 2.3 Äq.) und TBTU (717.6 mg, 2.24 mmol, 1.5 Äq.) über Nacht zum F-moc geschützten Octapeptid umgesetzt. Analog der Arbeitsvorschrift 7 erfolgt die Aufarbeitung des Polymeres.

Das Octapeptid wird vollständig vom Polymer in einer Lösung aus Essigsäure, Trifluorethanol und Dichlormethan (1:1:3) abgespalten. Zur Aufarbeitung wird das Harz über eine Fritte filtriert, das Filtrat im Vakuum aufkonzentriert und letztlich chromatographisch (Methode 32) aufgereinigt. Die Titelverbindung wird mit 56 mg (16% d. Th.) erhalten.
LC-MS (Methode 18): Rₜ = 3.48 min; MS (ESIpos): *m*/*z* (%) = 1613 (15) [M+H]⁺, 806 (60) [M + 2H]²⁺, 243 (100); MS (ESIneg): *m*/*z* (%) = 1611 (100) [M+H]⁺.

### Beispiel 50A

### [(3R)-3-Hydroxy-L-leucyl]-L-leucyl-{N⁵-[benzyloxycarbonyl]-N⁵-[{[benzyloxycarbonyl]-amino}(imino)methyl]-D-ornithyl}-L-isoleucyl-L-allothreonyl-glycyl-L-asparaginyl-L-serin

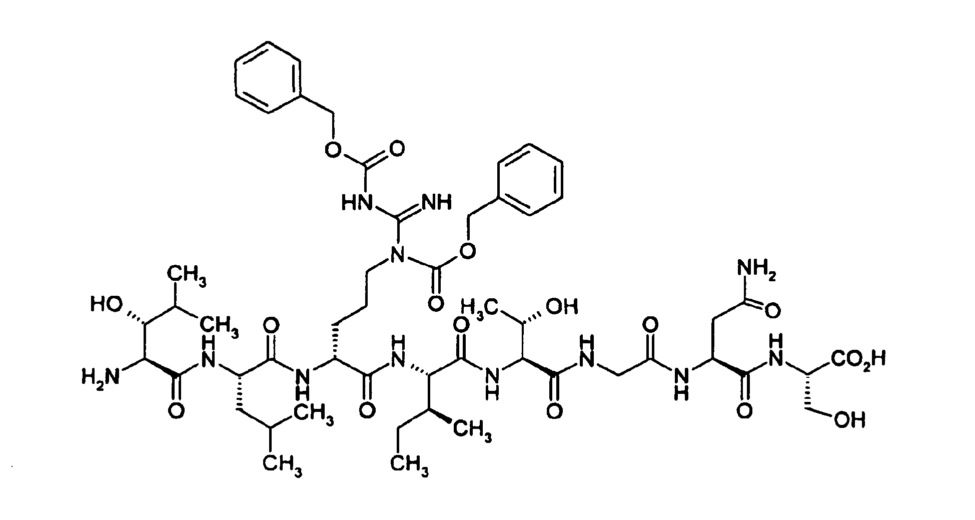

Das Peptid (Beispiel 49A, 22.0 mg, 13.65 µmol) wird in TFA (5.25 ml) und Wasser (0.25 ml) vorgelegt, anschließend mit *p*-Kresol (10.0 mg, 92.48 µmol) versetzt und die Lösung wird 1 h bei RT gerührt. Zur Aufarbeitung wird das Lösungsmittel am Rotationsverdampfer bei 30°C Badtemperatur entfernt. Nach der Feinreinigung des Rückstandes über die präparative HPLC (Methode 26) erhält man das Produkt mit 4.4 mg (27.9% d. Th.).
HPLC (Methode 1) Rₜ = 2.07 min
LC-MS (Methode 18): Rₜ = 2.12 min; MS (ESIpos): *m*/*z* (%) = 1158 (5) [M+H]⁺, 579 (100) [M + 2H]²⁺; MS (ESIneg.): *m*/*z* (%) = 1156 (100) [M - H]⁻.

### Beispiel 51A

### [N²-(Benzyloxycarbonyl)-3-tert-butyl-D-alanyl]-[3-tert-butyl-L-alanyl]-{(3R)-3-[(tert-butoxycarbonyl)amino]-L-phenylalanyl}-{(3R)-3-hydroxy-L-leucyl}-L-leucyl-{N⁵-[benzyloxycarbonyl]-N⁵-[{[benzyloxycarbonyl]amino}(imino)methyl]-D-ornithyl}-L-isoleucyl-L-allothreonyl-glycyl-L-asparaginyl-L-serin

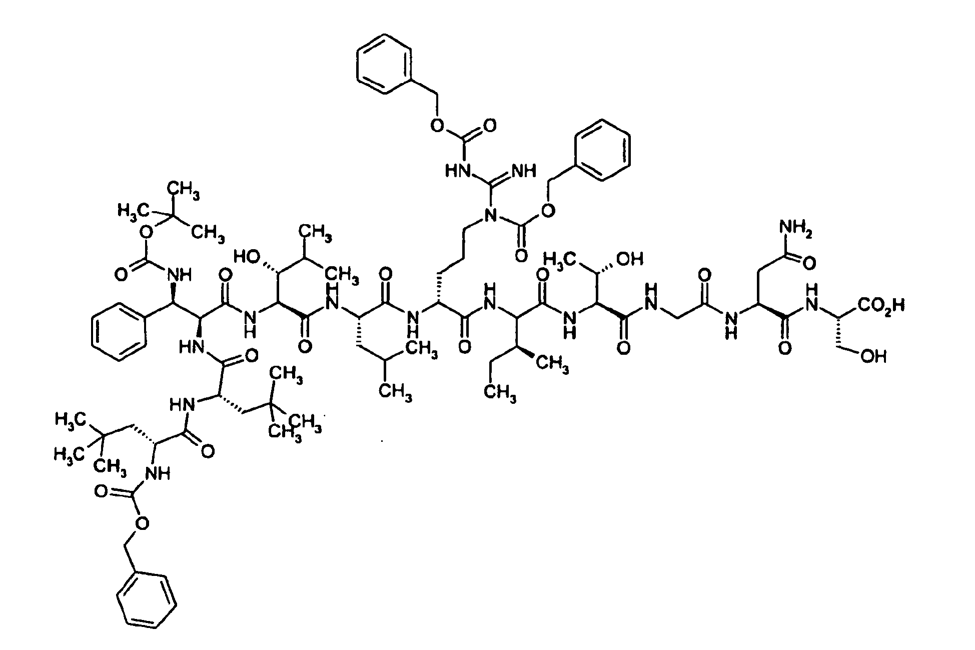

Das Amin (Beispiel 50A, 4.0 mg, 3.46 µmol) und die aktivierte Säure (Beispiel 40A, 3.2 mg, 3.82 µmol, 1.1 Äq.) werden in DMF (500 µl) vorgelegt, die Lösung auf 0°C gekühlt, abschließend mit DIEA (4 µl, 2.7 mg, 20.74 mmol, 6 Äq.) versetzt, die Reaktion auf RT erwärmt und bei dieser über Nacht gerührt. Zur Aufarbeitung wird das Lösungsmittel am Rotationsverdampfer bei 30°C Badtemperatur entfernt, der Rückstand gelchromatographisch, (Methode 45, Eluent: Methanol) vorgereinigt und nach der Feinreinigung über die präparative HPLC (Methode 26) wird das Produkt mit 3.0 mg (24.0% d. Th.) erhalten.
HPLC (Methode 10) Rₜ = 11.37 min
LC-MS (Methode 18): Rₜ = 3.37 min; MS (ESIpos): *m*/*z* (%) = 904 (100) [M + 2H]²⁺.

### Beispiel 52A

[*N*²-(Benzyloxycarbonyl)-3-*tert*-butyl-D-alanyl]-[3-*tert*-butyl-L-alanyl]-{(3*R*)-3-amino-L-phenylalanyl}-{(3*R*)-3-hydroxy-L-leucyl)-L-leucyl-{*N*⁵-[benzyloxycarbonyl]-*N*⁵-[{[benzyloxycarbonyl]aminol(imino)methyl]-D-ornithyl}-L-isoleucyl-L-allothreonyl-glycyl-L-asparaginyl-L-serin-trifluoracetat

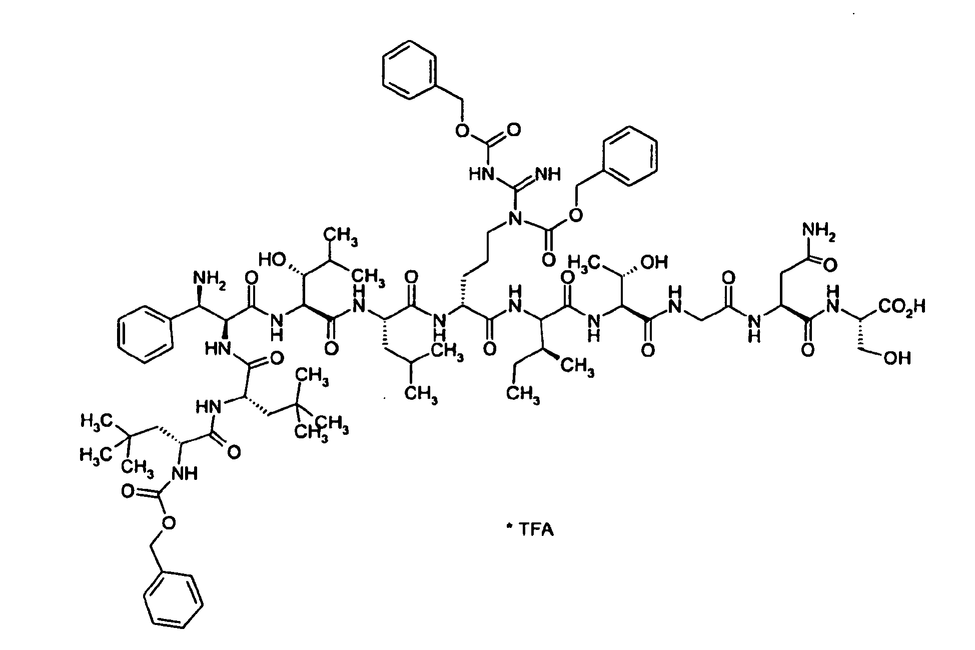

Die Boc-Schutzgruppe von der Beispielverbindung 51A (2.5 mg, 1.38 µmol) wird nach der allgemeinen Arbeitsvorschrift 1 abgespalten. Nach der Feinreinigung über die präparative HPLC (Methode 26) wird das Produkt mit 2.2 mg (87.3% d. Th.) erhalten.
HPLC (Methode 9) Rₜ = 24.14 min
LC-MS (Methode 18): Rₜ = 2.63 min; MS (ESIpos): *m*/*z* (%) = 854 (100) [M + 2H]²⁺; MS (ESIneg.): *m*/*z* (%) = 1706 (100) [M - H]⁻.
HR-TOF-MS (Methode 24): C₈₄H₁₂₃N₁₆O₂₂ [M+H]⁺ gef. 1707.8989, ber. 1707.8993.

### Beispiel 53A

### [N²-(Benzyloxycarbonyl)-3-tert-butyl-D-alanyl]-[3-tert-butyl-L-alanyl]-{(3R)-3-amino-L-phenylalanyl}-{(3R)-3-hydroxy-L-leucyl}-L-leucyl-{N⁵-[benzyloxycarbonyl]-N⁵-[{[benzyloxycarbonyl]amino}(imino)methyl]-D-ornithyl}-L-isoleucyl-L-allothreonyl-glycyl-L-asparaginyl-L-serin-trifluoracetat-C^{1.11}-N^{3.3}-lactam

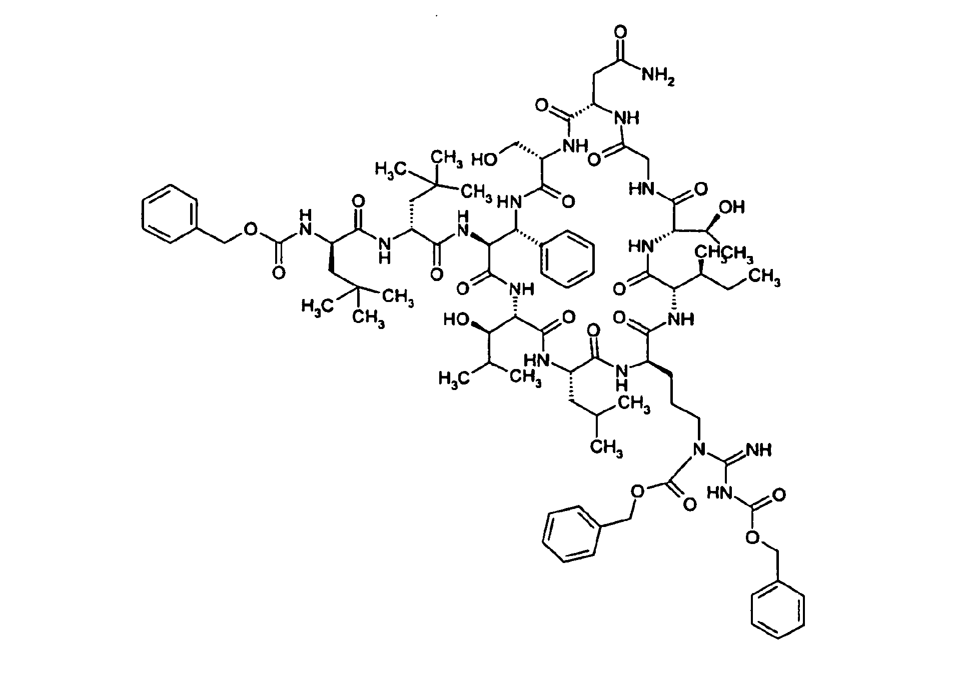

Unter Argonschutzgasatmosphäre wird das entschützte Amin (Beispiel 52A, 2.0 mg, 1.10 µmol) in DMF (1 ml) vorgelegt, die Lösung auf 0°C gekühlt, mit HATU (1.3 mg, 3.29 µmol, 3 Äq.) und NMM (0.7 µl, 0.7 mg, 6.58 µmol, 6 Äq.) versetzt, die Reaktion langsam auf RT gebracht und bei dieser Temperatur über Nacht nachgerührt. Zur Aufarbeitung wird die Lösung über die präparative HPLC (Methode 26) gereinigt. Man erhält 1.9 mg (90.1% d. Th.) von der Zielverbindung.
HPLC (Methode 10) Rₜ = 10.70 min.
LC-MS (Methode 18): Rₜ = 3.25 min; MS (ESIpos): *m*/*z* (%) = 1691 (53) [M+H]⁺, 846 (100) [M + 2H]²⁺; MS (ESIneg.): *m*/*z* (%) = 1688 (100) [M - H]⁻.
HR-TOF-MS (Methode 24): C₈₄H₁₂₁N₁₆O₂₁ [M+H]⁺ gef. 1689.8929, ber. 1689.8887.

### Beispiel 54A

### {(3R)-N²-(tert-Butoxycarbonyl)-3-hydroxy-L-leucyl}-L-leucyl-D-arginyl-L-isoleucyl-{O³-(tert-butyl)-L-allothreonyl}-glycyl-{N⁴-trityl-L-asparaginyl}-[O³-(tert-butyl)-L-serin]

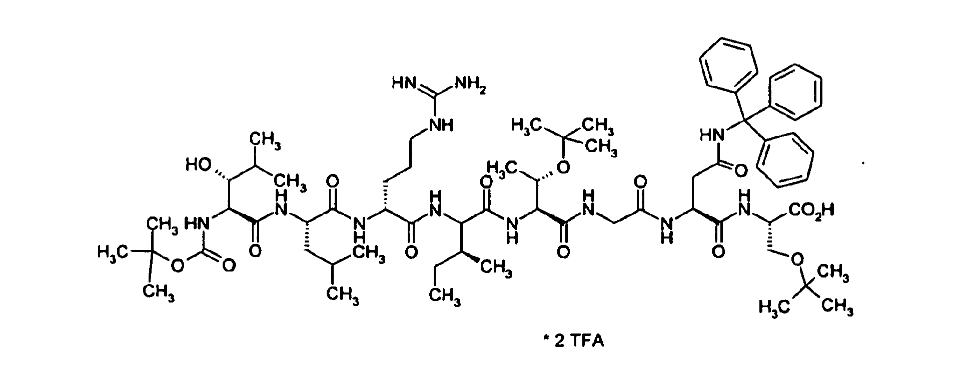

Die Benzylester-geschützte Verbindung (Beispiel 49A, 62.0 mg, 38.46 µmol) wird in Methanol (20 ml) hydrogenolytisch innerhalb 1 h zum Produkt umgesetzt. Nach dem Chromatographieren über die präparative HPLC (Methode 26) erhält man die Zielverbindung (34.3 mg, 61.2% d. Th.).
HPLC (Methode 1) Rₜ = 2.51 min.
LC-MS (Methode 20): Rₜ = 2.36 min; MS (ESIpos): *m*/*z* (%) = 1343 (38) [M+H]⁺, 243 (100); MS (ESIneg.): *m*/*z* (%) = 1341 (100) [M - H]⁻.

### Beispiel 55A

### [(3R)-3-hydroxy-L-leucyl]-L-leucyl-D-arginyl-L-isoleucyl-L-allothreonyl-glycyl-L-asparaginyl-L-serin-bistrifluoracetat

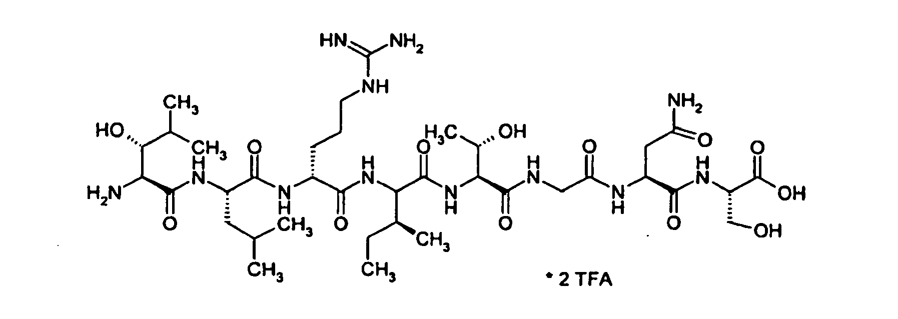

Die Beispielverbindung 54A (32.0 mg, 21.95 µmol) wird in einer Lösung aus TFA (7.3 ml), Wasser (250 µl) und Triisopropylsilan (323 µl) bei RT 2 h gerührt. Zur Aufarbeitung wird das Lösungsmittel am Rotationsverdampfer bei 30°C Badtemperatur entfernt und der Rückstand einmal mit Toluol und einmal mit Methylenchlorid bei 30°C Badtemperatur im Vakuum eingeengt. Nach dem Trocknen im Hochvakuum wird das Rohprodukt in 0.1%iger wässr. TFA gelöst, mit MTBE extrahiert, die abgetrennte wässr. Phase im Vakuum aufkonzentriert und anschließend über die präparative HPLC (Methode 27) feingereinigt. Man erhält 22.5 mg (91.8% d. Th.) der Titelverbindung.
HPLC (Methode 9) Rₜ = 8.38 min.
LC-MS (Methode 22): Rₜ = 2.30 min; MS (ESIpos): *m*/*z* (%) = 889 (12) [M+H]⁺, 445 (100) [M + 2H]²⁺; MS (ESIneg.): *m*/*z* (%) = 887 (100) [M - H]⁻.
HR-TOF-MS (Methode 24): C₃₇H₆₉N₁₂O₁₃ [M+H]⁺ gef. 889.5102, ber. 889.5102.

### Beispiel 56A

### [N²-(Benzyloxycarbonyl)-3-tert-butyl-D-alanyl]-[3-tert-butyl-L-alanyl]-{(3R)-3-[(tert-butoxycarbonyl)amino]-L-phenylalanyl}-{(3R)-3-hydroxy-L-leucyl}-L-leucyl-D-arginyl-L-isoleucyl-L-allothreonyl-glycyl-L-asparaginyl-L-serin-trifluoracetat

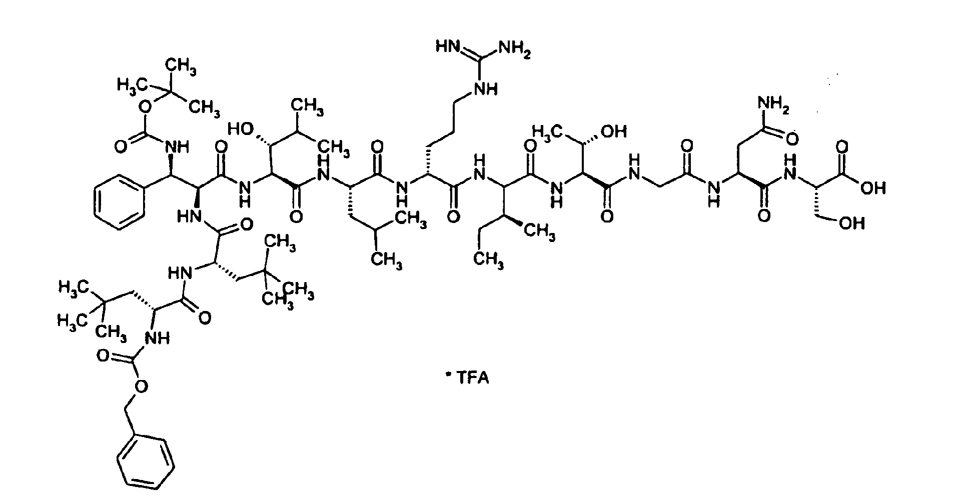

Die Beispielverbindung 55A (19.0 mg, 17.00 µmol) und 40A (15.6 mg, 18.70 µmol) werden in DMF (1 ml) vorgelegt, mit *N,N*-Diisopropylamin (18 µl, 13.2 mg, 102.00 mmol, 6 Äq.) bei 0°C versetzt und bei dieser Temperatur 2 h nachgerührt. Nach dem Erwärmen der Reaktion auf RT wird diese über Nacht nachgerührt. Zur Aufarbeitung wird die Lösung im Vakuum aufkonzentriert und abschließend über die präparative HPLC (Methode 26) feingereinigt. Man erhält 24.4 mg (86.7% d. Th.) der Zielverbindung.
HPLC (Methode 9) Rₜ = 23.17 min.
LC-MS (Methode 18): Rₜ = 2.37 min; MS (ESIpos): *m*/*z* (%) = 720 (100) [M - C₄H₈ - CO₂ + 2H]²⁺; MS (ESIneg.): *m*/*z* (%) = 1438 (100) [M - C₄H₈ - CO₂ - H]⁻.
HR-TOF-MS (Methode 24): C₇₃H₁₁₉N₁₆O₂₀ [M+H]⁺ gef. 1539.8772, ber. 1539.8782.

### Beispiel 57A

### [N²-(Benzyloxycarbonyl)-3-tert-butyl-D-alanyl]-[3-tert-butyl-L-alanyl]-{(3R)-3-amino-L-phenylalanyl}-{(3R)-3-hydroxy-L-leucyl}-L-leucyl-D-arginyl-L-isoleucyl-L-allothreonyl-glycyl-L-asparaginyl-L-serin-bistrifluoracetat

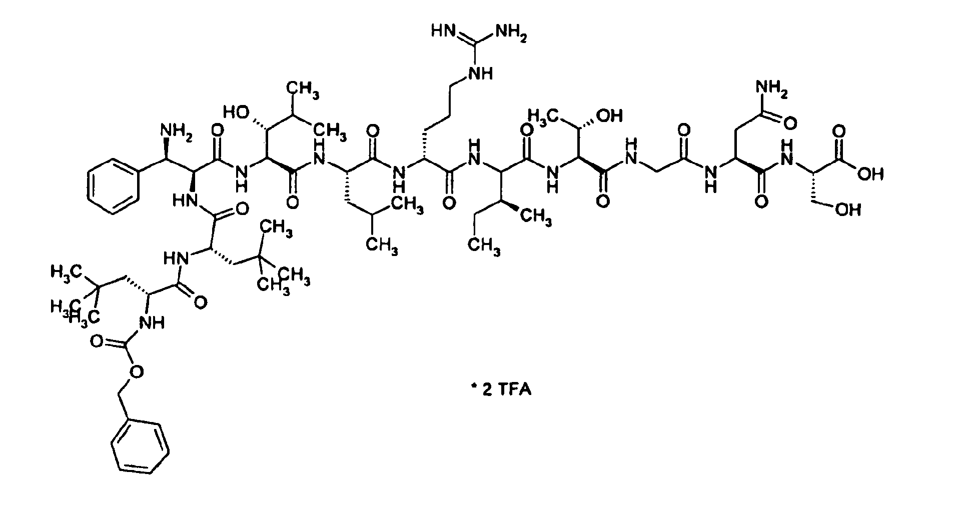

Gemäß der Arbeitsvorschrift 1 wird die Beispielverbindung 56A (23.0 mg, 13.91 µmol) zum entschützten Amin umgesetzt. Nach der Feinreinigung (Methode 26) erhält man 19.9 mg (85.8% d. Th.) der Titelverbindung.
HPLC (Methode 9) Rₜ = 17.05 min.
LC-MS (Methode 18): Rₜ = 1.98 min; MS (ESIpos): *m*/*z* (%) = 1441 (3) [M+H]⁺, 720 (100) [M + 2H]²⁺; MS (ESIneg.): *m*/*z* (%) = 1438 (100) [M - H]⁻.
HR-TOF-MS (Methode 24): C₆₈H₁₁₁N₁₆O₁₈ [M+H]⁺ gef. 1439.8271, ber. 1439.8257.

### Beispiel 58A

### [N²-(Benzyloxycarbonyl)-3-tert-butyl-D-alanyl]-[3-tert-butyl-L-alanyl]-{(3R)-3-amino-L-phenylalanyl}-{(3R)-3-hydroxy-L-leucyl}-L-leucyl-D-arginyl-L-isoleucyl-L-allothreonyl-glycyl-L-asparaginyl-L-serin-C^{1.11}-N^{3.3}-lactam-trifluoracetat

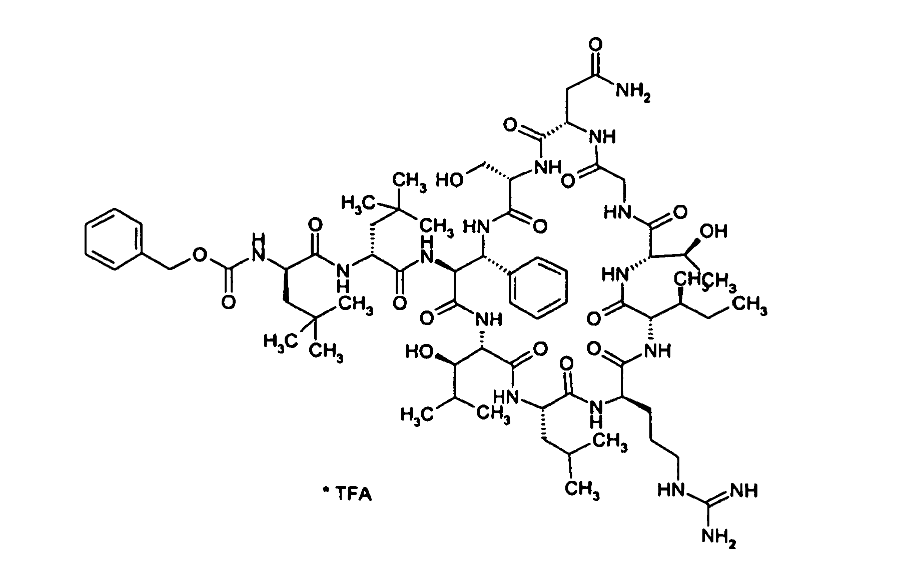

Unter Argonschutzgasatmosphäre wird das entschützte Amin (Beispiel 57A, 19.0 mg, 11.39 µmol) in DMF (10 ml) vorgelegt, die Lösung auf 0°C gekühlt, mit HATU (13.0 mg, 34.18 µmol, 3 Äq.) und NMM (8 µl, 6.9 mg, 68.35 µmol) versetzt, die Reaktion bei 4°C über 3 Tage stehen lassen. Zur Aufarbeitung wird der Ansatz bei 0°C mit Methanol versetzt, bei max. 30°C Badtemperatur im Vakuum aufkonzentriert und letztlich über die präparative HPLC (Methode 26) feingereinigt. Von der Titelverbindung wird 14.0 mg (80.0% d. Th.) erhalten.
HPLC (Methode 9) Rₜ = 22.44 min.
LC-MS (Methode 18): Rₜ = 2.30 min; MS (ESIpos): *m*/*z* (%) = 1422 (12) [M+H]⁺, 711 (100) [M + 2H]²⁺; MS (ESIneg.): *m*/*z* (%) = 1420 (28) [M - H]⁻, 710 (100) [M - 2H]²⁻.
HR-TOF-MS (Methode 24): C₆₈H₁₀₉N₁₆O₁₇ [M+H]⁺ gef. 1421.8165, ber. 1421.8152.

### Beispiel 59A

### (3R)-3-[(tert-Butyloxycarbonyl)amino]-L-phenylalanin

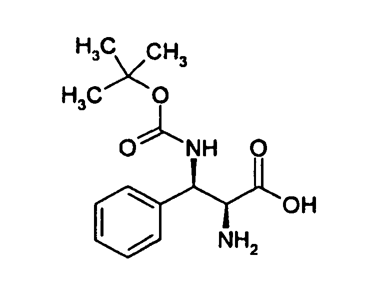

Gemäß der Arbeitvorschrift 5 wird die Beispielverbindung 16A (1000.0 mg, 2.41 mmol) mit 10%iger Palladiumkohle (100 mg, 93.98 µmol, 4 mol%) in Methanol (100 ml) innerhalb 1 h zur Titelverbindung umgesetzt. Nach dem Abfiltrieren des Katalysators über Kieselgur und Einengen werden 624.0 mg (92.3%) Rohprodukt erhalten.
HPLC (Methode 9) Rₜ = 10.27 min.
LC-MS (Methode 18): Rₜ = 1.52 min; MS (ESIpos.): *m*/*z* (%) = 281 (82) [M+H]⁺, 561 (10) [2M+H]⁺, 225 (100); MS (ESIneg.): *m*/*z* (%) = 279 (70) [M - H]⁻, 559 (5) [2M - H]⁻, 205 (100).
¹H NMR (500 MHz, *d*₆-DMSO): δ = 1.34 (s, 9H, OC(CH₃)₃), 3.51 (d, *J* = 4.8 Hz, 1H, H^{α}), 4.88 (dd, *J* = 8.6 Hz, 4.8Hz, 1H, H^{β}), 7.21-7.35 (m, 7H, ArH, NH₂), 8.04 (d, *J* = 8.6 Hz, NH).
¹³C NMR (126 MHz, *d₆*-DMSO): δ = 28.04 (3C), 53.81, 56.27, 78.06, 127.24, 127.42 (2C), 128.07 (2C), 138.99, 154.12, 167.93.
HR-TOF-MS (Methode 24): C₁₄H₂₁N₂O₄ [M+H]⁺ gef. 281.1506, ber. 281.1496.

### Beispiel 60A

### Methyl [N²-(Benzyloxycarbonyl)-D-leucyl]-L-leucinat

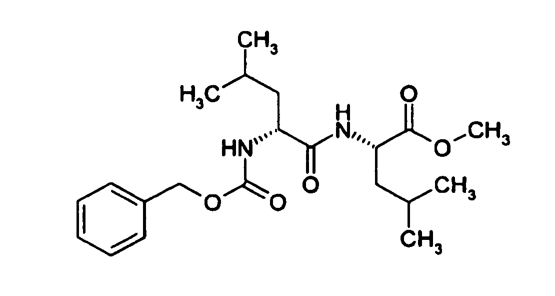

*N*²-(Benzyloxycarbonyl)-D-Leucin (BACHEM Cat No z13351.) (6.37 g, 24 mmol)) und Methyl L-Leucinat (3.49 g, 24 mmol, 1 Äq.) werden bei 0°C in DMF (75 ml) gelöst, dann werden NMM (5.28 ml, 48 mmol, 2 Äq.) und HATU (13.69 g, 36 mmol, 1.5 Äq.) zugesetzt. Der Ansatz wird drei Stunden bei Raumtemperatur gerührt. Man versetzt mit MTBE und gesättigter Natriumhydrogencarbonatlösung, und extrahiert. Die wässrige Phase wird mit einer zweiten Portion MTBE nachextrahiert, dann werden die vereinigten organischen Phasen mit 1 M Zitronensäure sowie abermals mit gesättigter Natriumhydrogencarbonatlösung gewaschen, über Natriumsulfat getrocknet, filtriert und im Vakuum eingeengt. Der Rückstand wird in zwei Portionen chromatographisch gereinigt (Biotage 40M, Cyclohexan/Ethylacetat 3+1). Ausbeute: 7.85 g (80% d. Th.).
HPLC (Methode 5): Rₜ = 4.82 min.
LCMS (Methode 18): Rₜ = 2.65 min; MS (ESIpos.): *m*/*z* (%) = 393 (100) [M+H]⁺.
[α]²⁰_{Na} = -5.2 ° (*c* = 0.52, MeOH).
¹H NMR (400 MHz, *d₆*-DMSO) δ (ppm) = 0.77-0.92 (m, 12H), 1.31-1.66 (m, 6H), 3.60 (s, 3H), 4.10 (m, 1H), 4.28 (m, 1H), 5.02 (s, 2H), 7.25-7.38 (m, 6H), 8.23 (d, 1H).
¹³C-NMR (126 MHz, *d₆*-DMSO) δ (ppm) = 21.1 (CH₃), 21.5 (CH₃), 22.8 (CH₃), 22.9 (CH₃), 24.2 (CH), 41.0 (CH₂), 50.0 (CH), 51.8 (CH₃, OCH₃), 52.9 (CH), 65.3 (CH₂, OCH₂Ph), 127.6 (CH, ar-C), 127.7 (CH, ar-C), 128.3 (CH, ar-C), 137.1 ((C quat, ar-C), 155.8 (C quat, NCOC(CH₃)₃), 172.4 (C quat, C=O), 172.9 (C quat, C=O).

### Beispiel 61A

### [N²-(Benzyloxycarbonyl)-D-leucyl]-L-leucin

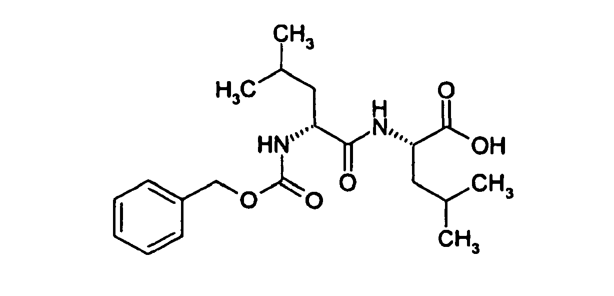

Verbindung 60A (7.70 g, 19.62 mmol) wird in 200 ml THF/ Wasser (3+1) aufgenommen, auf 0°C gekühlt und mit Lithiumhydroxid-Monohydrat (1.65 g, 39.24 mmol, 2 Äq.) versetzt. Man lässt bei 0°C rühren, bis nach HPLC- Kontrolle die Umsetzung vollständig abgelaufen ist (ca. 45 min). Man destilliert den Großteil des THF im Vakuum ab, stellt dann durch Zusatz von Zitronensäure auf ca. pH 4 und extrahiert mit 2 Portionen Ethylacetat. Die vereinigten org. Phasen werden über Natriumsulfat getrocknet, filtriert und eingeengt. Das Produkt wird als amorphe Substanz in einer Ausbeute von 6.87 g (89% d. Th.) erhalten.
HPLC (Methode 5): Rₜ = 4.45 min.
LCMS (Methode 18): Rₜ = 2.39 min, MS (ESIpos.) *m*/*z* (%) = 379 (100) [M+H]⁺, 757 (40) [2M+H]⁺.
[α]²⁰_{Na} = +4.7 ° (*c* = 0.50, MeOH).
¹H NMR (300 MHz,*d₆*-DMSO) δ (ppm) = 0.77-0.92 (m, 12H), 1.34-1.68 (m, 6H), 4.04-4.26 (m, 2H), 5.02 (s, 2H), 7.25-7.38 (m, 6H), 8.12 (d, 1H), 12.50 (br. s, 1H).
HR-TOF-MS (Methode 24): C₂₀H₃₁N₂O₅ [M+H]⁺ ber. 379.2228 , gef. 379.2216.

### Beispiel 62A

### Pentafluorophenyl N-[benzyloxycarbonyl]-D-leucyl-L-leucinat

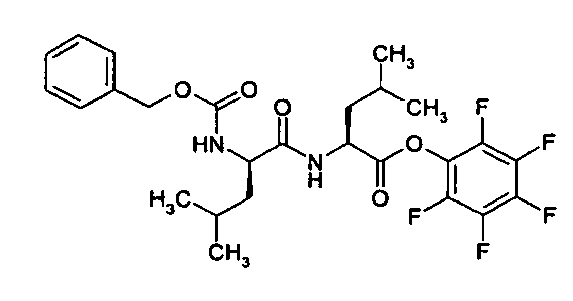

Gemäß der Herstellungsvorschrift (Beispiel 17A) wird die Beispielverbindung 61A (1000.0 mg, 2.64 mmol), Pentafluorphenol (2431.7 mg, 13.21 mmol, 5 Äq.) und EDC (759.8 mg, 3.96 mmol, 1.5 Äq.) zur Titelverbindung umgesetzt. Nach der Feinreinigung (Methode 28) wird das Produkt mit 1.3 g (93.0% d. Th.) isoliert und wird bei -25°C bis zur weiteren Verwendung gelagert.
HPLC (Methode 1) Rₜ = 3.08 min.
LC-MS (Methode 18): Rₜ = 3.26 min; MS (ESIpos.): *m*/*z* (%) = 545 (100) [M+H]⁺, 1089 (43) [2M+H]⁺.
¹H NMR (400 MHz, d₆-DMSO): δ = 0.78-0.99 (m, 12H, 2[CH(CH₃)₂]), 1.30-1.88 (m, 6H), 4.16 (m, 1H, H^{α}), 4.38-4.55 (2m, 1H, H^{α}), 4.95-5.11 (m, 2H, PhCH₂), 7.21-7.38 (m, 5H, ArH), 7.41 (d, *J* = 7.4 Hz, 1H, NH), 8.69 (d, *J* = 7.0 Hz, 1H, NH).

### Beispiel 63A

### {N²-(Benzyloxycarbonyl)-D-leucyl}-L-leucyl-[(3R)-3-[(tert-butoxycarbonyl)amino]-L-phenylalanin]

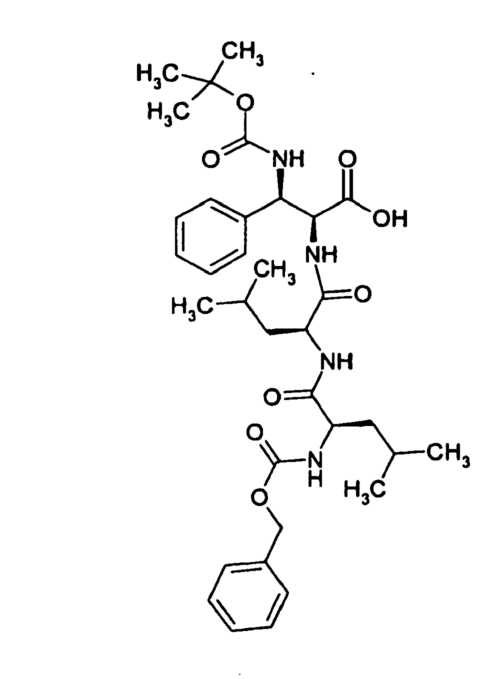

Unter Argonschutzgasatmosphäre werden die Aminosäure (Beispiel 59A, 493.0 mg, 1.76 mmol und das carbonsäureaktivierte Dipeptid (Beispiel 62A, 1197.0 mg, 1.76 mmol) in DMF (50 ml) vorgelegt und anschließend die Lösung mit DIEA (2.4 ml, 1818.4 mg, 14.07 mmol, 8 Äq.) bei 0°C versetzt. Die Reaktion wird nach Zugabe der Base langsam auf RT gebracht und bei dieser bis zum vollständigen Umsatz (3 h) gerührt. Zur Aufreinigung wird der Reaktion Kaliumdihydrogenphosphat (2.4 g, 17.59 mmol, 10 Äq.) zugegeben, 10 min nachgerührt, den Feststoff abfiltriert und das Filtrat im Vakuum bei 30°C Badtemperatur eingeengt. Die Titelverbindung wird nach der Feinreinigung (Methode 28) mit 750.0 mg (66.6% d. Th.) isoliert.
HPLC (Methode 1) Rₜ = 2.72 min.
LC-MS (Methode 18): Rₜ = 2.86 min; MS (ESIpos.): *m*/*z* (%) = 641 (100) [M+H]⁺, 541 (76) [M - C₄H₈ - CO₂ + H]⁺, 1281 (42) [2M+H]⁺; MS (ESIneg.): *m*/*z* (%) = 639 (100) [M - H]⁻, 1280 (92) [2M - H]⁻.

### Beispiel 64A

### Pentafluorphenyl N-[(benzyloxy)carbonyl]-D-leucyl-L-leucyl-[(3R)-3-[(tert-butoxycarbonyl)amino]-L-phenylalaninat]

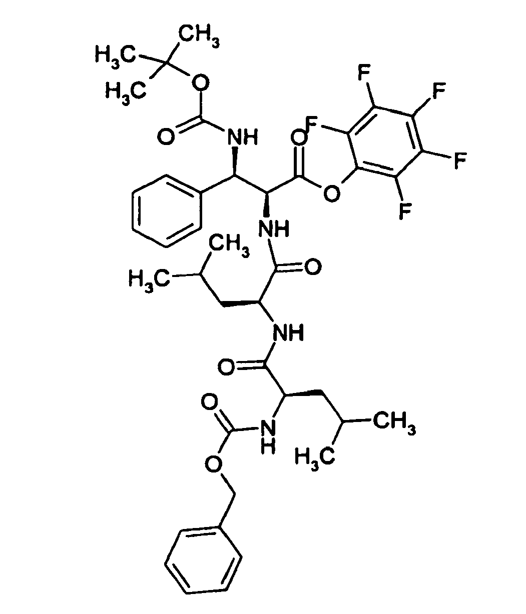

Die Titelverbindung wird gemäß der Beispielvorschrift (Beispiel 17A) aus dem Tripeptid (Beispiel 63A, 200 mg, 0.31 mmol) und Pentafluorphenol (287.3 mg, 1.56 mmol, 5 Äq.) in Dichlormethan (10 ml) unter Verwendung von EDC (89.8 mg, 0.49 mmol, 1.5 Äq.) dargestellt. Man erreicht einen vollständigen Umsatz, indem die Reaktion für 2 h bei 0°C gerührt und über Nacht bei 4°C im Kühlschrank stehen gelassen wird. Nach der Feinreinigung (Methode 32) werden 211.0 mg (83.8% d. Th.) Produkt erhalten.
HPLC (Methode 1) Rₜ = 3.23 min.
LC-MS (Methode 21): Rₜ = 3.22 min; MS (ESIpos.): *m*/*z* (%) = 807 (30) [M+H]⁺, 707 (100) [M - C₄H₈ - CO₂ + H]⁺; MS (ESIneg.): *m*/*z* (%) = 805 (10) [M - H]⁻, 183 (100).

### Beispiel 65A

### Chlortrityl-Harz gebundenes {O³-[tert-Butyl]-N²-[(9H-fluoren-9-ylmethoxy)carbonyl]-L-threonyl-glycyl-(N³-trityl-L-asparaginyl)-[O³-(tert-butyl)-L-serin]

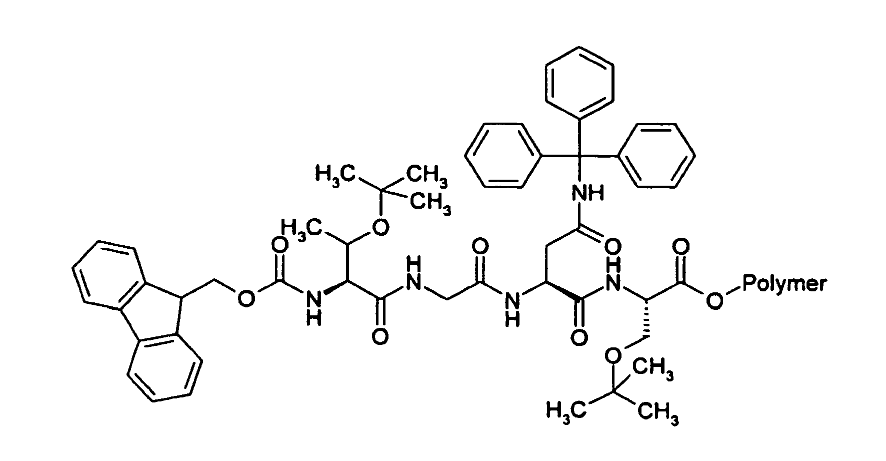

Die Fmoc- Schutzgruppe wird von dem Polymer (Beispiel 43A, 1000.0 mg, 1.49 mmol), wie in Arbeitsvorschrift 7 beschrieben ist, abgespalten. Anschließend wird das am Harz gebundene entschützte Tripeptid mit *O*-(*tert*-Butyl)-*N*-[(9*H*-fluoren-9-ylmethoxy)carbonyl]-L-threonin [Echner, Hartmut; Voelter, Wolfgang; Liebigs Ann. Chem.; GE; 1988; 1095-1098] (1184.5 mg, 2.98 mmol, 2 Äq.), DIEA (779 µl, 577.7 mg, 4.47 mmol, 3 Äq.) und TBTU (956.6 mg, 2.98 mmol, 2 Äq.) über Nacht zum F-moc geschützten Tetrapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
LC-MS (Methode 20): Rₜ = 3.07 min; MS (ESIpos): *m*/*z* (%) = 955 (80) [M+H]⁺, 243 (100); MS (ESIneg): *m*/*z* (%) = 953 (100) [M - H]⁻.
HR-TOF-MS (Methode 24): C₅₅H₆₄N₅O₁₀ [M+H]⁺ gef. 954.4636, ber. 954.4648.

### Beispiel 66A

### Chlortrityl-Harz gebundenes {N²-[(9H-Fluoren-9-ylmethoxy)carbonyl]-L-isoleucyl}-{[O³-(tert-butyl)]-L-threonyl}-glycyl-(N³-trityl-L-asparaginyl)-[O³-(tert-butyl)-L-serin]

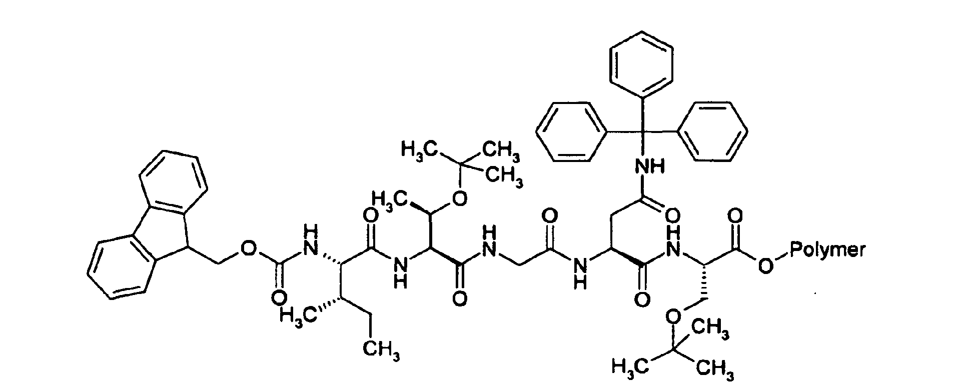

Die Fmoc-Schutzgruppe wird von dem Polymer (Beispiel 65A, 1000.0 mg, 1.49 mmol), wie in Arbeitsvorschrift 7 beschrieben ist, abgespalten. Anschließend wird das am Harz gebundene entschützte Tetrapeptid mit der *N*-[(9*H*-Fluoren-9-ylmethoxy)carbonyl]-L-isoleucin (1053.2 mg, 2.98 mmol, 2 Äq.) (H. Echner et al., Liebigs Ann. Chem. 1988, 1095-1098), DIEA (779 µl, 577.7 mg, 4.47 mmol, 3 Äq.) und TBTU (956.6 mg, 2.98 mmol, 2 Äq.) über Nacht zum F-moc geschützten Pentapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
HPLC (Methode 13) Rₜ = 2.61 min.
LC-MS (Methode 21): Rₜ = 3.26 min; MS (ESIpos): *m*/*z* (%) = 1068 (15) [M+H]⁺, 243 (100); MS (ESIneg): *m*/*z* (%) = 1066 (100) [M - H]⁻.
HR-TOF-MS (Methode 24): C₆₁H₇₅N₆O₁₁ [M+H]⁺ gef. 1067.5503, ber. 1067.5489.

### Beispiel 67A

### Chlortrityl-Harz gebundenes {N²-[(9H-Fluoren-9-ylmethoxy)carbonyl]-[N⁵-(imino{[(2,2,5,7,8-pentamethyl-3,4-dihydro-2H-chromen-6-yl)sulfonyl]amino}methyl)-D-ornithyl]}-L-isoteucyl-[O³-(tert-butyl)-L-threonyl]-glycyl-(N³-trityl-L-asparaginyl)-[O³-(tert-butyl)-L-serin]

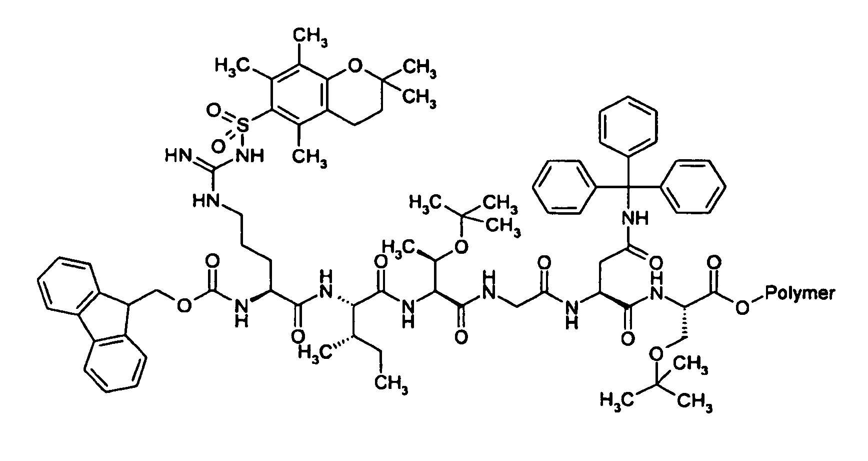

Die Fmoc-Schutzgruppe wird von dem Polymer (Beispiel 66A, 1000.0 mg, 1.49 mmol), wie in Arbeitsvorschrift 7 beschrieben ist, abgespalten. Anschließend wird das am Harz gebundene entschützte Pentapeptid mit *N*²-[(9*H*-Fluoren-9-ylmethoxy)carbonyl]-*N*⁵-(imino{[(2,2,5,7,8-pentamethyl-3,4-dihydro-2*H*-chromen-6-yl)sulfonyl]amino}methyl)-L-ornithin (1283.9 mg, 1.94 mmol, 1.3 Äq.), DIEA (779 µl, 577.7 mg, 4.47 mmol, 3 Äq.) und TBTU (956.6 mg, 2.98 mmol, 2 Äq.) über Nacht zum F-moc geschützten Hexapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
LC-MS (Methode 20): Rₜ = 3.24 min; MS (ESIpos): *m*/*z* (%) = 1490 (85) [M+H]⁺, 282 (100); MS (ESIpos): *m*/*z* (%) = 1488 (100) [M+H]⁺.
HR-TOF-MS (Methode 24): C₈₁H₁₀₅N₁₀O₁₅S [M+H]⁺ gef. 1489.7456, ber. 1489.7477.

### Beispiel 68A

### Chlortrityl-Harz gebundenes {N²-[(9H-Fluoren-9-ylmethoxy)carbonyl]-L-leucyl}-[N⁵-(imino{[(2,2,5,7,8-pentamethyl-3,4-dihydro-2H-chromen-6-yl)sulfonyl]amino}methyl)-D-ornithyl]-L-isoleucyl-[O³-(tert-butyl)-L-threonyl]-glycyl-(N'-trityl-L-asparaginyl)-[O³-(tert-butyl)-L-serin]

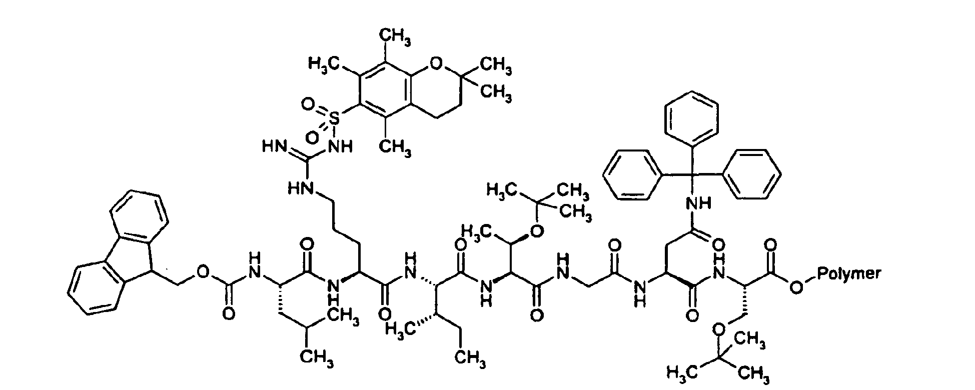

Die Fmoc-Schutzgruppe wird von dem Polymer (Beispiel 67A, 1000.0 mg, 1.49 mmol), wie in Arbeitsvorschrift 7 beschrieben ist, abgespalten. Anschließend wird das am Harz gebundene entschützte Hexapeptid mit *N*-[(9*H*-Fluoren-9-ylmethoxy)carbonyl]-L-leucin (1053.2 mg, 2.98 mmol, 2 Äq.), DIEA (779 µl, 577.7 mg, 4.47 mmol, 3 Äq.) und TBTU (956.8 mg, 2.98 mmol, 2 Äq.) über Nacht zum F-moc geschützten Heptapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
LC-MS (Methode 18): Rₜ = 3.54 min; MS (ESIpos): *m*/*z* (%) = 1602 (100) [M+H]⁺; MS (ESIpos): *m*/*z* (%) = 1600 (100) [M - H]⁻.

### Beispiel 69A

### [(3R)-N²-(tert-Butoxycarbonyl)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]-L-isoleucyl-[O³-(tert-butyl)-L-threonyl]-glycyl-(N³-trityl-L-asparaginyl)-[O³-(tert-butyl)-L-serin]

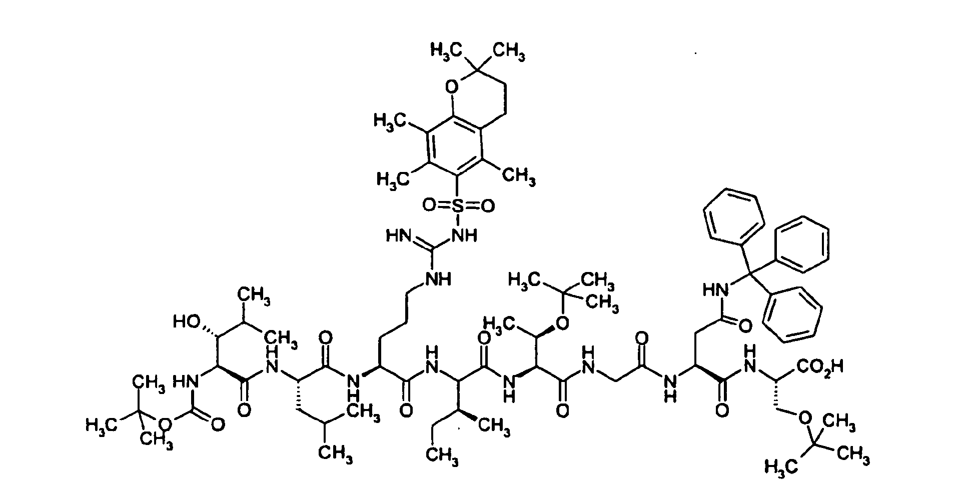

Die Fmoc-Schutzgruppe wird von dem Polymer (Beispiel 68A, 1000.0 mg, 1.49 mmol), wie in Arbeitsvorschrift 7 beschrieben ist, abgespalten. Anschließend wird das am Harz gebundene entschützte Heptapeptid mit (3*R*)-3-Hydroxy-*N*-(*tert-*butoxycarbonyl)-L-leucin (552.7 mg, 2.24 mmol, 1.5 Äq.), DIEA (597 µl, 442.9 mg, 3.43 mmol, 2.3 Äq.) und TBTU (717.6 mg, 2.24 mmol, 1.5 Äq.) über Nacht zum Fmoc-geschützten Octapeptid umgesetzt. Analog der Arbeitsvorschrift 7 erfolgt die Aufarbeitung des Polymers.

Das am Harz gebundene Octapeptid wird vollständig vom Polymer in einer Lösung aus Essigsäure, Trifluorethanol und Dichlormethan (1:1:3) abgespalten. Zur Aufarbeitung wird das Harz über eine Fritte filtriert, das Filtrat im Vakuum aufkonzentriert und letztlich chromatographisch (Methode 28) aufgereinigt. Die Titelverbindung wird mit 418.0 mg (17.4% d. Th.) erhalten.
HPLC (Methode 13) Rₜ = 2.85 min.
HPLC (Methode 1) Rₜ = 3.32 min.
LC-MS (Methode 18): Rₜ = 3.39 min; MS (ESIpos.): *m*/*z* (%) = 1610 (40) [M+H]⁺, 806 (100) [M + 2H]²⁺; MS (ESIneg.): *m*/*z* (%) = 1608 (100) [M - H]⁻.
HR-TOF-MS (Methode 24): C₈₃H₁₂₅N₁₂O₁₈S [M+H]⁺ gef. 1609.8971, ber. 1609.8951.

### Beispiel 70A

### [(3R)-3-Hydroxy-L-leucyl]-L-leucyl-D-arginyl-L-isoleucyl-L-threonyl-glycyl-L-asparaginyl-L-serin-bis-trifluoracetat

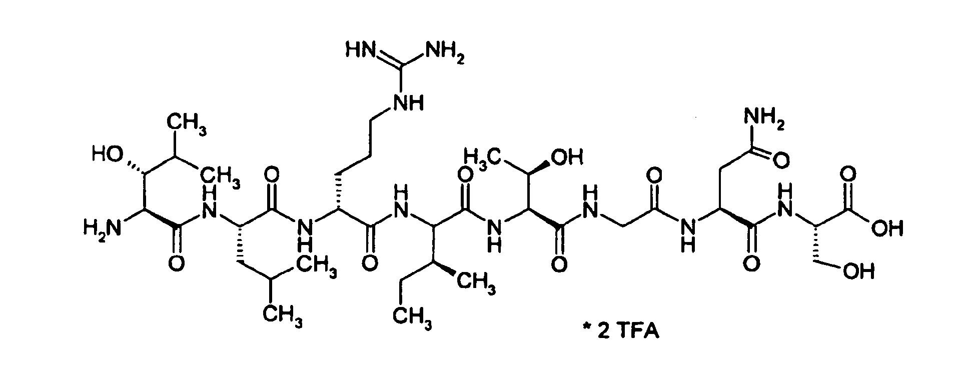

Unter Argonschutzgasatmosphäre wird die Beispielverbindung 69A (200.0 mg, 0.12 mmol) in einer Lösung aus TFA (25.3 ml), Wasser (667 µl) und Triisopropylsilan (667 µl) bei RT 1 h gerührt. Zur Aufarbeitung wird das Lösungsmittel am Rotationsverdampfer bei 30°C Badtemperatur entfernt und der Rückstand einmal mit Toluol und einmal mit Methylenchlorid bei 30°C Badtemperatur im Vakuum eingeengt. Der Rückstand wird in 0.1%iger wässr. TFA gelöst, einmal mit MTBE extrahiert, die abgetrennte wässr. Phase im Vakuum aufkonzentriert und anschließend über die präparative HPLC (Methode 26) feingereinigt. Man erhält 138.0 mg (99.5% d. Th.) der Titelverbindung.
HPLC (Methode 9) Rₜ = 8.60 min.
LC-MS (Methode 22): Rₜ = 2.43 min; MS (ESIpos): *m*/*z* (%) = 889 (15) [M+H]⁺, 445 (100) [M + 2H]²⁺; MS (ESIneg.): *m*/*z* (%) = 887 (100) [M - H]⁻.

### Beispiel 71A

### [N²-(Benzyloxycarbonyl)-D-leucyl]-L-leucyl-[(3R)-N³-(tert-butoxycarbonyl)-3-amino-L-phenylalanyl]-{(3R)-3-hydroxy-L-leucyl}-L-leucyl-D-arginyl-L-isoleucyl-L-threonylglycyl-L-asparaginyl-L-serin-trifluoracetat

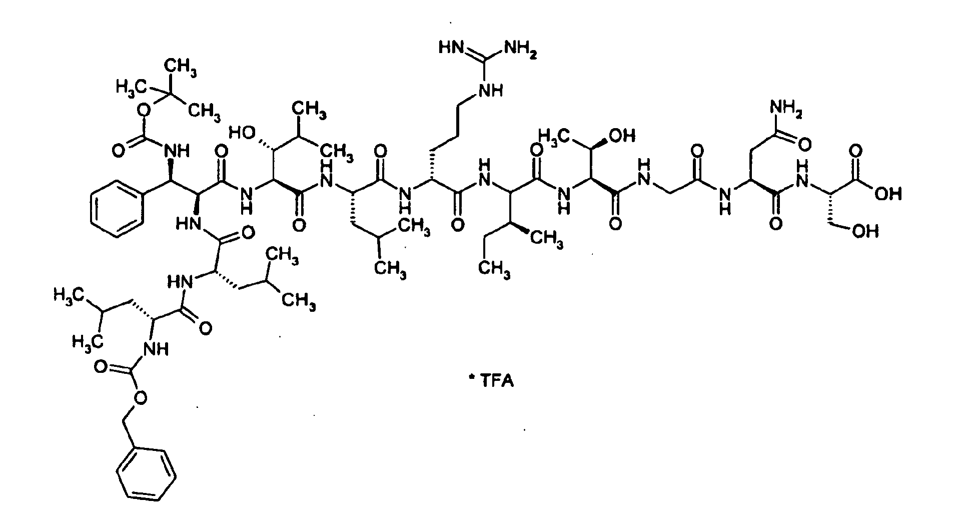

Aus dem Octapeptid (Beispiel 70A, 70.0 mg, 62.66 µmol), dem carbonsäureaktiviertem Tripeptid (Beispiel 64A, 60.7 mg, 75.19 µmol, 1.2 Äq.) und unter Verwendung von *N,N*-Diisopropylamin (65 µl, 48.6 mg, 375.96 µmol, 6 Äq.) wird, wie die Herstellungsvorschrift der Beispielverbindung 56A angibt, die Titelverbindung dargestellt. Die Reaktion wird bei RT über Nacht fast vollständig zum Umsatz gebracht. Nach dem Chromatographieren (Methode 26) werden 73.9 mg (72.5% d. Th.) Produkt erhalten.
HPLC (Methode 9) Rₜ = 22.16 min.
LC-MS (Methode 20): Rₜ = 2.07 min; MS (ESIpos): *m*/*z* (%) = 1512 (25) [M+H]⁺, 706 (100) [M + 2H]²⁺; MS (ESIneg.): *m*/*z* (%) = 1510 (18) [M - H]⁻, 700 (100).
HR-TOF-MS (Methode 24): C₇₁H₁₁₅N₁₆O₂₀ [M+H]⁺ gef. 1511.8474, ber. 1511.8469.

### Beispiel 72A

### [N²-(Benzyloxycarbonyl)-D-leucyl]-L-leucyl-[(3R)-3-amino-L-phenylalanyl]-[(3R)-3-hydroxy-L-leucyl]-L-leucyl-D-arginyl-L-isoleucyl-L-threonyl-glycyl-L-asparaginyl-L-serin-bistrifluoracetat

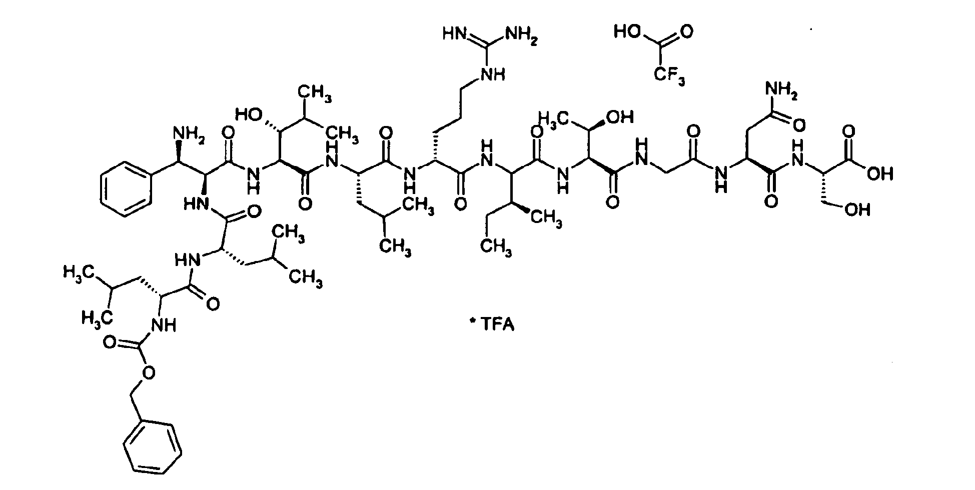

Gemäß der Arbeitsvorschrift 1 wird die Beispielverbindung 71A (70.0 mg, 43.06 µmol) zum entschützten Amin umgesetzt. Nach der Feinreinigung (Methode 26) erhält man 45.0 mg (63.7% d. Th.) der Titelverbindung.
HPLC (Methode 9) Rₜ = 16.77 min.
LC-MS (Methode 18): Rₜ = 1.85 min; MS (ESIpos): *m*/*z* (%) = 1412 (10) [M+H]⁺, 706 (100) [M + 2H]²⁺; MS (ESIneg.): *m*/*z* (%) = 1410 (90) [M - H]⁻, 650 (100).
HR-TOF-MS (Methode 24): C₆₆H₁₀₇N₁₆O₁₈ [M+H]⁺ gef. 1411.7958, ber. 1411.7944.

### Beispiel 73A

### [N²-(Benzyloxycarbonyl)-D-leucyl]-L-leucyl-[(3R)-3-amino-L-phenylalanyl]-[(3R)-3-hydroxy-L-leucyl]-L-leucyl-D-arginyl-L-isoleucyl-L-threonyl-glycyl-L-asparaginyl-L-serin-C^{1.11}-N^{3.3}-lactam-trifluoracetat

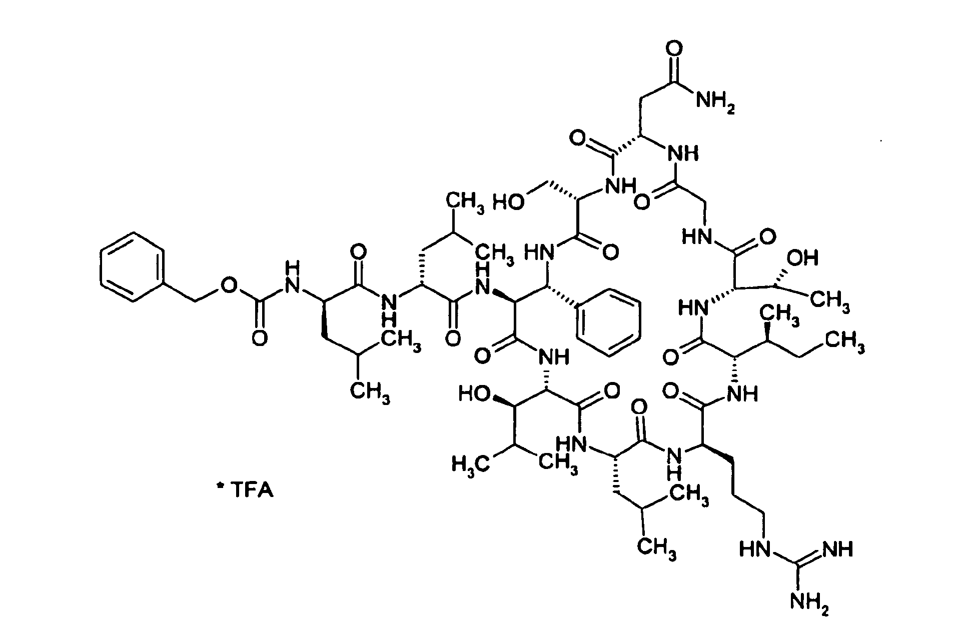

Gemäß der Herstellungsvorschrift (Beispiel 58A) wird aus der Beispielverbindung 73A (42.0 mg, 25.61 µmol), HATU (29.2 mg, 76.83 µmol, 3 Äq.) und NMM (17 µl, 15.5 mg, 153.66 µmol) die Titelverbindung dargestellt. Der vollständige Umsatz wird innerhalb von 3 h unter Rühren bei RT erreicht. Das Produkt wird nach der Feinreinigung (Methode 26) mit 34.1 mg (88.3% d. Th.) isoliert.
HPLC (Methode 9) Rₜ = 20.29 min.
LC-MS (Methode 18): Rₜ = 2.06 min; MS (ESIpos): *m*/*z* (%) = 1393 (32) [M+H]⁺, 797 (100) [M + 2H]²⁺; MS (ESIneg.): *m*/*z* (%) = 1391 (100) [M - H]⁻, 695 (32) [M - 2H]²⁻.
HR-TOF-MS (Methode 24): C₆₆H₁₀₅N₁₆O₁₇ [M+H]⁺ gef. 1393.7848, ber. 1393.7839.

### Beispiel 74A

### N⁵-[Benzyloxycarbonyl]-N²-[(9H-fluoren-9-ylmethoxy)carbonyl]-D-ornithin

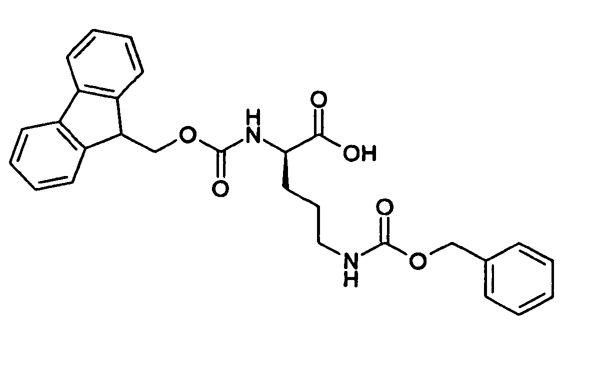

*N*⁵-[(Benzyloxy)carbonyl]-D-ornithin (3.8 g, 14.27 mmol) [Ulhaq, Saraj et al.; Bioorg.Med. Chem.; EN; 7; 9; 1999; 1787 - 1796] wird in Dichlormethan (190 ml) vorgelegt, mit DIEA (2.4 ml, 1.8 g, 17.27 mmol, 1 Äq.) und mit Chlortrimethylsilan (3.6 ml, 3.1 g, 28.53 mmol, 2 Äq.) versetzt und über Nacht bei Rückfluß gerührt. Zur abgekühlten Reaktion (0°C) wird nochmals DIEA (4.7 ml, 3.7 g, 28.54 mmol, 2 Äq.) und Chlorameisensäure-(9-fluorenylmethyl)-ester (3.7 g, 14.27 mmol, 1 Äq.) zugegeben, auf RT erwärmt und bei dieser Temperatur über Nacht nachgerührt. Zur Aufarbeitung wird die Reaktion mit Dichlormethan verdünnt, mit 10%iger wässr. Zitronensäurelösung gewaschen, die organische Phase über Natriumsulfat getrocknet, das Lösungsmittel am Rotationsverdampfer entfernt und im Vakuum getrocknet. Die Titelverbindung wird mit 6.5 g (93.2% d. Th.) erhalten.
LC-MS (Methode 18): Rₜ = 2.57 min; MS (ESIpos): *m*/*z* (%) = 489 (100) [M+H]⁺, 977 (100) [2M+H]⁺; MS (ESIneg.): *m*/*z* (%) = 487 (80) [M - H]⁻, 975 (100) [2M - H]⁻.

### Beispiel 75A

### Chlortrityl-Harz gebundenes {N⁵-[Benzyloxycarbonyl]-N²-[(9H-fluoren-9-ylmethoxy)carbonyl]-D-ornithyl}-L-isoleucyl-[O³-(tert-butyl)-L-allothreonyl]-glycyl-(N⁴-trityl-L-asparaginyl)-[O³-(tert-butyl)-L-serin]

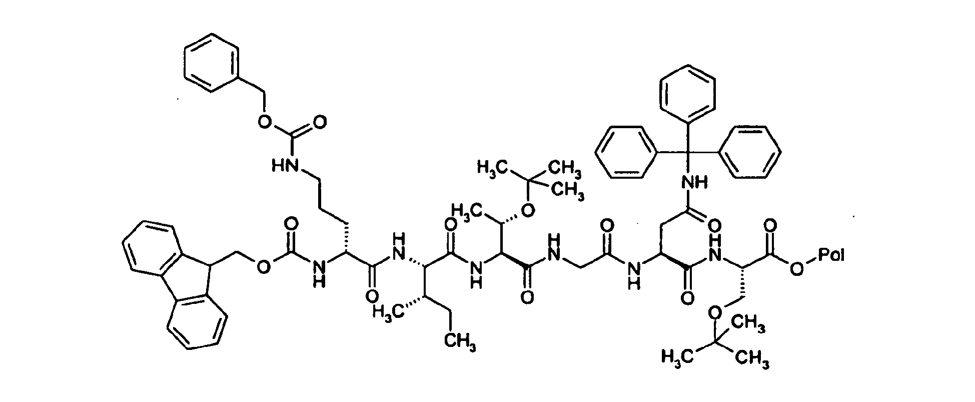

Die Fmoc-Schutzgruppe wird von dem Polymer (Beispiel 45A, 1000.0 mg, 1.49 mmol), wie in Arbeitsvorschrift 7 beschrieben ist, abgespalten. Anschließend wird das am Harz gebundene entschützte Pentapeptid mit der Fmoc-geschützen Aminosäure (Beispiel 74A, 1455.9 mg, 2.98 mmol, 2 Äq.), DIEA (779 µl, 577.7 mg, 4.47 mmol, 3 Äq.) und TBTU (956.8 mg, 2.98 mmol, 2 Äq.) über Nacht zum F-moc geschützten Hexapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
HPLC (Methode 13) Rₜ = 2.73 min.
LC-MS (Methode 18): Rₜ = 3.27 min; MS (ESIpos): *m*/*z* (%) = 1316 (100) [M+H]⁺; MS (ESIneg): *m*/*z* (%) = 1314 (100) [M+H]⁺.

### Beispiel 76A

### Chlortrityl-Harz gebundenes gebundenes {N²-[(9H-Fluoren-9-ylmethoxy)carbonyl]-L-leucyl}-{N⁵-[(Benzyloxy)carbonyl]-D-ornithyl}-L-isoleucyl-[O³-(tert-butyl)-L-allothreonyl]-glycyl-(N⁴-trityl-L-asparaginyl)-[O³-(tert-butyl)-L-serin]

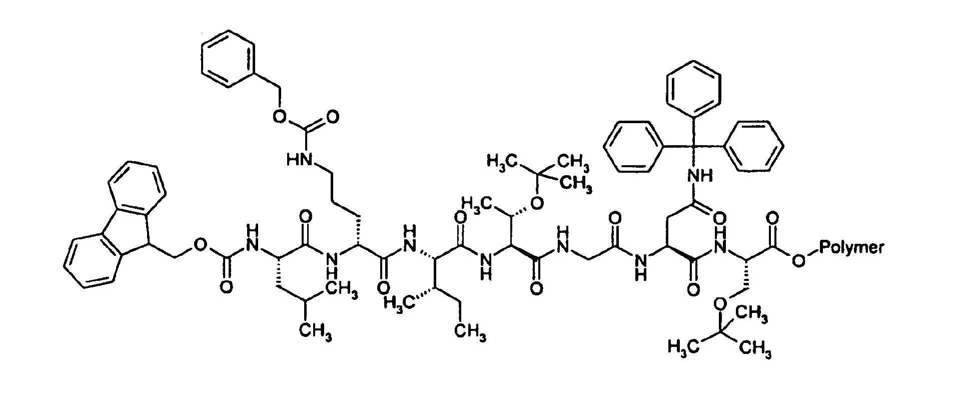

Die Fmoc-Schutzgruppe wird von dem Polymer (Beispiel 75A, 1000.0 mg, 1.49 mmol), wie in Arbeitsvorschrift 7 beschrieben ist, abgespalten. Anschließend wird das am Harz gebundene entschützte Hexapeptid mit *N*-[(9*H*-Fluoren-9-ylmethoxy)carbonyl]-L-leucin (1053.2 mg, 2.98 mmol, 2 Äq.), DIEA (779 µl, 577.7 mg, 4.47 mmol, 3 Äq.) und TBTU (956.8 mg, 2.98 mmol, 2 Äq.) über Nacht zum F-moc geschützten Heptapeptid umgesetzt. Analog der Arbeitsvorschrift 7 erfolgt die Aufarbeitung des Polymeres. Es wird das korrespondierende seitenkettengeschützte Peptid nach der Probeabspaltung bestätigt.
HPLC (Methode 13) Rₜ = 2.84 min.
LC-MS (Methode 18): Rₜ = 3.36 min; MS (ESIpos): *m*/*z* (%) = 1429 (95) [M+H]⁺, 1430 (100); MS (ESIpos): *m*/*z* (%) = 1427 (80) [M - H]⁻, 1428 (100).
HR-TOF-MS (Methode 24): C₆₁H₇₅N₆O₁₁ [M+H]⁺ gef. 1067.5488, ber. 1067.5489.

### Beispiel 77A

### [(3R)-N²-(tert-Butoxycarbonyl)-3-hydroxy-L-leucyl]-L-leucyl-[N⁵-(benzyloxycarbonyl)-D-ornithyl]-L-isoleucyl-[O³-(tert-butyl)-L-allothreonyl]-glycyl-[N⁴-trityl-L-asparaginyl]-[O³-(tert-butyl)-L-serin]

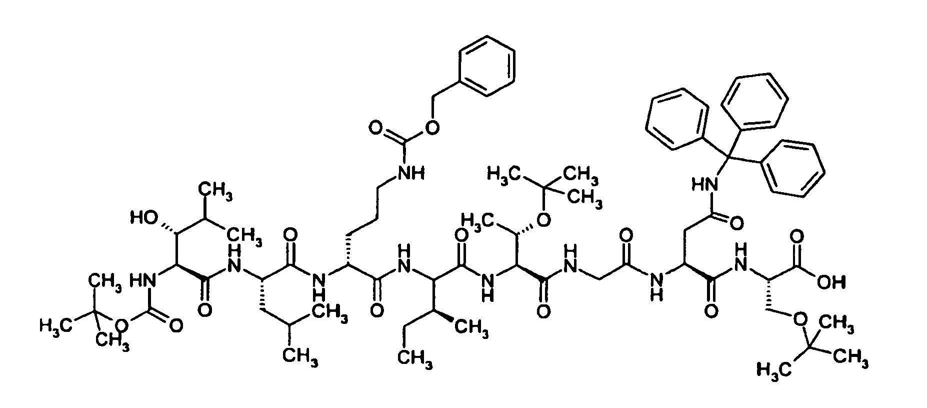

Die Fmoc-Schutzgruppe wird von dem Polymer (Beispiel 76A, 1000.0 mg, 1.49 mmol), wie in Arbeitsvorschrift 7 beschrieben ist, abgespalten. Anschließend wird das am Harz gebundene entschützte Heptapeptid mit (3*R*)-3-Hydroxy-*N*-(*tert-*butoxycarbonyl)-L-leucin [Oliyai, Reza, Siahaan, Teruna J., Stella, Valentino J.; Pharm. Res.; EN; 12; 3; 1995; 323 - 328] (552.7 mg, 2.24 mmol, 1.5 Äq.), DIEA (597 µl, 442.9 mg, 3.43 mmol, 2.3 Äq.) und TBTU (717.6 mg, 2.24 mmol, 1.5 Äq.) über Nacht zum Fmoc-geschützten Octapeptid umgesetzt. Analog der Arbeitsvorschrift 7 erfolgt die Aufarbeitung des Polymeres.

Das am Harz gebundene Octapeptid wird vollständig vom Polymer in einer Lösung aus Essigsäure, Trifluorethanol und Dichlormethan (1:1:3) abgespalten. Zur Aufarbeitung wird das Harz über eine Fritte filtriert, das Filtrat im Vakuum aufkonzentriert und letztlich chromatographisch (Methode 28) aufgereinigt. Die Titelverbindung wird mit 604.0 mg (28.3% d. Th.) erhalten.
HPLC (Methode 13) Rₜ = 2.68 min.
HPLC (Methode 1) Rₜ = 3.15 min.
LC-MS (Methode 18): Rₜ = 3.26 min; MS (ESIpos.): *m*/*z* (%) = 1437 (100) [M+H]⁺; MS (ESIneg.): *m*/*z* (%) = 1435 (100) [M - H]⁻.

### Beispiel 78A

### [(3R)-3-Hydroxy-L-leucyl]-L-leucyl-[N⁵-(benzyloxycarbonyl)-D-ornithyl]-L-isoleucyl-L-allothreonyl-glycyl-L-asparaginyl-L-serin-trifluoracetat

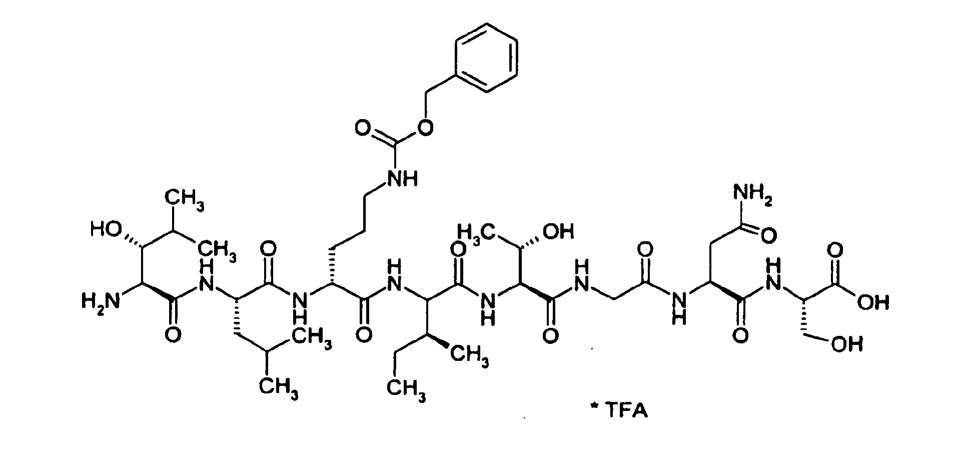

Die Titelverbindung wird aus der Beispielverbindung 77A (200.0 mg, 0.14 mmol), wie in der Beispielvorschrift (Beispiel 70A) beschrieben ist, dargestellt. Nach vollständiger Umsetzung bei RT (1 h) wird die Lösung im Vakuum bei 30°C Badtemperatur aufkonzentriert, einmal mit Toluol und einmal mit Methylenchlorid im Vakuum bei 30°C Badtemperatur eingeengt und abschließend über die präparative RP-HPLC (Methode 26) feingereinigt. Man erhält 121.0 mg (79.3% d. Th.) Produkt.
HPLC (Methode 9) Rₜ = 13.26 min.
LC-MS (Methode 22): Rₜ = 3.04 min; MS (ESIpos): *m*/*z* (%) = 981 (100) [M+H]⁺, 491 (10) [M + 2H]²⁺; MS (ESIneg.): *m*/*z* (%) = 979 (100) [M - H]⁻.

### Beispiel 79A

### [N²-(Benzyloxycarbonyl)-3-tert-butyl-D-alanyl]-[3-tert-butyl-L-alanyl]-{(3R)-N³-(tert-butoxycarbonyl)-3-amino-L-phenylalanyl}-{(3R)-3-hydroxy-L-leucyl}-L-leucyl-[N⁵-benzyloxycarbonyl-D-ornityl]-L-isoleucyl-L-allothreonyl-glycyl-L-asparaginyl-L-serin

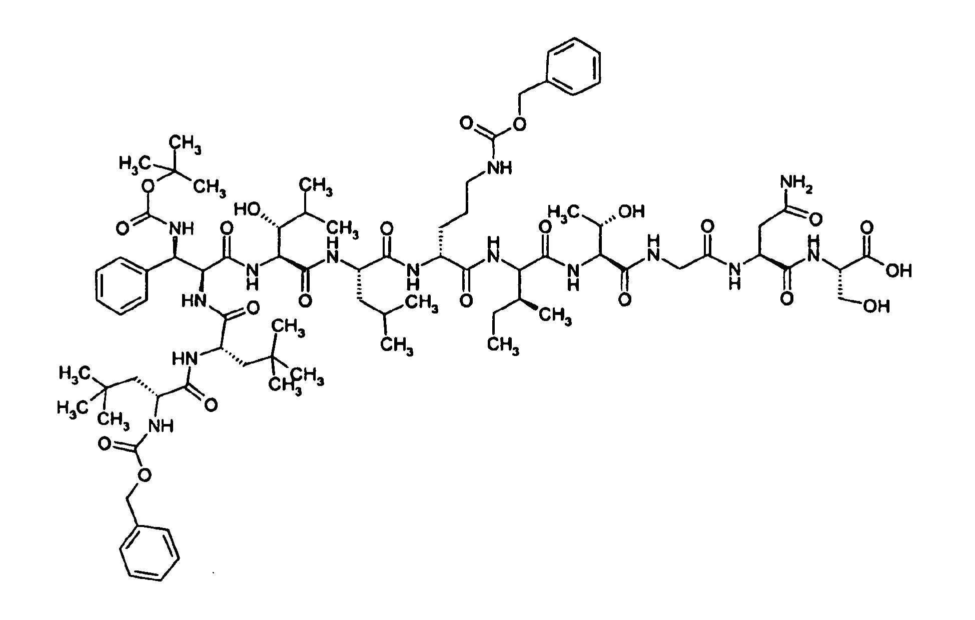

Aus dem Octapeptid (Beispiel 78A, 59.6 mg, 54.44 µmol), dem carbonsäureaktiviertem Tripeptid (Beispiel 40A, 50.0 mg, 59.89 µmol, 1.1 Äq.) und unter Verwendung von *N,N*-Diisopropylamin (57 µl, 42.2 mg, 326.7 µmol, 6 Äq.) wird, wie in der Beispielvorschrift 56A) beschrieben ist, die Titelverbindung dargestellt. Die Reaktion wird bei RT über Nacht vollständig zum Umsatz gebracht. Nach der Feinreinigung (Methode 26) wird 52.9 mg (59.5% d. Th.) Produkt erhalten.
HPLC (Methode 10) Rₜ = 10.32 min.
LC-MS (Methode 18): Rₜ = 3.07 min; MS (ESIpos): *m*/*z* (%) = 1633 (40) [M+H]⁺, 817 (90) [M + 2H]²⁺, 767 (100); MS (ESIneg.): *m*/*z* (%) = 1631 (65) [M - H]⁻, 761 (100).
HR-TOF-MS (Methode 24): C₈₀H₁₂₃N₁₄O₂₂ [M+H]⁺ gef. 1631.8898, ber. 1631.8931.

### Beispiel 80A

### [N²-(Benzyloxycarbonyl)-3-tert-butyl-D-alanyl]-[3-tert-butyl-L-alanyl]-{(3R)-3-amino-L-phenylalanyl}-{(3R)-3-hydroxy-L-leucyl}-[N⁵-(benzyloxycarbonyl)-D-ornityl]-L-isoleucyl-L-allothreonyl-glycyl-L-asparaginyl-L-serin-trifluoracetat

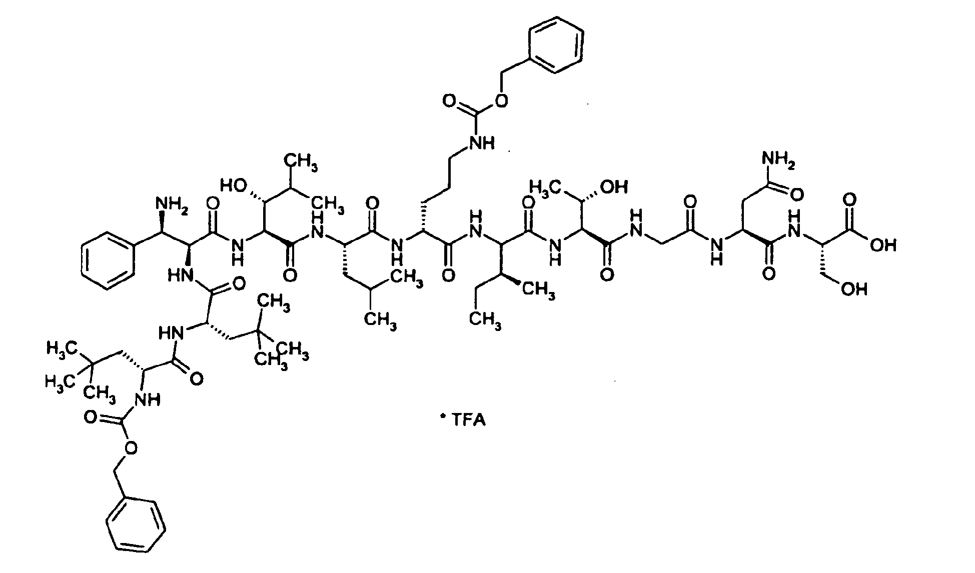

Gemäß der Arbeitsvorschrift 1 wird die Beispielverbindung 79A (50.0 mg, 30.64 µmol) zum entschützten Amin umgesetzt. Nach der Feinreinigung (Methode 26) erhält man 27.5 mg (54.5% d. Th.) der Titelverbindung.
HPLC (Methode 9) Rₜ = 21.85 min.
LC-MS (Methode 20): Rₜ = 2.18 min; MS (ESIpos): *m*/*z* (%) = 1533 (20) [M+H]⁺, 766 (100) [M + 2H]²⁺; MS (ESIneg.): *m*/*z* (%) = 1531 (58) [M - H]⁻, 764 (12) [M - 2H]²⁻, 710 (100).
HR-TOF-MS (Methode 24): C₇₅H₁₁₄N₁₄O₂₀ [M+H]⁺ gef. 1531.8385, ber. 1531.8407.

### Beispiel 81A

### [N²-(Benzyloxycarbonyl)-3-tert-butyl-D-alanyl]-[3-tert-butyl-L-alanyl]-{(3R)-3-amino-L-phenylalanyl}-[(3R)-3-hydroxy-L-leucyl]-L-leucyl-[N⁵-(benzyloxycarbonyl)-D-ornityl]-L-isoleucyl-L-allothreonyl-glycyl-L-asparaginyl-L-serin-C^{1.11}-N^{3.3}-lactam

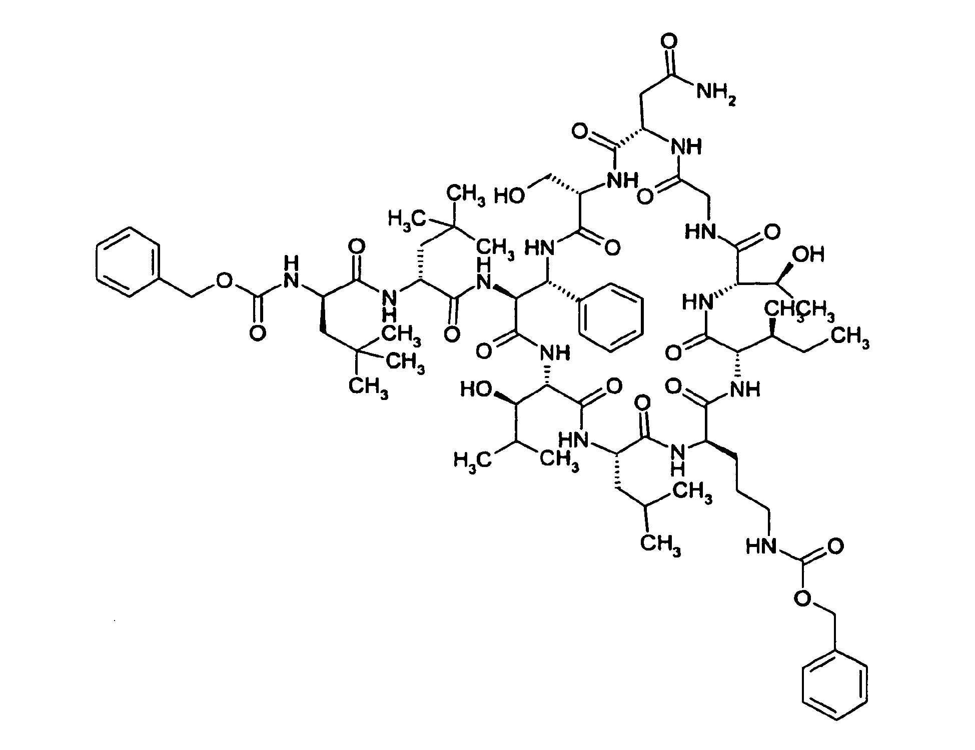

Gemäß der Herstellungsvorschrift (Beispiel 58A) wird aus der Beispielverbindung 80A (25.0 mg, 15.19 µmol), HATU (17.3 mg, 45.57 µmol, 3 Äq.) und NMM (10 µl, 9.2 mg, 91.14 µmol) die Titelverbindung dargestellt. Der vollständige Umsatz wird nach 3 Tagen bei 4°C erreicht. Das Produkt wird nach der Feinreinigung (Methode 26) mit 21.0 mg (91.3% d. Th.) isoliert.
HPLC (Methode 9) Rₜ = 26.28 min.
LC-MS (Methode 18): Rₜ = 2.98 min; MS (ESIpos): m/z (%) = 1514 (20) [M+H]⁺, 758 (100) [M + 2H]²⁺.
HR-TOF-MS (Methode 24): C₇₅H₁₁₃N₁₄O₁₉ [M+H]⁺ gef. 1513.8298, ber. 1513.8301.

### Beispiel 82A

### Methyl-(2R*,3R*)-N²-[(benzyloxy)carbonyl]-N²-[(benzyloxy)carbonylamino]-3-[(tert-butoxycarbonyl)amino]-O-methyl-tyrosinat

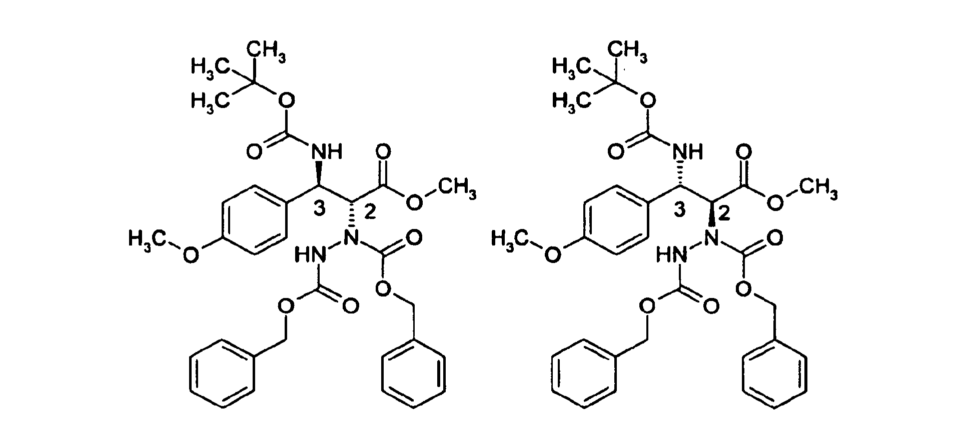

Unter Argonschutzgasatmosphäre wird eine 1 M LHMDS-Lösung (213.3 mmol, 213.3 ml, 2.2 Äq.) in THF in das Reaktionslösungsmittel THF (1.35 1) vorgelegt. Bei -78°C wird eine Lösung von (*rac*)-3-[(*tert*-Butoxycarbonyl)amino]-3-(4-methoxyphenyl)-propansäuremethylester (D. M. Kalvin, R. W. Woodard, J. Org. Chem., 50, 13, 1985, 2259-2263) (30 g, 96.9 mmol) langsam zugetropft. Man rührt 10 min bei -25°C und kühlt dann erneut auf -78°C herunter. Dibenzylazadicarboxylat (46.3 g, 155.2 mmol, 1.6 Äq.) wird in einer Portion zum Reaktionsgemisch gegeben. Es wird 2 h bei -60 bis -45°C gerührt. Um die Reaktion zu stoppen, wird erneut auf -78°C heruntergekühlt und mit Essigsäure (29.1 ml, 484.9 mmol, 5 Äq.) versetzt, und dann auf 0°C und schließlich RT erwärmt. Das Reaktionsgemisch wird im Vakuum eingedampft und in Essigsäureethylester (1000 ml) aufgenommen. Die Suspension wird zweimal mit ges. wässr. Natriumhydrogencarbonatlösung, zweimal mit Wasser, zweimal mit 5%iger wässr. Zitronensäure und einmal mit ges. wässr. Natriumchloridlösung gewaschen. Alle wässr. Phasen werden einzeln mit Essigsäureethylester reextrahiert. Alle org. Phasen werden im Vakuum eingedampft und erneut in Dichlormethan (2000 ml) aufgenommen, filtriert, über Magnesiumsulfat getrocknet, erneut filtriert und im Vakuum eingedampft. Der Rückstand wird mit Cyclohexan ausgerührt, filtriert und im Hochvakuum getrocknet. Man erhält 25.7 g (44% d. Th.) der Titelverbindung als Feststoff.
HPLC/UV-Vis (Methode 5): Rₜ = 4.97 min.
HPLC/UV-Vis (Methode 3): Rₜ = 5.00 min.
LC-MS (Methode 18): Rₜ = 2.87 min, MS (ESIpos.): *m*/*z* (%) = 508 (100), 608 (20) [M+H]⁺; MS (ESIneg.): *m*/*z* (%) = 532 (50), 606 (100) [M - H]⁻.

### Beispiel 83A

### Methyl-(2S*,3R*)-N²-[benzyloxycarbonyl]-N²-[(benzyloxycarbonyl)amino]-3-[(tert-butoxycarbonyl)amino]-O-methyl-tyrosinat

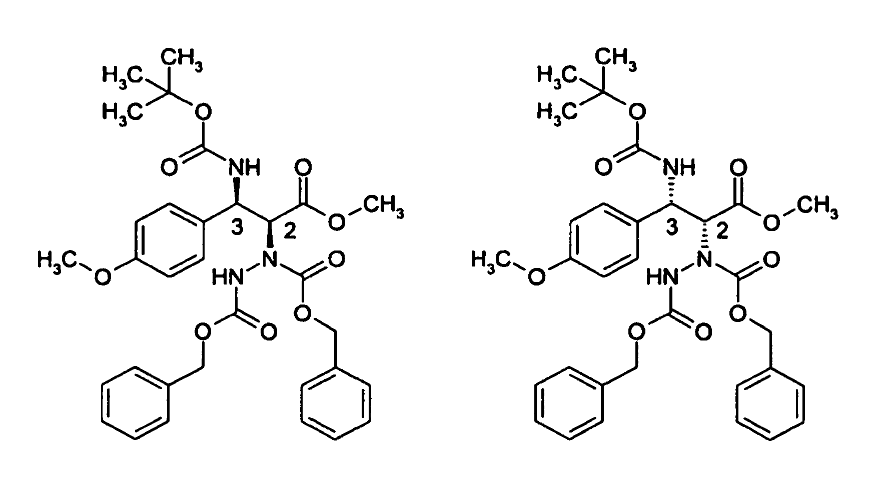

Unter Argonschutzgasatmosphäre wird eine Lösung von Beispiel 82A (2.5 g, 4.1 mmol) in trockenem DMF p.a. (87 ml) bei 0°C mit TMG (5.78 ml, 46.1 mmol, 11.2 Äq.) versetzt. Man lässt das Reaktionsgemisch auftauen und rührt bis das HPLC-Chromatogramm (Methode 5) vollständigen Umsatz (ca. 70% Produkt) anzeigt (ca. 24 h), um die Reaktion dann durch Zugabe von Essigsäure (pH 4-6) zu stoppen. Das Reaktionsgemisch wird im Vakuum bei RT eingedampft und in Essigsäureethylester aufgenommen. Die organische Phase wird zweimal mit Wasser, zweimal mit 5%iger Zitronensäure, einmal mit ges. wässr. Natriumhydrogencarbonatlösung, einmal mit ges. wässr. Natriumchloridlösung gewaschen, über Magnesiumsulfat getrocknet, filtriert, im Vakuum eingedampft, im Hochvakuum getrocknet. Das Rohprodukt wird mittels Flashchromatographie (Kieselgel, Toluol/Essigsäureethylester 20:1) gereinigt. Man erhält 1.89 g (73% d. Th.) der Titelverbindung.
HPLC/UV-Vis (Methode 5): Rₜ = 5.11 min.
HPLC/UV-Vis (Methode 4): Rₜ = 5.37 min.
LC-MS (Methode 18): Rₜ = 3.07 min, MS (ESIpos.): *m*/*z* (%) = 508 (100), 608 (20) [M+H]⁺; MS (ESIneg.): *m*/*z* (%) = 532 (100), 606 (30) [M - H]⁻.
IR vₘₐₓ (NaCl, cm⁻¹) : 3281, 2974, 1755, 1740, 1686, 1508, 1457, 1416, 1323, 1288, 1267, 1208, 1168, 1137, 1043, 1027.
¹H NMR (300 MHz, *d₆*-DMSO): δ = 1.29 (s, 9H, tBu), 3.29 (s, 3H, OCH₃), 3.72 (s, 3H, COOCH₃), 4.80-5.20(m, 6H), 6.84 (d, *J*= 8.5 Hz, 1H), 7.20-7.40 (m, 12H, ArH), 7.50-7.65 (m, 1H, NH).
HR-TOF-MS (Methode 24): C₃₂H₃₈N₃O₉ [M+H]⁺ ber. 608.2603, gef. 608.2588.

### Beispiel 84A

### (+)-Methyl-(2R,3S)-N²-[benzyloxycarbonyl]-N²-[(benzyloxycarbonyl)amino]-3-[(tert-butoxycarbonyl)amino]-O-methyl-tyrosinat

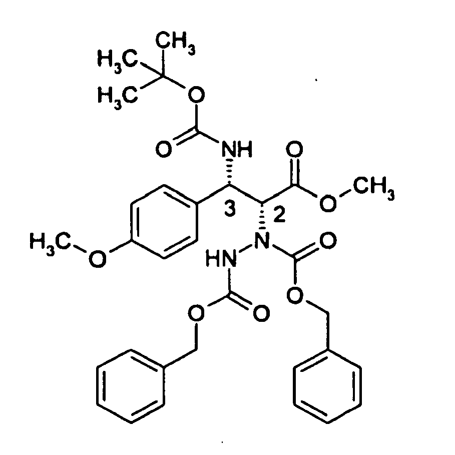

Das Enantiomerengemisch Beispiel 83A (10.6 g, 17.44 mmol) wird mittels präparativer HPLC (Methode 39) getrennt. Man erhält 4.18 g (99.5% ee, 79% d. Th.) der Titelverbindung und 5.2 g (99.5% ee, 98% d. Th.) des (-)-Enantiomers (Beispiel 85A).
Enantiomerenbestimmung nach Methode 15.
HPLC/UV-Vis (Methode 15): Rₜ = 3.86 min.
[α]²⁰Na = +54° (c = 0.24 in MeOH).
HPLC/UV-Vis (Methode 5): Rₜ = 5.11 min.
HPLC/UV-Vis (Methode 4): Rₜ = 5.23 min.
LC-MS (Methode 18): Rₜ = 3.07 min, MS (ESIpos.): *m*/*z* (%) = 508 (100), 608 (20) [M+H]⁺; MS (ESIneg.): *m*/*z* (%) = 532 (100), 606 (30) [M - H]⁻.
HR-TOF-MS (Methode 24): C₃₂H₃₈N₃O₉ [M+H]⁺ ber. 608.2603, gef. 608.2592.

### Beispiel 85A

### (-)-Methyl-(2S,3R)-N²-[benzyloxycarbonyl]-N²-[(benzyloxycarbonyl)amino]-3-[(tert-butoxycarbonyl)amino]-O-methyl-tyrosinat

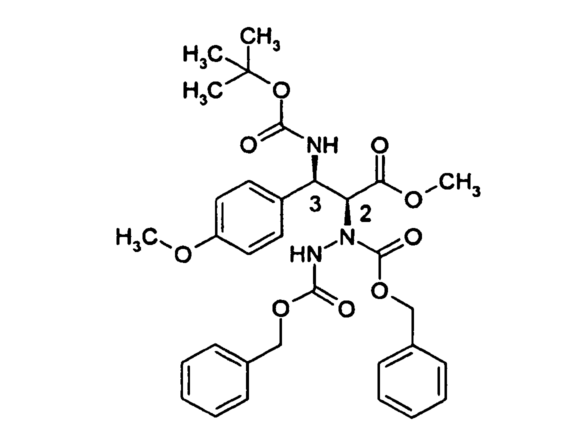

Die Herstellung erfolgt analog Beispiel 84A.
Enantiomerenbestimmung nach Methode 15.
HPLC/UV-Vis (Methode 15): Rₜ = 7.20 min.
[α]²⁰_{Na} = -34° (c = 0.27 in MeOH).
HPLC/UV-Vis (Methode 5): Rₜ = 5.11 min.
HPLC/UV-Vis (Methode 4): Rₜ = 5.21 min.
LC-MS (Methode 23): Rₜ = 2.87 min, MS (ESIpos.): *m*/*z* (%) = 508 (100), 608 (90) [M+H]⁺; MS (ESIneg.): *m*/*z* (%) = 532 (60), 606 (100) [M - H]⁻.
HR-TOF-MS (Methode 24): C₃₂H₃₈N₃O₉ [M+H]⁺ ber. 608.2603, gef. 608.2594.

### Beispiel 86A

### Methyl-(2S*,3R*)-3-[(tert-butoxycarbonyl)amino]-O-methyl-tyrosinat

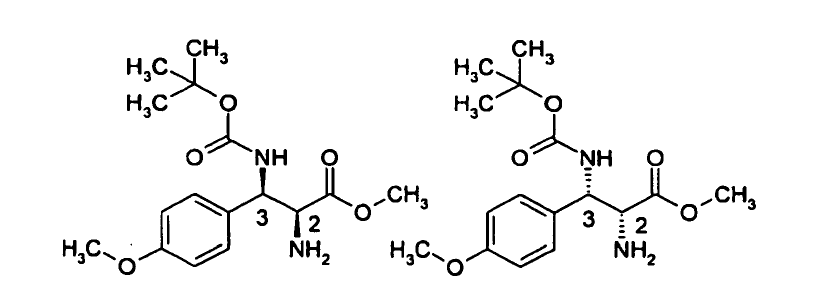

Unter Argonschutzgasatmosphäre wird zu einer Lösung von Beispiel 83A (2.81 g, 4.62 mmol) in Methanol/Dichlormethan 1:1 (100 ml) Raney-Nickel (ca. 1 g) gegeben. Das Reaktionsgemisch wird in einem Druck-Autoklaven bei 80 bar Wasserstoffdruck und RT hydriert (12 h). Das HPLC-Chromatogramm (Methode 8) zeigt vollständigen Umsatz. Das Reaktionsgemisch wird unter Argonschutzgasatmosphäre über einer Glasfritte / Kieselgurschicht filtriert, die Glasfritte wird mehrfach mit Methanol gewaschen. Das Filtrat wird im Vakuum eingedampft und am Hochvakuum getrocknet. Als Produkt erhält man einen Feststoff (1.50 g, quant.), der ohne Feinreinigung weiter umgesetzt wird.
HPLC/UV-Vis (Methode 8): Rₜ = 3.66 min.

### Beispiel 87A

### (+)-Methyl-(2R,3S)-3-[(tert-butoxycarbonyl)amino]-O-methyl-tyrosinat

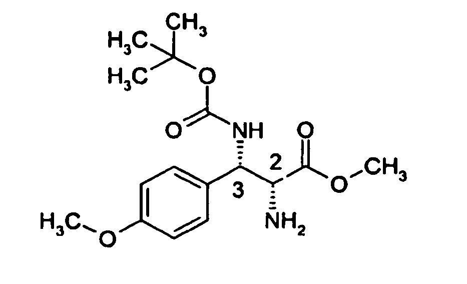

Unter Argonschutzgasatmosphäre wird zu einer Lösung von Beispiel 84A (4.00 g, 6.58 mmol) in Methanol/Dichlormethan 1:1 (100 ml) Raney-Nickel (ca. 0.8 g) gegeben. Das Reaktionsgemisch wird in einem Druck-Autoklaven bei 80 bar Wasserstoffdruck und RT hydriert (72 h). Das Reaktionsgemisch wird unter Argonschutzgasatmosphäre über einer Glasfritte/ Kieselgurschicht filtriert, die Glasfritte wird mehrfach mit Methanol gewaschen. Das Filtrat wird im Vakuum eingedampft und in Essigsäureethylester aufgenommen. Die Lösung wird mit einer Lösung von EDTA (2 g) in 50%iger wässr. Natriumhydrogencarbonatlösung (700 ml) versetzt. Die wässrige Phase wird 5x mit Essigsäureethylester (100 ml) extrahiert. Die vereinigten organischen Phasen werden dann zweimal mit ges. wässr. Natriumhydrogencarbonatlösung und zweimal mit ges. wässr. Natriumchloridlösung gewaschen. Alle wässr. Phasen werden einzeln mit Essigsäureethylester reextrahiert. Die vereinigten org. Phasen werden dann über Magnesiumsulfat getrocknet, filtriert und am Hochvakuum getrocknet. Als Produkt erhält man einen Feststoff (1.04 g, 49% d. Th., 96% ee).
HPLC/UV-Vis (Methode 16): Rₜ = 7.8 min.
[α]²⁰_{Na} = +7.7° (*c* = 0.34 in MeOH).
HPLC/UV-Vis (Methode 5): Rₜ = 3.69 min.
HPLC/UV-Vis (Methode 4): Rₜ = 3.87 min.
LC-MS (Methode 20): Rₜ = 1.17 min; MS (ESIpos.): *m*/*z* (%) = 208 (90), 269 (100), 325 (40) [M+H]⁺.
IR νₘₐₓ (NaCl, cm⁻¹): 2933, 1737, 1709, 1611, 1511, 1365, 1243, 1164, 1030.

### Beispiel 88A

### (-)-Methyl-(2S,3R)-3-[(tert-butoxycarbonyl)amino]-O-methyl-tyrosinat

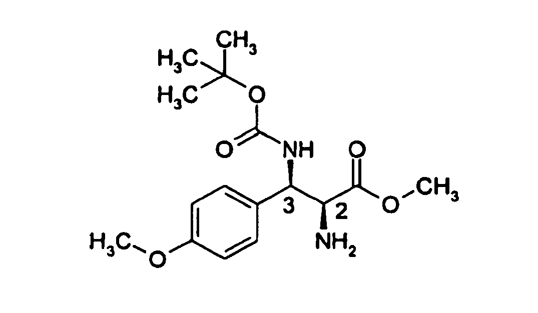

Unter Argonschutzgasatmosphäre wird zu einer Lösung von Beispiel 85A (5.20 g, 8.56 mmol) in Methanol/ Dichlormethan 1:1 (140 ml) Raney-Nickel (ca. 0.7 g) gegeben. Das Reaktionsgemisch wird in einem Druck-Autoklaven bei 80 bar Wasserstoffdruck und RT hydriert (72 h). Das Reaktionsgemisch wird unter Argonschutzasatmosphäre über einer Glasfritte/ Kieselgurschicht filtriert, die Glasfritte wird mehrfach mit Methanol gewaschen. Das Filtrat wird im Vakuum eingedampft und in Essigsäureethylester aufgenommen. Es wird mittels Kieselgelchromatographie (Cyclohexan/Essigsäureethylester 3:1 → 1:3) vorgereinigt, und mittels präparativer HPLC (Methode 31) getrennt. Man erhält die Titelverbindung (324 mg, 91% ee, 8% d. Th.).
HPLC/UV-Vis (Methode 16): Rₜ = 9.3 min.
HPLC/UV-Vis (Methode 5): Rₜ = 3.69 min.
HPLC/UV-Vis (Methode 4): Rₜ = 3.84 min.
LC-MS (Methode 20): Rₜ = 1.17 min; MS (ESIpos.): *m*/*z* (%) = 208 (90), 269 (100), 325 (40) [M+H]⁺.
¹H NMR (500 MHz, *d₆*-DMSO): δ = 1.37 (s, 9H, C(CH₃)₃), 3.53 (s, 3H, OCH₃), 3.74 (s, 3H, OCH₃), 4.26 (m, 1H, β-CH), 5.00 (t, *J* = 9.0 Hz, 1H, β-CH), 6.93 (d, *J* = 8.0 Hz, 2H, ArH), 7.23 (d, *J* = 8.0 Hz, 2H, ArH), 7.52 (d, *J* = 10.0 Hz, 1H, NH), 8.45 (br. s, 2H, NH₂).

### Beispiel 89A

### Methyl-(2S*,3R*)-N²-[benzyloxycarbonyl]-3-[(tert-butoxycarbonyl)amino]-O-methyltyrosinat

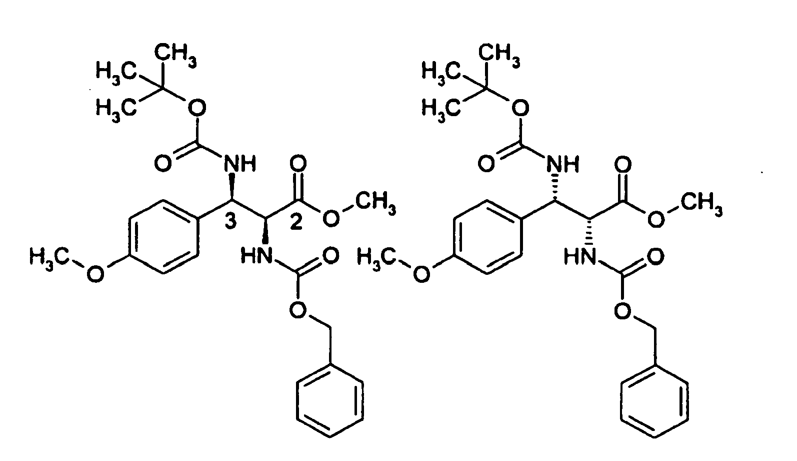

Unter Argonschutzgasatmosphäre wird zu einer Lösung von Beispiel 86A (1.5 g, 4.62 mmol) und *N*-Benzyloxycarbonyloxysuccinimidester (1268 mg, 5.09 mmol, 1.1 Äq.) in Dichlormethan/Wasser (1:2, 150 ml) bei 0°C NaHCO₃ (0.58 g, 6.94 mmol, 1.5 Äq.) und Tetra-*N*-butylammoniumiodid (0.17 g, 0.47 mmol, 0.1 Äq.) zugegeben. Das Reaktionsgemisch wärmt sich langsam auf (12 h), wobei vollständiger Umsatz mittels HPLC (Methode 1) beobachtet wird. Die Lösung wird mit einer Lösung von Citronsäure (5%iger) versetzt. Die wässr. Phase wird dreimal mit Dichlormethan (100 ml) extrahiert. Die vereinigten organischen Phasen werden dann zweimal mit ges. wässr. Natriumhydrogencarbonatlösung und zweimal mit ges. wässr. Natriumchloridlösung gewaschen. Die vereinigten org. Phasen werden dann über Magnesiumsulfat getrocknet, filtriert und am Hochvakuum getrocknet. Als Produkt erhält man die Titelverbindung als Feststoff (2.1 g, 99% d. Th.).
HPLC/UV-Vis (Methode 5): Rₜ = 4.73 min.
HPLC/UV-Vis (Methode 4): Rₜ = 4.68 min.
IR νₘₐₓ (NaCl, cm⁻¹): 3356, 1737, 1680, 1514, 1242, 1161, 1026, 1004.
¹H NMR (500 MHz, *d₆*-DMSO): δ = 1.36 (s, 9H, C(CH₃)₃), 3.61 (s, 3H, OCH₃), 3.73 (s, 3H, OCH₃), 4.51 (dd, *J* = 9.5, 4.0 Hz, 1H, α-H/β-H), 4.94 (m, 2H, PhCH₂O), 5.20 (dd, *J* = 10.0, 4.0 Hz, 1H, α-H/β-H), 6.87 (d, *J* = 8.5 Hz, 2H, ArH), 7.15-7.22 (m, 4H, ArH), 7.29-7.37 (m, 3H, ArH), 7.50 (d, *J* = 10.0 Hz, 1H, NH), 7.57 (d, *J* = 10.0 Hz, 1H, NH).
¹³C NMR (126 MHz, *d₆*-DMSO): δ = 27.9, 51.9, 53.8, 54.9, 58.9, 65.4, 78.3, 113.5, 126.8, 127.3, 127.5, 127.7, 128.2, 130.9, 136.6, 154.6, 155.9, 158.2, 170.6,
HR-TOF-MS (Methode 24): C₂₅H₃₀N₂O₇ [M+H]⁺ gef. 459.2140, ber. 459.2126.

### Beispiel 90A

### (+)-Methyl-(2R,3S)-N²-[benzyloxycarbonyl]-3-[(tert-butoxycarbonyl)amino]-O-methyltyrosinat

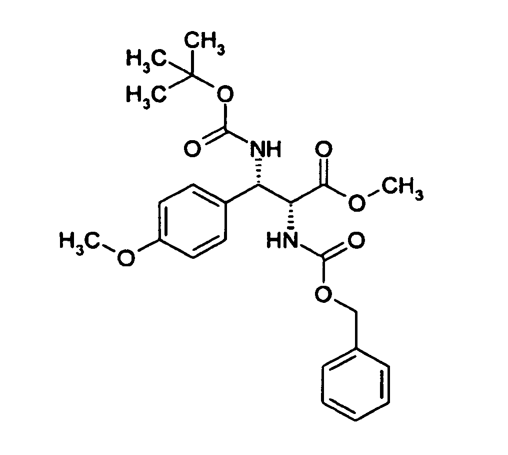

Analog zur der Herstellungsvorschrift für Beispiel 89A wird Beispiel 87A (1.0 g, 3.08 mmol) umgesetzt (Reaktionszeit: 12 h). Das Rohprodukt wird mittels präparativer HPLC (Methode 32) gereinigt. Man erhält 1.4 g (61% d. Th., 96% ee) der Titelverbindung.
HPLC/UV-Vis (Methode 16): Rₜ = 7.3 min.
[α]²⁰_{Na} = +4.0° (c = 0.17 in MeOH).
HPLC/UV-Vis (Methode 5): Rₜ = 4.73 min.
HPLC/UV-Vis (Methode 4): Rₜ = 4.81 min.
IR νₘₐₓ (NaCl, cm⁻¹): 3346, 1741, 1707, 1680, 1517, 1270, 1248, 1160, 1028, 1007.
LC-MS (Methode 23): Rₜ = 2.63 min; MS (ESIpos.): *m*/*z* (%) = 298 (100), 459 (30) [M+H]⁺; MS (ESIneg.): *m*/*z* (%) = 383 (100), 457 (10) [M - H]⁻.
HR-TOF-MS (Methode 24): C₂₅H₃₀N₂O₇ [M+H]⁺ gef. 459.2128, ber. 459.2126.

### Beispiel 91A

### (-)-Methyl-(2S,3R)-N²-[benzyloxycarbonyl]-3-[(tert-butoxycarbonyl)amino]-O-methyltyrosinat

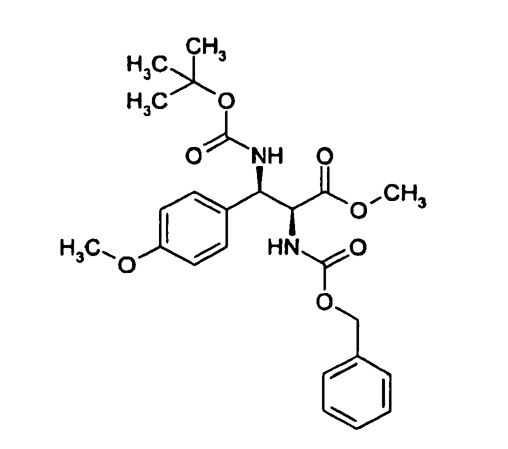

Analog zur der Herstellungsvorschrift für Beispiel 89A wird Beispiel 88A (0.32 g, 0.98 mmol) umgesetzt (Reaktionszeit: 12 h). Als Produkt erhält man die Titelverbindung als Feststoff (0.38 g, 85% d. Th.).
HPLC/UV-Vis (Methode 16): Rₜ = 6.3 min.
HPLC/UV-Vis (Methode 5): Rₜ = 4.73 min.
HPLC/UV-Vis (Methode 4): Rₜ = 4.81 min.
LC-MS (Methode 23): Rₜ = 2.60 min; MS (ESIpos.): *m*/*z* (%) = 298 (100), 459 (70) [M+H]⁺; MS (ESIneg.): *m*/*z* (%) = 383 (100), 457 (10) [M - H]⁻.

### Beispiel 92A

### (2S*,3R*)-N²-[Benzyloxycarbonyl]-3-[(tert-butoxycarbonyl)amino]-O-methyl-tyrosin

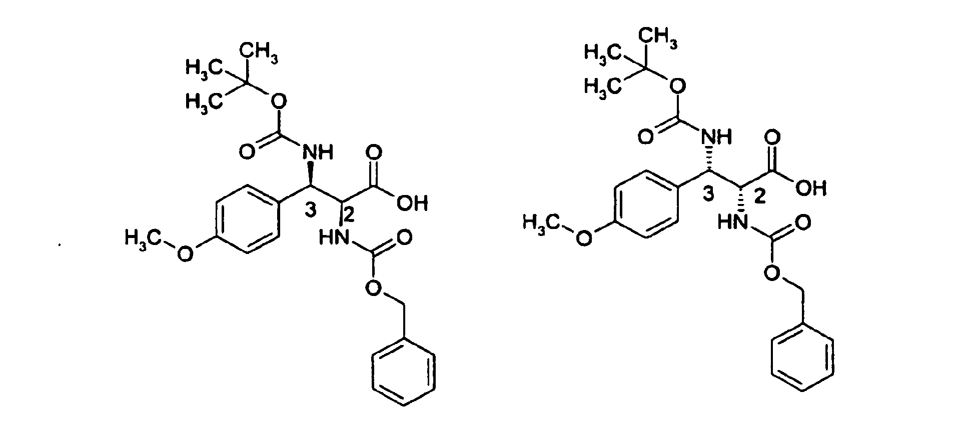

Unter Argonschutzgasatmosphäre wird eine Lösung von Beispiel 89A (759 mg, 1.69 mmol) in THF/Wasser 2:1 (12 ml) vorgelegt. Bei 0°C wird unter starkem Rühren eine wässr. Lösung von Lithiumhydroxid-Monohydrat (81 mg, 3.39 mmol, 2.05 Äq.) langsam zugetropft. Man rührt bei RT so lange nach, bis das HPLC-Chromatogramm (Methode 5) vollständigen Umsatz anzeigt (ca. 3 h). Anschließend versetzt man mit Kaliumhydrogenphosphat (225 mg), konzentriert das Reaktionsgemisch im Vakuum auf und überschichtet mit Essigsäureethylester (100 ml). Die wässr. Phase wird nun mit 5%iger Zitronensäure angesäuert (pH 2-3) und dann dreimal mit Essigsäureethylester (50 ml) extrahiert. Die vereinigten org. Phasen werden zweimal mit ges. wässr. Natriumchloridlösung (20 ml) gewaschen, über Natriumsulfat getrocknet, filtriert, im Vakuum eingeengt und im Hochvakuum getrocknet. Man erhält die Titelverbindung (735 mg quant.).
HPLC/UV-Vis (Methode 5): Rₜ = 4.45 min.
LC-MS (Methode 21): Rₜ = 2.33 min; MS (ESIpos.): *m*/*z* (%) = 389 (100), 445 (90) [M+H]⁺; MS (ESIneg.): *m*/*z* (%) = 335 (60), 443 (100) [M - H]⁻.
¹H NMR (400 MHz, *d₆*-DMSO): δ = 1.35 (s, 9H, C(CH₃)₃), 3.72 (s, 3H, OMe), 4.35-4.40 (m, 1H, α-H), 4.94 (s, 2H, PhCH₂O), 5.19 (dd, *J* = 10.0, 4.0 Hz, 1H, β-H), 6.86 (d, *J* = 9.0 Hz, 2H), 7.17-7.34 (m, 7H, ArH), 7.44-7.48 (m, 2H, 2 NH), 12.9 (s, br, 1H, CO₂H).

### Beispiel 93A

### (+)-(3S)-N²-[Benzyloxycarbonyl]-3-[(tert-butoxycarbonyl)amino]-O-methyl-D-tyrosin

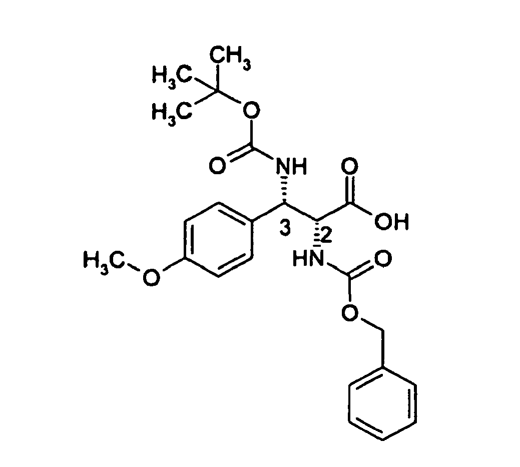

Methode A: Das Enantiomerengemisch von Beispiel 92A (735 mg, 2.83 mmol) wird mittels präparativer HPLC (Methode 40) getrennt. Man erhält 319 mg (99.5% ee, 43% d. Th.) der Titelverbindung und 307 mg (99.5% ee, 42% d. Th.) des anderen Enantiomers (Beispiel 94A).
Enantiomerenbestimmung nach Methode 16.
HPLC/UV-Vis (Methode 16): Rₜ = 10.9 min.
[α]²⁰_{Na} = +8° (c = 0.04 in MeOH).
HPLC/UV-Vis (Methode 5): Rₜ = 4.45 min.
LC-MS (Methode 21): Rₜ = 2.33 min; MS (ESIpos.): *m*/*z* (%) = 389 (100), 445 (90) [M+H]⁺; MS (ESIneg.): *m*/*z* (%) = 335 (60), 443 (100) [M - H]⁻.
IR νₘₐₓ, (NaCl, cm⁻¹): 3358, 2978, 1705, 1683, 1615, 1515, 1245, 1167, 1027.
HR-TOF-MS (Methode 24): C₂₃H₂₉N₂O₇ [M+H]⁺ gef. 445.1976, ber. 445.1970.

Methode B: Analog zur der Herstellungsvorschrift für Beispiel 92A wird Beispiel 93A (1.30 g, 2.83 mmol) umgesetzt (Reaktionszeit: 2 h). Als Produkt erhält man die Titelverbindung als Feststoff (0.61 g, 48% d. Th., 95% ee).
HPLC/UV-Vis (Methode 16): Rₜ = 10.9 min.
[α]²⁰_{Na} = +15° (c = 0.23 in MeOH).
HPLC/UV-Vis (Methode 5): Rₜ = 4.41 min.
HPLC/UV-Vis (Methode 4): Rₜ = 4.54 min.
HR-TOF-MS (Methode 24): C₂₃H₂₈N₂O₇Na [M + Na]⁺ gef. 467.1788, ber. 467.1794.

### Beispiel 94A

### (-)-(3R)-N²-[Benzyloxycarbonyl]-3-[(tert-butoxycarbonyl)amino]-O-methyl-L-tyrosin

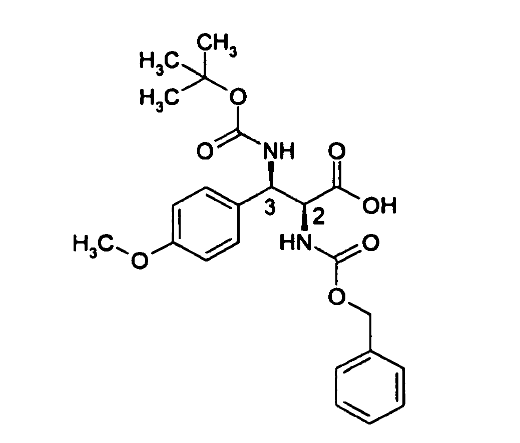

Methode A: Darstellungsvorschrift unter Beispiel 93A (Methode A)
HPLC/UV-Vis (Methode 16): Rₜ = 13.3 min.
[α]²⁰_{Na} = -10° (c = 0.12 in MeOH).
HPLC/UV-Vis (Methode 5): Rₜ = 4.45 min.
LC-MS (Methode 21): Rₜ = 2.33 min; MS (ESIpos.): *m*/*z* (%) = 389 (100), 445 (90) [M+H]⁺; MS (ESIneg.): *m*/*z* (%) = 335 (60), 443 (100) [M - H]⁻.
HR-TOF-MS (Methode 24): C₂₃H₂₉N₂O₇ [M+H]⁺ gef. 445.1976, ber. 445.1970.

Methode B: Analog zur der Herstellungsvorschrift für Beispiel 92A wird Beispiel 90A (0.38 g, 0.84 mmol) umgesetzt (Reaktionszeit: 2 h). Als Produkt erhält man die Titelverbindung als Feststoff (0.132 g, 35% d. Th., 94% ee).
HPLC/UV-Vis (Methode 16): Rₜ = 13.3 min.
[α]²⁰_{Na} = -5° (c = 0.1 in MeOH).
HPLC/UV-Vis (Methode 5): Rₜ = 4.41 min.
HPLC/UV-Vis (Methode 4): Rₜ = 4.49 min.
LC-MS (Methode 18): Rₜ = 2.38 min; MS (ESIpos.): *m*/*z* (%) = 389 (100), 445 (100) [M+H]⁺; MS (ESIneg.): *m*/*z* (%) = 335 (60), 443 (100) [M - H]⁻.
¹³C NMR (126 MHz, *d₆*-DMSO): δ = 28.0, 53.9, 54.9, 58.8, 65.2, 78.2, 113.4, 127.2, 127.3, 127.6, 128.1, 128.2, 131.7, 136.8, 154.7, 156.0, 158.1, 171.4.
HR-TOF-MS (Methode 24): C₂₃H₂₉N₂O₇ [M+H]⁺ gef. 445.1966, ber. 445.1970.

### Beispiel 95A

### (1S,2R,5R)-2-Isopropyl-5-methylcyclohexyl-[(3S)-N²-[benzyloxycarbonyl]-3-[(tert-butoxycarbonyl)amino]]-O-methyl-D-tyrosinat

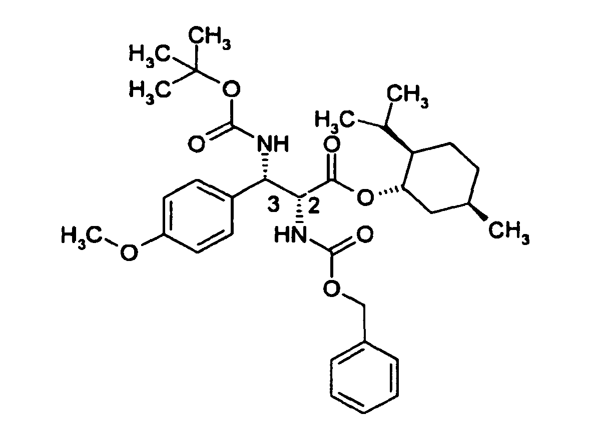

Unter Argonschutzgasatmosphäre wird eine Lösung von Beispiel 93A (34 mg, 76 µmol, 1 Äq.) und (1*S*,2*R*,5*S*)-(+)-Menthol (12 mg, 76 µmol, 1 Äq.) in Dichlormethan vorgelegt. Bei 0°C wird DMAP (17.3 mg, 84 µmol, 1.1 Äq.) zugegeben und eine Lösung von DCC (17.3 mg, 84 µmol, 1.1 Äq.) in Dichlormethan (0.5 ml) langsam zugetropft. Man rührt bei RT so lange nach, bis das HPLC-Chromatogramm (Methode 5) vollständigen Umsatz anzeigt (ca. 2 h). Anschließend filtriert man über Kieselgur, konzentriert das Reaktionsgemisch im Vakuum auf und versetzt mit Essigsäureethylester/Petrolether 3:1. Ein weißer Feststoff fällt aus und wird abfiltriert. Das Filtrat wird eingeengt und mittels Kieselgelchromatographie (Petrolether/Essigsäureethylester 3:1) vorgereinigt. Es wird mittels präparativer HPLC (Methode 43) gereinigt. Man erhält die Titelverbindung (17 mg, 38% d. Th.).
HPLC/UV-Vis (Methode 5): Rₜ = 5.74 min.
HPLC/UV-Vis (Methode 4): Rₜ = 5.72 min.
LC-MS (Methode 18): Rₜ = 3.46 min; MS (ESIpos.): *m*/*z* (%) = 483 (100), 527 (80), 583 (40) [M+H]⁺.
¹H NMR (500 MHz, *d₆*-DMSO): δ = 0.71 (d, 3H, *J* =7 Hz, CH₃), 0.81 (d, 3H, *J* =7 Hz, CH₃), 0.81-0.84 (m, 3H), 0.87 (d, 3H, *J* =7 Hz, CH₃), 1.00-1.06 (m, 1H), 1.22-1.28 (m, 1H), 1.35 (s, 9H, C(CH₃)₃), 1.61-1.70 (m, 3H), 1.89-1.92 (m, 1H), 3.72 (s, 3H, OMe), 4.49 (dd, 1H, *J* = 4.5, 9.5 Hz, β-H), 4.59 (dt, *J* = 4.0, 11.0 Hz, 1H, O-CH), 4.91 (d, *J* = 12.0 Hz, 1H, PhCH₂O), 4.97 (d, *J* = 12.0 Hz, 1H, PhCH₂O) 5.17 (dd, *J* = 4.5, 9.5 Hz, 1H, β-H), 6.86 (d, 2H, *J* = 9.0 Hz, ArH), 7.16-7.24 (m, 4H, ArH), 7.28-7.34 (m, 3H, ArH), 7.46 (d, 1H, *J* = 8.0 Hz, NH), 7.55 (d, 1H, *J* = 8.0 Hz, NH).

### Beispiel 96A

### (+)-2-(Trimethylsilyl)ethyl-(3S)-N²-[benzyloxycarbonyl]-3-[(tert-butoxycarbonyl)-amino]-O-methyl-D-tyrosinat

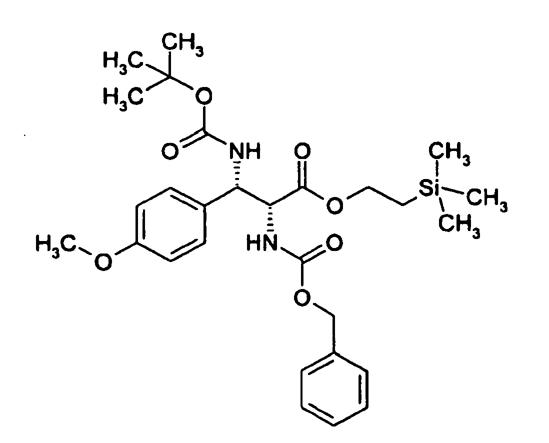

Eine Mischung von Beispiel 93A (261 mg, 0.59 mmol), 2-(Trimethylsilyl)ethanol (694 mg, 5.87 mmol, 10 Äq.) und Molsieb 4 Å (ca. 20 mg) in trockenem Dichlormethan p.a. (10 ml) wird für 10 min bei RT unter Argonschutzgasatmosphäre gerührt. Anschließend werden bei -30°C DCC (244 mg, 1.17 mmol, 2 Äq.) und DMAP (71 mg, 0.59 mmol, 1 Äq.) zugegeben. Man lässt das Reaktionsgemisch auftauen (ca. 12 h) und rührt bei RT so lange nach, bis das HPLC-Chromatogramm (Methode 5) vollständigen Umsatz anzeigt (12 h). Das Reaktionsgemisch wird bei RT im Vakuum eingedampft und mittels Kieselgelchromatographie (Petrolether/Essigsäureethylester 3:1) gereinigt. Man erhält 244 mg (76% d. Th., 99% ee) der Titelverbindung.
HPLC/UV-Vis (Methode 16): Rₜ = 8.2 min.
[α]²⁰_{Na} = +10° (*c* = 0.1 in MeOH).
HPLC/UV-Vis (Methode 5): Rₜ = 5.34 min.
HPLC/UV-Vis (Methode 4): Rₜ = 5.42 min.
IR νₘₐₓ (NaCl, cm⁻¹): 3361, 2931, 2118, 1735, 1706, 1681, 1515, 1247, 1165, 1019.
LC-MS (Methode 21): Rₜ = 3.16 min; MS (ESIpos.): *m*/*z* (%) = 166 (100), 545 (15) [M+H]⁺.
HR-TOF-MS (Methode 24): C₇₈H₄₁N₂O₇Si [M+H]⁺ gef. 545.2678, ber. 545.2678.

### Beispiel 97A

### (-)-2-(Trimethylsilyl)ethyl-(3R)-N²-[benzyloxycarbonyl]-3-[(tert-butoxycarbonyl)amino]-O-methyl-L-tyrosinat

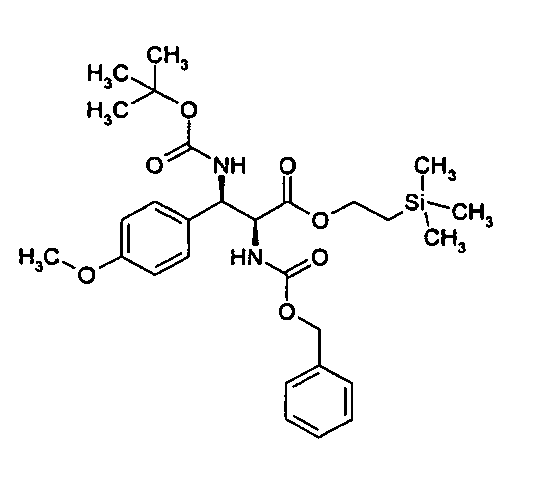

Analog zur der Herstellungsvorschrift für Beispiel 96A wird Beispiel 94A (130 mg, 0.29 mmol) umgesetzt (Reaktionszeit: 12 h). Als Produkt erhält man die Titelverbindung als Feststoff (149 mg, 94% d. Th., >99.5% ee).
HPLC/UV-Vis (Methode 16): Rₜ = 7.2 min.
[α]²⁰_{Na} = -9° (c = 0.1 in MeOH).
HPLC/UV-Vis (Methode 5): Rₜ = 5.34 min.
HPLC/UV-Vis (Methode 4): Rₜ = 5.32 min.
LC-MS (Methode 18): Rₜ = 3.24 min; MS (ESIpos.): *m*/*z* (%) = 545 (100) [M+H]⁺.

### Beispiel 98A

### (+)-2-(Trimethylsilyl)ethyl-(3S)-3-amino-N²-[benzyloxycarbonyl]-O-methyl-D-tyrosinat-trifluoroacetat

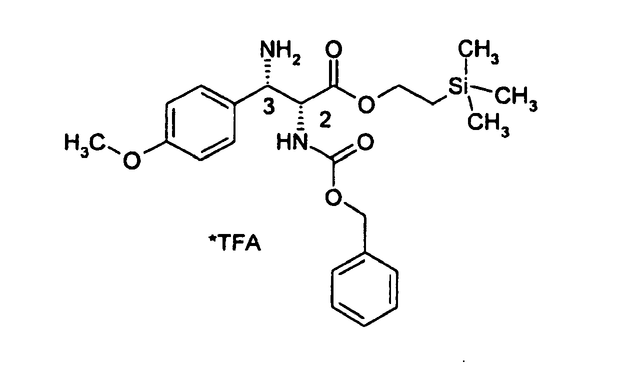

Beispiel 96A (222 mg, 0.41 mmol, 1 Äq.) wird in Dichlormethan suspendiert (3 ml) und anschließend unter Argon-Schutzgasatmosphäre mit Trifluoressigsäure (9 ml) versetzt und 15 min bei RT gerührt bis das HPLC-Chromatogramm vollständigen Umsatz zeigt (Methode 5). Dann wird das Lösungsmittel im Vakuum abdestilliert, wobei die Badtemperatur 30°C nicht übersteigen soll. Das Rohprodukt wird mittels präparativer HPLC (Methode 43) gereinigt. Man erhält 179 mg (79% d. Th., >99.5% ee) der Titelverbindung.
HPLC/UV-Vis (Methode 16): Rₜ = 8.66 min.
[α]²⁰Na = +20° (c = 0.22 in MeOH).
HPLC/UV-Vis (Methode 5): Rₜ = 4.33 min.
HPLC/UV-Vis (Methode 4): Rₜ = 4.55 min.
IR vₘₐₓ (NaCl, cm⁻¹): 2956, 1672, 1613, 1518, 1251, 1181, 1138, 1029.
LC-MS (Methode 18): Rₜ = 2.18 min; MS (ESIpos.): *m*/*z* (%) = 225 (100), 549 (100) [M+H]⁺.
¹H NMR (500 MHz, *d₆*-DMSO): δ = -0.07 (s, 9H, Si(CH₃)₃), 0.43-0.52 (m, 2H, CH₂Si), 3.75 (s, 3H, OMe), 3.74-3.78 (m, 1H, C*H*₂CH₂Si), 3.82-3.88 (m, 1H, C*H*₂CH₇Si), 4.38-4.42 (m, 1H, β-H), 4.58 (tₐₚₚ, *J* = ca.9.0 Hz, 1H, α-H), 5.08 (s, 2H, PhCH₂O), 6.98 (d, 2H, *J* = 8.5 Hz, ArH), 7.34-7.40 (m, 8H, ArH), 8.07 (d, 1H, *J* = 8.0 Hz, NH), 8.49 (s, br, 2H, 2 NH).
¹³C NMR (125 MHz, *d₆*-DMSO): δ = -1.8 (3C, (Si(CH₃)₃)), 16.3 (CH₂Si), 54.5 (C^{β}), 55.1 (C^{α}, 57.7 (OMe), 62.9 (CH₇CH₂Si), 66.0 (PhCH₂O), 113.9 (2C), 125.3, 127.8 (2C), 127.9, 128.3 (2C), 129.4 (2C), 136.4, 155.8 (CO₂Bn), 159.9, 168.9 (CO₂TMSE).
HR-TOF-MS (Methode 24): C₂₃H₃₃N₂O₅Si [M+H]⁺ gef. 445.2168, ber. 445.2154.

### Beispiel 99A

### (-)-2-(Trimethylsilyl)ethyl-(3R)-3-amino-N²-[benzyloxycarbonyl]-O-methyl-L-tyrosinat-trifluoroacetat

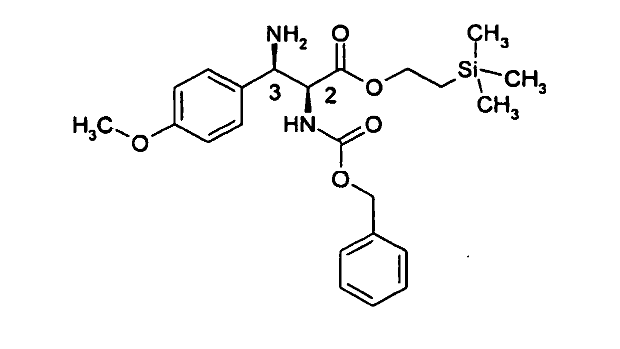

Analog zur der Herstellungsvorschrift für Beispiel 98A wird Beispiel 96A (120 mg, 0.22 mmol) umgesetzt (Reaktionszeit: 50 min). Als Produkt erhält man die Titelverbindung als Feststoff (71 mg, 56% d. Th., >99.5% ee).
HPLC/UV-Vis (Methode 16): Rₜ = 7.49 min.
HPLC/UV-Vis (Methode 5): Rₜ = 4.33 min.
HPLC/UV-Vis (Methode 4): Rₜ = 4.50 min.
IR vₘₐₓ (NaCl, cm⁻¹): 2956, 1672, 1613, 1518, 1251, 1181, 1138, 1029.
HR-TOF-MS (Methode 24): C₂₃H₃₃N₂O₅Si [M+H]⁺ gef. 445.2151, ber. 445.2154.

### Beispiel 100A

### [(3R)-N²-(tert-Butoxycarbonyl)-3-hydroxy-L-leucyl]-L-leucyl-D-arginyl-L-isoleucyl-L-allothreonyl-glycyl-[(3S)-3-hydroxy-L-asparaginyl]-L-serin-trifluoracetat

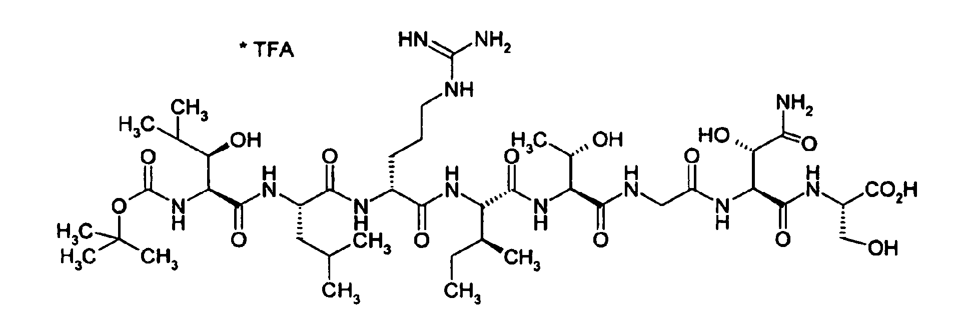

Methode A: Zu einer Lösung des Abbauproduktes aus Beispiel 3A (690 mg, 680 µmol) in Wasser-Dioxan 1:2 (30 ml) werden unter Argonschutzgasatmosphäre Di-*tert*-butyldicarbonat (369 mg, 1.69 mmol, 2.5 Äq.) und *N*-Methylmorpholin (68 mg, 680 µmol, 1 Äq.) gegeben. Das Reaktionsgemisch wird so lange gerührt bis das HPLC-Chromatogramm (Methode 1) vollständigen Umsatz zeigt (ca. 48 h). Das Reaktionsgemisch wird mit Kaliumdihydrogenphosphat (5 Äq.) versetzt, im Vakuum eingeengt und mittels präparativer HPLC (Methode 43 oder Methode 30 gefolgt von anschließendem Umsalzen des Chromatographieproduktes durch Zusatz von TFA (2000 µmol, als 0.05%ige Lösung in Acetonitil-Wasser 1:1)) gereinigt. Man erhält 531 mg (70% d. Th.) der Titelverbindung.

Methode B: Edman*^{3.0}*-Vorstufe-trifluoracetat (100 mg, 0.1 mmol, 1 Äq.) wird zuerst gelchromatographiert (Methode 45, MeOH, 0.2% TFA). Zu einer Lösung dieses Materials in Wasser (1 ml) und Dioxan (2 ml) wird bei RT Di-*tert*-butyl-dicarbonat (43 mg, 0.2 mmol, 2 Äq.) gegeben und NMM (21.6 µL, 0.2 mmol, 2 Äq.) getropft. Das Reaktionsgemisch wird bei RT solange (ca. 12 h) gerührt, bis die analytische HPLC-Kontrolle (Methode 8) ausreichenden Umsatz anzeigt (>99%). Kaliumdihydrogenphosphat (67 mg, 0.49 mmol, 5 Äq.) wird zugegeben, das Reaktionsgemisch wird einrotiert und dann mittels präparativer HPLC (Methode 26) feingereinigt. Man erhält 83 mg (76% d. Th.) der Titelverbindung.
[a]²⁰_{Na} = -18.0° (c = 0.19 in Methanol).
HPLC/UV-Vis (Methode 2): Rₜ = 1.7 min.
HPLC/UV-Vis (Methode 5): Rₜ = 3.57 min.
HPLC/UV-Vis (Methode 4): Rₜ = 3.79 min.
LC-MS (Methode 18): Rₜ = 1.70 min; MS (ESIpos.): *m*/*z* (%)=453 (100), 1005 (80) [M+H]⁺; MS (ESIneg.): *m*/*z* (%) = 1003 (100) [M - H]⁻.
LC-MS (Methode 23): Rₜ = 4.6 min; MS (ESIpos.): *m*/*z* (%) = 453.5 (60) [M - C₄H₈ - CO₂ + 2H]²⁺, 1006 (100) [M+H]⁺; MS (ESIneg.): *m*/*z* (%) = 1004 (100) [M - H]⁻.
IR νₘₐₓ (NaCl, cm⁻¹): 3288, 2966, 2359, 2341, 1645, 1520, 1368, 1160, 1137, 1054.
¹H NMR (500 MHz, *d*₅-Pyridin) δ = 9.72 (br. s, 1H, NH-Glu⁶), 9.31 (br d, *J* = ca. 7.0 Hz, 1H, NH-Arg³), 9.23 (d, *J* = 6.0 Hz, 1H, NH-Leu²), 9.19 (br. s, 1H, εNH-Arg³), 9.00 (d, *J* = 9.0 Hz, 1H, NH-βOH-Asn⁷), 8.88 (d, *J* = 7.0 Hz, 1H, NH-Thr⁵), 8.78 (d, *J* = 7.0 Hz, 1H, NH-Ile⁴), 8.64 (d, *J* = 7.5 Hz, 1H, NH-Ser⁸), 8.40 (br. s, 1H, NH₂-βOH-Asn⁷), 8.34 (br. s, 1H, NH₂-βOH-Asn⁷), 5.96 (d, *J* = 8.0 Hz, 1H, αCH-βOH-ASn⁷), 5.51 (s, 1H, βCH-βOH-Asn⁷), 5.11-5.09 (m, 1H, αCH-Ser⁸), 5.04 (t, *J* = 7.5 Hz, 1H, αCH-Thr⁵), 4.95-4.91 (m, 3H, αCH-Arg³ und αCH-βOH-Leu¹ und αCH-Leu²), 4.82 (t, *J* = 7.0 Hz, 1H, αCH-Ile⁴), 4.71-4.66 (m, 1H, βCH-Thr⁵), 4.44 (dd, *J* = 4.5 und 10.5 Hz, 1H, βCH-Ser⁸), 4.31-4.26 (m, 3H, βCH-Ser⁸ und βCH-βOH-Leu¹ und αCH-Gly⁶), 4.20 (dd, *J* = 6.0 und 16.5 Hz, 1H, αCH-Gly⁶), 3.43 (br d, *J* = ca. 6.0 Hz, 2H, δ CH-Arg³), 2.39-2.36 (m, 2H, βCH-Arg³ und βCH-Ile⁴), 2.27-2.20 (m, 1H, βCH-Arg³), 2.10-2.05 (m, 2H, γCH-βOH-Leu¹ und γCH-Arg³), 1.93-1.79 (m, 4H, βCH-Leu², γCH-Leu², γCH-Arg³ und γCH-Ile⁴), 1.55 (d, *J* = = 6.0 Hz, 3H, γCH₃-Thr⁵), 1.45 (s, 9H, O-tBu), ca. 1.47 (hidden, 1H, γCH-Ile⁴) 1.17-1.15 (m, 4H, δCH₃-βOH-Leu¹ und γCH-Ile⁴), 1.05 (d, *J* = 6.0 Hz, 3H, δCH₃-βOH-Leu¹), 1.05 (t, *J* = 7.0 Hz, 3H, δCH₃-Ile⁴), 0.80 (d, *J* = 6.0 Hz, 3H, δCH₃-Leu²), 0.78 (d, *J* = 6.0 Hz, 3H, δCH₃-Leu²).
¹³C NMR (125 MHz, *d₅*-Pyridin) δ = 175.58 (γCO-βOH-Asn⁷), 174.23 (CO-βOH-Leu¹), 173.97 (CO-Arg³), 173.89 (CO-Ser⁸ oder CO-Leu², 173.69 (CO-Thr⁵), 173.55 (CO-Ser⁸ oder CO-Leu²), 172.48 (CO-Ile⁴), 170.90 (CO-βOH-Asn⁷), 170.50 (CO-Gly⁶), 158.67 (ζC-Arg³), 157.06 (CO-Boc), 79.13 (C-Boc), 77.09 (βCH-βOH-Leu¹), 72.29 (βCH-βOH-Asn⁷), 68.32 (βCH-Thr⁵), 63.24 (βCH-Ser⁸), 59.79 (αCH-Ile⁴), 59.60 (αCH-Thr⁵), 58.35 (αCH-βOH-Leu¹), 57.11 (αCH-βOH-Asn⁷), 56.94 (αCH-Ser⁸), 54.06 (αCH-Arg³), 53.60 (αCH-Leu²), 44.32 (αCH₂-Gly⁶), 41.75 (δCH₂-Arg³), 40.41 (βCH₂-Leu²), 36.70 (βCH-Ile⁴), 31.56 (γCH-βOH-Leu¹), 29.47 (βCH₂-Arg³), 28.48 (Me₃-Boc), 26.11 (γCH-Arg³), 25.74 (γCH₂-Ile⁴), 24.93 (γCH-Leu²), 22.94 (δCH₃-Leu²), 21.71 (δCH₃-Leu²), 21.20 (γCH-Thr⁵), 19.48 (δCH₃-βOH-Leu¹), 19.43 (δCH₃-βOH-Leu¹), 16.02 (γCH-Ile⁴), 11.20 (δCH₃-Ile⁴).
HR-TOF-MS (Methode 24): C₄₂H₇₇N₁₂O₁₆ [M+H]⁺ gef. 1005.5560, ber. 1005.5576.

### Beispiel 101A

### N²-(Benzyloxycarbonyl)-N³-[-N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3S)-3-amino-O-methyl-D-tyrosin-(2-(trimethylsilyl)ethyl)ester-trifluoracetat

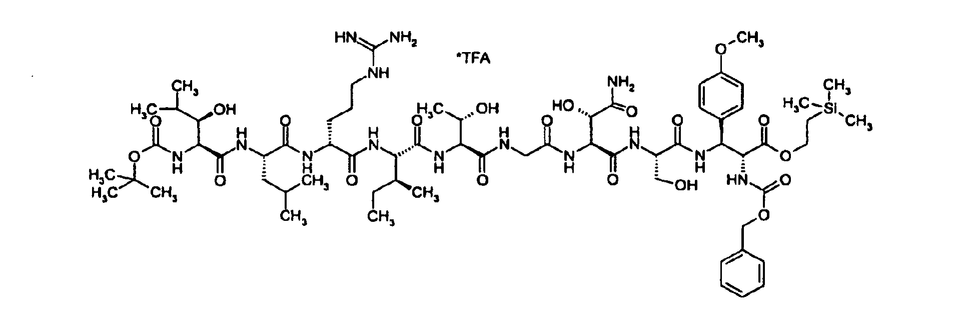

Zu einer Lösung aus Beispiel 98A (1.2 Äq., 50 mg, 89 µmol), der Octapeptidsäure (Beispiel 100A, 1.0 Äq., 83 mg, 75 µmol) und *N*-Methylmorpholin (3.5 Äq., 29 µmol) in trockenem Dimethylformamid (0.5 ml) wird bei 0°C unter Argon-Schutzgasatmosphäre zunächst HATU (2.5 Äq., 71 mg, 186 µmol) gegeben. Das Reaktionsgemisch wird bei 0°C gerührt (ca. 3 h). Das Reaktionsgemisch zeigt dann vollständigen Umsatz der Amin-Komponente (HPLC-Kontrolle, Methode 5). Das Reaktionsgemisch wird mit festem Kaliumdihydrogenphosphat (101 mg, 746 µmol, 10 Äq.) versetzt, filtriert und dann im Hochvakuum eingedampft und chromatographisch aufgereinigt (Methode 43). Man erhält 96.5 mg (84% d. Th.) Produkt.
[α]²⁰_{Na} = -6° (c = 0.04 in Methanol).
HPLC/UV-Vis (Methode 5): Rₜ = 4.47 min.
HPLC/UV-Vis (Methode 4): Rₜ = 4.88 min.
LC-MS (Methode 18): Rₜ = 2.26 min; MS (ESIpos.): *m*/*z* (%) = 652 (100), 1432 (50) [M+H]⁺; MS (ESIneg.): *m*/*z* (%) = 1430 (100) [M - H]⁻.
HR-TOF-MS (Methode 24): C₆₅H₁₀₇N₁₄O₂₀Si [M+H]⁺ gef. 1431.7570, ber. 1431.7550.

### Beispiel 102A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3R)-3-amino-O-methyl-L-tyrosin-(2-(trimethylsilyl)ethyl)ester-trifluoracetat

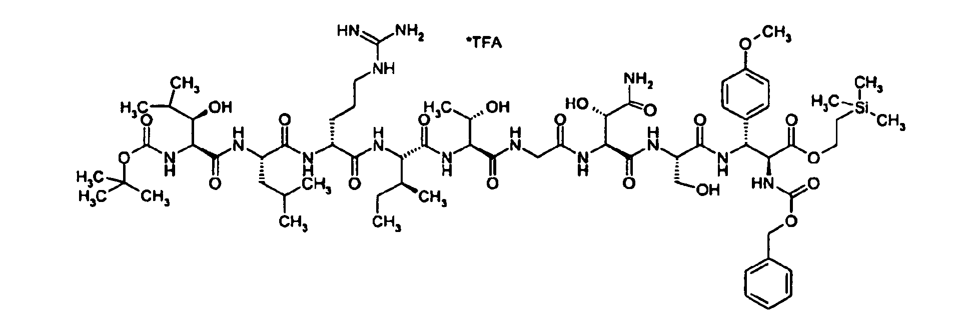

Analog zur der Herstellungsvorschrift für Beispiel 101A wird Beispiel 99A (65 mg, 0.116 mmol) umgesetzt (Reaktionszeit: 150 min). Als Produkt erhält man die Titelverbindung als Feststoff (111 mg, 74% d. Th.).
[α]²⁰_{Na} = -17° (c = 0.09 in Methanol).
HPLC/UV-Vis (Methode 5): Rₜ = 4.47 min.
HPLC/UV-Vis (Methode 4): Rₜ = 4.87 min.
LC-MS (Methode 18): Rₜ = 2.16 min; MS (ESIpos.): *m*/*z* (%) = 652 (100), 1432 (60) [M+H]⁺; MS (ESIneg.): *m*/*z* (%) = 1430 (100) [M - H]⁻.
HR-TOF-MS (Methode 24): C₆₅H₁₀₇Ni₁₄O₂₀Si [M+H]⁺ gef. 1431.7509, ber. 1431.7550.

### Beispiel 103A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3S)-3-amino-O-methyl-D-tyrosin-trifluoracetat

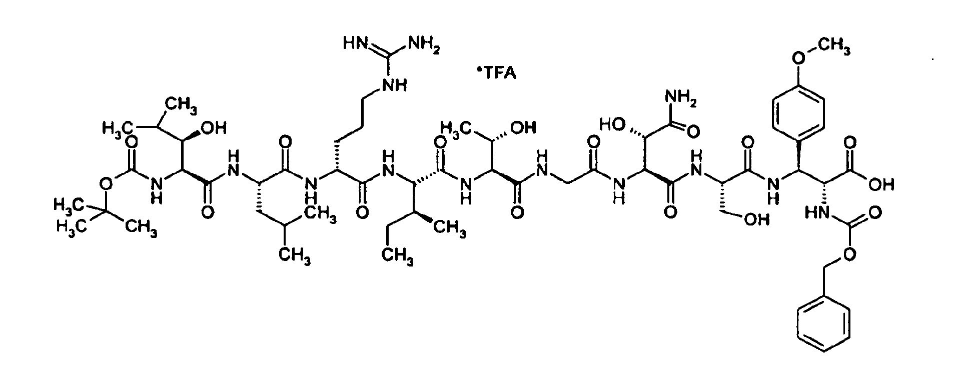

Beispiel 101A (96 mg, 62 µmol) wird in trockenem THF (4 ml) unter Argonschutzgasatmosphäre vorgelegt. Bei RT wird unter starkem Rühren eine 1 N TBAF-THF-Lösung (310 µl, 5 Äq.) zugetropft. Weiteren Portione TBAF (je 5 Äq.) werden in regelmässigen Abständen von 30 min zugegeben. Nach ca. 45 Äq. zeigt das HPLC-Chromatogramm (Methode 5) vollständigen Umsatz. Das Reaktionsgemisch wird nun mit Kaliumdihydrogenphosphat neutralisiert (253 mg, ca. 30 Äq.). Das Reaktionsgemisch wird filtriert, im Vakuum eingedampft und mittels präparativer HPLC (Methode 43) gereinigt. Man erhält 83 mg (93% d. Th.) der Titelverbindung.
HPLC/UV-Vis (Methode 5): Rₜ = 3.93 min.
HPLC/UV-Vis (Methode 4): Rₜ = 4.27 min.
LC-MS (Methode 18): Rₜ = 2.00 min; MS (ESIpos.): *m*/*z* (%) =616 (100), 1332 (20) [M+H]⁺; MS (ESIneg.): *m*/*z* (%) = 1330 (100) [M - H]⁻.
HR-TOF-MS (Methode 24): C₆₀H₉₅N₁₄O₂₀ [M+H]⁺ gef. 1331.6844, ber. 1331.6842.

### Beispiel 104A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3R)-3-amino-O-methyl-L-tyrosin-trifluoracetat

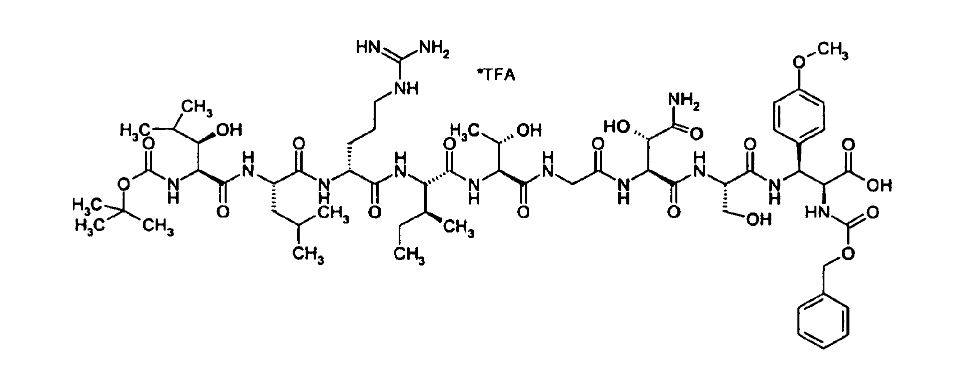

Analog zur der Herstellungsvorschrift für Beispiel 103A wird Beispiel 102A (105 mg, 0.068 mmol) umgesetzt (insgesamt 45 Äq. TBAF). Als Produkt erhält man der Titelverbindung als Feststoff (90 mg, 99% d. Th.).
[α]²⁰_{Na} = -9° (c = 0.135 in Methanol).
HPLC/UV-Vis (Methode 5): Rₜ = 3.93 min.
HPLC/UV-Vis (Methode 4): Rₜ = 4.15 min.
LC-MS (Methode 20): Rₜ = 1.80 min; MS (ESIpos.): *m*/*z* (%) = 616 (100), 1332 (40) [M+H]⁺; MS (ESIneg.): *m*/*z* (%) = 664 (60), 1330 (100) [M - H]⁻.
HR-TOF-MS (Methode 24): C₆₀H₉₅N₁₄O₂₀ [M+H]⁺ gef. 1331.6840, ber. 1331.6842.

### Beispiel 105A

### N²-(Benzyloxycarbonyl)-N³-{[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3S)-3-amino-O-methyl-D-tyrosin-trifluoracetat

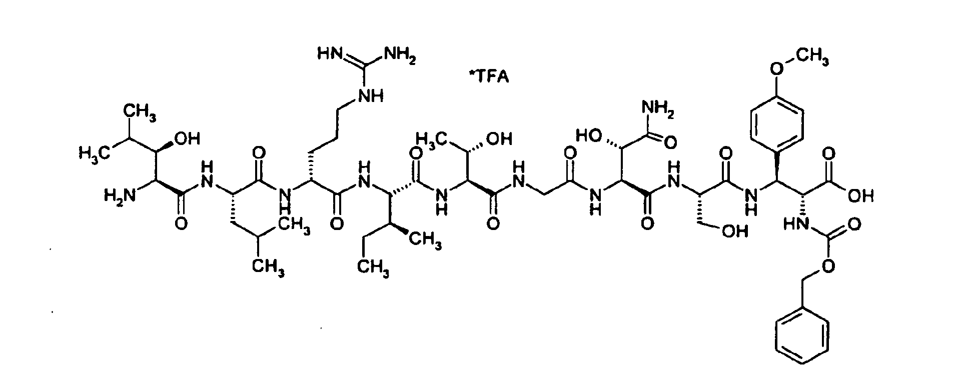

Gemäß der Arbeitsvorschrift 1 wird das Beispiel 103A (10 mg, 7 µmol) unter Argonschutzgasatmosphäre umgesetzt. Man erhält nach der Gefriertrocknung 9.3 mg (99.9% d. Th.) der Titelverbindung.
HPLC/UV-Vis (Methode 5): Rₜ = 3.47 min.
LC-MS (Methode 18): Rₜ = 1.50 min; MS (ESIpos.): *m*/*z* (%) = 616 (100), 1232 (5) [M+H]⁺; MS (ESIneg.): *m*/*z* (%) = 1230 (100) [M - H]⁻.
HR-TOF-MS (Methode 24): C₅₅H₈₇N₁₄O₁₈ [M+H]⁺ gef. 1231.6300, ber. 1231.6318.

### Beispiel 106A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl)-(3R)-3-amino-O-methyl-L-tyrosin

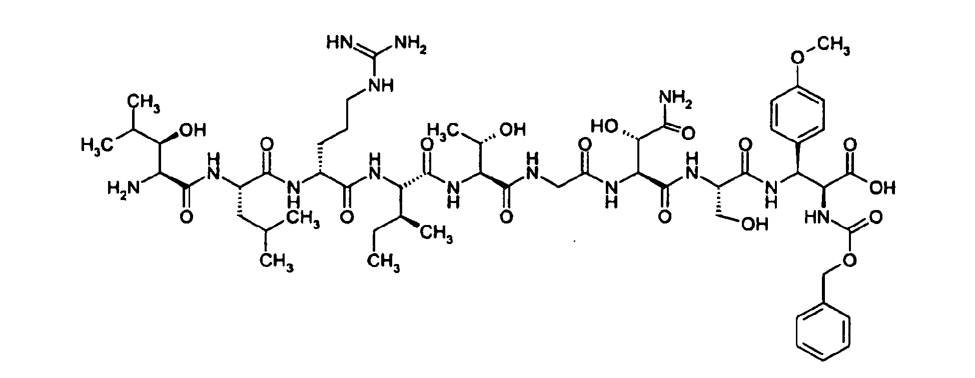

Gemäß der Arbeitsvorschrift 1 wird das Beispiel 104A (240 mg, 166 µmol) unter Argonschutzgasatmosphäre umgesetzt. Man erhält nach der Gefriertrocknung 116 mg (57% d. Th.) der Titelverbindung.
[α]²⁰_{Na} = -24° (*c* = 0.07 in Methanol).
HPLC/UV-Vis (Methode 5): Rₜ = 3.47 min.
HPLC/UV-Vis (Methode 4): Rₜ = 3.83 min.
LC-MS (Methode 18): Rₜ = 1.51 min; MS (ESIpos.): *m*/*z* (%) = 616 (100), 1232 (5) [M+H]⁺; MS (ESIneg.): *m*/*z* (%) = 1230 (100) [M - H]⁻.
HR-TOF-MS (Methode 24): C₅₅H₈₇N₁₄O₁₈ [M+H]⁺ gef. 1231.6361, ber. 1231.6318.

### Beispiel 107A

### N^{2.1}-(Benzyloxycarbonyl)-[(3S)-3-amino-O-methyl-D-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-trifluoracetat

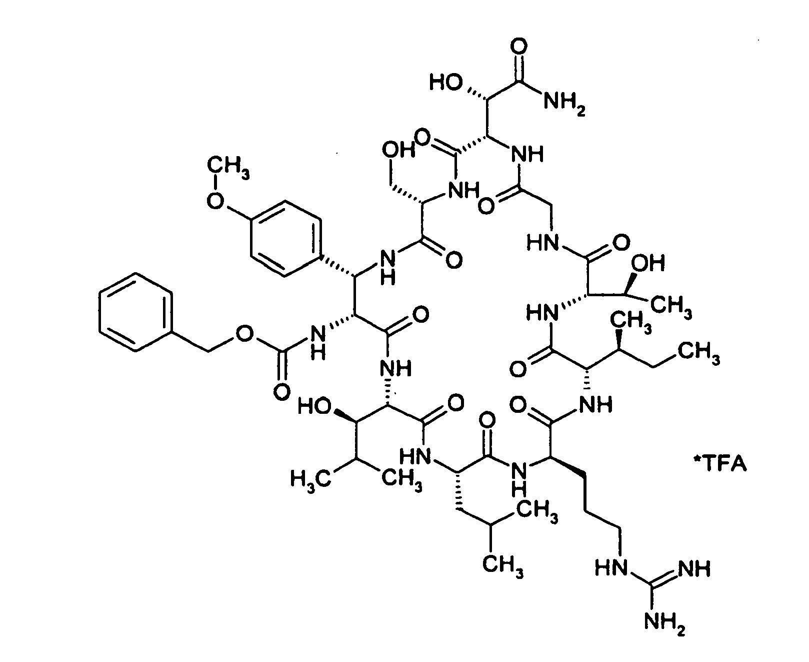

Zu einer Lösung von Beispiel 105A (1.9 mg, 2 µmol, 1 Äq.) und NMM (1.7 µL, 15 µmol, 10 Äq.) in trockenem DMF (200 µl) wird bei 0°C unter Argonschutzgasatmosphäre zunächst HATU (1.5 mg, 8 µmol, 5 Äq.) zugegeben. Das Reaktionsgemisch wird bei 0°C gerührt (ca. 90 min). Das Reaktionsgemisch zeigt dann vollständigen Umsatz der Amin-Komponente (HPLC-Kontrolle, Methode 5). Das Reaktionsgemisch wird mit festem Kaliumdihydrogenphosphat (20 Äq., 4.2 mg, 31 µmol) versetzt, filtriert und dann im Hochvakuum eingedampft und chromatographisch aufgereinigt (Methode 45). Man erhält 1.8 mg (96% d. Th.) Produkt.
HPLC/UV-Vis (Methode 5): Rₜ = 3.65 min.
HPLC/UV-Vis (Methode 4): Rₜ = 3.91 min.
LC-MS (Methode 18): Rₜ = 1.67 min; MS (ESIpos.): *m*/*z* (%) = 1214 (100) [M+H]⁺.
HR-TOF-MS (Methode 24): C₅₅H₈₅N₁₄O₁₇ [M+H]⁺ gef. 1213.6232, ber. 1213.6312.

### Beispiel 108A

### N^{2.1}-(Benzyloxycarbonyl)-[(3R)-3-amino-O-methyl-L-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-trifluoracetat

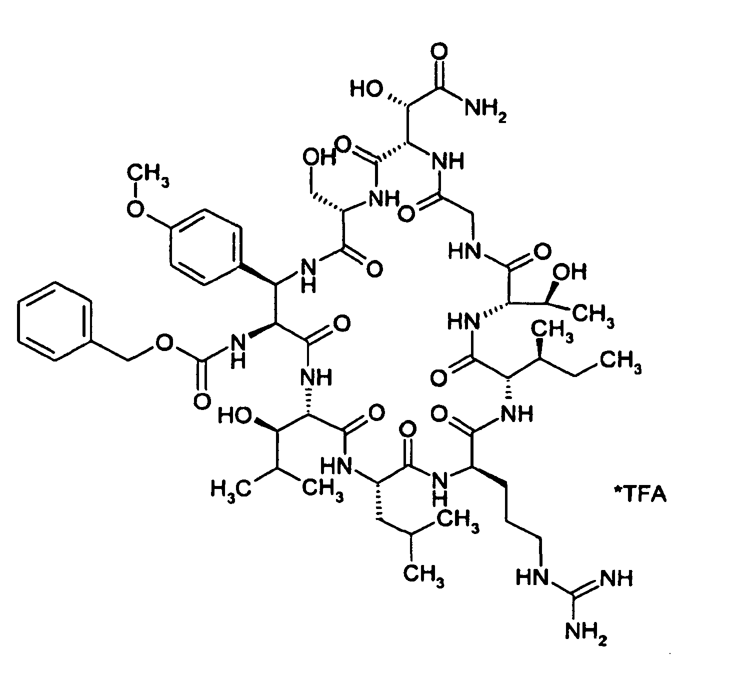

Analog zur der Herstellungsvorschrift für Beispiel 107A wird Beispiel 106A (120 mg, 0.097 mmol) umgesetzt (Reaktionszeit 1 h). Man erhält die Titelverbindung als Feststoff (39 mg, 33% d. Th.).
[α]²⁰_{Na} = -58° (c = 0.07 in Methanol).
HPLC/UV-Vis (Methode 5): Rₜ = 3.71 min.
HPLC/UV-Vis (Methode 4): Rₜ = 3.98 min.
LC-MS (Methode 18): Rₜ = 1.74 min; MS (ESIpos.): *m*/*z* (%) = 1214 (100) [M+H]⁺; MS (ESIneg.): *m*/*z* (%) = 1212 (100) [M - H]⁻.
HR-TOF-MS (Methode 24): C₅₅H₈₅N₁₄O₁₇ [M+H]⁺ gef. 1213.6227, ber. 1213.6312.

### Beispiel 109A

### [(3S)-3-Amino-O-methyl-D-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-bistrifluoroacetat

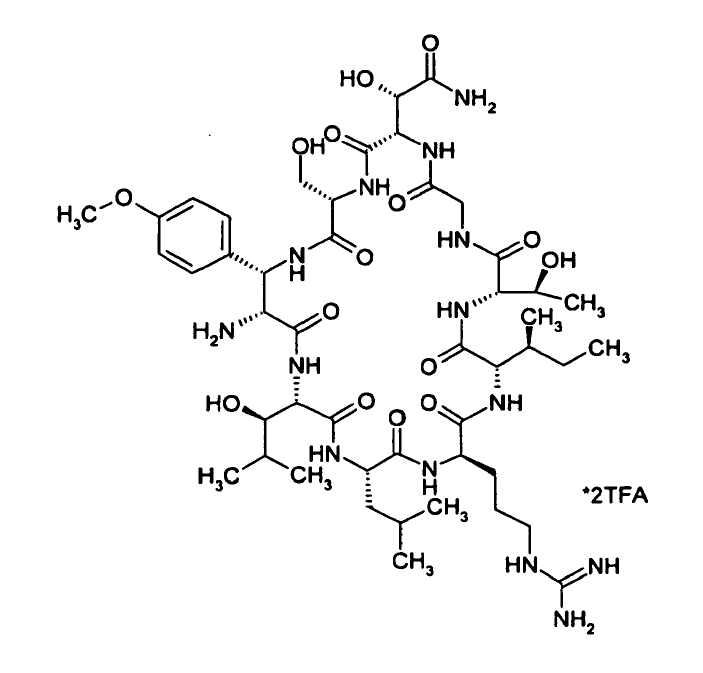

Das Beispiel 107A (65 mg, 49 µmol) wird in Methanol (5 ml) gelöst und unter Argonschutzgasatmosphäre mit 10proz. Palladium-Kohle (10 mg) sowie wässriger 1 N Salzsäure (250 µl) versetzt. Bei RT und Normaldruck wird so lange hydriert (ca. 90 min) bis die analytische HPLC (Methode 5) vollständigen Umsatz anzeigt. Das Reaktionsgemisch wird filtriert (über Kieselgur, Celite^{®} oder einem Spritzenfilter, Fa. Biotage, PTFE), im Vakuum eingeengt und chromatographisch aufgereinigt (Methode 43). Man erhält 40.6 mg (63% d. Th.) der Titelverbindung.
[α]²⁰_{Na} = -21° (c = 0.06 in Methanol).
HPLC/UV-Vis (Methode 5): Rₜ = 3.28 min.
HPLC/UV-Vis (Methode 4): Rₜ = 3.55 min.
LC-MS (Methode 18): Rₜ = 1.20 min; MS (ESIpos.): *m*/*z* (%) = 540 (100), 1079 (20) [M+H]⁺; MS (ESIneg.): *m*/*z* (%) = 1077 (100) [M - H]⁻.
HR-TOF-MS (Methode 24): C₄₇H₇₈N₁₄O₁₅ [M+H]⁺ gef. 1079.5835, ber. 1079.5844.

### Beispiel 110A

### [(3R)-3-Amino-O-methyl-L-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-bistrifluoroacetat

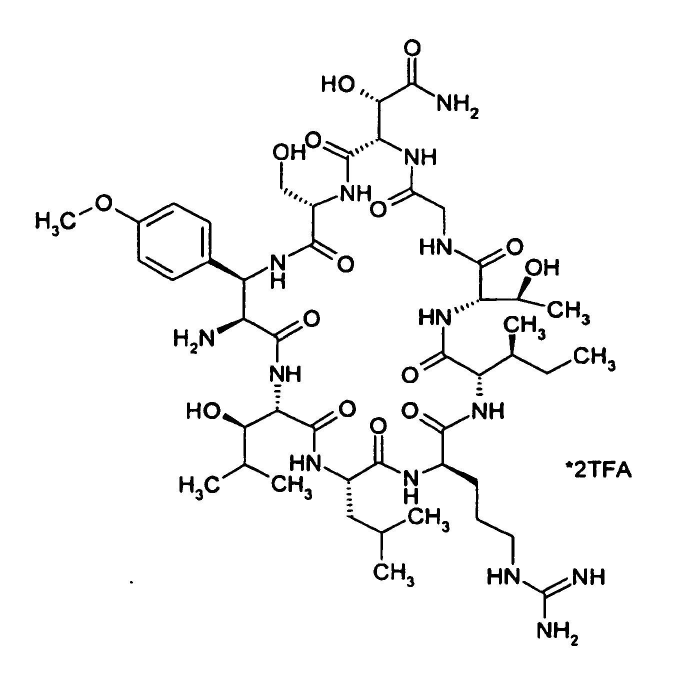

Analog zur der Herstellungsvorschrift für Beispiel 109A wird Beispiel 108A (12 mg, 9 µmol) umgesetzt (Reaktionszeit 90 min). Als Produkt erhält man die Titelverbindung als Feststoff (10.3 mg, 87% d. Th.).
[α]²⁰_{Na} = -52° (c = 0.03 in Methanol).
HPLC/UV-Vis (Methode 5): Rₜ = 3.18 min.
HPLC/UV-Vis (Methode 4): Rₜ = 3.52 min.
LC-MS (Methode 18): Rₜ = 1.25 min; MS (ESIpos.): *m*/*z* (%) = 540 (100), 1079 (5) [M+H]⁺; MS (ESIneg.): *m*/*z* (%) = 1077 (100) [M - H]⁻.

### Beispiel 111A

### [N²-(Benzyloxycarbonyl)-3-tert-butyl-D-alanyl]-[3-tert-butyl-L-alanyl]-[(3S)-3-amino-O-methyl-D-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam-trifluoracetat

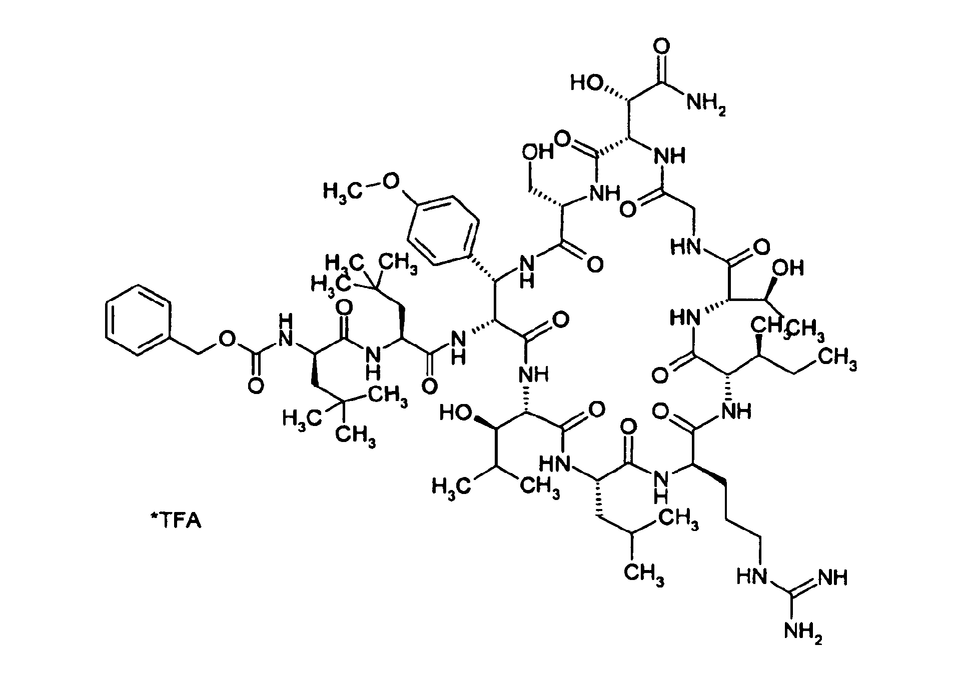

Zu einer Lösung des Beispiels 109A (3.0 mg, 2 µmol, 1.0 Äq.), der Dipeptidsäure (Beispiel 10A, 2.3 mg, 6 µmol, 2.5 Äq.) und NMM (11 µmol, 5 Äq.) in trockenem DMF (1 ml) wird bei -30°C unter Argonschutzgasatmosphäre zunächst HATU (2.2 mg, 6 µmol, 2.5 Äq.) gegeben. Das Reaktionsgemisch wird gerührt und erwärmt sich langsam (ca. 1 h) auf 0°C. Das Reaktionsgemisch wird in regelmäßigen Abständen (30 min) mit NMM (6 µmol, je 2.5 Äq.) und HATU (6 µmol, je 2.5 Äq.) versetzt, bis die HPLC-Kontrolle (Methode 5) vollständigen Umsatz der Amin-Komponente anzeigt. Insgesamt ca. 20 Äq. NMM und HATU werden hierfür benötigt. Das Reaktionsgemisch wird mit festem Kaliumdihydrogenphosphat (9.4 mg, 69 µmol, 30 Äq.) versetzt, filtriert und dann im Hochvakuum eingedampft und chromatographisch aufgereinigt (Methode 43). Man erhält 2 mg (55% d. Th.) Produkt.
HPLC/UV-Vis (Methode 5): Rₜ = 4.2 min.
HPLC/UV-Vis (Methode 4): Rₜ = 4.49 min.
LC-MS (Methode 18): Rₜ = 2.07 min; MS (ESIpos.): *m*/*z* (%) = 734 (100) [M + 2H]²⁺, 1468 (20) [M+H]⁺; MS (ESIneg.): *m*/*z* (%) = 678 (100), 732 (5) [M - 2H]²⁻, 1466 (10) [M - H]⁻.
HR-TOF-MS (Methode 24): C₆₉H₁₁₁N₁₆O₁₉ [M+H]⁺ gef. 1467.8228, ber. 1467.8206.

### Beispiel 112A

### [N²-(Benzyloxycarbonyl)-3-tert-butyl-D-alanyl]-[3-tert-butyl-L-alanyl]-[(3R)-3-amino-O-methyl-L-tyrosyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam-trifluoracetat

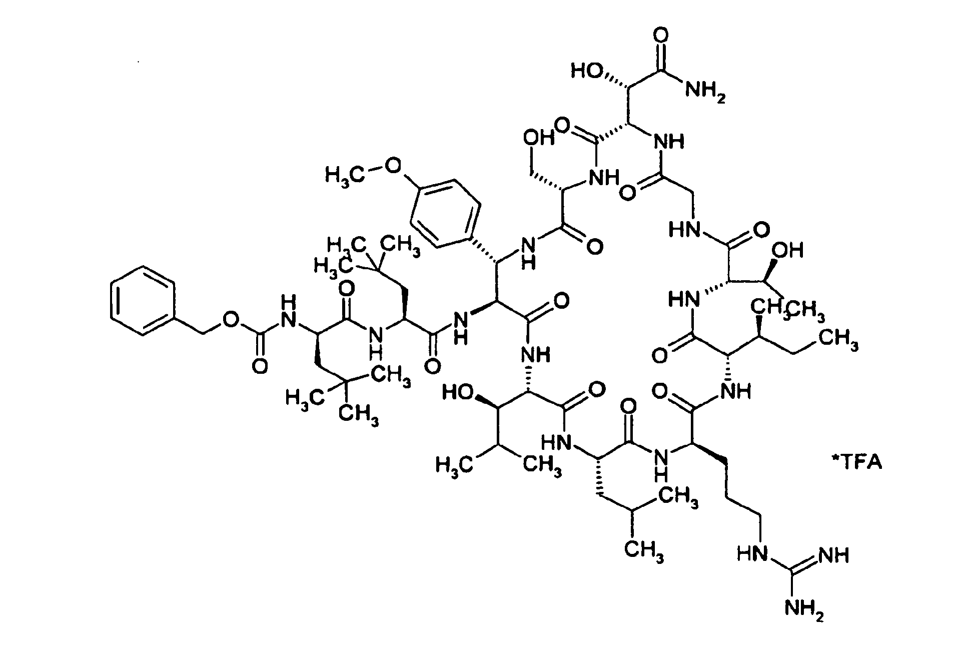

Herstellung analog Beispiel 111A aus der Verbindung aus Beispiel 109A (30 mg, 23 µmol) und Beispielverbindung 10A (23.3 mg, 57 µmol, 2.5 Äq.). Das Rohprodukt wird chromatographisch (Methode 43) aufgereinigt, wobei man nach Gefriertrocknung 25 mg (69% d. Th.) Produkt als Feststoff erhält.
[α]²⁰_{Na} = -62° (c = 0.06 in Methanol).
HPLC/UV-Vis (Methode 5): Rₜ = 4.4 min.
LC-MS (Methode 18): Rₜ = 2.31 min.
MS (ESIpos.): *m*/*z* (%) = 734 (100) [M + 2H]²⁺, 1468 (20) [M+H]⁺;
MS (ESIneg.): *m*/*z* (%) = 678 (70), 733 (100) [M - 2H]²⁻, 1466 (10) [M - H]⁻.
HR-TOF-MS (Methode 24): C₆₉H₁₁₁N₁₆O₁₉ [M+H]⁺ gef. 1467.8206, ber. 1467.8206.

### Beispiel 113A

### 3-Amino-3-(4-bromphenyl)-propionsäure

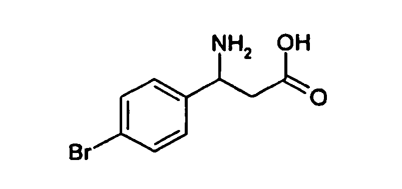

4-Brombenzaldehyd (50.0 g, 270 mmol), Malonsäure (28.1 g, 270 mmol) und Ammoniumacetat (27.7 g, 359 mmol, 1.3 Äq.) werden in Ethanol (400 ml) gelöst. Die Mischung wird 16 h zum Rückfluss erhitzt. Der enstandene Feststoff wird auf einem Glasfiltertiegel gesammelt, abgesaugt und mit Ethanol gewaschen. Dann wird aus Methanol (80 ml) umkristallisiert. Im Anschluss wird mit Ethylacetat verrieben und abgesaugt. Man erhält 41.0 g (0.16 mmol, 62% d. Th.) der Titelverbindung als farblosen kristallinen Feststoff.
HPLC (Methode 5): Rₜ = 3.13 min.
LC-MS (Methode 20): Rₜ = 0.97 min, MS (ESIneg.): *m*/*z* (%) = 242.0 (100) und 244.0 (80) [M - H]⁻.
¹H NMR (300 MHz, *d₆*-DMSO) δ = 2.36-2.38 (m, 2H, 2-H), 4.25 ("t", *J* = 6.8, 7.6 Hz, 2H, 3-H), 6.56 (d, *J* = 16.1 Hz, 1H, N-*H*), 7.38 (d, J = 8.5 Hz, 1H, N-*H*), 7.52-7.71 (m, 4H, arom. H).
¹³C-NMR (126 MHz, DCOOD) δ = 36.5 (CH₂, C-2), 51.9 (CH, C-3), 123.7 (C quat), 129.2 (CH), 132.4 (CH), 133.5 (C quat), 174,0 (C quat, C-1).
HR-TOF-MS (Methode 24): C₉H₁₁NO₂Br [M+H]⁺ ber. 243.9968, gef. 243.9980.

### Beispiel 114A

### Methyl 3-Amino-3-(4-bromphenyl)-propionat-hydrochlorid

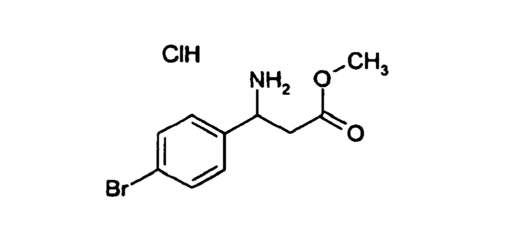

41.0 g (157 mmol) der Verbindung 113A werden in Methanol (1000 ml) unter Argonschutzgasatmosphäre suspendiert. 102 ml (410 mmol, 2.6 Äq.) 4 M Salzsäure in 1,4-Dioxan wird zugegeben. Das Edukt löst sich spontan bei Zugabe der Salzsäure, der Ansatz wird bei RT über Nacht gerührt. Dann wird im Vakuum zur Trockne eingeengt. Das Produkt (51.8 g, Reinheit ca. 69%, Ausbeute 77% d. Th.) wird als Feststoff erhalten und ohne weitere Reinigung im nächsten Schritt eingesetzt.
HPLC (Methode 5): Rₜ = 3.35 min.
LC-MS (Methode 20): Rₜ = 1.24 min, MS (ESIpos.): *m*/*z* (%) = 240.9 (20) und 242.8 (20) [M - NH₂ + H]⁺.
¹H NMR (300 MHz, *d₆*-DMSO) δ = 2.99 (dd, *J*_{2,3} = 8.7 Hz, *J*_{2,2} = 16.4 Hz, 1H, 2-H_{A}), 3.16 (dd, *J*_{2,3} = 4.0 Hz, *J*_{2,2} = 16.4 Hz, 1H, 2-H₈), 3.56 (s, 3H, OC*H*₃), 4.62 (br. s, 1H, 3-H), 7.49 (d, *J* = 8.5 Hz, 2H, 2'-H), 7.65 (d, *J* = 8.5 Hz, 2H, 2'-H), 8.64 (br. s, 2 H, N*H*₂).
¹³C-NMR (126 MHz, CDCl₃) δ = 43.8 (CH₂, C-2), 51.8 (CH₃, OCH₃), 52.1 (CH, C-3), 121.2 (C quat, C-4'), 128.0 (CH, C-arom.), 131.7 (CH, C-arom.), 143.6 (C quat, C-1'), 172.2 (C quat, C-1).
HR-TOF-MS(Methode 24): C₁₀H₁₃NO₂Br [M+H]⁺ ber. 258.0125, gef. 258.0119.

### Beispiel 115A

### Methyl N-Butoxycarbonyl-3-amino-3-(4-bromphenyl)-propionat

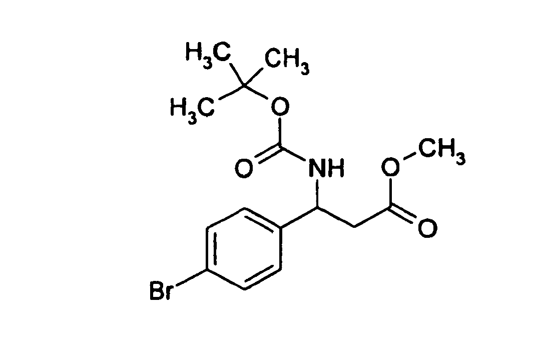

Verbindung 114A (51.8 g, 121 mmol, 69% Reinheit) werden in 1,4-Dioxan/Wasser (2:1, 540 ml) gelöst. Di-*tert*-butyldicarbonat (29.1 g, 133 mmol, 1.1 Äq.) und Triethylamin (38.8 ml, 28.22 g, 2.3 Äq.) werden zugesetzt und die Mischung wird bei RT über 150 min gerührt. Das Dioxan wird im Vakuum abdestilliert und 150 ml Ethylacetat sowie 200 ml ges. wässr. Natriumhydrogencarbonatlösung werden zugegeben. Ein Teil des Produkts 115A fällt spontan aus und wird durch Filtration isoliert. Die Phasen des Filtrates werden getrennt, die organische Phase mit 0.5 M Zitronensäure gewaschen, über Natriumsulfat getrocknet und eingeengt. Aus dem Rückstand wird durch Anreiben mit Methanol eine weitere Fraktion 115A gewonnen. Gesamtausbeute: 32.9 g (92 mmol, 76% d. Th.) der Titelverbindung als Feststoff.
HPLC (Methode 5): Rₜ = 4.80 min.
LC-MS (Methode 20): Rₜ = 2.37 min, MS (ESIpos.): *m*/*z* (%) = 358.0 (15) und 360.0 (15) [M+H]⁺.
¹H NMR (300 MHz, *d₆*-DMSO) δ = 1.34 (s, 9H, COC(C*H*₃)₃), 2.62-2.79 (m, 2H, 2-H), 3.55 (s, 3H, OC*H*₃), 4.87 (dd, *J₁* = 7.9 Hz, *J₂* = 15.1 Hz, 1H, 3-H), 7.26 (d, *J_{2',3'}* = 8.3 Hz, 2H, 2'-H), 7.51 (d, *J_{2',3'}* = 8.3 Hz, 2H, 3'-H).
¹³C-NMR (126 MHz, CDCl₃) δ = 28.3 (CH₃, COC(CH₃)₃), 40.4 (CH₂, C-2), 50.6 (CH, C-3), 51.9 (CH₃, OCH₃), 79.9 (C quat, COC(CH₃)₃), 121.4 (C quat, C-4'),127.9 (CH, C-arom.), 131.7 (CH, C-arom.), 140.3 (C quat, C-1'), 155.0 (C quat, COC(CH₃)₃, 171.2 (C quat, C-1).
HR-TOF-MS (Methode 24): C₁₅H₂₁NO₄Br [M+H]⁺ ber. 358.0649, gef. 358.0659.

### Beispiel 116A

### Methyl (S)-N-Butoxycarbonyl-3-amino-3-(4-bromphenyl)-propionat

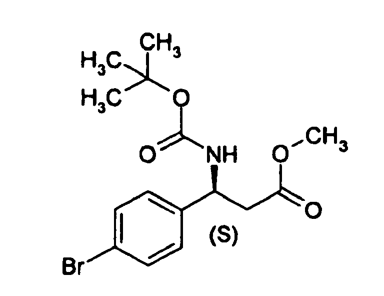

Die racemische Verbindung 115A wird durch chirale HPLC (Methode 42) in die Titelverbindung (96% ee) und das andere Enantiomer (>99% ee) getrennt. Es werden pro Lauf 750 mg 115A aufgegeben. Das (*S*)-Enantiomer (Beispiel 116A) und das (*R*)-Enantiomer werden in jeweils 50% d. Th. quantitativ isoliert.
Enantiomerenbestimmung nach Methode 17.
HPLC/UV-VIS (Methode 17): Rₜ = 4.31 min. (Titelverbindung)
[α]²⁰_{Na} = -38.6 (c = 1.0, MeOH). (Titelverbindung)
HPLC/UV-VIS (Methode 17): Rₜ = 3.82 min. (weiteres Enantiomer)
[α]²⁰_{Na} = + 36.8 (c = 1.0, MeOH). (weiteres Enantiomer)

### Beispiel 117A

### Methyl (S)-N-Butoxycarbonyl-3-amino-3-(4-dimethylaminophenyl)-propionat

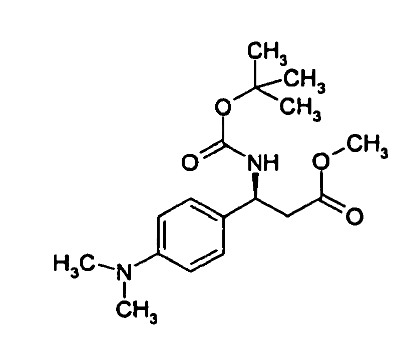

Bei der Durchführung dieser Reaktion ist streng auf den Ausschluss von Sauerstoff und Feuchtigkeit zu achten und die Reagenzien unter einer trocknenen Argonschutzgasatmosphäre halten, da sonst nur unzureichende Umsetzung erfolgt. Weil die Größe der Mikrowellengefäße die Ansatzgröße limitiert, werden größere Mengen auf Portionen von ca. 7 mmol Beispiel 116A aufgeteilt und sequentiell abgearbeitet.

In einem 20 ml Mikrowellen-Reaktionsgefäß wird Beispiel 116A (2.5 g, 6.98 mmol), Dimethylamin (1 M in THF, 16 ml, 32 mmol, 4.6 Äq.), XPHOS (0.4 g, 0.84 mmol, 0.12 Äq.), Cäsiumcarbonat (3.2 g, 9.75 mmol, 1.4 Äq.) und Bis(dibenzylidenaceton)palladium(0) (0.20 g, 0.35 mmol, 0.05 Äq.) unter Argonschutzgasatmosphäre gemischt und zugebördelt. Die Mischung wird im Mikrowellenreaktor (Emrys Optimizer Single-Mode Labormikrowellenreaktor) über 1 h auf 170°C erwärmt. Nach Abkühlen wird zwischen Wasser und Ethylacetat ausgeschüttelt und die organische Phase über Natriumsulfat getrocknet. Das Lösungsmittel wird abgezogen, der Rückstand wird in Methanol gelöst, filtriert und das Filtrat eingeengt. 117A wird in für weitere Umsetzung ausreichender Reinheit und einer Ausbeute von 2.0 g (6.19 mmol, 89% d. Th.) gewonnen.

Für analytische Zwecke wird eine Probe von Beispiel 117A durch präparative HPLC (Methode 33) feingereinigt.
HPLC (Methode 5): Rₜ = 3.60 min.
LC-MS (Methode 20): Rₜ = 1.84 min, MS (ESIpos.): *m*/*z* (%) = 323.3 (100) [M+H]⁺.
[α]²⁰_{Na} = -31.0 (c = 1.0, MeOH).
¹H NMR (400 MHz, *d₆*-DMSO) δ = 1.35 (s, 9H, COC(C*H*₃)₃), 2.63 (dd, *J_{2,3}* = 6.6 Hz, *J_{gem}* = 15.5 Hz, 1H, 2-H_{A}), 2.72 (dd, *J_{2,3}* = 8.8 Hz, *J_{gem}* = 15.5 Hz, 1H, 2-H_{B}), 2.93 (s, 6H, N(C*H*₃)₂), 3.54 (s, 3H, OC*H*₃), 4.84 (m, 1H, 3-H), 6.88 (br. s, 2H, 2'-H), 7.20 (d, *J_{2',3'}* = 8.3 Hz, 2H, 3'-H), 7.36 (d, *J* = 8.8 Hz, 1H, N-*H*).
¹³C-NMR (126 MHz, CDCl₃) δ = 28.3 (CH₃, COC(CH₃)₃), 40.3 (CH₂, C-2), 44.9 (CH₃, N(CH₃)₂), 50.3 (CH, C-3), 52.0 (CH₃, OCH₃), 80.1 (COC(CH₃)₃), 119.1 (CH, C-3'), 128.0 (CH, C-2'), 140.3 (C quat, C-1'), 144.1 (C quat, C-4'), 155.1 (C quat, COC(CH₃)₃), 171.2 (C quat, C-1).
HR-TOF-MS (Methode 24): C₁₇H₂₇N₂O₄ [M+H]⁺ ber. 322.1893, gef. 322.1890.

### Beispiel 118A

Methyl (3*R*)-*N²*-[(benzyloxy)carbonyl]-*N²*-[(benzyloxy)carbonylamino]-3-[(*tert*-butoxycarbonyl)amino]-3-(4-dimethylaminophenyl)-D-alaninat im Gemisch mit Methyl (3*R*)-*N²*-[(benzyloxy)carbonyl]-*N²*-[(benzyloxy)carbonylamino]-3-[(*tert*-butoxycarbonyl)amino]-3-(4-dimethylaminophenyl)-L-alaninat

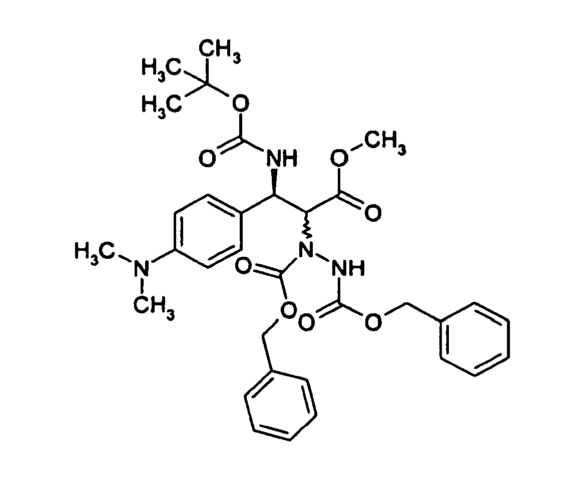

1 M LHMDS-Lösung in THF (21.3 ml, 21.29 mmol, 2.2 Äq.) wird unter Argonschutzgasatmosphäre zu 70 ml abs. THF gegeben und auf -78°C gekühlt. Eine Lösung von Verbindung 117A (3.1 g, 9.68 mmol) in THF (35 ml) wird zugegeben und die Lösung auf -25°C erwärmt, bei welcher Temperatur sie noch 10 min gerührt wird. Dann wird erneut auf -78°C gekühlt, Dibenzylazadicarboxylat (3.2 g, 10.65 mmol, 1.1 Äq.) wird auf einmal zugefügt und der Ansatz wird 3 h gerührt. Am Ende wird mit Essigsäure (2.8 ml, 48.39 mmol, 5 Äq.) bei -78°C abgestoppt und weitere 15 min. bei dieser Temperatur gerührt. Nach dem Erwärmen auf RT wird mit wässriger konz. Natriumhydrogencarbonatlösung versetzt und mit mehreren Portionen Ethylacetat extrahiert. Die vereinigten organischen Extrakte werden mit wässr. ges. Natriumchloridlösung gewaschen, über Natriumsulfat getrocknet und eingeengt. Der orange-braune amorphe Rückstand wird mit Diethylether überschichtet und einige Stunden stehengelassen. Ein Teil des Produktes des Beispiels 118A fällt während dieser Zeit in Form von Kristallen aus. Aus dem Filtrat kann durch Flashchromatographie (Biotage 40M Kieselgel, Cyclohexan/Ethylacetat 3:1) und erneute Kristallisation in Gegenwart von Diethylether das übrige Produkt des Beispiels 118A isoliert werden, das im Gemisch mit dem Diastereomer aus Beispiel 119A anfällt. Gesamtausbeute aller Stereoisomehren und Gemische: 1.5 g (2.48 mmol, 18% d. Th.).

Für analytische Zwecke wird eine Probe reines Diastereomer Beispiel 118A durch präparative HPLC (Methode 33) feingereinigt.
HPLC (Methode 5): Rₜ = 4.52 min.
LC-MS (Methode 20): Rₜ = 2.87 min, MS (ESIpos.): *m*/*z* (%) = 621.5 (50) [M+H]⁺.
[α]²⁰_{Na} = -22.8 (c = 1.0, MeOH).
¹H NMR (400 MHz, *d₆*-DMSO) δ = 1.30 (s, 9H, COC(C*H*₃)₃), 2.86 (s, 6H, N(C*H*₃)₂), 3.30 (s, 3H, OC*H*₃), 4.74-5.11 (m, 6H), 6.62 (d, *J* = 8.8 Hz, 2H), 7.15-7.51 (m, 12H, arom. H).

### Beispiel 119A

### Methyl (3R)-N²-[(benzyloxy)carbonyl]-N²-[(benzyloxy)carbonylamino]-3-[(tert-butoxycarbonyl)amino]-3-(4-dimethylaminophenyl)-L-alaninat

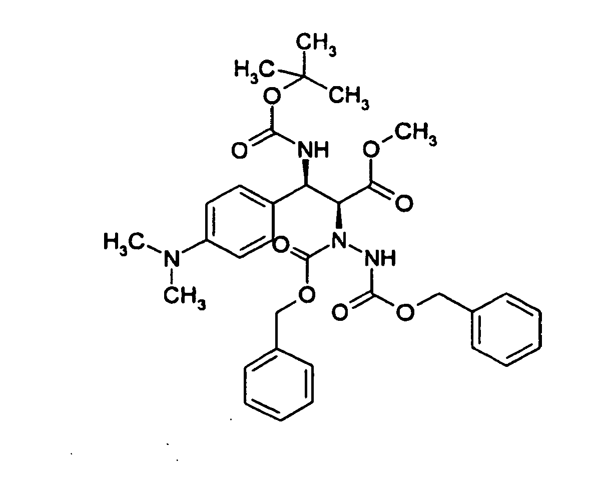

Die Verbindung aus Beispiel 118A (1.6 g, 1.90 mmol) wird in Dichlormethan (60 ml) gelöst. Bei 0°C wird TMG (2.6 ml, 20.93 mmol, 11 Äq.) zugegeben und die Reaktion über Nacht bei RT gerührt. Zum Abstoppen wird der Ansatz zunächst auf 0°C abgekühlt und dann Essigsäure (1.3 ml, 22.83 mmol, 12 Äq.) zugegeben. Man verdünnt mit Wasser und extrahiert mit 3 Portionen Dichlormethan, die vereinigten organischen Extrakte werden eingeengt. Der Rückstand wird mit Ethylacetat/Diethylether-Gemisch angerieben. Die Verbindung aus Beispiel 119A kristallisiert aus, im Überstand verbleibt vorwiegend die Verbindung aus Beispiel 118A. Dieses wird eingeengt und erneut der Isomerisierung unterworfen mit 2 ml TMG und Dichlormethan (40 ml). Dabei wird eine zweite Portion der Titelverbindung Beispiel 119A erhalten. Der Überstand der Kristallisation wird chromatographiert (Methode 33), wieder isomerisiert und eine dritte Portion Beispiel 119A wird gewonnen. Gesamtausbeute: 1.1 g (1.72 mmol, 90% d. Th.) Beispiel 119A in Form von Kristallen.
HPLC (Methode 5): Rₜ = 4.76 min.
LC-MS (Methode 20): Rₜ = 3.12 min, MS (ESIpos.): *m*/*z* (%) = 621.5 (90) [M+H]⁺; MS (ESIneg): *m*/*z* (%) = 619.5 (60) [M - H]⁻.
[α]²⁰_{Na} = -118 ° (c = 1.0, CHCl₃).
¹H NMR (400 MHz, *d₆*-DMSO) δ = 1.30 (s, 9H, COC(C*H*₃)₃), 2.86 (s, 6H, N(C*H*₃)₂), 3.30 (s, 3H, OC*H*₃), 4.75-5.23 (m, 4H), 6.62 (d, *J* = 8.8 Hz, 2H), 7.15-7.61 (m, 12H), 8.29 (br. s, 1H, N-*H*), 8.72 (br. s, 1H, N-*H*).
¹³C-NMR (126 MHz,) δ = 27.94 *und* 28.07 (CH₃, C(CH₃)₃), 51.31 (CH₃), 51.62 (CH₃), 51.98 (CH, C-7), 61.81 (CH, C-6), 66.18 *und* 66.32 *und* 66.50 (CH₂, OCH₂Ph), 67.49 *und* 67.65 (CH₂, OCH₂Ph), 78.68 *und* 78.90 (C quat, C-11), 111.74 (CH, C-3'), 123.54 (CH), 124.06 (CH), 126.76 (CH), 127.18 (CH), 127.30 (CH), 127.55 (CH), 127.76 (CH), 127.83 (CH), 127.98 (CH), 128.24 (CH), 128.30 (CH), 128.79 (CH), 135.59 (C quat), 135.77 (C quat), 135.99 (C quat), 136.11 (C quat), 149.74 (C quat), 154.21 (C quat), 154.68 (C quat), 154.92 (C quat), 155.03 (C quat), 155.14 (C quat), 156.09 (C quat), 156.24 (C quat), 166.97 (C quat, C=O).
HR-TOF-MS (Methode 24): C₃₃H₄₁N₄O₈ [M+H]⁺ ber. 621.2919, gef. 621.2922.

### Beispiel 120A

### Methyl (3R)-3-[(tert-Butoxycarbonyl)amino]-4-(dimethylaminophenyl)-L-alaninat

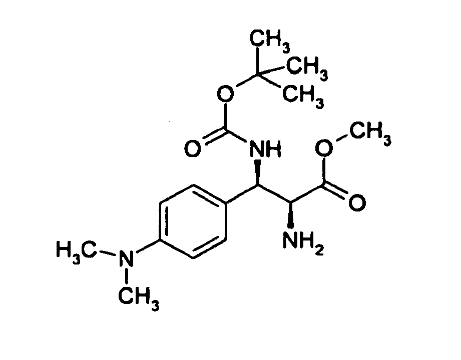

Die Verbindung aus Beispiel 119A (938 mg, 1.51 mmol) wird in Dichlormethan-Methanol 1:1 (30 ml) gelöst. Raney-Nickel (ca. 200 mg) wird unter Argonschutzgasatmosphäre dazugegeben und die Mischung wird bei 80 bar über 4 d hydriert. Wenn per HPLC (Methode 5) kein Ausgangsmaterial mehr nachgewiesen werden kann, filtriert man den Katalysator ab. Das Filtrat wird eingeengt. Es werden 0.6 g Rohprodukt (Reinheit ca 70%) erhalten, welches ohne weitere Reinigung im nächsten Schritt eingesetzt wird. Für analytische Zwecke wird eine Probe der reinen Verbindung aus Beispiel 120A durch präparative HPLC (Methode 27) feingereinigt.
HPLC (Methode 5): Rₜ = 3.25 min.
LC-MS (Methode 20): Rₜ = 1.34 min, MS (ESIpos.): *m*/*z* (%) = 338.2 (30) [M+H]⁺.
[α]²⁰_{Na} = -11.5° (c = 1.0, MeOH).
¹H NMR (500 MHz, *d₆*-DMSO) δ = 1.37 (s, 9H, COC(C*H*₃)₃), 2.90 (s, 6H, N(C*H*₃)₂), 3.54 (s, 3H, OC*H*₃), 4.24 (d, *J* = 5.4 Hz, 1H, β-H), 4.96 (dd, *J* = 8.4 Hz, *J* = 8.0 Hz, 1H, α-H), 6.73 (d, *J_{2',3'}* = 8.0 Hz, 2H, 3'-H), 7.13 (d, *J_{2',3'}* = 8.0 Hz, 2H, 2'-H), 7.46 (d, *J* = 9.6 Hz, 1H, N*H*), 8.470 (br. s, 2H, N*H*₂).
¹³C-NMR (126 MHz, *d₆*-DMSO) δ = 28.07 (CH₃, COC(CH₃)₃), 40.19 (CH₃, N(CH₃)₂), 52.68 (CH₃, OCH₃), 54.11 (CH, C-β), 56.79 (CH, C-α), 78.87 (C quat, COC(CH₃)₃), 112.53 (CH, C-3'), 127.65 (CH, C-2'), 149.87 (C quat), 154.61 (C quat, COC(CH₃)₃), 167.96 (C quat, CO₂CH₃).
HR-TOF-MS (Methode 24): C₁₇H₂₈N₃O₄ [M+H]⁺ ber. 338.2075, gef. 338.2064.

### Beispiel 121A

### Methyl (3R)-N²-(Benzyloxy)carbonyl)-3-[(tert-butoxycarbonyl)amino]-4-(dimethylaminophenyl)-L-alaninat

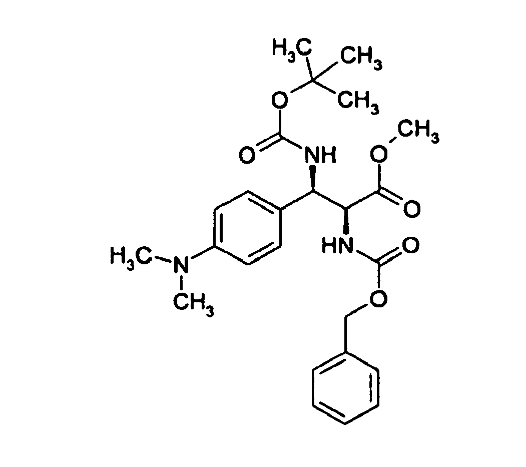

550 mg (1.14 mmol, 70% Reinheit) der Verbindung aus Beispiel 120A werden in Dioxan/Wasser 8:2.8 gelöst. Natriumhydrogencarbonat (479 mg, 5.71 mmol, 5 Äq.) und Benzylchlorformiat (212 µl, 1.48 mmol, 1.3 Äq.) werden zugesetzt und die Mischung wird 2.5 h bei RT gerührt. Dann wird zwischen Wasser und Ethylacetat ausgeschüttelt, die organische Phase über Natriumsulfat getrocknet und eingeengt. Aus dem Rückstand kann durch Anreiben mit Diethylether die Titelverbindung Beispiel 121A kristallin erhalten werden. Ausbeute: 490 mg (1.04 mmol, 91% d. Th.).
HPLC (Methode 5): Rₜ = 4.14 min.
LC-MS (Methode 20): Rₜ = 2.52 min, MS (ESIpos.): *m*/*z* (%) = 472.3 (100) [M+H]⁺.
[α]²⁰_{Na} = -13.4° (*c* = 1.0, MeOH).
¹H NMR (400 MHz, *d₆*-DMSO) δ = 1.36 (s, 9H, COC(C*H*₃)₃), 2.86 (s, 6H, N(C*H*₃)₂), 3.30 (s, 3H, OC*H*₃), 4.45 (dd, *J*_{α,β} = 4.2 Hz, *J* = 9.3 Hz, 1H, β-H), 4.94 (d, *J_{gem}* = 12.5 Hz, 1H, OC*H*₂Ph), 4.99 (d, *J_{gem}* = 12.5 Hz, 1H, OC*H*₂Ph), 5.15 (dd, *J*_{α,β} = 4.2 Hz, *J* = 10.3 Hz, 1H, α-H), 6.65 (d, *J_{2',3'}* = 8.8 Hz, 2H, 3'-H), 7.10 (d, *J_{2',3'}* = 8.8 Hz, 2H, 2'-H), 7.23-7.37 (m, 5H), 7.54 (d, *J* = 9.3 Hz, 1H, N*H*).
"C-NMR- (126 MHz, d₆-DMSO) δ = 28.0 (CH₃, COC(CH₃)₃), 41.1 (CH₃, N(CH₃)₂), 52.0 (CH₃, OCH₃), 53.8 (CH, C-β), 59.0 (CH, C-α), 65.4 (CH₂, OCH₂Ph), 78.3 (C quat, COC(CH₃)₃), 113.7 (CH), 126.9 (C quat), 127.3 (CH), 127.5 (CH), 127.8 (CH), 128.2 (C quat), 128.3 (CH), 136.6 (C quat), 154.6 (C quat, NCO₂), 156.0 (NCO₂), 170.7 (CO₂CH₃).
HR-TOF-MS: C₂₅H₃₄N₃O₆ [M+H]⁺ ber. 472.2443, gef. 472.2460.

### Beispiel 122A

### (3R)-N²-[(Benzyloxy)carbonyl]-3-[(tert-butoxycarbonyl)amino]-4-(dimethylaminophenyl)-L-alanin

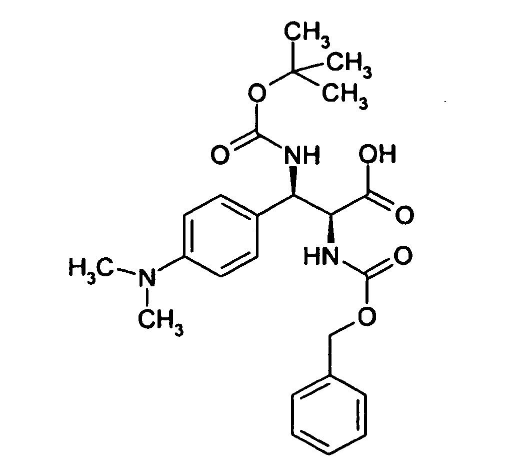

Die Verbindung 121A (444 mg, 0.94 mmol) wird in einer Mischung aus 5 ml THF und 830 µl Wasser gelöst. Bei 0°C werden 99 mg (2.35 mmol, 2.5 Äq.) Lithiumhydroxid Monohydrate dazugegeben. Die Reaktion wird bei 0°C über etwa 2 h durchgeführt, bis kein Ausgangsmaterial 121A mehr nachgewiesen werden kann (HPLC, Methode 5). Dann wird mit Essigsäure (620 µl, 2.83 mmol, 3 Äq.) abgestoppt. Man verdünnt mit Wasser und extrahiert mit Ethylacetat. Die organische Phase wird über Natriumsulfat getrocknet und das Lösemittel abdestilliert. Ausbeute: 430 mg (quant.).
HPLC (Methode 5): Rₜ = 3.80 min.
LC-MS (Methode 20): Rₜ = 2.21 min, MS (ESIpos.): *m*/*z* (%) = 458.2 (100) [M+H]⁺; MS (ESIneg): *m*/*z* (%) = 456.3 (100) [M - H]⁻.
[α]²⁰_{Na} = -16.5 (c = 1.0, MeOH).
¹H NMR (500 MHz, *d₆*-DMSO) δ = 4.39 (dd, *J*_{α,β} = 3.5 Hz, *J* = 9.6 Hz, 1H, β-H), 4.92 (d, *J_{gem}* = 12.8 Hz, 1H, OC*H*₂Ph), 4.97 (d, *J_{gem}* = 12.8 Hz, 1H, OC*H*₂Ph), 5.19 (dd, *J*_{α,β} = 3.5 Hz, *J* = 10.0 Hz, 1H, α-H), 6.86 (br. s, 2H), 7.17 (d, *J* = 8.1 Hz, 2H), 7.24 (d, *J* = 7.0 Hz, 2H), 7.23-7.29 (m, 2H), 7.41 (d, *J* = 10.1 Hz, 1H, N*H*), 7.48 (d, *J* = 1 Hz, 1H, N*H*), 13.0 (br. s, 1H, COO*H*).
¹³C-NMR (126 MHz, *d₆*-DMSO) δ = 28.0 (CH₃, COC(CH₃)₃), 41.2 (CH₃, N(CH₃)₂), 53.9 (CH, C-β), 58.8 (CH, C-α), 65.3 (CH₂, OCH₂Ph), 78.1 (C quat, COC(CH₃)₃), 113.7 (CH), 126.7 (C quat), 127.1 (CH), 127.3 (CH), 127.6 (CH), 128.1 (C quat), 128.2 (CH), 136.9 (C quat), 154.7 (C quat, NCO₂), 156.1 (NCO₂), 171.4 (CO₂H).
HR-TOF-MS (Methode 24): C₂₄H₃₂N₃O₆ [M+H]⁺ ber. 458.2286, gef. 458.2277.

### Beispiel 123A

### Pentafluorophenyl (3R)-N-[(Benzyloxy)carbonyl]-3-[(tert-butoxycarbonyl)amino]-4-(dimethylaminophenyl)-L-alaninat

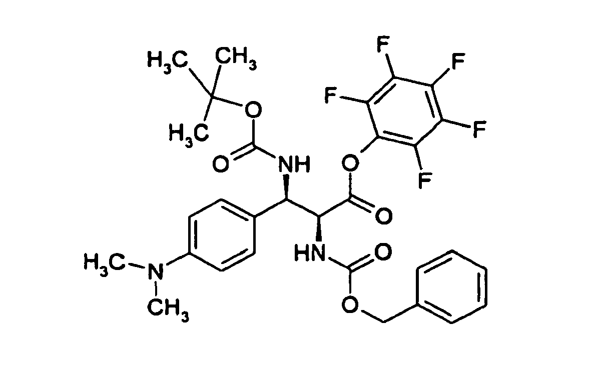

Verbindung 122A (408 mg, 0.89 mmol) wird in trocknenem THF (11 ml) bei 0°C unter Argonschutzgasatmosphäre gelöst. Pentafluorophenyldiphenylphosphinat (514 mg, 1.34 mmol, 1.5 Äq.) und NMM (360 mg, 3.57 mmol, 4 Äq.) werden zugegeben und die Reaktionsmischung wird 16 h bei RT gerührt. Dann wird das Lösemittel abgezogen und der Rückstand chromatographisch gereinigt (Methode 33: modifizierten Gradienten: 0-10 min 10% B, Rampe, 30 min 65% B, 36-38 min 10% B). Neben 123A (250 mg, 0.40 mmol, 45% d. Th.), wurde nicht umgesetztes Edukt (Beispiel 122A, 41 mg, 9 µmol, 10% d. Th.) isoliert.
HPLC (Methode 5): Rₜ = 3.66 min.
LC-MS (Methode 20): Rₜ = 3.23 min, MS (ESIpos.): *m*/*z* (%) = 624.4 (100) [M+H]⁺.
[α]²⁰_{Na} = -19.8° (*c* = 0.5, CH₂Cl₂).
¹H NMR (400 MHz, *d₆*-DMSO) δ = 1.36 (s, 9H, COC(C*H*₃)₃, 2.90 (s, 6H, N(C*H*₃)₂), 4.96-5.04 (m, 3H, β-H und OC*H*₂Ph), 5.39 (dd, *J*_{α,β} = 4.4 Hz, *J* = 10.2 Hz, 1H, α-H), 6.75 (br. s, 2H), 7.21-7.33 (m, 7H), 7.57 (d, *J* = 10.3 Hz, 1H, N*H*), 7.89 (d, *J* = 9.3 Hz, 1H, N*H*).
¹³C-NMR (126 MHz, *d₆*-DMSO) δ = 28.2 (CH₃, COC(CH₃)₃), 43.8. (CH₃, N(CH₃)₂), 55.6 (CH, C-β), 59.1 (CH, C-α), 67.5 (CH₂, OCH₂Ph), 80.9 (C quat, COC(CH₃)₃), 117.8 (CH), 124.4 (C quat, m),128.1 (CH), 128.4 (CH), 128.6 (CH), 128.7 (CH), 132.9 (C quat, m), 136.8 (C quat, m), 138.9 (C quat, m), 139.9 (C quat, m), 141.9 (C quat, m), 155.7 (C quat, NCO₂), 156.2 (NCO₂), 166.2 (CO₂H).

### Beispiel 124A

### {(3R)-N-[(Benzyloxy)carbonyl]-3-[(tert-butoxycarbonyl)amino]-4-(dimethylaminophenyl)-L-alanyl}-[(3R)-3-Hydroxy-L-leucyl]-L-leucyl-D-arginyl-L-isoleucyl-L-allothreonyl-glycyl-[(3S)-3-hydroxy-L-asparaginyl]-L-serin-bis-trifluoracetat

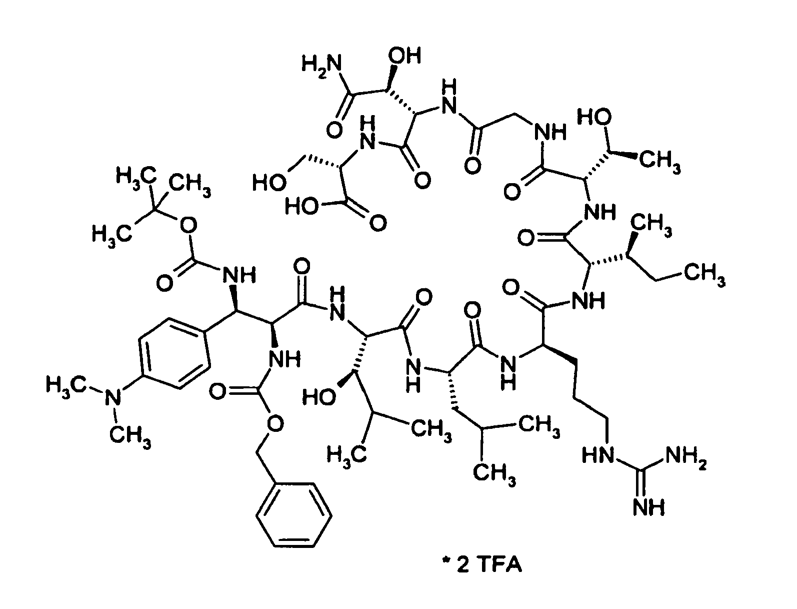

Verbindung 123A (237 mg, 0.38 mmol) und Verbindung Edman^{3.0} (Beispielverbindung 3, 465 mg, 0.46 mmol, 1.2 Äq.) werden bei 0°C in DMF (40 ml) gelöst und mit DIEA (0.40 ml, 2.28 mmol, 6 Äq.) versetzt. Die Reaktionsmischung wird über Nacht weitergerührt, wobei sie sich langsam auf RT erwärmt. Das DMF wird danach am Rotationsverdampfer abgezogen und der Rückstand chromatographisch gereinigt (Methode 44). Ausbeute: 550 mg (92% d. Th.).
HPLC (Methode 5): Rₜ = 3.62 min.
LC-MS (Methode 20): Rₜ = 2.01 min, MS (ESIpos.): *m*/*z* (%) = 673.3 (100) [M + 2H]²⁺, MS (ESIneg) *m*/*z* (%) = 1343 [M - H]⁻.
HR-TOF-MS (Methode 24): C₆₁H₉₈N₁₅O₁₉ [M+H]⁺ ber. 1344.7158, gef. 1344.7155.

### Beispiel 125A

### {(3R)-3-amino-N-[(benzyloxy)carbonyl]-4-(dimethylaminophenyl)-L-alanyl}-[(3R)-3-Hydroxy-L-leucyl]-L-leucyl-D-arginyl-L-isoleucyl-L-allothreonyl-glycyl-[(3S)-3-hydroxy-L-asparaginyl]-L-serin-bis-trifluoracetat tris-trifluoracetat

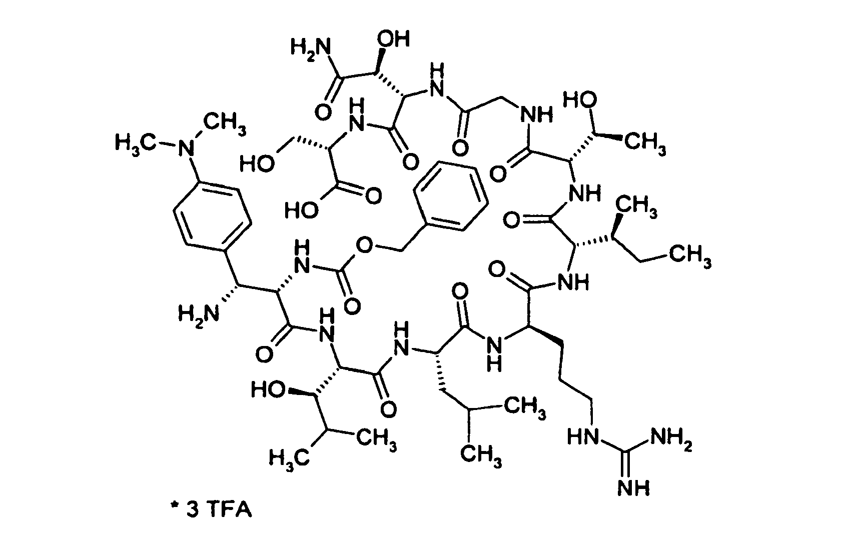

550 mg (0.35 mmol) der Verbindung aus Beispiel 124A werden in Dichlormethan (2 ml) gelöst und dann mit 14 ml 30% TFA in Dichlormethan versetzt. Man rührt 15 min bei RT, engt dann ein und trocknet am Ölpumpenvakuum. Das Rohprodukt (644 mg, ca 70% rein, 0.28 mmol, 80% d. Th.) wird ohne Reinigung weiter umgesetzt.
HPLC (Methode 7): Rₜ = 3.46 min.
LC-MS (Methode 20): Rₜ = 1.56 min, MS (ESIpos.): *m*/*z* (%) = 622.9 (100), [M + 2H]²⁺, MS (ESIneg.): *m*/*z* (%) = 1242.6 [M - H]⁻.

### Beispiel 126A

### N^{2.1}-(Benzyloxycarbonyl)-[(3R)-3-amino-4-(dimethylamino)-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-bis-trifluoracetat

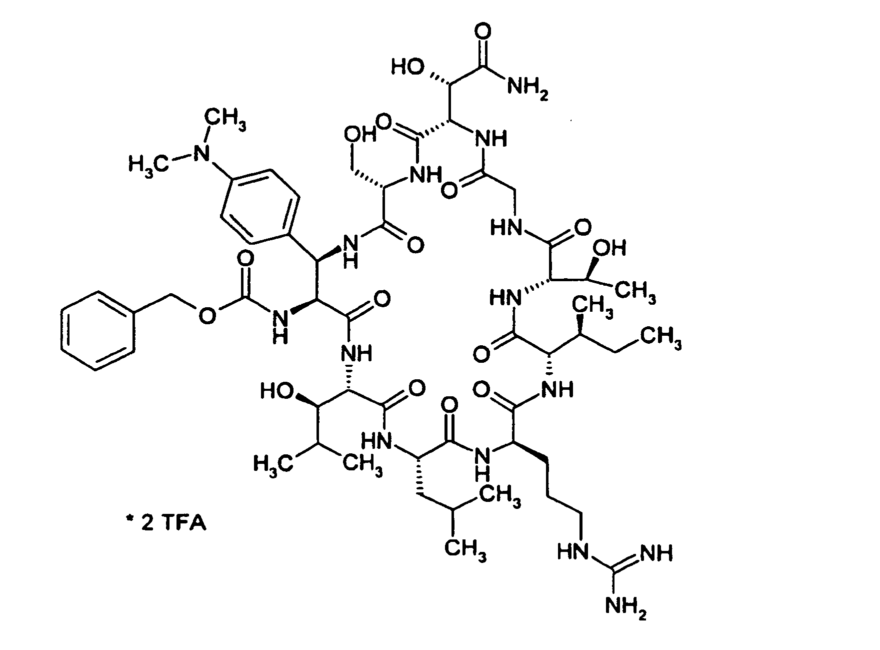

Das Rohprodukt aus Beispiel 125A (644 mg, 0.28 mmol) wird in DMF (30 ml) gelöst und auf 0°C gekühlt. HATU (349 mg, 0.92 mmol, 3 Äq.) wird gefolgt von NMM (200 µl, 1.84 mmol, 6 Äq.) zugegeben. Nach 5 min entfernt man das Eisbad und läßt dann noch 3 h bei RT rühren. Die Reaktion wird durch Zugabe von Methanol abgestoppt und zunächst gelchromatographisch (Methode 45, Eluent: Methanol) vorgereinigt. Die produkthaltigen Fraktionen werden vereinigt und durch die präparative HPLC (Methode 33) feingereinigt. Ausbeute: 211 mg (52% d. Th.).
HPLC (Methode 5): Rₜ = 3.64 min.
LC-MS (Methode 20): Rₜ = 1.70 min, MS (ESIpos.): *m*/*z* (%) = 614 (100) [M + 2H]²⁺, 1226.8 (20) [M+H]⁺; MS (ESIneg.): *m*/*z* (%) = 1224.9 (100) [M - H]⁻.
HR-TOF-MS (Methode 24): C₅₆H₈₈N₁₅O₁₆ [M+H]⁺ ber. 1226.6528, gef. 1226.6534.

### Beispiel 127A

### [(3R)-3-amino-4-(dimethylaminophenyl)-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam tris-hydrochlorid

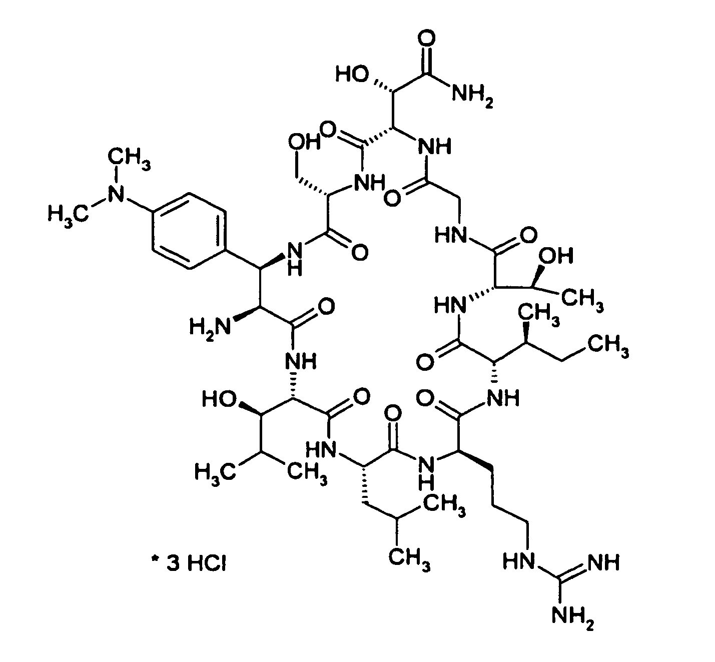

Die Verbindung aus Beispiel 126A (209 mg, 0.14 mmol) wird in Methanol (50 ml) gelöst und mit 1 M wässr. Salzsäure (860 µl, 6 Äq.) sowie 10% Palladiumkohle versetzt. Man hydriert über 90 min bei Umgebungsdruck und RT. Anschließend wird vom Katalysator abfiltriert und eingeengt. Der Rückstand wird in Wasser aufgenommen und lyophilisiert. Man erhält 170 mg (98% d. Th.) der Titelverbindung.
HPLC (Methode 7): Rₜ = 3.19 min.
LC-MS (Methode 20): Rₜ = 1.26 min, MS (ESIpos.): *m*/*z* (%) = 547.1 (100) [M + 2H]²⁺, 1092.8 (5) [M+H]⁺.
HR-TOF-MS (Methode 24): C₄₈H₈₂N₁₅O₁₄ [M+H]⁺ ber. 1092.6161, gef. 1092.6133.

### Beispiel 128A

### N^{2.1}-(tert-Butoxycarbonyl)-[3-tert-butyl-D-alanyl]-[3-tert-butyl-L-alanyl]-[(3R)-3-amino-4-(dimethylaminophenyl)-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam bis-hydrochlorid

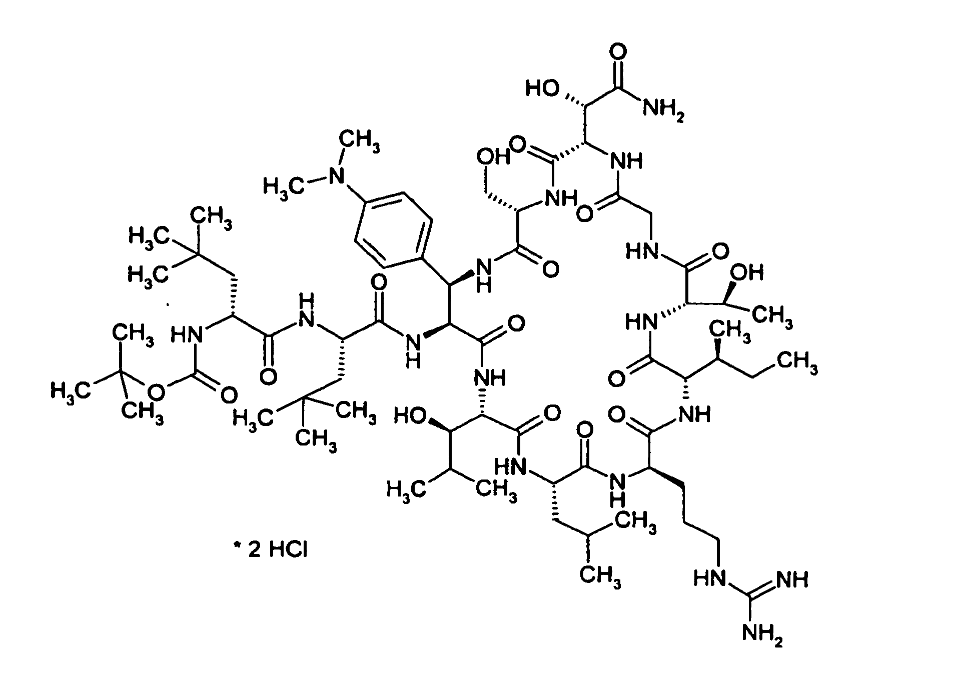

Die Verbindung aus Beispiel 127A (85 mg, 71 µmol) und das Dipeptid (Beispiel 9A, 40 mg, 106 µmol, 1.5 Äq.) werden in DMF (4 ml) gelöst und auf 0°C gekühlt. Dann werden NMM (31 µl, 281 µmol, 4 Äq.) und HATU (43 mg, 113 µmol, 1.6 Äq.) zugegeben und dann das Eisbad entfernt. Der Ansatz erwärmt sich langsam auf Umgebungstemperatur. Nach 1 h wird durch Zusatz von Methanol die Reaktion abgestoppt und mit Methanol als Laufmittel (Methode 45) chromatographiert. Das Eluat wird mit HPLC (Methode 7) überprüft, die produkthaltigen Fraktionen werden vereinigt und eingeengt. Man erhält 105 mg der Titelverbindung ca. 66% rein (65% d. Th.). Dieses Material wird ohne weitere Reinigung in der Folgestufe eingesetzt.
HPLC (Methode 7): Rₜ = 4.47 min.
LC-MS (Methode 20): Rₜ = 2.24 min, MS (ESIpos.): *m*/*z* (%) = 724.3 (100) [M + 2H]²⁺, 1448.3 (5) [M+H]⁺.

### Beispiel 129A

### (2S)-N-(tert-Butoxycarbonyl)-3-(trimethylsilyl)alanin

und

### Beispiel 130A

### (2R)-N-(tert-Butoxycarbonyl)-3-(trimethylsilyl)alanin

Nach M. Merget, K. Günther, M. Bernd, E. Günther, R. Tacke, J. Organomet. Chem. 2001 628, 183-194 wird die Zielverbindung dargestellt. Die Trennung der Enantiomere wird durch die präparative HPLC (Methode 41) an chiraler Phase erreicht und die Zuordnung der Isomere erfolgt durch HPLC-Vergleich mit einer authentischen Probe von *N*-(*tert*-Butoxycarbonyl)-L-3-trimethylsilylalanin (2*R* Isomer, Mercachem AMR 39.260).

### Beispiel 129A

### N-(tert-Butoxycarbonyl)-3-(trimethylsilyl)-D-alanin

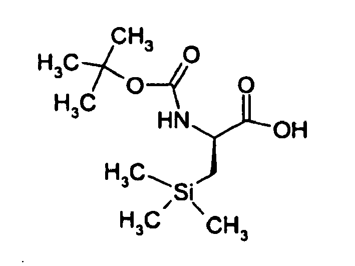

Chirale HPLC (Methode 41): Rₜ = 4.16 min, e.e.> 99%.
[α]_{D}²⁰ = + 1.1 (c = 0.83, Methanol)

### Beispiel 130A

### N-(tert-Butoxycarbonyl)-3-(trimethylsilyl)-L-alanin

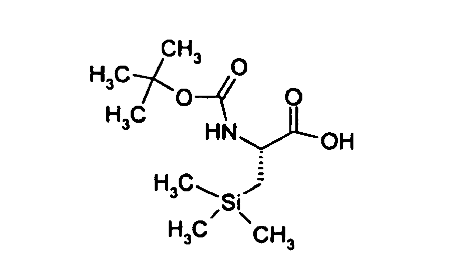

Chirale HPLC (Methode 41): Rₜ = 9.27 min, e.e.> 99%
[α]_{D}²⁰ = - 1.6 (c = 0.66, Methanol)

### Beispiel 131A

### Methyl [N²-(tert-Butoxycarbonyl)-3-(trimethylsilyl)-D-alanyl]-2-tert-butyl-L-alaninat

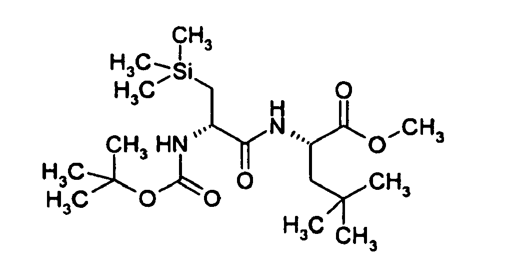

Methyl 2-*tert*-Butyl-L-alaninat Trifluoracetat (215 mg, 0.82 mmol) [S.D. Bull, S.G. Davies, A. C. Garner, M. D. O'Shea, J. Chem. Soc. Perkin Trans.1, 2001, 3281-3287] und Beispiel 129A (225 mg, 0.82 mmol, 1 Äq.) werden bei 0°C in DMF (5 ml) gelöst. Dann werden NMM (360 µl, 3.29 mmol, 4 Äq.) und HATU (470 mg, 1.24 mmol, 1.5 Äq.) zugegeben und über 2 h bei RT gerührt. Man schüttelt zweimal mit MTBE gegen ges. wässr. Natriumhydrogencarbonatlösung aus, wäscht die vereinigten organischen Phasen mit 1 M Zitronensäure und erneut mit ges. wässr. Natriumhydrogencarbonatlösung, trocknet über Natriumsulfat und engt ein. Nach chromatographischer Reinigung (Methode 33) erhält man 290 mg (0.72 mmol, 88% d. Th.) der Titelverbindung.
HPLC (Methode 7): Rₜ = 5.37 min.
LC-MS (Methode 18): Rₜ = 3.02 min, MS (ESIpos.): *m*/*z* (%) = 403.5 (100) [M+H]⁺.
¹H NMR (400 MHz, *d₆*-DMSO) δ = 0.01 (s, 9H, Si(C*H*₃)₃), 0.89 (m, 11H), 1.37 (s, 9H, COC(C*H*₃)₃), 1.55 (dd, *J* = 8.8 Hz, 14.2 Hz, 1H), 1.63 (dd, *J* = 3.4 Hz, 14.2 Hz, 1H), 3.62 (s, 3H, OC*H*₃), 4.04 (m, 1H), 4.27 (m, 1H), 6.70 (d, *J*= 9.0 Hz, 1H, N*H*), 7.96 (d, *J*= 8.1 Hz, 1H, N*H*).

### Beispiel 132A

### [N²-(tert-Butoxycarbonyl)-3-(trimethylsilyl)-D-alanyl]-2-tert-butyl-L-alanin

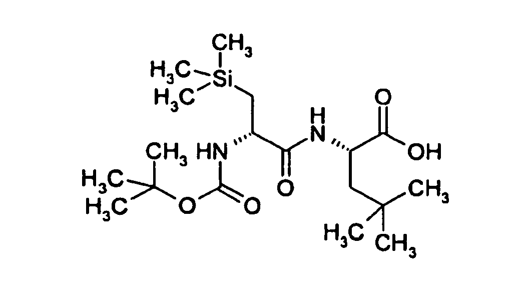

Beispiel 131A (274 mg, 0.68 mmol) wird in THF/Wasser (3:1, 21 ml) aufgenommen und auf 0°C gekühlt. Lithiumhydroxid Monohydrat (57 mg, 1.36 mmol, 2 Äq.) wird zugegeben und bei 0°C 1 h gerührt. Man entfernt größtenteils das THF im Vakuum, stellt durch Zusatz von 1 M Zitronensäure auf pH 4 ein und extrahiert zweimal mit Ethylacetat. Die vereinigten organischen Phasen werden über Natriumsulfat getrocknet und eingeengt. Nach chromatographischer Reinigung (Methode 33 mit modifiziertem Gradienten: 0 - 2 min 30% B, Rampe, 30 - 35 min - 90% B) erhält man 237 mg (0.61 mmol, 90% d. Th.) der Titelverbindung als farblosen Feststoff.
HPLC (Methode 7): Rₜ = 4.93 min.
LC-MS (Methode 18): Rₜ = 2.76 min, MS (ESIpos.): *m*/*z* (%) = 389.4 (100) [M+H]⁺.
¹H NMR (400 MHz, *d₆*-DMSO) δ = 0.00 (s, 9H, Si(C*H*₃)₃), 0.88 (m, 11H), 1.36 (s, 9H, COC(C*H*₃)₃), 1.51 (dd, *J* = 9.3 Hz, 14.2 Hz, 1H), 1.64 (dd, *J* = 3.0 Hz, 14.2 Hz, 1H), 4.02 (m, 1H), 4.20 (m, 1H), 6.71 (d, *J* = 9.3 Hz, 1H, N*H*), 7.80 (d, *J* = 8.1, 1H, N*H*), 12.49 (br s, 1H, COO*H*).

### Beispiel 133A

### N^{2.1}-(tert-Butoxycarbonyl)-[3-trimethylsilyl-D-alanyl]-[3-tert-butyl-L-alanyl]-[(3R)-3-amino-4-(dimethylaminophenyl)-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam bis-hydrochlorid

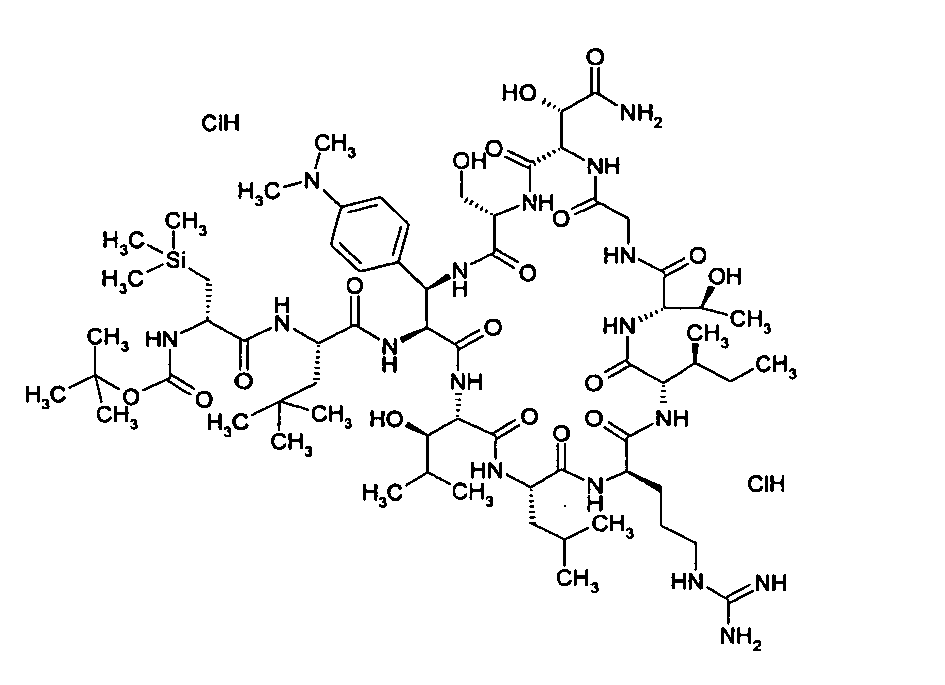

Die Verbindung aus Beispiel 127A (102 mg, 85 µmol) und das Dipeptid (Beispiel 132A, 50 mg, 127 µmol, 1.5 Äq.) werden in DMF (4 ml) gelöst und auf 0°C gekühlt. Dann werden NMM (37 µl, 340 µmol, 4 Äq.) und HATU (65 mg, 170 µmol, 2.0 Äq.) zugegeben und dann das Eisbad entfernt. Der Ansatz erwärmt sich langsam auf Umgebungstemperatur. Nach 1 h wird durch Zusatz von Methanol die Reaktion abgestoppt und mit Methanol als Laufmittel über Sephadex LH20 (Methode 45) chromatographiert. Das Eluat wird mit HPLC (Methode 7) überprüft, die produkthaltigen Fraktionen werden vereinigt und eingeengt. Man erhält 126 mg (82 µmol, 96% d. Th.) der Titelverbindung.
HPLC (Methode 7): Rₜ = 4.55 min.

### Beispiel 134A

### Benzyl N²-(tert-Butoxycarbonyl)-D-phenylalanyl-L-phenylalaninat

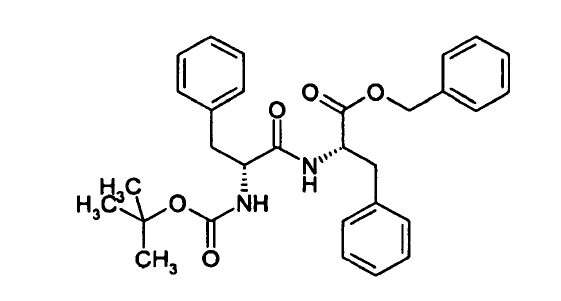

Zu einer Lösung von Benzyl L-phenylalaninat-hydrochlorid(1.1 g, 4.15 mmol, 1.1 Äq.) in Dichlormethan p.a. (365 ml) werden bei -10°C nacheinander HOBt (2.0 g, 15.08 mmol, 4 Äq.), NMM (1.2 ml, 1.1 g, 11.31 mmol, 3 Äq.), *N*-(*tert*-Butoxycarbonyl)-D-phenylalanin (1.0 g, 3.77 mmol, 1 Äq.) [Anderson, McGregor; J. Am. Chem. Soc.; 79; 1957; 6180,6181], EDC (1.4 g, 7.54 mmol, 2 Äq.) und erneut NMM (0.8 ml, 0.8 g, 7.54 mmol, 2 Äq.) gegeben. Das Reaktionsgemisch erwärmt sich langsam auf RT und wird bei dieser Temperatur über Nacht nachgerührt. Das Lösungsmittel wird vollständig entfernt, der Rückstand mit Ethylacetat versetzt, anschließend mit ges. wässr. Natriumhydrogencarbonatlösung, mit 5%iger wässr. Zitronensäure, ges. wässr. Natriumhydrogencarbonatlösung (500 ml) und ges. wässr. Natriumchloridlösung gewaschen. Man trocknet über Natriumsulfat und filtriert. Im Vakuum wird bis zur Trockne eingedampft und anschließend im Hochvakuum nachgetrocknet. Man erhält 1.8 g (96% d. Th.) der Titelverbindung, die ohne weitere Reinigung umgesetzt wird.
HPLC (Methode 12): Rₜ = 9.16 min.
HPLC (Methode 4): Rₜ = 5.16 min.
¹H NMR (400 MHz, *d₆*-DMSO): δ = 1.18/1.27 (2s, 9H, OtBu), 2.47-2.57 (m unterm H-DMSO-Signal, 1H, β-H), 2.66 (dd, *J*= 13.8, 3.7 Hz, 1H, β-CH), 2.91 (dd, *J*= 13.8, 9.4 Hz, 1H, β-CH), 3.09 (dd, *J*= 13.8, 3.1 Hz, 1H, β-CH), 4.19 (td, *J*= 9.3, 4.2 Hz, 1H, α-CH), 4.57 (td, *J*= 8.8, 5.9 Hz, 1H, α-CH), 5.12(s, 2H, OCH₂Ph), 6.76 (d, *J*= 9.2 Hz, 1H, NH), 7.08-7.43 (m, 15H, Aryl-H), 8.48 (d, *J*= 8.1 Hz, 1H, NH).
HR-TOF-MS (Methode 24): C₃₀H₃₆N₂O₅ [M+H]⁺ gef. 503.2566, ber. 503.2546.

### Beispiel 135A

### N²-(tert-Butoxycarbonyl)-D-phenylalanyl-L-phenylalanin

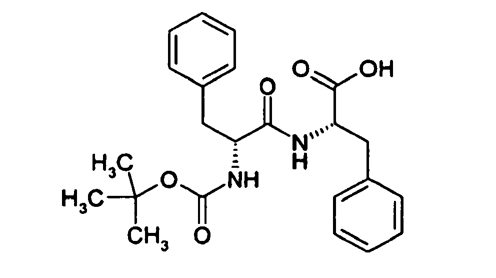

Die Titelverbindung wird aus der Verbindung aus Beispiel 134A (563.0 mg, 1.12 mmol) nach der Arbeitsvorschrift 5 dargestellt. Nach 3 h Rühren in der Wasserstoffatmosphäre bei Normaldruck und RT wird laut der Reaktionskontrolle (Methode 12) der vollständige Umsatz erreicht. Man erhält 452.2 mg (97.9% d. Th.) Produkt.
[α]²⁰Na = +33.2° (*c* = 0.57 in CH₂Cl₂).
HPLC (Methode 12): Rₜ = 7.64 min.
HPLC (Methode 4): Rₜ = 4.45 min.
¹H NMR (400 MHz, *d₆*-DMSO): δ= 1.19/1.26 (2s, 9H, OtBu), 2.44-2.56 (m unterm *H-*DMSO-Signal, 1H, β-H), 2.69 (dd, *J*= 14.0, 3.8 Hz, 1H, β-CH), 2.86 (dd, *J*= 14.0, 9.5 Hz, 1H, β-CH), 3.08 (dd, *J* = 14.0, 4.6 Hz, 1H, β-CH), 4.18 (td, *J* = 9.7, 3.57 Hz, 1H, α-CH), 4.48 (td, *J* = 9.3, 4.5 Hz, 1H, α-CH), 6.71 (d, *J* = 8.7 Hz, 1H, NH), 7.08-7.32 (m, 10H, Aryl-H), 8.27 (d, *J* = 8.0 Hz, 1H, NH), 12.77 (s br., 1H, COOH).
HR-TOF-MS (Methode 24): C₂₃H₃₀N₂O₅ [M+H]⁺ gef. 413.2082, ber. 413.2076.

### Beispiel 136A

### 2-(Trimethylsilyl)ethyl N²-[(benzyloxy)carbonyl]-3-[(tert-butoxycarbonyl)amino]-L-alaninat

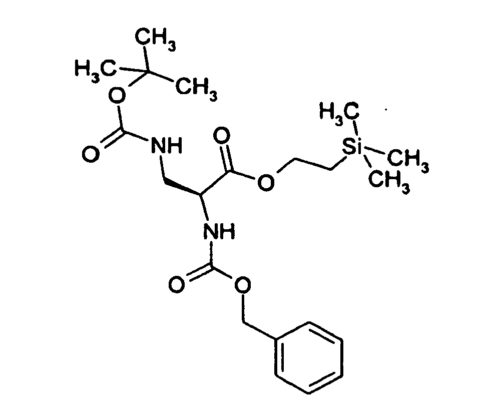

*N*-[(Benzyloxy)carbonyl]-3-[(*tert*-butoxycarbonyl)amino]-L-alanin (1000.0 mg, 2.96 mmol) [Rane, F.Dinanath et al.; Tetrahedron Lett.; EN; 34; 20; 1993; 3201-3204] werden in Dichlormethan (20 ml) gelöst und die Lösung bei -20°C nacheinander mit Trimethylsilylethanol (4.2 ml, 3.5 g, 29.55 mmol, 10 Äq.), DCC (1.22 g, 5.91 mmol, 2 Äq.) und DMAP (361.1 mg, 2.96 mmol, 1 Äq.) versetzt. Die Reaktion wird auf RT erwärmt, bei dieser Temperatur über Nacht nachgerührt und zum vollständigen Umsatz gebracht. Zur Aufarbeitung wird der Ansatz eingeengt, der Rückstand mit Diethylether aufgenommen und anschließend nacheinander zweimal mit Wasser und einmal mit ges. wässr. Natriumchloridlösung ausgeschüttelt. Die etherische Phase wird über Natriumsulfat getrocknet, das Trockenmittel über Kieselgel abfiltriert und vom Filtrat das Lösungsmittel größtenteils am Rotationsverdampfer entfernt. Sollte beim Einengen Niederschlag ausfallen, so kann dieser ggf. nochmals über Kieselgel abgetrennt werden. Der erhaltene Rückstand wird anschließend chromatographisch (Methode 31) feingereinigt und man erhält 1.1 g der (86.4% d. Th.) Titelverbindung.
HPLC (Methode 12): Rₜ = 9.31 min.
LC-MS (Methode 18): Rₜ = 2.97 min, MS (ESIpos.): *m*/*z* (%) = 439 (100) [M+H]⁺.

### Beispiel 137A

### 2-(Trimethylsilyl)ethyl 3-Amino-N²-(benzyloxycarbonyl)-L-alaninat trifluoracetat

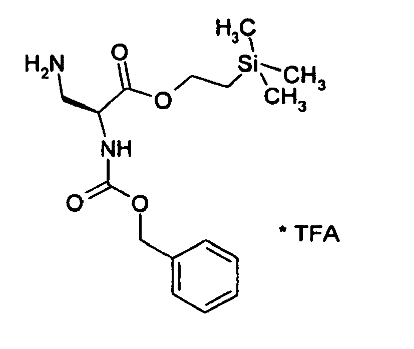

Die Titelverbindung wird aus der Verbindung aus Beispiel 136A (100.0 mg, 0.23 mmol), wie in der Arbeitsvorschrift 1 beschrieben ist, dargestellt. Aus dem Rohprodukt wird nach der Feinreinigung (Methode 35) 41 mg (39.7% d. Th) des Produktes isoliert.
HPLC (Methode 12): Rₜ = 6.33 min.
LC-MS (Methode 23): Rₜ = 4.83 min, MS (ESIpos.): *m*/*z* (%)=339 (15) [M+H]⁺, 311 (100).

### Beispiel 138A

### N²-(Benzyloxycarbonyl)-N²-(N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl)-(3R)-3-amino-L-alanin-[2-(trimethylsilyl)ethyl]ester-trifluoracetat

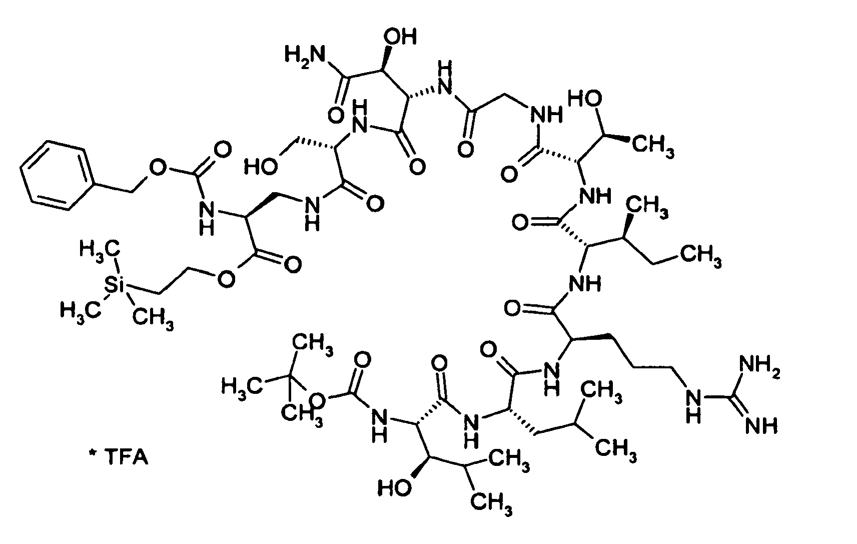

Das Octapeptid (Beispiel 100A, 9.0 mg, 8.04 µmol) und die Beispielverbindung 137A (17.0 mg, 39.95 µmol, 5 Äqivalente) werden unter Argonschutzgasatmosphäre und bei 0°C in DMF (900 µl) vorgelegt. Anschließend wird der Ansatz nacheinander mit Base (9 µl, 0.8 mg, 8.04 µmol, 1 Äq.) aus einer NMM-Stammlösung (0.91 mmol/ml in DMF), HATU (6.1 mg, 16.08 µmol, 2 Äq.) und nochmals mit NMM-Lösung (9 µl, 0.8 mg, 8.04 µmol, 1 Äq.) versetzt. Nach 15 min Rühren wird abschließend NMM-Stammlösung (22 µl, 2 mg, 20.10 µmol, 2.5 Äq.) zugegeben, langsam auf RT erwärmt und über Nacht bei dieser Temperatur nachgerührt. Die Lösung wird über die präparative HPLC (Methode 36) vorgereinigt und gelchromatographisch (Methode 45, Eluent: Methanol mit 0.25% Eisessig) feingereinigt. Man erhält 8.0 mg (69.1% d. Th.) der Titelverbindung.
HPLC (Methode 12): Rₜ = 7.21 min.
LC-MS (Methode 23): Rₜ = 5.30 min, MS (ESIpos.): *m*/*z* (%) = 1325 (75) [M+H]⁺; MS (ESIneg.): *m*/*z* (%) = 1323 (100) [M - H]⁻.

### Beispiel 139A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3R)-3-amino-L-alanin-trifluoracetat

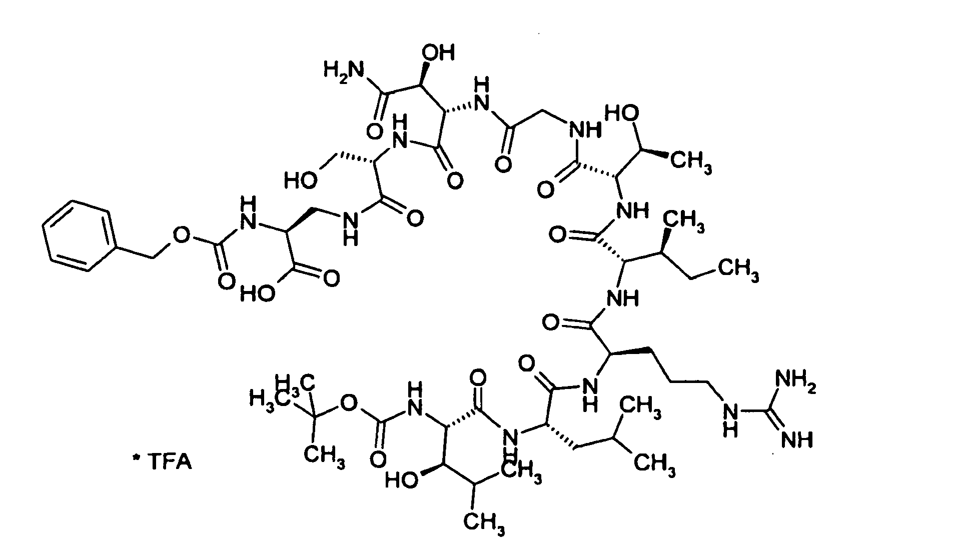

Die Verbindung aus Beispiel 138A (7.5 mg, 5.21 µmol) wird in 900 µl THF vorgelegt, mit 4 Portionen 1 M TBAF- Lösung in THF (26 µl, 6.8 mg, 26.05 µmol, 5 Äq.) versetzt und nach 15 Äq. Base wird des weiteren Molsieb (4 Ä) zugegeben. Die Reaktion wird 1 h bei RT nachgerührt und der vollständige Umsatz durch HPLC- Kontrolle (Methode 12) festgestellt. Zur Aufarbeitung wird der Ansatz mit Eisessig neutral gestellt, anschließend im Vakuum aufkonzentriert und über die präparative HPLC (Methode 29) chromatographiert. Es wird 6.2 mg (88.9% d. Th.) der Titelverbindung erhalten.
HPLC (Methode 12): Rₜ = 6.24 min.
LC-MS (Methode 23): Rₜ = 4.88 min, MS (ESIpos.): *m*/*z* (%) = 1224 (80) [M+H]⁺, 242 (100); MS (ESIneg.): *m*/*z* (%) = 1223 (100) [M - H]⁻.

### Beispiel 140A

### N²-(Benzyloxycarbonyl)-N³-{N^{2.1}-tert-butoxycarbonyl[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3R)-3-amino-L-alanin-pentafluorphenylester-trifluoracetat

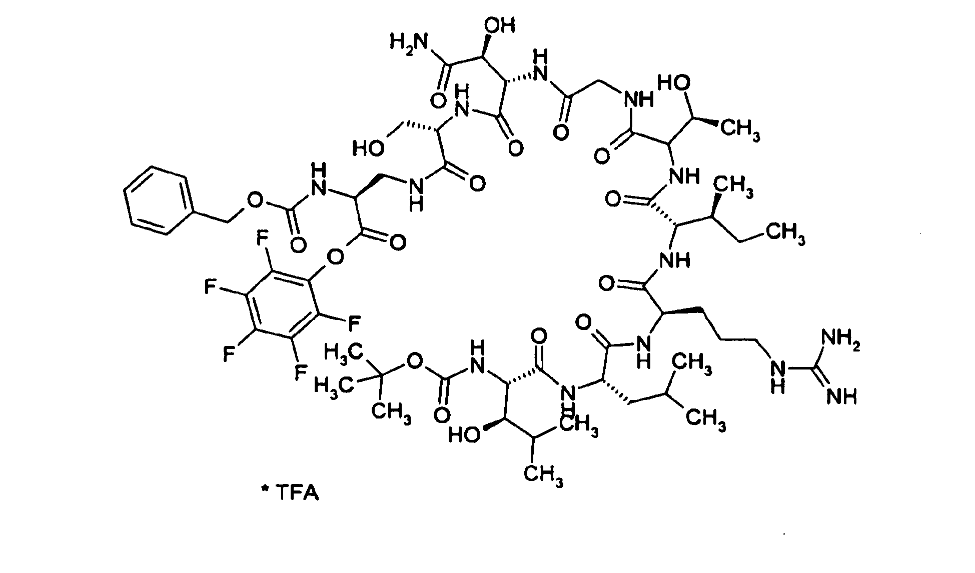

Unter Argonschutzgasatmosphäre wird die Verbindung aus Beispiel 139A (3.1 mg, 2.31 µmol) in Dichlormethan (1 ml) vorgelegt und Pentafluorphenol (4.3 mg, 23.14 µmol, 10 Äq.) zugegeben. Die Reaktion wird auf -20°C gekühlt, abschließend mit EDC (1.0 mg, 5.32 µmol, 2.3 Äq.) versetzt, auf RT erwärmt und bei dieser Temperatur über Nacht nachgerührt. Zur Aufarbeitung wird das Lösungsmittel vollständig entfernt und der Rückstand gefriergetrocknet. Die Titelverbindung wird als Rohprodukt weiter umgesetzt und es wird eine quantitative Ausbeute angenommen.
HPLC (Methode 12): Rₜ = 7.18 min.
LC-MS (Methode 23): Rₜ = 5.56 min, MS (ESIpos.): *m*/*z* (%) = 1392 (80) [M+H]⁺.

### Beispiel 141A

### N²-(Benzyloxycarbonyl)-N³-{[(3R)-3-hydroxy-L-leucyl]-L-leucyl-D-arginyl-L-isoleucyl-L-allothreonyl-glycyl-[(3S)-3-hydroxy-L-asparaginyl]-L-seryl}-(3R)-3-amino-L-alaninpentafluorphenylester-bis-hydrochlorid

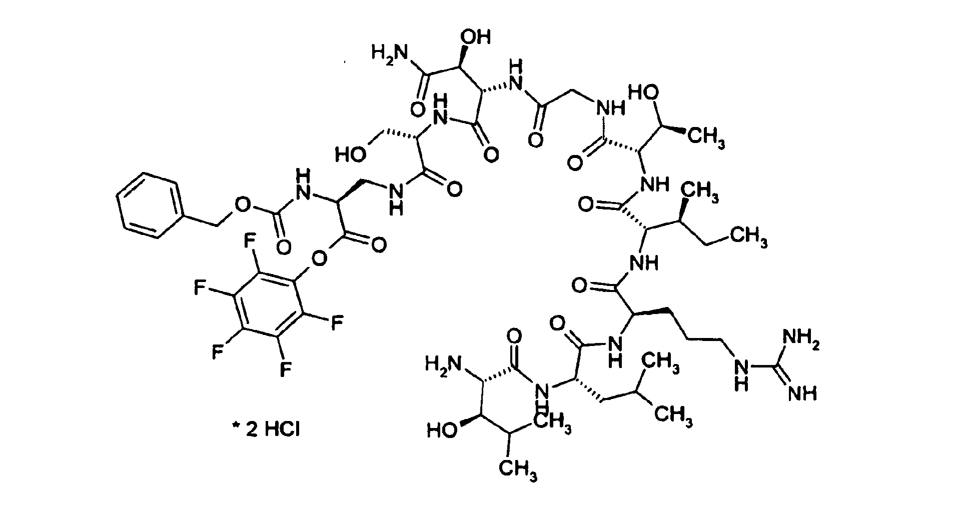

Aus der Verbindung aus Beispiel 140A (3.0 mg, 1.99 µmol) wird nach der Arbeitsvorschrift 3 die Titelverbindung dargestellt. Nach Zugabe der Salzsäure bei 0°C wird der vollständige Umsatz durch HPLC-Kontrolle (Methode 12) nach 30 min festgestellt. Man erhält die Titelverbindung als Rohprodukt, wobei eine quantitative Ausbeute angenommen wird.
LC-MS (Methode 23): Rₜ = 4.47 min, MS (ESIpos.): *m*/*z* (%) = 1292 (3) [M+H]⁺, 646 (100) [M + 2H]²⁺, MS (ESIneg.): *m*/*z* (%) = 1290 (40) [M - H]⁻, 1106 (100).

### Beispiel 142A

### N^{2.1}-(Benzyloxycarbonyl)-[(3R)-3-amino-L-alanyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-hydrochlorid

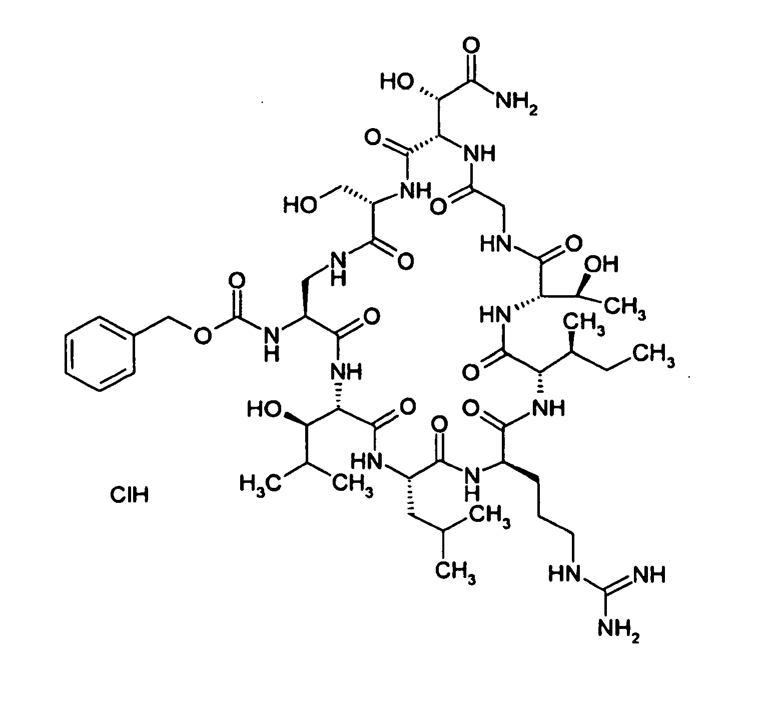

Unter Argonschutzgasatmosphäre wird die Verbindung aus Beispiel 141A (3.0 mg, 2.20 µmol) in Chloroform (20 ml) vorgelegt. Triethylamin (22 µl, 15.8 mg, 131.94 µmol) wird in Chloroform (25 ml) gelöst und innerhalb von 1 h zur Lösung zugetropft. Nach beendeter Zugabe wird die Reaktion über Nacht bei RT nachgerührt. Zur Aufarbeitung wird der Ansatz mit TFA neutral gestellt, anschließend im Vakuum aufkonzentriert und gelchromatographisch (Methode 45, Eluent: Methanol mit 0.25% Eisessig) vorgereinigt. Das Produkt wird nach der Feinreinigung über die präparative HPLC (Trennphase: Xterra 10 mm, Eluent: 0.1% TFA, Acetonitril-Wasser-Gradient) mit 0.4 mg (15.9% d. Th.) isoliert.
HPLC (Methode 12): Rₜ = 5.96 min.
LC-MS (Methode 18): Rₜ = 1.72 min, MS (ESIpos.): *m*/*z* (%) = 1108 (48) [M+H]⁺, 132 (100); MS (ESIneg.): *m*/*z* (%) = 1106 (100) [M - H]⁻.

### Beispiel 143A

### [(3R)-3-amino-L-alanyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-bishydrochlorid

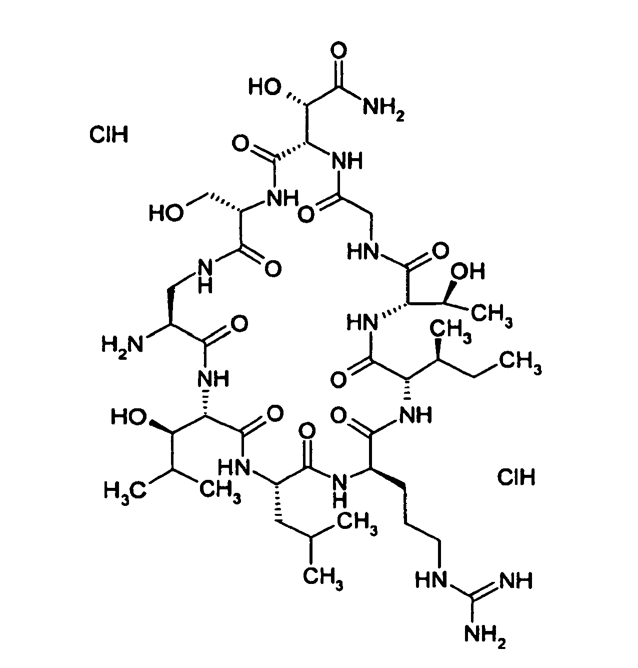

Die Titelverbindung (0.4 mg, 0.35 µmol) wird aus der Verbindung aus Beispiel 142A, wie in der Arbeitsvorschrift 4 beschrieben ist, dargestellt. Der vollständige Umsatz wird nach 6 h Hydrierung bei Normaldruck und RT erreicht. Man erhält 0.3 mg (81.9% d. Th.) die Titelverbindung als Rohprodukt, welches weiter umgesetzt wird.

### Beispiel 144A

### N^{2.1}-(tert-Butoxycarbonyl)-[D-phenylalanyl]-[L-phenylalanyl]-[(3R)-3-amino-L-alanyl)]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam-trifluoracetat

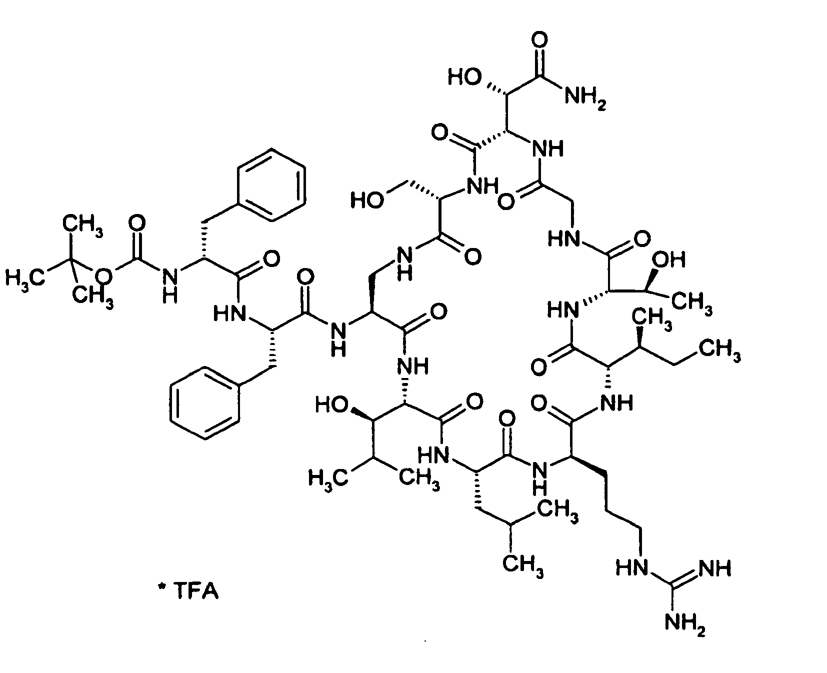

Das entschützte Amin (Beispiel 143A, 0.3 mg, 0.29 µmol) und das Dipeptid (1.2 mg, 2.86 µmol, 10 Äq.) werden unter Argonschutzgasatmosphäre in DMF (100 µl) vorgelegt und die Lösung auf 0°C gekühlt. Aus der NMM- Stammlösung (90.9 µmol/ml in DMF) wird die Base (16 µl, 147 µg, 1.43 µmol, 5 Äq.), HATU (1.1 mg, 2.95 µmol, 10.3 Äq.) und weitere NMM- Stammlösung (16 µl, 147 µg, 1.43 µmol, 5 Äq.) zugegeben und die Reaktion 15 min nachgerührt. Abschließend wird der Ansatz nochmals mit Base (40 µl, 367 µg, 3.62 µmol, 12.5 Äq.) versetzt und unter Rühren auf RT gebracht. Bei dieser Temperatur wird über Nacht nachgerührt. Zur Aufarbeitung wird im Vakuum aufkonzentriert und gelchromatographisch (Methode 45, Eluent: Methanol) feingereinigt. Von der Titelverbindung wird 0.3 mg (70.6% d. Th.) erhalten.
HPLC (Methode 12) Rₜ = 7.80 min.
LC-MS (Methode 18): Rₜ = 2.17 min; MS (ESIpos): *m*/*z* (%) = 1368 (5) [M+H]⁺, 634 (100); MS (ESIneg.): *m*/*z* (%) = 1367 (30) [M - H]⁻, 683 (100) [M - 2H]²⁻.

### Beispiel 145A

### Polymergebundenes [N²-Fluorenylmethoxycarbonyl-L-isoleucyl]-O³-(tert-butyl)-L-serin

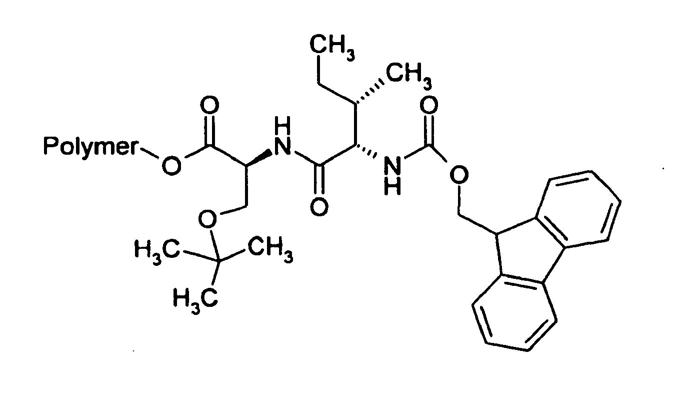

Die Fmoc-Schutzgruppe wird von dem Polymer (Beispiel 41A, 500 mg, 0.75 mmol), wie in Arbeitsvorschrift 7 beschrieben ist, abgespalten. Anschließend wird die am Harz gebundene entschützte Aminosäure mit N²-[(9H-Fluoren-9-ylmethoxy)carbonyl]-L-isoleucin (1.05 g, 2.98 mmol, 4 Äq.), DIEA (0.78 ml, 577 mg, 4.47 mmol, 6 Äq.) und TBTU (957 mg, 2.98 mmol, 4 Äquivalente) über Nacht zum F-moc geschützten Dipeptid umgesetzt. Analog der Arbeitsvorschrift 7 erfolgt die Aufarbeitung des Polymeres. Es wird das korrespondierende seitenkettengeschützte Dipeptid nach der Probeabspaltung bestätigt.
LC-MS (Methode 21): Rₜ = 2.68 min; MS (ESIpos): *m*/*z* (%) = 497.5 (60) [M+H]⁺, 441.4 (100); MS (ESIneg): *m*/*z* (%) = 495.5 (5) [M - H]⁻, 273.4 (100).

### Beispiel 146A

### Polymergebundenes [(N²-(9H-Fluoren-9-ylmethoxy)carbonyl)-N⁵-(imino{[(2,2,5,7,8-pentamethyl-3,4-dihydro-2H-chromen-6-yl)sulfonyl]amino}methyl)-D-ornithyl]-L-isoleucyl-O³-(tert-butyl)-L-serin

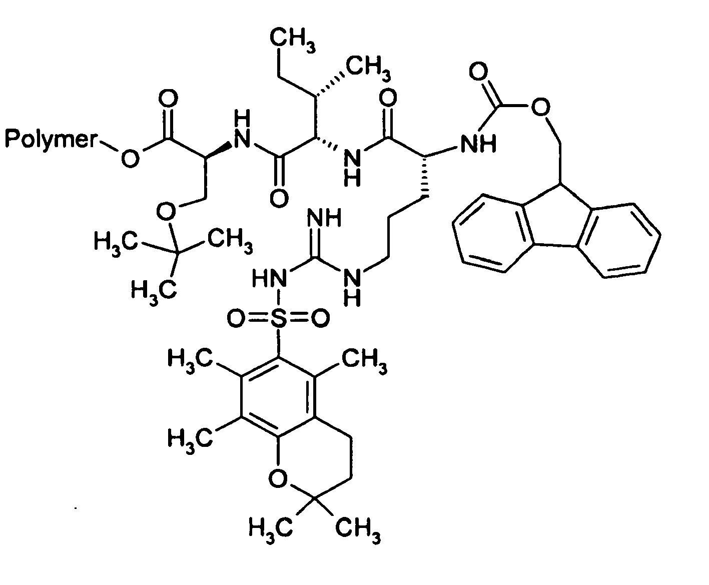

Die Fmoc-Schutzgruppe wird von dem Polymer (Beispiel 145A, 500 mg, 0.75 mmol), wie in Arbeitsvorschrift 7 beschrieben ist, abgespalten. Anschließend wird die am Harz gebundene entschützte Aminosäure mit (*N²*-(9*H*-Fluoren-9-ylmethoxy)carbonyl)-*N⁵*-(imino{[(2,2,5,7,8-pentamethyl-3,4-dihydro-2*H*-chromen-6-yl)sulfonyl]amino}methyl)-D-ornithin (740 mg, 1.12 mmol, 1.5 Äq.), DIEA (0.39 ml, 289 mg, 2.24 mmol, 3 Äq.) und TBTU (318 mg, 0.99 mmol, 2 Äquivalente) über Nacht zum F-moc geschützten Tripeptid umgesetzt. Analog der Arbeitsvorschrift 7 erfolgt die Aufarbeitung des Polymeres. Es wird das korrespondierende seitenkettengeschützte Dipeptid nach der Probeabspaltung bestätigt.
LC-MS (Methode 21): Rₜ = 2.89 min; MS (ESIpos): *m*/*z* (%) = 919.5 (100) [M+H]⁺; MS (ESIneg): *m*/*z* (%) = 917.7 (100) [M - H]⁻.

### Beispiel 147A

### Polymergebundenes [(N²-(9H-Fluoren-9-ylmethoxy)carbonyl)-L-leucyl]-[N⁵-(imino{[(2,2,5,7,8-pentamethyl-3,4-dihydro-2H-chromen-6-yl)sulfonyl]amino}methyl)-D-ornithyl]-L-isoleucyl-O³-(tert-butyl)-L-serin

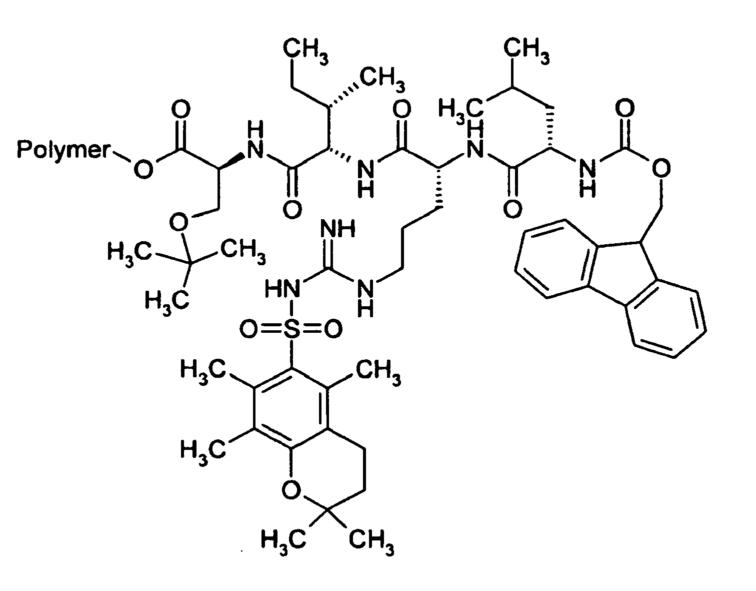

Die Fmoc-Schutzgruppe wird von dem Polymer (Beispiel 146A, 500 mg, 0.75 mmol), wie in Arbeitsvorschrift 7 beschrieben ist, abgespalten. Anschließend wird die am Harz gebundene entschützte Aminosäure mit *N²*-(9*H*-Fluoren-9-ylmethoxy)carbonyl-L-leucin (1.05 g, 2.98 mmol, 4 Äq.), DIEA (0.78 ml, 577 mg, 4.47 mmol, 6 Äq.) und TBTU (956 mg, 2.98 mmol, 4 Äquivalente) über Nacht zum F-moc geschützten Tetrapeptid umgesetzt. Analog der Arbeitsvorschrift 7 erfolgt die Aufarbeitung des Polymeres. Es wird das korrespondierende seitenkettengeschützte Dipeptid nach der Probeabspaltung bestätigt.
LC-MS (Methode 21): Rₜ = 3.27 min; MS (ESIpos): *m*/*z* (%) = 1033.3 (100) [M+H]⁺; MS (ESIneg): *m*/*z* (%) = 1032.2 (100) [M - H]⁻.

### Beispiel 148A

### [(3R)-N²-(tert-Butoxycarbonyl)-3-hydroxy-L-leucyl]-L-leucyl-[N⁵-(imino([(2,2,5,7,8-pentamethyl-3,4-dihydro-2H-chromen-6-yl)sulfonyl]amino}methyl)-D-ornithyl]}-L-isoleucyl-O³-(tert-butyl)-L-serin

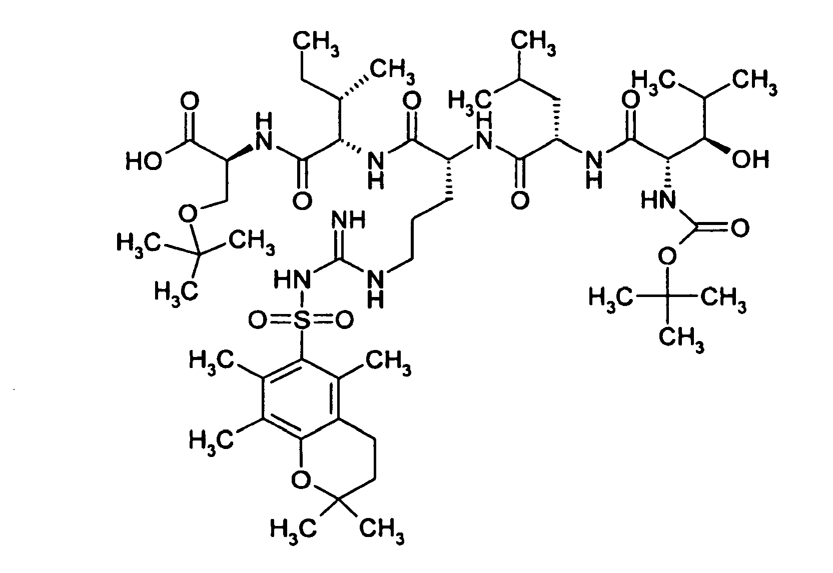

Die Fmoc-Schutzgruppe wird von dem Polymer (Beispiel 147A, 500 mg, 0.75 mmol), wie in Arbeitsvorschrift 7 beschrieben ist, abgespalten. Anschließend wird die am Harz gebundene entschützte Aminosäure mit (3*R*)-*N²*-(*tert*-Butoxycarbonyl)-3-hydroxy-L-leucin (276 mg, 1.12 mmol, 1.5 Äq.), DIEA (0.26 ml, 193 mg, 1.49 mmol, 2 Äq.) und TBTU (359 mg, 1.12 mmol, 1.5 Äquivalente) über Nacht zum Boc geschützten Pentapeptid umgesetzt. Dann wird mit DMF, THF und schließlich Dichlormethan gewaschen und mit einer Mischung aus Eisessig, Trifluorethanol und Dichlormethan (1+1+3) vom Harz abgespalten. Das rohe Reaktionsprodukt wird chromatographisch gereinigt. Die Titelverbindung wird in einer Ausbeute von 145 mg (19% d.Th.) isoliert.
LC-MS (Methode 21): Rₜ = 3.17 min; MS (ESIpos): *m*/*z* (%) = 1040.0 (100) [M+H]⁺; MS (ESIneg): *m*/*z* (%) = 1038.0 (100) [M - H]⁻.

### Beispiel 149A

### Benzyl L-Serinat-trifluoracetat

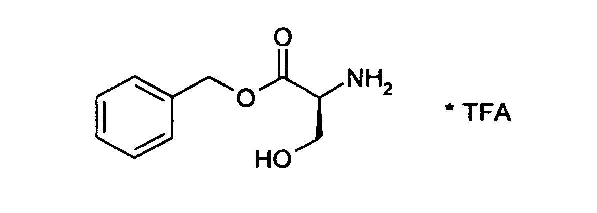

500 mg (1.69 mmol) Benzyl *N*-*tert*-Butoxycarbonyl-L-Serinat werden nach Arbeitsvorschrift 2 in 5.50 ml der Reagenzlösung umgesetzt. Das Produkt wird ohne weitere Reinigung umgesetzt. Ausbeute: 505 mg (1.63 mmol, 96% d. Th).
HPLC (Methode 3): Rₜ = 3.10 min.
LC-MS (Methode 22): Rₜ = 1.94 min; MS (ESIpos) *m*/*z* (%) = 196.0 (100) [M+H]⁺.

### Beispiel 150A

### Benzyl [N²-(tert-Butoxycarbonyl)-L-seryl]-L-serinat

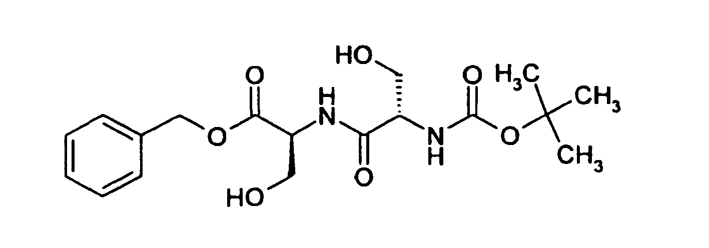

Die Verbindung aus Beispiel 149A (500 mg, 1.62 mmol) und *N*-*tert*-Butoxycarbonyl-L-serin (332 mg, 1.62 mmol, 1 Äquivalent) werden in Dichlormethan (8.2 ml) gelöst und auf 0 °C gekühlt. HATU (922 mg, 2.43 mmol, 1.5 Äquivalente) wird zugegeben, dann wird innerhalb von 15 min eine Lösung von N,N-Diisopropylethylamin (1.41 ml, 8.08 mmol, 5 Äquivalente) in Dichlormethan (5 ml)zugetropft. Der Ansatz wird dann 72 h bei Raumtemperatur gerührt, anschließend mit Natriumhydrogencarbonat gewaschen und die organische Phase getrocknet und eingeengt. Das Rohprodukt wird chromatographisch gereinigt (Methode 34). Produkthaltige Fraktionen werden vereinigt und lyophilisiert. Ausbeute: 308 mg (0.81 mmol, 50% d. Th.) als farbloser Feststoff.
HPLC (Methode 4): Rₜ = 3.81 min.
LC-MS (Methode 18): Rₜ = 1.98 min; MS (ESIpos): *m*/*z* (%) = 283.5 (100) [M-Boc + H]⁺; 383.6 (30) [M+H]⁺.

### Beispiel 151A

### Benzyl [N²-(tert-Butoxycarbonyl)-3-(3-pyridyl)-L-alanyl]-L-serinat

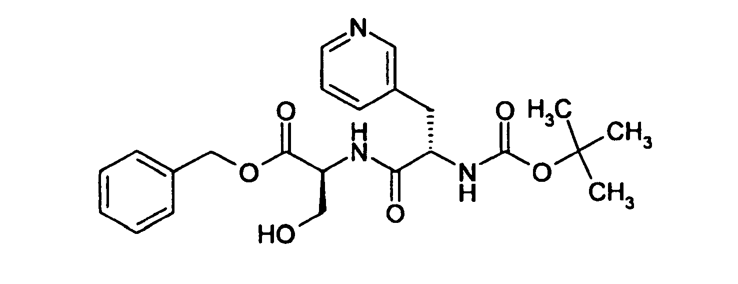

Gemäß der Darstellungsvorschrift der Verbindung des Beispiel 150A wird aus der Verbindung aus Beispiel 149A (780 mg, 2.52 mmol) und *N²*-*tert*.-Butoxycarbonyl-3-(3-pyridyl)-L-alanin (707 mg, 2.52 mmol) die Titelverbindung in einer Ausbeute von 780 mg (69% d. Th.) erhalten.
HPLC (Methode 5): Rₜ = 3.61 min.
LC-MS (Methode 22): Rₜ = 2.82 min, MS (ESIpos): *m*/*z* (%) = 444.2 (100), [M+H]⁺.

### Beispiel 152A

### Benzyl [N²-(tert-Butoxycarbonyl)-L-phenylalanyl]-L-serinat

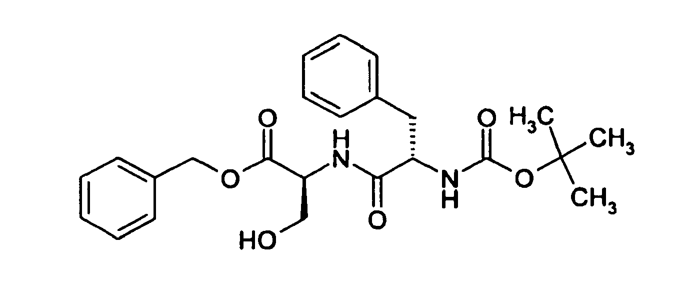

Gemäß der Darstellungsvorschrift der Verbindung des Beispiel 150A wird aus der Verbindung aus Beispiel 149A (350 mg, 1.13 mmol) und *N²*-*tert*.-Butoxycarbonyl-L-phenylalanin (300 mg, 1.13 mmol) die Titelverbindung in einer Ausbeute von 275 mg (55% d. Th.) erhalten.
HPLC (Methode 6): Rₜ = 4.50 min.
LC-MS (Methode 20): Rₜ = 2.23 min, MS (ESIpos): m/z (%) = 443.2 (100) [M+H]⁺; MS (ESIneg): m/z (%) = 441 (50) [M-H]⁻.
HR-TOF-MS (Methode 24): C₂₄H₃₁N₂O₆ ber. 443.2177, gef. 443.2180 [M+H]⁺.

### Beispiel 153A

### Benzyl [N²-(tert-Butoxycarbonyl)-L-threonyl]-O³-tert-butyl-L-serinat

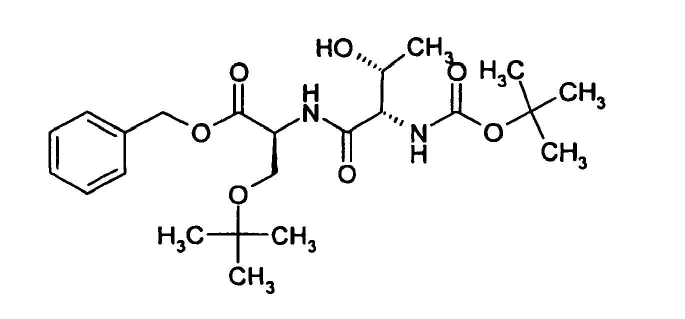

Gemäß der Darstellungsvorschrift der Verbindung des Beispiel 150A wird aus Benzyl *O³*-(*tert*-Butyl)-L-serinat (320 mg, 0.88 mmol) und *N²*-*tert*.-Butoxycarbonyl-threonin (192 mg, 0.88 mmol) die Titelverbindung in einer Ausbeute von 196 mg (49% d. Th.) erhalten.
HPLC (Methode 5): Rₜ = 4.73 min.
LC-MS (Methode 19): Rₜ = 2.56 min, MS (ESIpos): m/z (%) = 453.0 (100) [M+H]⁺.

### Beispiel 154A

### Benzyl [N²-(tert-Butoxycarbonyl)-L-allothreonyl]-L-serinat

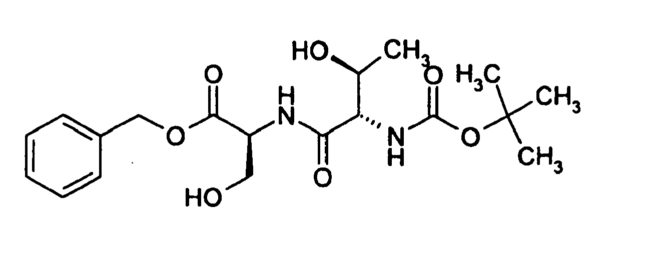

Benzyl L-serinat (314 mg, 1.02 mmol) und NMM (335 µl, 3.05 mmol, 3 Äquivalente) werden bei 0°C in DMF vorgelegt. Dann wird (*N²*-*tert*.-Butoxycarbonyl)-allothreonin (223 mg, 1.02 mmol, 1 Äquivalent) zugegeben gefolgt von TCTU (506 mg, 1.42 mmol, 1.4 Äquivalente). Der Ansatz wird über Nacht gerührt und dann nach Methode 45 chromatographisch aufgearbeitet. Man erhält die Titelverbindung in einer Ausbeute von 298 mg (73% d. Th.).
HPLC (Methode 5): Rₜ = 3.89 min.
LC-MS (Methode 19): Rₜ = 1.91 min, MS (ESIpos): m/z (%) = 397.0 (100) [M+H]⁺.
HR-TOF-MS (Methode 24): C₁₉H₂₉N₂O₇ ber. 397.1970, gef. 397.1971 [M+H]⁺.

### Beispiel 155A

### Benzyl [N²-(tert-Butoxycarbonyl)-L-alanyl]-L-serinat

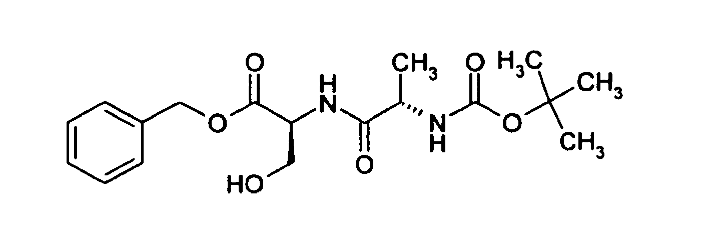

Gemäß der Darstellungsvorschrift der Verbindung des Beispiel 105A wird aus Benzyl L-serinat (300 mg, 0.97 mmol) und *N²*-*tert*.-Butoxycarbonyl-alanin (220 mg, 1.16 mmol) die Titelverbindung in einer Ausbeute von 277 mg (78% d. Th.) erhalten.
HPLC (Methode 5): Rₜ = 4.05 min.
LC-MS (Methode 20): Rₜ = 1.81 min, MS (ESIpos): m/z (%) = 367.2 (50) [M+H]⁺.

### Beispiel 156A

### Benzyl [N²-(tert-Butoxycarbonyl)-L-asparaginyl]-L-serinat

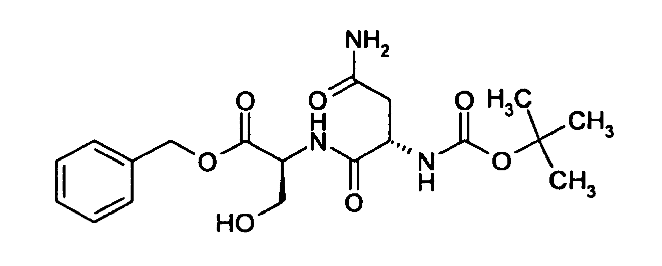

Gemäß der Darstellungsvorschrift der Verbindung des Beispiel 150A wird aus Benzyl L-serinat (610 mg, 1.97 mmol) und *N²*-*tert*.-Butoxycarbonyl-asparagin (458 mg, 1.97 mmol, 1 Äquivalent) die Titelverbindung in einer Ausbeute von 313 mg (39% d. Th.) erhalten.
HPLC (Methode 5): Rₜ = 3.70 min.
LC-MS (Methode 20): Rₜ = 1.78 min, MS (ESIpos): m/z (%) = 410.1 (100) [M+H]⁺.
HR-TOF-MS (Methode 24): C₁₉H₂₇N₃O₇ ber. 410.1922, gef. 410.1914 [M+H]⁺.

### Beispiel 157A

### Benzyl L-Seryl-L-serinat-trifluoracetat

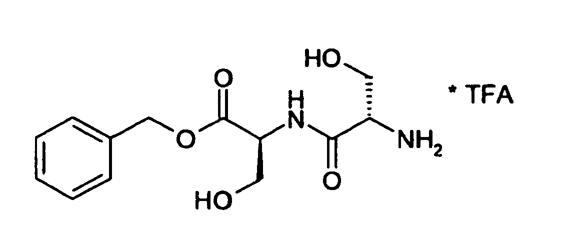

Die Verbindung aus Beispiel 150A (290 mg, 0.76 mmol) wird nach Arbeitsvorschrift 2 umgesetzt. Man erhält 451 mg (quant.) der rohen Titelverbindung, die ohne weitere Aufreinigung weiter umgesetzt wird.

LC-MS (Methode 22): Rₜ = 2.16 min; MS (ESIpos) *m*/*z* (%) = 283.0 (100) [M+H]⁺.

### Beispiel 158A

### Benzyl [3-(3-Pyridyl)-L-alanyl]-L-serinat bis-trifluoracetat

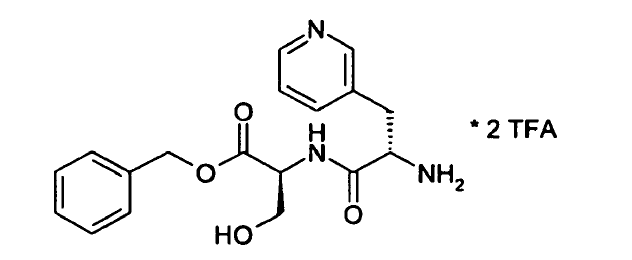

Die Verbindung aus Beispiel 151A (460 mg, 0.99 mmol) wird nach Arbeitsvorschrift 2 umgesetzt. Man erhält 565 mg (quant.) der rohen Titelverbindung, die ohne weitere Aufreinigung weiter umgesetzt wird.
HPLC (Methode 5): Rₜ = 3.03 min.
LC-MS (Methode 22): Rₜ = 2.23 min, MS (ESIpos): m/z (%) = 344.2 (30) [M+H]⁺.

### Beispiel 159A

### Benzyl L-Phenylalanyl-L-serinat-trifluoracetat

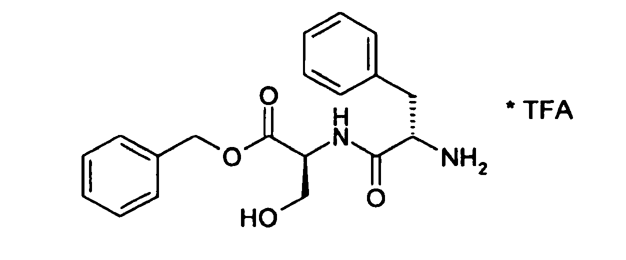

Die Verbindung aus Beispiel 152A (275 mg, 0.62 mmol) wird nach Arbeitsvorschrift 2 umgesetzt. Man erhält 285 mg (93% d. Th.) der rohen Titelverbindung, die ohne weitere Aufreinigung weiter umgesetzt wird.
HPLC (Methode 6): Rₜ = 3.33 min.
LC-MS (Methode 20): Rₜ = 1.14 min, MS (ESIpos): m/z (%) = 343.3 (100) [M+H]⁺; MS (ESIneg): m/z (%) = 341.3 (100) [M-H]⁻.

### Beispiel 160A

### Benzyl L-Threonyl-L-serinat-trifluoracetat

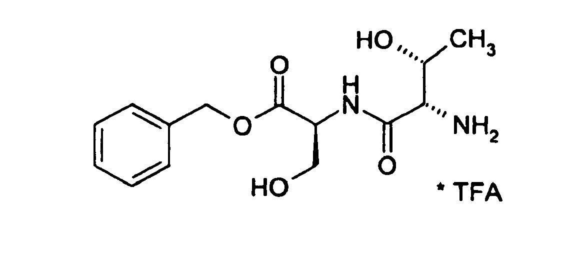

Die Verbindung aus Beispiel 153A (195 mg, 0.43 mmol) wird nach Arbeitsvorschrift 2 umgesetzt. Man erhält 235 mg (quant.) der rohen Titelverbindung, die ohne weitere Aufreinigung weiter umgesetzt wird.
HPLC (Methode 5): Rₜ = 3.20 min.
LC-MS (Methode 22): Rₜ = 2.32 min, MS (ESIpos): m/z (%) = 297 (100) [M+H]⁺.

### Beispiel 161A

### Benzyl L-Allothreonyl- L-serinat-trifluoracetat

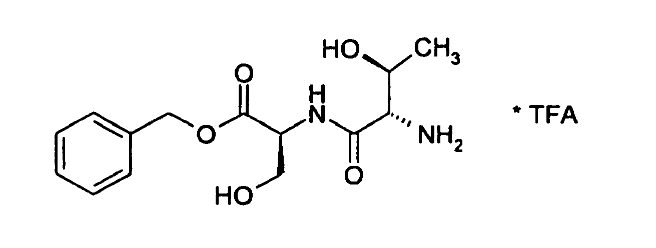

Die Verbindung aus Beispiel 154A (286 mg, 0.72 mmol) wird nach Arbeitsvorschrift 2 umgesetzt. Man erhält 326 mg (quant.) der rohen Titelverbindung, die ohne weitere Aufreinigung weiter umgesetzt wird.
HPLC (Methode 5): Rₜ = 3.16 min.
LC-MS (Methode 19): Rₜ = 1.38 min, MS (ESIpos): m/z (%) = 297 (15) [M+H]⁺.
HR-TOF-MS (Methode 24): C₁₄H₁₂N₂O₅ ber. 297.1445 , gef. 297.1436 [M+H]⁺.

### Beispiel 162A

### Benzyl L-Alanyl-L-serinat-trifluoracetat

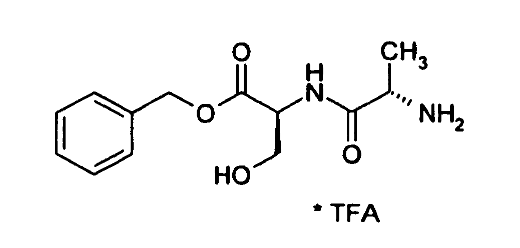

Die Verbindung aus Beispiel 155A (277 mg, 0.76 mmol) wird nach Arbeitsvorschrift 2 umgesetzt. Man erhält 285 mg (quant.) der rohen Titelverbindung, die ohne weitere Aufreinigung weiter umgesetzt wird.
HPLC (Methode 5): Rₜ = 3.20 min.
LC-MS (Methode 22): Rₜ = 2.35 min, MS (ESIpos): m/z (%) = 267 (100) [M+H]⁺.

### Beispiel 163A

### Benzyl L-Asparaginyl-L-serinat-trifluoracetat

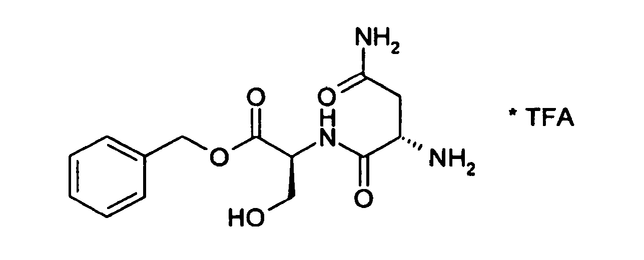

Die Verbindung aus Beispiel 156A (313 mg, 0.76 mmol) wird nach Arbeitsvorschrift 2 umgesetzt. Man erhält 428 mg (quant.) der rohen Titelverbindung, die ohne weitere Aufreinigung weiter umgesetzt wird.
HPLC (Methode 6): Rₜ = 2.96 min.
LC-MS (Methode 22): Rₜ = 2.07 min, MS (ESIpos): m/z (%) = 310 (100) [M+H]⁺.

### Beispiel 164A

### Benzyl [N²-(tert-Butoxycarbonyl)glycyl]-L-seryl-L-serinat

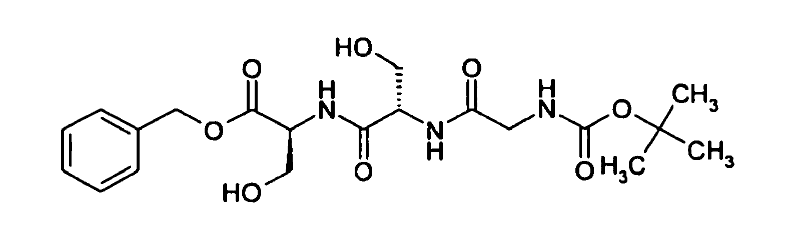

Die Verbindung aus Beispiel 157A (360 mg, 0.85 mmol) und *N*-*tert*-Butoxycarbonyl-glycin (149 mg, 0.85 mmol, 1 Äquivalent) werden in Dichlormethan (4.4 ml) gelöst und auf 0 °C gekühlt. HATU (485 mg, 1.28 mmol, 1.5 Äquivalente) wird zugegeben, dann wird innerhalb von 15 min eine Lösung von *N,N*-Diisopropylethylamin (0.74 ml, 4.26 mmol, 5 Äquivalente) in Dichlormethan (3 ml) zugetropft. Der Ansatz wird dann 24 h bei Raumtemp. gerührt, anschließend mit Natriumhydrogencarbonat gewaschen und die organische Phase getrocknet (Natriumsulfat) und eingeengt. Das Rohprodukt wird chromatographisch gereinigt (Methode 34). Produkthaltige Fraktionen werden vereinigt und lyophilisiert. Ausbeute: 220 mg (0.50 mmol, 59% d. Th.) als farbloser Feststoff.
HPLC (Methode 3) Rₜ = 3.70 min.
LC-MS (Methode 1): Rₜ = 1.93 min, MS (ESIpos): *m*/*z* (%) = 440.7 (100).

### Beispiel 165A

### Benzyl [N²-(tert-Butoxycarbonyl)glycyl]-[3-(3-pyridyl)-L-alanyl]-L-serinat

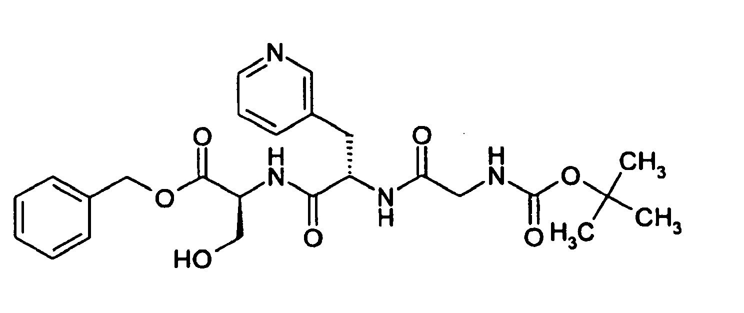

Gemäß der Darstellungsvorschrift der Verbindung aus Beispiel 164A mit DMF (1.8 ml) an Stelle von Dichlormethan als Lösemittel wird aus der Verbindung aus Beispiel 158A (565 mg, 0.95 mmol) und *N*-*tert*-Butoxycarbonyl-glycin (166 mg, 0.95 mmol, 1 Äquivalent) die Titelverbindung in einer Ausbeute von 405 mg (85% d. Th.) erhalten.
HPLC (Methode 5): Rₜ = 3.52 min.
LC-MS (Methode 20): Rₜ = 1.49 min, MS (ESIpos): m/z (%) = 501.4 (100) [M+H]⁺.

### Beispiel 166A

### Benzyl [N²-(tert-Butoxycarbonyl)glycyl]-L-phenylalanyl-L-serinat

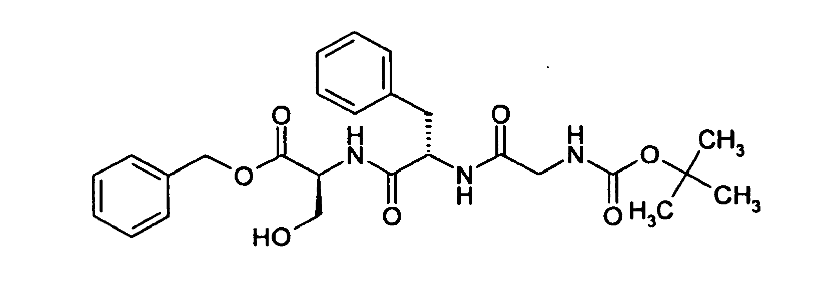

Gemäß der Darstellungsvorschrift der Verbindung aus Beispiel 164A mit DMF (2.0 ml) an Stelle von Dichlormethan als Lösemittel wird aus der Verbindung aus Beispiel 159A (285 mg, 0.58 mmol) und *N*-*tert*-Butoxycarbonyl-glycin (122 mg, 0.70 mmol, 1 Äquivalent) die Titelverbindung in einer Ausbeute von 237 mg (82% d. Th.) erhalten.
HPLC (Methode 6): Rₜ = 4.28 min.
LC-MS (Methode 19): Rₜ = 2.29 min, MS (ESIpos): m/z (%) = 500.2 (100) [M+H]⁺.
HR-TOF-MS (Methode 24): C₂₆H₃₄N₃O₇ ber. 500.2392, gef. 500.2399 [M+H]⁺.

### Beispiel 167A

### Benzyl [N²-tert.-Butoxycarbonyl-glycyl]-L-threonyl-L-serinat

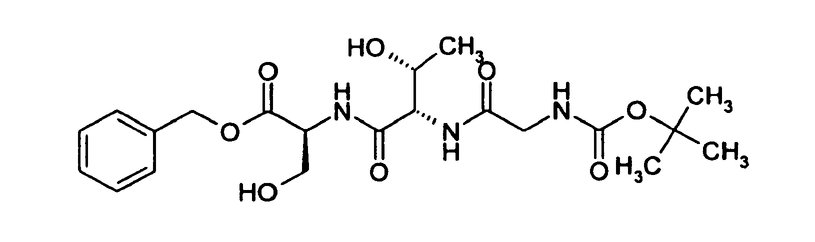

Gemäß der Darstellungsvorschrift der Verbindung aus Beispiel 164A mit DMF (1.8 ml) an Stelle von Dichlormethan als Lösemittel wird aus der Verbindung aus Beispiel 160A (173 mg, 0.49 mmol) und *N²*-*tert*-Butoxycarbonyl-glycin (103 mg, 0.59 mmol, 1.2 Äquivalente) die Titelverbindung in einer Ausbeute von 84 mg (34% d. Th.) erhalten.
HPLC (Methode 5): Rₜ = 4.45 min.
LC-MS (Methode 19): Rₜ = 2.38 min, MS (ESIpos): m/z (%) = 510.2 (100) [M+H]⁺.

### Beispiel 168A

### Benzyl [N²-tert.-Butoxycarbonyl-glycyl]-L-allothreonyl- L-serinat

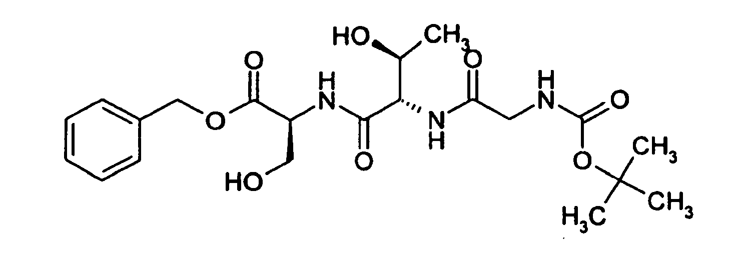

Die Verbindung aus Beispiel 161A (295 mg, 0.72 mmol) und *N²*-*tert*-Butoxycarbonyl-glycin (126 mg, 0.72 mmol, 1.0 Äquivalente) werden bei 0°C in DMF vorgelegt. Dann werden TCTU (329 mg, 0.87 mmol, 1.2 Äquivalente) und NMM (238 µl, 2.16 mmol, 3 Äquivalente) zugegeben. Der Ansatz wird über Nacht bei Raumtemperatur gerührt und dann nach Methode 45 chromatographisch aufgearbeitet. Produkthaltige Fraktionen werden vereinigt und nach der modifizierten Methode 32 (Gradient: 0-5 min 10%B, 5.01-35 min Rampe 85%B, 35.01-40 min 85%B) chromatographisch gereinigt. Man erhält die Titelverbindung in einer Ausbeute von 170 mg (52% d. Th.).
HPLC (Methode 5): Rₜ = 3.73 min.
LC-MS (Methode 19): Rₜ = 1.82 min, MS (ESIpos): m/z (%) = 454.1 (80) [M+H]⁺.
HR-TOF-MS (Methode 24): C₂₁H₃₂N₃O₈ ber. 454.2184 , gef. 454.2177 [M+H]⁺.

### Beispiel 169A

### Benzyl [N²-tert.-Butoxycarbonyl-glycyl]-L-alanyl-L-serinat

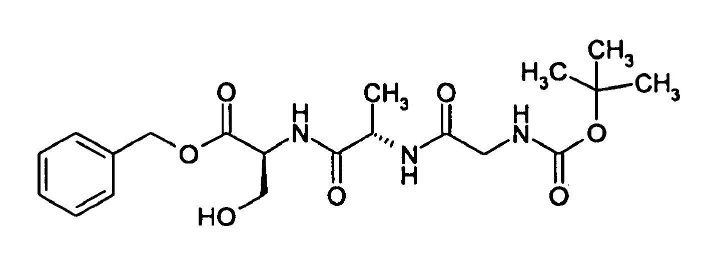

Gemäß der Darstellungsvorschrift der Verbindung aus Beispiel 164A mit DMF (1.8 ml) an Stelle von Dichlormethan als Lösemittel wird aus der Verbindung aus Beispiel 162A (285 mg, 0.75 mmol) und *N²*-*tert*-Butoxycarbonyl-glycin (157 mg, 0.90 mmol, 1.2 Äquivalente) die Titelverbindung in einer Ausbeute von 172 mg (43% d. Th.) erhalten.
HPLC (Methode 5): Rₜ = 3.83 min.
LC-MS (Methode 21): Rₜ = 1.85 min, MS (ESIpos): m/z (%) = 424.4 (100) [M+H]⁺.

### Beispiel 170A

### Benzyl [N²-(tert-Butoxycarbonyl)-glycyl]-L-asparaginyl-L-serinat

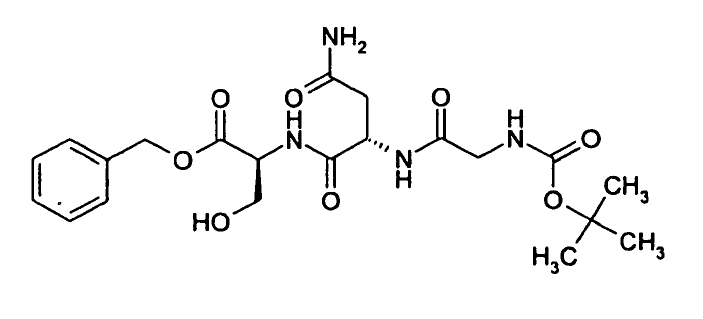

Gemäß der Darstellungsvorschrift der Verbindung aus Beispiel 164A mit DMF (2.0 ml) an Stelle von Dichlormethan als Lösemittel wird aus der Verbindung aus Beispiel 163A (428 mg, 0.80 mmol) und *N²*-*tert*-Butoxycarbonyl-glycin (167 mg, 0.96 mmol, 1.2 Äquivalente) die Titelverbindung in einer Ausbeute von 208 mg (56% d. Th.) erhalten.
HPLC (Methode 6): Rₜ = 3.60 min.
LC-MS (Methode 21): Rₜ = 1.75 min, MS (ESIpos): m/z (%) = 467.0 (100) [M+H]⁺.
HR-TOF-MS (Methode 24): C₂₁H₃₁N₄O₈ ber. 467.2137 , gef. 467.2142 [M+H]⁺.

### Beispiel 171A

### Benzyl Glycyl-L-seryl-L-serinat-trifluoracetat

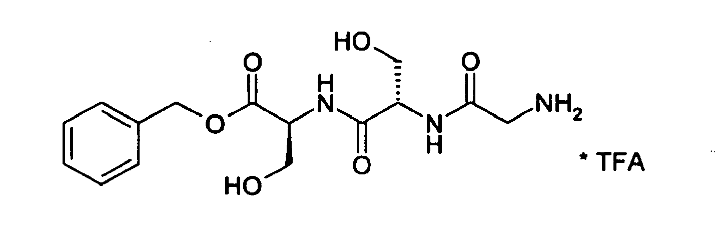

Die Verbindung aus Beispiel 164A (88 mg, 0.20 mmol) wird nach Arbeitsvorschrift 2 in 1.5 ml der Reagenzlösung umgesetzt. Das Rohprodukt wird ohne weitere Reinigung weiter umgesetzt. Ausbeute: 118 mg (quant.)
HPLC (Methode 3): Rₜ = 3.08 min.
LC-MS (Methode 22): Rₜ = 2.56 min, MS (ESIpos): m/z (%) = 340.0 (100) [M+H]⁺.

### Beispiel 172A

### Benzyl [Glycyl]-[3-(3-pyridyl)-L-alanyl]-L-serinat bis-trifluoracetat

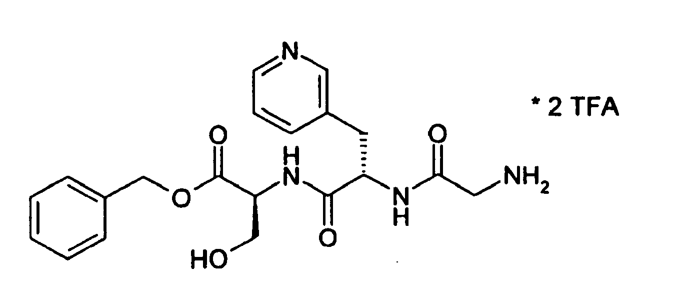

Die Verbindung aus Beispiel 165A (60 mg, 0.12 mmol) wird nach Arbeitsvorschrift 2 in 1.5 ml der Reagenzlösung umgesetzt. Das Rohprodukt wird ohne weitere Reinigung weiter umgesetzt. Ausbeute: 81 mg (quant.)
HPLC (Methode 5): Rₜ = 3.05 min.
LC-MS (Methode 22): Rₜ = 2.36 min, MS (ESIpos): m/z (%) = 401.0 (40) [M+H]⁺.

### Beispiel 173A

### Benzyl Glycyl-L-Phenylalanyl-L-serinat-trifluoracetat

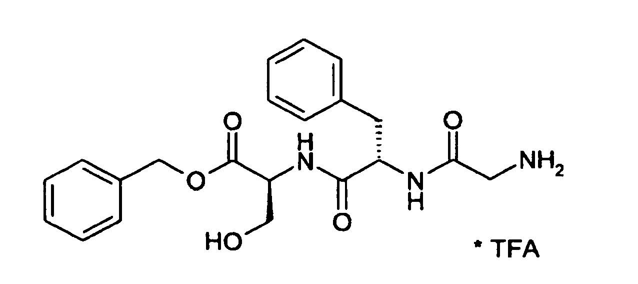

Die Verbindung aus Beispiel 166A (63 mg, 0.13 mmol) wird nach Arbeitsvorschrift 2 in 1.0 ml der Reagenzlösung umgesetzt. Das Rohprodukt wird ohne weitere Reinigung weiter umgesetzt. Ausbeute: 65 mg (96% d. Th.).
HPLC (Methode 6): Rₜ = 3.35 min.
LC-MS (Methode 22): Rₜ = 1.26 min, MS (ESIpos): m/z (%) = 400.1 (90) [M+H]⁺; MS (ESIneg): m/z (%) = 398.1 (100) [M-H]⁻.
HR-TOF-MS (Methode 24): C₂₁H₂₆N₃O₅ ber. 400.1867, gef. 400.1869 [M+H]⁺.

### Beispiel 174A

### Benzyl Glycyl-L-threonyl-L-serinat-trifluoracetat

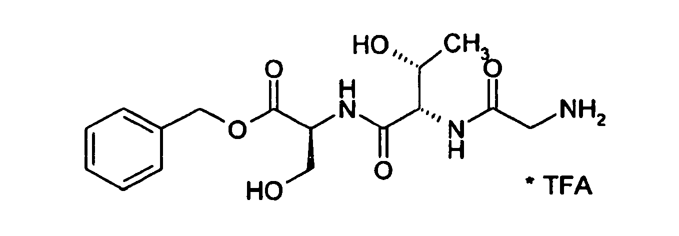

Die Verbindung aus Beispiel 167A (82 mg, 0.16 mmol) wird nach Arbeitsvorschrift 2 in 1.5 ml der Reagenzlösung umgesetzt. Das Rohprodukt wird chromatographisch gereinigt (Methode 44). Ausbeute: 43 mg (76% d. Th.).
HPLC (Methode 5): Rₜ = 3.16 min.
LC-MS (Methode 22): Rₜ = 2.35 min, MS (ESIpos): m/z (%) = 354 (50) µ[M+H]⁺.

### Beispiel 175A

### Benzyl Glycyl-L-allothreonyl-L-serinat-trifluoracetat

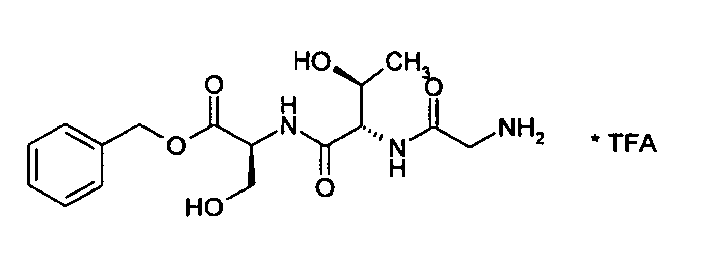

Die Verbindung aus Beispiel 168A (169 mg, 0.37 mmol) wird nach Arbeitsvorschrift 2 in 1.5 ml der Reagenzlösung umgesetzt. Das Rohprodukt (194 mg, ca. quant.) wird ohne weitere Reinigung weiter umgesetzt.
HPLC (Methode 5): Rₜ = 3.16 min.
LC-MS (Methode 21): Rₜ = 0.78 min, MS (ESIpos): m/z (%) = 354.3 (100) [M+H]⁺.
HR-TOF-MS (Methode 24): C₁₆H₂₄N₉O₆ ber. 354.1660 , gef. 354.1648 [M+H]⁺.

### Beispiel 176A

### Benzyl Glycyl-L-alanyl-L-serinat-trifluoracetat

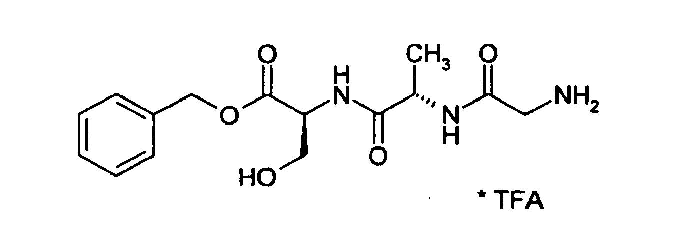

Die Verbindung aus Beispiel 169A (172 mg, 0.32 mmol) wird nach Arbeitsvorschrift 2 in 3.0 ml der Reagenzlösung umgesetzt. Das Rohprodukt wird ohne Reinigung weiter umgesetzt. Ausbeute: 175 mg (99% d. Th.).
HPLC (Methode 5): Rₜ = 3.20 min.
LC-MS (Methode 22): Rₜ = 2.41 min, MS (ESIpos): m/z (%) = 324 (40) [M+H]⁺.

### Beispiel 177A

### Benzyl Glycyl-L-asparaginyl-L-serinat-trifluoracetat

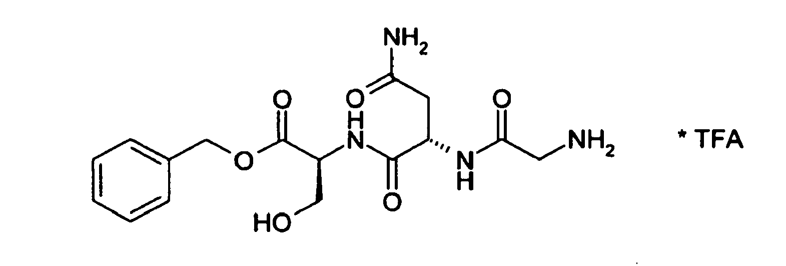

Die Verbindung aus Beispiel 170A (60 mg, 0.13 mmol) wird nach Arbeitsvorschrift 2 in 1.0 ml der Reagenzlösung umgesetzt. Das Rohprodukt wird ohne Reinigung weiter umgesetzt. Ausbeute: 62 mg (quant.).
HPLC (Methode 6): Rₜ = 2.89 min.
LC-MS (Methode 22): Rₜ = 2.14 min, MS (ESIpos): m/z (%) = 367 (100) [M+H]⁺.
HR-TOF-MS (Methode 24): C₁₆H₂₃N₄O₆ ber. 367.1613 , gef. 367.1609 [M+H]⁺.

### Beispiel 178A

### Benzyl [(3R)-N²-(tert-Butoxycarbonyl)-3-hydroxy-L-leucyl]-L-leucyl-D-arginyl-L-isoleucyl-L-allothreonyl-glycyl-L-seryl-L-serinat-trifluoracetat

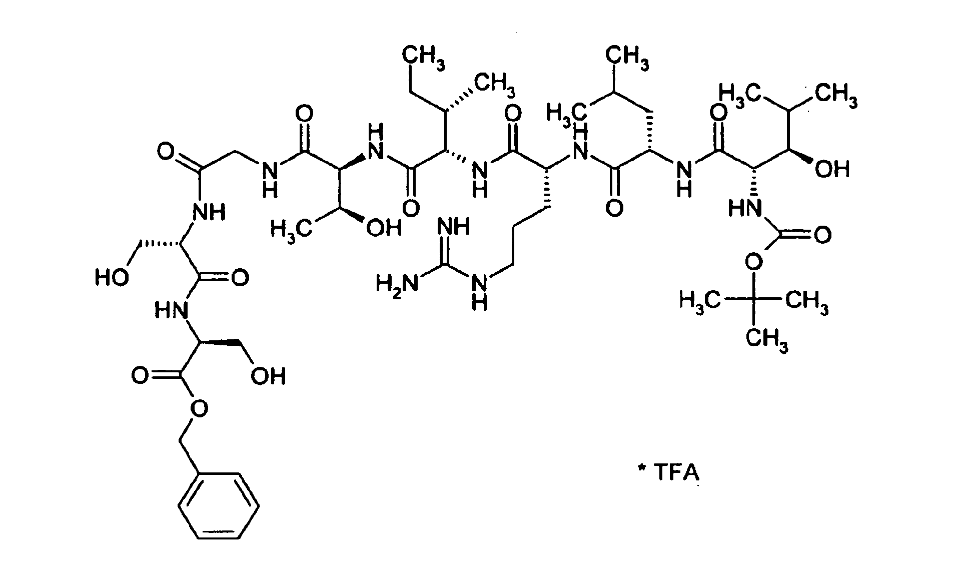

Die Verbindungen aus Beispiel 32A (75 mg, 38 µmol) und 171A (26 mg, 45 µmol, 1.2 Äquivalente) werden in DMF (950 µl) gelöst und die Lösung auf -20 °C gekühlt. 4-Methylmorpholin (29 µl, 263 µmol, 7 Äquivalente) und HATU (23 mg, 60 µmol, 1.6 Äquivalente) werden zugegeben und der Ansatz 16 h bei Raumtemperatur gerührt. Dann wird der gesamte Ansatz auf eine HPLC-Säule gegeben und nach Methode 33 chromatographisch gereinigt. Produkthaltige Fraktionen werden vereinigt und lyophilisiert. Man erhält 15 mg (34% d. Th.) der Titelverbindung als farblosen Feststoff.
HPLC (Methode 5): Rₜ = 3.89 min.
LC-MS (Methode 19): Rₜ = 1.81 min, MS (ESIpos): m/z (%) = 1053 (90) [M+H]⁺.

### Beispiel 179A

### Benzyl [(3R)-N²-(tert-Butoxycarbonyl)-3-hydroxy-L-leucyl]-L-leucyl-L-arginyl-L-isoleucyl-L-allothreonyl-glycyl-[3-(3-pyridyl)-L-alanyl]-L-serinat-trifluoracetat

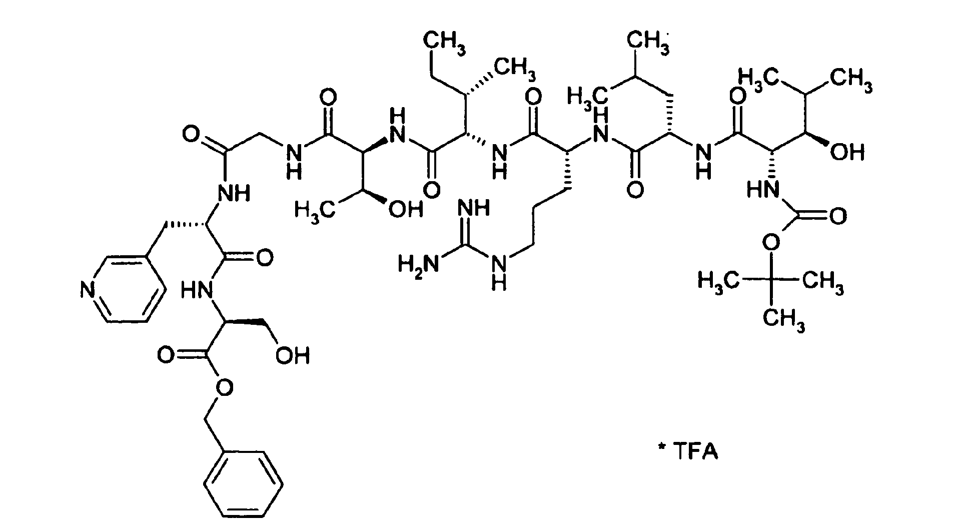

Gemäß der Darstellungsvorschrift der Verbindung aus Beispiel 178A wird aus der Verbindung aus Beispiel 32A (80 mg, 83 µmol) und der Verbindung aus Beispiel 172A (79 mg, 125 µmol, 1.5 Äquivalente) die Titelverbindung in einer Ausbeute von 34 mg (33% d. Th.) erhalten.
HPLC (Methode 5): Rₜ = 3.75 min.
LC-MS (Methode 18): Rₜ = 1.52 min, MS (ESIpos): m/z (%)= 557.6 (100) [M+2H]²⁺; 1113.8 (10) [M+H]⁺.

### Beispiel 180A

### Benzyl [(3R)-N²-(tert-Butoxycarbonyl)-3-hydroxy-L-leucyl]-L-leucyl-D-arginyl-L-isoleucyl-L-allothreonyl-glycyl-L-phenylalanyl-L-serinat-trifluoracetat

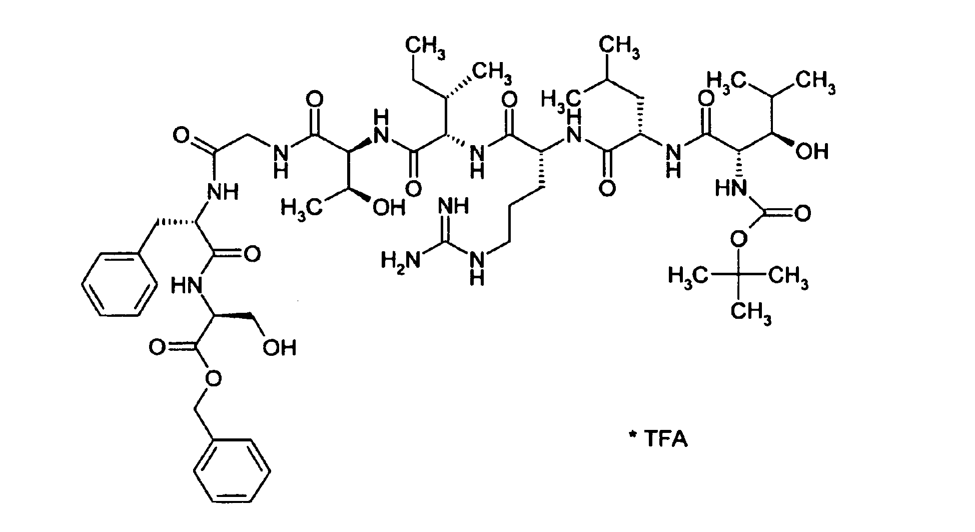

Gemäß der Darstellungsvorschrift der Verbindung aus Beispiel 178A wird aus der Verbindung aus Beispiel 32A (82 mg, 97 µmol) und der Verbindung aus Beispiel 173A (65 mg, 126 µmol, 1.3 Äquivalente) die Titelverbindung in einer Ausbeute von 39 mg (33% d. Th.) erhalten.
HPLC (Methode 6): Rₜ = 3.96 min.
LC-MS (Methode 19): Rₜ = 1.87 min, MS (ESIpos): m/z (%) = 506.8 (90) [M+2H]²⁺; 1112.5 (100) [M+H]⁺; MS (ESIneg): m/z (%) = 1110.5 (100) [M-H]⁻.
HR-TOF-MS (Methode 24): C₅₄H₈₆N₁₁O₁₄ ber. 1112.6351, gef. 1112.6343 [M+H]⁺.

### Beispiel 181A

### Benzyl [(3R)-N²-(tert-Butoxycarbonyl)-3-hydroxy-L-leucyl]-L-leucyl-D-arginyl-L-isoleucyl-L-allothreonyl-glycyl-L-threonyl-L-serinat-trifluoracetat

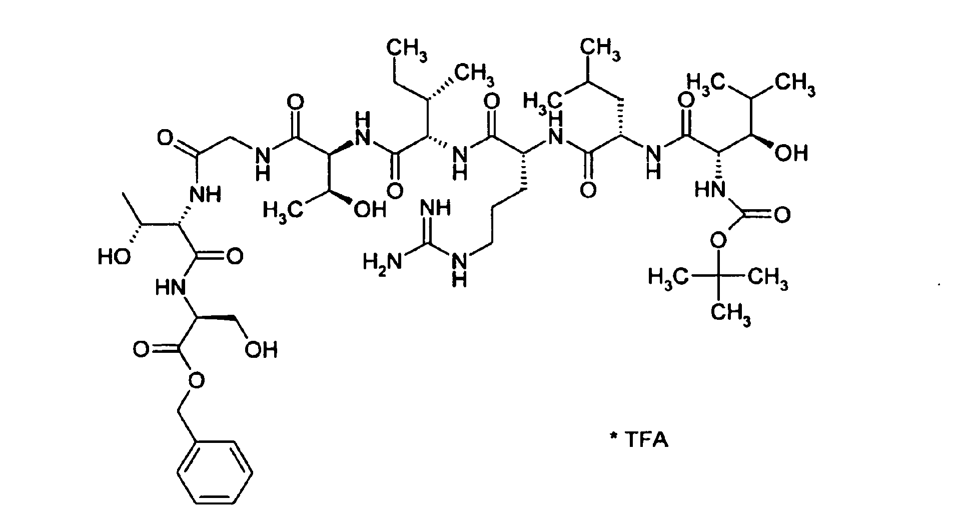

Gemäß der Darstellungsvorschrift der Verbindung aus Beispiel 178A wird aus der Verbindung aus Beispiel 32A (67 mg, 80 µmol) und der Verbindung aus Beispiel 174A (31 mg, 88 µmol, 1.1 Äquivalente) die Titelverbindung in einer Ausbeute von 21 mg (20% d. Th.) erhalten.
HPLC (Methode 5): Rₜ = 3.89 min.
LC-MS (Methode 19): Rₜ = 1.71 min, MS (ESIpos): m/z (%) = 1066.7 (100) [M+H]⁺.
HR-TOF-MS (Methode 24): C₄₉H₈₄N₁₁O₁₅ ber. 1066.6143, gef. 1066.6130 [M+H]⁺.

### Beispiel 182A

### Benzyl [(3R)-N²-(tert-Butoxycarbonyl)-3-hydroxy-L-leucyl]-L-leucyl-D-arginyl-L-isoleucyl-L-allothreonyl-glycyl-L-allothreonyl- L-serinat-trifluoracetat

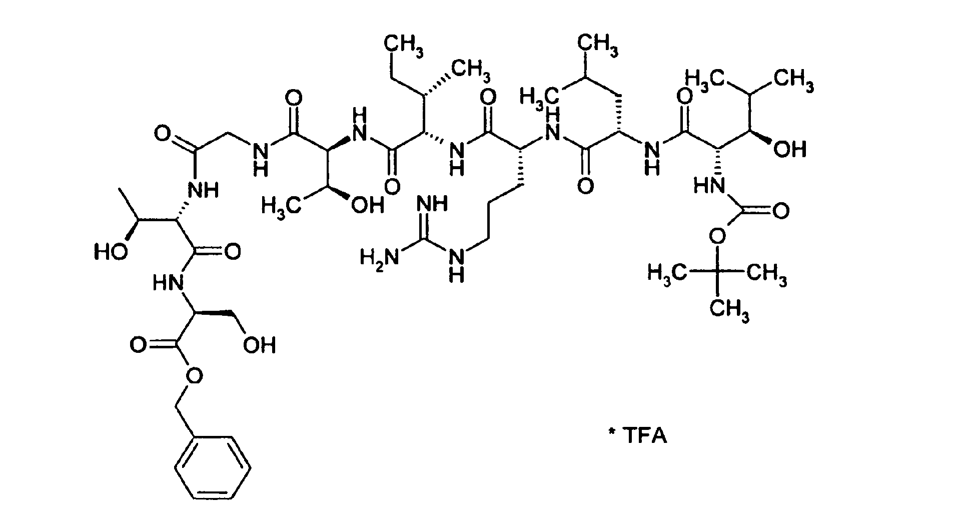

Gemäß der Darstellungsvorschrift der Verbindung aus Beispiel 178A werden die Verbindung aus Beispiel 32A (57 mg, 59 µmol) und die Verbindung aus Beispiel 175A (48 mg, 71 µmol, 1.2 Äquivalente) umgesetzt. Die Aufarbeitung erfolgt an Sephadex LH20 (Methode 45) gefolgt von Chromatographie nach der modifizierten Methode 44 (Gradient: 0-5 min 5% B, Rampe, 30-35 min 75% B, 35.01-40 min 75% B). Die Titelverbindung wird in einer Ausbeute von 65 mg (93% d. Th.) erhalten.
HPLC (Methode 6): Rₜ = 3.70 min.
LC-MS (Methode 19): Rₜ = 1.70 min, MS (ESIpos): m/z (%) = 483.9 (100) [M-Boc+2H]²⁺, 1066.6 (70) [M+H]⁺.
HR-TOF-MS (Methode 24): C₄₉H₈₄N₁₁O₁₅ ber. 1066.6143, gef. 1066.6134 [M+H]⁺.

### Beispiel 183A

### Benzyl (3R)-N²-(tert-Butoxycarbonyl)-3-hydroxy-L-leucyl]-L-leucyl-D-arginyl-L-isoleucyl-L-allothreonyl-glycyl-L-alanyl-L-serinat-trifluoracetat

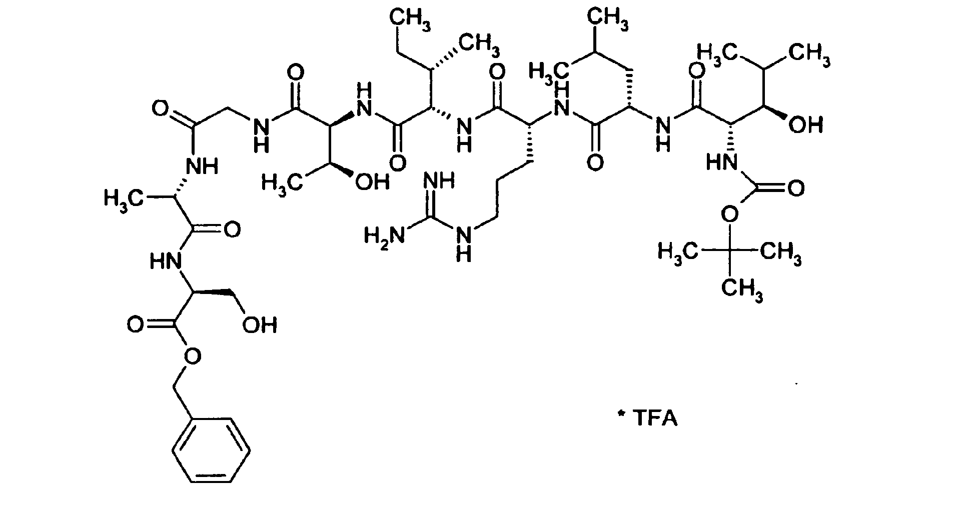

Gemäß der Darstellungsvorschrift der Verbindung aus Beispiel 178A und nach einer zusätzlichen chromatographischen Reinigung (Kromasil RP-18 5 µm, 100 Å, 250 x 20 mm; Eluens A: Wasser + 0.05% TFA, Eluens B: Acetonitril + 0.05% TFA: Fluss: 10 ml/min; 60% A, 40% B isokratisch) wird aus der Verbindung aus Beispiel 32A (80 mg, 84 µmol) und der Verbindung aus Beispiel 176A (60 mg, 109 µmol, 1.3 Äquivalente) die Titelverbindung in einer Ausbeute von 48 mg (50% d. Th.) erhalten.
HPLC (Methode 5): Rₜ = 3.99 min.
LC-MS (Methode 19): Rₜ = 1.70 min, MS (ESIpos): m/z (%) = 1036.6 (100) [M+H]⁺.
HR-TOF-MS (Methode 24): C₄₈H₈₁N₁₁O₁₄ ber. 1036.6038, gef. 1036.6027 [M+H]⁺.

### Beispiel 184A

### Benzyl [(3R)-N²-(tert-Butoxycarbonyl)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]}-L-isoleucyl-[O³-(tert-butyl)-L-seryl]-glycyl-[(3S)-3-hydroxy-L-asparaginyl]-L-serinat

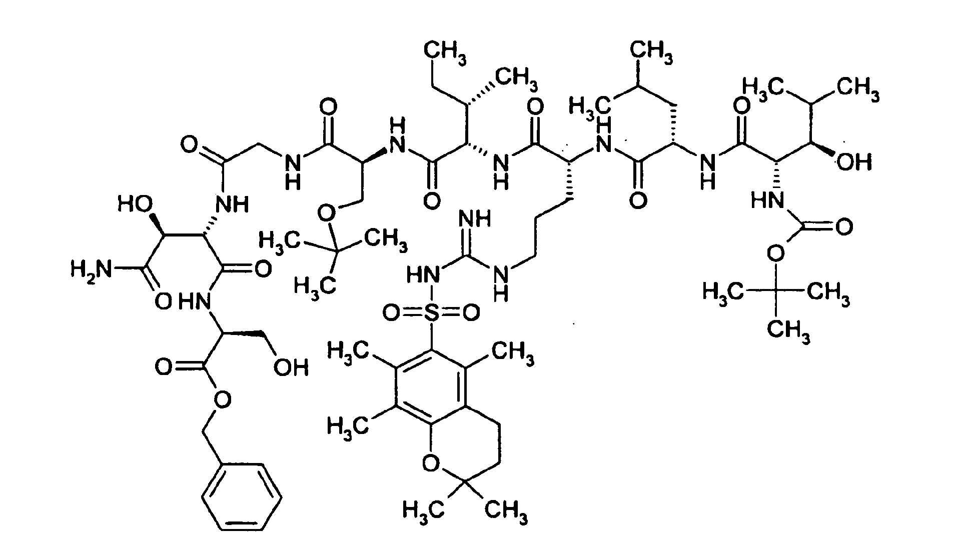

Gemäß der Darstellungsvorschrift der Verbindung aus Beispiel 178A und nach einer zusätzlichen chromatographischen Reinigung (Kromasil RP-18 5 µm, 100 Ä, 250 x 20 mm; Eluens A: Wasser + 0.05% TFA, Eluens B: Acetonitril + 0.05% TFA: Fluss: 10 ml/min; 60% A, 40% B isokratisch) wird aus der Verbindung aus Beispiel 148A (87 mg, 84 µmol) und der Verbindung aus Beispiel 36A (50 mg, 101 µmol, 1.2 Äquivalente) die Titelverbindung in einer Ausbeute von 62 mg (49% d. Th.) erhalten.
HPLC (Methode 5): Rₜ = 4.40 min.
LC-MS (Methode 19): Rₜ = 2.97 min, MS (ESIpos): m/z (%) = 1403.7 (100) [M+H]⁺.
HR-TOF-MS (Methode 24): C₆₆H₁₀₇N₁₂O₁₉S ber. 1403.7491, gef. 1403.7517 [M+H]⁺.

### Beispiel 185A

### Benzyl [(3R)-N²-(tert-Butoxycarbonyl)-3-hydroxy-L-leucyl]-L-leucyl-D-arginyl-L-isoleucyl-L-allothreonyl-glycyl-L-asparaginyl-L-serinat-trifluoracetat

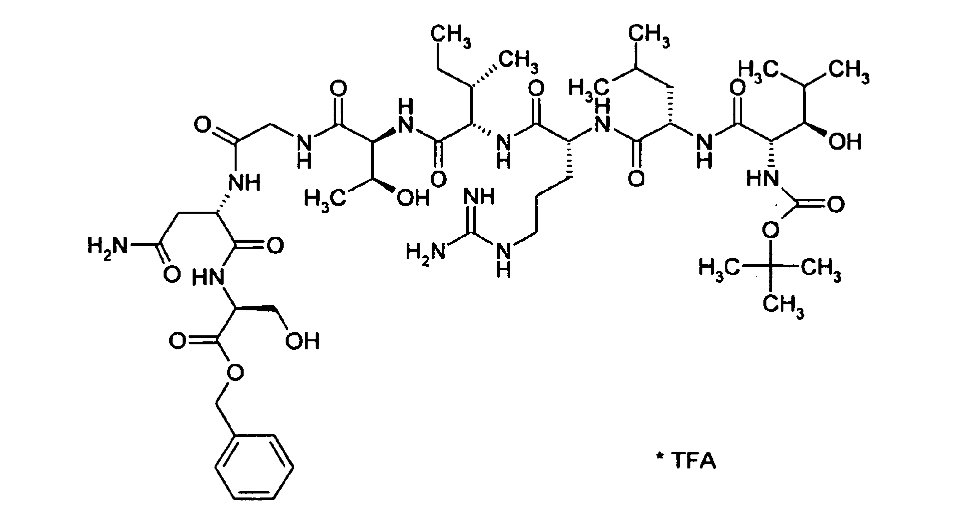

Gemäß der Darstellungsvorschrift der Verbindung aus Beispiel 178A wird aus der Verbindung aus Beispiel 32A (100 mg, 118 µmol) und der Verbindung aus Beispiel 177A (62 mg, 130 µmol, 1.1 Äquivalente) die Titelverbindung in einer Ausbeute von 48 mg (31% d. Th.) erhalten.
HPLC (Methode 6): Rₜ = 3.66 min.
LC-MS (Methode 22): Rₜ = 1.66 min, MS (ESIpos): m/z (%) = 490.3 (100) [M-Boc +2H]²⁺, 1079.6 (80) [M+H]⁺.
HR-TOF-MS (Methode 24): C₄₉H₈₃N₁₂O₁₅ ber. 1079.6096, gef. 1079.6090 [M+H]⁺.

### Beispiel 186A

### [(3R)-N²-(tert-Butoxycarbonyl)-3-hydroxy-L-leucyl]-L-leucyl-D-arginyl-L-isoleucyl-L-allothreonyl-glycyl-L-seryl-L-serin-trifluoracetat

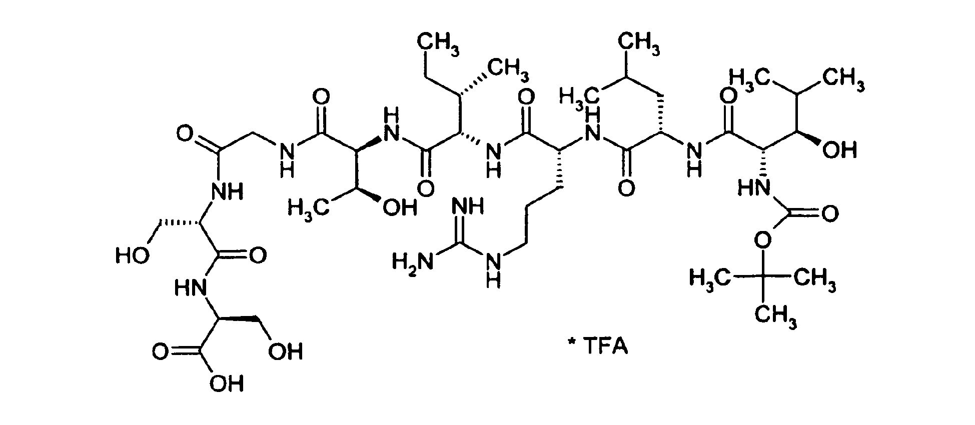

Die Verbindung aus Beispiel 178A (21 mg, 18 µmol) wird in 750 µl Essigsäure in Gegenwart von 5 mg 10% Palladium-Kohle bei Raumtemperatur und Normaldruck über 2 h hydriert. Man filtriert vom Katalysator ab und trocknet am Ölpumpenvakuum bis zur Gewichtskonstanz. Die Titelverbindung wird in quantitativer Ausbeute (20 mg) erhalten und ohne Reinigung weiter umgesetzt.
HPLC (Methode 5): Rₜ = 3.56 min.
LC-MS (Methode 19): Rₜ = 1.72 min, MS (ESIpos): m/z (%) = 963.0 (90) [M+H]⁺.
HR-TOF-MS (Methode 24): C₄₁H₇₆N₁₁O₁₅ ber. 962.5517, gef. 962.5537 [M+H]⁺.

### Beispiel 187A

### [(3R)-N²-(tert-Butoxycarbonyl)-3-hydroxy-L-leucyl]-L-leucyl-D-arginyl-L-isoleucyl-L-allothreonyl-glycyl-[3-(3-pyridyl)-L-alanyl]-L-serin-bis-trifluoracetat

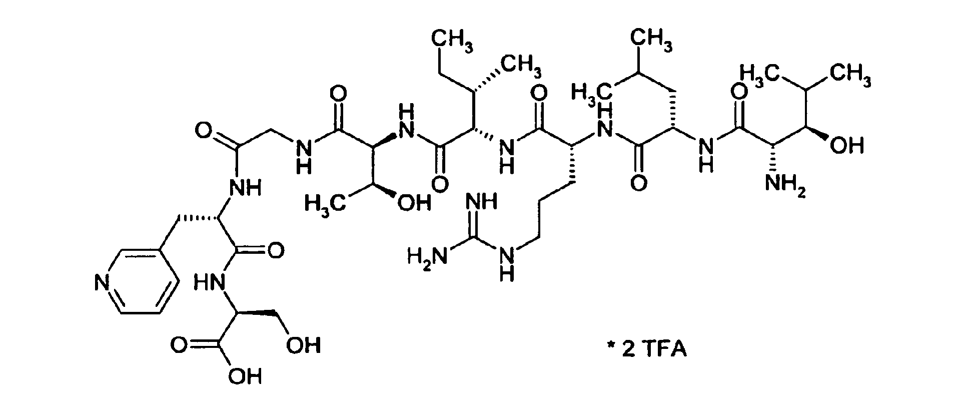

Gemäß der Herstellungsvorschrift der Verbindung aus Beispiel 186A wird die Verbindung aus Beispiel 179A (55 mg, 45 µmol) hydriert. Nach dem Einengen des Rohproduktes wird dieses direkt der Boc-Abspaltung nach Arbeitsvorschrift 2 unterworfen und erneut eingeengt. Man erhält 41 mg (88% d. Th.) der Titelverbindung, die ohne weitere Reinigung weiter umgesetzt werden.
HPLC (Methode 5): Rₜ = 3.56 min.
LC-MS (Methode 22): Rₜ = 2.19 min, MS (ESIpos): m/z (%) = 462 (40) [M+H]⁺; (ESIneg): m/z (%) = [M-H]⁻.
HR-TOF-MS (Methode 24): C₄₁H₇₁N₁₇O₁₂ ber. 923.5309, gef. 923.5321 [M+H]⁺.

### Beispiel 188A

### [(3R)-N²-(tert-Butoxycarbonyl)-3-hydroxy-L-leucyl]-L-leucyl-D-arginyl-L-isoleucyl-L-allothreonyl-glycyl-L-phenylalanyl-L-serin-trifluoracetat

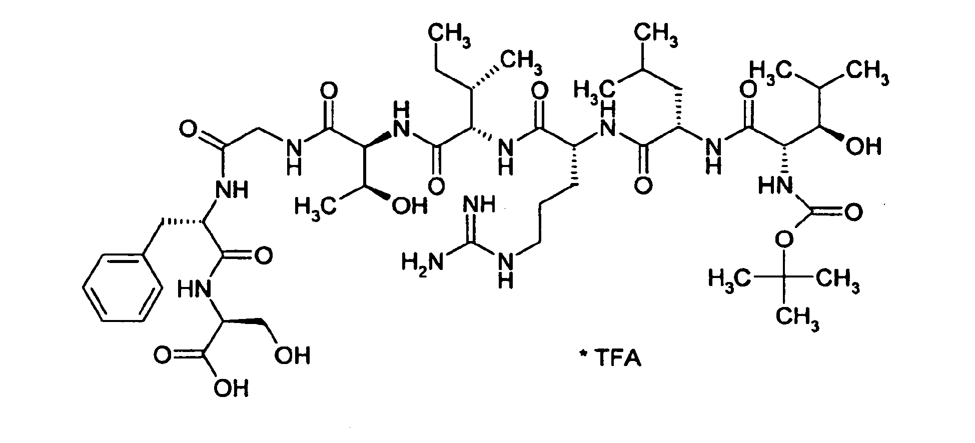

Gemäß der Herstellungsvorschrift zur Darstellung der Verbindung aus Beispiel 186A wird die Verbindung aus Beispiel 180A (39 mg, 32 µmol) hydriert. Man erhält 36 mg (quant.) der Titelverbindung, die ohne weitere Reinigung weiter umgesetzt werden.
HPLC (Methode 6): Rₜ = 3.55 min.
LC-MS (Methode 18): Rₜ = 1.75 min, MS (ESIpos): m/z (%) = 462 (100) [M-Boc+2 H]²⁺, 1022.1 (10) [M+H]⁺; (ESIneg): m/z (%) = 1020.8 [M-H]⁻.
HR-TOF-MS (Methode 24): C₄₇H₈₀N₁₁O₁₄ ber. 1022.5881, gef. 1022.5876 [M+H]⁺.

### Beispiel 189A

### [(3R)-N²-(tert-Butoxycarbonyl)-3-hydroxy-L-leucyl]-L-leucyl-D-arginyl-L-isoleucyl-L-allothreonyl-glycyl-L-threonyl-L-serin-bis-trifluoracetat

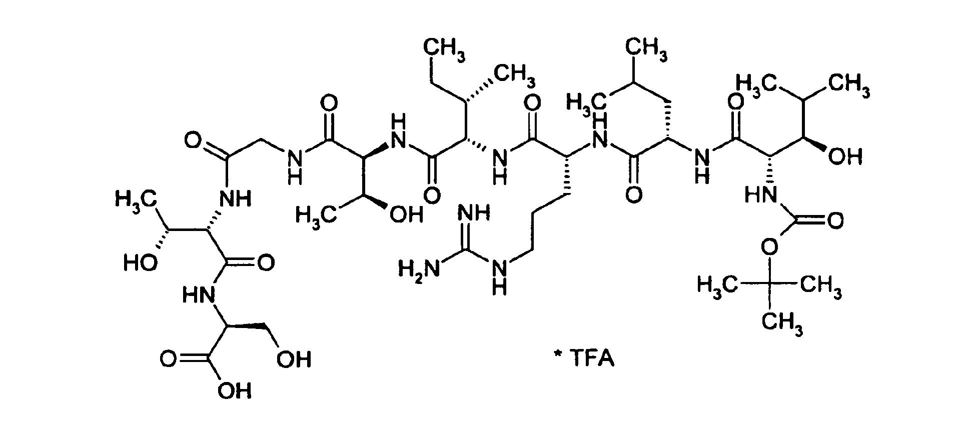

Gemäß der Herstellungsvorschrift der Verbindung aus Beispiel 186A wird die Verbindung aus Beispiel 181A (21 mg, 18 µmol) hydriert. Man erhält 18 mg (98% d. Th.) der Titelverbindung als farblosen Feststoff.
HPLC (Methode 5): Rₜ = 3.58 min.
LC-MS (Methode 19): Rₜ = 1.58 min, MS (ESIpos): m/z (%) = 976.6 (80) [M+H]⁺.

### Beispiel 190A

### [(3R)-3-Hydroxy-L-leucyl]-L-leucyl-D-arginyl-L-isoleucyl-L-allothreonyl-glycyl-L-allothreonyl-L-serin-bis-trifluoracetat

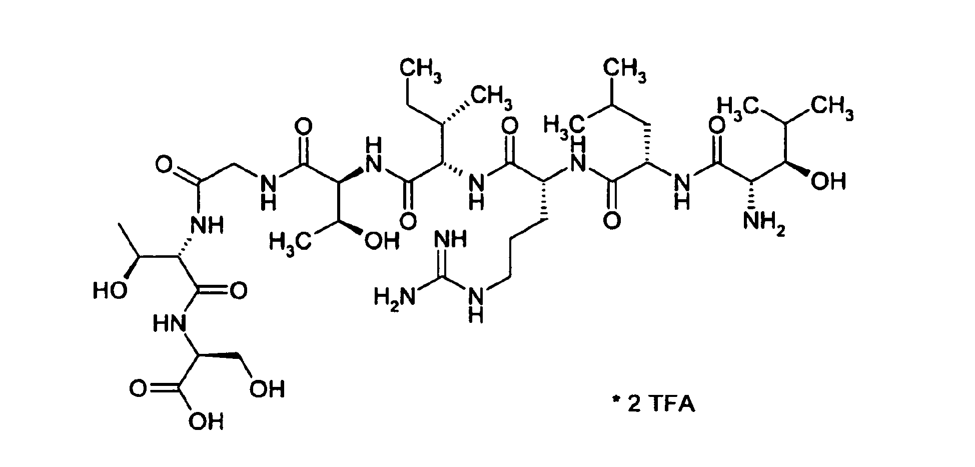

Die Verbindung aus Beispiel 197A (44 mg, 37 µmol) wird in Methanol (5.0 ml) gelöst, 10% Pd/C (20 mg) wird zugesetzt und bei Raumtemperatur und Normaldruck 2 h hydriert. Dann filtriert man vom Katalysator ab und engt das Filtrat ein. Man erhält 42 mg (quant.) der rohen Titelverbindung, die ohne Aufreinigung weiter umgesetzt wird.
HPLC (Methode 5): Rₜ = 3.00 min.
LC-MS (Methode 22): Rₜ = 2.32 min, MS (ESIpos): m/z (%) = 439 (100) [M+2H]²⁺; MS (ESIneg): m/z (%) = 875 (100) [M-H]⁻.
HR-TOF-MS (Methode 24): C₃₇H₆₉N₁₁O₁₃ ber. 876.5150, gef. 876.5146 [M+H]⁺.

### Beispiel 191A

### [(3R)-N²-(tert-Butoxycarbonyl)-3-hydroxy-L-leucyl]-L-leucyl-D-arginyl-L-isoleucyl-L-allothreonyl-glycyl-L-alanyl-L-serin-trifluoracetat

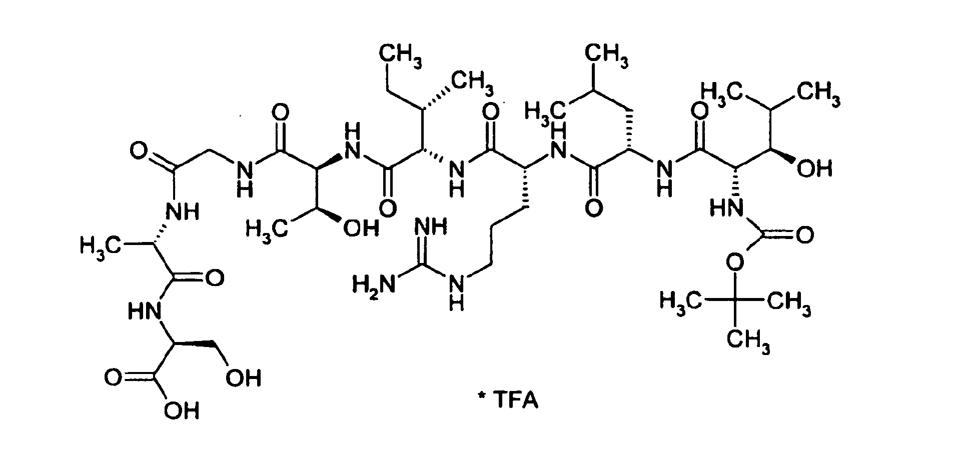

Gemäß der Herstellungsvorschrift der Verbindung aus Beispiel 186A wird die Verbindung aus Beispiel 183A (47 mg, 41 µmol) hydriert. Man erhält 43 mg (99% d. Th.) der Titelverbindung als Feststoff.
HPLC (Methode 5): Rₜ = 3.62 min.
LC-MS (Methode 19): Rₜ = 1.58 min, MS (ESIpos): m/z (%) = 946.5 (100) [M+H]⁺.
HR-TOF-MS (Methode 24): C₄₁H₇₆N₁₁O₁₄ ber. 946.5568, gef. 946.5569 [M+H]⁺.

### Beispiel 192A

### [(3R)-N²-(tert-Butoxycarbonyl)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]}-L-isoleucyl-[O³-(tert-butyl)-L-seryl]-glycyl-[(3S)-3-hydroxy-L-asparaginyl]-L-serin

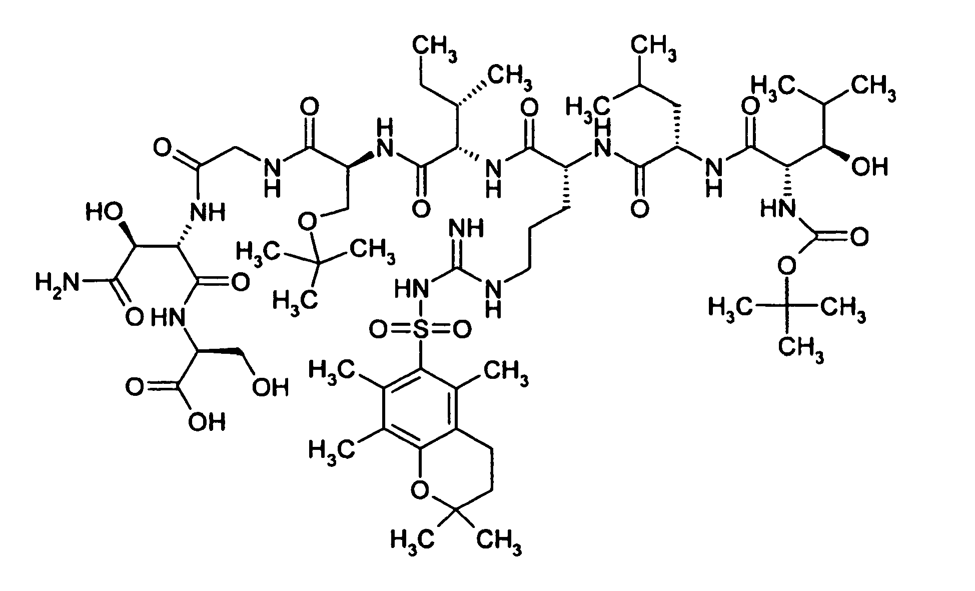

Gemäß der Herstellungsvorschrift der Verbindung aus Beispiel 186A wird die Verbindung aus Beispiel 184A (62 mg, 41 µmol) hydriert. Man erhält 56 mg (96% d. Th.) der Titelverbindung als Feststoff.
HPLC (Methode 5): Rₜ = 4.57 min.
LC-MS (Methode 19): Rₜ = 3.06 min, MS (ESIpos): m/z (%) = 1313.7 (100) [M+H]⁺.
HR-TOF-MS (Methode 24): C₅₉H₁₀₁N₁₂O₁₉S ber. 1313.7022, gef. 1313.7020 [M+H]⁺.

### Beispiel 193A

### [(3R)-N²-(tert-Butoxycarbonyl)-3-hydroxy-L-leucyl]-L-leucyl-D-arginyl-L-isoleucyl-L-allothreonyl-glycyl-L-asparaginyl-L-serin-trifluoracetat

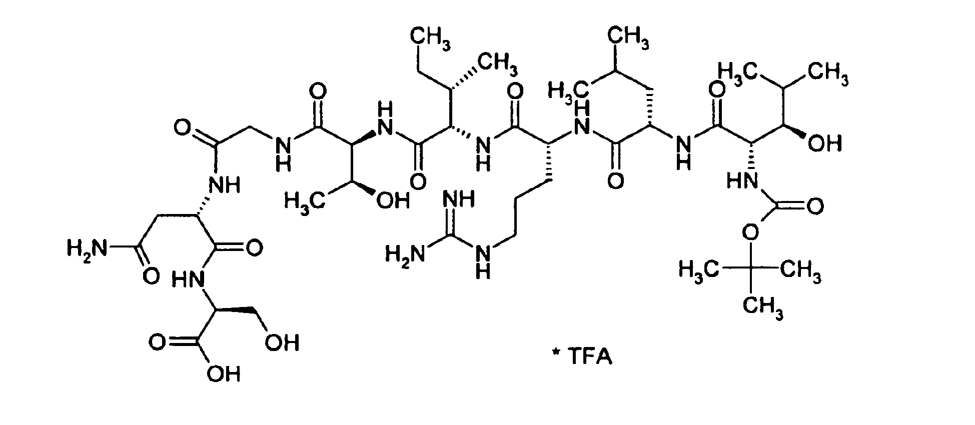

Gemäß der Herstellungsvorschrift der Verbindung aus Beispiel 186A wird die Verbindung aus Beispiel 185A (48 mg, 40 µmol) hydriert. Man erhält 42 mg (99% d. Th.) der Titelverbindung als farblosen Feststoff.
HPLC (Methode 5): Rₜ = 3.40 min.
LC-MS (Methode 19): Rₜ = 1.57 min, MS (ESIpos): m/z (%) = 445.3 (100) [M+2H]²⁺, 9989.6 (70) [M+H]⁺.
HR-TOF-MS (Methode 24): C₄₂H₇₇N₁₂O₁₅ ber. 989.5626, gef. 989.5619 [M+H]⁺.

### Beispiel 194A

### [(3R)-3-Hydroxy-L-leucyl]-L-leucyl-D-arginyl-L-isoleucyl-L-allothreonyl-glycyl-L-seryl-L-serin-bis-trifluoracetat

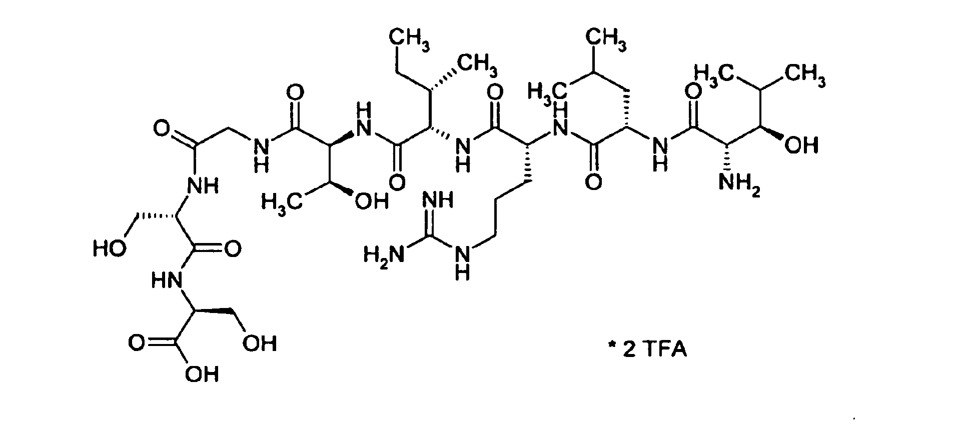

Die Verbindung aus Beispiel 186A (20 mg, 18 µmol) wird nach Arbeitsvorschrift 2 umgesetzt. Man erhält 20 mg (99% d. Th.) der Titelverbindung als Feststoff.
HPLC (Methode 5): Rₜ = 2.96 min.
LC-MS (Methode 22): Rₜ = 2.25 min, MS (ESIpos): m/z (%) = 432 (100) [M+2H]²⁺, 862.6 (1) [M+H]⁺.
HR-TOF-MS (Methode 24): C₃₆H₆₈N₁₁O₁₃ ber. 862.4993, gef. 862.5027 [M+H]⁺.

### Beispiel 195A

### [(3R) 3-Hydroxy-L-leucyl]-L-leucyl-D-arginyl-L-isoleucyl-L-allothreonyl-glycyl-L-phenylalanyl-L-serin-bis-trifluoracetat

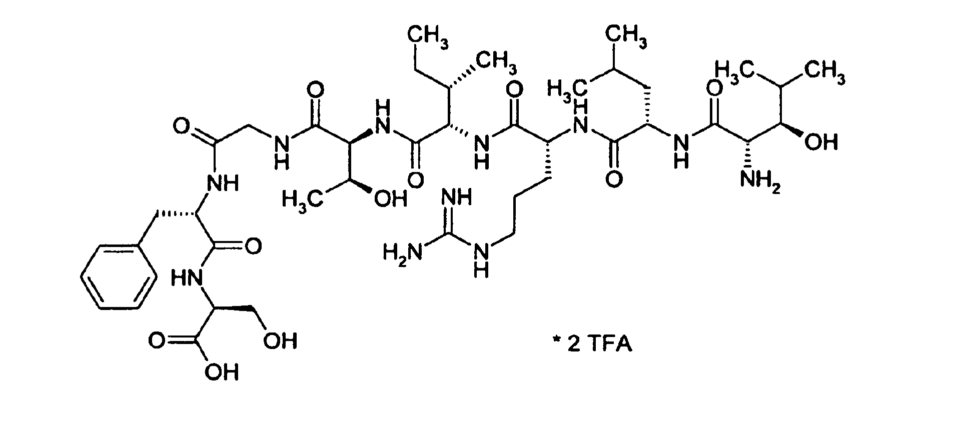

Die Verbindung aus Beispiel 188A (36 mg, 32 µmol) wird nach Arbeitsvorschrift 2 umgesetzt. Man erhält 36 mg (99% d. Th.) der Titelverbindung als Feststoff.
HPLC (Methode 6): Rₜ = 2.98 min.
LC-MS (Methode 19): Rₜ = 1.11 min, MS (ESIpos): m/z (%) = 461.8 (100) [M+2H]²⁺, 922.5 (10) [M+H]⁺; MS (ESIpos): m/z (%) = 920.5 (100) [M-H]⁻.
HR-TOF-MS (Methode 24): C₄₂H₇₂N₁₁O₁₂ ber. 922.5357, gef. 922.5357 [M+H]⁺.

### Beispiel 196A

### [(3-R)-3-Hydroxy-L-leucyl]-L-leucyl-D-arginyl-L-isoleucyl-L-allothreonyl-glycyl-L-threonyl-L-serin-bis-trifluoracetat

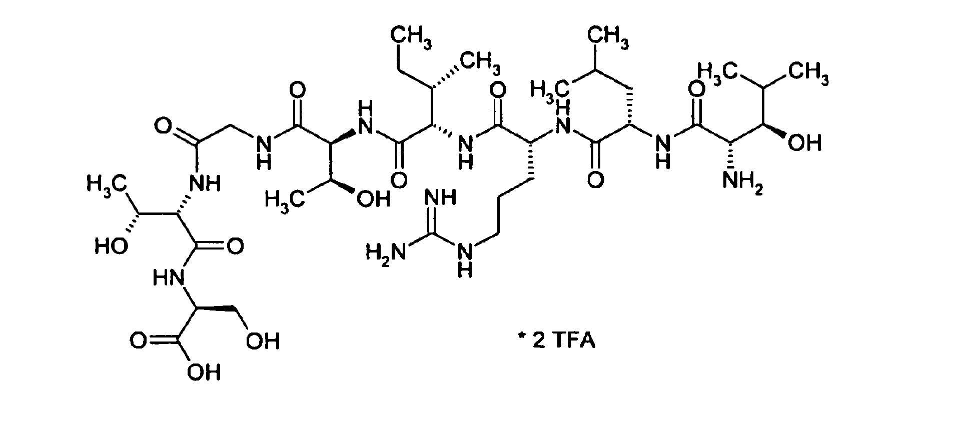

Die Verbindung aus Beispiel 189A (18 mg, 17 µmol) wird nach Arbeitsvorschrift 2 umgesetzt. Man erhält 18 mg (94% d. Th.) der Titelverbindung als Feststoff.
HPLC (Methode 5): Rₜ = 2.99 min.
LC-MS (Methode 22): Rₜ = 2.47 min, MS (ESIpos): m/z (%) = 439 (100) [M+2H]²⁺.
HR-TOF-MS (Methode 24): C₃₇H₇₀N₁₁O₁₃ ber. 876.5150, gef. 876.5161 [M+H]⁺.

### Beispiel 197A

### Benzyl [(3R)-3-hydroxy-L-leucyl]-L-leucyl-D-arginyl-L-isoleucyl-L-allothreonyl-glycyl-L-allothreonyl- L-serinat-bis-trilluoracetat

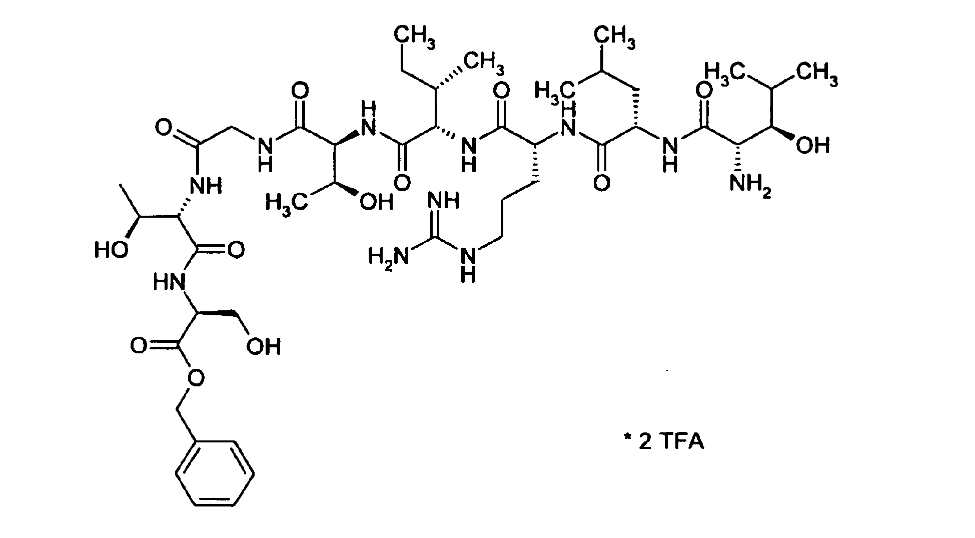

Die Verbindung aus Beispiel 182A (57 mg, 48 µmol) wird nach Arbeitsvorschrift 2 umgesetzt. Nach chromatographischer Reinigung nach der modifizierten Methode 44 (Gradient: 0-5 min 5% B, Rampe, 30-35 min 60% B, 35.01-40 min 60% B) erhält man 45 mg (78% d. Th.) der Titelverbindung.
HPLC (Methode 5): Rₜ = 3.40 min.
LC-MS (Methode 19): Rₜ = 1.12 min, MS (ESIpos): m/z (%) = 483.9 (100) [M+2H]²⁺, 966.6 (10) [M+H]⁺.
HR-TOF-MS (Methode 24): C₄₄H₇₅N₁₁O₁₃ ber. 966.5619, gef. 966.5643 [M+H]⁺.

### Beispiel 198A

### [(3R)-3-Hydroxy-L-leucyl]-L-leucyl-D-arginyl-L-isoleucyl-L-allothreonyl-glycyl-L-alanyl-L-serin-bis-trifluoracetat

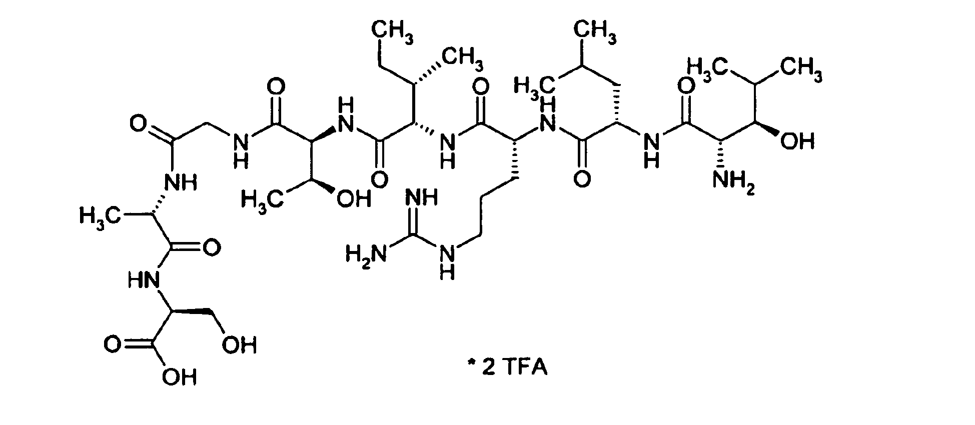

Die Verbindung aus Beispiel 191A (43 mg, 38 µmol) wird nach Arbeitsvorschrift 2 in 2 ml der Reagenzlösung umgesetzt. Man erhält 45 mg der rohen Titelverbindung als Feststoff.
HPLC (Methode 5): Rₜ = 2.99 min.
LC-MS (Methode 22): Rₜ = 2.32 min, MS (ESIpos): m/z (%) = 424 (100) [M+2H]²⁺.
HR-TOF-MS (Methode 24): C₃₆H₆₇N₁₁O₁₂ ber. 846.5044, gef. 846.5029 [M+H]⁺.

### Beispiel 199A

### [(3R)-3-Hydroxy-L-leucyl]-L-leucyl-D-arginyl-L-isoleucyl-L-seryl-glycyl-[(3S)-3-hydroxy-L-asparaginyl]-L-serinat-bis-trifluoracetat

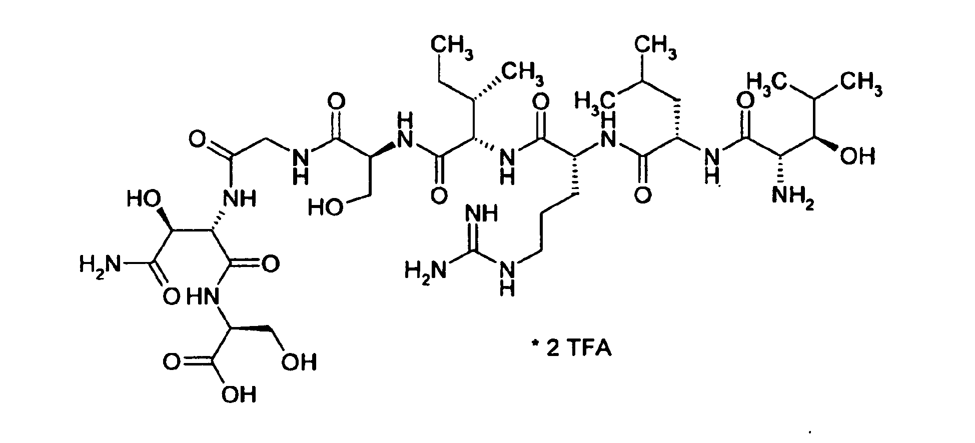

Die Verbindung aus Beispiel 192A (56 mg, 39 µmol), 11 µl Wasser und 11 µl Triisopropylsilan werden in 4.0 ml TFA aufgenommen. Nach 2 h wird der Ansatz eingeengt und chromatographisch gereinigt (Methode 45). Man erhält 40 mg der Titelverbindung (90% d. Th.) als Feststoff.
HPLC (Methode 5): Rₜ = 2.96 min.
LC-MS (Methode 22): Rₜ = 2.30 min, MS (ESIpos): m/z (%) = 446 (100) [M+2H]²⁺.

### Beispiel 200A

### [(3R)-3-Hydroxy-L-leucyl]-L-leucyl-D-arginyl-L-isoleucyl-L-allothreonyl-glycyl-L-asparaginyl-L-serin-bis-trifluoracetat

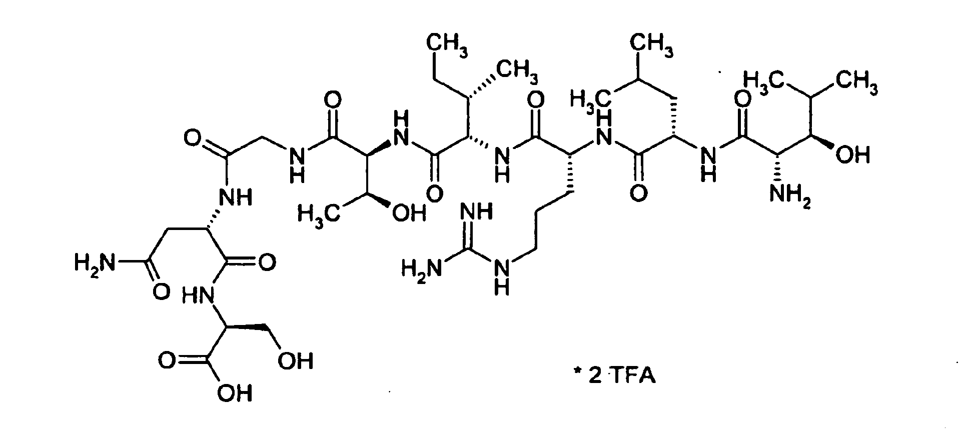

Die Verbindung aus Beispiel 193A (42 mg, 38 µmol) wird nach Arbeitsvorschrift 2 in 2 ml der Reagenzlösung umgesetzt. Man erhält 42 mg (99% d. Th.) der rohen Titelverbindung als Feststoff.
HPLC (Methode 5): Rₜ = 2.77 min.
LC-MS (Methode 22): Rₜ = 2.16 min, MS (ESIpos): m/z (%) = 446 (100) [M+2H]²⁺, 890 (20) [M+H]⁺; MS (ESIneg): m/z (%) = 888 (100) [M-H].
HR-TOF-MS (Methode 24): C₃₇H₆₉N₁₂O₁₃ ber. 889.5102, gef. 889.5095 [M+H]⁺.

### Beispiel 201A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-L-seryl-L-serin-trifluoracetat

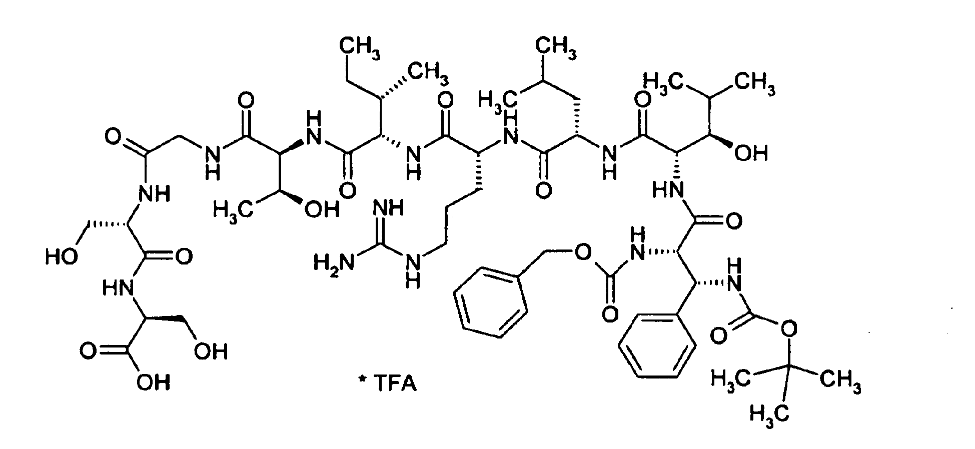

Die Verbindung aus Beispiel 194A (19 mg, 17 µmol) und die Verbindung aus Beispiel 17A (12 mg, 19 µmol, 1.1 Äquivalente) werden bei 0°C in DMF (530 µl) gelöst und mit DIEA (15 µl, 87 µmol, 5 Äq.) versetzt. Die Reaktionsmischung wird über Nacht weitergerührt, wobei sie sich langsam auf RT erwärmt. Der Ansatz wird in Acetonitril aufgenommen und chromatographisch gereinigt (Methode 44). Ausbeute: 14 mg, (10 µmol 59% d. Th.) der Titelverbindung als Feststoff.
HPLC (Methode 5): Rₜ = 3.94 min.
LC-MS (Methode 19): Rₜ = 1.93 min, MS (ESIpos): m/z (%) = 1259.1 (50) [M+H]⁺.
HR-TOF-MS (Methode 24): C₅₈H₉₂N₁₃O₁₈ ber. 1258.6678, gef. 1258.6675 [M+H]⁺.

### Beispiel 202A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin-trifluoracetat

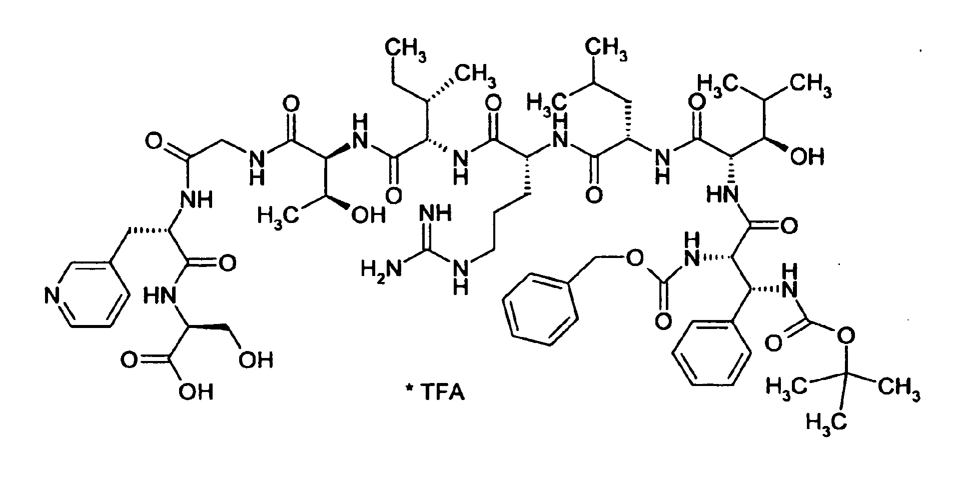

Gemäß der Herstellungsvorschrift der Verbindung aus Beispiel 201A wird die Verbindung aus Beispiel 187A (41 mg, 36 µmol) mit der Verbindung aus Beispiel 17A (23 mg, 39 µmol, 1.1 Äquivalente) umgesetzt. Man erhält 37 mg (73% d. Th.) der Titelverbindung.
HPLC (Methode 5): Rₜ = 3.87 min.
LC-MS (Methode 19): Rₜ = 1.72 min, MS (ESIpos): m/z (%) = 660.5 (100) [M+2H]²⁺, 1319.7 (5) [M+H]⁺; (ESIneg): m/z (%) = 1317.5 (100) [M-H]⁻.
HR-TOF-MS (Methode 24): C₆₃H₉₅N₁₄O₁₇ ber. 1319.6995, gef. 1319.6978 [M+H]⁺.

### Beispiel 203A

### [(3R)-N²-(Benzyloxycarbonyl)-3-{(tert-butoxycarbonyl)amino}-L-phenylalanyl]-[(3R) 3-hydroxy-L-leucyl]-L-leucyl-D-arginyl-L-isoleucyl-L-allothreonyl-glycyl-L-phenylalanyl-L-serin-bis-trifluoracetat

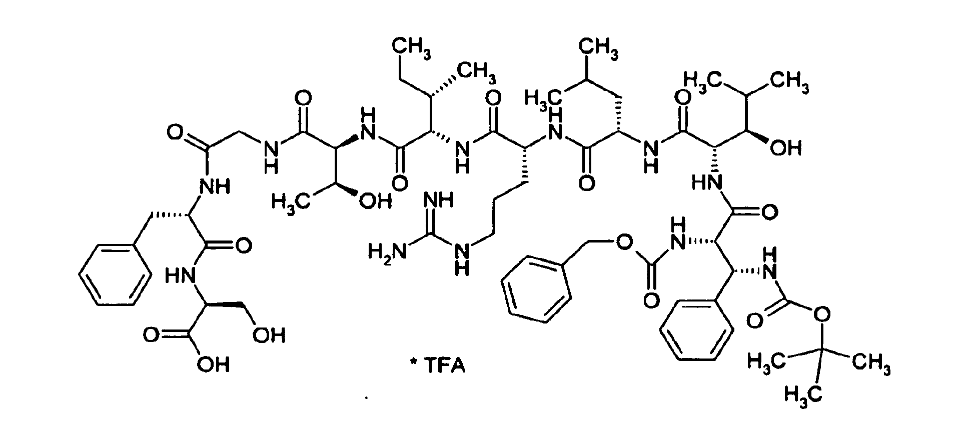

Gemäß der Herstellungsvorschrift der Verbindung aus Beispiel 201A wird die Verbindung aus Beispiel 195A (36 mg, 31 µmol) mit der Verbindung aus Beispiel 17A (22 mg, 39 µmol, 1.2 Äquivalente) umgesetzt. Man erhält 34 mg (70% d. Th.) der Titelverbindung.
HPLC (Methode 6): Rₜ = 3.93 min.
LC-MS (Methode 19): Rₜ = 2.01 min, MS (ESIpos): m/z (%) = 610.1 (100) [M-Boc+2H]²⁺, 1318.8 (20) [M+H]⁺; (ESIneg): m/z (%) = 1316.7 (100) [M-H]⁻.
HR-TOF-MS (Methode 24): C₆₄H₉₆N₁₃O₁₇ ber. 1318.7042, gef. 1318.7036 [M+H]⁺.

### Beispiel 204A

### [(3R)-N²-(Benzyloxycarbonyl)-3-[(tert-butoxycarbonyl)amino}-L-phenylalanyl]-[(3R)-3-hydroxy-L-leucyl]-L-leucyl-D-arginyl-L-isoleucyl-L-allothreonyl-glycyl-L-threonyl-L-serin-trifluoracetat

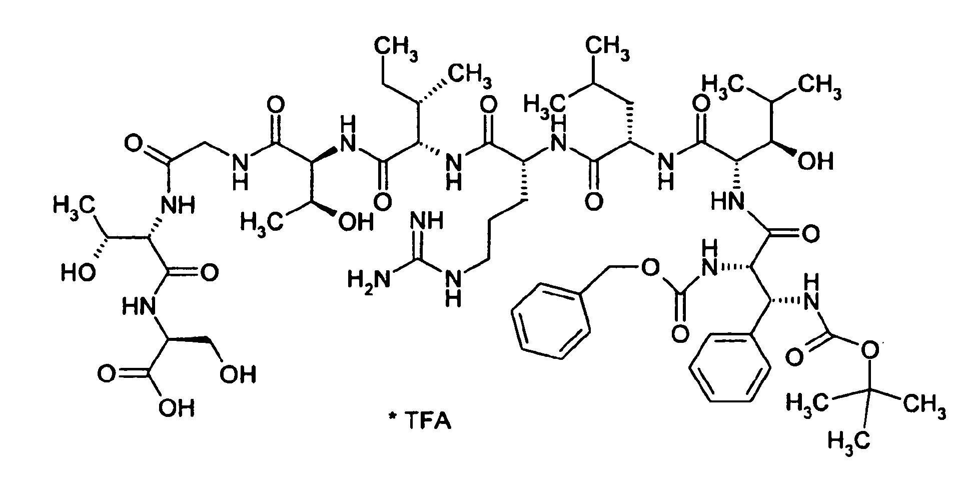

Gemäß der Herstellungsvorschrift der Verbindung aus Beispiel 201A wird die Verbindung aus Beispiel 196A (18 mg, 17 µmol) mit der Verbindung aus Beispiel 17A (12 mg, 21 µmol, 1.1 Äquivalente) umgesetzt. Nach Reinigung entsprechend Methode 45 werden 17 mg (76% d. Th.) der Titelverbindung isoliert.
HPLC (Methode 5): Rₜ = 3.96 min.
LC-MS (Methode 19): Rₜ = 1.97 min, MS (ESIpos): m/z (%) = 1272.8 (40) [M+H]⁺
HR-TOF-MS (Methode 24): C₅₉H₉₄N₁₃O₁₈ ber. 1272.6835, gef. 1272.6853 [M+H]⁺.

### Beispiel 205A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-L-allothreonyl-L-serinat-trifluoracetat

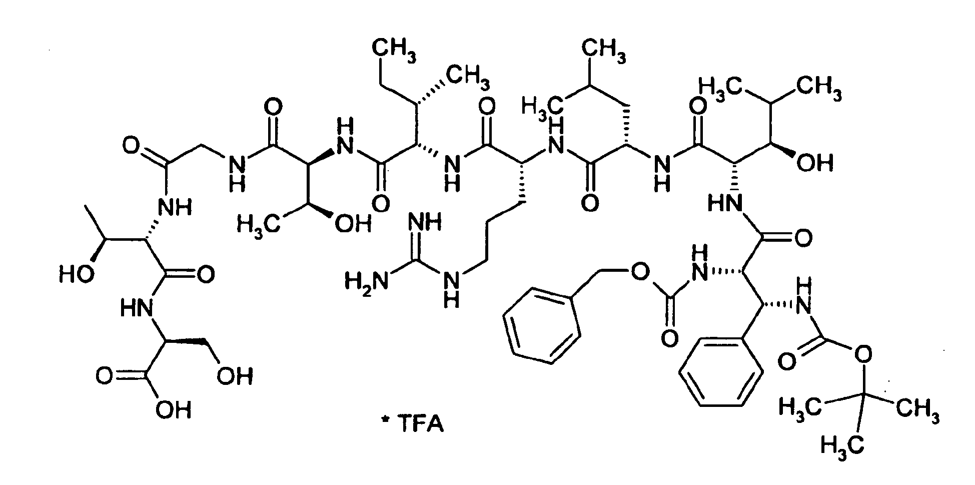

Gemäß der Herstellungsvorschrift der Verbindung aus Beispiel 201A wird die Verbindung aus Beispiel 190A (44 mg, 40 µmol) mit der Verbindung aus Beispiel 17A (25 mg, 44 µmol, 1.1 Äquivalente) umgesetzt. Nach Reinigung entsprechend Methode 45 werden 50 mg (90% d. Th.) der Titelverbindung isoliert.
HPLC (Methode 5): Rₜ = 3.80 min.
LC-MS (Methode 19): R, = 1.98 min, MS (ESIpos): m/z (%) = 1272.7 (20) [M+H]⁺
HR-TOF-MS (Methode 24): C₅₉H₉₄N₁₃O₁₈ ber. 1272.6835, gef. 1272.6846 [M+H]⁺.

### Beispiel 206A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-L-alanyl-L-serin-trifluoracetat

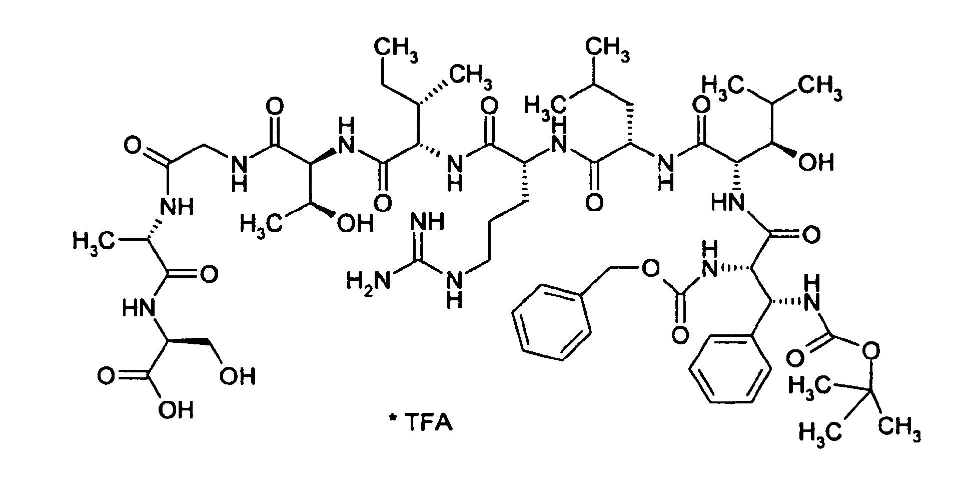

Gemäß der Herstellungsvorschrift der Verbindung aus Beispiel 201A wird die Verbindung aus Beispiel 198A (45 mg, 42 µmol) mit der Verbindung aus Beispiel 17A (32 mg, 54 µmol, 1.3 Äquivalente) umgesetzt. Nach Reinigung entsprechend Methode 45 werden 38 mg (67% d. Th.) der Titelverbindung isoliert.
HPLC (Methode 5): Rₜ = 3.99 min.
LC-MS (Methode 19): Rₜ = 2.12 min, MS (ESIpos): m/z (%) = 571.9 (90) [M-Boc +2H]²⁺; 1242.7 (100) [M+H]⁺.
HR-TOF-MS (Methode 24): C₅₈H₉₂N₁₃O₁₇ ber. 1242.6729, gef. 1242.6691 [M+H]⁺.

### Beispiel 207A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-seryl-glycyl-[(3S)-3-hydroxy-L-asparaginyl]-L-serinat-trifluoracetat

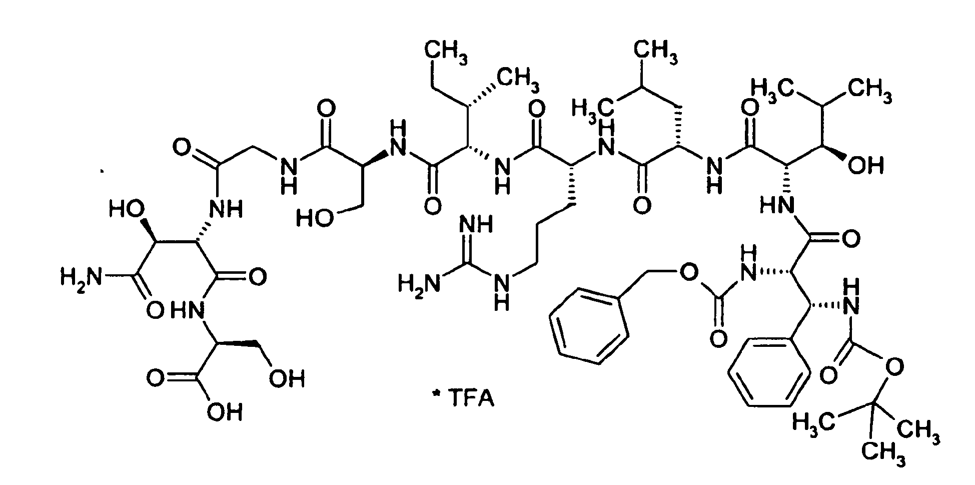

Gemäß der Herstellungsvorschrift der Verbindung aus Beispiel 201A wird die Verbindung aus Beispiel 199A (39 mg, 35 µmol) mit der Verbindung aus Beispiel 17A (24 mg, 42 µmol, 1.2 Äquivalente) umgesetzt. Nach Reinigung entsprechend Methode 45 werden 42 mg (80% d. Th.) der Titelverbindung isoliert.
HPLC (Methode 5): Rₜ = 3.93 min.
LC-MS (Methode 19): Rₜ = 1.93 min, MS (ESIpos): m/z (%) = 594.4 (100) [M-Boc +2H]²⁺; 1287.7 (30) [M+H]⁺.

### Beispiel 208A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-L-asparaginyl-L-serin-trifluoracetat

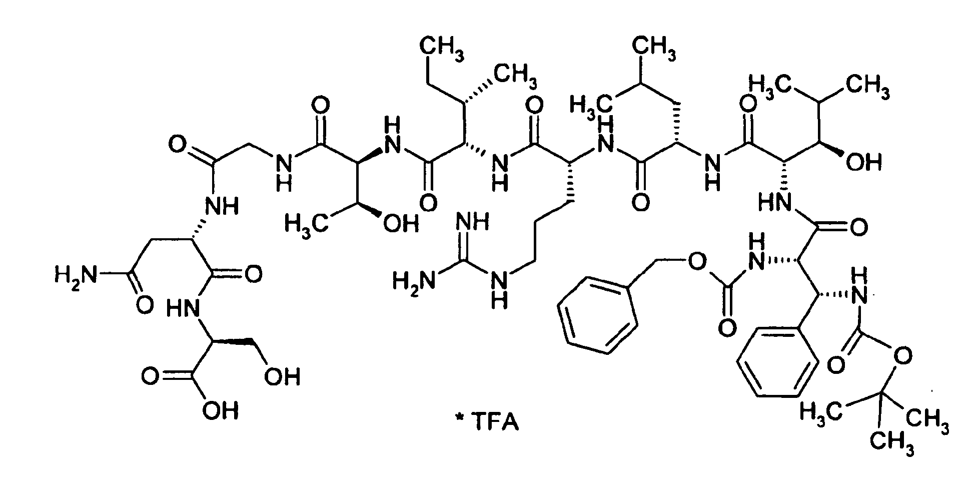

Gemäß der Herstellungsvorschrift der Verbindung aus Beispiel 201A wird die Verbindung aus Beispiel 200A (42 mg, 38 µmol) mit der Verbindung aus Beispiel 17A (26 mg, 45 µmol, 1.2 Äquivalente) umgesetzt. Nach Reinigung entsprechend Methode 45 werden 46 mg (81% d. Th.) der Titelverbindung isoliert.
HPLC (Methode 5): Rₜ = 3.78 min.
LC-MS (Methode 19): Rₜ = 1.91 min, MS (ESIpos): m/z (%) = 1285.7 (40) [M+H]⁺.
HR-TOF-MS (Methode 24): C₅₉H₉₃N₁₄O₁₈ ber.1285.6787, gef. 1285.6777 [M+H]⁺.

### Beispiel 209A

### [(3R)-N²-(Benzyloxycarbonyl)-3-amino}-L-phenylalanyl]-[(3R)-3-hydroxy-L-leucyl]-L-leucyl-D-arginyl-L-isoleucyl-L-allothreonyl-glycyl-L-seryl-L-serin bis-trifluoracetat

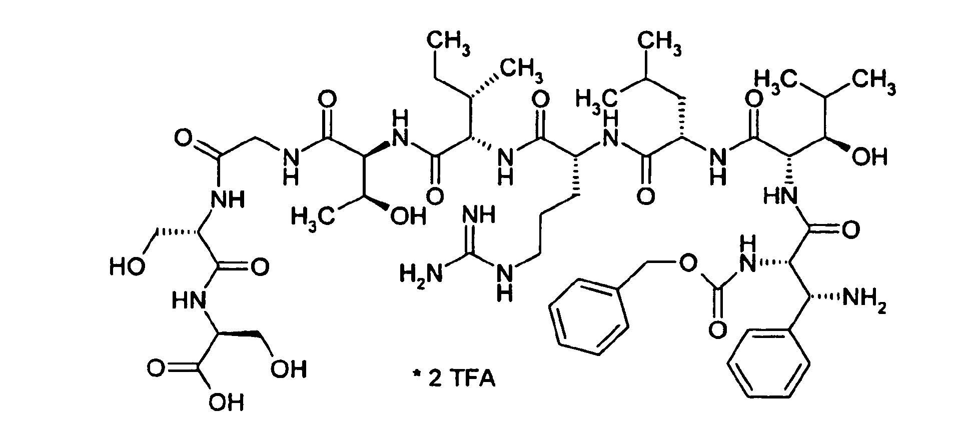

Die Verbindung aus Beispiel 201A (14 mg, 10 µmol) wird nach Arbeitsvorschrift 2 mit 0.5 ml der Reagenzlösung umgesetzt. Ohne weitere Aufreinigung erhält man 13 mg (9.3 µmol, 95% d. Th.) der Titelverbindung als Feststoff.
HPLC (Methode 5): Rₜ = 3.44 min.
LC-MS (Methode 20): Rₜ = 1.19 min, MS (ESIpos): m/z (%) = 580 (100) [M+2H]²⁺, 1158.6 (5) [M+H]⁺.
HR-TOF-MS (Methode 24): C₅₃H₈₄N₁₃O₁₆ ber. 1158.6154, gef. 1158.6183 [M+H]⁺.

### Beispiel 210A

### [(3R)-N²-(Benzyloxycarbonyl)-3-amino}-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin bistrifluoracetat

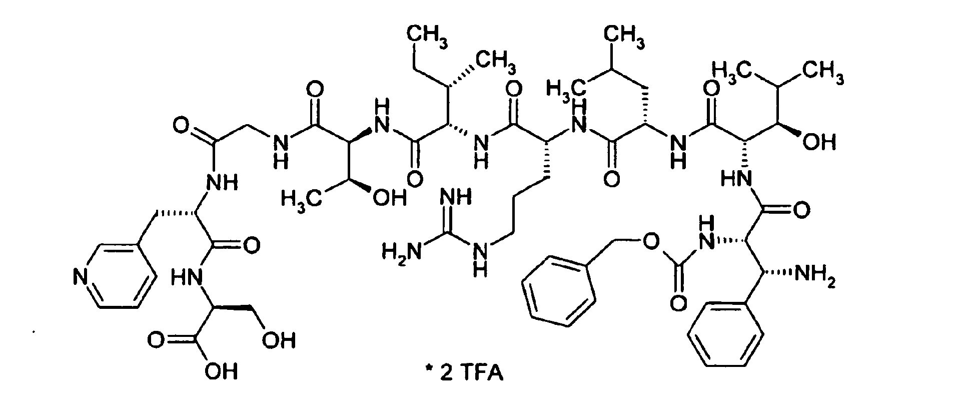

Die Verbindung aus Beispiel 202A (37 mg, 26 µmol) wird nach Arbeitsvorschrift 2 mit 0.5 ml der Reagenzlösung umgesetzt. Ohne weitere Aufreinigung erhält man 46 mg der rohen Titelverbindung.
HPLC (Methode 5): Rₜ = 3.39 min.
LC-MS (Methode 22): Rₜ = 2.67 min, MS (ESIpos): m/z (%) = 610 (20) [M+2H]⁺; (ESIneg m/z (%) = 1218 (100) [M-H]⁻.
HR-TOF-MS (Methode 24): C₅₈H₈₇N₁₄O₁₅ ber. 1219.6470, gef. 1219.6472 [M+H]⁺.

### Beispiel 211A

### [(3R)-N²-(Benzyloxycarbonyl)-3-amino}-L-phenylalanyl]-[(3R) 3-hydroxy-L-leucyl]-L-leucyl-D-arginyl-L-isoleucyl-L-allothreonyl-glycyl-L-phenylalanyl-L-serin-bis-trifluoracetat

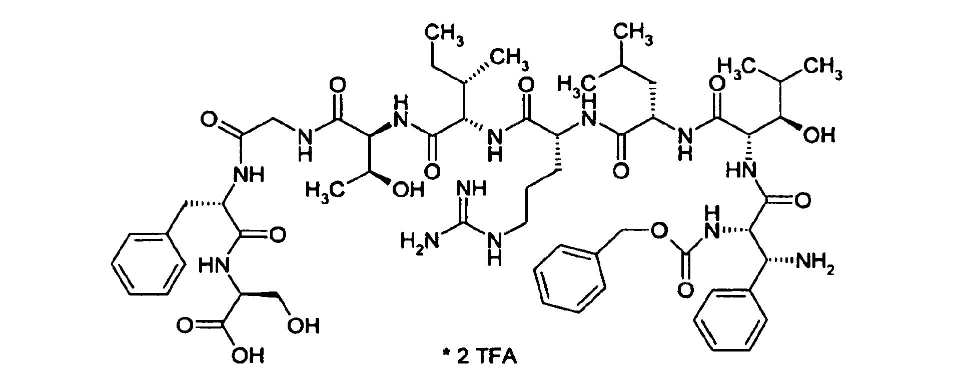

Die Verbindung aus Beispiel 203A (34 mg, 22 µmol) wird nach Arbeitsvorschrift 2 mit 2.0 ml der Reagenzlösung umgesetzt. Ohne weitere Aufreinigung erhält man 34 mg (quant.) der rohen Titelverbindung.
HPLC (Methode 5): Rₜ = 3.34 min.
LC-MS (Methode 20): Rₜ = 1.37 min, MS (ESIpos): m/z (%) = 610.1 (100) [M+2H]²⁺, 1218.8 (5) [M+H]⁺; (ESIneg m/z (%) = 1216.8 (100) [M-H]⁻.
HR-TOF-MS (Methode 24): C₅₉N₈₈N₁₃O₁₅ ber. 1218.6518, gef. 1218.6539 [M+H]⁺.

### Beispiel 212A

### [(3R)-N²-(Benzyloxycarbonyl)-3-amino}-L-phenylalanyl]-[(3R)-3-hydroxy-L-leucyl]-L-leucyl-D-arginyl-L-isoleucyl-L-allothreonyl-glycyl-L-threonyl-L-serin-bis-trifluoracetat

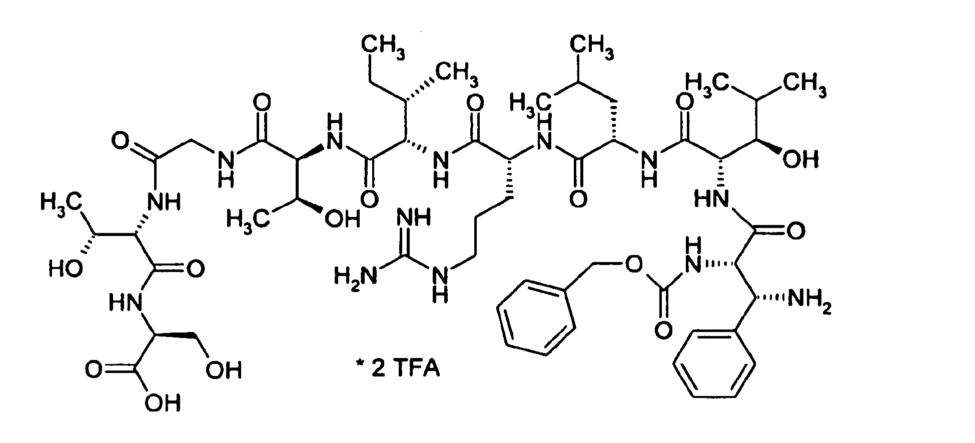

Die Verbindung aus Beispiel 204A (17 mg, 12 µmol) wird nach Arbeitsvorschrift 2 mit 1.0 ml der Reagenzlösung umgesetzt. Ohne weitere Aufreinigung erhält man 17 mg (quant.) der rohen Titelverbindung.
HPLC (Methode 5): Rₜ = 3.48 min.
LC-MS (Methode 21): Rₜ = 1.49 min, MS (ESIpos): m/z (%) = 587.2 (100) [M+2H]²⁺, 1172.7 (5) [M+H]⁺.
HR-TOF-MS (Methode 24): C₅₄H₈₆N₁₃O₁₆ ber. 1172.6310, gef. 1172.6328 [M+H]⁺.

### Beispiel 213A

### [(3R)-N²-(Benzyloxycarbonyl)-3-aminol-L-phenylalanyl]-[(3R)-3-hydroxy-L-leucyl]-L-leucyl-D-arginyl-L-isoleucyl-L-allothreonyl-glycyl-L-allothreonyl-L-serinat-bistrifluoracetat

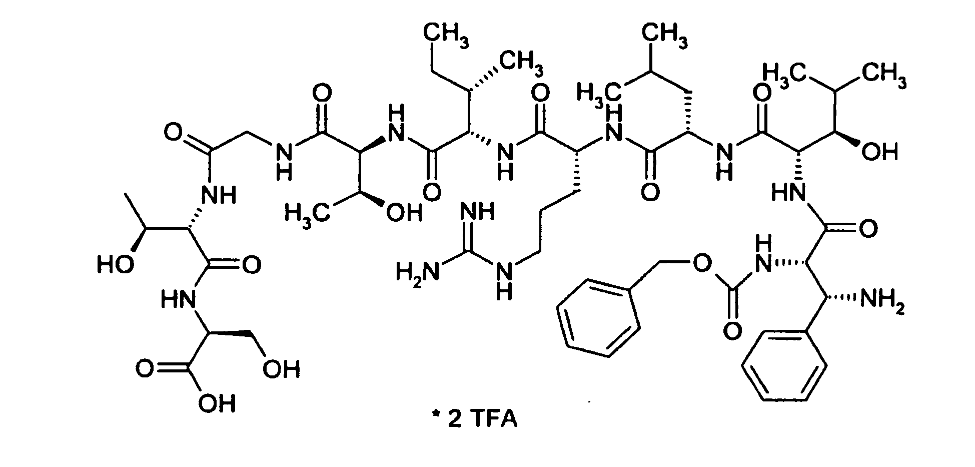

Die Verbindung aus Beispiel 205A (50 mg, 36 µmol) wird nach Arbeitsvorschrift 2 mit 4.8 ml der Reagenzlösung umgesetzt. Ohne weitere Aufreinigung erhält man 56 mg (quant.) der rohen Titelverbindung, die ohne Aufreinigung weiter umgesetzt wird.
HPLC (Methode 6): Rₜ = 3.22 min.
LC-MS (Methode 19): Rₜ = 1.32 min, MS (ESIpos): m/z (%) = 586.9 (100) [M+2H]²⁺.
HR-TOF-MS (Methode 24): C₅₄H₈₆N₁₃O₁₆ ber. 1172.6310, gef. 1172.6323 [M+H]⁺.

### Beispiel 214A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-alanyl-L-serin-bis-trifluoracetat

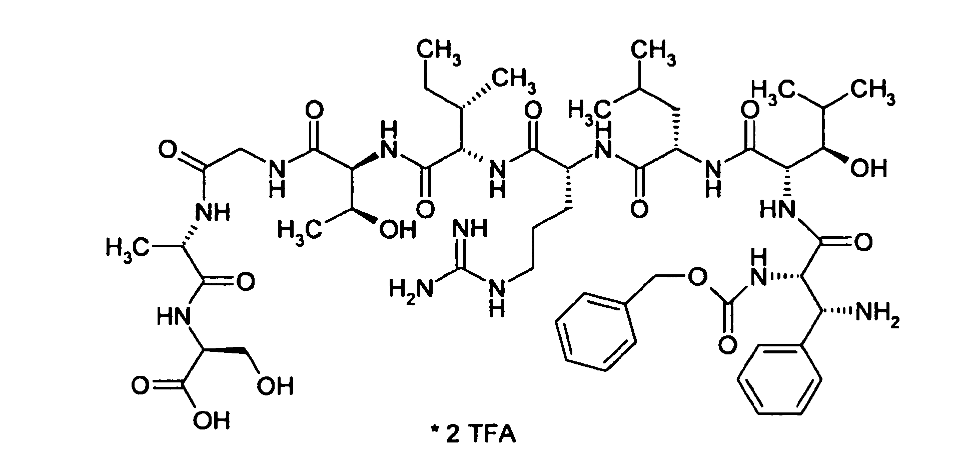

Die Verbindung aus Beispiel 206A (49 mg, 36 µmol) wird nach Arbeitsvorschrift 2 mit 1.0 ml der Reagenzlösung über 1 h umgesetzt. Ohne weitere Aufreinigung erhält man 49 mg (quant.) der rohen Titelverbindung.
HPLC (Methode 5): Rₜ = 3.46 min.
LC-MS (Methode 19): Rₜ = 1.49 min, MS (ESIpos): m/z (%) = 572.0 (100) [M+2H]²⁺, 1142.6 (5) [M+H]⁺.
HR-TOF-MS (Methode 24): C₅₃H₈₄N₁₃O₁₅ ber. 1142.6205, gef. 1142.6221 [M+H]⁺.

### Beispiel 215A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-seryl-glycyl-[(3S)-3-hydroxy-L-asparaginyl]-L-serinat-bistrifluoracetat

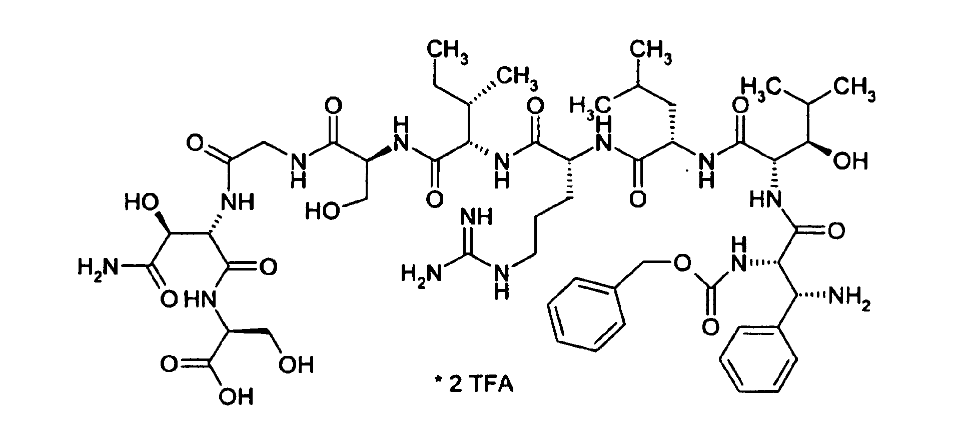

Die Verbindung aus Beispiel 207A (42 mg, 28 µmol) wird nach Arbeitsvorschrift 2 mit 1.0 ml der Reagenzlösung über 1 h umgesetzt. Ohne weitere Aufreinigung erhält man 43 mg (quant.) der rohen Titelverbindung.
HPLC (Methode 5): Rₜ = 3.43 min.
LC-MS (Methode 19): Rₜ = 1.34 min, MS (ESIpos): m/z (%) = 594.5 (100) [M+2H]²⁺, 1187.6 (5) [M+H]⁺.
HR-TOF-MS (Methode 24): C₅₃H₈₃N₁₄O₁₇ ber. 1187.6056, gef. 1187.6074 [M+H]⁺.

### Beispiel 216A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-bistrifluoracetat

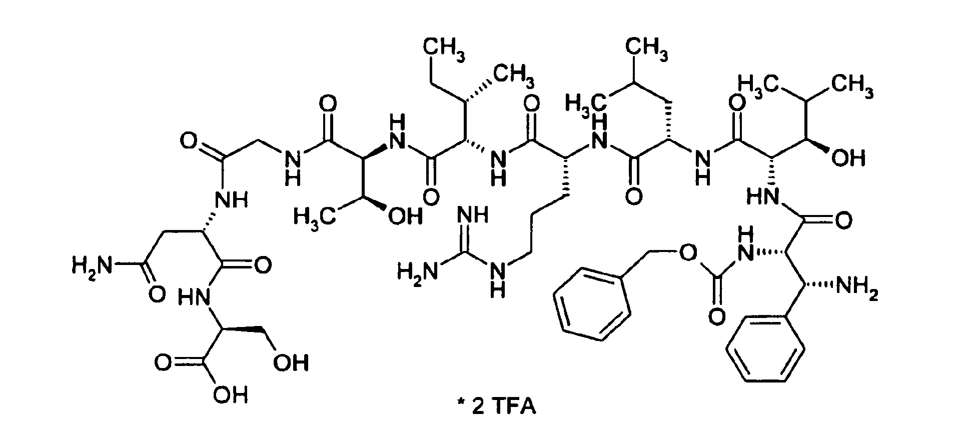

Die Verbindung aus Beispiel 208A (46 mg, 30 µmol) wird nach Arbeitsvorschrift 2 mit 1.0 ml der Reagenzlösung über 30 min umgesetzt. Ohne weitere Aufreinigung erhält man 46 mg (quant.) der rohen Titelverbindung.
HPLC (Methode 5): Rₜ = 3.21 min.
LC-MS (Methode 19): Rₜ = 1.31 min, MS (ESIpos): m/z (%) = 593.5 (100) [M+2H]²⁺, 1185.5 (5) [M+H]⁺.
HR-TOF-MS (Methode 24): C₅₄H₈₅N₁₄O₁₆ ber. 1185.6263, gef. 1185.6241 [M+H]⁺.

### Beispiel 217A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-seryl-L-serin-C^{1.9} -N^{3.1}-lactam-trifluoracetat

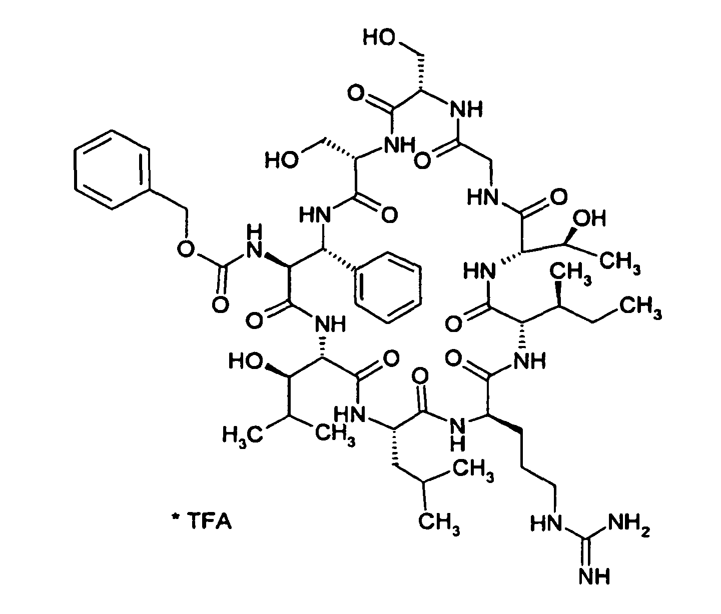

Die Verbindung aus Beispiel 209A (13 mg, 9.3 µmol) wird in DMF (480 µL) gelöst und die Lösung auf 0 °C gekühlt. 4-Methylmorpholin (6 µL, 56 µmol, 6 Äquivalente) und HATU (11 mg, 28 µmol, 3 Äquivalente) werden zugegeben und der Ansatz 2 h bei 0°C gerührt. Dann wird der gesamte Ansatz auf eine HPLC-Säule gegeben und nach Methode 44 chromatographisch gereinigt. Produkthaltige Fraktionen werden vereinigt und lyophilisiert. Man erhält 5 mg (4.0 µmol, 43% d. Th.) der Titelverbindung als Feststoff.
HPLC (Methode 5): Rₜ = 3.70 min.
LC-MS (Methode 20): Rₜ = 1.49 min, MS (ESIpos): m/z (%) = 570.9 (80) [M+2H]²⁺, 1140.6 (90) [M+H]⁺.
HR-TOF-MS (Methode 24): C₅₃H₈₂N₁₃O₁₅ ber. 1140.6048, gef. 1140.6066 [M+H]⁺.
**Beispiel 218A**

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin-C^{1.9}-N^{3.1}-lactam-trifluoracetat

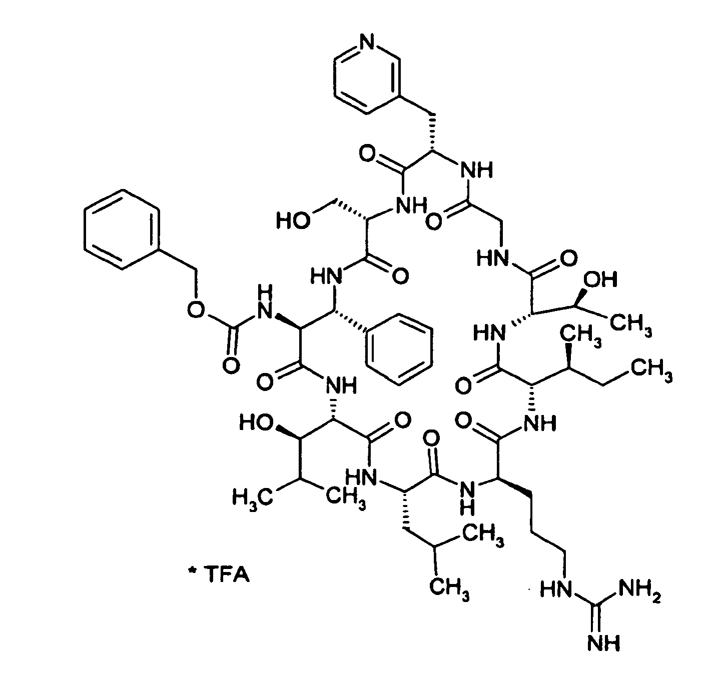

Gemäß der Herstellungsvorschrift der Verbindung aus Beispiel 217A wird aus der Verbindung aus Beispiel 210A (37 mg, 26 µmol) die Titelverbindung in einer Ausbeute von 17 mg (51% d. Th.) erhalten.
HPLC (Methode 5): Rₜ = 3.60 min.
LC-MS (Methode 19): Rₜ = 1.45 min, MS (ESIpos): m/z (%) = 601.5 (100) [M+2H]²⁺, 1201.6 (20) [M+H]⁺.
HR-TOF-MS (Methode 24): C₅₈H₈₅N₁₄O₁₄ ber. 1204.6365, gef. 1201.6338 [M+H]⁺.

### Beispiel 219A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-phenylalanyl]-L-serin- C^{1.9}-N^{3.1}-lactam-trifluoracetat

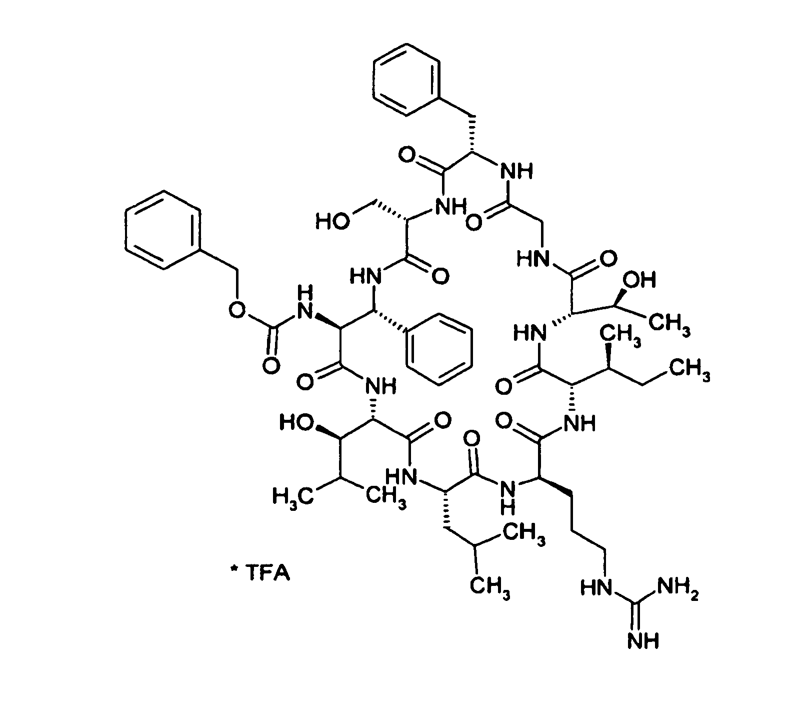

Gemäß der Herstellungsvorschrift der Verbindung aus Beispiel 217A wird aus der Verbindung aus Beispiel 211A (34 mg, 22 µmol) die Titelverbindung in einer Ausbeute von 28 mg (96% d. Th.) erhalten.
HPLC (Methode 6): Rₜ = 3.78 min.
LC-MS (Methode 20): Rₜ = 1.75 min, MS (ESIpos): m/z (%) = 601.0 (50) [M+2H]²⁺, 1200.8 (100) [M+H]⁺; MS (ESIneg): m/z (%) = 1198.8 (100) [M-H]⁻.

### Beispiel 220A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-threonyl-L-serin-C^{1.9}-N^{3.1}-lactam-trifluoracetat

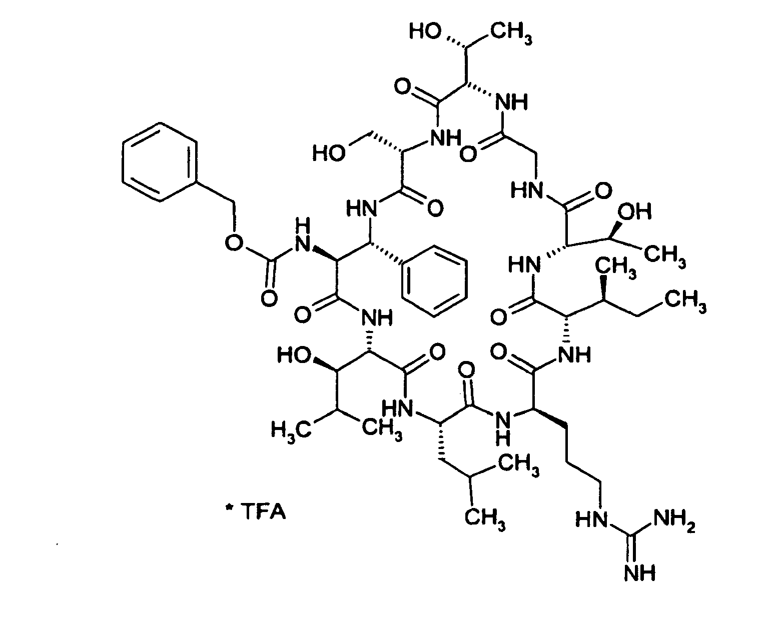

Gemäß der Herstellungsvorschrift der Verbindung aus Beispiel 217A, wobei die Aufreinigung nach Methode 45 erfolgt, wird aus der Verbindung aus Beispiel 212A (17 mg, 12 µmol) die Titelverbindung in einer Ausbeute von 10 mg (65% d. Th.) erhalten.
HPLC (Methode 5): Rₜ = 3.75 min.
LC-MS (Methode 19): Rₜ = 1.59 min, MS (ESIpos): m/z (%) = 1154.7 (100) [M+H]⁺.
HR-TOF-MS (Methode 24): C₅₄H₈₄N₁₃O₁₅ ber. 1154.6205, gef. 1154.6194 [M+H]⁺.

### Beispiel 221A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-allothreonyl-L-serinat-C^{1.9}-N^{3.1}-lactam trifluoracetat

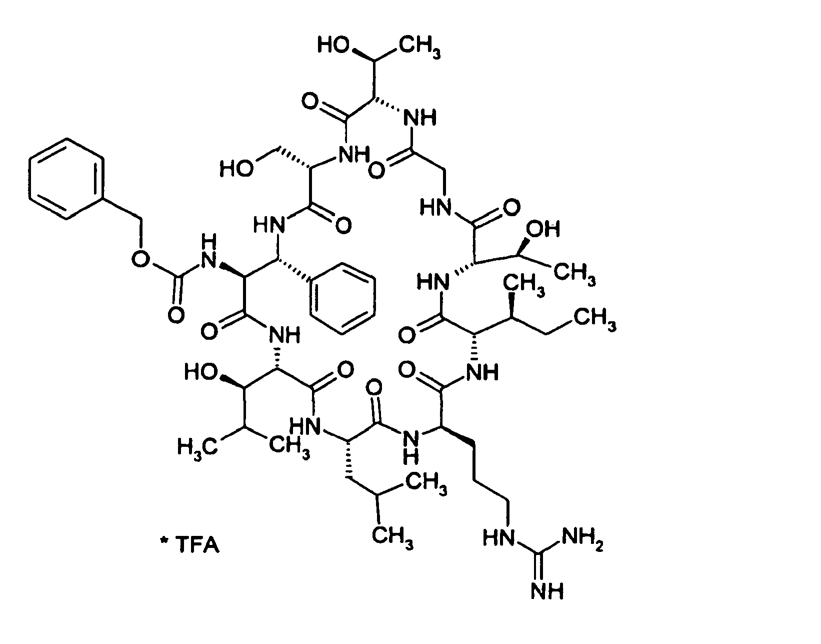

Gemäß der Herstellungsvorschrift der Verbindung aus Beispiel 217A wird die Verbindung aus Beispiel 213A (50 mg, 36 µmol) cyclisiert. Dann wird nach Methode 45 chromatographiert. Die Titelverbindung wird in einer Rohausbeute von 52 mg (75% rein, 86% d. Th.) erhalten und nicht weiter aufgereinigt.
HPLC (Methode 6): Rₜ = 3.55 min.
LC-MS (Methode 19): Rₜ = 1.60 min, MS (ESIpos): m/z (%) = 1154.7 (100) [M+H]⁺.
HR-TOF-MS (Methode 24): C₅₄H₈₄N₁₃O₁₅ ber. 1154.6205, gef. 1154.6171 [M+H]⁺.

### Beispiel 222A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-alanyl-L-serin-C^{1.9}-N^{3.1}-lactam-trifluoracetat

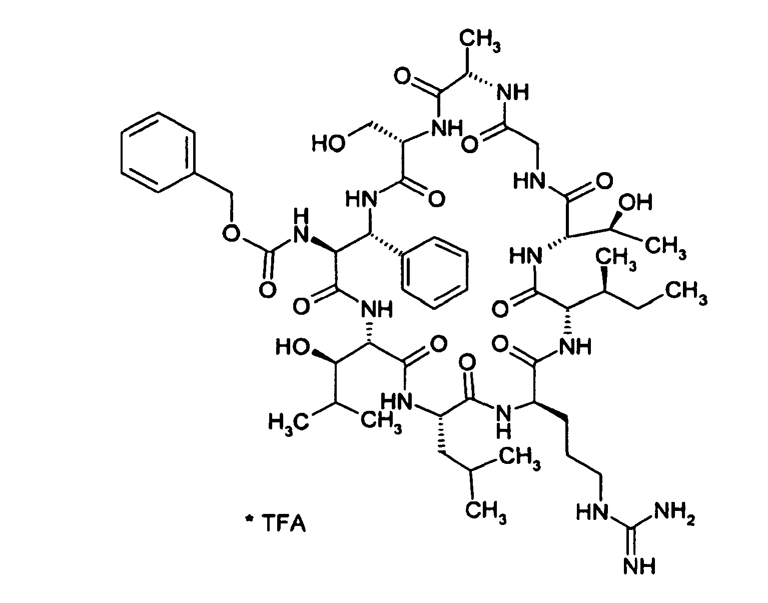

Gemäß der Herstellungsvorschrift der Verbindung aus Beispiel 217A, wobei die Aufreinigung nach Methode 45 erfolgt, wird aus der Verbindung aus Beispiel 214A (49 mg, 36 µmol) die Titelverbindung in einer Ausbeute von 34 mg (77% d. Th.) erhalten.
HPLC (Methode 5): Rₜ = 3.78 min.
LC-MS (Methode 19): Rₜ = 1.61 min, MS (ESIpos): m/z (%) = 1124.6 (100) [M+H]⁺.
HR-TOF-MS (Methode 24): C₅₃H₈₂N₁₃O₁₄ ber. 1154.6099, gef. 1124.6073 [M+H]⁺.

### Beispiel 223A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-trifluoracetat

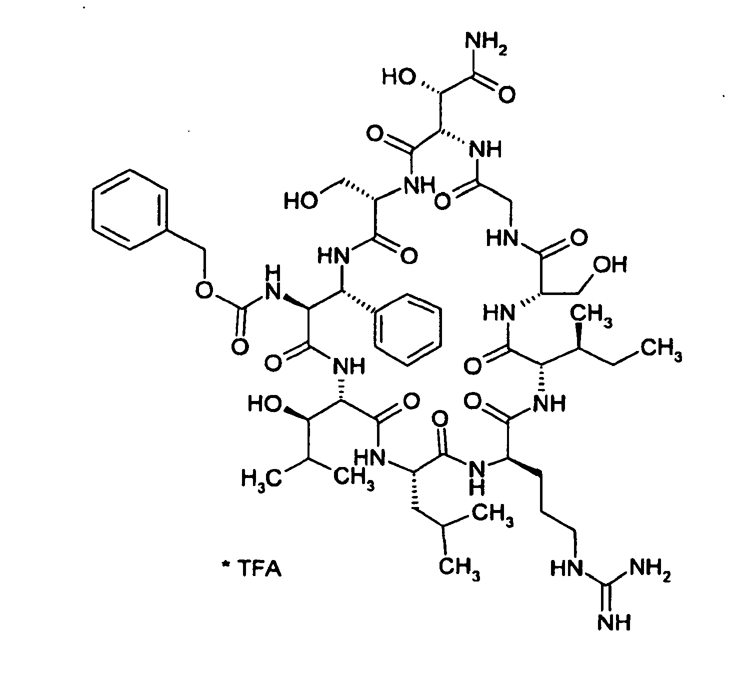

Gemäß der Herstellungsvorschrift der Verbindung aus Beispiel 217A, wobei die Aufreinigung nach Methode 45 erfolgt, wird aus der Verbindung aus Beispiel 215A (42 mg, 27 µmol) die Titelverbindung in einer Ausbeute von 28 mg (73% d. Th.) erhalten.
HPLC (Methode 5): Rₜ = 3.68 min.
LC-MS (Methode 19): Rₜ = 1.57 min, MS (ESIpos): m/z (%) = 1169.6 (100) [M+H]⁺.
HR-TOF-MS (Methode 24): C₅₃H₈₁N₁₄O₁₆ ber. 1169.5950, gef. 1169.5939 [M+H]⁺.

### Beispiel 224A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-C^{1.9}-N^{3.1}-lactam-trifluoracetat

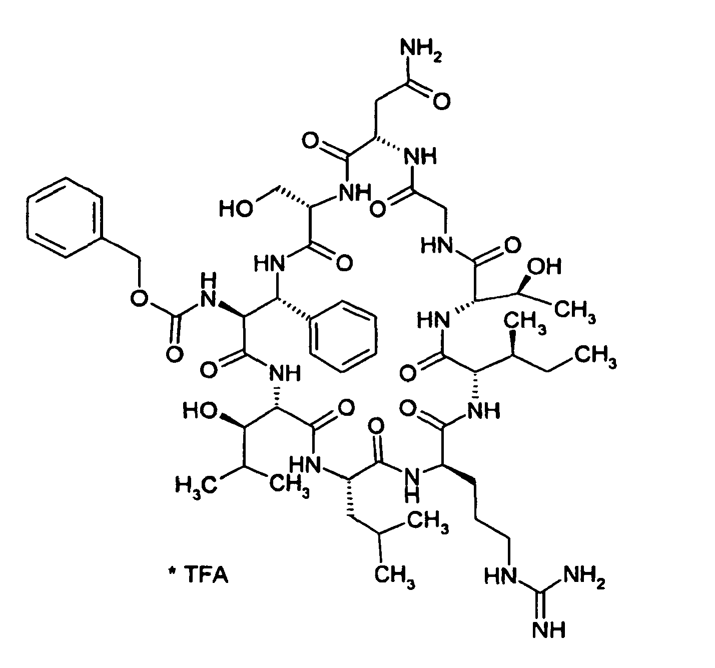

Gemäß der Herstellungsvorschrift der Verbindung aus Beispiel 217A, wobei die Aufreinigung nach Methode 45 erfolgt, wird aus der Verbindung aus Beispiel 216A (45 mg, 30 µmol) die Titelverbindung in einer Ausbeute von 29 mg (75% d. Th.) erhalten.
HPLC (Methode 5): Rₜ = 3.52 min.
LC-MS (Methode 19): Rₜ = 1.56 min, MS (ESIpos): m/z (%) = 1167.5 (100) [M+H]⁺.
HR-TOF-MS (Methode 24): C₅₄H₈₃N₁₄O₁₅ ber. 1167.6157, gef. 1167.6152 [M+H]⁺.

### Beispiel 225A

### [(3R)-3-Amino-L-phenylalanyl]-[(3R)-3-hydroxy-L-leucyl]-L-leucyl-D-arginyl-L-isoleucyl-L-allothreonyl-glycyl-L-seryl-L-serin-C^{1.9}-N^{3.1}-lactam-bis-hydrochlorid

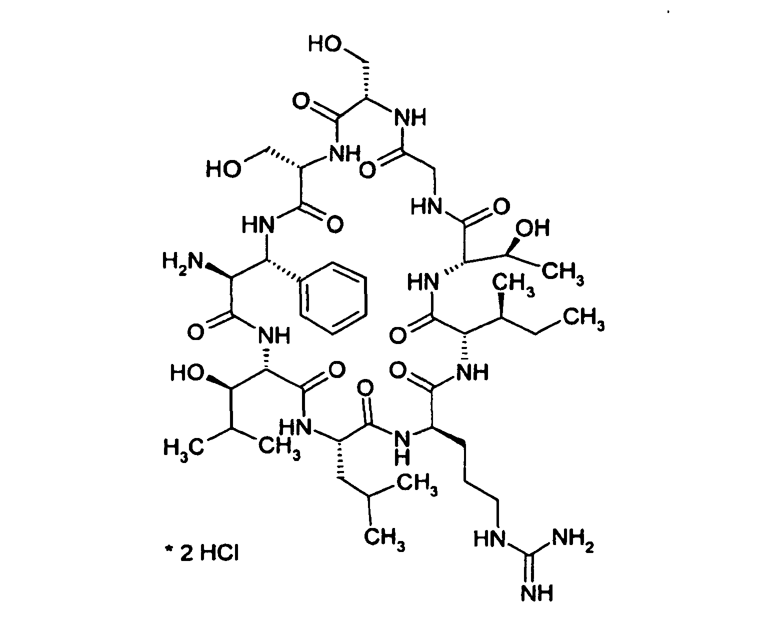

Die Verbindung aus Beispiel 217A wird in Methanol (750 µL) in Gegenwart von 1 M Salzsäure (24 µL, 6 Äquivalente) gelöst und über 1.5 h bei Normaldruck und Raumtemperatur in Gegenwart von 3 mg 10% Palladium-Kohle hydriert. Die Lösung wird vom Katalysator abfiltriert und eingeengt. Die Titelverbindung wird in einer Ausbeute von 4.3 mg (quant.) als Feststoff erhalten.
HPLC (Methode 5): Rₜ = 3.21 min.
LC-MS (Methode 20): Rₜ = 0.90 min, MS (ESIpos): m/z (%) = 503.9 (100) [M+2H]²⁺, 1006.6 (3) [M+H]⁺.
HR-TOF-MS (Methode 24): C₄₅H₇₆N₁₃O₁₃ ber. 1006.5681, gef. 1006.5670 [M+H]⁺.

### Beispiel 226A

### [(3R)-3-amino-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin-C^{1.9}-N^{3.1}-lactam-bis-hydrochlorid

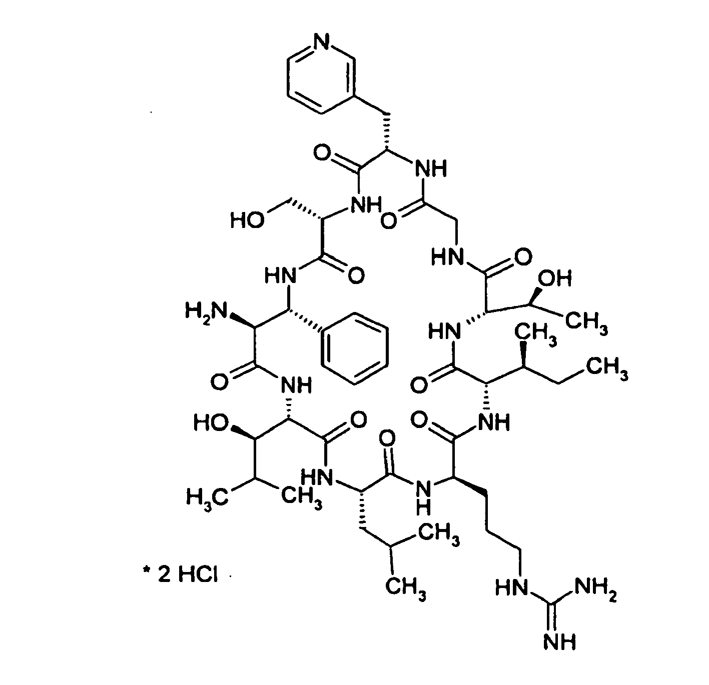

Gemäß der Herstellungsvorschrift der Verbindung aus Beispiel 225A wird aus der Verbindung aus Beispiel 218A (17 mg, 13 µmol) die Titelverbindung in einer Ausbeute von 17 mg (quant.) erhalten.
HPLC (Methode 5): Rₜ = 3.17 min.
LC-MS (Methode 22): Rₜ = 2.34 min, MS (ESIpos): m/z (%) = 534 (20) [M+2H]²⁺; (ESIneg): m/z (%) = 1065 (100) [M-H]⁻.
HR-TOF-MS (Methode 24): C₅₀H₇₉N₁₄O₁₂ ber. 1067.5997, gef. 1067.6006 [M+H]⁺.

### Beispiel 227A

### [(3R)-3-amino-L-phenylalanyl]-[(3R)-3-hydroxy-L-leucyl]-L-leucyl-D-arginyl-L-isoleucyl-L-allothreonyl-glycyl-L-phenylalanyl-L-serin- C^{1.9}-N^{3.1}-lactam-bis-hydrochlorid

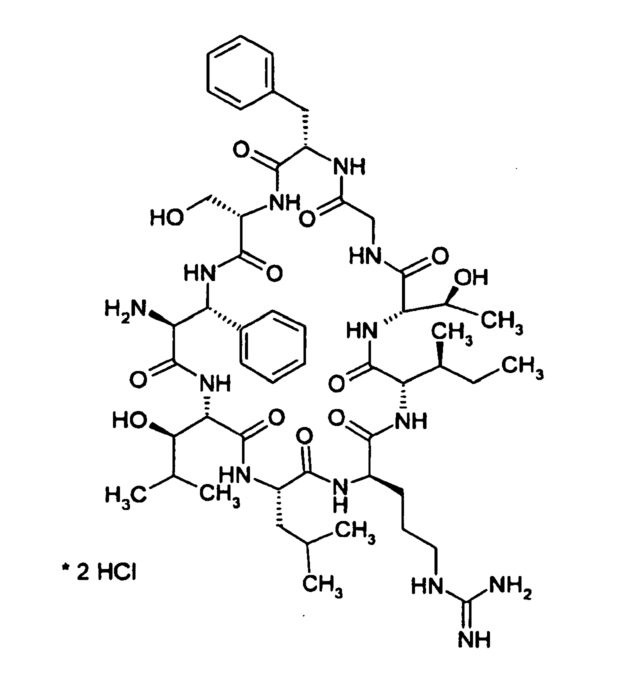

Gemäß der Herstellungsvorschrift der Verbindung aus Beispiel 225A wird aus der Verbindung aus Beispiel 219A (28 mg, 21 µmol) die Titelverbindung in einer Ausbeute von 24 mg (99% d. Th.) erhalten.
HPLC (Methode 5): Rₜ = 3.17 min.
LC-MS (Methode 21): Rₜ = 1.15 min, MS (ESIpos): m/z (%) = 534 (100) [M+2H]²⁺; (ESIneg): m/z (%) = 1064.8 (50) [M-H]⁻.
HR-TOF-MS (Methode 24): C₅₁H₈₀N₁₃O₁₂ ber. 1066.6044, gef. 1066.6013 [M+H]⁺.

### Beispiel 228A

### [(3R)-3-amino-L-phenylalanyl]-[(3R)-3-hydroxy-L-leucyl]-L-leucyl-D-arginyl-L-isoleucyl-L-allothreonyl-glycyl-L-threonyl-L-serin-C^{1.9}-N^{3.1}-lactam-bis-hydrochlorid

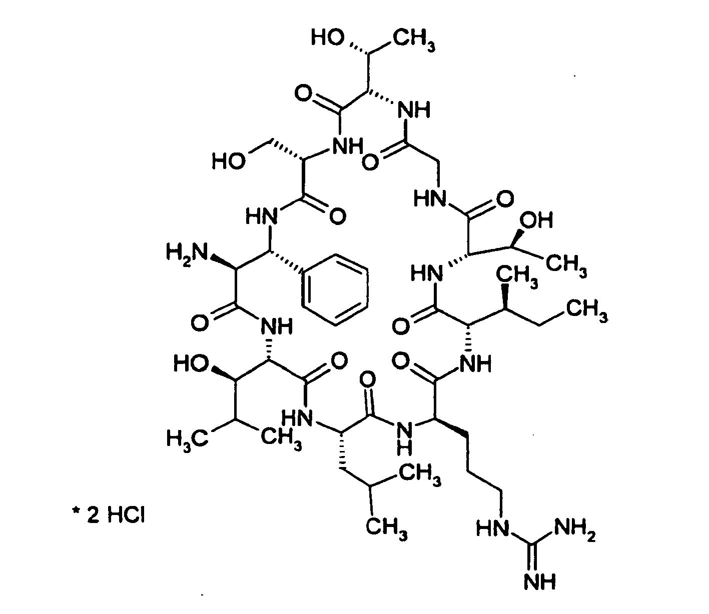

Gemäß der Herstellungsvorschrift der Verbindung aus Beispiel 225A wird aus der Verbindung aus Beispiel 220A (10 mg, 8 µmol) die Titelverbindung in einer Ausbeute von 8.5 mg (99% d. Th.) erhalten.
HPLC (Methode 5): Rₜ = 3.25 min.
LC-MS (Methode 22): Rₜ = 2.49 min, MS (ESIpos): m/z (%) = 511 (100) [M+2H]²⁺.
HR-TOF-MS (Methode 24): C₄₆H₇₈N₁₃O₁₃ ber. 1020.5837, gef. 1020.5831 [M+H]⁺.

### Beispiel 229A

### [(3R)-3-amino-L-phenylalanyl]-[(3R)-3-hydroxy-L-leucyl]-L-leucyl-D-arginyl-L-isoleucyl-L-allothreonyl-glycyl-L-allothreonyl-L-serinat-C^{1.9}-N^{3.1}-lactam-bis-hydrochlorid

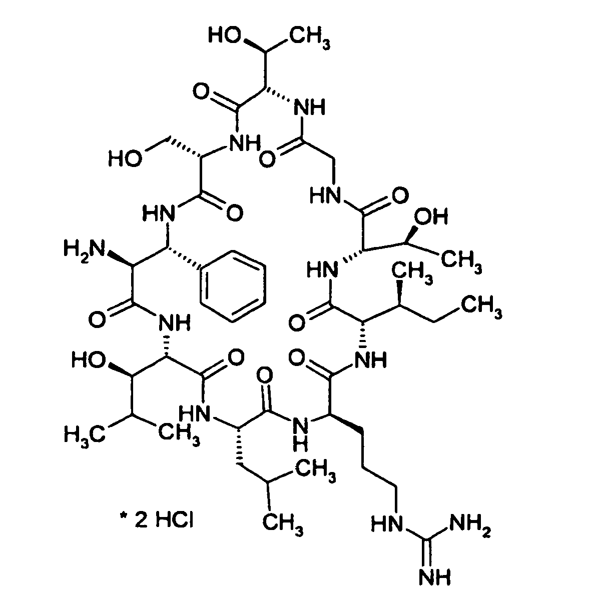

Gemäß der Herstellungsvorschrift der Verbindung aus Beispiel 225A wird aus der Verbindung aus Beispiel 221A (52 mg, 41 µmol) die Titelverbindung in einer Ausbeute von 40 mg (86% d. Th.) erhalten.
HPLC (Methode 6): Rₜ = 2.93 min.

### Beispiel 230A

### [(3R)-3-amino-L-phenylalanyl]-[(3R)-3-hydroxy-L-leucyl]-L-leucyl-D-arginyl-L-isoleucyl-L-allothreonyl-glycyl-L-alanyl-L-serin-C^{1.9}-N^{3.1}-lactam-bis-hydrochlorid

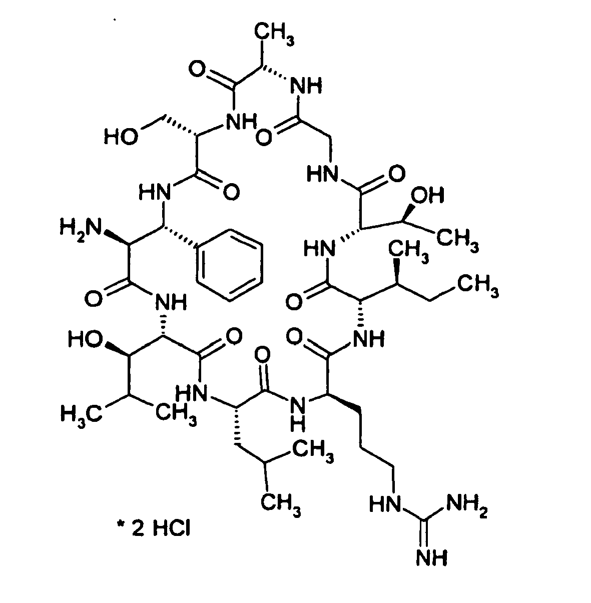

Gemäß der Herstellungsvorschrift der Verbindung aus Beispiel 225A wird aus der Verbindung aus Beispiel 222A (34 mg, 27 µmol) die Titelverbindung in einer Ausbeute von 29 mg (99% d. Th.) erhalten.
HPLC (Methode 5): Rₜ = 3.24 min.
LC-MS (Methode 22): Rₜ = 2.48 min, MS (ESIpos): m/z (%) = 496 (100) [M+2H]²⁺.
HR-TOF-MS (Methode 24): C₄₅H₇₆N₁₃O₁₂ ber. 990.5731, gef. 990.5726 [M+H]⁺.

### Beispiel 231A

### [(3R)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.9}-N^{3.1}-lactam-bis-hydrochlorid

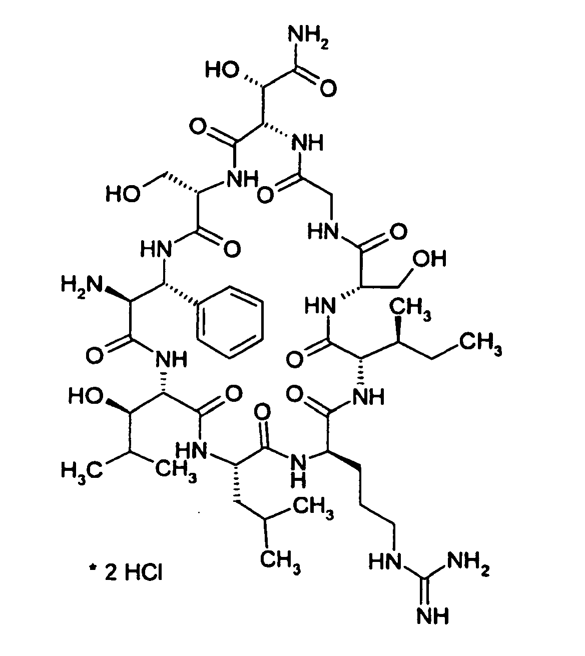

Gemäß der Herstellungsvorschrift der Verbindung aus Beispiel 225A wird aus der Verbindung aus Beispiel 223A (28 mg, 20 µmol) die Titelverbindung in einer Ausbeute von 21 mg (95% d. Th.) erhalten.
HPLC (Methode 5): Rₜ = 3.16 min.
LC-MS (Methode 22): Rₜ = 2.43 min, MS (ESIpos): m/z (%) = 519 (100) [M+2H]²⁺.
HR-TOF-MS (Methode 24): C₄₅H₇₅N₁₄O₁₄ ber. 1035.5582, gef. 1035.5585 [M+H]⁺.

### Beispiel 232A

### [(3R)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-C^{1.9}-N^{3.1}-lactam-bis-hydrochlorid

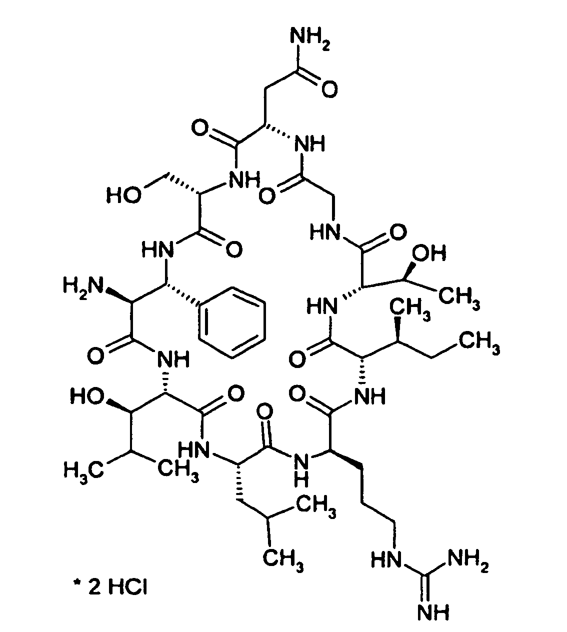

Gemäß der Herstellungsvorschrift der Verbindung aus Beispiel 225A wird aus der Verbindung aus Beispiel 224A (29 mg, 23 µmol) die Titelverbindung in einer Ausbeute von 24 mg (96% d. Th.) erhalten.
HPLC (Methode 5): Rₜ = 2.90 min.
LC-MS (Methode 22): Rₜ = 2.32 min, MS (ESIpos): m/z (%) = 517 (100) [M+2H]²⁺, 1033 (5) [M+H]⁺.
HR-TOF-MS (Methode 24): C₄₆H₇₆N₁₄O₁₃ ber. 1033.5790, gef. 1033.5773 [M+H]⁺.

### Beispiel 233A

### [N²-(Benzyloxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-seryl-L-serin-C^{1.11}-N^{3.3}-lactam-hydrochlorid

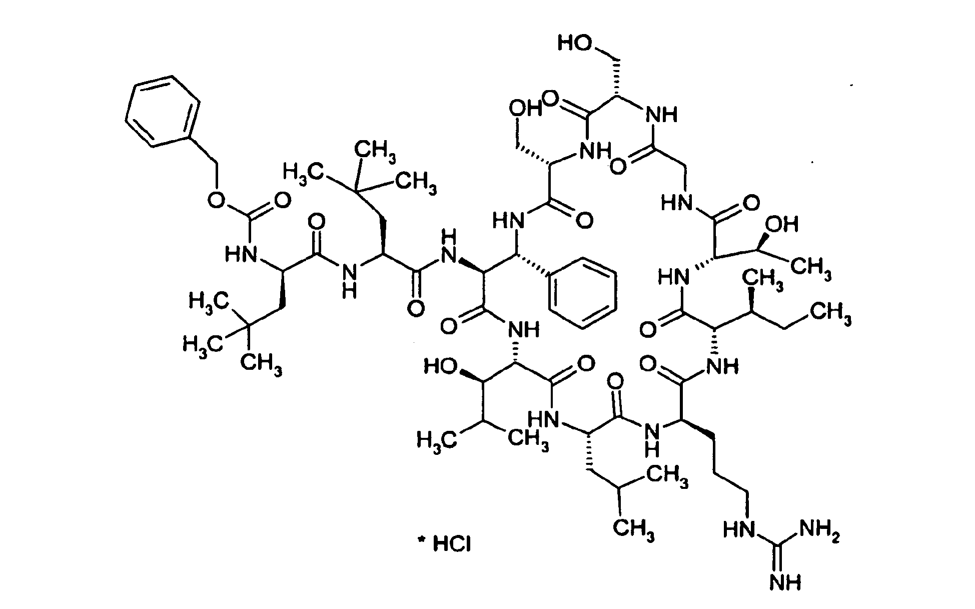

Die Verbindung aus Beispiel 225A (4.3 mg, 4 µmol) und die Verbindung aus Beispiel 10A (2.5 mg, 6 µmol, 1.5 Äquivalente) werden in DMF (300 µl) gelöst und die Lösung auf 0°C gekühlt. 4-Methylmorpholin (20 µl, 16 µmol, 4 Äquivalente) und HATU (2.4 mg, 6 µmol, 1.6 Äquivalente) werden zugegeben und der Ansatz 2 h bei Raumtemperatur gerührt. Dann wird mit 3 ml Methanol abgestoppt und nach Methode 45 chromatographisch gereinigt. Produkthaltige Fraktionen werden vereinigt und eingeengt. Man erhält 4 mg (2.8 µmol 70% d. Th.) der Titelverbindung als Feststoff.
HPLC (Methode 5): Rₜ = 4.45 min.
LC-MS (Methode 19): Rₜ = 2.08 min, MS (ESIpos): m/z (%) = 698.6 (100) [M+2H]²⁺, 1395.4 (30) [M+H]⁺.
HR-TOF-MS (Methode 24): C₆₇H₁₀₉N₁₅O₁₇ ber. 1394.8043, gef. 1394.8070 [M+H]⁺.

### Beispiel 234A

### [N²-(Benzyloxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin-C^{1.11}-N^{3.3}-lactam-hydrochlorid

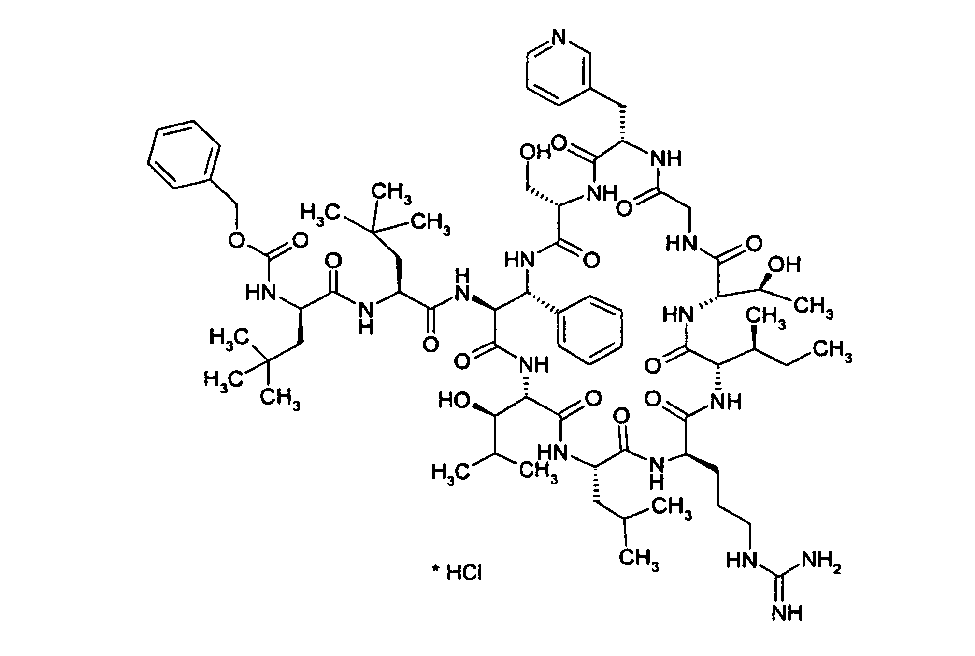

Gemäß der Herstellungsvorschrift zur Darstellung von Beispielverbindung 233A wird aus Beispielverbindung 226A (17 mg, 13 µmol) die Titelverbindung in einer Ausbeute von 12 mg (54% d. Th.) erhalten.
HPLC (Methode 5): Rₜ = 4.25 min.
LC-MS (Methode 22): Rₜ = 3.61 min, MS (ESIpos): m/z (%) = 729 (100) [M+2H]²⁺; (ESIneg): m/z (%) = 1454 (100) [M-H]⁻.
HR-TOF-MS (Methode 24): C₇₂H₁₁₁N₁₆O₁₆ ber. 1455.8359, gef. 1455.8347 [M+H]⁺.

### Beispiel 235A

### [N²-(tert-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-phenylalanyl-L-serin-C^{1.11}-N^{3.3}-lactam-hydrochlorid

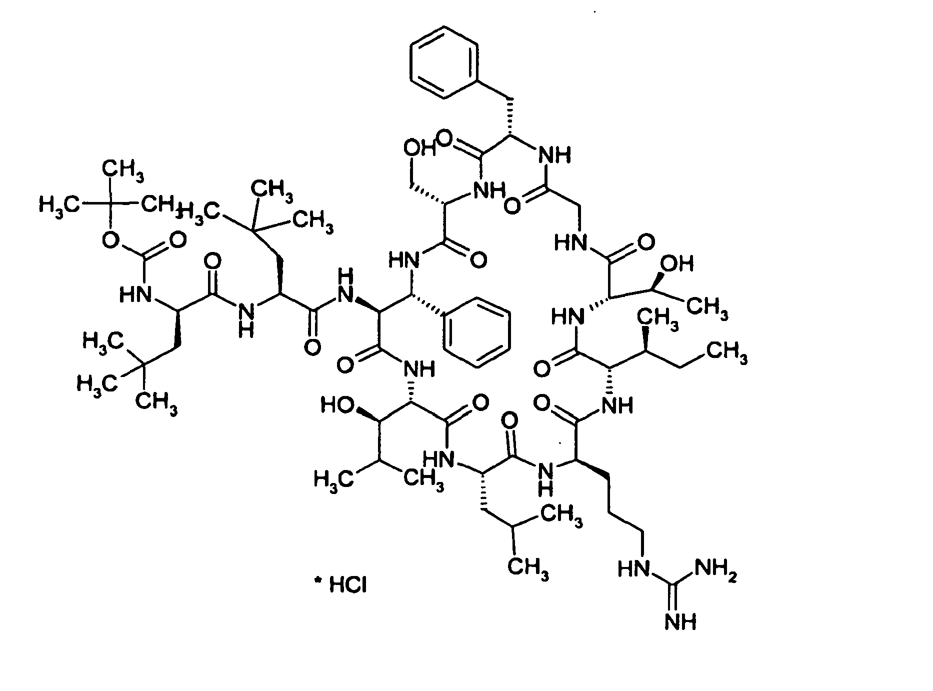

Gemäß der Herstellungsvorschrift der Verbindung aus Beispiel 233A wird aus der Verbindung aus Beispiel 227A (24 mg, 21 µmol) und dem Dipeptid aus Beispiel 8A (10 mg, 27 µmol, 1.3 Äquivalente) die Titelverbindung in einer Ausbeute von 19 mg (62% d. Th.) erhalten.
HPLC (Methode 6): Rₜ = 4.57 min.
LC-MS (Methode 20): Rₜ = 2.291 min, MS (ESIpos): m/z (%) = 661.2 (100) [M-Boc +2H]²⁺, 1421.0 (30) [M+H]⁺; (ESIneg): m/z (%) = 1419.1 (20) [M-H]⁻.
HR-TOF-MS (Methode 24): C₇₀H₁₁₃N₁₅O₁₆ ber. 1420.8563, gef. 1420.8558 [M+H]⁺.

### Beispiel 236A

### [N²-(tert.-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-[L-threonyl]-L-serin C^{1.11}-N^{3.3}-lactam-hydrochlorid

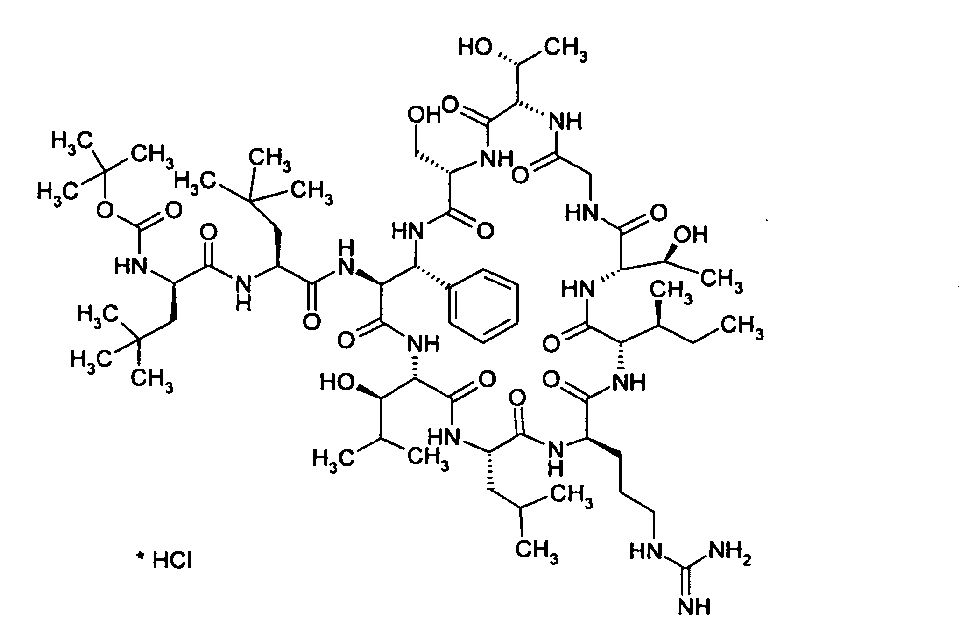

Gemäß der Herstellungsvorschrift der Verbindung aus Beispiel 233A wird aus der Verbindung aus Beispiel 228A (8.5 mg, 8 µmol) und dem Dipeptid aus Beispiel 8A (4 mg, 10 µmol, 1.3 Äquivalente) die Titelverbindung in einer Ausbeute von 10 mg (91% d. Th.) erhalten.
HPLC (Methode 5): Rₜ = 4.65 min.
LC-MS (Methode 19): Rₜ = 2.09 min, MS (ESIpos): m/z (%) = 1374.8 (30) [M+H]⁺
HR-TOF-MS (Methode 24): C₆₅H₁₁₂N₁₅O₁₇ ber. 1374.8356, gef. 1374.8376 [M+H]⁺.

### Beispiel 237A

[*N*²-(*tert*.-Butoxycarbonyl)-3-*tert*-butyl-D-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-L-allothreonyl-L-serinat-*C^{1.11}-N^{3.3}*-lactam- hydrochlorid

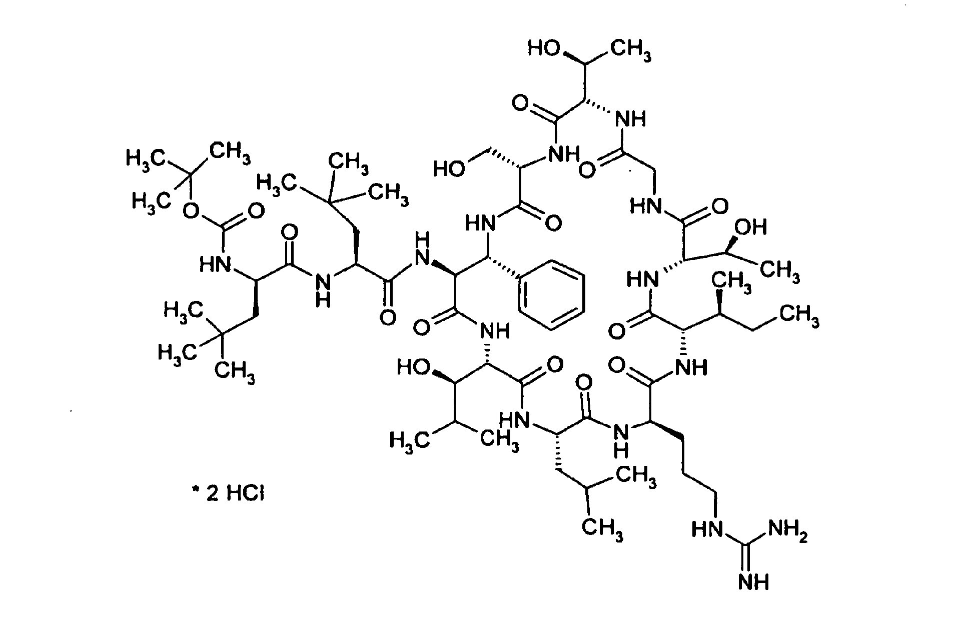

Gemäß der Herstellungsvorschrift der Verbindung aus Beispiel 233A wird aus der Verbindung aus Beispiel 229A (40 mg, 35 µmol) und dem Dipeptid aus Beispiel 8A (17 mg, 46 µmol, 1.5 Äquivalente) die Titelverbindung in einer Ausbeute von 47 mg (Reinheit 77%, 69% d. Th.) erhalten.
HPLC (Methode 6): Rₜ = 4.31 min.
LC-MS (Methode 19): Rₜ = 2.08 min, MS (ESIpos): m/z (%) = 1374.8 (50) [M+H]⁺
HR-TOF-MS (Methode 24): C₆₅H₁₁₂N₁₅O₁₇ ber. 1374.8356, gef. 1374.8362 [M+H]⁺.

### Beispiel 238A

### [N²-(tert.-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-alanyl-L-serin C^{1.11}-N^{3.3}-lactam- hydrochlorid

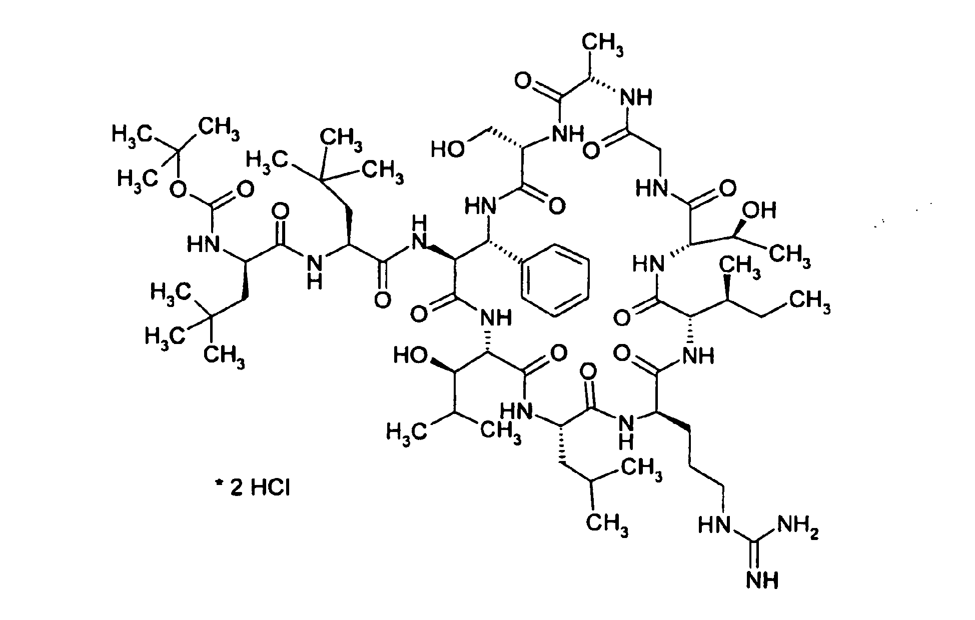

Gemäß der Herstellungsvorschrift der Verbindung aus Beispiel 233A wird aus der Verbindung aus Beispiel 230A (29 mg, 27 µmol) und dem Dipeptid aus Beispiel 8A (13 mg, 35 µmol, 1.3 Äquivalente) die Titelverbindung in einer Ausbeute von 37 mg (98% d. Th.) erhalten.
HPLC (Methode 5): Rₜ = 4.60 min.
LC-MS (Methode 19): Rₜ = 2.10 min, MS (ESIpos): m/z (%) = 623.0 (100) [M - Boc +2H]²⁺; 1344.9 (90) [M+H]⁺
HR-TOF-MS (Methode 24): C₆₄H₁₀₉N₁₅O₁₆ ber. 1344.8250, gef. 1344.8241 [M+H]⁺.

### Beispiel 239A

### [N²-(tert.-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin C^{1.11}-N^{3.3}-lactam-hydrochlorid

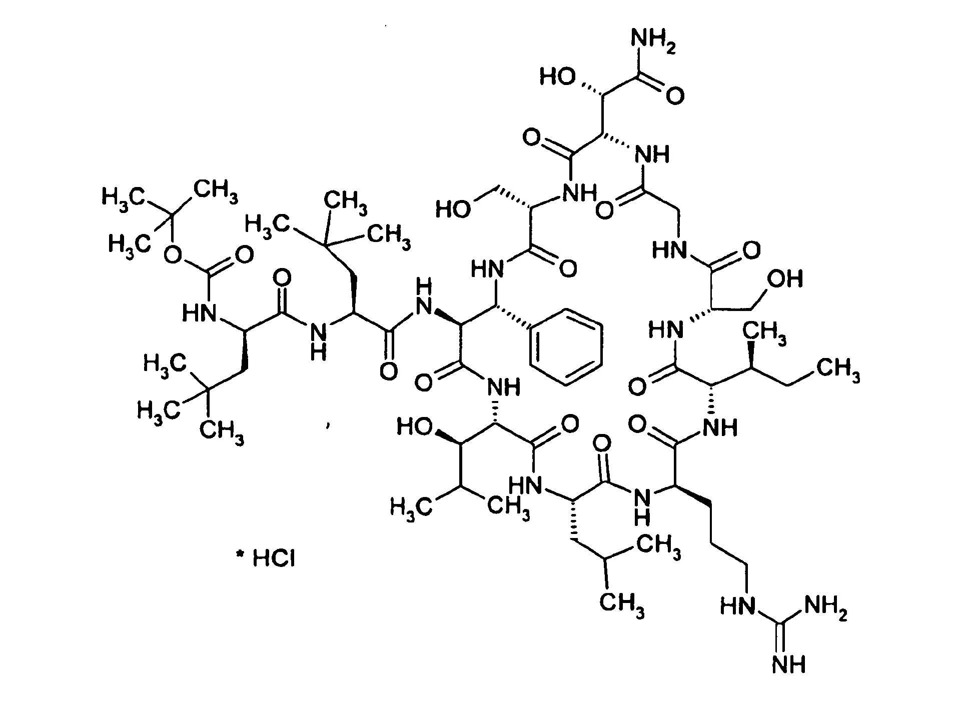

Gemäß der Herstellungsvorschrift der Verbindung aus Beispiel 233A wird aus der Verbindung aus Beispiel 231A (21 mg, 19 µmol) und dem Dipeptid aus Beispiel 8A (9 mg, 25 µmol, 1.3 Äquivalente) die Titelverbindung in einer Ausbeute von 24 mg (89% d. Th.) erhalten.
HPLC (Methode 5): Rₜ = 4.42 min.
LC-MS (Methode 19): Rₜ = 2.03 min, MS (ESIpos): m/z (%) = 645.5 (100) [M - Boc +2H]²⁺; 1389.9 (40) [M+H]⁺
HR-TOF-MS (Methode 24): C₆₄H₁₀₈N₁₆O₁₈ ber. 1389.8101, gef. 1389.8105 [M+H]⁺.

### Beispiel 240A

### [N²-(tert.-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-C^{1.9}-N^{3.1}-lactam-hydrochlorid

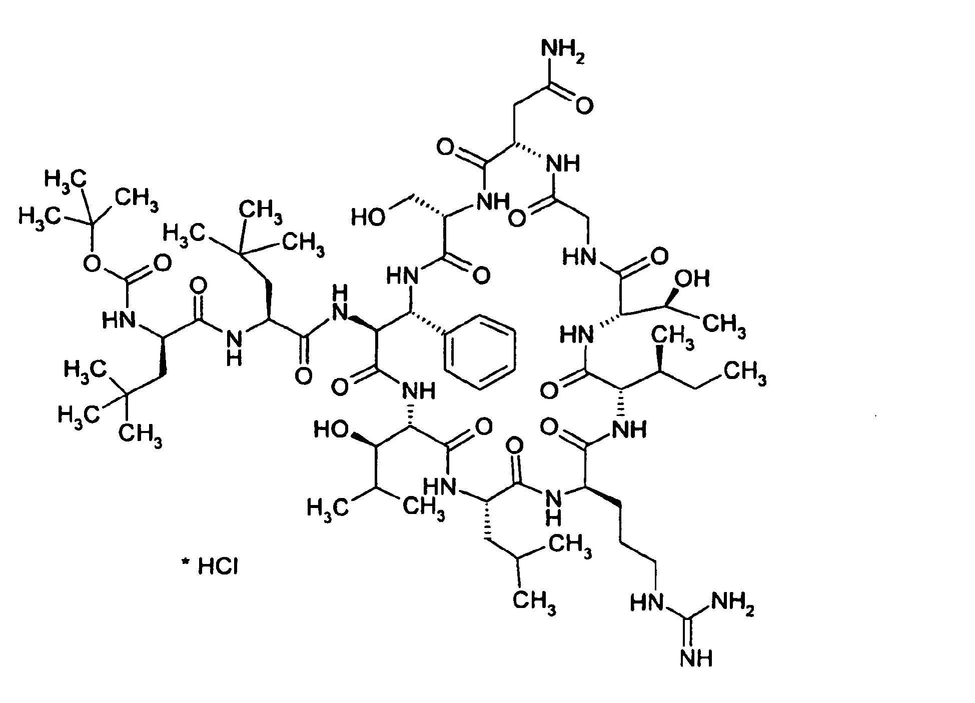

Gemäß der Herstellungsvorschrift der Verbindung aus Beispiel 233A wird aus der Verbindung aus Beispiel 232A (24 mg, 22 µmol) und dem Dipeptid aus Beispiel 8A (11 mg, 28 µmol, 1.3 Äquivalente) die Titelverbindung in einer Ausbeute von 30 mg (97% d. Th.) erhalten.
HPLC (Methode 5): Rₜ = 4.26 min.
LC-MS (Methode 21): Rₜ = 2.19 min, MS (ESIpos): m/z (%) = 644.7 (100) [M - Boc +2H]²⁺.
HR-TOF-MS (Methode 24): C₆₅H₁₁₁N₁₆O₁₇ ber. 1387.8308, gef. 1387.8304 [M+H]⁺.

### Beispiel 241A

### Benzyl N²-(tert-Butoxycarbonyl)-L-Alaninat

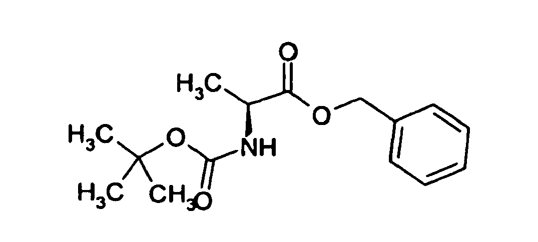

N²-(*tert-*Butoxycarbonyl)-L-Alanin (1.13 g, 5.83 mmol) wird in trockenem DCM (6 ml) gelöst, etwas Molekularsieb 3 Å wird zugegeben und der Ansatz dann auf 0 °C gekühlt. Benzylalkohol (1.81 ml, 17.48 mmol, 3 Äquivalente), EDCI (2.23 g, 11.65 mmol, 2 Äquivalente), und DMAP (71 mg, 0.58 mmol, 0.1 Äquivalente) werden zugegeben. Der Ansatz wird noch 150 min gerührt, wobei man ihn langsam auf RT erwärmen lässt. Dann wird zur Trockne eingeengt und der Rückstand chromatographisch aufgearbeitet (Biotage, Cyclohexan-Ethylacetat 9:1). Produkthaltige Fraktionen werden vereinigt und eingeengt. Die Titelverbindung wird in einer Ausbeute von 1.03 g (63% d. Th.) erhalten.
HPLC (Methode 5): Rₜ = 4.70 min.
LC-MS (Methode 21): Rₜ = 2.44 min, MS (ESIpos): m/z (%) = 280.3 (30) [M+H]⁺.

### Beispiel 242A

### Benzyl L-Alaninat-trifluoracetat

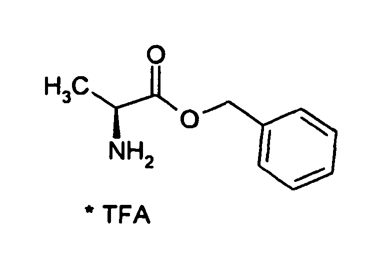

Die Verbindung aus Beispiel 241A (200 mg, 716 µmol) wird nach Arbeitsvorschrift 2 mit 2.0 ml der Reagenzlösung über 60 min umgesetzt. Ohne weitere Aufreinigung erhält man 200 mg (95% d. Th.) der rohen Titelverbindung.
LC-MS (Methode 22): Rₜ = 2.30 min, MS (ESIpos): m/z (%) = 180 (100) [M+H]⁺.

### Beispiel 243A

### Benzyl [N²-(tert-Butoxycarbonyl)-glycyl)-[(3S)-3-hydroxy-O⁴-methyl-L-aspartyl]-L-alaninat

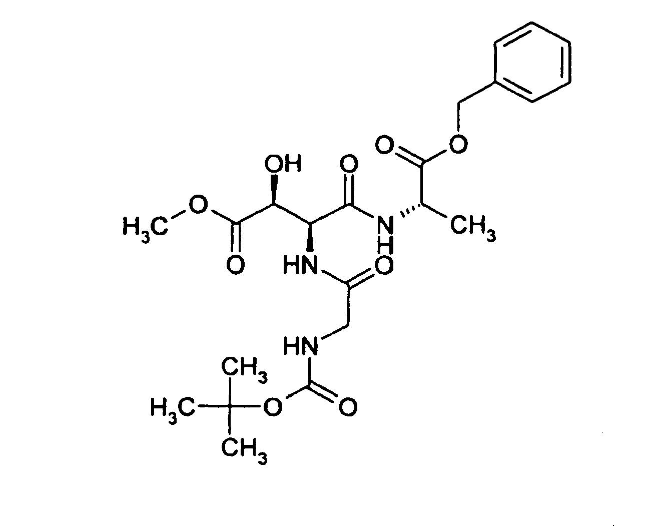

Die Verbindung aus Beispiel 33A (441 mg, 895 µmol) und die Verbindung aus Beispiel 242A (315 mg, 1.07 mmol, 1.2 Äquivalente) werden in DMF (4.8 ml) gelöst und auf -20 °C gekühlt. Dann werden 4-Methylmorpholin (492 µl, 4.48 mmol, 5 Äquivalente) und HATU (510 mg, 1.34 mmol, 1.5 Äquivalente) dazugegeben, der Ansatz langsam auf Raumtemperatur erwärmt und über ca 16 h gerührt. Dann wird chromatographisch in zwei Schritten (Methode 45, dann Methode 34) gereinigt. Die Titelverbindung wird in einer Ausbeute von 277 mg (64% d. Th.) erhalten.
HPLC (Methode 5): Rₜ = 4.06 min.
LC-MS (Methode 19): Rₜ = 2.13 min, MS (ESIpos): m/z (%) = 482.1 (100) [M+H]⁺.
HR-TOF-MS (Methode 24): C₂₂H₃₂N₃O₉ ber. 482.2134, gef. 482.2120 [M+H]⁺.

### Beispiel 244A

### Benzyl [N²-(tert-Butoxycarbonyl)-glycyl]-[(3S)-3-hydroxy-L-asparaginyl]-L-alaninat

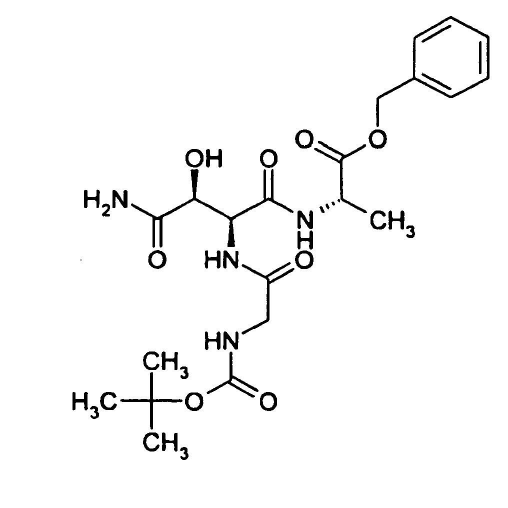

Die Verbindung aus Beispiel 243A (139 mg, 288 µmol) wird in 5.1 ml Acetonitril vorgelegt und auf 0 °C gekühlt; 3.1 ml konz. wässrige Ammoniaklösung werden zugegeben und weiter bei 0 °C gerührt. Nach 20 min wird mit Eisessig (2.6 ml) abgestoppt, mit Wasser verdünnt und mit Ethylacetat extrahiert. Der organische Extrakt wird mit konz Kochsalzlösung gewaschen, über Natriumsulfat getrocknet und eingeengt. Ausbeute: 122 mg (75% d. Th.) der rohen Titelverbindung, die ohne Reinigung weiter umgesetzt wird.
HPLC (Methode 6): Rₜ = 3.78 min.
LC-MS (Methode 20): Rₜ = 1.69 min, MS (ESIpos): m/z (%) = 467.1 (100) [M+H]⁺; MS (ESIneg): m/z (%) = 465.2 (100) [M-H]⁻.
HR-TOF-MS (Methode 24): C₂₁H₃₁N₄O₈ ber. 467.2137, gef. 467.2137 [M+H]⁺.

### Beispiel 245A

### Benzyl Glycyl-[(3S)-3-hydroxy-L-asparaginyl]-L-alaninat-trifluoracetat

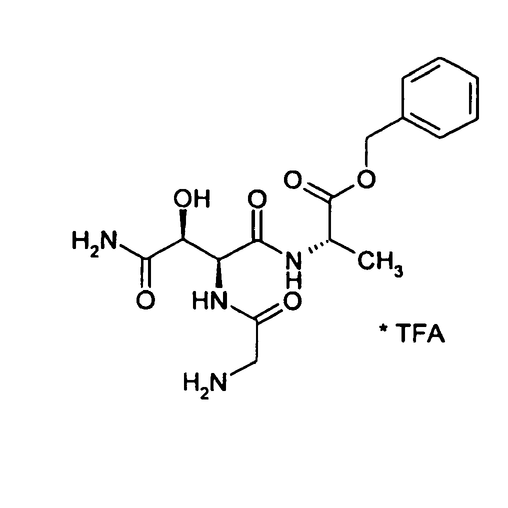

Die Verbindung aus Beispiel 241A (122 mg, 217 µmol) wird nach Arbeitsvorschrift 2 mit 2.0 ml der Reagenzlösung über 60 min umgesetzt. Ohne weitere Aufreinigung erhält man 100 mg (96% d.Th.) der rohen Titelverbindung.
HPLC (Methode 6): Rₜ = 3.05 min.
LC-MS (Methode 22): Rₜ = 2.23 min, MS (ESIpos): m/z (%) = 367 (100) [M+H]⁺.
HR-TOF-MS (Methode 24): C₁₆H₂₃N₄O₆ ber. 367.1613, gef. 367.1622 [M+H]⁺.

### Beispiel 246A

### Benzyl [(3R)-N²-(tert-Butoxycarbonyl)-3-hydroxy-L-leucyl]-L-leucyl-D-arginyl-L-isoleucyl-L-allothreonyl-glycyl-[(3S)-3-hydroxy-L-asparaginyl]-L-alaninat-trifluoracetat

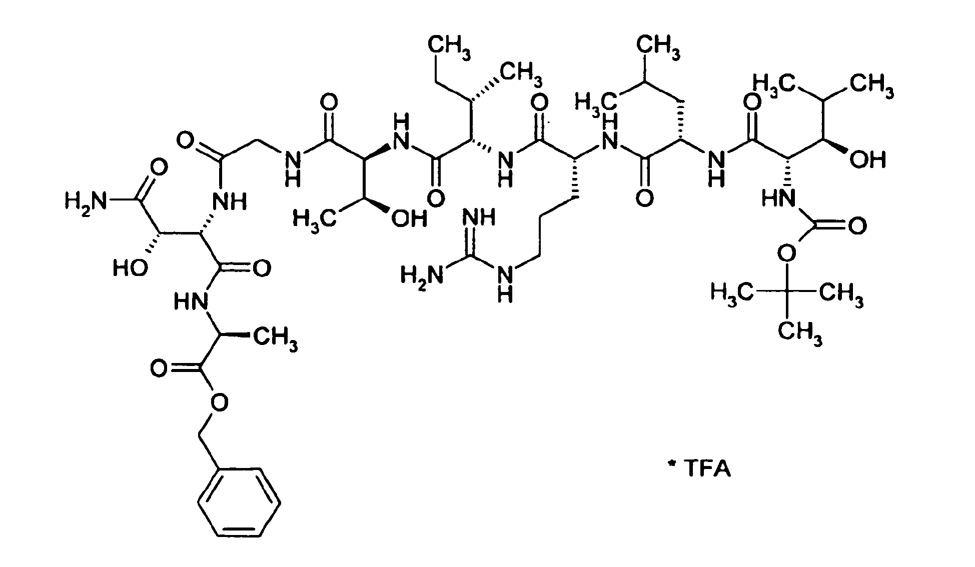

Die Verbindung aus Beispiel 32A (133 mg, 139 mmol) und die Verbindung aus Beispiel 245A (100 mg, 208 µmol, 1.5 Äquivalente) werden in DMF (950 µl) gelöst und die Lösung auf -20°C gekühlt. 4-Methylmorpholin (46 µl, 416 µmol, 3 Äquivalente) und HATU (84 mg, 222 µmol, 1.6 Äquivalente) werden zugegeben und der Ansatz 16 h bei Raumtemperatur gerührt. Dann wird der gesamte Ansatz nach Methode 45 chromatographisch gereinigt. Produkthaltige Fraktionen werden vereinigt und lyophilisiert. Man erhält 157 mg (95% d. Th.) der Titelverbindung als Feststoff.
HPLC (Methode 6): Rₜ = 3.66 min.
LC-MS (Methode 19): Rₜ = 1.71 min, MS (ESIpos): m/z (%) = 1079.1 (80) [M+H]⁺.
HR-TOF-MS (Methode 24): C₄₉H₆₃N₁₂O₁₅ ber. 1079.6096, gef. 1079.6094 [M+H]⁺.

### Beispiel 247A

### [(3R)-N²-(tert-Butoxycarbonyl)-3-hydroxy-L-leucyl]-L-leucyl-D-arginyl-L-isoleucyl-L-allothreonyl-glycyl-[(3S)-3-hydroxy-L-asparaginyl]-L-alanin-trifluoracetat

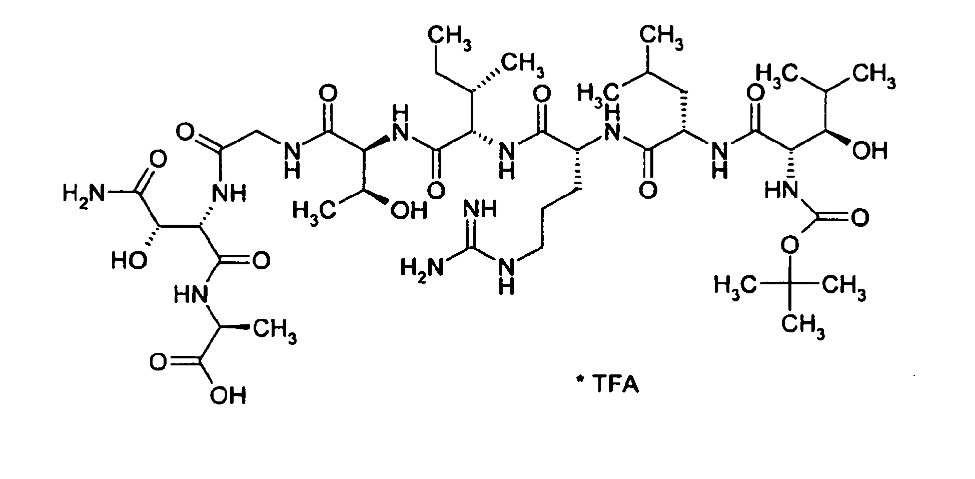

Gemäß der Vorschrift zur Darstellung der Verbindung aus Beispiel 186A wird die Verbindung aus Beispiel 246A (190 mg, 159 µmol) hydriert. Man erhält 172 mg (98% d. Th.) der Titelverbindung als Feststoff.
HPLC (Methode 6): Rₜ = 3.39 min.
LC-MS (Methode 19): Rₜ = 1.55 min, MS (ESIpos): m/z (%) = 445.3 (100) [M+2H]²⁺, 989.5 (60) [M+H]⁺; MS (ESIneg): m/z (%) = 987.5 (100) [M-H]⁻.
HR-TOF-MS (Methode 24): C₄₂H₇₇N₁₂O₁₅ ber. 989.5626, gef. 989.5651 [M+H]⁺.

### Beispiel 248A

### [(3R)-3-hydroxy-L-leucyl]-L-leucyl-D-arginyl-L-isoleucyl-L-allothreonyl-glycyl-[(3S)-3-hydroxy-L-asparaginyl]-L-alanin-bis-trifluoracetat

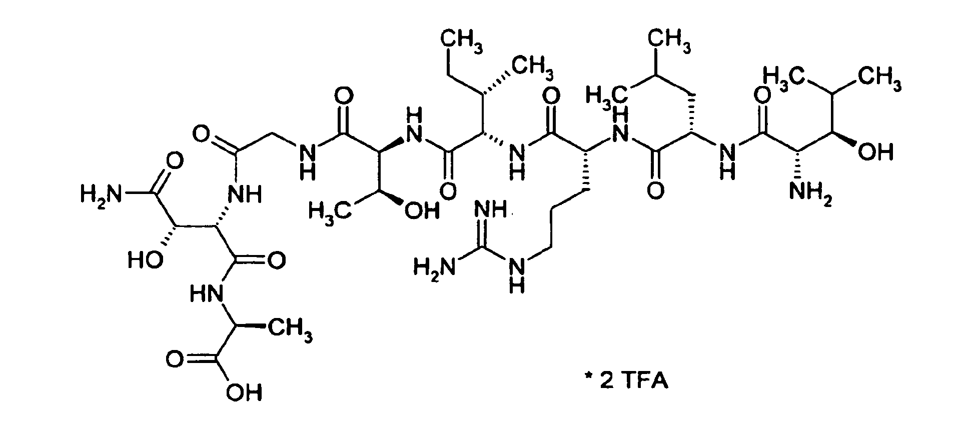

Die Verbindung aus Beispiel 247A (172 mg, 156 µmol) wird nach Arbeitsvorschrift 2 mit 2.0 ml der Reagenzlösung über 30 min umgesetzt. Ohne weitere Aufreinigung erhält man 174 mg (quant.) der rohen Titelverbindung.
HPLC (Methode 6): Rₜ = 2.78 min.
LC-MS (Methode 22): Rₜ = 2.17 min, MS (ESIpos): m/z (%) = 889 (80) [M+H]⁺; MS (ESIneg): m/z (%) = 887 (100), [M-H]⁻.
HR-TOF-MS (Methode 24): C₃₇H₆₉N₁₂O₁₃ ber. 889.5102, gef. 889.5096 [M+H]⁺.

### Beispiel 249A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-trifluoracetat

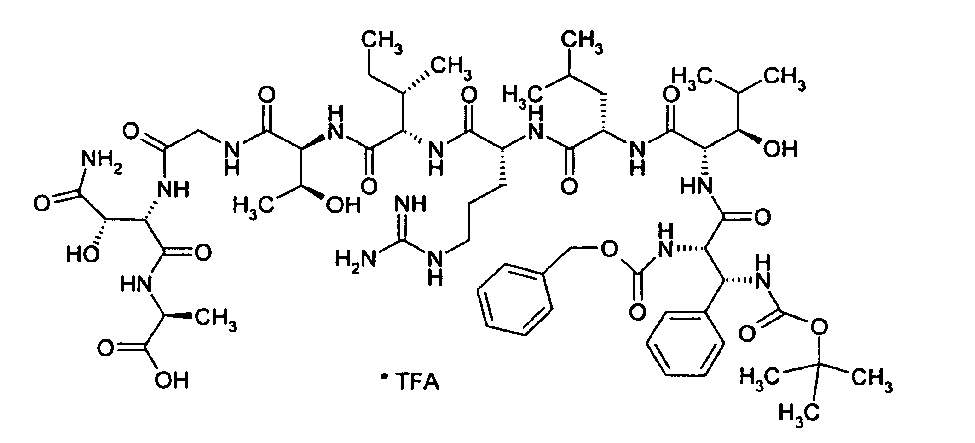

Gemäß der Vorschrift zur Darstellung der Verbindung aus Beispiel 201A wird die Verbindung aus Beispiel 248A (174 mg, 156 µmol) mit der Verbindung aus Beispiel 17A (99 mg, 171 µmol, 1.1 Äquivalente) umgesetzt. Nach chromatographischer Reinigung (Variante von Methode 36, statt des Gradienten wird isokratisch mit Eluent A:Eluent B = 3:2 getrennt) werden 81 mg (34% d. Th.) der Titelverbindung isoliert.
HPLC (Methode 6): Rₜ = 3.76 min.
LC-MS (Methode 19): Rₜ = 1.89 min, MS (ESIpos): m/z (%) = 593.4 (100) [M-Boc+2H]²⁺, 1285.7 (30) [M+H]⁺; (ESIneg): m/z (%) = 1283.5 (100) [M-H]⁻.
HR-TOF-MS (Methode 24): C₅₉H₉₃N₁₄O₁₈ ber. 1285.6787, gef. 1285.6781 [M+H]⁺.

### Beispiel 250A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-bis-trifluoracetat

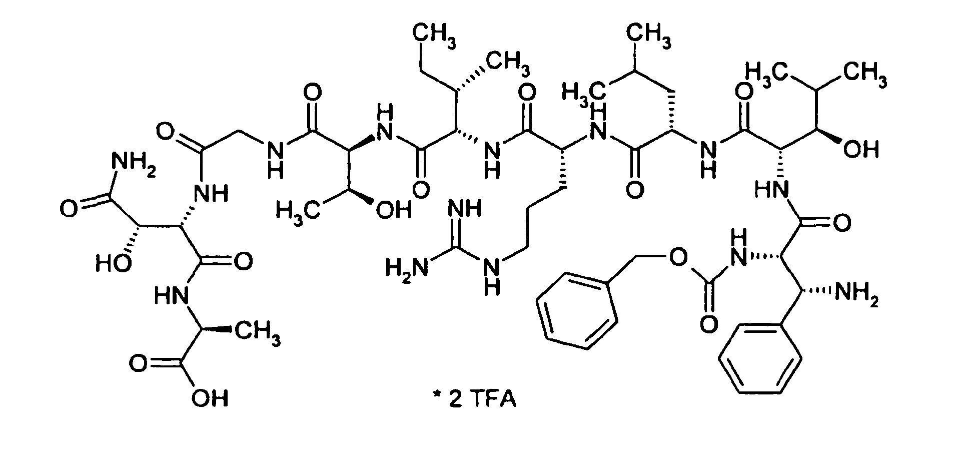

Die Verbindung aus Beispiel 249A (81 mg, 54 µmol) wird nach Arbeitsvorschrift 2 mit 2.0 ml der Reagenzlösung über 60 min umgesetzt. Ohne weitere Aufreinigung erhält man 81 mg (99% d. Th.) der rohen Titelverbindung.
HPLC (Methode 6): Rₜ = 3.20 min.
LC-MS (Methode 19): Rₜ = 1.29 min, MS (ESIpos): m/z (%) = 593.7 (100) [M+2H]²⁺, 1185.6 (5) [M+H]⁺; MS (ESIneg): m/z (%) = 1183.7 (100), [M-H]⁻.
HR-TOF-MS (Methode 24): C₅₄H₈₅N₁₄O₁₆ ber. 1185.6263, gef. 1185.6249 [M+H]⁺.

### Beispiel 251A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-C^{1.9}-N^{3.1}-lactam-trifluoracetat

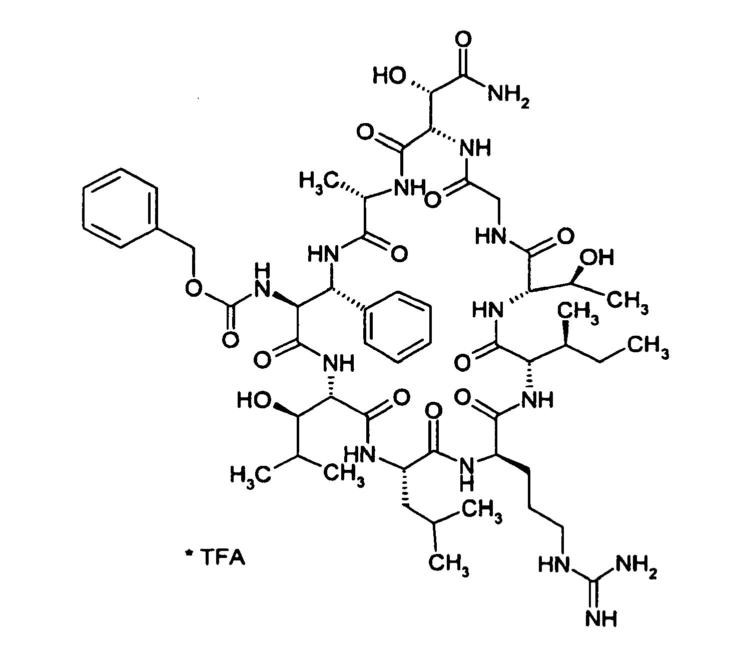

Gemäß der Vorschrift zur Darstellung der Verbindung aus Beispiel 217A, wobei die Aufreinigung nach Methode 45 erfolgt, wird aus der Verbindung aus Beispiel 250A (81 mg, 53 µmol) die Titelverbindung in einer Ausbeute von 69 mg (93% d. Th.) erhalten.
HPLC (Methode 6): Rₜ = 3.56 min.
LC-MS (Methode 19): Rₜ = 1.67 min, MS (ESIpos): m/z (%) = 1167.6 (100) [M+H]⁺; MS (ESIneg): m/z (%) = 1165.6 (100), [M-H]⁻.
HR-TOF-MS (Methode 24): C₅₄H₈₃N₁₄O₁₅ ber. 1167.6157, gef. 1167.6154 [M+H]⁺.

### Beispiel 252A

### [(3R)-3-Amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-C^{1.9}-N^{3.1}-lactam-bis-hydrochlorid

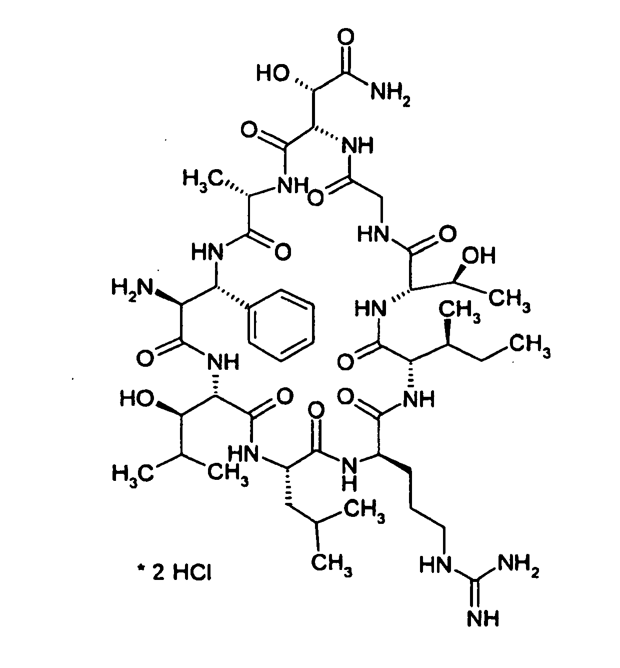

Gemäß der Vorschrift zur Darstellung der Verbindung aus Beispiel 225A wird aus der Verbindung aus Beispiel 251A (69 mg, 49 µmol) die Titelverbindung in einer Ausbeute von 53 mg (97% d. Th.) erhalten.
HPLC (Methode 6): Rₜ = 2.98 min.
LC-MS (Methode 20): Rₜ = 0.96 min, MS (ESIpos): m/z (%) = 517.5 (100) [M+2H]²⁺, 1033.7 (10) [M+H]⁺; MS (ESIneg): m/z (%) = 1031.7 (60) [M-H]⁻, 1077.7 (100) [M+HCOOH-H]⁻.
HR-TOF-MS (Methode 24): C₄₆H₇₇N₁₄O₁₃ ber. 1033.5790, gef. 1033.5782 [M+H]⁺.

### Beispiel 253A

### [N²-(tert.-Butoxycarbonyl)-3-tert-butyl-D-alanyl]-[3-tert-butyl-L-alanyl]- [(3R)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-C^{1.11}-N^{3.3}-lactam-hydrochlorid

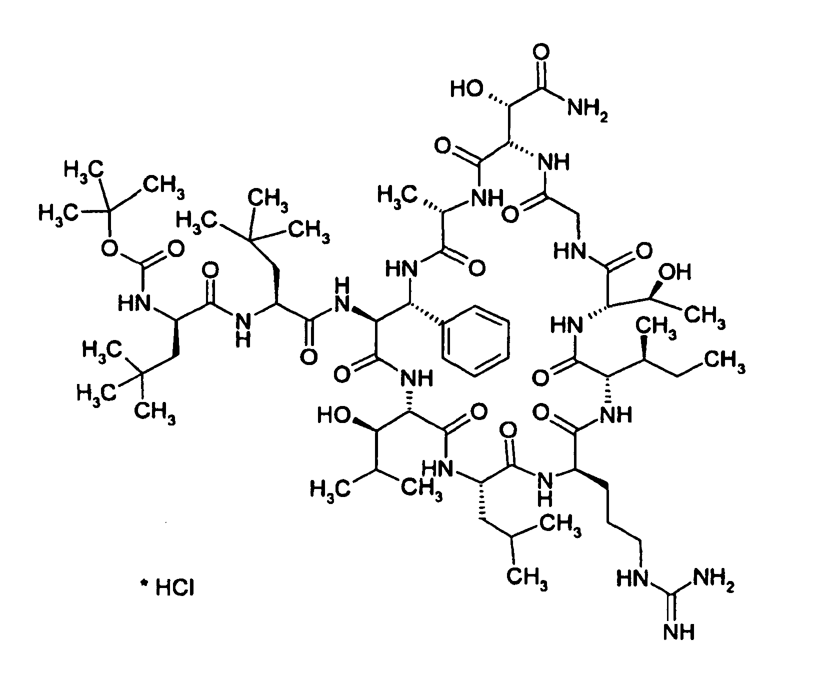

Gemäß der Vorschrift zur Darstellung der Verbindung aus Beispiel 233A wird aus der Verbindung aus Beispiel 252A (53 mg, 48 µmol) und dem Dipeptid aus Beispiel 8A (23 mg, 62 µmol, 1.3 Äquivalente) die Titelverbindung in einer Ausbeute von 58 mg (85% d. Th.) erhalten.
HPLC (Methode 6): Rₜ = 4.35 min.
LC-MS (Methode 19): Rₜ = 2.08 min, MS (ESIpos): m/z (%) = 1387.9 (100) [M+H]⁺.
HR-TOF-MS (Methode 24): C₆₅H₁₁₁N₁₆O₁₇ ber. 1387.8308, gef. 1387.8314 [M+H]⁺.

### Beispiel 254A

### Pentafluorphenyl [(3R)-N²-(tert-Butoxycarbonyl)-3-hydroxy-L-leucyl]-L-leucyl-D-arginyl-L-isoleucyl-L-allothreoninat-trifluoracetat

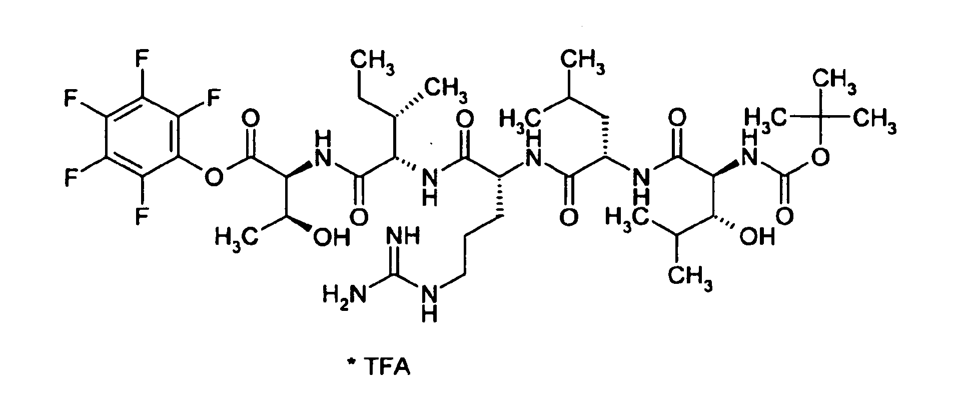

Die Verbindung aus Beispiel 32A (60 mg, 71 µmol), Pentafluorphenol (65 mg, 355 µmol, 5 Äquivalente) und EDCI (20 mg, 107 µmol, 1.5 Äquivalente) werden in Dichlormethan (0.9 ml) bei 0°C unter Argon gelöst und bei Raumtemperatur über Nacht gerührt. Das Lösemittel wird abgezogen und der Rückstand chromatographiert (Methode 34). Die Titelverbindung wird in einer Ausbeute von 32 mg (45% d. Th.) isoliert.
HPLC (Methode 6): Rₜ = 4.12 min.
LC-MS (Methode 19): Rₜ = 1.93 min, MS (ESIpos): m/z (%) = 897.4 (100) [M+H]⁺.

### Beispiel 255A

### Pentafluorphenyl N²-(tert-Butoxycarbonyl)-D-alanin

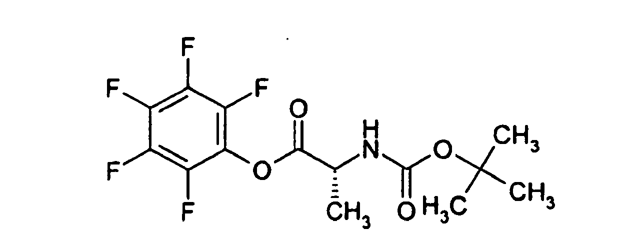

Analog zur Darstellung der Verbindung aus Beispiel 254A wird aus *N²-*(*tert-*Butoxycarbonyl)-D-alanin (390 mg, 2.06 mmol) die Titelverbindung in einer Ausbeute von 471 mg (64% d. Th.) isoliert.
HPLC (Methode 5): Rₜ = 5.00 min.
LC-MS (Methode 21): Rₜ = 2.74 min, MS (ESIpos): m/z (%) = 356 (5) [M+H]⁺.

### Beispiel 256A

### [N²-(tert-Butoxycaibonyl)-D-alanyl]-(3S)-O⁴-Methyl-3-hydroxy-L-asparaginsäure

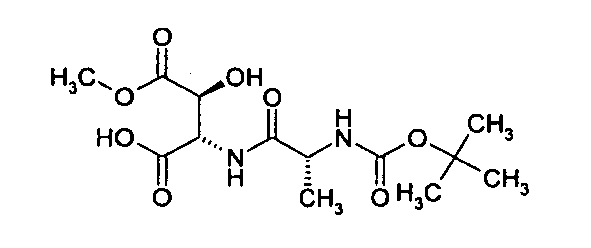

Die Verbindung aus Beispiel 255A (470 mg, 1.32 mmol) und (2S,3S)-2-Amino-3-hydroxy-4-methoxy-4-oxobuttersäure Hydrochlorid (Darstellung siehe Beispiel 33A, 407 mg, 2.04 mmol, 1.6 Äquivalente) werden bei 0°C in DMF (20 ml) gelöst, DIPEA (1.78 ml, 10.20 mmol, 7.7 Äquivalente) werden hinzugefügt. Der Ansatz wird ca 16 h bei Raumtemperatur gerührt, dann wird er nach Methode 45 chromatographiert und nach Methode 32 feingereinigt. Ausbeute: 200 mg (29% d. Th.).
HPLC (Methode 5): Rₜ = 3.26 min.
LC-MS (Methode 19): Rₜ = 1.48 min, MS (ESIpos): m/z (%) = 335.2 (90) [M+H]⁺.

### Beispiel 257A

### Benzyl [N²-(tert-Butoxycarbonyl)-D-alanyl]-[(3S)-O⁴-Methyl-3-hydroxy-L-aspartyl]-L-serinat

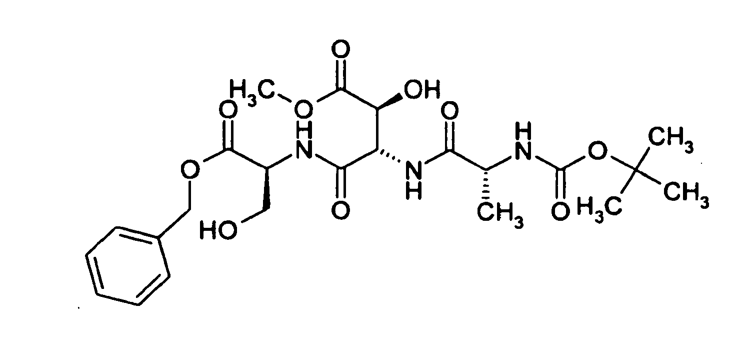

Die Verbindung aus Beispiel 256A (195 mg, 0.58 mmol) und die Verbindung aus Beispiel 149A (190 mg, 0.61 mmol, 1.1 Äquivalente) werden in DMF (5.0 ml) gelöst und die Lösung auf 0°C gekühlt. 4-Methylmorpholin (203 µl, 1.84 mmol, 3 Äquivalente) und TCTU (326 mg, 0.92 mmol, 1.5 Äquivalente) werden zugegeben und der Ansatz 2.5 h bei Raumtemperatur gerührt. Dann wird der gesamte Ansatz chromatographiert (Methode 45) und nach Methode 32 feingereinigt. Produkthaltige Fraktionen werden vereinigt und lyophilisiert. Man erhält 146 mg (47% d. Th.) der Titelverbindung als Feststoff.
HPLC (Methode 5): Rₜ = 3.89 min.
LC-MS (Methode 19): Rₜ = 1.94 min, MS (ESIpos): m/z (%) = 511.1 (100) [M+H]⁺.
[α]²⁰_{Na} = +15.9 (c = 0.5, MeOH).

### Beispiel 258A

### [N²-(tert-Butoxycarbonyl)-D-alan yl]-[(3S)-O⁴-Methyl-3-hydroxy-L-aspartyl]-L-serin

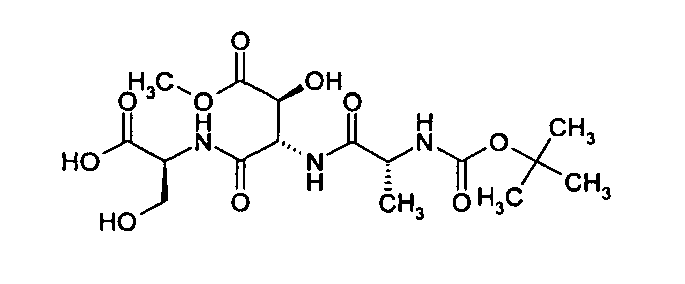

Die Verbindung aus Beispiel 257A (135 mg, 264 µmol) wird in Methanol (8.0 ml) in Anwesenheit von 30 mg 10% Palladiumkohle 2 h bei Raumtemperatur hydriert. Man filtriert vom Katalysator ab, engt ein und chromatographiert das Rohprodukt (Methode 32). Ausbeute: 117 mg (quant.).
HPLC (Methode 5): Rₜ = 3.11 min.
LC-MS (Methode 19): Rₜ = 1.40 min, MS (ESIpos): m/z (%) = 422.1 (100) [M+H]⁺.
HR-TOF-MS (Methode 24): C₁₆H₂₈N₃O₁₀ ber. 422.1770, gef. 422.1776 [M+H]⁺.

### Beispiel 259A

### [N²-(tert-Butoxycarbonyl)-D-alanyl]-[(3S)-3-hydroxy-L-asparaginyl]-L-serin

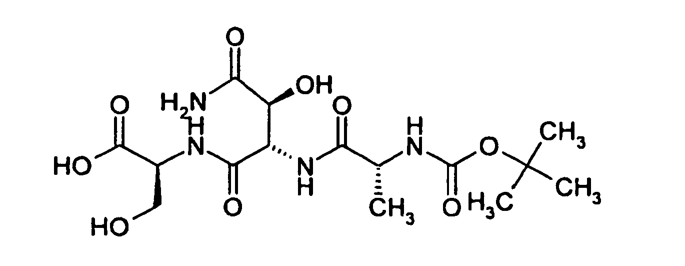

Die Verbindung aus Beispiel 258A (116 mg, 275 µmol) wird in 5.0 ml Acetonitril vorgelegt und auf 0°C gekühlt; 3.0 ml konz. wässrige Ammoniaklösung werden zugegeben und weiter bei 0°C gerührt. Nach 5 min wird mit Eisessig (1.2 ml) abgestoppt, eingeengt und chromatographiert (Methode 32). Ausbeute: 64 mg (57% d. Th.).
HPLC (Methode 6): Rₜ = 2.82 min.
LC-MS (Methode 19): Rₜ = 1.12 min, MS (ESIpos): m/z (%) = 407.0 (90) [M+H]⁺; MS (ESIneg): m/z (%) = 405.0 (100) [M-H]⁻.
HR-TOF-MS (Methode 24): C₁₅H₂₁N₄O₉ ber. 407.1773, gef. 407.1781 [M+H]⁺.

### Beispiel 260A

### [D-alanyl]-[(3S)-3-hydroxy-L-aspartyl-L-serin-trifluoracetat

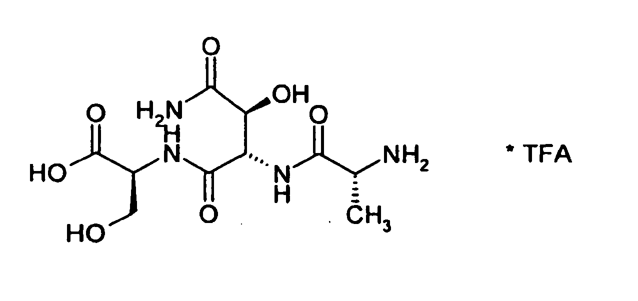

Die Verbindung aus Beispiel 259A (63 mg, 155 µmol) wird nach Arbeitsvorschrift 2 mit 2.0 ml der Reagenzlösung über 30 min umgesetzt. Ohne weitere Aufreinigung erhält man 67 mg (quant.) der rohen Titelverbindung.
HPLC (Methode 6): Rₜ = 0.72 min.
LC-MS (Methode 19): Rₜ = 0.52 min, MS (ESIpos): m/z (%) = 307 (100) [M+H]⁺.
HR-TOF-MS (Methode 24): C₁₀H₁₉N₄O₇ ber. 307.1249, gef. 307.1245 [M+H]⁺.

### Beispiel 261A

### [(3R)-N²-(tert-Butoxycarbonyl)-3-hydroxy-L-leucyl]-L-leucyl-D-arginyl-L-isoleucyl-L-allothreonyl-D-alanyl-[(3S)-3-hydroxy-L-asparaginyl]-L-serinat-trifluoracetat

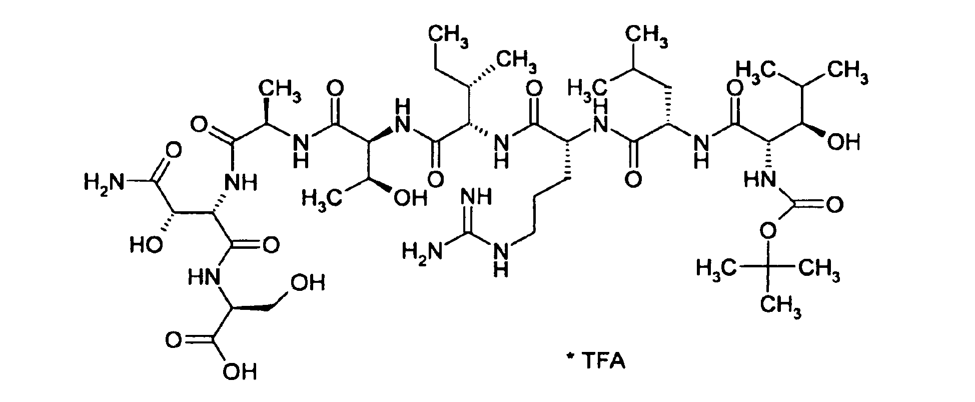

Die Verbindung aus Beispiel 254A (32 mg, 32 µmol) und die Verbindung aus Beispiel 260A, 13 mg, 32 µmol, 1 Äquivalent) werden bei 0 °C in DMF (1.5 ml) gelöst, DIPEA (28 µl, 158 µmol, 5 Äquivalente) werden hinzugefügt. Der Ansatz wird ca 16 h bei Raumtemperatur gerührt, dann wird er nach Methode 45 chromatographiert. Ausbeute: 30 mg (84% d. Th.).
HPLC (Methode 6): Rₜ = 3.40 min.
LC-MS (Methode 19): Rₜ = 1.60 min, MS (ESIpos): m/z (%) = 460.3 (100) [M-Boc+2H]²⁺, 1019.6 (60) [M+H]⁺.
HR-TOF-MS (Methode 24): C₄₃H₇₉N₁₂O₁₆ ber. 1019.5732, gef. 1019.5716 [M+H]⁺.

### Beispiel 262A

### [(3R)-3-Hydroxy-L-leucyl]-L-leucyl-D-arginyl-L-isoleucyl-L-allothreonyl-D-alanyl-[(3S)-3-hydroxy-L-asparaginyl]-L-serinat-bis-trifluoracetat

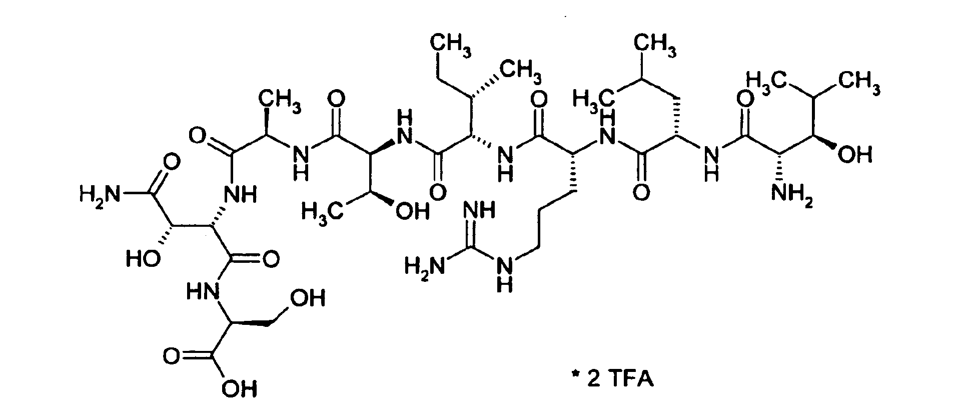

Die Verbindung aus Beispiel 261A (59 mg, 52 µmol) wird nach Arbeitsvorschrift 2 mit 2.0 ml der Reagenzlösung über 30 min umgesetzt. Ohne weitere Aufreinigung erhält man 64 mg (quant.) der rohen Titelverbindung.
HPLC (Methode 6): Rₜ = 2.91 min.
LC-MS (Methode 22): Rₜ = 2.20 min, MS (ESIpos): m/z (%) = 461 (100) [M+2H]²⁺; MS (ESIneg): m/z (%) = 918 (100) [M-H]⁻.
HR-TOF-MS (Methode 24): C₃₈H₇₁N₁₂O₁₄ ber. 919.5208, gef. 919.5212 [M+H]⁺.

### Beispiel 263A

### [(3R)-N²-(Benzyloxycarbonyl)-3-{(tert-butoxycarbonyl)amino}-L-phenylalanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin-trifluoracetat

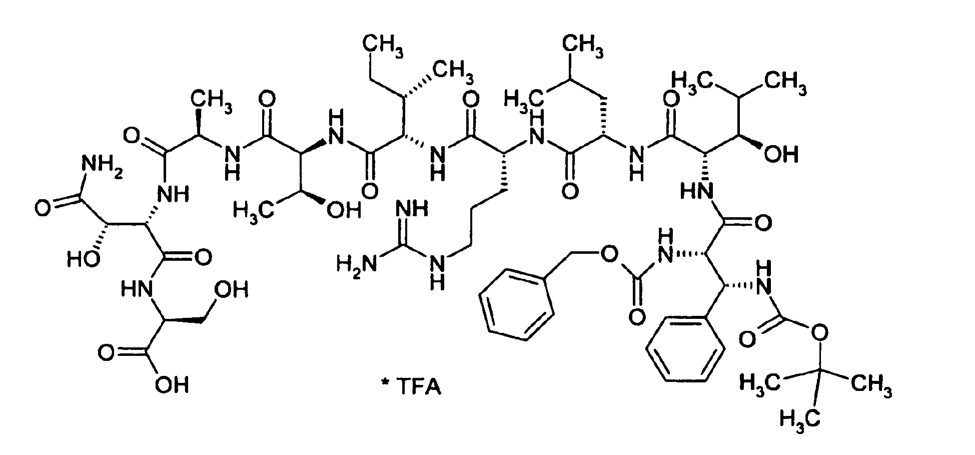

Gemäß der Vorschrift zur Darstellung der Verbindung aus Beispiel 201A wird die Verbindung aus Beispiel 262A (92 mg, 80 µmol) mit der Verbindung aus Beispiel 17A (51 mg, 88 µmol, 1.1 Äquivalente) umgesetzt. Nach chromatographischer Reinigung (Methode 44) werden 35 mg (31% d. Th.) der Titelverbindung isoliert.
HPLC (Methode 6): Rₜ = 3.76 min.
LC-MS (Methode 19): Rₜ = 1.94 min, MS (ESIpos): m/z (%) = 608.4 (100) [M-Boc+2H]²⁺, 1315.6 (30) [M+H]⁺; MS (ESIneg): m/z (%) = 1313.7 (100) [M-H]⁻.
HR-TOF-MS (Methode 24): C₆₀H₉₅N₁₄O₁₉ ber. 1315.6893, gef. 1315.6873 [M+H]⁺.

### Beispiel 264A

### [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin-bis-trifluoracetat

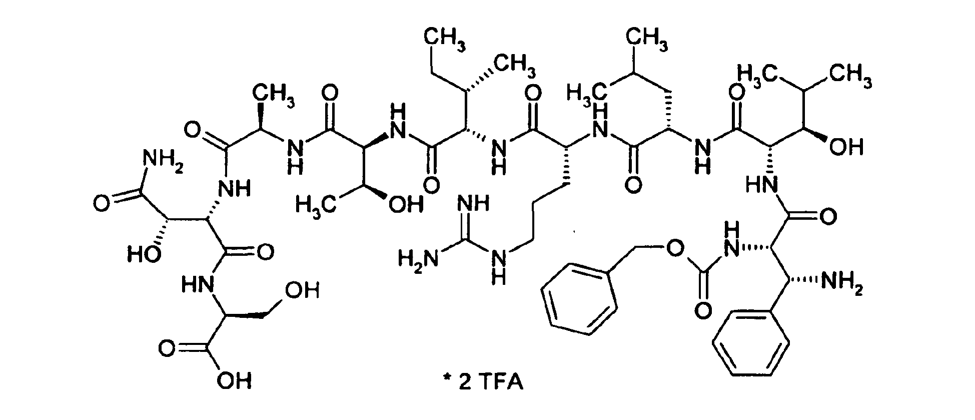

Die Verbindung aus Beispiel 262A (35 mg, 24 µmol) wird nach Arbeitsvorschrift 2 mit 2.0 ml der Reagenzlösung über 30 min umgesetzt. Ohne weitere Aufreinigung erhält man 37 mg (quant.) der rohen Titelverbindung.
HPLC (Methode 6): Rₜ = 3.20 min.
LC-MS (Methode 19): Rₜ = 1.36 min, MS (ESIpos): m/z (%) = 608.3 (100) [M+2H]²⁺, 1215.7 (5) [M+H]⁺; MS (ESIneg): m/z (%) = 1213.7 (100) [M-H]⁻.
HR-TOF-MS (Methode 24): C₅₅H₈₇N₁₄O₁₇ ber. 1215.6369, gef. 1215.6340 [M+H]⁺.

### Beispiel 265A

### [(3R)-N²-(Benzyloxycarbonyl)-3-amino-L-phenylatanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin-C^{1.9}-N^{3.1}-lactam-trifluoracetat

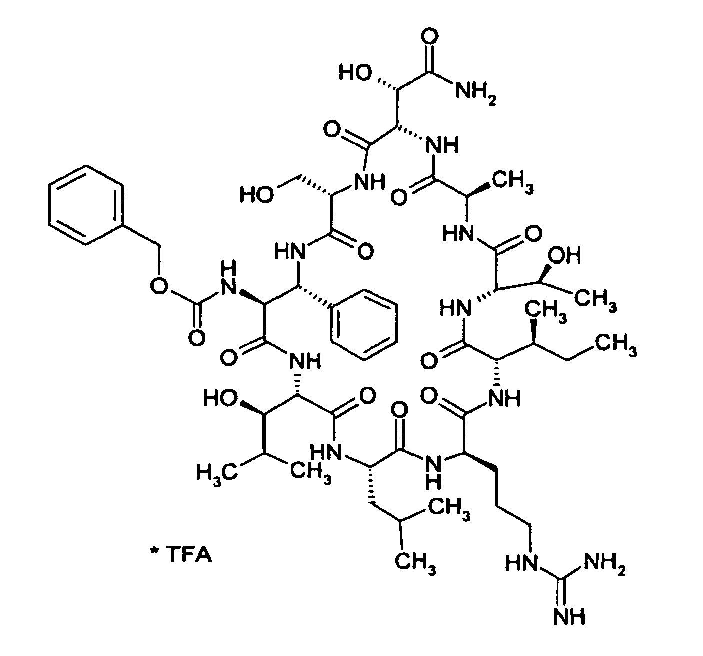

Gemäß der Vorschrift zur Darstellung der Verbindung aus Beispiel 217A, wobei die Aufreinigung nach Methode 45 erfolgt, wird aus der Verbindung aus Beispiel 264A (36 mg, 25 µmol) die Titelverbindung in einer Ausbeute von 46 mg (48% d. Th. (ca 34 % rein)) erhalten.
HPLC (Methode 6): Rₜ = 3.55 min.
LC-MS (Methode 19): Rₜ = 1.61 min, MS (ESIpos): m/z (%) = 599.3 (100) [M+2H]²⁺; 1197.6 (30) [M+H]⁺; MS (ESIneg): m/z (%) = 1195.6 (100), [M-H]⁻.
HR-TOF-MS (Methode 24): C₅₅H₈₅N₁₄O₁₆ ber. 1197.6263, gef. 1197.6290 [M+H]⁺.

### Beispiel 266A

### [(3R)-3-Amino-L-phenylalanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin-C^{1.9}-N^{3.1}-lactam-trifluoracetat

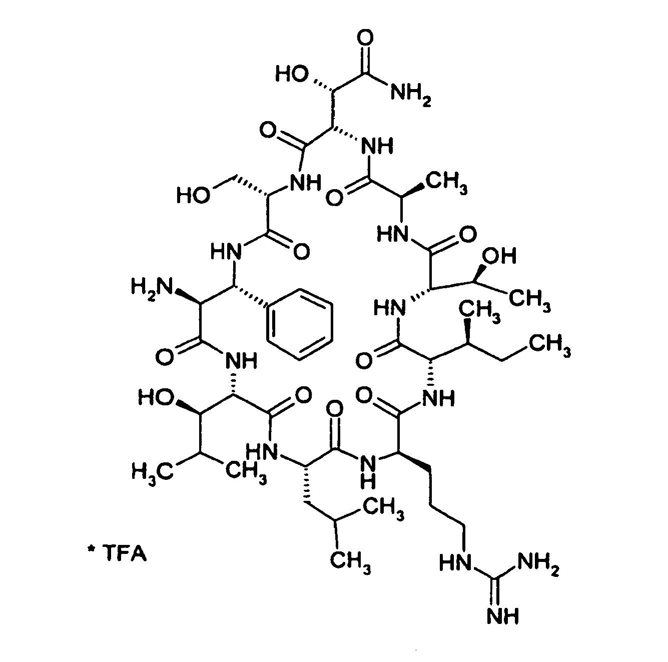

Die Verbindung aus Beispiel 265A (46 mg, Reinheit ca 34%, ca 12 µmol) wird in Methanol in Gegenwart von einer Spatelspitz 10%igem Palladium auf Kohle bei Normaldruck und Raumtemperatur über 1 h hydriert. Man filtriert vom Katalysator ab und engt ein. Das Rohprodukt (42 g) wird ohne Aufreinigung weiter umgesetzt.
HPLC (Methode 6): Rₜ = 2.98 min.
LC-MS (Methode 22): Rₜ = 2.33 min, MS (ESIpos): m/z (%) = 533 (100) [M+2H]²⁺, 1064 (10) [M+H]⁺; MS (ESIneg) m/z (%) = 1062 (100) [M]⁻.
HR-TOF-MS (Methode 24): C₄₇H₇₉N₁₄O₁₄ ber. 1063.5895, gef. 1063.5869 [M+H]⁺.

### Beispiel 267A

### [N²-(tert.-Butoxycarbonyl)-3-tert-butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin- C^{1.11}-N^{3.3}-lactam-trifluoracetat

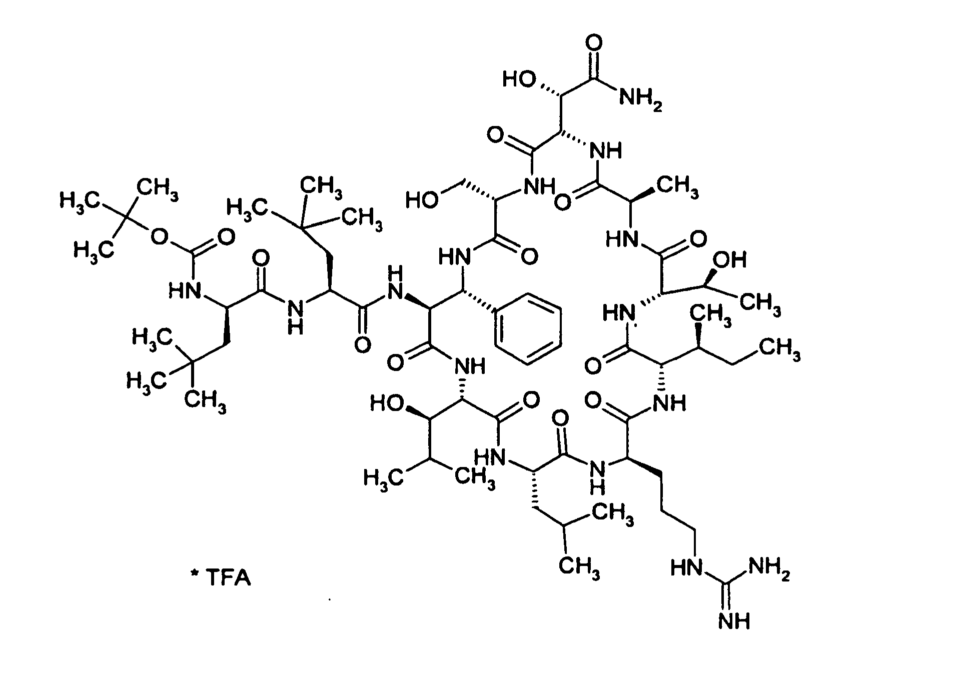

Gemäß der Vorschrift zur Darstellung der Verbindung aus Beispiel 233A wird aus der Verbindung aus Beispiel 266A (34 mg Rohprodukt, ca 12 µmol) und dem Dipeptid aus Beispiel 8A (13 mg, 35 µmol, ca 2.4 Äquivalente) die Titelverbindung in einer Rohausbeute von 28 mg erhalten. Das Produkt wird ohne weitere Reinigung im nächsten Syntheseschritt eingesetzt.
HPLC (Methode 6): Rₜ = 4.27 min.
LC-MS (Methode 19): Rₜ = 2.12 min, MS (ESIpos): m/z (%) = 659.6 (100) [M-Boc+2H]²⁺ 1417.8 (50) [M+H]⁺.

### Beispiel 268A

### rac N-(tert-Butoxycarbonyl)-3-(trimethylsilyl)alanin

Die Synthese erfolgt nach M. Merget, K. Günther, M. Bernd, E. Günther, R. Tacke, J. Organomet. Chem. 2001 628, 183-194. Die Trennung der Enantiomere erfolgt durch präparative HPLC an chiraler Phase:
Gilson Abimed HPLC, UV-Detektor 212 nm, Säule: Daicel Chiralpak AD-H 5 µm; 250 x 20 mm; Fluss: 15 mL/min; Eluent A: iso-Hexan, Eluent B: 0.2% Essigsäure/1% Wasser/2-Propanol; isokratisch.

### Beispiel 269A

### (2S Verbindung bzw N-(tert-Butoxycarbonyl)-D-3-trimethylsilylalanin):

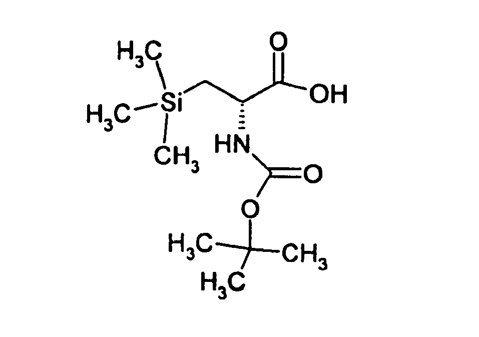

Präparative HPLC: Rₜ = 4.16 min
[α]_{D}²⁰ = +1.1 (c = 0.83, Methanol)

### Beispiel 270A

### (2R bzw N-(tert-Butoxycarbonyl)-L-3-trimethylsilylalanin):

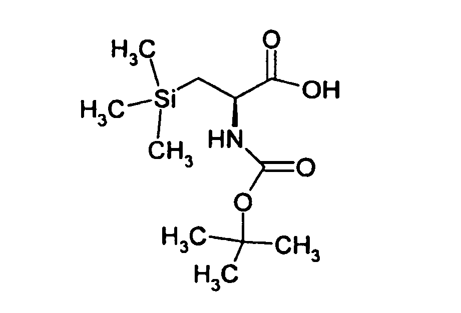

Präparative HPLC: Rₜ = 9.27 min
[α]_{D}²⁰ = -1.6 (c = 0.66, Methanol)

### Beispiel 271A

### Methyl N-(tert-Butoxycarbonyl)-3-pyridin-3-yl-L-alaninat

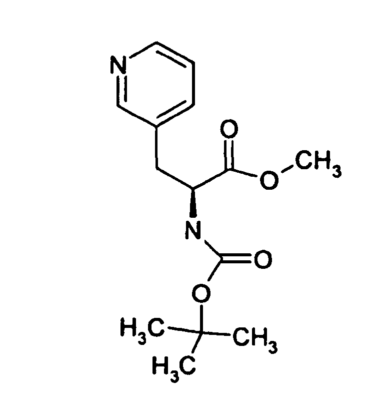

Analog B. Neises, W. Steglich, Org. Synth. 1985, 63, 183-187.

(2*S*)-*N*-(*tert-*Butoxycarbonyl)-3-(3-pyridyl)alanin (25.00 g, 93.88 mmol) wird unter Argon in 300 mL Dichlormethan gelöst. Methanol (11.4 mL, 9,02 g, 281 mmol, 3 Äquivalente) und ein Körnchen DMAP werden hinzugefügt. Dann wird die Mischung auf 0 °C gekühlt. EDC (19.80 g, 103 mmol, 1.1 Äquivalente) wird zugesetzt. Nach 5 min. entfernt man das Eisbad und lässt über 1 h bei Raumtemperatur rühren. Dann engt man im Vakuum ein, versetzt den Rückstand mit Ethylacetat und schüttelt gegen conc. Natriumhydrogencarbonat aus. Die wässrige Phase wird einmal mit Ethylacetat nachextrahiert, dann werden die vereinigten organischen Phasen mit 0,5 M Citronensäure und anschließend nochmals mit conc. Natriumhydrogencarbonat gewaschen. Die organische Phase wird über Natriumsulfat getrocknet, filtriert und im Vakuum eingeengt. Es verbleibt ein klares Öl, das beim Trocknen im Ölpumpenvakuum kristallisiert. Ausbeute: 23,60 g (84.2 mmol, 90 % d. Th.).
HPLC/UV-Vis (Methode 5): Rₜ = 3.28 min.
¹H NMR (400 MHz, *d*₆-DMSO) δ 1.30 (s, 9 H), 2.86 (m, 1 H), 3.04 (m, 1 H), 3.63 (s, 3 H), 4.22 (m, 1 H), 7.28 - 7.39 (m, 2 H), 7.69 (d, 1 H), 8.43 (m, 2 H).
LC-MS (Methode 18): Rₜ 1.21 min, MS (ESIpos.): *m*/*z* (%) = 281 (100) [M + H]⁺.

### Beispiel 272A

### 3-[(2S)-2-Ammonio-3-methoxy-3-oxopropyl]pyridinium bis(trifluoracetat)

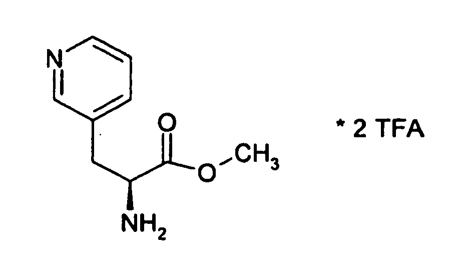

Verbindung 271A (11.8 g, 42.09 mmol) wird in 30 % TFA in Dichlormethan (160 mL) gelöst und 30 min. bei Raumtemperatur gerührt. Dann engt man im Vakuum ein. Der Rückstand wird in etwas Wasser aufgenommen und lyophilisiert. Dann wird das Lyophilisat mit Toluol versetzt und im Vakuum eingeengt. Schließlich wird bis zur Gewichtskonstanz im Ölpumpenvakuum getrocknet. Ausbeute: 17.15 g (quant.).
HPLC/UV-Vis (Methode 5): Rₜ = 0.88 min.
¹H NMR (400 MHz, *d*₆-DMSO) δ 2.79 (dd, 1 H), 2.92 (dd, 1 H), 3.60 (s, 3 H), 3.63 (m, 1 H), 7.30 (m, 1 H), 7.62 (d, 1 H), 8.41 (m, 2 H).
LC-MS (Methode 18): Rₜ 0.46 min, MS (ESIpos.): *m*/*z* (%) = 181 (100) [M + H]⁺.

### Beispiel 273A

### Methyl N-(tert-Butoxycarbonyl)-3-(trimethylsilyl)-D-alanyl-3-pyridin-3-yl-L-alaninat

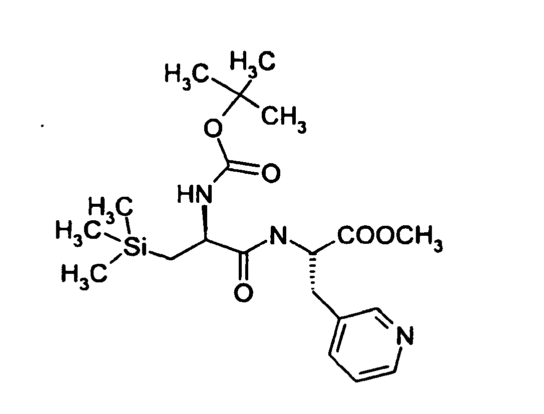

Verbindung 269A (10.31 g, 39.4 mmol) und Verbindung 272A (16.10 g, 39.4 mmol, 1 Äquivalent) werden bei 0 °C in DMF (186 mL) gelöst. Dann werden NMM (17,34 mL, 16.00 g, 4 Äquivalente) und HATU (22.49 g, 59.16 mmol, 1.5 Äquivalente) zugesetzt. Der Ansatz wird zwei Stunden bei Raumtemperatur gerührt. Man versetzt mit *tert-*Butylmethylether und wäscht mit conc. Natriumcarbonat. Die wässrige Phase wird einmal mit *tert-*Butylmethylether nachextrahiert, dann werden die vereinigten organischen Phasen mit 1 M wässriger Citronensäure sowie abermals mit conc. Natriumcarbonat. gewaschen, über Na₂SO₄ getrocknet, filtriert und im Vakuum eingeengt. Es wird über Kieselgel filtriert (Cyclohexan / Ethylacetat 2+1). Ausbeute: 14.1 g (84 % d. Th.).
HPLC/UV-Vis (Methode 5): Rₜ = 3.91 min.
¹H NMR (400 MHz, *d*₆-DMSO) δ -0.09 (s, 9 H), 0.56 - 0.75 (m, 2 H), 1.47 (s, 9 H), 2.90 (dd, 1 H), 3.09 (dd, 1 H), 3.62 (s, 3 H), 3.98 (m, 1 H), 4.49 (m, 1 H), 3.68 (d, 1 H), 7.26 (dd, 1 H), 7.61 (m, 1 H), 8.20 (d, 1 H), 8.40 (m, 2 H).
LC-MS (Methode 18): Rₜ 1.90 min, MS (ESIpos.): *m*/*z* (%) = 424 (50) [M + H]⁺.

### Beispiel 274A

### N-(tert-Butoxycarbonyl)-3-(trimethylsilyl)-D-alanyl-3-pyridin-3-yl-L-alanin

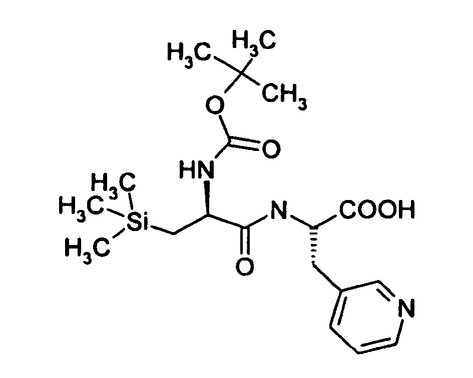

Verbindung 273A (7.4 g, 17.56 mmol) wird in THF / Wasser (6+4) aufgenommen, auf 0 °C gekühlt und mit Lithiumhydroxid-Monohydrat (1.47 g, 35.13 mmol, 2 Äquivalente) versetzt. Man lässt bei 0 °C rühren. Nach einer Stunde wird ein weiteres Äquivalent (0.74 g) Lithiumhydroxid-Monohydrat zugesetzt und eine weitere Stunde gerührt. Man entfernt den Großteil des THF im Vakuum und stellt dann durch Zusatz von Citronensäure auf pH 4. Ein Feststoff fällt aus. Es wird mit drei Portionen Ethylacetat extrahiert, wobei sich der Feststoff löst. Die vereinigten organischen Phasen werden über Natriumsulfat getrocknet, filtriert und eingeengt. Das Rohprodukt wird durch Chromatographie an Sephadex LH 20 (Methode 45) gereinigt. Ausbeute: 6.67 g (93 % d. Th.).
HPLC/UV-Vis (Methode 5): Rₜ = 3.73 min.
¹H NMR (300 MHz, *d*₆-DMSO) δ -0.09 (s, 9 H), 0.56 - 0.75 (m, 2 H), 1.35 (s, 9 H), 2.90 (dd, 1 H), 3.09 (dd, 1 H), 3.98 (m, 1 H), 4.41 (m, 1 H), 6.70 (d, 1 H), 7.26 (dd, 1 H), 7.60 (m, 1 H), 8.00 (d, 1 H), 8.37 (m, 2 H).
LC-MS (Methode 18): Rₜ 1.71 min, MS (ESIpos.): *m*/*z* (%) = 410 (100) [M + H]⁺.

### Beispiel 275A

### (3R)-3-[(tert-Butoxycarbonyl)amino]-L-phenylalanin

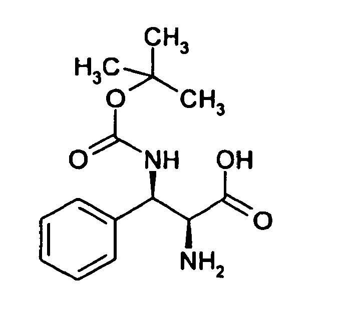

Beispielverbindung 16A (3.00 g, 7.24 mmol) wird in Methanol (60 mL) gelöst, 10% Palladium auf Aktivkohle (50 mg) werden dazugegeben und der Ansatz wird über 6 h bei Raumtemperatur und Normaldruck unter einer Wasserstoffatmosphäre gerührt. Dann filtriert man vom Katalysator ab und engt ein. Das rohe Reaktionsprodukt (2.20 g, -quant.) wird ohne Aufreinigung im nächsten Schritt eingesetzt.
HPLC (Methode 6): Rₜ = 3.21 min.
LC-MS (Methode 51): Rₜ = 1.55 min, MS (ESIpos): *m*/*z* (%) = 281.3 (30) [M+H]⁺, MS (ESIneg): *m*/*z* (%) = 279.1 (100) [M-H]⁻.
[α]²⁰_{Na} = -23.4 (*c* = 1.0, MeOH).
¹H-NMR (400 MHz, *d*₆-DMSO) δ (ppm) = 1.34 (s, 9H), 3.52 (d, *J* = 4.6 Hz, 1H), 4.88 (dd, *J* = 4.6, *J* = 8.4 Hz, 1H), 7.29 (m, 5H), 8.02 (d, *J* = 8.6 Hz, 1H).
HR-TOF-MS: C₁₄H₂₁N₂O₄ ber. 281.1496, gef. 281.1503 [M+H]⁺.

### Beispiel 276A

### (βR)-β-[(tert-Butoxycarbonyl)amino]-N²-[(9H-fluoren-9-ylmethoxy)carbonyl]-L-phenylalanin

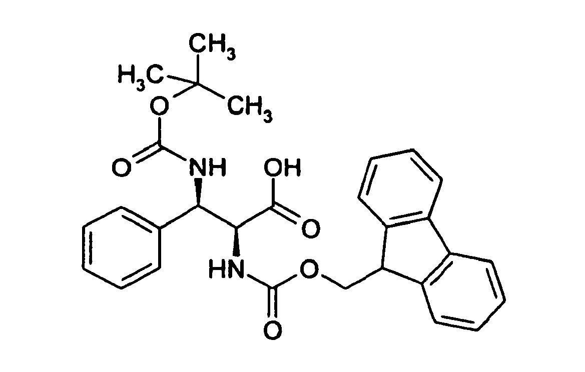

Beispielverbindung 275A (2.2 g Rohprodukt, 7.24 mmol) und (9H-Fluoren-9-ylmethoxy)carbonylsuccinimid (2.90 g, 8.58 mmol, 1.2 Äquivalente) sowie Natriumcarbonat (1.18 g, 11.70 mmol, 1.5 Äquivalente) werden in 1,4-Dioxan-Wasser (7+3) gelöst und über Nacht bei Raumtemperatur gerührt. Dann wird durch Zugabe von 5% wässriger Citronensäure abgestoppt und mit zwei Portionen Ethylacetat extrahiert. Die vereinigten organischen Extrakte werden über Natriumsulfat getrocknet, eingeengt und der Rückstand mit Wasser und Acetonitril angerieben. Das kristalline Rohprodukt wird dann chromatographisch nach Methode 34 gereinigt. Ausbeute: 2.58 g (66% d. Th.) als farbloser Feststoff.
HPLC (Methode 6): Rₜ = 4.86 min.
LC-MS (Methode 20): Rₜ = 2.51 min, MS (ESIpos): *m*/*z* (%) = 503.2 (70) [M+H]⁺, MS (ESIneg): *m*/*z* (%) = 501.3 (100) [M-H]⁻.
¹H NMR (400 MHz, *d*₆-DMSO) δ (ppm) = 1.38 (s, 9H), 4.75 (dd, *J* **=** 3.9, *J* = 9.8 Hz, 1H), 5.29 (dd, *J* = 3.9, *J* = 10.0 Hz 1H), 7.21-7.60 (m, 13H), 7.87 (d, *J* = 7.4 Hz, 1H).
HR-TOF-MS: C₂₉H₃₁N₂O₆ ber. 503.2177, gef. 503.2158 [M+H]⁺.

### Beispiel 277A

### Pentafluorphenyl (3R)-3-[(tert-Butoxycarbonyl)amino]-N²-[(9H-fluoren-9-ylmethoxy)-carbonyl]-L-phenylalaninat

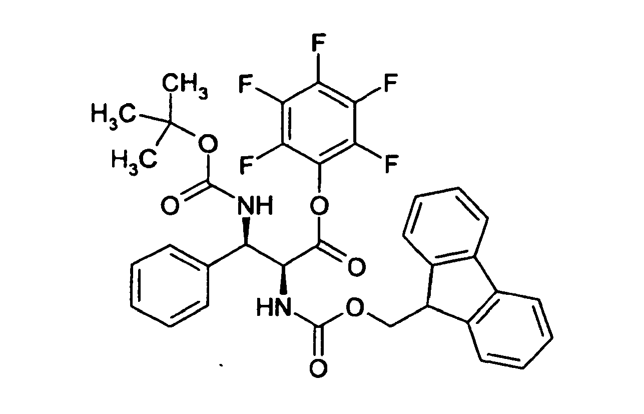

Beispielverbindung 276A (2.58 g, 5.14 mmol) und Pentafluorphenol (4.72 g, 25.68 mmol, 5 Äquivalente) werden in Dichlormethan (350 mL) gelöst und auf 0 °C gekühlt. EDCI (1.48 g, 7.70 mmol, 1.5 Äquivalente) wird zugegeben und der Ansatz 6 h unter langsamer Erwärmung auf Raumtemperatur gerührt. Dann wird das Lösungsmittel abdestilliert und der Rückstand chromatographisch in mehreren Portionen nach Methode 34 gereinigt. Ausbeute: 2.80 g (81% d. Th.) der Titelverbindung als farbloser Feststoff.
HPLC (Methode 54) Rₜ = 5.09 min.
LC-MS (Methode 19): Rₜ = 3.34 min, MS (ESIpos): *m*/*z* (%) = 6693.0 (20) [M+H]⁺.
¹H NMR (400 MHz, *d*₆-DMSO) δ (ppm) = 1.39 (s, 9H), 4.11-4.26 (m, 3H), 5.16 (dd, *J* = 4.4, *J* = 9.5 Hz, 1H), 5.52 (dd, *J* = 4.4, *J* = 10.0 Hz, 1H), 7.27-7.57 (m, 9H), 7.56 (t, *J* = 6.6 Hz, 2H), 7.73 (d, *J* = 10.3 Hz, 1H), 7.87 (d, *J* = 7.4 Hz, 2H), 7.93 (d, *J* = 9.6 Hz, 1H).
HR-TOF-MS: C₃₅H₃₀N₂O₆ ber. 669.2019, gef. 669.1996 [M+H]⁺.

### Beispiel 278A

### Ethyl (3R)-3-[(tert-Butoxycarbonyl)aminol-N²-(diphenylmethylen)-L-leucinat

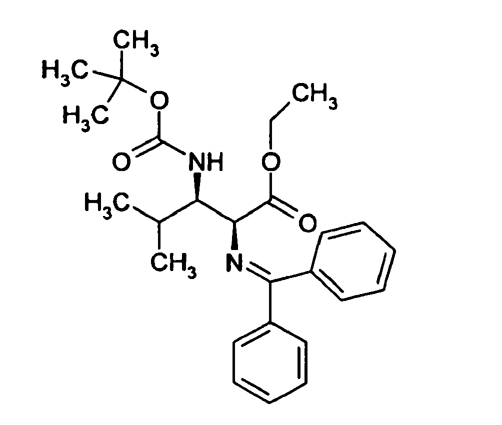

Die Mannich-Reaktion erfolgt in Anlehnung an eine Methode zur Addition von Kupferenolaten an Sulfonylimine: L. Bernardi, A. Gothelf, R. G. Hazell, K. A. Jørgensen, J. Org. Chem. 2003, 68, 2583-2591. *tert*-Butyl [(1E)-phenylmethylen]carbamat kann nach der Literatur hergestellt werden: A. Klepacz, A. Zwierzak, Tetrahedron Lett. 2002 43; 1079 - 1080.

Frisch aktiviertes Molekularsieb 3 Å, Tetrakis(acetonitril)kupfer(I) hexafluorphosphat (293 mg, 0.79 mmol, 0.035 Äquivalente) und (*R*)-(+)-2-(2-(Diphenylphosphino)phenyl)-4-isopropyl-2-oxazolin (335 mg, 0.90 mmol, 0.04 Äquivalente) werden unter Argon vorgelegt und mit abs. THF (228 mL) versetzt. Dann wird Triethylamin (109 µL, 80 mg, 0.79 mmol, 0.035 Äquivalente) dazupipettiert Die Mischung wird auf -20 °C gekühlt, und, sobald diese Temperatur erreicht ist, werden Ethyl *N*-(diphenylmethylen)glycinat (6.00 g, 22.44 mmol) und *tert-*Butyl [(1E)-phenylmethylen]carbamat (6.92 g, 40.4 mmol, 1.8 Äquivalente) dazugegeben. Der Ansatz wird über 16 h gerührt, wobei man ihn langsam auf Raumtemperatur kommen lässt. Danach wird Kieselgel (ca. 15 g) dazugegeben und das Lösemittel im Vakuum abdestilliert. Der Rückstand wird auf eine mit Kieselgel gepackte Glassäule geladen und mit Cyclohexan-Ethylacetat 9+1 eluiert. Die Titelverbindung (R_{f} = 0.22, CyHex-EtOAc 9+1) enthält das D-threo konfigurierte Enantiomere und eine geringere Menge (ca. 36%) der beiden erythro-konfigurierten Enantiomeren, die auf einer späteren Stufe abgetrennt werden. Das Produkt ist sehr empfindlich gegenüber Säuren, Untersuchungen an HPLC (Methode 6) und LC-MS (Methode 19) zeigen immer einen Anteil Benzophenon, das unter den Chromatographiebedingungen abgespaltenen wird. Ausbeute: 2.66 g (27% d. Th.).
LC-MS (Methode 19): Rₜ = 3.23 min (21%, erythro), MS (ESIpos): *m*/*z* (%) = 439.4 (100) [M+H]⁺ und 3.23 min (48%, threo), MS (ESIpos): *m*/*z* (%) = 439.3 (100) [M+H]⁺.
¹H NMR (400 MHz, *d*₆-DMSO) δ (ppm) = 0.62 (d, *J* = 6.6 Hz, 3H), 0.83 (d, *J* = 6.6 Hz, 3H), 1.13 (t, *J* = 7.1 Hz, 3H), 1.38 (s, 9H), 1.61 (m, 1H), 3.74 (ddd, *J₁* = 2.7, *J₂* = *J₃* = 9.6 Hz, 1H), 3.96 (d, *J* = 2.7 Hz, 1H), 3.99-4.06 (m, 2H), 6.51 (d, *J* = 10.0 Hz, 1H), 7.07 (m, 2H), 7.38-7.52 (m, 6H), 7.67-7.77 (m, 2H).
HR-TOF-MS: C₂₆H₃₅N₂O₄ ber. 439.2592, gef. 439.2606 [M+H]⁺.

### Beispiel 279A

### Ethyl (3R)-3-[(tert-Butoxycarbonyl)amino]-L-leucinat-trifluoracetat

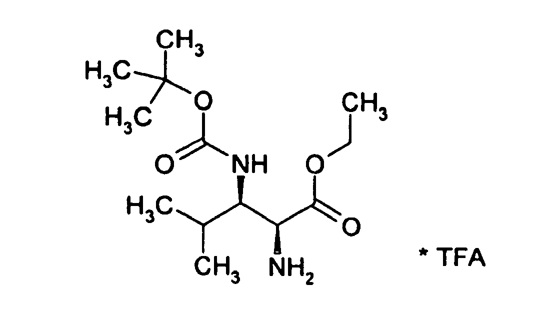

Die Titelverbindung aus Beispiel 278A (4.44 g, 10.12 mmol) wird in Dichlormethan (87 mL) gelöst, 857 µL TFA (11.13 mmol, 1.1 Äquivalente) und Wasser (273 µL, 15.18 µmol, 1.5 Äquivalente) werden zugegeben und der Ansatz 1 h bei Raumtemperatur gerührt. Alle flüchtigen Bestandteile des Reaktionsgemischs werden im Vakuum abdestilliert, der Rückstand (5.97 g) wird ohne Aufreinigung im nächsten Schritt eingesetzt.
HPLC (Methode 6): Rₜ = 3.41 min.
LC-MS (Methode 19): Rₜ = 1.31 min, MS (ESIpos): *m*/*z* (%) = 275.2 (50) [M+H]⁺.

### Beispiel 280A

### Ethyl (3R)-N²-(Benzyloxy)carbonyl-3-[(tert-butoxycarbonyl)amino]-L-leucinat

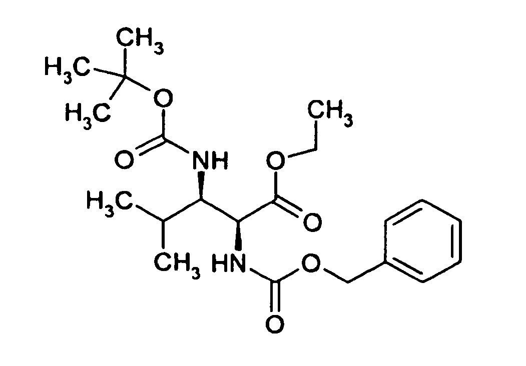

Beispielverbindung 279A (5.97 g Rohprodukt, ca. 10.11 mmol) und Benzyloxycarbonylsuccinmid (3.02 g, 12.13 mmol, 1.2 Äquivalente) werden in Dichlormethan-Wasser (4+1) gelöst, der Ansatz wird auf 0 °C gekühlt. Unter kräftigem Rühren werden Natriumhydrogencarbonat (2.55 g, 30.33 mmol, 3 Äquivalente) und Tetrabutylammoniumiodid (373 mg, 1.01 mmol, 0.1 Äquivalent) dazugegeben und der Ansatz über 16 h bei Raumtemperatur kräftig gerührt. Die organische Phase wird abgetrennt, mit conc. NaCl gewaschen, über Natriumsulfat getrocknet und eingeengt. Das Rohprodukt wird in mehreren Portionen nach Methode 34 isoliert, wobei das Benzophenon und ein Teil des erythro-Diastereomers abgetrennt werden. Insgesamt erhält man 1.65 g (4.03 mmol, 40% d. Th.) der Titelverbindung.
R_{f} = 0.076 (CyHex-EtOAc 9+1).
HPLC (Methode 54): Rₜ = 4.33 min (erythro-Diastereomer) und 4.52 min (threo-Diastereomer).
LC-MS (Methode 19): Rₜ = 2.71 min, MS (ESIpos): *m*/*z* (%) = 409.3 (30) [M+H]⁺, erythro-Isomer und Rₜ = 2.75 min, MS (ESIpos): *m*/*z* (%) = 409.0 (70) [M+H]⁺ threo-Isomer).
¹H NMR (400 MHz, *d*₆-DMSO) δ (ppm) = 0.71-0.86 (m, 6H), 1.16 (t, *J* = 7.1 Hz, 3H), 1.34 (s, 9H), 1.59 (m, 1H), 3.70 (ddd, *J* = 3.4, *J*¹ = *J*² = 10.3 Hz, 1H), 3.99-4.08 (m, 2H), 4.32 (dd, *J* = 3.4, *J* = 9.5 Hz, 1H), 5.03 (d, *J* = 12.4 Hz, 1H), 5.10 (d, *J* = 12.4 Hz, 1H), 6.62 (d, *J* = 10.5 Hz, 1H), 7.31-7.40 (m, 5H), 7.44 (d, *J* = 9.3 Hz, 1H).
HR-TOF-MS: C₂₁H₃₃N₂O₆ ber. 409.2334, gef. 409.2329 [M+H]⁺.

### Beispiel 281A

### (3R)-3-[(tert-Butoxycarbonyl)aminol-N²-(Benzyloxy)carbonyl-L-leucin

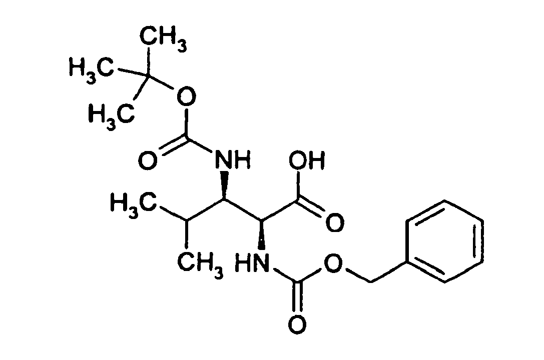

Die Titelverbindung aus Beispiel 280A (2.78 g, 6.82 mmol) wird in einem Gemisch aus THF-Wasser (1+1, 40 mL) bei 0 °C gelöst, Lithiumhydroxid-Monohydrat (629 mg, 15.0 mmol, 2.2 Äquivalente) wird dazugegeben und 3 h bei 0 °C gerührt. Dann wird das THF abdestilliert, der wässrige Rückstand mit 1 M Citronensäure angesäuert und mit zwei Portionen Ethylacetat extrahiert. Die vereinigten organischen Extrakte werden über Natriumsulfat getrocknet und eingeengt. Der rohe Rückstand (2.42 g, 93% d. Th.) wird durch Chromatographie an einer chiralen Phase nach Methode 55 getrennt. Dabei werden die Enantiomeren des threo-Diastereomers und diejenigen des erythro-Minderdiastereomers jeweils einzeln erhalten. Der e.e. des Hauptisomers (Titelverbindung) nach der chiralen Chromatographie wird nach Methode 56 bestimmt, er beträgt 100%. Die Ausbeute der Titelverbindung beträgt 780 mg (30% d. Th. bezogen auf die Ausgangsverbindung 280A).
HPLC (Methode 6): Rₜ = 4.51 min.
LC-MS (Methode 19): Rₜ = 2.44 min, MS (ESIpos): *m*/*z* (%) = 381.1 (30) [M+H]⁺, MS (ESIneg): *m*/*z* (%) = 379.2 (100) [M-H]⁻.
¹H NMR (400 MHz, *d*₆-DMSO) δ (ppm) = 0.82 (d, *J* = 9.3 Hz, 3H), 0.85 (d, *J* = 6.6 Hz, 3H), 1.35 (s, 9H), 3.72 (ddd, *J*¹ = 3.2, *J*² = *J*³ = 10.0 Hz, 1H), 4.26 (dd, *J* = 3.2, *J* = 9.6 Hz, 1H), 5.03 (d, *J* = 12.7 Hz, 1H), 5.08 (d, *J* = 12.4 Hz, 1H), 6.63 (d, *J* = 10.5 Hz, 1H), 7.31-7.40 (m, 6H), 12.62 (br s, 1H).
HR-TOF-MS: C₁₉H₂₈N₂O₆Na ber. 403.1845, gef. 403.1832 [M+Na]⁺.
Chirale HPLC (Methode 56): Rₜ = 8.97 min.

### Beispiel 282A

### Pentafluorphenyl (3R)-3-[(tert-Butoxycarbonyl)amino]-N²-(Benzyloxy)carbonylamino-L-leucinat

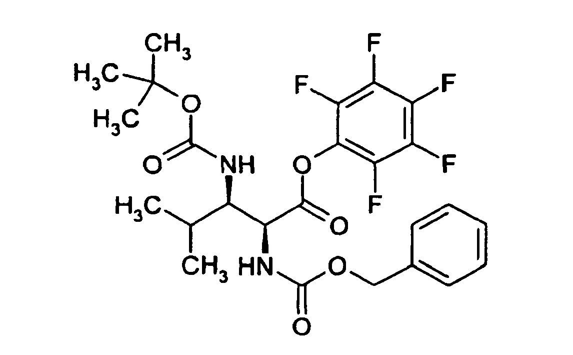

Beispielverbindung 281A (100 mg, 263 µmol) und Pentafluorphenol (242 mg, 1.31 mmol, 5 Äquivalente) werden in Dichlormethan (20 mL) bei Raumtemperatur gelöst, dann wird EDCI (76 mg, 394 µmol, 1.5 Äquivalente) hinzugefügt und der Ansatz ca. 12 h im Kühlschrank stehen gelassen. Anschließend wird das Lösungsmittel im Vakuum abdestilliert und der Rückstand chromatographisch gereinigt (Methode 32). Die Titelverbindung wird in einer Ausbeute von 132 mg (92% d. Th.) als farbloser Feststoff erhalten.
HPLC (Methode 54): Rₜ = 4.89 min.
LC-MS (Methode 19): Rₜ = 3.19 min, MS (ESIpos): *m*/*z* (%) = 547.2 (40) [M+H]⁺.

### Beispiel 283A

### Benzyl N²-(tert-Butoxycarbonyl)-3-{[(9H-fluoren-9-ylmethoxy)carbonyl]amino}-L-alanyl-L-serinat

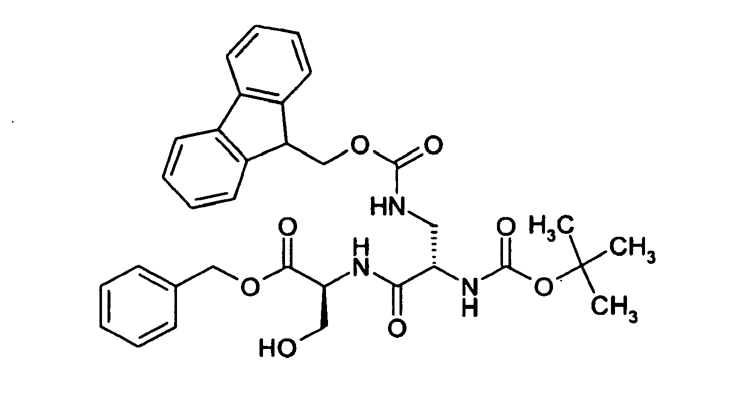

Verbindung Beispiel 149A (2.08 g, 6.73 mmol) und *N-*(*tert-*Butoxycarbonyl)-3-([(9H-fluoren-9-ylmethoxy)carbonyl]amino)-L-alanin (22.73 g, 6.39 mmol, 0.95 Äquivalente) werden in DMF (32 mL) gelöst und auf 0 °C gekühlt. HATU (3.84 g, 10.09 mmol, 1.5 Äquivalente) und 4-Methylmorpholin (2.22 mL, 20.18 mmol, 3 Äquivalente) werden zugegeben. Der Ansatz wird dann 2.5 h bei Raumtemperatur gerührt. Anschließend wird der gesamte Ansatz auf eine Sephadex LH20 Säule geladen und nach Methode 45 chromatographiert. Produkthaltige Fraktionen werden vereinigt und nach Methode 34 aufgereinigt. Ausbeute: 2.41 g (59 % d. Th.) als farbloser Feststoff.
HPLC (Methode 6): Rₜ = 4.81 min.
LC-MS (Methode 51): Rₜ = 3.94 min, MS (ESIpos): *m*/*z* (%) = 504.4 (100) [M-BOC+H⁺]⁺, 604.4 (40) [M+H]⁺.
[α]²⁰Na = +1.5 (c = 0.5, MeOH).
¹H NMR (400 MHz, *d*₆-DMSO) δ (ppm) = 1.36 (s, 9H), 3.18 (m, 1H), 3.66 (m, 1H), 3.75 (m, 1H), 4.15-4.28 (m, 4H), 4.42 (m, 1H), 5.90 (t, J = 5.6 Hz, 1H), 5.20 (s, 2H), 6.82 (d, *J* = 8.04 Hz, 1H), 7.21 (m, 1H), 7.31-7.43 (m, 10H), 7.67 (d, *J* = 7.8 Hz, 1H), 7.89 (d, *J* = 7.6 Hz, 1H), 8.14 (d, J = 7.6 Hz, 1H).
HR-TOF-MS: C₃₃H₃₈N₃O₈ ber. 604.2654, gef. 604.2646 [M+H]⁺.

### Beispiel 284A

### Benzyl 3-{[(9H-fluoren-9-ylmethoxy)carbonyl]amino}-L-alanyl-L-serinat-trifluoracetat

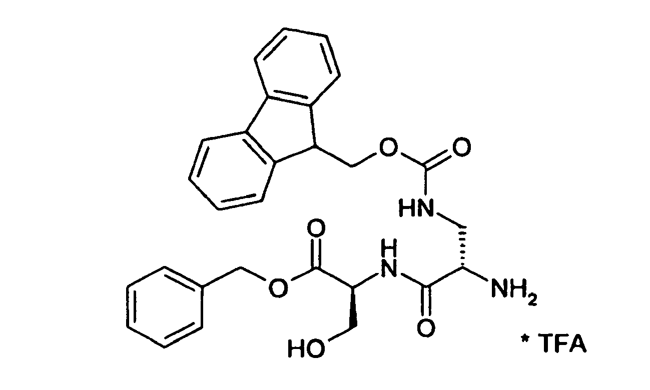

Beispielverbindung 283A (2.39 g, 3.96 mmol) wird nach Arbeitsvorschrift 2 umgesetzt. Man erhält 2.96 g (quant.) der rohen Titelverbindung, die ohne weitere Aufreinigung weiter umgesetzt wird.
HPLC (Methode 6): Rₜ = 3.84 min.
LC-MS (Methode 22): Rₜ = 3.14 min; MS (ESIpos) *m*/*z* (%) = 504.0 (100) [M+H]⁺.
[α]²⁰_{Na} = -0.5 (*c* = 1.0, MeOH).
HR-TOF-MS: C₂₈H₃₀N₃O₆ ber. 504.2130, gef. 504.2140 [M+H]⁺.

### Beispiel 285A

### Benzyl [N-(tert-Butoxycarbonyl)glycyl]-[3-{[(9H-fluoren-9-ylmethoxy)carbonyl]amino}-L-atanyl]-L-serinat

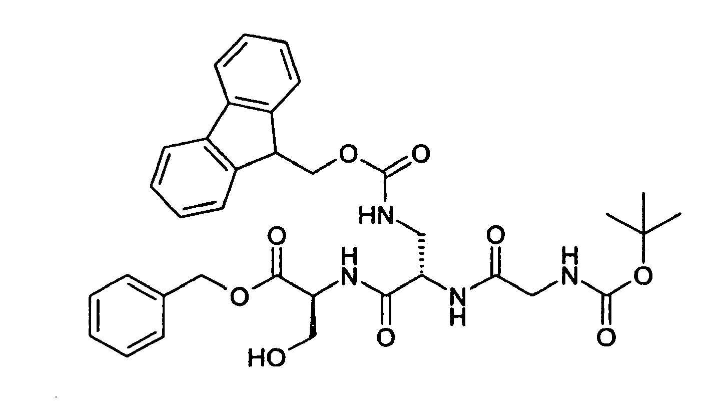

Beispielverbindung 284A (2.90 g, ∼83% rein, 3.89 mmol) und *N-tert-*Butoxycarbonyl-glycin (751 mg, 4.29 mmol, 1 Äquivalent) werden in DMF (20 mL) gelöst und auf 0 °C gekühlt. HATU (2.22 g, 5.85 mmol, 1.5 Äquivalente) und 4-Methylmorpholin (2.14 mL, 19.49 mmol, 5 Äquivalente) werden zugegeben. Die Reaktionsmischung wird 2.5 h bei 0 °C gerührt, dann wird durch Zugabe von 5% wässriger Citronensäurelösung abgestoppt und mit 5 Portionen MTBE extrahiert. Die vereinigten organischen Extrakte werden getrocknet (Natriumsulfat) und eingeengt. Das Rohprodukt wird chromatographisch gereinigt (Methode 34). Produkthaltige Fraktionen werden vereinigt und lyophilisiert. Ausbeute: 1.54 g (60 % d. Th.) als farbloser Feststoff.
HPLC (Methode 3) Rₜ = 4.64 min.
LC-MS (Methode 19): Rₜ = 2.65 min, MS (ESIpos): *m*/*z* (%) = 661.2 (100) [M+H]⁺.
[α]²⁰_{Na} = -8.7 (c = 0.5, MeOH).
HR-TOF-MS: C₃₅H₄₁N₄O₉ ber. 661.2869, gef. 661.2864 [M+H]⁺

### Beispiel 286A

### Benzyl Glycyl]-[3-{[(9H-fluoren-9-ylmethoxy)carbonyl]amino}-L-alanyl]-L-serinat-trifluoracetat

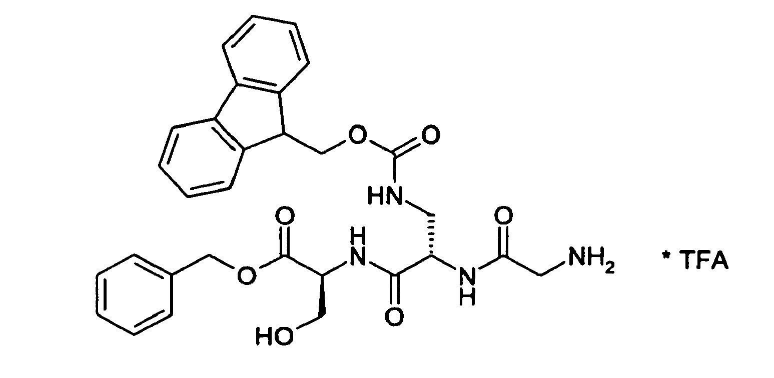

Die Titelverbindung aus Beispiel 285A (88 mg, 0.20 mmol) wird nach Arbeitsvorschrift 2 umgesetzt. Das Rohprodukt wird ohne weitere Reinigung weiter umgesetzt. Ausbeute: 1.84 g (quant.)
HPLC (Methode 3): Rₜ = 3.78 min.
LC-MS (Methode 19): Rₜ = 1.74 min, MS (ESIpos): *m*/*z* (%) = 561.2 (100) [M+H]⁺.
[α]²⁰_{Na} = 0.0 (*c* = 1.0, MeOH).
HR-TOF-MS: C₃₀H₃₃N₄O₇ber. 561.2344, gef. 561.2343 [M+H]⁺.

### Beispiel 287A

### Benzyl [(3R)-N²-(tert-Butoxycarbonyl)-3-hydroxy-L-leucyl]-L-leucyl-D-arginyl-L-isoleucyl-L-allothreonyl-glycyl-[3-{[(9H-fluoren-9-ylmethoxy)carbonyl]amino}-L-alanyl]-L-serinat-trifluoracetat

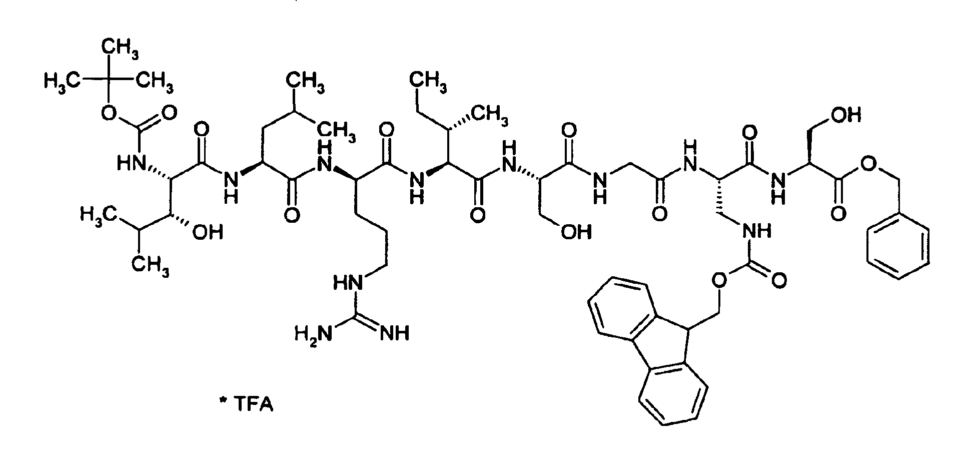

Die Beispielverbindung 32A (701 mg, 830 µmol) und 286A (700 mg, 830 µmol, 1 Äquivalent) werden in DMF (11.2 mL) gelöst und die Lösung auf 0°C gekühlt. 4-Methylmorpholin (274 µL, 2.49 mmol, 3 Äquivalente) und HATU (473 mg, 1.25 mmol, 1.5 Äquivalente) werden zugegeben und der Ansatz 3 h bei 0 °C gerührt. Dann wird der gesamte Ansatz auf eine Sephadex LH-20-Säule gegeben und nach Methode 45 chromatographiert. Produkthaltige Fraktionen werden vereinigt, eingeengt und nach Methode 34 aufgereinigt. Produkthaltige Fraktionen werden vereinigt und lyophilisiert. Man erhält 643 mg (56 % d. Th.) der Titelverbindung als farblosen Feststoff.
HPLC (Methode 6): Rₜ = 4.27 min.
LC-MS (Methode 19): Rₜ = 2.13 min, MS (ESIpos): *m*/*z* (%) = 1273.9 (30) [M+H]⁺, MS (ESIneg) *m*/*z* (%) = 1317.9 (100) [M+HCOO⁻]⁻.
HR-TOF-MS: C₆₃H₉₃N₁₂O₁₆ ber. 1273.6826, gef. 1273.6851 [M+H]⁺.

### Beispiel 288A

### Benzyl [(3R)-3-Hydroxy-L-leucyl]-L-leucyl-D-arginyl-L-isoleucyl-L-allothreonyl-glycyl-[3-{[(9H-fluoren-9-ylmethoxy)carbonyl]amino}-L-alanyl]-L-serinat-bis-trifluoracetat

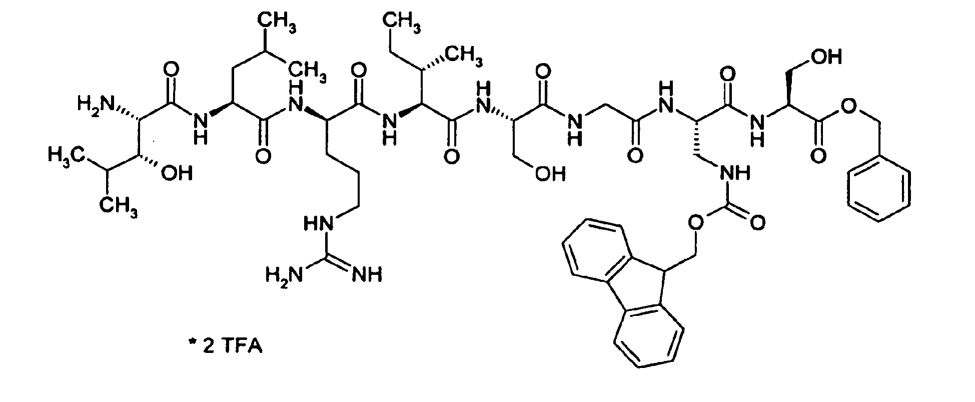

Verbindung 287A (643 mg, 463 µmol) wird nach Arbeitsvorschrift 2 umgesetzt. Die Titelverbindung wird in ungefähr quantitativer Ausbeute (783 mg) roh erhalten und ohne Reinigung weiter umgesetzt.
HPLC (Methode 5): Rₜ = 3.61 min.
LC-MS (Methode 51): Rₜ = 2.53 min, MS (ESIpos): *m*/*z* (%) = 588.1 (100) [M+2H]²⁺.
HR-TOF-MS: C₃₈H₈₅N₁₂O₁₄ ber. 1173.6306, gef. 1173.6304 [M+H]⁺.

### Polymergebundene Peptide

### Beispiel 289A

### Polymergebundenes N-(tert.-Butoxycarbonyl)-O-tert-butyl-L-threonyl-L-leucyl-N⁵-(imino{[(2,2,5,7,8-pentamethyl-3,4-dihydro-2H-chromen-6-yl)sulfonyl]amino}-methyl)-D-ornithyl-L-isoleucyl-O-tert-butyl-L-allothreonyl-glycyl-O-tert-butyl-L-seryl-O-tert-butyl-L-serin

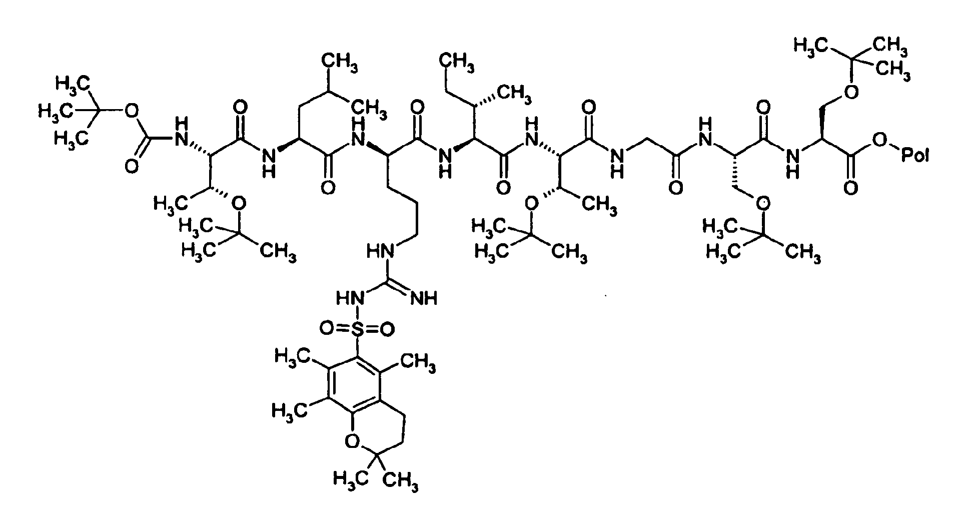

34.00g (50.66 mmol) 2-Chlortrityl-Harz (Iris Biotech, CAS No 42074-68-0, Beladung 1.49 mmol/g) werden in 400 mL Dichlormethan vorgelegt und mit 67.989 g (177.31 mmol) *O*-*tert*.-Butyl-*N*-[(9H-fluoren-9-ylmethoxy)carbonyl]-L-serin und 65.476 g (506.60 mmol) *N*-Ethyl-*N*-isopropylpropan-2-amin (DIEA) versetzt. Man schüttelt bei RT über Nacht. Man versetzt mit 5 mL Methanol und schüttelt 30 min bei RT. Man saugt ab, wäscht dreimal mit jeweils 400 mL Dimethylformamid, Methanol und Dichlormethan und saugt ab. Anschließend wird zweimal nacheinander wie folgt mit Piperidin behandelt: Man versetzt das Harz mit 400 mL 20%-iger Piperidin-Lösung in Dimethylformamid, schüttelt für 30 min und saugt ab.

Man legt das so erhaltene Harz in 400 mL Dimethylformamid vor und gibt 48.564 g (126.65 mmol) *O*-tert.-Butyl-*N*-[(9H-fluoren-9-ylmethoxy)carbonyl]-L-serin, 40.665 g (126.65 mmol) *N*-[(1H-Benzotriazol-1-yloxy)(dimethylamino)methylen]-*N-*methylmethanaminium tetrafluoroborat (TBTU) und 24.553 g (189.975 mmol) *N-*Ethyl-*N-*isopropylpropan-2-amin (DIEA) hinzu. Man schüttelt über Nacht bei RT. Man saugt ab, wäscht dreimal mit jeweils 400 mL Dimethylformamid, Methanol und Dichlormethan und saugt ab. Anschließend wird zweimal nacheinander wie folgt mit Piperidin behandelt: Man versetzt das Harz mit 400 mL 20%-iger Piperidin-Lösung in Dimethylformamid, schüttelt für 30 min und saugt ab.

Man legt das so erhaltene Harz in 400 mL Dimethylformamid vor und gibt 37.665 g (126.65 mmol) *N-*[(9H-Fluoren-9-ylmethoxy)carbonyl]glycin, 40.665 g (126.65 mmol) *N-*[(1H-Benzotriazol-1-yloxy)(dimethylamino)methylen]-*N-*methylmethanaminium tetrafluoroborat (TBTU) und 24.553 g (189.975 mmol) *N-*Ethyl-*N-*isopropylpropan-2-amin (DIEA) hinzu. Man schüttelt über Nacht bei RT. Man saugt ab, wäscht dreimal mit jeweils 400 mL Dimethylformamid, Methanol und Dichlormethan und saugt ab. Anschließend wird zweimal nacheinander wie folgt mit Piperidin behandelt: Man versetzt das Harz mit 400 mL 20%-iger Piperidin-Lösung in Dimethylformamid, schüttelt für 30 min und saugt ab.

Man legt das so erhaltene Harz in 400 mL Dimethylformamid vor und gibt 50.340 g (126.65 mmol) *O*-*tert*.-Butyl-*N*-[(9H-fluoren-9-ylmethoxy)carbonyl]-L-allothreonin, 40.665 g (126.65 mmol) *N*-[(1H-Benzotriazol-1-yloxy)(dimethylamino)methylen]-N-methylmethanaminium tetrafluoroborat (TBTU) und 24.553 g (189.975 mmol) *N-*Ethyl-*N-*isopropylpropan-2-amin (DIEA) hinzu. Man schüttelt über Nacht bei RT. Man saugt ab, wäscht dreimal mit jeweils 400 mL Dimethylformamid, Methanol und Dichlormethan und saugt ab. Anschließend wird zweimal nacheinander wie folgt mit Piperidin behandelt: Man versetzt das Harz mit 400 mL 20%-iger Piperidin-Lösung in Dimethylformamid, schüttelt für 30 min, saugt ab und trocknet im Vakuum.

Man legt 32 g (47.68 mmol; Annahme: 1.49 mmol/g Beladung) des so erhaltenen Harzes in 400 mL Dimethylformamid vor und gibt 42.128 g (119.20 mmol) *N-*[(9H-Fluoren-9-ylmethoxy)carbonyl]-L-isoleucin, 38.273 g (119.20 mmol) *N-*[(1H-Benzotriazol-1-yloxy)(dimethylamino)methylen]-*N-*methylmethanaminium tetrafluoroborat (TBTU) und 23.109 g (178.80 mmol) *N-*Ethyl-*N-*isopropylpropan-2-amin (DIEA) hinzu. Man schüttelt über Nacht bei RT. Man saugt ab, wäscht dreimal mit jeweils 400 mL Dimethylformamid, Methanol und Dichlormethan und saugt ab. Aufgrund unvollständiger Kupplung (laut HPLC) wird erneut mit 400 mL Dimethylformamid, 25.277 g (71.52 mmol) *N-*[(9H-Fluoren-9-ylmethoxy)carbonyl]-L-isoleucin, 22.964 g (71.52 mmol) *N-*[(1H-Benzotriazol-1-yloxy)(dimethylamino)methylen]-*N-*methylmethanaminium tetrafluoroborat (TBTU) und 13.865 g (107.28 mmol) *N-*Ethyl-*N-*isopropylpropan-2-amin (DIEA) versetzt und über Nacht geschüttelt. Man saugt ab, wäscht dreimal mit jeweils 400 mL Dimethylformamid, Methanol und Dichlormethan und saugt ab. Anschließend wird zweimal nacheinander wie folgt mit Piperidin behandelt: Man versetzt das Harz mit 400 mL 20%-iger Piperidin-Lösung in Dimethylformamid, schüttelt für 30 min, saugt ab und trocknet im Vakuum.

Man legt 14 g (20.86 mmol; Annahme: 1.49 mmol/g Beladung) des so erhaltenen Harzes in 200 mL Dimethylformamid vor und gibt 34.566 g (52.15 mmol) *N*²-[(9H-Fluoren-9-ylmethoxy)carbonyl]-*N*⁵-(imino{[(2,2,5,7,8-pentamethyl-3,4-dihydro-2H-chromen-6-yl)sulfonyl]amino}methyl)-D-ornithin, 16.745 g (52.15 mmol) *N-*[(1H-Benzotriazol-1-yloxy)(dimethylamino)methylen]-*N-*methylmethanaminium tetrafluoroborat (TBTU) und 13.48 g (104.30 mmol) *N*-Ethyl-*N*-isopropylpropan-2-amin (DIEA) hinzu. Man schüttelt über Nacht bei RT. Man saugt ab, wäscht dreimal mit jeweils 200 mL Dimethylformamid, Methanol und Dichlormethan und saugt ab. Anschließend wird zweimal nacheinander wie folgt mit Piperidin behandelt: Man versetzt das Harz mit 200 mL 20%-iger Piperidin-Lösung in Dimethylformamid, schüttelt für 30 min, saugt ab und trocknet im Vakuum.

Man legt das so erhaltene Harz in 200 mL Dimethylformamid vor und gibt 18.431 g (52.15 mmol) *N-*[(9H-Fluoren-9-ylmethoxy)carbonyl]-L-leucin, 16.745 g (52.15 mmol) N-[(1H-Benzotriazol-1-yloxy)(dimethylamino)methylen]-*N-*methylmethanaminium tetrafluoroborat (TBTU) und 13.480 g (104.30 mmol) *N-*Ethyl-*N-*isopropylpropan-2-amin (DIEA) hinzu. Man schüttelt über Nacht bei RT. Man saugt ab, wäscht dreimal mit jeweils 200 mL Dimethylformamid, Methanol und Dichlormethan und saugt ab. Anschließend wird zweimal nacheinander wie folgt mit Piperidin behandelt: Man versetzt das Harz mit 200 mL 20%-iger Piperidin-Lösung in Dimethylformamid, schüttelt für 30 min, saugt ab und trocknet im Vakuum.

Man legt 2.0 g (2.98 mmol; Annahme: 1.49 mmol/g Beladung) des so erhaltenen Harzes in 20 mL Dimethylformamid vor und gibt 2.462 g (8.94 mmol) *N-(tert* Butoxycarbonyl)-*O*-*tert*.-butyl-L-threonin, 2.870 g (8.94 mmol) *N-*[(1H-Benzotriazol-1-yloxy)(dimethylamino)methylen]-*N*-methylmethanaminium tetrafluoroborat (TBTU) und 2.311 g (17.88 mmol) N-Ethyl-N-isopropylpropan-2-amin (DIEA) hinzu. Man schüttelt über Nacht bei RT. Man saugt ab, wäscht dreimal mit jeweils 20 mL Dimethylformamid, Methanol und Dichlormethan und saugt ab.

### Beispiel 290A

### N-[(9H-Fluoren-9-ylmethoxy)carbonyl]glycyl-L-serin

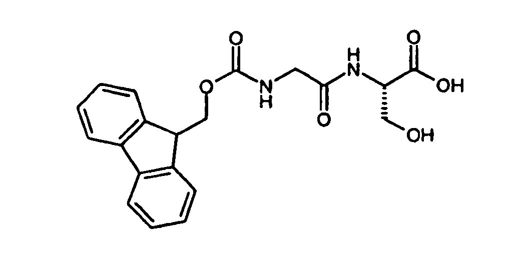

1 g (0.62 mmol) eines mit *O*-*tert*.-Butyl-N-[(9H-fluoren-9-ylmethoxy)carbonyl]-L-serin vorbeladenen Wang-Harzes (Merck Biosciences; Beladung: 0.62 mmol/g) wird zweimal nacheinander wie folgt mit Piperidin behandelt: Man versetzt das Harz mit 10 mL 20%-iger Piperidin-Lösung in Dimethylformamid, schüttelt für 30 min und saugt ab. Man legt das so erhaltene Harz in 10 mL Dimethylformamid vor und gibt 369 mg (1.24 mmol) *N-*[(9H-Fluoren-9-ylmethoxy)carbonyl]glycin, 398 mg (1.24 mmol) *N-*[(1H-Benzotriazol-1-yloxy)(dimethylamino)methylen]-*N*-methylmethanaminium tetrafluoroborat (TBTU) und 240 mg (1.86 mmol) N-Ethyl-N-isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Man versetzt mit 20 mL einer 50%-igen Trifluoressigsäure-Lösung in Dichlormethan, lässt 60 min einwirken, filtert das Harz über eine Fritte ab und wäscht das Harz dreimal mit 10 mL Dichlormethan nach. Das Filtrat wird eingeengt und die Zielverbindung als Rohprodukt erhalten.
LC-MS (Methode: LC-MS (Methode 19): Rₜ = 2.10 min, MS (ESIpos): *m*/*z* (%) =): 385.0 [M+H]⁺.

### Beispiel 291A

### Polymergebundenes [N-tertButoxycarbonyl-(3R)-3-hydroxy-L-leucyl]-[3-tertbutyl-L-alanyl]-[N⁵-(imino{[(2,2,5,7,8-pentamethyl-3,4-dihydro-2H-chromen-6-yl)sulfonyl]-amino}methyl)-D-ornithyl]-L-isoleucyl-L-allothreonyl-glycyl-[O³-tertbutyl-L-seryl]-O³-tertbutyl-L-serin

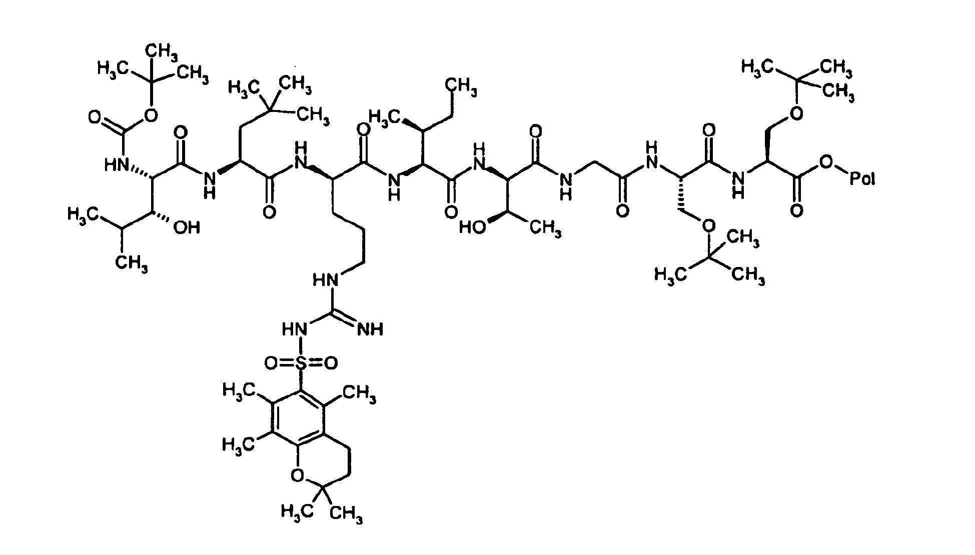

1 g (0.62 mmol) eines mit *O*-*tert*.-Butyl-*N-*[(9H-fluoren-9-ylmethoxy)carbonyl]-L-serin vorbeladenen Wang-Harzes (Merck Biosciences; Beladung: 0.62 mmol/g) wird zweimal nacheinander wie folgt mit Piperidin behandelt: Man versetzt das Harz mit 10 mL 20%-iger Piperidin-Lösung in Dimethylformamid, schüttelt für 30 min und saugt ab. Man legt das so erhaltene Harz in 10 mL Dimethylformamid vor und gibt 477 mg (1.24 mmol) der Beispielverbindung 290A, 398 mg (1.24 mmol) *N-*[(1H-Benzotriazol-1-yloxy)(dimethylamino)methylen]-*N-*methylmethanaminium tetrafluoroborat (TBTU) und 320 mg (2.48 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 423 mg (1.24 mmol) *N-*[(9H-Fluoren-9-ylmethoxy)carbonyl]-D-allothreonin, 399 mg (1.24 mmol) *N-*[(1H-Benzotriazol-1-yloxy)(dimethylamino)methylen]-*N-*methylmethanaminium tetrafluoroborat (TBTU) und 320 mg (2.48 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 438 mg (1.24 mmol) *N-*[(9H-Fluoren-9-ylmethoxy)carbonyl]-L-isoleucin, 399 mg (1.24 mmol) *N-*[(1H-Benzotriazol-1-yloxy)(dimethylamino)methylen]-*N-*methylmethanaminium tetrafluoroborat (TBTU) und 240 mg (1.86 mmol) N-Ethyl-N-isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 821 mg (1.24 mmol) *N*²-[(9H-fluoren-9-ylmethoxy)carbonyl]-*N*⁵-(imino{[(2,2,5,7,8-pentamethyl-3,4-dihydro-2H-chromen-6-yl)sulfonyl]amino}methyl)-D-ornithin, 399 mg (1.24 mmol) *N*-[(1H-Benzotriazol-1-yloxy)(dimethylamino)methylen]-N-methylmethanaminium tetrafluoroborat (TBTU) und 240 mg (1.86 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt 812 mg (0.503 mmol) des so erhaltenen Harzes in 10 mL Dimethylformamid vor und gibt 370 mg (1.01 mmol) *N-*[(9H-Fluoren-9-ylmethoxy)carbonyl]-4-methyl-L-leucin, 323 mg (1.01 mmol) *N-*[(1H-Benzotriazol-1-yloxy)(dimethylamino)methylen]-*N-*methylmethanaminium tetrafluoroborat (TBTU) und 195 mg (1.51 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 157 mg (0.633 mmol) *N-*(*tert-*Butoxycarbonyl)-(3*R*)-3-hydroxy-L-leucin, 203 mg (0.633 mmol) *N-*[(1H-Benzotriazol-1-yloxy)(dimethylamino)methylen]-N-methylmethanaminium tetrafluoroborat (TBTU) und 196 mg (1.52 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

### Beispiel 292A

### Polymergebundenes [N-tertButoxycarbonyl-(3R)-3-hydrOxy-L-leucyl]-[3-trimethylsilyl-L-alanyl]-[N⁵-(imino{[(2,2,5,7,8-pentamethyl-3,4-dihydro-2H-chromen-6-yl)sulfonyl]-amino}methyl)-D-ornithyl]-L-isoleucyl-[O-tertbutyl-L-allothreonyl]-glycyl-[O³-tertbutyl-L-seryl]-O³-tertbutyl-L-serin

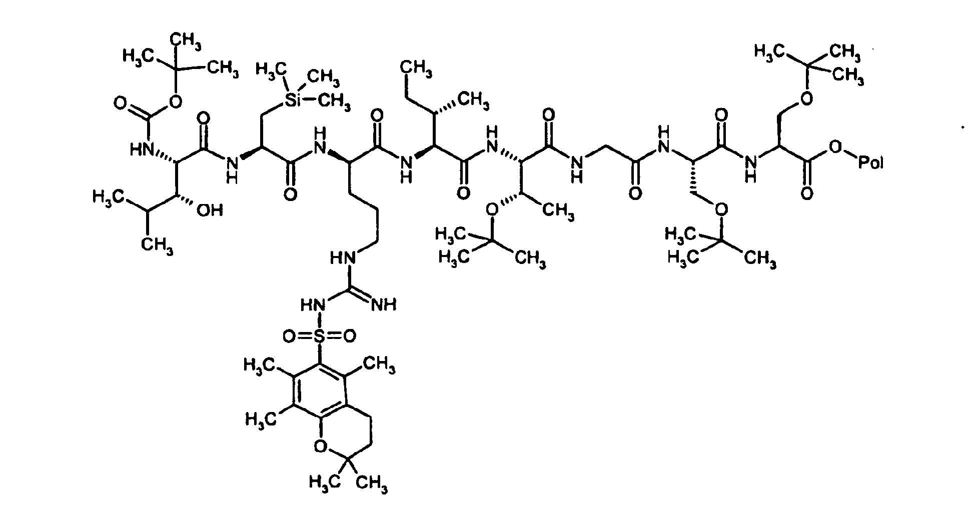

1 g Wang-Harz (Rapp Polymere; Beladung: 1.28 mmol/g) wird in 10 mL Dimethylformamid vorgelegt. Man gibt 982 mg (2.56 mmol) O-*tert*.-Butyl-*N-*[(9H-fluoren-9-ylmethoxy)carbonyl]-L-serin, 910 mg (2.56 mmol) N-{[(6-Chlor-1H-benzotriazol-1-yl)oxy](dimethylamino)methylen}-*N-*methylmethanaminium tetrafluoroborat (TCTU) und 496 mg (3.84 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man führt eine weitere Kupplung mit *O*-*tert.*-Butyl-N-[(9H-fluoren-9-ylmethoxy)carbonyl]-L-serin unter Benutzung der gleichen Mengenverhältnisse und unter den beschriebenen Bedingungen durch (siehe direkt oben).

Man legt das so erhaltene Harz in 10 mL Dimethylformamid vor und gibt 761mg (2.56 mmol) N-[(9H-Fluoren-9-ylmethoxy)carbonyl]glycin, 910 mg (2.56 mmol) *N-*{[(6-Chlor-1H-benzotriazol-1-yl)oxy](dimethylamino)methylen}-*N*-methylmethanaminium tetrafluoroborat (TCTU) und 496 mg (3.84 mmol) *N-*Ethyl-*N-*isopropylpropan-2-amin (DIEA) hinzu. Man schüttelt fünf Stunden bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 1.017 g (2.56 mmol) *O*-*tert*.-Butyl-*N*-[(9H-fluoren-9-ylmethoxy)carbonyl]-D-allothreonin, 910 mg (2.56 mmol) *N*-{[(6-Chlor-1H-benzotriazol-1-yl)oxy](dimethylamino)methylen}-*N-*methylmethanaminium tetrafluoroborat (TCTU) und 496 mg (3.84 mmol) *N-*Ethyl-*N*-isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 904 mg (2.56 mmol) *N-*[(9H-Fluoren-9-ylmethoxy)carbonyl]-L-isoleucin, 822 mg (2.56 mmol) *N*-[(1H-Benzotriazol-1-yloxy)(dimethylamino)methylen]-*N*-methylmethanaminium tetrafluoroborat (TBTU) und 496 mg (3.84 mmol) *N*-Ethyl-*N*-isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 1.697 g (2.56 mmol) *N*²-[(9H-Fluoren-9-ylmethoxy)carbonyl]-*N*⁵-(imino{[(2,2,5,7,8-pentamethyl-3,4-dihydro-2H-chromen-6-yl)sulfonyl]amino}methyl)-D-ornithin, 822 mg (2.56 mmol) *N*-[(1H-Benzotriazol-1-yloxy)(dimethylamino)methylen]-*N*-methylmethanaminium tetrafluoroborat (TBTU) und 662 mg (5.12 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 982 mg (2.56 mmol) *N-*[(9H-fluoren-9-ylmethoxy)carbonyl]-3-(trimethylsilyl)-L-alanin, 822 mg (2.56 mmol) *N-*[(1H-Benzotriazol-1-yloxy)(dimethylamino)methylen]-*N-*methylmethanaminium tetrafluoroborat (TBTU) und 662 mg (5.12 mmol) *N-*Ethyl-*N-*isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab. Anschließend wird zweimal nacheinander wie folgt mit Piperidin behandelt: Man versetzt das Harz mit 10 mL 20%-iger Piperidin-Lösung in Dimethylformamid, schüttelt für 30 min und saugt ab.

Man legt das so erhaltene Harz in 10 mL Dimethylformamid vor und gibt 261 mg (0.705 mmol) *threo N-*(*tert-*Butoxycarbonyl)-(3*R*)-3-hydroxy-L-leucin, 339 mg (1.057 mmol) *N-*[(1H-Benzotriazol-1-yloxy)(dimethylamino)methylen]-*N-*methylmethanaminium tetrafluoroborat (TBTU) und 273 mg (2.114 mmol) *N-*Ethyl-N-isopropylpropan-2-amin (DIEA) hinzu. Man schüttelt über Nacht bei RT. Man saugt ab, wäscht dreimal mit jeweils 10 mL Dimethylformamid, Methanol und Dichlormethan und saugt ab.

Die in den nachfolgenden Tabellen dargestellten Verbindungen 293A-458A wurden gemäß den angegebenen Arbeitsvorschriften aus den angegebenen Edukten hergestellt.

| Nr | **Struktur** | |
|---|---|---|
| | **Name** | **Synthesemethode** |
| 293A | 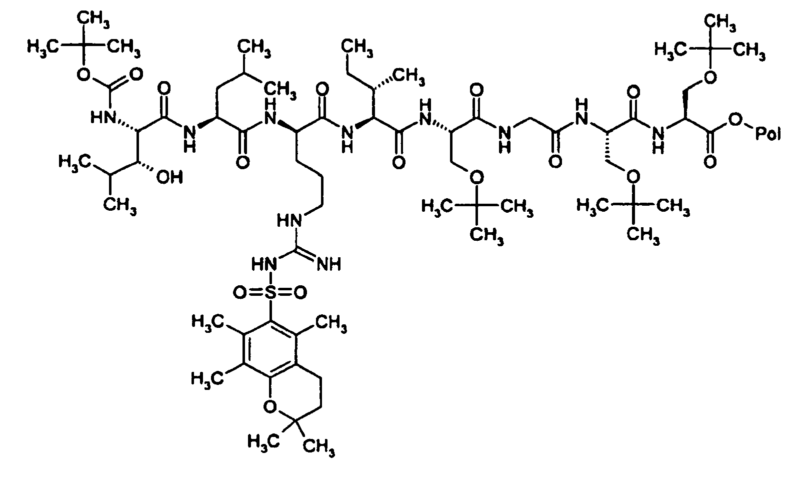 | |
| | Polymergebundenes [*N-tert*Butoxycarbonyl-(3R)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]-L-isoleucyl-[*O*³-*tert*butyl-L-seryl]-glycyl-[*O*³-*tert*butyl-1-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes. |
| 294A | 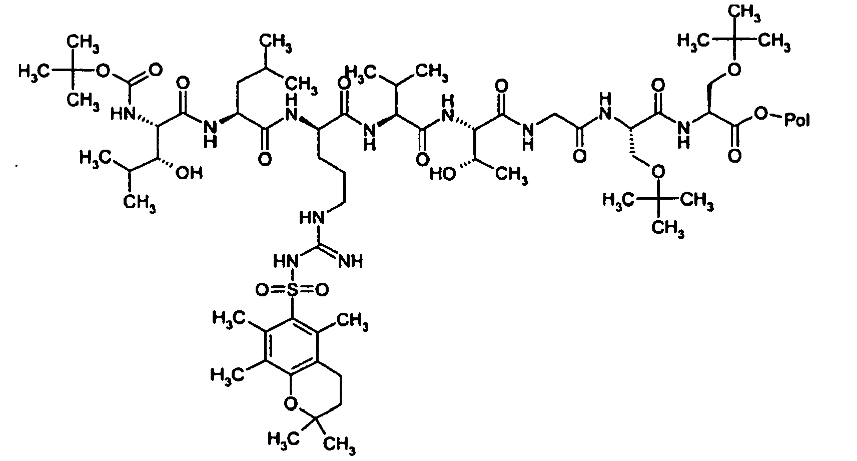 | |
| | Polymergebundenes [*N*-*tert*Butoxycarbonyl-(3*R*)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]-L-valyl-L-allothreonyl-glycyl-[*O*³-*tert*butyl-L-seryl]-*O*³-*tert*butyl-Lserin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 295A | 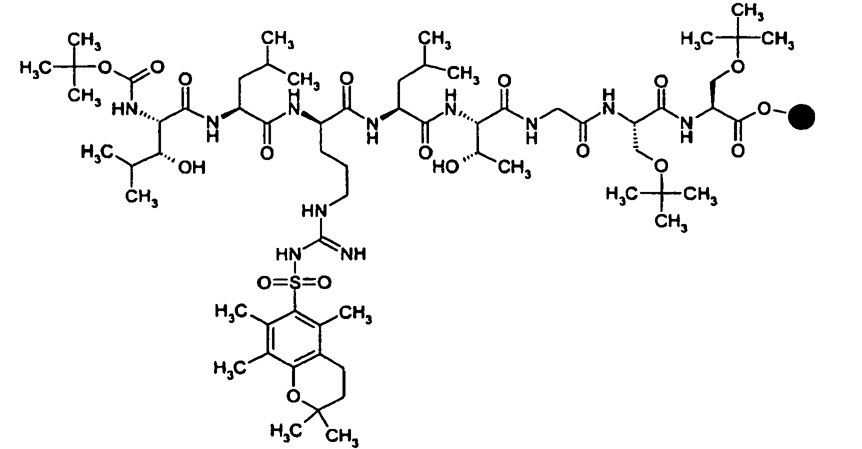 | |
| | Polymergebundenes [*N*-*tert*Butoxycarbonyl-(3*R*)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]-L-leucyl-L-allothreonyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N-*[(9H-fluoren-9-ylmethoxy)carbonyl]-L-serin pro Gramm beladenen 2-Chlortritylchlorid-Harzes |
| 296A | 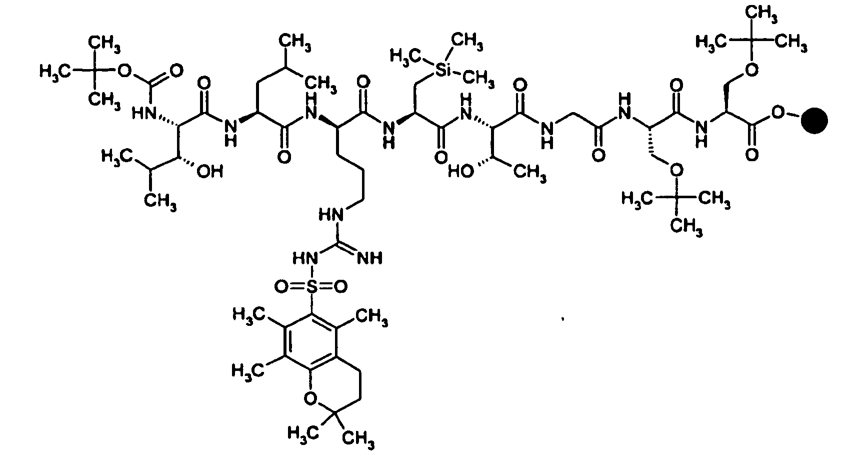 | |
| | Polymergebundenes [*N-tert*Butoxycarbonyl-(3*R*)-3-hydroxy-L-leucyl]-L-leucyl-[N⁵-(imino{[(2,2,5,7,8-pentamethyl-3,4-dihydro-2H-chromen-6-yl)sulfonyl]amino}-methyl)-D-2H-chromen-6-yl)sulfonyl]amino}methyl)-D-ornithyl]-[3-Trimethylsilyl]-L-alanyl-L-allothreonyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 297A | 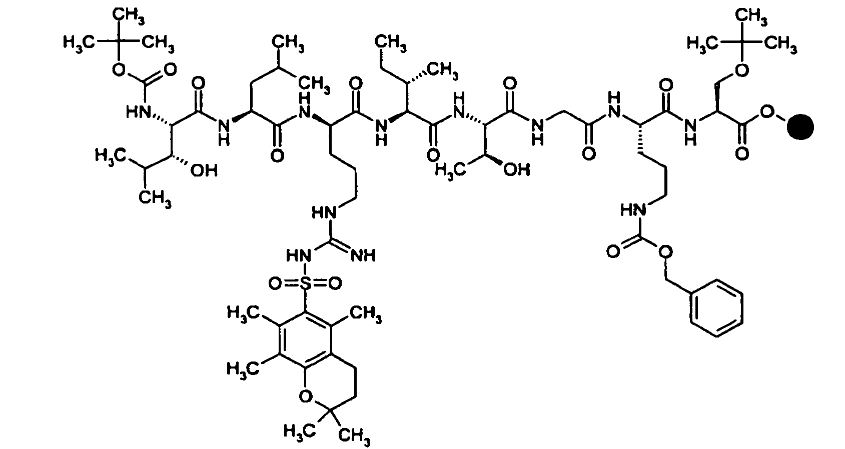 | |
| | Polymergebundenes [*N-tert*Butoxycarbonyl-(3*R*)-3-hydroxy-L-leucyl]-L-leucyl-[N⁵-(imino{[(2,2,5,7,8-pentamethyl-3,4-dihydro-2H-chromen-6-yl)sulfonyl]amino}methyl)-D-ornithyl]-L-phenylalanyl-L-allothreonyl-glycyl-[*N*⁵-benzyloxycarbonyl-L-ornithyl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 298A | 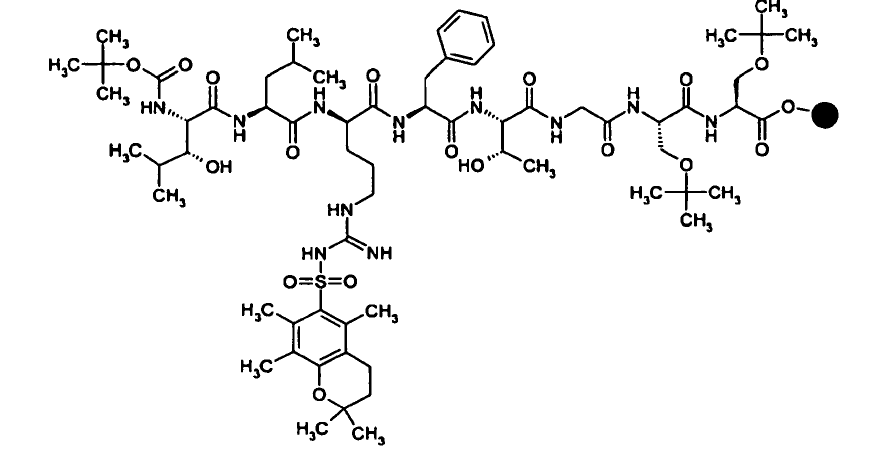 | |
| | Polymergebundenes [*N-tert*Butoxycarbonyl-(3*R*)-3-hydroxy-L-leucyl]-L-leucyl-[N'-(imino{[(2,2,5,7,8-pentamethyl-3,4-dihydro-2H-chromen-6-yl)sulfonyl]amino}methyl)-D-ornithyl]-L-phenylalanyl-L-allothreonyl-glycyl-[*O*³-*tert*Butyl-L-seryl]-*O*³-*tert*butyl-L-serin | Dargestellt nach Arbeitsvorschrift 8 aus 1.75 g des mit 0.85 mmol *O*³-*tert*Butyl-*N-*[(9H-fluoren-9-ylmethoxy)carbonyl]-L-serin pro Gramm beladenen 2-Chlortritylchlorid-Harzes |
| 299A | 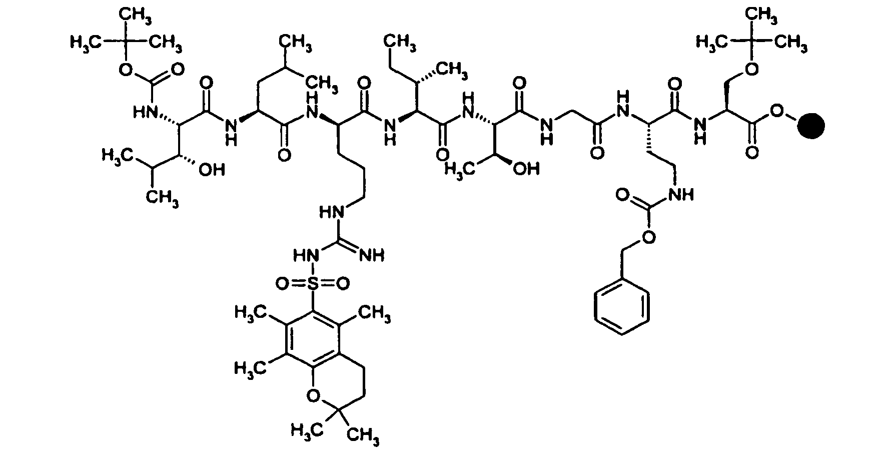 | |
| | Polymergebundenes [*N-tert*Butoxycarbonyl-(3*R*)-3-hydroxy-L-leucyl]-L-leucyl-[*N*⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-glycyl-[(2S)-2-amino-4-benzyloxycarbonylamino-butyryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 300A | 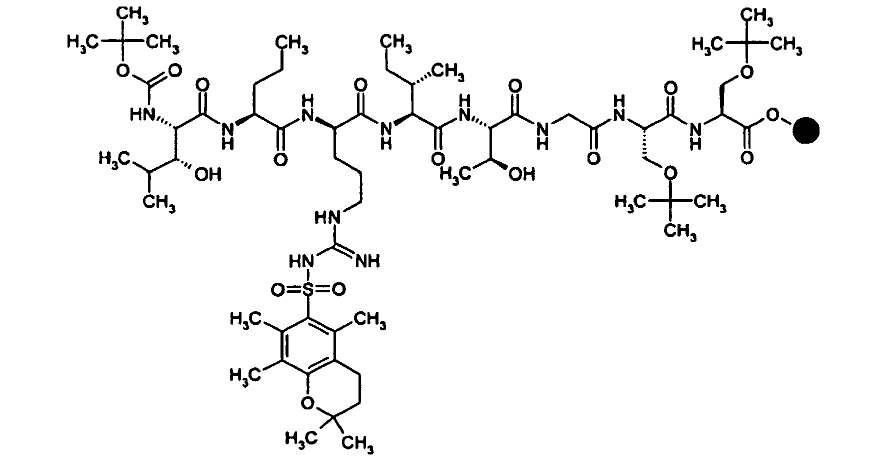 | |
| | Polymergebundenes [*N-tert*Butoxycarbonyl-(3*R*)-3-hydroxy-L-leucyl]-L-norvalyl-[N⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-glycyl-[*O*³-*tert*butyl-L-seryl]-*O*³-*tert*butvl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 301A | 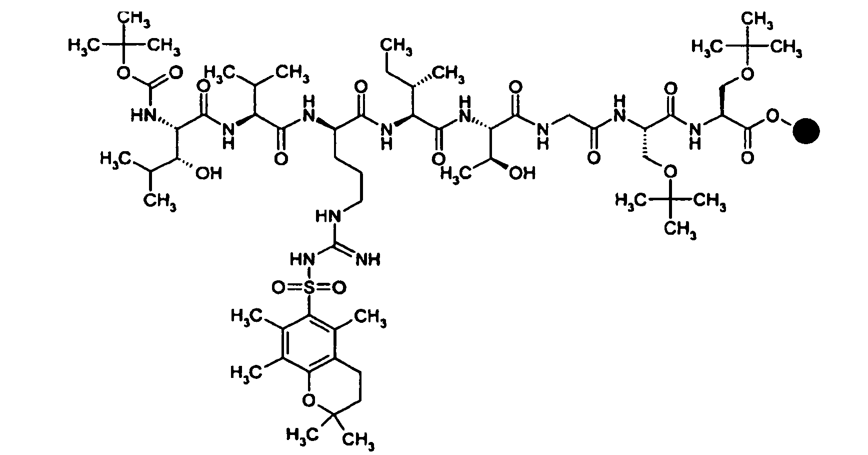 | |
| | Polymergebundenes [*N*-*tert*Butoxycarbonyl-(3*R*)-3-hydroxy-L-leucyl]-L-valyl-[*N*⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 302A | 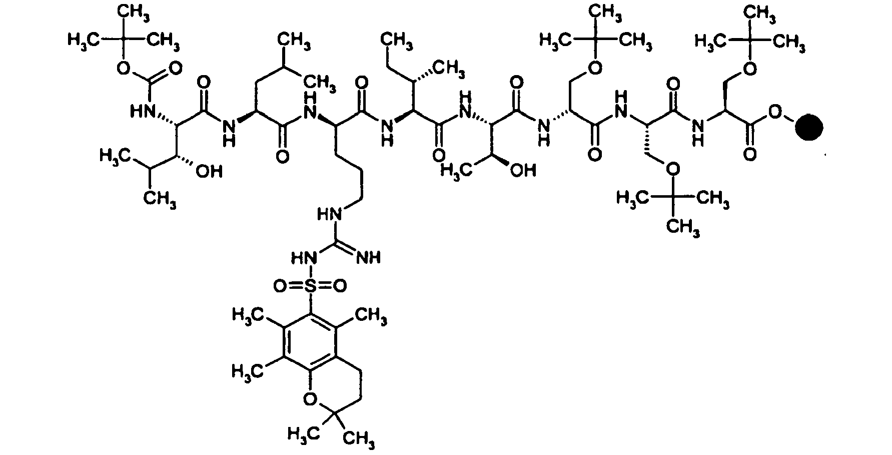 | |
| | Polymergebundenes [*N-tert*Butoxycarbonyl-(3*R*)-3-hydroxy-L-leucyl]-L-leucyl-[*N*⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-[*O*³-*tert*butyl-D-seryl]-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-Gramm beladenen 2-pro Chlortritylchlorid-Harzes |
| 303A | 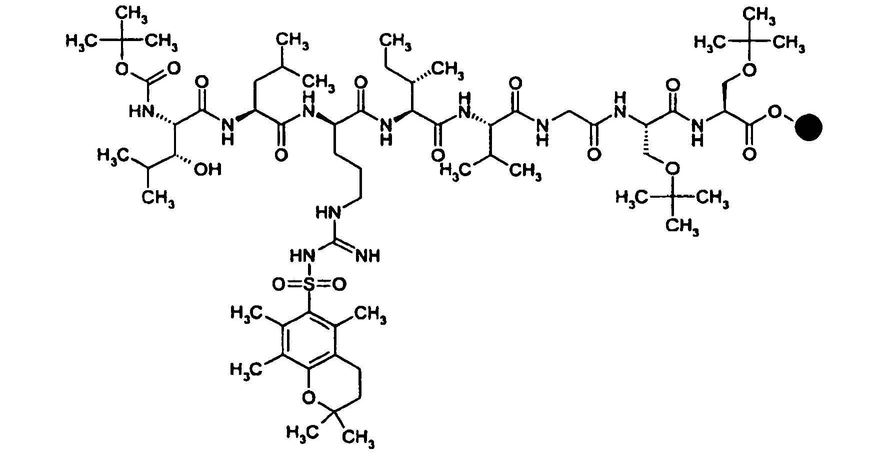 | |
| | Polymergebundenes [*N*-*tert*Butoxycarbonyl-(3*R*)-3-hydroxy-L-leucyl]-L-leucyl-[*N*³-(imino{[(2,2,5,7,8-pentamethyl-3,4-dihydro-2H-chromen-6-yl)sulfonyl]aminolmethyl)-D-ornithyl]-L-isoleucyl-L-valyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*butyl-N-[(9H-fluoren-9-ylmethoxy)carbonyl]-L-serin pro Gramm beladenen 2-Chlortritylchlorid-Harzes |
| 304A | 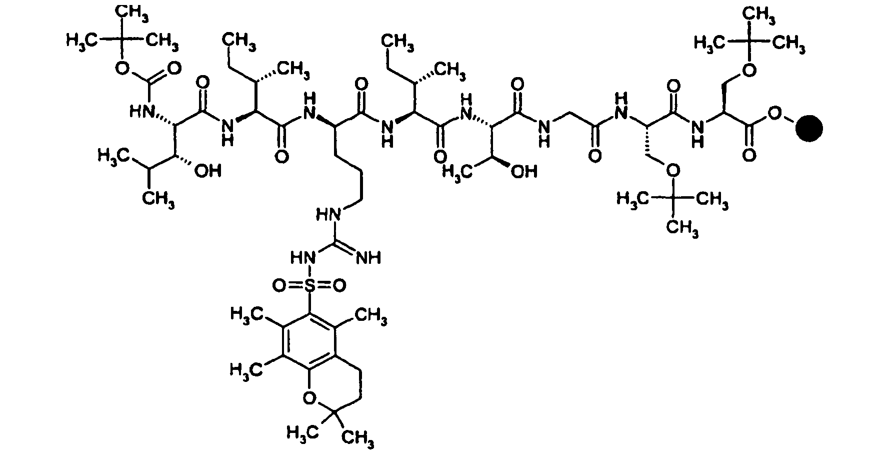 | |
| | Polymergebundenes [*N*-*tert*Butoxycarbonyl-(3*R*)-3-hydroxy-L-leucyl]-L-isoleucyl-[*N*⁵-(imino{[(2,2,5,7,8-pentamethyl-3,4-dihydro-2H-chromen-6-yl)sulfonyl]amino)methyl)-D-ornithyl]-L-isoleucyl-L-allothreonyl-glycyl-[O³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 305A | 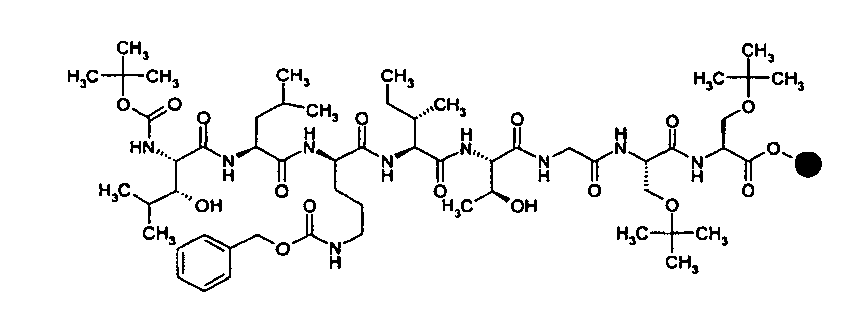 | |
| | Polymergebundenes [*N*-*tert*Butoxycarbonyl-(3*R*)-3-hydroxy-L-leucyl]-L-leucyl-[*N*⁵-benzyloxycarbonyl-D-ornithyl]-L-isoleucyl-L-allothreonyl-glycyl-[*O*³-*tert*butyl-t,-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 306A | 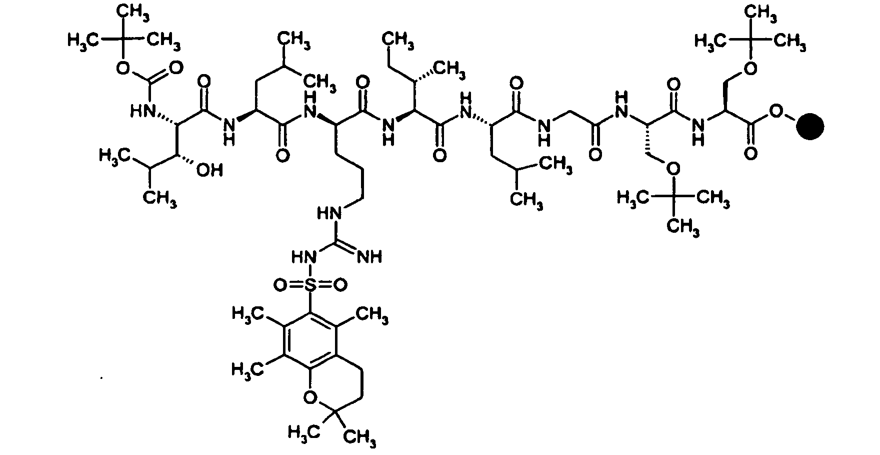 | |
| | Polymergebundenes [*N-tert*Butoxycarbonyl-(3*R*)-3-hydroXy-L-leucyl]-L-leucyl-[*N*⁵-(imino{[(2,2,5,7,8-pentamethyl-3,4-dihydro-2H-chromen-6-yl)sulfonyl]amino}methyl)-D-ornithyl)-L-isoleucyl-L-leucyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 307A | 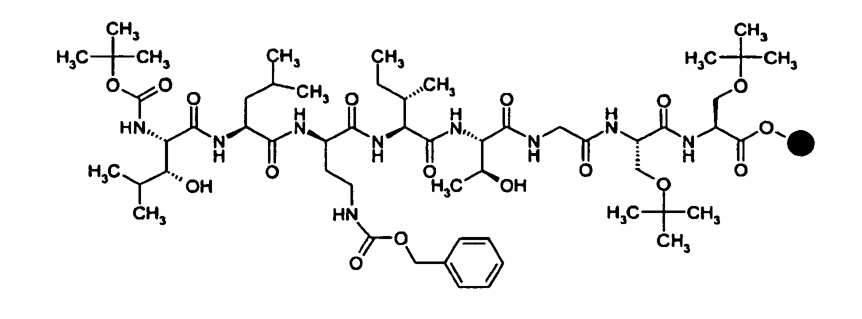 | |
| | Polymergebundenes [*N*-*tert*Butoxycarbonyl-(3*R*)-3-hydroxy-L-leucyl]-L-leucyl-[(2*R*)-2-amino-4-benzyloxycarbonylamino-butyry]-L-isoleucyl-L-allothreonyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*butyl-*N*-[(9H-fluoren-9-ylmethoxy)carbonyl]-L-serin pro Gramm beladenen 2-Chlortritylchlorid-Harzes |
| 308A | 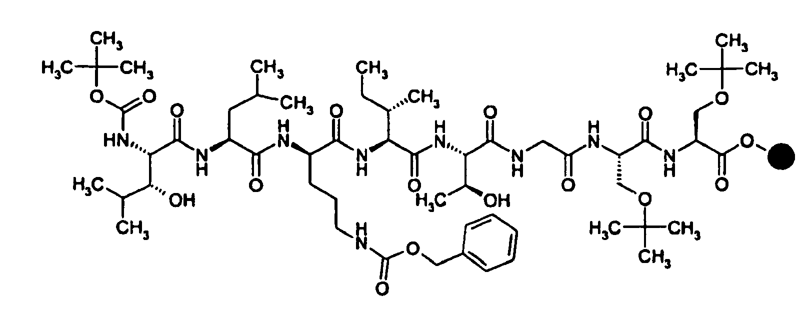 | |
| | Polymergebundenes [*N*-*tert*Butoxycarbonyl-(3*R*)-3-hydroxy-L-leucyl]-L-leucyl-[*N*⁶-benyloxycarbonyl-L-lysyl]-L-isoleucyl-L-allothreonyl-glycyl-[*O*³-*tert*butyl-L-seryl]-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 309A | 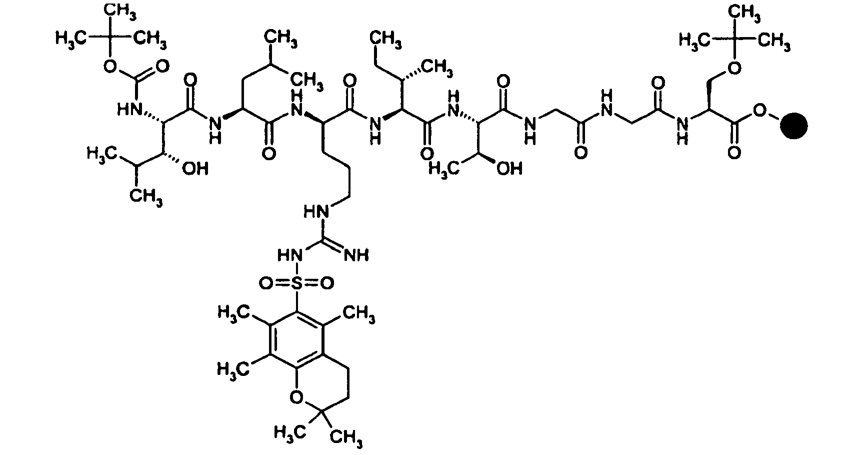 | |
| | Polymergebundenes [*N*-*tert*Butoxycarbonyl-(3*R*)-3-hydroxy-L-leucyl]-L-leucyl-[*N*⁵-(imino{[(2,2,5,7,8-pentamethyl-3,4-dihydro-2H-chromen-6-yl)sulfonyl]amino}methyl)-D-ornithyl)-L-isoleucyl-L-allothreonyl-glycyl-glycyl-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |
| 310A | 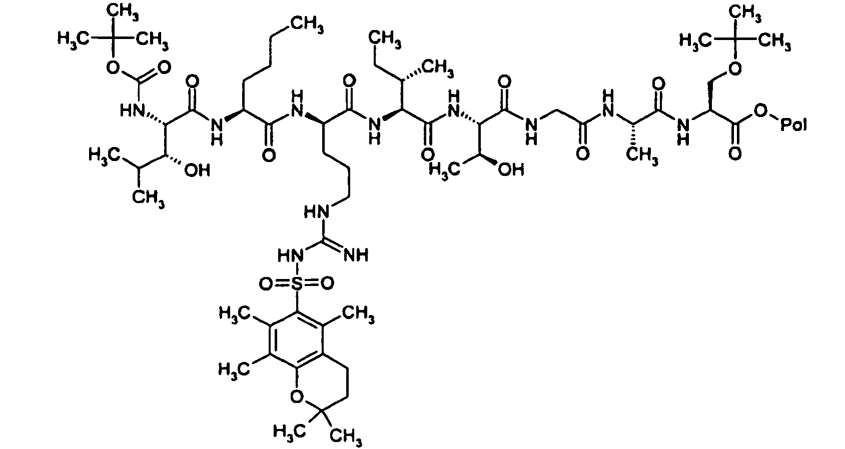 | |
| | Polymergebundenes [*N-tert*Butoxycarbonyl-(3*R*)-3-hydroxy-L-leucyl]-L-norleucyl-[*N*⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-glycyl-L-alanyl-*O*³-*tert*butyl-L-serin | Dargestellt nach Arbeitsvorschrift 8 aus 1.75 g des mit 0.85 mmol *O*³*tert*Butyl-*N*-[(9H-fluoren-9-ylmethoxy)carbonyl]-L-serin pro Gramm beladenen 2Chlortritylchlorid-Harzes |
| 311A | 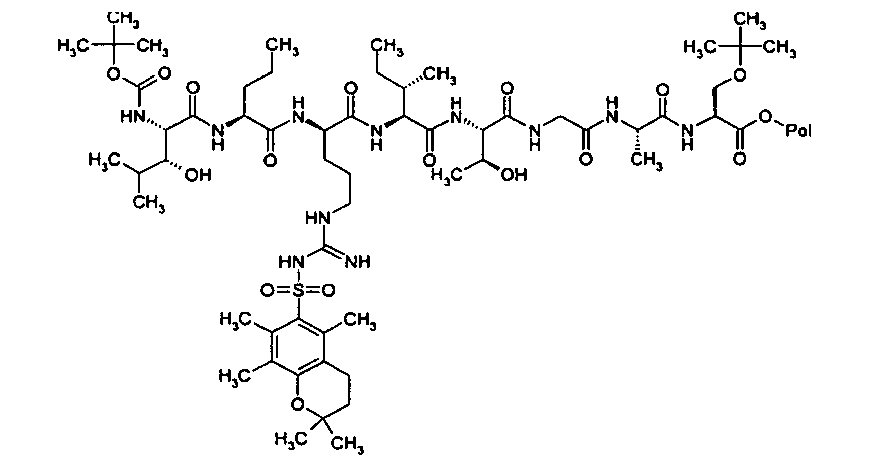 | |
| | Polymergebundenes [*N-tert*Butoxycarbonyl-(3*R*)-3-hydroxy-L-leucyl]-L-norvalyl-[*N*⁵-(imino{[(2,2,5,7,8-pentamethyl-3,4-dihydro-2H-chromen-6-yl)sulfonyl]amino}methyl)-D-ornithyl]-L-isoleucyl-L-allothreonyl-glycyl-L-alanyl-*O*³-*tert*butyl-L-serin | Dargestellt nach Arbeitsvorschrift 8 aus 1.75 g des mit 0.85 mmol *O*³-*tert*Butyl-*N*-[(9H-fluoren-9-ylmethoxy)carbonyl]-L-serin pro Gramm beladenen 2-Chlortritylchlorid-Harzes |

### Entschützte Octapeptide

| Nr | Struktur | |
|---|---|---|
| | Name | Analytik |
| | Ausbeute, Synthesemethode | |
| 312A | 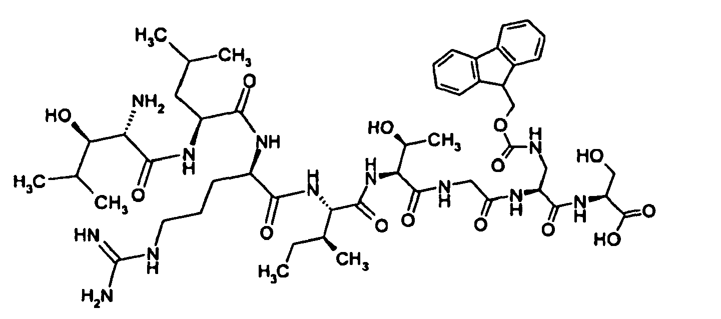 | |
| | [(3*R*)-3-hydroxy-L-leucyl]-L-leucyl-D-arginyl-L-isoleucyl-L-allothreonyl-glycyl-[(2*S*)-2-amino-3-(9H-fluoren-9-ylmethoxycarbonyl)aminobutyryl] -L-serin-bis-trifluoracetat | HPLC (Methode 6): Rₜ = 3.35 min; LC-MS (Methode 19): Rₜ = 1.50 min, MS (ESIpos): *m*/*z* (%) = 543 (100) [M+2H]²⁺, 1083 (20) [M+H]⁺, MS (ESI-neg): *m*/*z* (%) = 1081 (100) [M-H]⁻; HR-TOF-MS: C₅₁H₇₉N₁₂O₁₄ ber. 1083.5834, gef. 1083.5815 [M+H]⁺. |
| | 647 mg (98% d. Th.) aus 701 mg (0.50 mmol) der Verbindung 288A nach Arbeitsvorschrift 5 | |
| 313A | 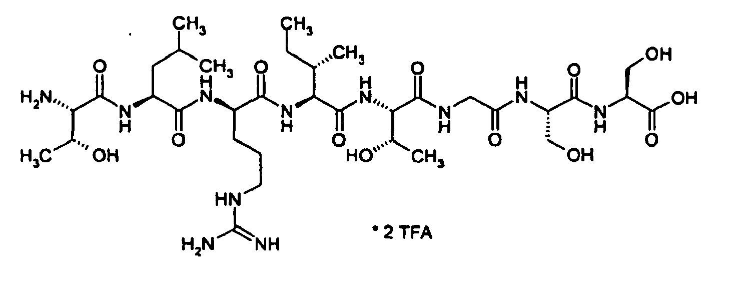 | |
| | L-Threonyl-L-leucyl-D-arginyl-L-isoleucyl-L-allothreonyl-glycyl-L-seryl-L-serin-bis-trifluoracetat | (LC-MS (Methode 22): Rₜ = 2.08 min, MS (ESIpos): m/z (%) = 418 (100) [M+2H]²⁺. HR-TOF-MS: C₃₄ H₆₄ N₁₁ O₁₃ ber. 834.4680, gef. 834.4653 [M+H]⁺. |
| | 31 mg aus 40 mg 289A nach Arbeitsvorschrift 9 | |
| 314A | 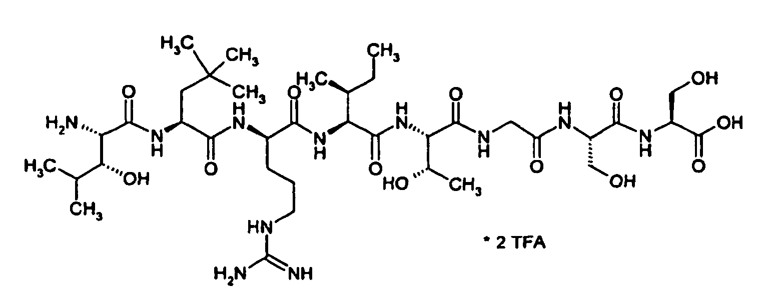 | |
| | [(*3R*)-3-hydroxy-L-leucyl]-[3-*tert*butyl-L-alanyl]-D-arginyl-L-isoteucyl-L-allothreonyl-glycyl-L-seryl-L-serin-bis-trifluoracetat | |
| | 250 mg Rohausbeute aus dem in Beispiel 291A beschriebenen Ansatz nach der allgemeinen Arbeitsvorschrift 9 | |
| 315A | 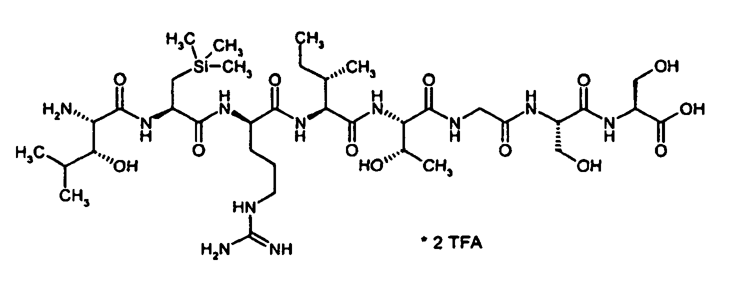 | |
| | [(*3R*)-3-hydroxy-L-leucyl]-[3-trimethylsilyl-L-alanyl]-D-arginyl-L-isoleucyl-L-allothreonyl-glycyl-L-seryl-L-serin-bis-trifluoracetat | LC-MS (Methode 22): Rₜ = 2.29 min, MS (ESIpos): *m*/*z* = 892.0 [M+H]⁺. |
| | 53 mg Rohausbeute aus dem in Beispiel 292A beschriebenen Ansatz nach der allgemeinen Arbeitsvorschrift 9 | |
| 316A | 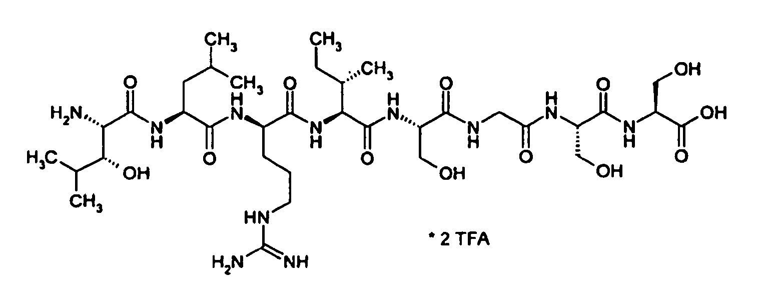 | |
| | [(3R)-3-hydroxy-L-leucyl]-L-leucyl-D-arginyl-L-isoleucyl-L-seryl-glycyl-L-seryl-L-serin-bis-trifluoracetat | HPLC (Methode 6): Rₜ = 2.79 min; LC-MS (Methode 22): Rₜ = 2.10 min, MS (ESIpos): *m*/*z* (%) = 425 (100) [M+2H]²⁺; MS (ESIneg): *m*/*z* (%) = 846 (100) [M-H]⁻; HR-TOF-MS: C₃₅H₆₆N₁₁O₁₃ ber. 848.4837 , gef. 848.4806 [M+H]⁺. |
| | 900 mg aus Beispielverbindung 293A nach Arbeitsvorschrift 9 | |
| 317A | 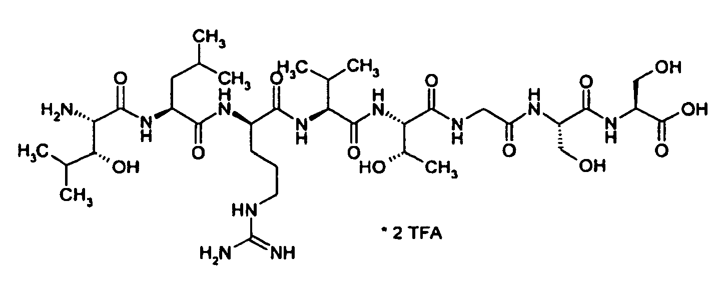 | |
| | [(3*R*)-3-hydroxy-L-leucyl]-L-leucyl-D-arginyl-L-valyl-L-allothreonyl-glycyl-L-seryl-L-serin-bis-trifluoracetat | HPLC (Methode 6): Rₜ = 2.70 min; LC-MS (Methode 22): Rₜ = 2.05 min, MS (ESIpos): *m*/*z* (%) = 425 (100) [M+2H]²⁺, MS (ESIneg) *m*/*z* (%) = 846 (100) [M-H] ; HR-TOF-MS: C₃₅H₆₆N₁₁O₁₃ ber. 848.4837, gef. 848.4810 [M+H]⁺. |
| | 1.01 g aus Beispielverbindung 294 A nach Arbeitsvorschrift 9 | |
| 318A | 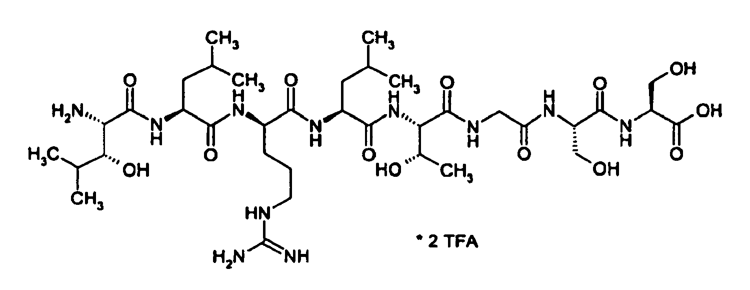 | |
| | [(*3R*)-3-hydroxy-L-leucyl]-L-leucyl-D-arginyl-L-leucyl-L-allothreonyl-glycyl-L-seryl-L-serin-bis-trifluoracetat | HPLC (Methode 6): Rₜ = 2.84 min; LC-MS (Methode 22): Rₜ = 2.20 min, MS (ESIpos): *m*/*z* (%) = 432 (100) |
| | 1.11 g (40% rein) aus Beispielverbindung 295A nach Arbeitsvorschrift 9 und Arbeitsvorschrift 9 | [M+2H]²⁺, MS (ESIneg): *m*/*z* (%) = 861 (5) [M-H] ; HR-TOF-MS: C₃₆H₃₆N₁₁O₁₃ ber. 862.4993, gef. 862.4977 [M+H]⁺. |
| 319A | 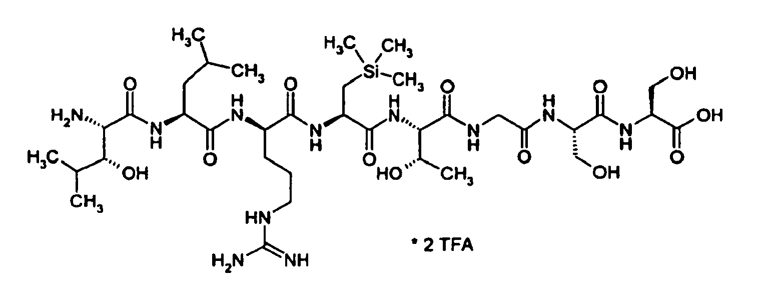 | |
| | [(*3R*)-3-hydroxy-L-leucyl]-L-leucyl-n-arginyl-[3-trimethylsilyl-L-alanlyl]-L-allothreonyl-glycyl-L-seryl-L-serin-bis-trifluoracetat | HPLC (Methode 6): Rₜ = 2.96 min; LC-MS (Methode 22): Rₜ = 2.31 min, MS (ESIpos): *m*/*z* (%) = 447 (100) [M+2H]²⁺, MS (ESIneg): *m*/*z* (%) = 890 [M+2H]²⁺, MS (ESIneg): *m*/*z* (%) = 890 C₃₆H₇₀N₁₁O₁₃Si ber. 892.4919, gef. 892.4955 [M+H]⁺ |
| | 1.50 g (64% rein) aus Beispielverbindung 296A nach Arbeitsvorschrift 9, Reinigung nach Methode 45. | |
| 320A | 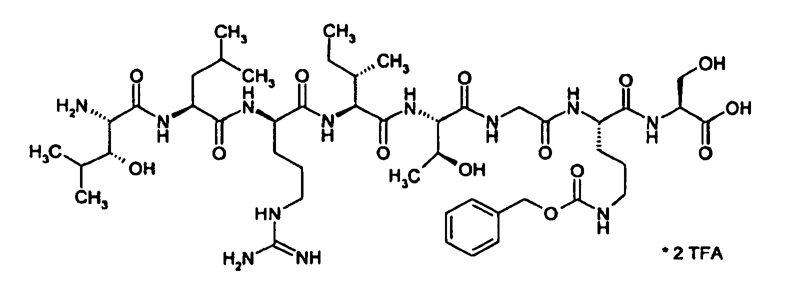 | |
| | [(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-bis-trifluoracetat | HPLC (Methode 6): Rₜ = 3.17 min; LC-MS (Methode 19): Rₜ = 1.30 min, MS (ESIpos): *m*/*z* (%) = 512.4 (100) [M+2H]²⁺, MS (ESIneg): *m*/*z* (%) = 1021.7 (100) [M-H] HR-TOF-MS: C₄₆H₇₉N₁₂C₁₄ ber. 1023.5834, gef. 1023.5842 [M+H]⁺. |
| | 1.27 g (45% rein) aus Beispielverbindung 297A nach Arbeitsvorschrift 9, Reinigung nach Methode 45. | |
| 321A | 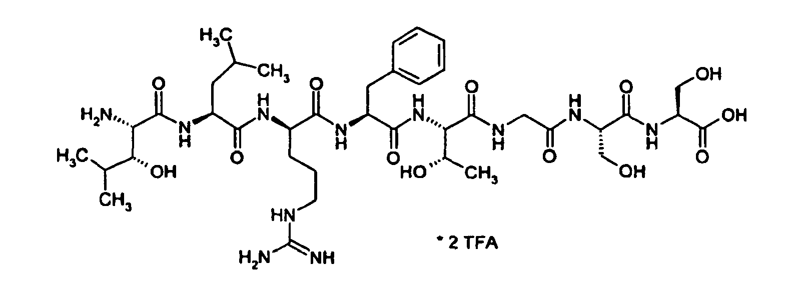 | |
| | [(3*R*)-3-hydroxy-L-leucyl]-L-leucyl-D-arginyl-L-phenylalanlyl-L-allothreonyl-glycyl-L-seryl-L-serin-bis-trifluoracetat | HPLC (Methode 6): Rₜ = 2.86 min; LC-MS (Methode 22): Rₜ = 2.19 min, MS (ESIpos): *m*/*z* (%) = 449 (100) [M+2H]²⁺, MS (ESIneg): *m*/*z* (%) = 894 (100) [M-H]; HR-TOF-MS: C₃₉H₆₆N₁₁O₁₃ ber. 896.4837, gef. 896.4849 [M+H]⁺. |
| | 943 mg (66% rein) aus Beispielverbindung 298A nach Arbeitsvorschrift 9, Reinigung nach Methode | |
| 322A | 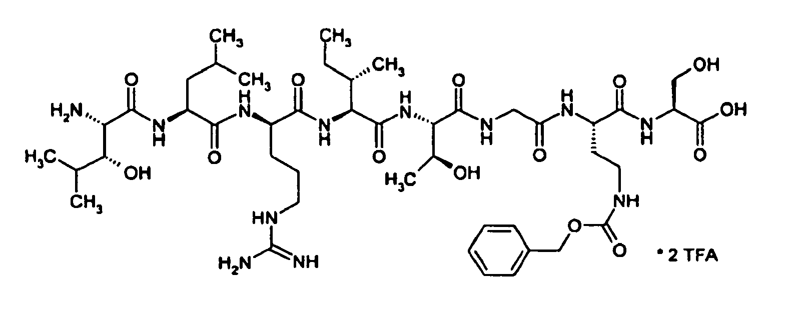 | |
| | [(*3R*)-3-hydroxy-L-leucyl]-L-leucyl-D-arginyl-L-isoleucyl-L-allothreonyl-glycyl-[(2S)-2-amino-4-benzyloxycarbonylamino-butyryl]-L-serin-bis-trifluoracetat | HPLC (Methode 6): Rₜ = 3.13 min; LC-MS (Methode 19): Rₜ = 1.26 min, MS (ESIpos): *m*/*z* (%) = 505 (100) [M+2H]²⁺, MS (ESIneg): *m*/*z* (%) = 1007.8 (100) [M-H] ; HR-TOF-MS: C₄₅H₇₇N₁₂O₁₄ ber. 1009.5677, gef. 1009.5704 [M+H]⁺. |
| | 980 mg (67% rein) aus Beispielverbindung 299A nach Arbeitsvorschrift 9, Reinigung nach Methode 45 | |
| 323A | 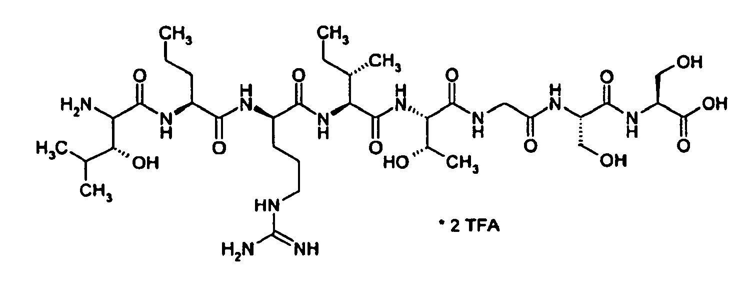 | |
| | [(3R)-3-hydroxy-L-leucyl]-L-norvalyl-D-arginyl-L-isoleucyl-L-allothreonyl-glycyl-L-seryl-L-serin. bis-trifluoracetat | HPLC (Methode 6): Rₜ = 2.69 min; LC-MS (Methode 22): Rₜ = 2.03 min, MS (ESIpos): *m*/*z* (%) = 848.5 (60) [M+H]⁺, MS (ESIneg): *m*/*z* (%) = 846.5 (100) [M-H]. |
| | 1320 mg (35% rein) aus Beispielverbindung 300A nach Arbeitsvorschrift 9, Reinigung nach Methode 45 | |
| 324A | 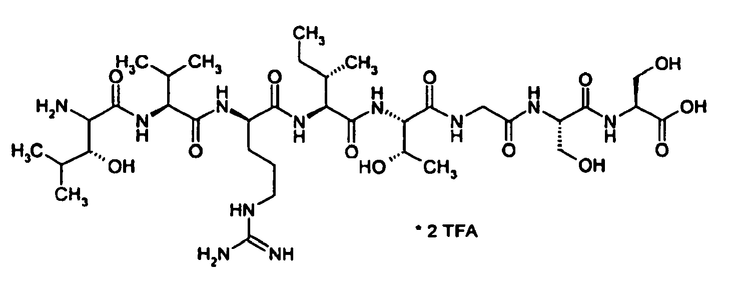 | |
| | [(*3R*)-3-hydroxy-L-leucyl]-L-valyl-D-arginyl-L-isoleucyl-L-allothreonyl-glycyl-L-seryl-L-serin-bis-trifluoracetat | HPLC (Methode 6): Rₜ = 2.74 min; LC-MS (Methode 22): Rₜ = 2.01 min, MS (Methode 22): Rₜ 2.01 min, MS (ESIpos): *m*/*z* (%) = 424.8 (100) [M+2H]²⁺, MS (ESIneg): *m*/*z* (%) = 846.4 (100) [M-H]⁺. |
| | 843 mg (50% rein) aus Beispielverbindung 301A nach Arbeitsvorschrift 9, Reinigung nach Methode 45 | |
| 325A | 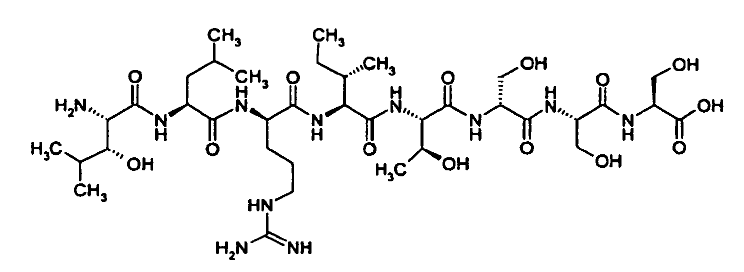 | |
| | [(*3R*)-3-hydroxy-L-leucyl]-L-leucyl-D-arginyl-L-isoleucyl-L-allothreonyl-D-seryl-L-seryl-L-serin-bis-trifluoracetat | HPLC (Methode 6): Rₜ = 2.81 min; LC-MS (Methode 22): Rₜ = 2.19 min, MS (ESIpos): *m*/*z* (%) = 447 (100) [M+2H]²⁺, 892 (50) [M+H]⁺, MS (ESI-neg): *mlz* C₃₇H₇₀N₁₁O₁₄ ber. 892.5099, gef. 892.5120[M+H]⁺. |
| | 900 mg (33% rein) aus Beispielverbindung 302A nach Arbeitsvorschrift 9, Reinigung nach Methode 45 | |
| 326A | 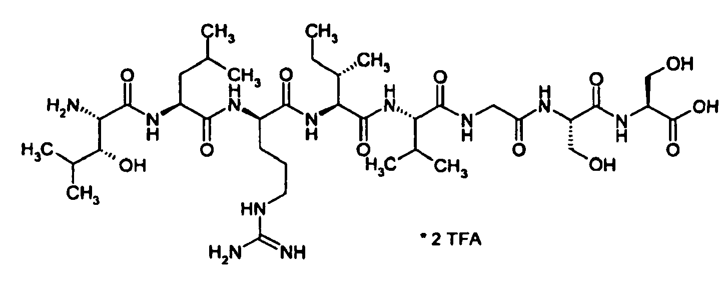 | |
| | [(3*R*)-3-hydroxy-L-leucyl]-L-leucyl-D-arginyl-L-isoleucyl-L-valyl-glycyl-L-seryl-L-serin-bis-trifluoracetat | HPLC (Methode 6): Rₜ = 2.92 min; LC-MS (Methode 22): Rₜ = 2.26 min, MS (ESIpos): *m*/*z* (%) = 430.9 (100) [M+2H]²⁺, MS (ESIneg): *m*/*z* (%) = 858.4 (100) [M-H]⁻; HR-TOF-MS: C₃₇H₇₀N₁₁O₁₂ ber. 860.5200, gef. 860.5173 [M+H]⁺. |
| | 720 mg (72% rein) aus Beispielverbindung 303A nach Arbeitsvorschrift 9, Reinigung nach Methode 45 | |
| 327A | 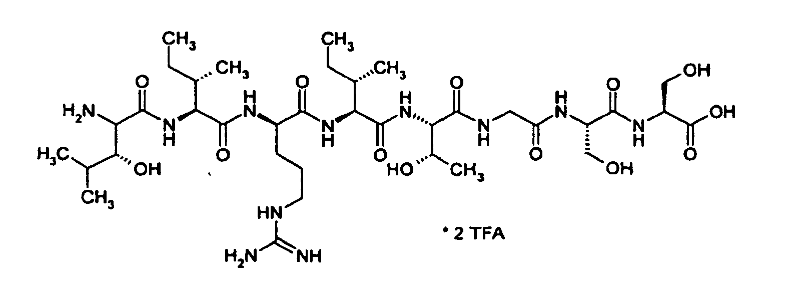 | |
| | [(3*R*)-3-hydroxy-L-leucyl]-L-isoleucyl-D-arginyl-L-isoleucyl-L-allothreonyl-glycyl-L-seryl-L-serin-bis-trifluoracetat | HPLC (Methode 6): Rₜ = 2.73 min; LC-MS (Methode 22): Rₜ = 2.08 min, MS (ESIpos): *m*/*z* (%) = 862.2 (100) [M+H]⁺, MS (ESIneg): *m*/*z* (%) = 860.2 (100) [M-H]⁻; HR-TOF-MS: C₃₆H₆₈N₁₁O₁₃ ber. 862.4993, 862.5002 [M+H]⁺. gef. |
| | 1030 mg (45% rein) aus Beispielverbindung 304A nach Arbeitsvorschrift 9, Reinigung nach Methode 45 | |
| 328A | 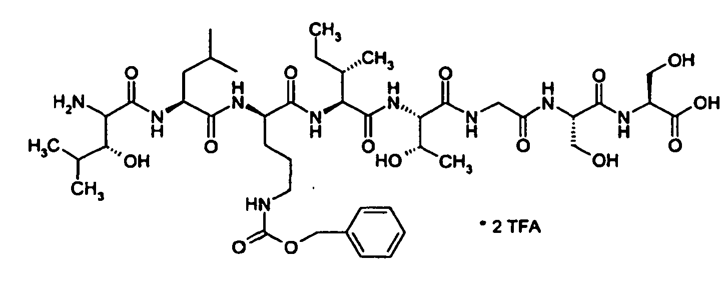 | |
| | [(3*R*)-3-hydroxy-L-leucyl]-L-leucyl-(*N*⁵-benzyloxycarbonyl-D-ornithyl)-L-isoleucyl-L-allothreonyl-glycyl-L-seryl-L-serin-bis-trifluoracetat | HPLC (Methode 6): Rₜ = 3.45 min; LC-MS (Methode 19): Rₜ = 1.66 min, MS (ESIpos): *m*/*z* (%) = 954.6 (100) [M+H]⁺, MS (ESIneg): *m*/*z* (%) = 952.5 (100) [M-H]⁻; HR-TOF-MS: C₄₃H₇₂N₉O₁₅ ber. 954.5143, gef. 954.5155 [M+H]⁺. |
| | 1090 mg (28% rein) aus Beispielverbindung 305A nach Arbeitsvorschrift 9, Reinigung nach Methode 45 | |
| 329A | 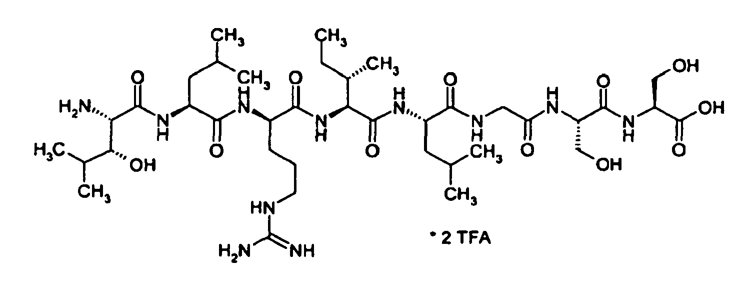 | |
| | [(3*R*)-3-hydroxy-L-leucyl]-L-leucyl-D-arginyl-L-isoleucyl-L-leucyl-glycyl-L-seryl-L-serin-bis-trifluoracetat | HPLC (Methode 6): Rₜ = 3.03 min; LC-MS (Methode 19): Rₜ = 1.17 min, MS (ESIpos): *m*/*z* (%) = 438.0 (100) [M+2H]²⁺, MS (ESIneg): *m*/*z* (%) = 954.5 (100) [M-H] ⁻; HR-TOF-MS: C₃₈H₇₂N₁₁O₁₂ ber. 874.5357, gef. 874.5373 [M+H]⁺. |
| | 680 mg (36% rein) aus Beispielverbindung 306A nach Arbeitsvorschrift 9, Reinigung nach Methode 45 | |
| 330A | 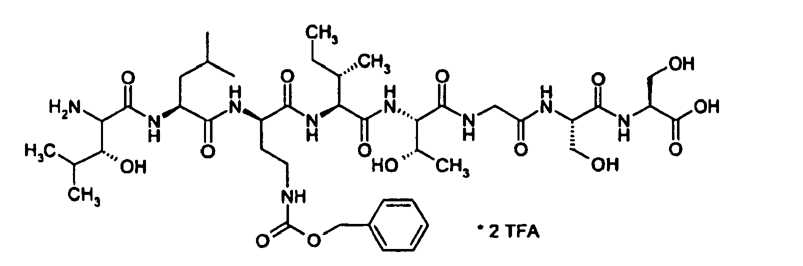 | |
| | [(3*R*)-3-hydroxy-L-leucyl]-L-leucyl-[(2R)-2-amino-4-benzyloxycarbonylamino-butyryl]-L-isoleucyl-L-allothreonyl-glycyl-L-seryl-L-serin-bis-trifluoracetat | HPLC (Methode 6): Rₜ, = 3.43 min; LC-MS (Methode 19): Rₜ = 1.64 min, MS (ESIpos): *m*/*z* (%) = 940.5 (100) [M+H]⁺, MS (ESIneg): *m*/*z* (%) = 938.6 (100) [M-H]⁻; HR-TOF-MS: C₄₂H₇₀N₉O₁₅ ber. 940.4986, gef. 940.4999 [M+H]⁺. |
| | 1120 mg (64% rein) aus Beispielverbindung 307A nach Arbeitsvorschrift 9, Reinigung nach Methode 45 | |
| 331A | 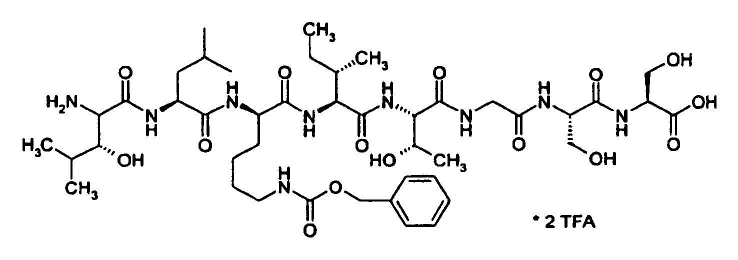 | |
| | [(3*R*)-3-hydroxy-L-leucyl]-L-leucyl-(*N*⁶-benzyloxycarbonyl-D-lysyl)-L-isoleucyl-L-allothreonyl-glycyl-L-seryl-L-serin-bis-trifluoracetat | HPLC (Methode 6): Rₜ = 3.46 min; LC-MS (Methode 19): Rₜ = 1.70 min, MS (ESIpos): *m*/*z* (%) = 968.6 (100) [M+H]⁺, MS (ESIneg): *m*/*z* (%) = 966.6 (100) [M-H]⁻; HR-TOF-MS: C₄₄H₇₄N₉O₁₅ ber. 968.5255, gef. 968.5272 [M+H]⁺. |
| | 930 mg (58% rein) aus Beispielverbindung 308A nach Arbeitsvorschrift 9. | |
| 332A | 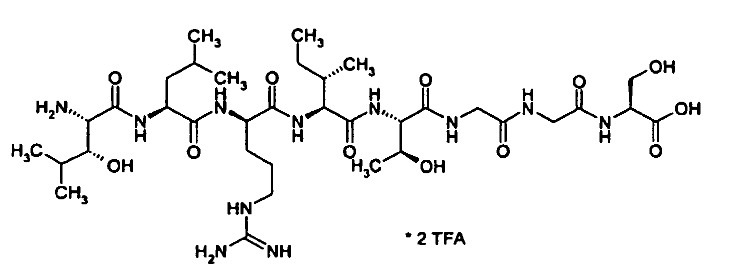 | |
| | [(3*R*)-3-hydroxy-L-leucyl]-L-leucyl-D-arginyl-L-isoleucyl-L-allothreonyl-glycyl-glycyl-L-serin-bis-trifluoracetat | HPLC (Methode 6): Rₜ = 2.82 min; LC-MS (Methode 22): Rₜ = 2.12 min, MS (ESIpos): *m*/*z* (%) = 417 (100) [M+2H]²⁺, MS (ESIneg): *m*/*z* (%) = 830 (100) [M-H]⁻; HR-TOF-MS: C₃₅H₆₆N₁₁O₁₂ ber. 832.4887, gef. 832.4868 [M+H]⁺. |
| | 1.37 g (60% rein) aus Beispielverbindung 310A nach Arbeitsvorschrift 9, Reinigung nach Methode 45 | |
| 333A | 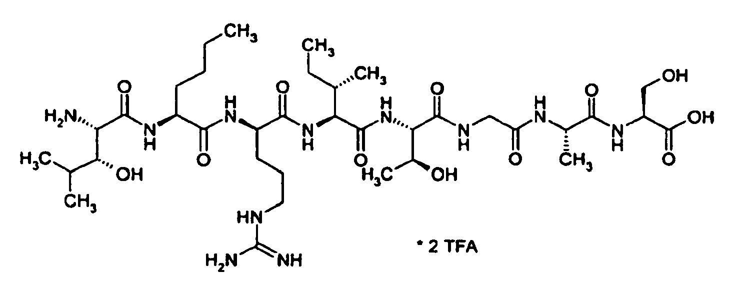 | |
| | [(3*R*)-3-hydroxy-L-leucyl]-L-norleucyl-D-arginyl-L-isoleucyl-L-allothreonyl-glycyl-L-alanyl-L-serin-bis-trifluoracetat | HPLC (Methode 6): Rₜ = 2.84 min; LC-MS (Methode 22): Rₜ = 2.16 min, MS (ESIpos): *m*/*z* (%) = 423.9 (100) [M+2H]²⁺, MS (ESIneg): *m*/*z* (%) = 844.3 (100) [M-H]⁻; HR-TOF-MS: C₃₆H₆₈N₁₁O₁₂ ber. 846.5044, gef. 846.5025 [M+H]⁺. |
| | 984 mg (60% rein) aus Beispielverbindung 309A nach Arbeitsvorschrift 9, Reinigung nach Methode 45. | |
| 334A | 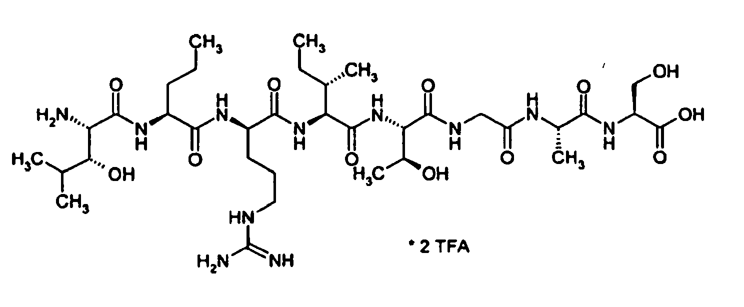 | |
| | [(3*R*)-3-hydroxy-L-leucyl]-L-norvalyl-D-arginyl-L-isoleucyl-L-allothreonyl-glycyl-L-alanyl-L-serin-bis-trifluoracetat | HPLC (Methode 6): Rₜ = 2.73 min; LC-MS (Methode 22): Rₜ = 2.04 min, MS (ESIpos): *m*/*z* (%) = 417 (100) [M+2H]²⁺, MS (ESIneg): *m*/*z* (%) = 830.4 (100) [M-H]⁻; HR-TOF-MS: C₃₅H₆₆N₁₁O₁₂ ber. 832.4887, gef. 832.4899 [M+H]⁺. |
| | 1.21 g (41% rein) aus Beispielverbindung 311A nach Arbeitsvorschrift 9, Reinigung nach Methode 45 | |

### Geschützte Nonapeptide

| **Nr** | **Struktur** | |
|---|---|---|
| | **Name** | **Analytik** |
| | **Ausbeute, Synthesemethode** | |
| 335A | 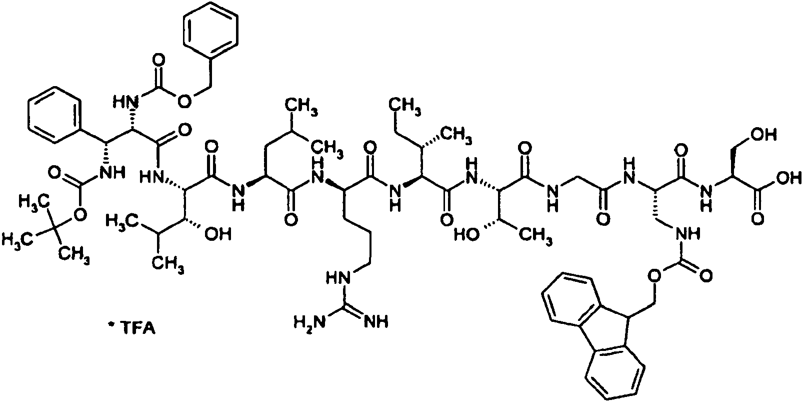 | |
| | (3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-isoleucyl-L-allothreonyl-glycyl-[(2S)-2-amino-3-(9H-fluoren-9-ylmethoxycarbonyl)aminobutyryl]-L-serin- | HPLC (Methode 6): Rₜ = 4.17 min; LC-MS (Methode 19): Rₜ 2.27 min, MS (ESIpos): *m*/*z* (%) 1481 (20) [M+H]⁺, MS (ESIneg): *m*/*z* (%) = 1478 (20) [M-H]⁻; HR-TOF-MS: C₄₈H₈₁N₁₄O₁₇ ber. 1125.5899, gef. 1125.5891 [M+H]⁺. |
| | Ausbeute: 1008 mg (83% rein, -quant) als farbloser Feststoff aus 647 mg der Beispielverbindung 312A und Beispielverbindung 17A nach Arbeitsvorschrift 10, Reinigung nach Methode 45 und Methode 44 | |
| 336A | 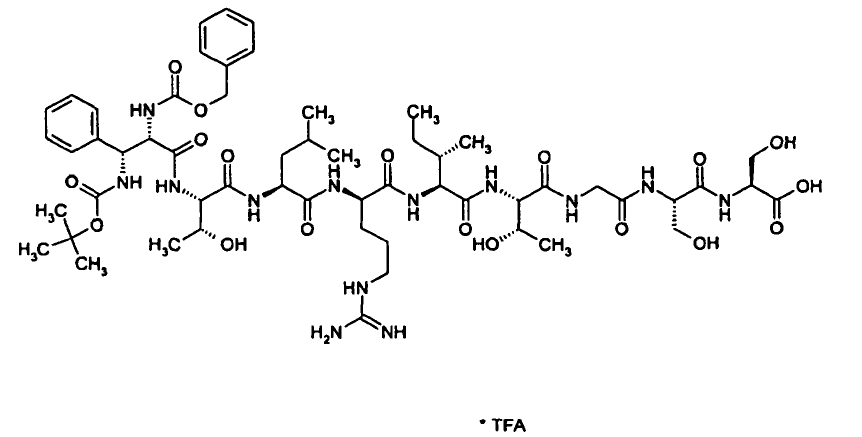 | |
| | [(3*R*)-*N*²-(Benzyloxycarbonyl)-3-{(*tert*-butoxycarbonyl)amino}-L-phenylalanyl]-L-threonyl-L-leucyl-D-arginyl-1-isoleucyl-L-allothreonyl-glycyl-L-seryl-L-serin-trifluoracetat | HPLC (Methode 6): Rₜ = 3.71 min; LC-MS (Methode 19): Rₜ = 1.89 min, MS (ESIpos): *m*/*z* (%) = 1230 (30) [M+H]⁺, MS (ESIneg): *m*/*z* (%) = 1228.7 (100) [M-H]. 45. |
| | Ausbeute: 21 mg (64% d. Th.) aus Beispielverbindung 313A (26 mg, 24 µmol) und 17A nach Arbeitsvorschrift 10, Reinigung nach Methode | |
| 337A | 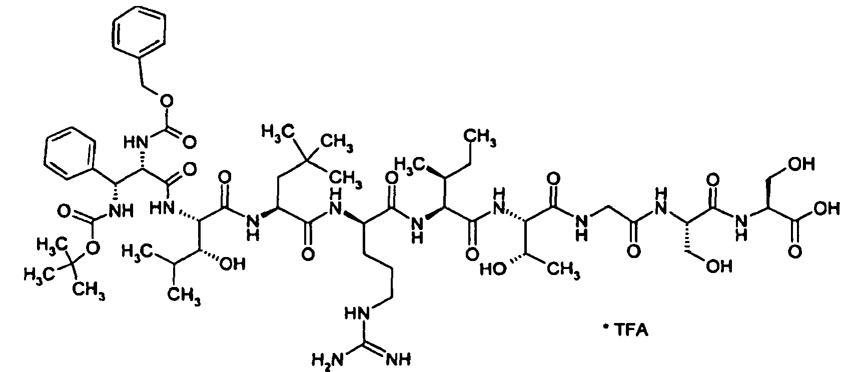 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-[3-*tert*butyl-L-alanyl]-D-arginyl-L-isoleucyl-L-allothreonyl-glycyl-L-seryl-L-serin-trifluoracetat | HPLC (Methode 6): Rₜ = 3.85 min; LC-MS (Methode 19): Rₜ = 1.93min, MS (ESIpos): *m*/*z* (%) = 1272.8 (100) [M+H]⁺, MS (ESIneg) *m*/*z* (%) = 1270.9 (100) |
| | Ausbeute: 37 mg (74% d. Th.) aus Beispielverbindung 314A (40 mg, 36 µmol) und 17A nach Arbeitsvorschrift 10, Reinigung nach Methode 45. | [M-H]⁻; HR-TOF-MS: C₅₉H₉₄N₁₃O₁₈ ber. 1272.6835, gef. 1272.6824 [M+H]⁺. |
| 338A | 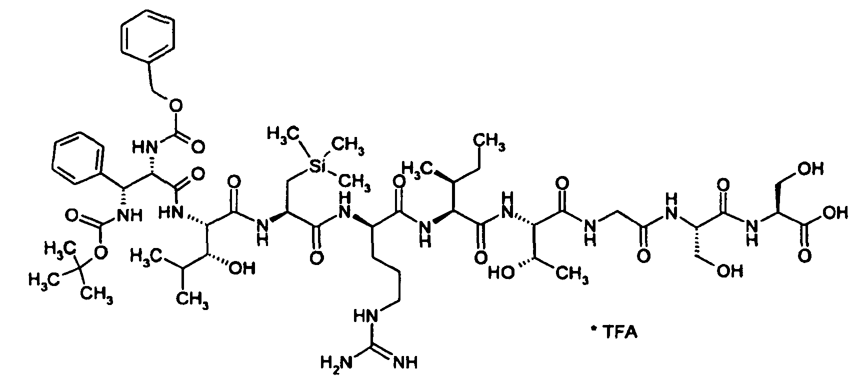 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-(3-trimethylsilyl-L-alanyl)-D-arginyl-L-isoleucyl-l-allothreonyl-glycyl-L-seryl-L-serin-trifluoracetat | HPLC (Methode 6): Rₜ = 3.90 min; LC-MS (Methode 19): Rₜ = 1.99 min, MS (ESIpos): *m*/*z* (%) = 1288.9 (40) [M+H]⁺; HR-TOF-MS: C₅₈H₉₄N₁₃O₁₉Si ber. 1288.6604, gef. 1288.6608 [M+H]⁺. |
| | Ausbeute: 56 mg (85% d. Th.) aus Beispielverbindung 315A (53 mg, 47 µmol) und 17A nach Arbeitsvorschrift 10, Reinigung nach Methode 45 | |
| 339A | 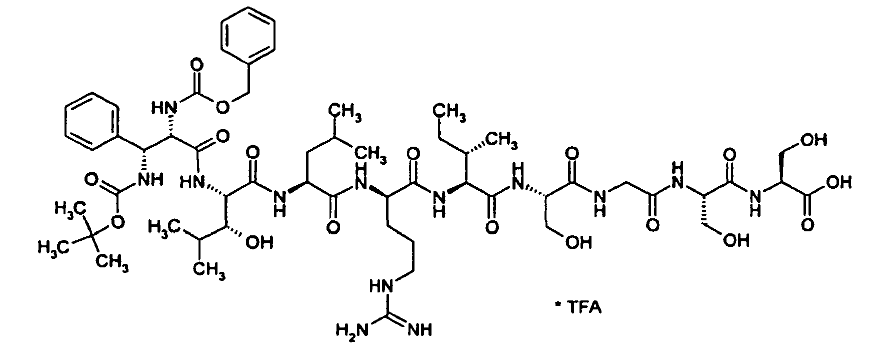 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-isoleucyl-L-seryl-glycyl-L-seryl-L-serin-trifluoracetat | HPLC (Methode 6): Rₜ = 3.78 min; LC-MS (Methode 19): Rₜ = 1.95 min, MS (ESIpos): *m*/*z* (%) = 1244.8 (30) [M+H]⁺, MS (ESIneg): *m*/*z* (%) = 1242.8 (100) |
| | Ausbeute: 42 mg (30% d. Th.) aus Beispielverbindung 316A (108 mg, 100 µmol) und 17A nach Arbeitsvorschrift 10, Reinigung nach Methode 45 und Methode 32 | [M-H]⁻; HR-TOF-MS:C₅₇H₉₀N₁₃O₁₈ ber. 1244.6522, gef. 1244.6544 [M+H]⁺. |
| 340A | 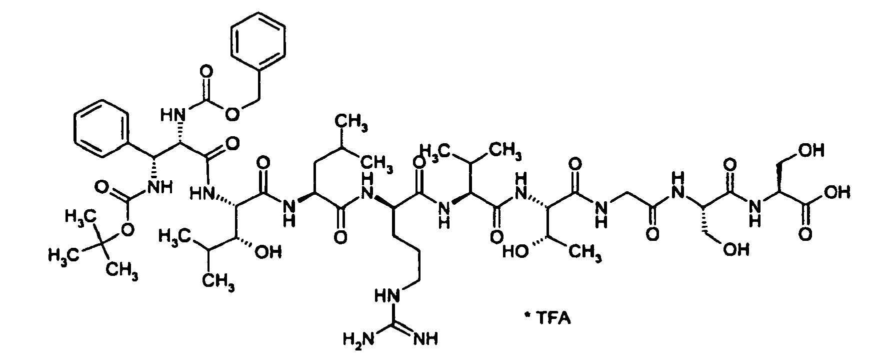 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-valyl-L-allothreonyl-glycyl-L-seryl-L-serin-trifluoracetat | HPLC (Methode 6): Rₜ = 3.75 min; LC-MS (Methode 19): Rₜ = 1.93 min, MS (ESIpos): *m*/*z* (%) = 1244.7 (30) [M+H]⁺, MS (ESIneg): *m*/*z* (%) = 1242.8 (100) [M-H]⁻; HR-TOF-MS:C₅₇H₉₀N₁₃O₁₈ ber. 1244.6522, gef. 1244.6490 [M+H]⁺. |
| | Ausbeute: 48 mg (42% d. Th.) aus Beispielverbindung 317A (90 mg, 84 µmol) und 17A nach Arbeitsvorschrift 10, Reinigung nach Methode 44 | |
| 341A | 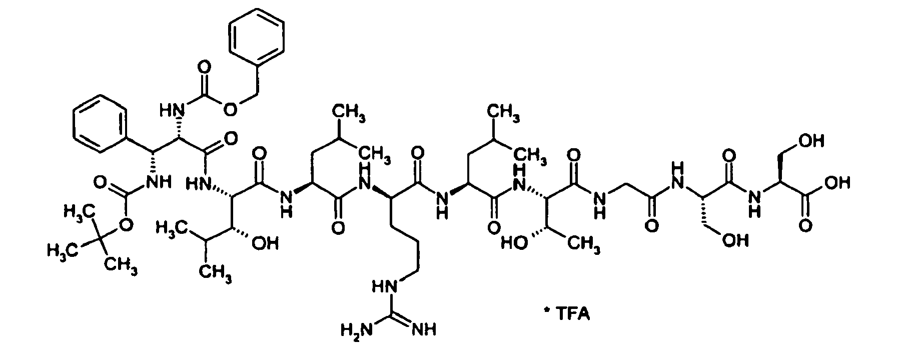 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-leucyl-L-allothreonyl-glycyl-L-seryl-L-serin-trifluoracetat | HPLC (Methode 6): Rₜ = 3.81 min; LC-MS (Methode 19): Rₜ = 1.98 min, MS (ESIpos): *m*/*z* (%) = 1258.8 (50) [M+H]⁺, MS (ESIneg): *m*/*z* (%) = 1256.8 (90) |
| | Ausbeute: 49 mg (43% d. Th.) aus Beispielverbindung 318A (90 mg, 83 µmol) und 17A nach Arbeitsvorschrift 10, Reinigung nach Methode 44 | [M-H] ⁻; HR-TOF-MS: C₅₈H₉₂N₁₃O₁₈ ber. 1258.6678, gef. 1258.6677 [M+H]⁺. |
| 342A | 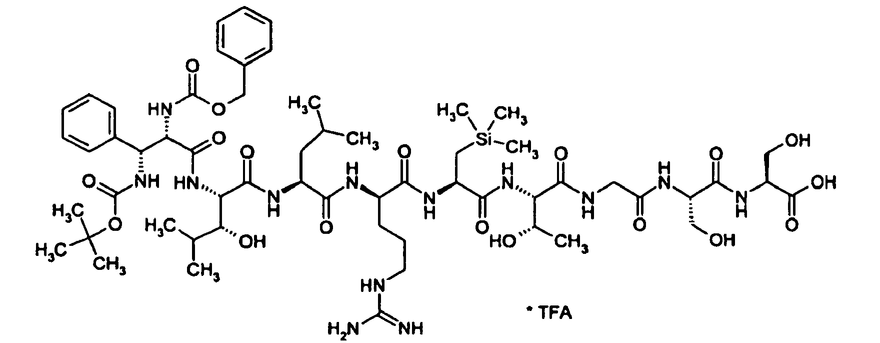 | |
| | [(3*R*)-*N*²-(Benzyloxycarbonyl)-3-{(*tert*butoxycarbonyl)amino}1-L-phenylalanyl]-[(3*R*)-3-hydroxy-L-leucyl]-L-leucyl-D-arginyl-[3-trimethylsilyl-L-alanlyl]-L-allothreonyl-glycyl-L-seryl-L-serin-trifluoracetat | HPLC (Methode 6): Rₜ = 3.89 min; LC-MS (Methode 19): Rₜ = 2.06 min, MS (ESIpos): *m*/*z* (%) = 1288.7 (40) [M+H]⁺, MS (ESIneg): *m*/*z* (%) = 1286.7 (100) [M-H]⁻: HR-TOF-MS: C₅₈H₉₄N₁₃O₁₈Si ber. 1288.6604, gef. 1288.6587 [M+H]⁺. |
| | Ausbeute: 42 mg (33% d. Th.) aus Beispielverbindung 319A (90 mg, 80 µmol) und 17A nach Arbeitsvorschrift 10, Reinigung nach Methode 44 | |
| 343A | 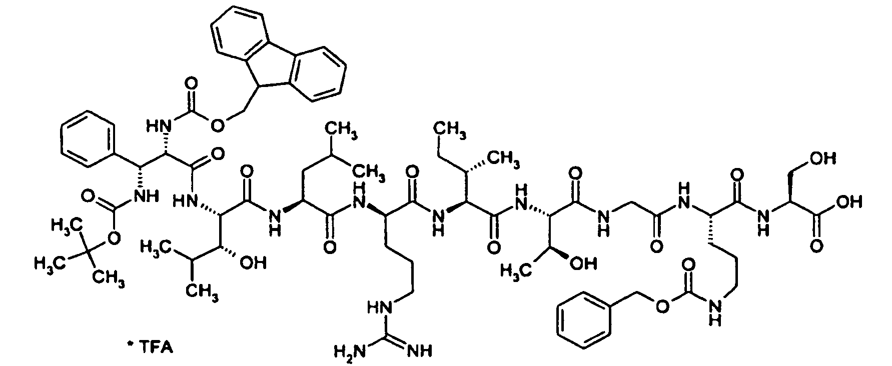 | |
| | {(3*R*)-*N*²-[(9H-fluoren-9-ylmethoxy)carbonyl]-3-[(*tert*-butoxycarbonyl)amino]-L-phenylalanyl}-[(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-trifluoracetat | HPLC (Methode 6): Rₜ = 4.21 min. |
| | Ausbeute: 42 mg (33% d. Th.) aus Beispielverbindung 320A (90 mg, 80 µmol) und 277A nach Arbeitsvorschrift 10, Reinigung nach Methode 44 | |
| 344A | 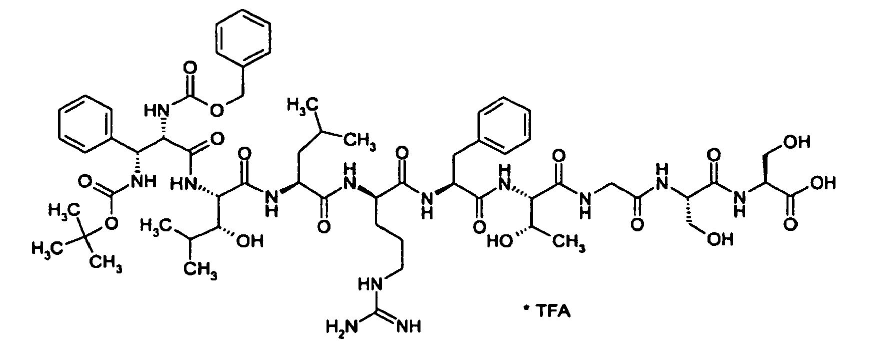 | |
| | glycyl-L-seryl-L-serin-trifluoracetat [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-phenylalanlyl-L-allothreonylglycyl-L-seryl-L-serin-trifluoracetat | HPLC (Methode 6): Rₜ = 3.80 min; LC-MS (Methode 19): Rₜ = 1.97 min, MS (ESIpos): *m*/*z* (%) = 1292.7 (30) [M+H]⁺, MS (ESIneg): *m*/*z* (%) = 1290.7 (80) [M-H]⁻. |
| | Ausbeute: 55 mg (49% d. Th.) aus Beispielverbindung 321A (90 mg, 80 µmol) und 17A nach Arbeitsvorschrift 10, Reinigung nach Methode 44 | |
| 345A | 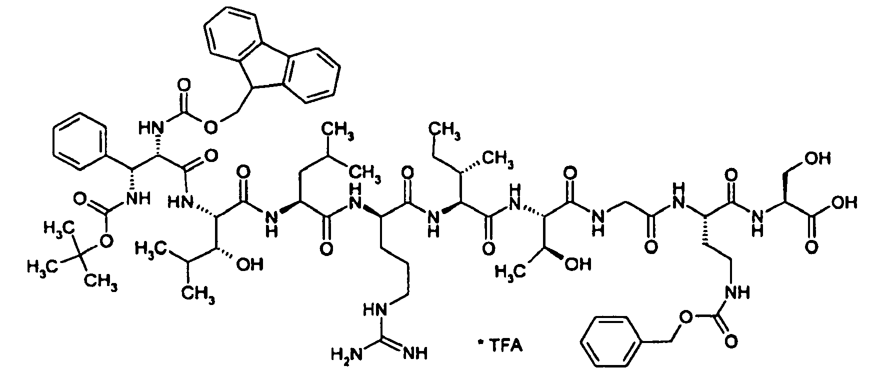 | |
| | {(3*R*)-*N*²-[(9H-fluoren-9-ylmethoxy)carbonyl]-3-[(*tert*-butoxycarbonyl)amino]-L-phenylalanyl}-[(3*R*)-3-hydroxy-L-leucyl]-L-leucyl-D-arginyl-L-isoleucyl-L-allothreonyl-glycyl-[(2S)-2-amino-4-benzyloxycarbonylamino-butyryl]-L-serin-trifluoracetat | HPLC (Methode 6): Rₜ = 4.20 min; LC-MS (Methode 19): Rₜ = 2.28 min, MS (ESIpos): *m*/*z* (%) = 1494.7 (20) [M+H]⁺, MS (ESIneg): *m*/*z* (%) = 1493.1 (50) [M-H]⁻; HR-TOF-MS: C₇₄H₁₀₅N₁₄O₁₉ ber. 1493.7675, gef. 1493.7666 |
| | Ausbeute: 57 mg (27% d. Th.) aus Beispielverbindung 322A (100 mg, 81 µmol) und 277A nach Arbeitsvorschrift 10, Reinigung nach Methode 44 | [M+H]⁺. |
| 346A | 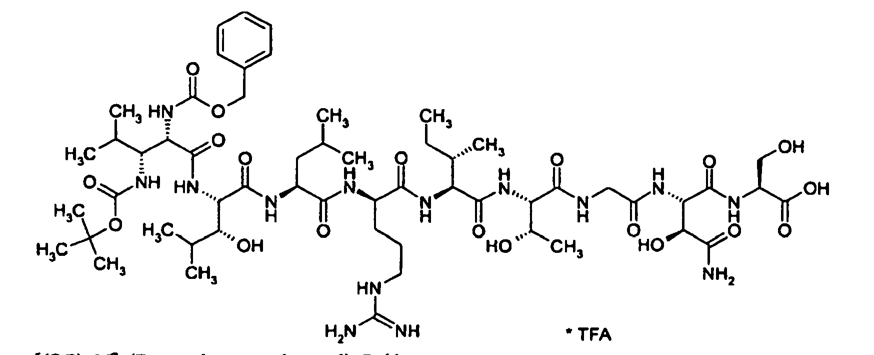 | |
| | [(3*R*)-*N*²-(Benzyloxycarbonyl)-3-{(*tert*-butoxycarbonyl)amino}-L-leucyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-trifluoracetat | HPLC (Methode 6): Rₜ = 3.76 min; LC-MS (Methode 19): Rₜ = 1.95 min, MS (ESIpos): *m*/*z* (%) = 1267.8 (20) [M+H]⁺, MS (ESIneg): *m*/*z* (%) = 1265.9 (100) |
| | Ausbeute: 311 mg (94% d. Th.) aus Beispielverbindung 3A (272 mg, 240 µmol) und 282A nach Arbeitsvorschrift 10, Reinigung nach Methode 45 | [M-H]⁻; HR-TOF-MS: C₅₆H₉₅N₁₄O₁₉ ber. 1267.6893, gef. 1267.6907 [M+H]⁺. |
| 347A | 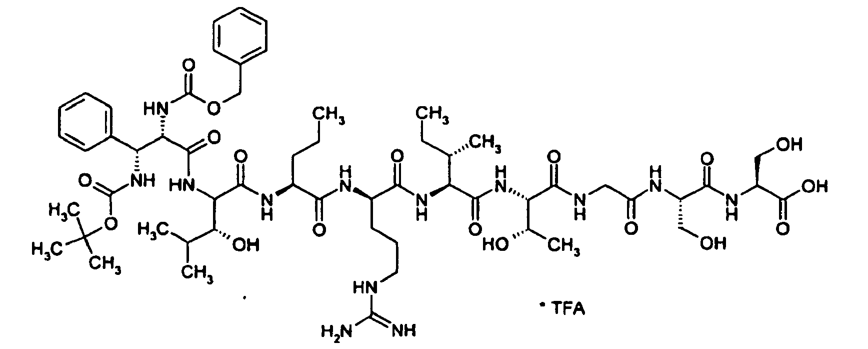 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-norvalyl-D-arginyl-L-isoleucyl-L-allothreonyl-glycyl-L-seryl-L-serin-trifluoracetat | HPLC (Methode 6): Rₜ = 3.75 min; LC-MS (Methode 19): Rₜ = 1.94 min, MS (ESIpos): *m*/*z* (%) = 1244.7 (30) [M+H]⁺, MS (ESIneg): *m*/*z* (%) = 1242.8 (100) |
| | Ausbeute: 173 mg (91% d. Th.) aus Beispielverbindung 323A (150 mg, 139 mmol) und 17A nach Arbeitsvorschrift 10, Reinigung nach Methode 44 | [M-H]⁻; HR-TOF-MS: C₅₇H₉₀N₁₃O₁₈ ber. 1244.6522, gef. 1244.6555 [M+H]⁺. |
| 348A | 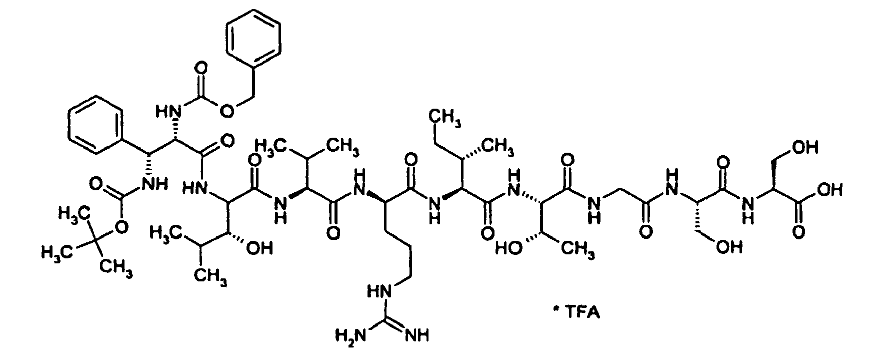 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-valyl-D-arginyl-L-isoleucyl-L-allothreonyl-glycyl-L-seryl-L-serin-trifluoracetat | HPLC (Methode 6): Rₜ = 3.74 min; LC-MS (Methode 19): Rₜ = 1.94 min, MS (ESIPOS): *m*/*z* (%) = 1244.7 (30) [M+H]⁺, MS (ESIneg): *m*/*z* (%) = 1243.7 (40) [M-H]⁻; HR-TOF-MS: C₅₇H₉₀N₁₃O₁₈ ber. 1244.6522, gef. 1244.6528 10, [M+H]⁺. |
| | Ausbeute: 61 mg (32% d. Th.) aus Beispielverbindung 324A (150 mg, 139 µmol) und 17A nach Arbeitsvorschrift Reinigung nach Methode 34 | |
| 349A | 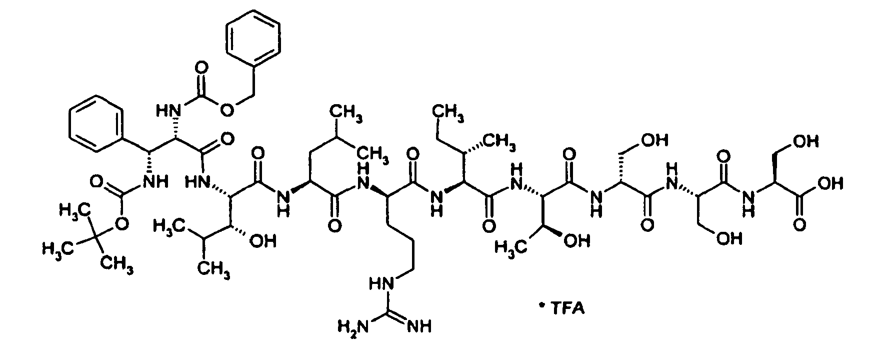 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-isoleucyl-L-allothreonyl-D-seryl-L-seryl-L-serin-trifluoracetat | HPLC (Methode 6): Rₜ = 3.78 min; LC-MS (Methode 19): Rₜ = 1.97 (%) = 1288.7 (40) [M+H]⁺, MS (ESIneg): *m*/*z* (%) = 1286.6 (100) [M-H]⁻; HR-TOF-MS: C₅₉H₉₄N₁₃O₁₉ ber. 1288.6784, gef. 1288.6792 [M+H]⁺. |
| | Ausbeute: 73 mg (29% d. Th.) aus Beispielverbindung 325A (200 mg, 179 µmol) und 17A nach Arbeitsvorschrift 10, Reinigung nach Methode 44. | |
| 350A | 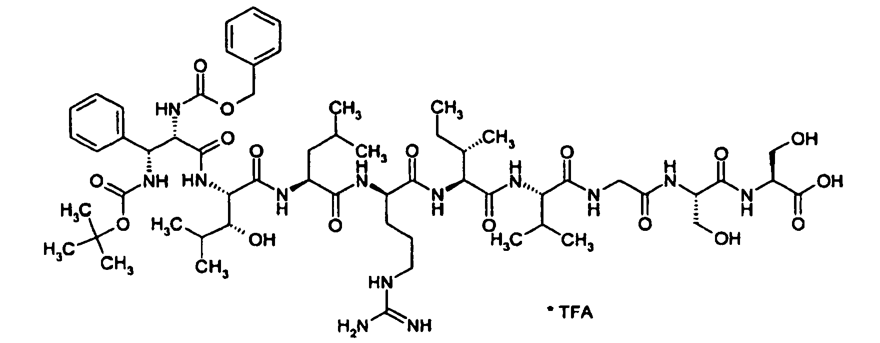 | |
| | [(3*R*)-*N*²-(Benzyloxycarbonyl)-3-{(*tert*butoxycarbonyl)amino}-L-phenylalanyl]-[(3*R*)-3-hydroxy-1,-leucyl]-L-leucyl-Darginyl-L-isoleucyl-L-valyl-glycyl-L-seryl-L-serin-trifluoracetat | HPLC (Methode 6): Rₜ = 3.88 min; LC-MS (Methode 19): Rₜ = 2.02 min, MS (ESIpos): *m*/*z* (%) =1256.8 (20) [M+H]⁺, MS (ESIneg): *m*/*z* (%) = 1254.8 (100) [M-H]⁻. |
| | Ausbeute: 44 mg (24% d. Th.) aus Beispielverbindung 326A (200 mg, 132 µmol) und 17A nach Arbeitsvorschrift 10, Reinigung nach Methode 44. | |
| 351A | 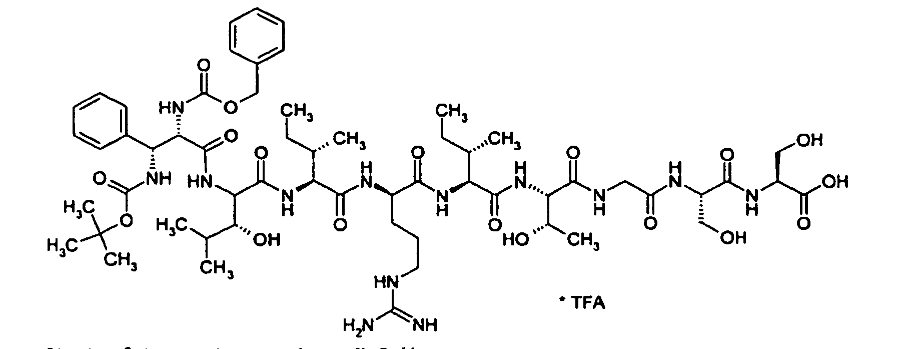 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-isoleucyl-D-arginyl-L-isoleucyl-L-allothreonyl-glycyl-L-seryl-L-serin-trifluoracetat | HPLC (Methode 6): Rₜ = 3.79 min; LC-MS (Methode 51): Rₜ = 2.98 min, MS (ESIpos): *m*/*z* (%)= 1258.9 (10) [M+H]⁺, MS (ESIneg): *m*/*z* (%) [M+H]⁺, (5) [M-H] ⁻; HR-TOF-MS: C₅₈H₉₂N₁₃O₁₈ ber. 1258.6678, gef. 1258.6698 [M+H]⁺. |
| | Ausbeute: 39 mg (18% d. Th.) aus Beispielverbindung 327A(175 mg, 161 µmol) und 17A nach Arbeitsvorschrift 10, Reinigung nach Methode 32. | |
| 352A | 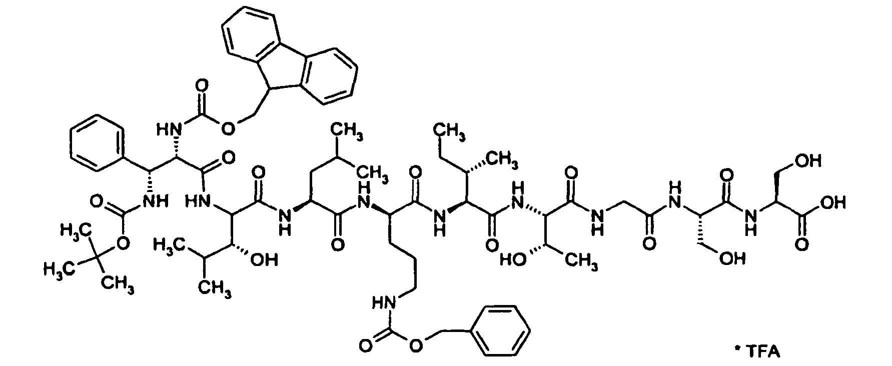 | |
| | [(3*R*)-*N*²-[(9H-fluoren-9-ylmethoxy)carbonyl]-3-[(*tert*-butoxycarbonyl)amino]L-phenylalanyl}-[(3*R*)-3-hydroxy-L-leucyl]-L-leucyl-(*N*⁵-benzyloxycarbonyl-D-ornithyl)-L-isoleucyl-L-allothreonyl-glycyl-L-seryl-L-serin-trifluoracetat | HPLC (Methode 6): Rₜ = 4.70 min; LC-MS (Methode 19): Rₜ = 3.26 min, MS (ESIpos): *m*/*z* (%) = 1439.7 (100) [M+H]⁺, MS (ESIneg): *m*/*z* (%) = 1437.8 (80) [M-H]⁻; HR-TOF-MS: C₇₂H₁₀₀N₁₁O₂₀ ber. 1438.7148, gef. 1438.7141 [M+H]⁺. |
| | Ausbeute: 103 mg (33% rein, 17% d. Th.) aus Beispielverbindung 328A (150 mg, 140 µmol) und 277A nach Arbeitsvorschrift 10, Reinigung nach Methode 34. | |
| 353A | 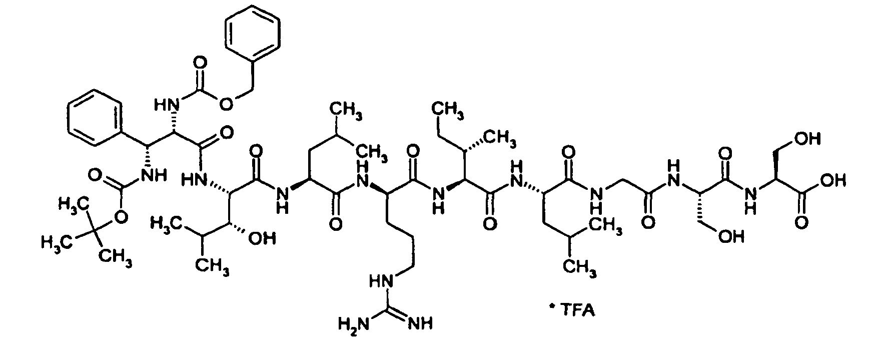 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-L-leucyl-D-arginyl-L-isoleucyl-L-leucyl-glycyl-L-seryl-L-serin-trifluoracetat | HPLC (Methode 6): Rₜ = 3.89 min; LC-MS (Methode 19): Rₜ = 2.08 min, MS (ESIpos): *m*/*z* (%) = 1270.8 (100) [M+H]⁺, MS (ESIneg): *m*/*z* (%) = 1268.8 (100) [M-H]⁻; HR-TOF-MS: C₆₀H₉₆N₁₃O₁₇ ber. 1270.7042, gef 1270.7047 [M+H]⁺. |
| | Ausbeute: 14 mg (14% d. Th.) aus Beispielverbindung 329A (130 mg, 71 µmol) und 17A nach Arbeitsvorschrift 10, Reinigung nach Methode 32. | |
| 354A | 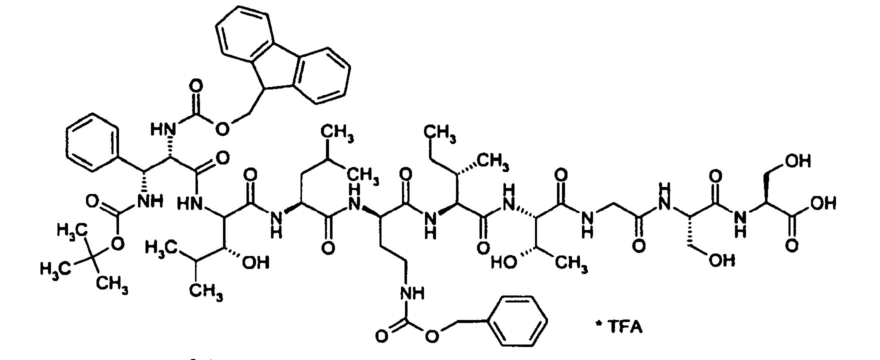 | |
| | {(3*R*)-*N*²-[(9H-fluoren-9-ylmethoxy)carbonyl]-3-[(*tert*-butoxycarbonyl)amino]-L-phenylalanyl}-[(3*R*)-3-hydroxy-L-leucyl]-L-leucyl-[(2*R*)-2-amino-4-benzyloxycarbonylamino-butyryl]-L-isoleucyl-L-allothreonyl-glycyl-L-seryl-L-serin-trifluoracetat | HPLC (Methode 6): Rₜ = 4.71 min; LC-MS (Methode 19): Rₜ = 3.23 min, MS (ESIpos): *m*/*z* (%) = 1425.5 (100) [M+H]⁺, MS (ESIneg): *m*/*z* (%) = [M+H]⁺ , (100) [M-H]⁻; HR-TOF-MS: C₇₁H₉₈N₁₁O₂₀ ber. 1424.6985, gef. 1424.6980 [M+H]⁺. |
| | Ausbeute: 106 mg (60% rein, 31% d. Th.) aus Beispielverbindung 330A (150 mg, 142 µmol) und 277A nach Arbeitsvorschrift 10, Reinigung nach Methode 34. | |
| 355A | 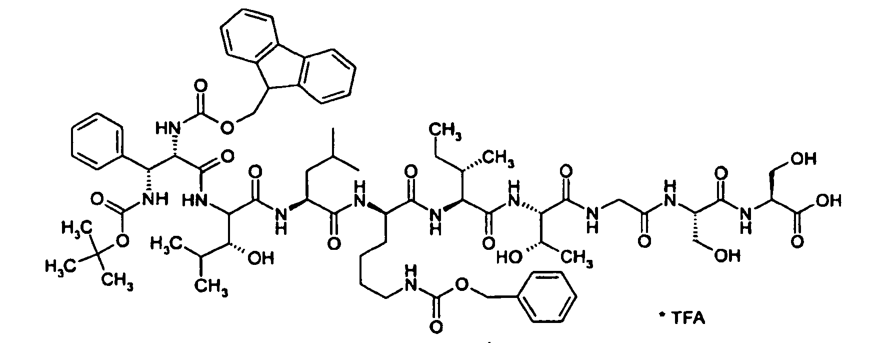 | |
| | {(3*R*)-*N*²-[(9H-fluoren-9-ylmethoxy)carbonyl]-3-[(*tert*-butoxycarbonyl)amino]-L-phenylalanyl}-[(3*R*)-3-hydroxy-L-leucyl]-L-leucyl-(*N*⁶-benzyloxycarbonyl-D-lysyl)-L-isoteucyl-L-allothreonyl-glycyl-L-seryl-L-serin-trifluoracetat | HPLC (Methode 6): Rₜ = 4.71 min; LC-MS (Methode 19): Rₜ = 3.35 min, MS (ESIpos): *m*/*z* (%) = 1452.7 (90) [M+H]⁺, MS (ESIneg): *m*/*z* (%) = 1451.7 (100) [M-H]⁻; HR-TOF-MS: C₇₃H₁₀₂N₁₁O₂₀ ber. 1452.7298, gef. 1452.7283 |
| | Ausbeute: 49 mg (21% d. Th.) aus Beispielverbindung 331A (180 mg, 150 µmol) und 277A nach Arbeitsvorschrift 10, Reinigung nach Methode 32. | [M+H]⁺. |
| 356A | 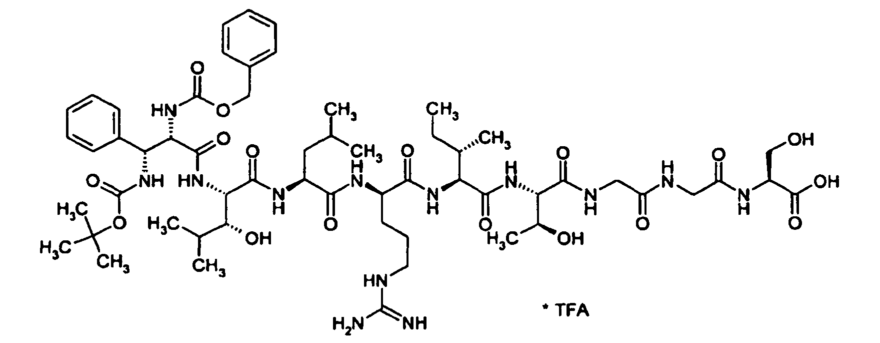 | |
| | [(3*R*)-*N*²-(Benzyloxycarbonyl)-3-{(*tert*-butoxycarbonyl)aminol-L-phenylalanyl]-[(*3R*)-3-hydroxy-L-leucyl]-L-leucyl-D-arginyl-L-isoleucyl-L-allothreonyl-glycyl-glycyl-L-serin-trifluoracetat | HPLC (Methode 6): Rₜ = 3.83 min; HPLC (Methode 6): 19): Rₜ = 1.96 min, MS (ESIpos): *m*/*z* (%) = 1228.7 (20) [M+H]⁺, MS (ESIneg): *m*/*z* (%) = 1226.8 (100) [M-H]⁻; HR-TOF-MS: C₅₇H₉₀N₁₃O₁₇ ber. 1228.6573, gef. 1228.6533 [M+H]⁺. |
| | Ausbeute: 133 mg (60% rein, 84% d. Th.) aus Beispielverbindung 332A (100 mg, 70 µmol) und 17A nach Arbeitsvorschrift 10, Reinigung nach Methode 32. | |
| 357A | 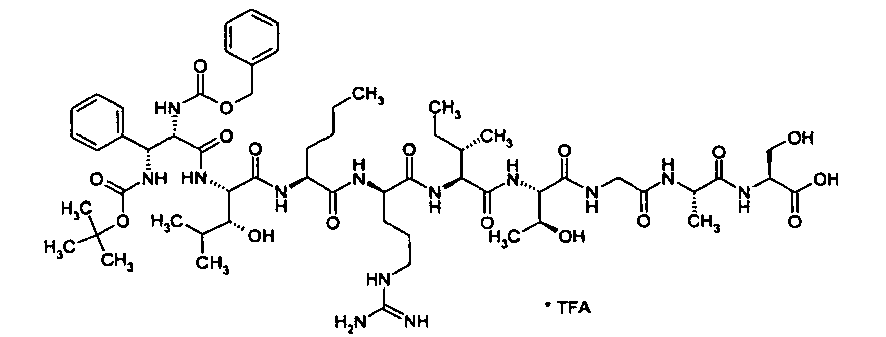 | |
| | [(3*R*)-*N*²-(Benzyloxycarbonyl)-3-{(*tert*-butoxycarbonyl)amino}-L-phenylalanyl]-[(3*R*)-3-hydroxy-L-leucyl]-t,-norleucyl-D-arginyl-L-isoleucyl-L-allothreonyl-glycyl-L-alanyl-L-serin-trifluoracetat | HPLC (Methode 6): Rₜ = 3.83 min; LC-MS (Methode 52): Rₜ = 1.51 min, MS (ESIpos): *m*/*z* (%) = 1242.8 (90) [M+H]⁺, MS (ESIneg): *m*/*z* (%) = 1241.8 (100) [M-H]⁻; HR-TOF-MS: C₅₈H₉₂N₁₃O₁₇ ber. 1242.6729, gef. 1242.6741 [M+H]⁺. |
| | Ausbeute: 95 mg (25% d. Th.) aus Beispielverbindung 333A (300 mg, 279 µmol) und 17A nach Arbeitsvorschrift 10, Reinigung nach Methode 34. | |
| 358A | 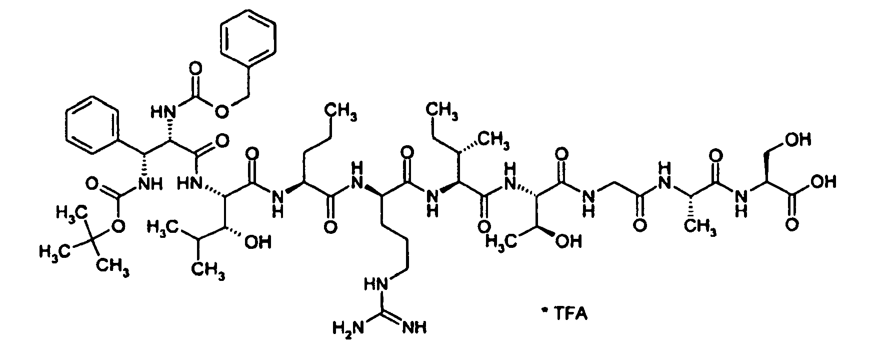 | |
| | [(3*R*)-*N*²-(Benzyloxycarbonyl)-3-1(*tert*-butoxycarbonyl)amino}-L-phenylalanyl]-[(*3R*)-3-hydroxy-L-leucyl]-L-norvalyl-D-arginyl-L-isoleucyl-L-allothreonyl-glycyl-L-alanyl-L-serin-trifluoracetat | HPLC (Methode 6): Rₜ = 3.78 min; LC-MS (Methode 19): Rₜ = 1.94 min, MS (ESIpos): *m*/*z* (%) = 1228.7 (60)[M+H]⁺, MS (ESIneg): *m*/*z* (%) = 1226.7 (100) [M-H]⁻; HR-TOF-MS: C₅₇H₉₀N₁₃O₁₇ ber. 1228.6573, gef. 1228.6592 [M+H]⁺. |
| | Ausbeute: 87 mg (23% d. Th.) aus Beispielverbindung 334A (300 mg, 283 µmol) und 17A nach Arbeitsvorschrift 10, Reinigung nach Methode 34. | |

### Teilweise entschützte Nonapeptide

| Nr | Struktur | |
|---|---|---|
| | Name | Analytik |
| | Ausbeute, Synthesemethode | |
| 359A | 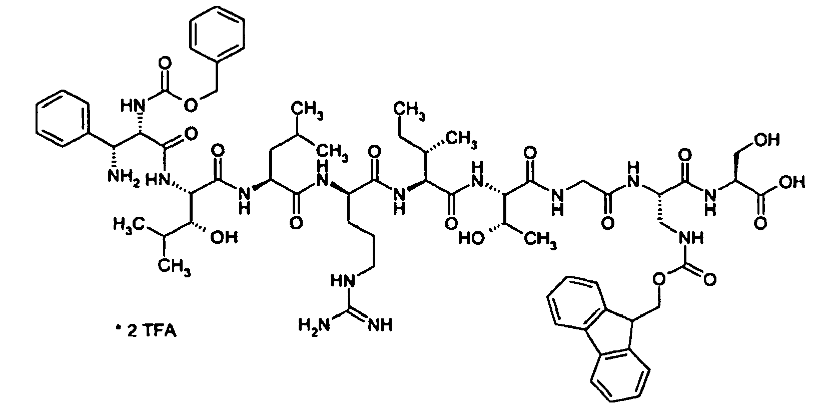 | |
| | [(3R)-N²-(Benzyloxycarbonyl)-3-amino-L-phenylalanyl]-[(3R)-3-hydroxy-L-leucyl]-L-leucyl-D-arginyl-L-isoleucyl-L-allothreonyl-glycyl-[(2S)-2-amino-3-(9H-fluoren-9-ylmethoxycarbonyl)aminobutyryl]-L-serin-bis-trifluoracetat | HPLC (Methode 6): Rₜ = 3.60 min; LC-MS (Methode 51): Rₜ = 2.72 min, MS (ESIpos): *m*/*z* (%) = 691.2 (100) [M+2H]²⁺; HR-TOF-MS: C₆₈H₉₅N₁₄O₁₇ ber. 1379.6995, gef. 1379.6998 [M+H]⁺. |
| | Ausbeute: 1110 mg (-quant) als farbloser Feststoff aus 1070 mg der Beispielverbindung 335A nach Arbeitsvorschrift 2, Rohprodukt ohne Aufreinigung weiter umgesetzt. | |
| 360A | 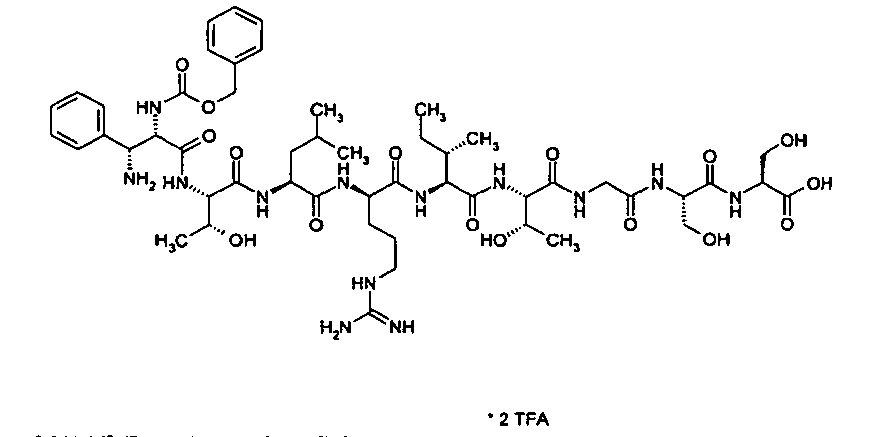 | |
| | [(3R)-N²-(Benzyloxycarbonyl)-3-amino-L-phenylalanyl]-L-threonyl-L-leucyl-D-arginyl-L-isoleucyl-L-allothreonyl-glycyl-L-seryl-L-serin-bis-trifluoracetat | HPLC (Methode 6): Rₜ = 3.11 min; LC-MS (Methode 20): Rₜ = 1.13 min, MS (ESIpos): *m*/*z* (%) = 566.0 (100) [M+2H]²⁺; MS (ESIneg) *m*/*z* (%) = 1128.8 (100) [M-H]⁻; HR-TOF-MS: C₅₁H₈₀N₁₃O₁₆ ber. 1130.5841, gef. 1130.5847 [M+H]⁺. |
| | Ausbeute: 21 mg (99% d. Th.) als farbloser Feststoff aus 21 mg der Beispielverbindung 336A nach Arbeitsvorschrift 2, Rohprodukt ohne Aufreinigung weiter umgesetzt. | |
| 361A | 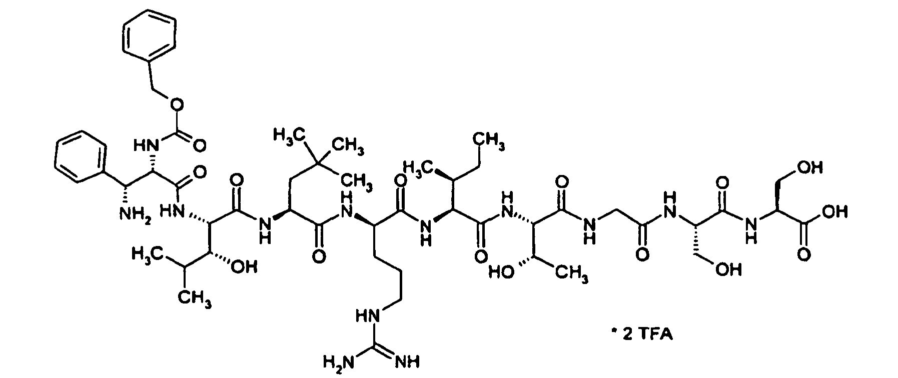 | |
| | [(3R)-N²-(Benzyloxycarbonyl)-3-amino-L-phenylalanyl]-[(3R)-3-hydroxy-L-leucyl]-[3-tertbutyl-L-alanyl]-D-arginyl-L-isoleucyl-L-allothreonyl-glyCyl-L-Seryl-L-serin-bis-trifluoracetat | HPLC (Methode 6): Rₜ = 3.28 min; LC-MS (Methode 19): Rₜ = 1.34 min, MS (ESIpos): *m*/*z* (%) = 587.2 (100) [M+2H]²⁺, 1172.8 (10) [M+H]⁺, MS (ESIneg): *m*/*z* (%) = 1170.8 (100) [M-H]⁻; HR-TOF-MS: C₅₄H₈₆N₁₃O₁₆ ber. 1172.6310, gef. 1172.6310 [M+H]⁺. |
| | Ausbeute: 37 mg (99% d. Th.) als farbloser Feststoff aus 37 mg der Beispielverbindung 337A nach Arbeitsvorschrift 2, Rohprodukt ohne Aufreinigung weiter umgesetzt. | |
| 362A | 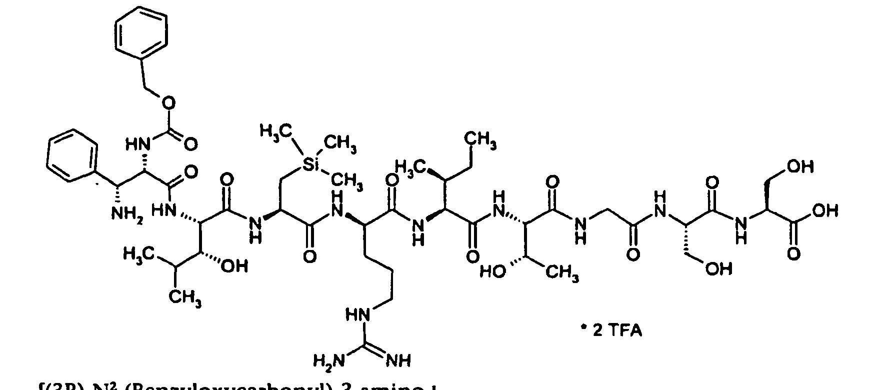 | |
| | [(3R)-N²-(Benzyloxycarbonyl)-3-amino-L-phenylalanyl]-[(3R)-3-hydroxy-L-leucyl]-(3-trimethylsilyl-L-alanyl)-D-arginyl-L-isoleucyl-L-allothreonyl-glycyl-L-seryl-L-serin-bis-trifluoracetat | HPLC (Methode 6): Rₜ = 3.33 min; LC-MS (Methode 22): Rₜ = 2.73 min, MS (ESIpos): *m*/*z* (%) = 595.3 (100) [M+2H]²⁺, 1189.0 (10) [M+H]⁺, MS (ESIneg): *m*/*z* (%) = 1186.9 (100) [M-H]⁻; HR-TOF-MS: C₅₃H₈₆N₁₃O₁₆Si ber. 1188.6080, gef. 1188.6074 [M+H]⁺. |
| | Ausbeute: 54 mg (97% d. Th.) als farbloser Feststoff aus 55 mg der Beispielverbindung . 338A nach Arbeitsvorschrift 2, Rohprodukt ohne Aufreinigung weiter umgesetzt. | |
| 363A | 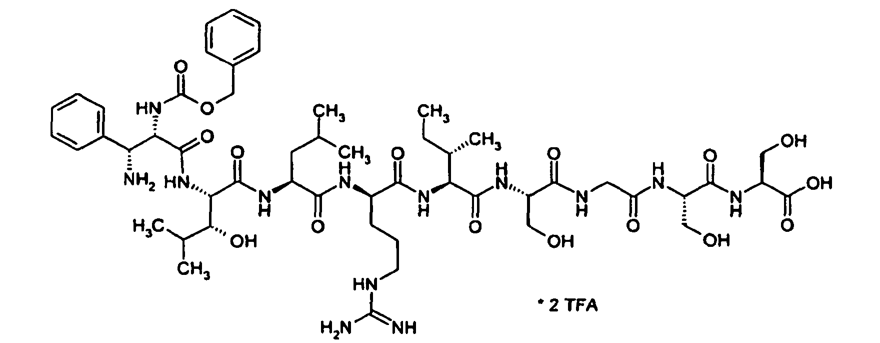 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-seryl-glycyl-L-seryl-L-serin-bis- trifluoracetat | HPLC (Methode 6): Rₜ = 3.22 min; LC-MS (Methode 19): Rₜ = 1.35 min, MS (ESIpos): *m*/*z* (%) = 573.0 (100) [M+2H]²⁺, MS (ESIneg): *m*/*z* (%) = 1142.8 (100) [M-H]⁻; HR-TOF-MS: C₅₂H₈₂N₁₃O₁₆ ber. 1144.5997, gef. 1144.5963 [M+H]⁺. |
| | Ausbeute: 48 mg (94% rein, -quant.) als farbloser Feststoff aus 42 mg der Beispielverbindung 339A nach Arbeitsvorschrift 2, Rohprodukt ohne Aufreinigung weiter umgesetzt. | |
| 364A | 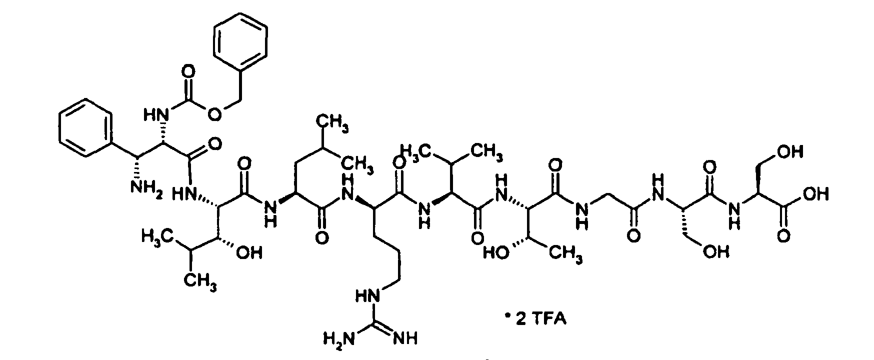 | |
| | [(3R)-N²-(Benzyloxycarbonyl)-3-amino-L-phenylalanyl]-[(3R)-3-hydroxy-L-leucyl]-L-leucyl-D-arginyl-L-valyl-L-allothreonyl-glycyl-L-seryl-L-serin-bis-trifluoracetat | HPLC (Methode 6): Rₜ = 3.17 min; LC-MS (Methode 19): Rₜ = 1.30 min, MS (ESIpos): *m*/*z* (%) = 573.1 (100) [M+2H]²⁺, 1144.6 (10) [M+H]⁺, MS (ESIneg): *m*/*z* (%) = 1142.8 (100) [M-H]⁻; HR-TOF-MS:C₅₂H₈₂N₁₃O₁₆ ber 1144.5997, gef. 1144.5988 [M+H]⁺. |
| | Ausbeute: 54 mg (70% rein, ∼quant.) als farbloser Feststoff aus 48 mg der Beispielverbindung 340A nach Arbeitsvorschrift 2, Rohprodukt ohne Aufreinigung weiter umgesetzt. | |
| 365A | 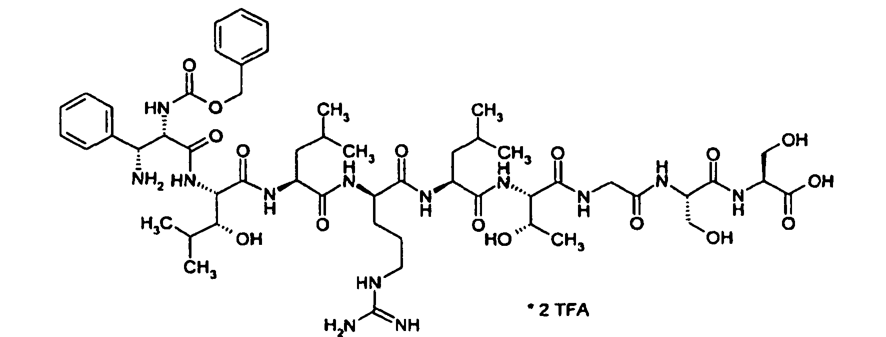 | |
| | [(3R)-N²-(Benzyloxycarbonyl)-3-amino-L-phenylalanyl]-[(3R)-3-hydroxy-L-leucyl]-L-leucyl-D-arginyl-L-leucyl-L-allothreonyl-glycyl-L-seryl-L-serin-bis-trifluoracetat | HPLC (Methode 6): Rₜ = 3.23 min; LC-MS (Methode 19): Rₜ = 1.37 min, MS (ESIpos): *m*/*z* (%) = 580 (100) MS [M+2H]²⁺, 1158.6 (5) [M+H]⁺, MS [M+2H]²⁺, 1158.6 [M+H]⁺, MS (ESIneg): *m*/*z* (%) = 1156.8 (100) [M-H]⁻; HR-TOF-MS: C₅₃H₈₄N₁₃O₁₆ ber. 1158.6154, gef. 1158.6183 [M+H]⁺. |
| | Ausbeute: 55 mg ( 70% rein, ∼quant.) als farbloser Feststoff aus 49 mg der Beispielverbindung 341A nach Arbeitsvorschrift 2, Rohprodukt ohne Aufreinigung weiter umgesetzt. | |
| 366A | 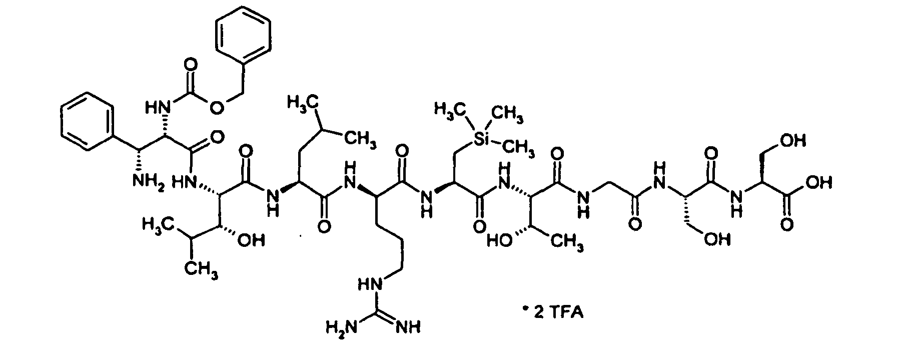 | |
| | [(3*R*)-*N*²-(Benzyloxycarbonyl)-3-amino-L-phenylalanyl]-[(3R)-3-hydroxy-L-leucyl]-L-leucyl-D-arginyl-[3-trimethylsilyl-L-alanlyl]-L-allothreonyl-glycyl-L-seryl-L-serin-bis-trifluoracetat | HPLC (Methode 6): Rₜ = 3.31 min; LC-MS (Methode 19): Rₜ = 1.47 min, MS (ESIpos): *m*/*z* (%) = 595.0 (100) [M+2H]²⁺, 1188.7 (5) [M+H]⁺, MS (ESIneg): *m*/*z* (%) = 1187.7 (100) [M-H]⁻; HR-TOF-MS: C₅₃H₈₆N₁₃O₁₆Si ber. 1188.6080, gef. 1188.6067 [M+H]⁺. |
| | Ausbeute: 49 mg (-quant.) als farbloser Feststoff aus 42 mg der Beispielverbindung 342A nach Arbeitsvorschrift 2, Rohprodukt ohne Aufreinigung weiter umgesetzt. | |
| 367A | 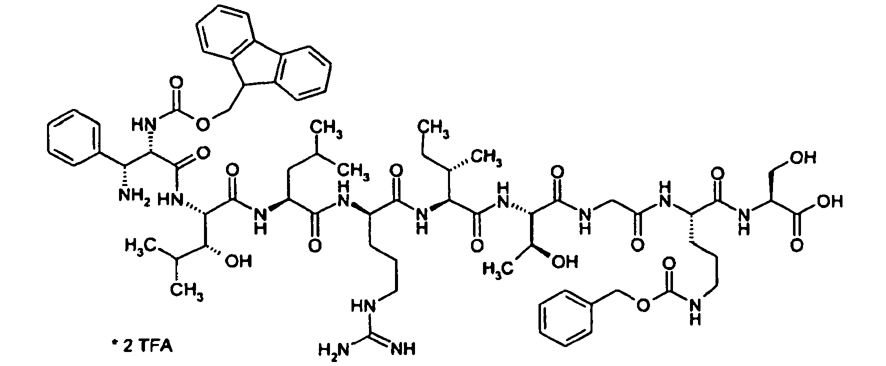 | |
| | {(3*R*)-N²-[(9H-fluoren-9-ylmethoxy)carbonyl]-3-amino-L-phenylalanyl}-[(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-bis-trifluoracetat | HPLC (Methode 6): Rₜ = 3.62 min; LC-MS (Methode 19): Rₜ = 1.79 min, MS (ESIpos): *m*/*z* (%) = 704.5 (100) [M+2H]²⁺, MS (ESIneg): *m*/*z* (%) = 1406 (100) [M-H]⁻; HR-TOF-MS: C₇₀H₉₉N₁₄O₁₇ ber. 1407.7308, gef. 1407.7306 [M+H]⁺. |
| | Ausbeute: 42 mg (-quant.) als farbloser Feststoff aus 39 mg der Beispielverbindung 343A nach Arbeitsvorschrift 2, Rohprodukt ohne Aufreinigung weiter umgesetzt. | |
| 368A | 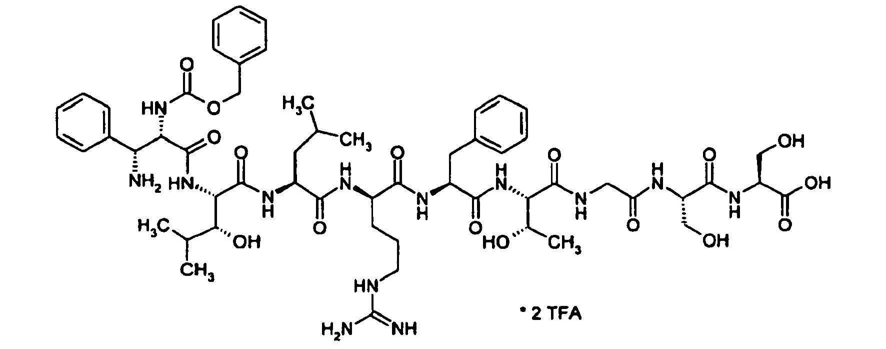 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-phenylalanlyl-L-allothreonyl-glycyl-L-seryl-L-serin-bis-trifluoracetat | HPLC (Methode 6): Rₜ = 3.26 min; LC-MS (Methode 19): Rₜ = 1.42 min, MS (ESIpos): *m*/*z* (%) = 597.1 (100) [M+2H]²⁺, MS (ESIneg): *m*/*z* (%) = 1190.7 (100) [M-H]⁻. |
| | Ausbeute: 33 mg (59% d. Th.) als farbloser Feststoff aus 55 mg der Beispielverbindung 344A nach Arbeitsvorschrift 2, Rohprodukt ohne Aufreinigung weiter umgesetzt. | |
| 369A | 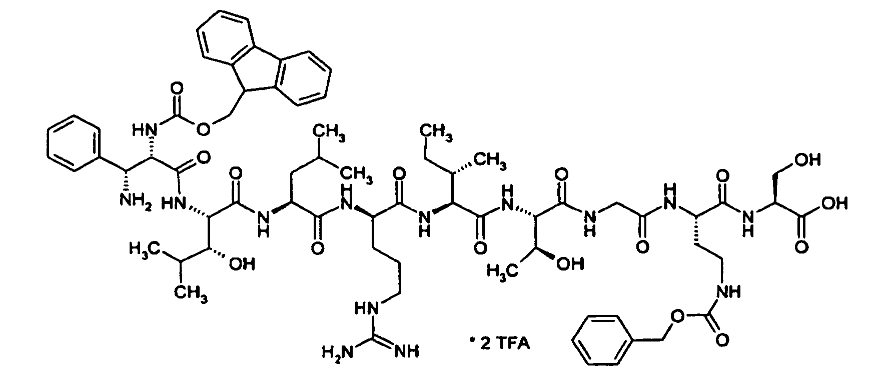 | |
| | (3*R*)-*N*²-[(9H-fluoren-9-ylmethoxy)carbonyl]-3-amino-L-phenylalanyl}-[(3*R*)-3-hydroxy-L-leucyl]-L-leucyl-D-arginyl-L-isoleucyl-L-allothreonyl-glycyl-[(2S)-2-amino-4-benzyloxycarbonylamino-butyryl]-L-serin-bis-trifluoracetat | HPLC (Methode 6): Rₜ = 3.63 min; LC-MS (Methode 19): Rₜ = 1.76 min, MS = 1.76 (ESIpos): *m*/*z* (%) 679.5 (100) [M+2H]²⁺, MS (ESIneg): *m*/*z* (%) = 1391.7 (100) [M-H]⁻; HR-TOF-MS: C₆₉H₉₇N₁₄O₁₇ ber. 1393.7151, gef. 1393.7189 |
| | Ausbeute: 87 mg (-quant.) als farbloser Feststoff aus 72 mg der Beispielverbindung 345A nach Arbeitsvorschrift 2, Rohprodukt ohne Aufreinigung weiter umgesetzt. | |
| 370A | 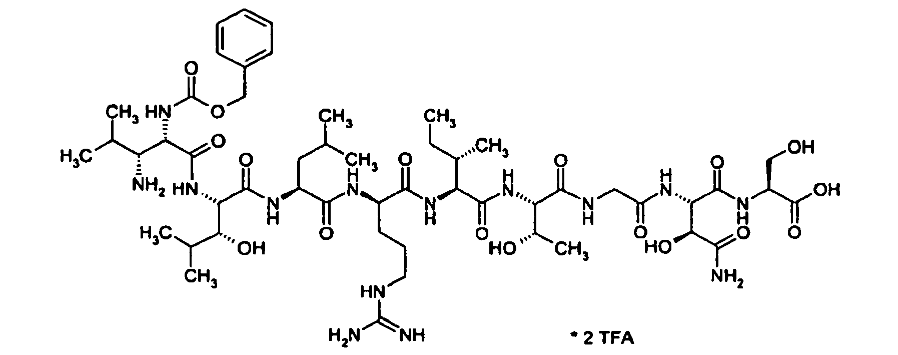 | |
| | [(3*R*)-*N*²-(Benzyloxycarbonyl)-3-amino-L-leucyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-bis-trifluoracetat | HPLC (Methode 6): Rₜ = 3.18 min; LC-MS (Methode 19): Rₜ = 1.30 min, MS (ESIpos): *m*/*z* (%) = 584.6 (100) [M+2H]²⁺, MS (ESIneg): *m*/*z* (%) = 1165.7 (100) [M-H]⁻. |
| | Ausbeute: 399 mg (96% rein, -quant.) als farbloser Feststoff aus 311 mg der Beispielverbindung 346A nach Arbeitsvorschrift 2, Rohprodukt ohne Aufreinigung weiter umgesetzt. | |
| 371A | 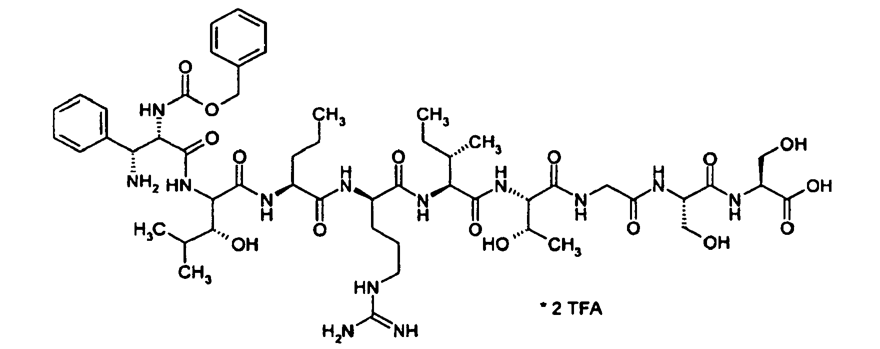 | |
| | [(3*R*)-*N*²-(Benzyloxycarbonyl)-3-amino-L-phenylalanyl]-[(3*R*)-3-hydroxy-L-leucyl]-L-norvalyl-D-arginyl-L-isoleucyl-L-allothreonylglycyl-L-seryl-L-serin-bis-trifluoracetat | HPLC (Methode 6): Rₜ = 3.18 min; LC-MS (Methode 19): Rₜ = 1.32 min; LC-(ESIpos): *m*/*z* (%) = 572.9 (100) [M+2H]²⁺, 1144.7 (5) [M+H]⁺, MS (ESIneg): *m*/*z* (%) = 1142.7 (100) [M-H]⁻; HR-TOF-MS: C₅₂H₈₂N₁₃O₁₆ ber. 1144.5997, gef. 1144.6023 [M+H]⁺. |
| | Ausbeute: 195 mg (-quant.) als farbloser Feststoff aus 173 mg der Beispielverbindung 347A nach Arbeitsvorschrift 2, Rohprodukt ohne Aufreinigung weiter umgesetzt. | |
| 372A | 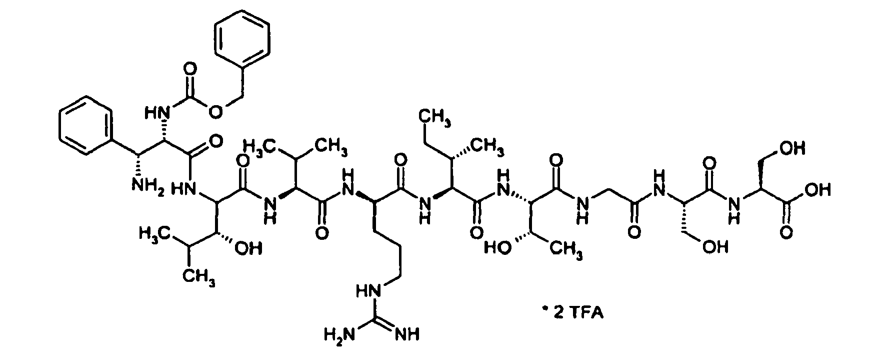 | |
| | [(3*R*)-*N*²-(Benzyloxycarbonyl)-3-amino-L-phenylalanyl]-[(3*R*)-3-hydroxy-L-leucyl]-L-valyl-D-arginyl-L-isoleuCyl-L-allothreonyl- glycyl-L-seryl-L-serin-bis-trifluoracetat | HPLC (Methode 6): Rₜ = 3.16 min; LC-MS (Methode 19): Rₜ = 1.30 min, MS (ESIpos): *m*/*z* (%) = 572.9 (100) [M+2H]²⁺, 1144.6 (5) [M+H]⁺, MS (ESIneg): *m*/*z* (%) = 1142.8 (100) [M-H]⁻; HR-TOF-MS: C₅₂H₈₂N₁₃O₁₆ ber. 1144.5997, gef. 1144.5995 [M+H]⁺. |
| | Ausbeute: 67 mg (-quant.) als farbloser Feststoff aus 61 mg der Beispielverbindung 348A nach Arbeitsvorschrift 2, Rohprodukt ohne Aufreinigung weiter umgesetzt. | |
| 373A | 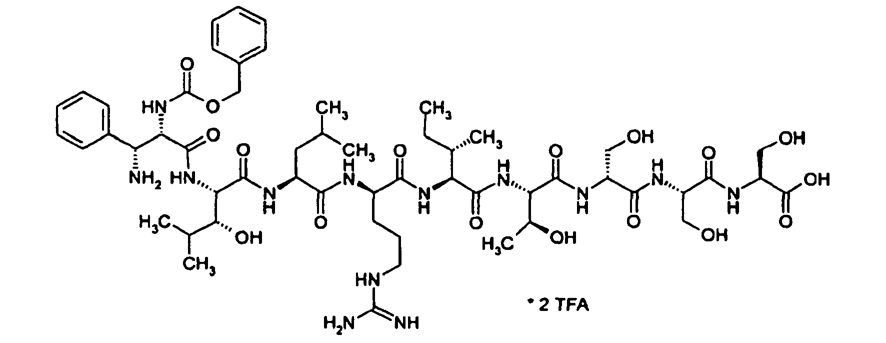 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-allothreonyl-D-seryl-L-seryl-L-serin-bis-trifluoracetat | HPLC (Methode 6): Rₜ = 3.64 min. |
| | Ausbeute: 89 mg (-quant.) als farbloser Feststoff aus 73 mg der Beispielverbindung 349A nach Arbeitsvorschrift 2, Rohprodukt ohne Aufreinigung weiter umgesetzt. | |
| 374A | 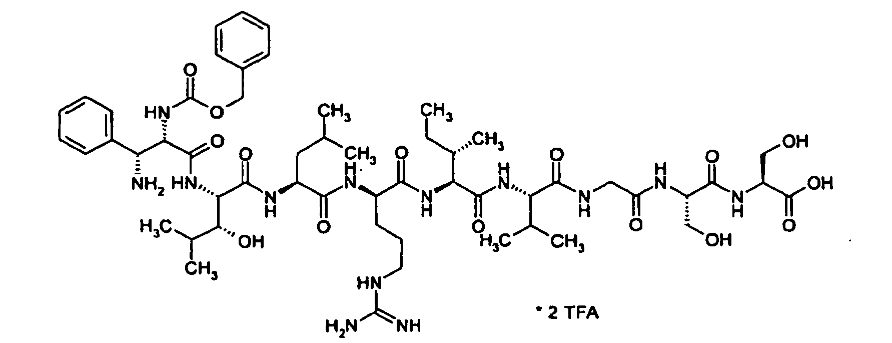 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-valyl-glycyl-L-seryl-L-serin-bis-trifluoracetat | HPLC (Methode 6): Rₜ = 3.29 min; LC-MS (Methode 19): Rₜ = 1.44 min, MS (ESIpos): *m*/*z* (%) = 579.0 (100) [N4+2H]²⁻, 1156.7 (10) [M+H]⁺, MS (ESIneg): *m*/*z* (%) = 1154.8 (100) [M-H]⁻, HR-TOF-MS:C₅₄H₈₆N₁₃O₁₅ ber. 1156.6361, gef. 1156.6384 [M+H]⁺. |
| | Ausbeute: 55 mg (99% d. Th.) als farbloser Feststoff aus 55 mg der Beispielverbindung 350A nach Arbeitsvorschrift 2, Rohprodukt ohne Aufreinigung weiter umgesetzt. | |
| 375A | 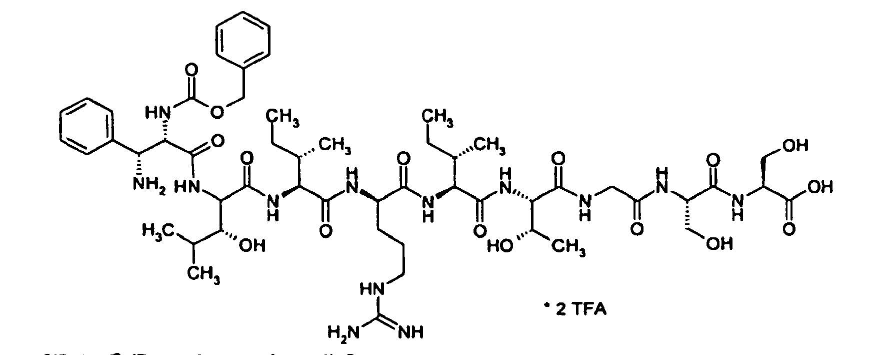 | |
| | [(3*R*)-*N*²-(Benzyloxycarbonyl)-3-amino-L-phenylalanyl]-[(3*R*)-3-hydroxy-L-leucyl]-L-isoleucyl-D-arginyl-L-isoleucyl-L-allothreonyl-glycyl-L-seryl-L-serin-bis-trifluoracetat | HPLC (Methode 6): Rₜ = 3.21 min; LC-MS (Methode 19): Rₜ = 1.35 min, MS (ESIpos): *m*/*z* (%) = 580.0 (100) [M+2H]²⁺, MS (ESIneg): *m*/*z* (%) = 1156.7 (100) [M-H]⁻; HR-TOF-MS: C₅₃H₈₄N₁₃O₁₆ ber. 1158.6154, gef. 1158.6184 [M+H]⁺. |
| | Ausbeute: 39 mg (96% d. Th.) als farbloser Feststoff aus 391 mg der Beispielverbindung 351A nach Arbeitsvorschrift 2, Rohprodukt ohne Aufreinigung weiter umgesetzt. | |
| 376A | 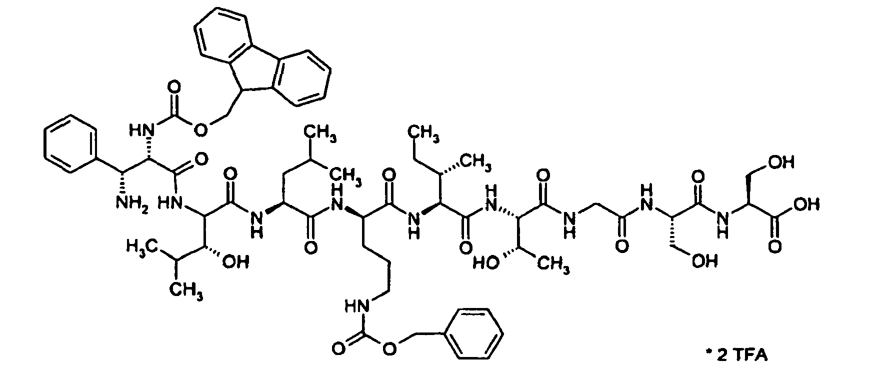 | |
| | [(3*R*)-*N*²-[(9H-fluoren-9-ylmethoxy)carbonyl]-3-amino-L-phenylalanyl)-[(3*R*)-3-hydroxy-L-leucyl]-L-leucyl-(N⁵-benzyloxycarbonyl-D-ornithyl)-L-isoleucyl-L-allothreonyl-glycyl-L-seryl-L-serin-bis-trifluoracetat | HPLC (Methode 6): Rₜ = 3.95 min; LC-MS (Methode 19): Rₜ = 2.15 min, MS (ESIpos): *m*/*z* (%) = 1338.7 (100) [M+H]⁺, MS (ESIneg): *m*/*z* (%) = 1336.7 (100) [M-H]⁻; HR-TOF-MS: C₆₇H₉₂N₁₁O₁₈ ber. 1338.6617, gef. 1338.6619 [M+H]⁺. |
| | Ausbeute: 123 mg (∼quant.) als farbloser Feststoff aus 103 mg der Beispielverbindung 352A nach Arbeitsvorschrift 2, Rohprodukt ohne Aufreinigung weiter umgesetzt. | |
| 377A | 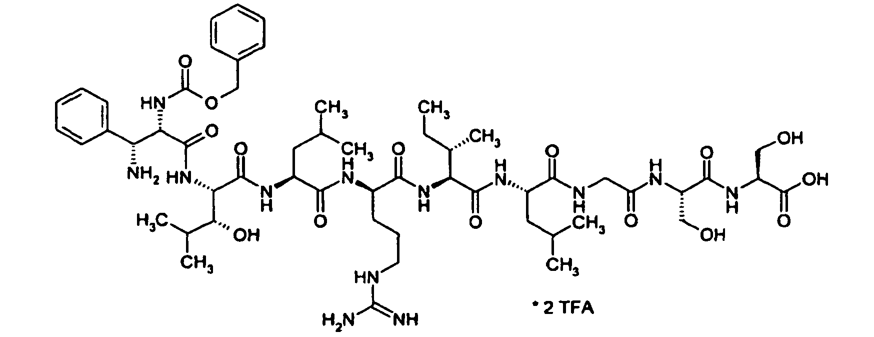 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-leucyl-glycyl-L-seryl-L-Serin-bis-trifluoracetat | HPLC (Methode 6): Rₜ = 3.32 min, LC-MS (Methode 19): Rₜ = 1.51 min, MS (ESIpos): *m*/*z* (%) = 586.0 (100) [M+2H]²⁺, 1170.0 (5) [M+H]⁺, MS (ESIneg): *m*/*z* (%) = 1168.7 (100) [M-H]⁻; HR-TOF-MS: C₅₅H₈₈N₁₃O₁₅ ber. 1170.6518, gef. 1170.6501 [M+H]⁺. |
| | Ausbeute: 40 mg (-quant.) als farbloser Feststoff aus 40 mg der Beispielverbindung 353A nach Arbeitsvorschrift 2, Rohprodukt ohne Aufreinigung weiter umgesetzt. | |
| 378A | 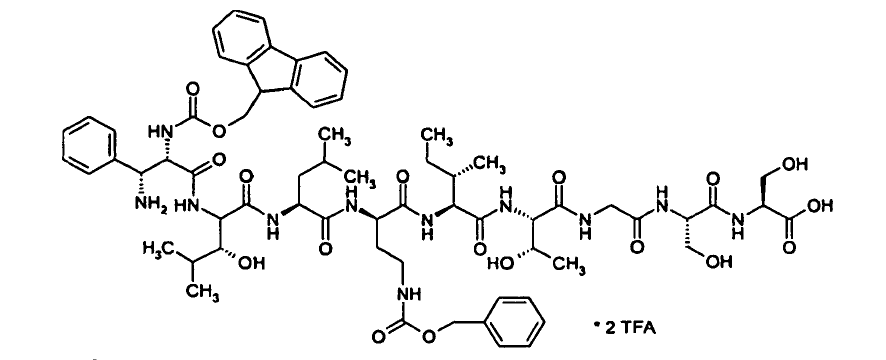 | |
| | [(3*R*)-*N*²-[(9H-fluoren-9-ylmethoxy)carbonyl]-3-amino-L-phenylalanyl)-[(3*R*)-3-hydroxy-L-leucyl]-L-leucyl-[(2*R*)-2-amino-4-benzyloxycarbonylamino-butyryl]-L-isoleucyl-L-allothreonyl-glycyl-L-seryl-L-serin-bis-trifluoracetat | HPLC (Methode 6): Rₜ = 3.92 min; LC-MS (Methode 19): Rₜ = 2.14 min, MS (ESIpos): *m*/*z* (%) = 1324.6 (100) MS [M+H]⁺, MS (ESIneg): *m*/*z* (%) = 1322.7 (100) [M-H]⁻; *m*/*z* (%) = C₆₆H₉₀N₁₁O₁₈ ber. 1324.6460, gef. 1324.6471 [M+H]⁺. gef. 1324.6471 [M+H]⁺. umgesetzt. |
| | Ausbeute: 131 mg (58% rein, 70% d. Th.) als farbloser Feststoff aus 106 mg der Beispielverbindung 354A nach Arbeitsvorschrift 2, Rohprodukt ohne Aufreinigung weiter | |
| 379A | 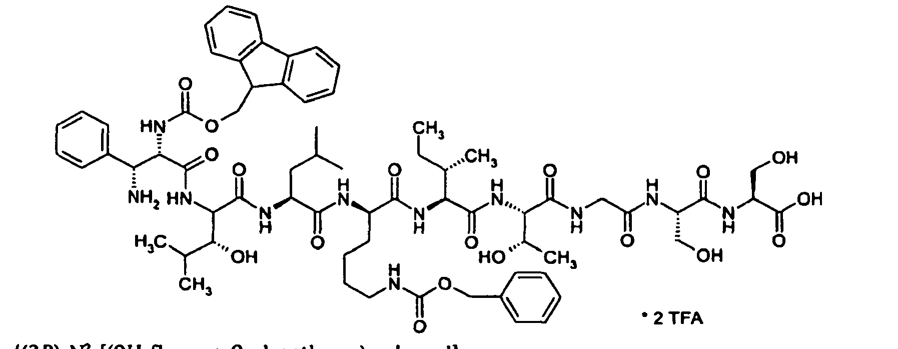 | |
| | {(3*R*)-*N*²-[(9H-fluoren-9-ylmemoxy)carbonyl]-3-amino-L-phenylatanyl}-[(3*R*)-3-hydroxy-L-leucyl]-L-leucyl-(*N*⁶-benzyloxycarbonyl-D-lysyl)-L-isoleucyl-L-allothreonyl-glycyl-L-seryl-L-serin-bis-trifluoracetat | HPLC (Methode 6): Rₜ = 3.97 min; LC-MS (Methode 19): Rₜ = 2.18 min, MS (ESIpos): *m*/*z* (%) = 1352.7 (100) [M+H]⁺, MS (ESIneg): *m*/*z* (%) = 1351.7 (100) [M-H]⁻; HR-TOF-MS: C₆₈H₉₄N₁₁O₁₈ ber. 1352.6773, gef. 1352.6771 [M+H]⁺. |
| | Ausbeute: 48 mg (90% d. Th.) als farbloser Feststoff aus 49 mg der Beispielverbindung 355A nach Arbeitsvorschrift 2, Rohprodukt ohne Aufreinigung weiter umgesetzt. | |
| 380A | 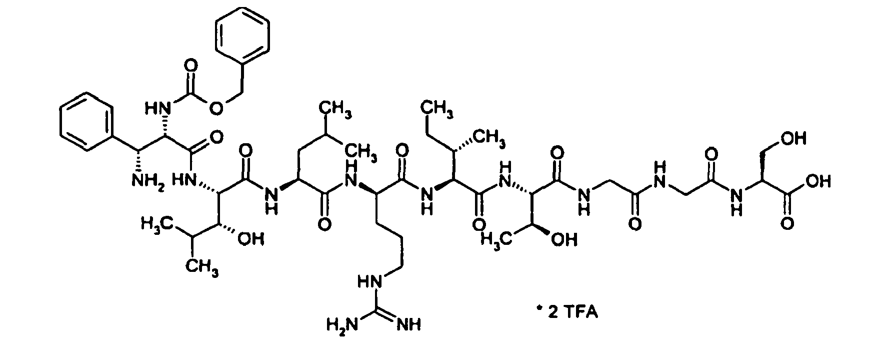 | |
| | [(3*R*)-N²-(Benzyloxycarbonyl)-3-amino-L-phenylalanyl]-[(3*R*)-3-hydroxy-L-leucyl]-L-leucyl-D-arginyl-L-isoleucyl-L-allothreonyl-glycyl-glycyl-L-serin-bis-trifluoracetat | HPLC (Methode 6): Rₜ = 3.25 min; LC-MS (Methode 19): Rₜ = 1.38 min, MS (ESIpos): *m*/*z* (%) = 565.0 (100) [M+2H]²⁺, MS (ESIneg): *m*/*z* (%) = 1126.8 (100) [M-H]⁻; HR-TOF-MS: C₅₂H₈₂N₁₃O₁₅ ber. 1128.6048, gef. 1128.6061 [M+H]⁺. |
| | Ausbeute: 167 mg (-quant.) als farbloser Feststoff aus 133 mg der Beispielverbindung 356A nach Arbeitsvorschrift 2, Rohprodukt ohne Aufreinigung weiter umgesetzt. | |
| 381A | 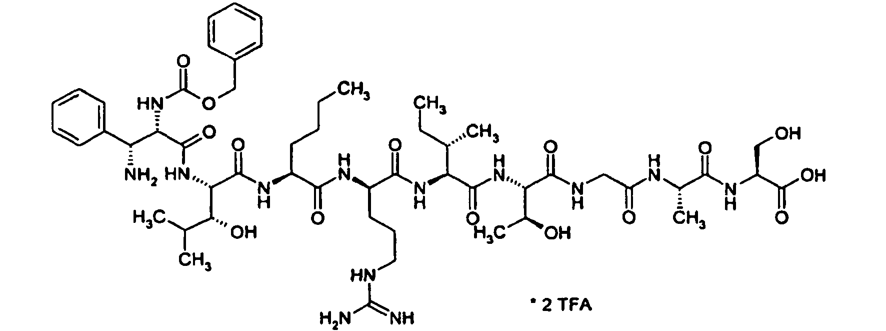 | |
| | [(3*R*)-*N*²-(Benzyloxycarbonyl)-3-amino-L-phenylalanyl)-[(3*R*)-3-hydroxy-L-leucyl]-L-norleucyl-D-arginyl-L-isoleucyl-L-allothreonyltrifluoracetat glycyl-L-alanyl-L-serin- | HPLC (Methode 6): Rₜ = 3.25 min; LC-MS (Methode 52): Rₜ = 1.00 min, MS (ESIpos): *m*/*z* (%) = 572.0 (100) [M+2H]²⁺, MS (ESIneg); *m*/*z* (%) = 1140.7 (100) [M-H]⁻; HR-TOF-MS: C₅₃H₈₄N₁₃O₁₅ ber. 1142.6205, gef. 1142.6184 [M+H]⁺. |
| | Ausbeute: 104 mg (quant.) aus Beispielverbindung 357A (95 mg, 70 µmol) nach Arbeitsvorschrift 2, Rohprodukt ohne Aufreinigung weiter umgesetzt. | |
| 382A | 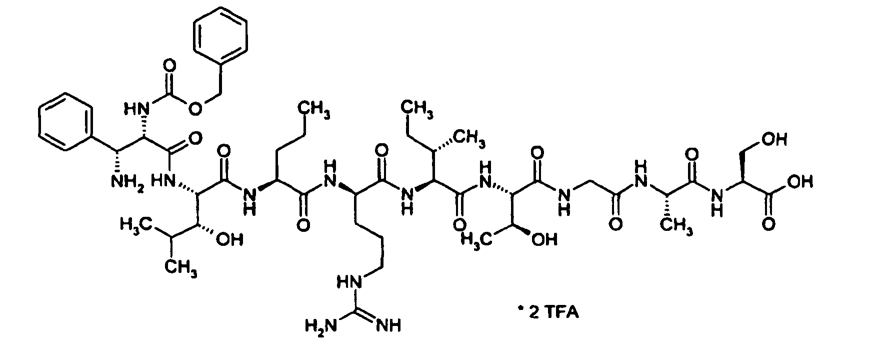 | |
| | [(3*R*)-*N*²-(Benzyloxycarbonyl)-3-amino-L-phenylalanyl]-[(3*R*)-3-hydroxy-L-leucyl]-L-norvalyl-D-arginyl-L-isoleucyl-L-allothreonyl-glycyl-L-alanyl-L-serin-trifluoracetat | HPLC (Methode 6): Rₜ = 3.18 min; LC-MS (Methode 52): Rₜ = 0.91 min, MS (ESIpos): *m*/*z* (%) = 1128.7 (10) [M+H]⁻, 565.0 (100) N+2H]²⁺, MS (ESIneg): *m*/*z* (%) = 1126.7 (100) [M-H] HR-TOF-MS: C₅₂H₈₁N₁₃O₁₅ ber. 1128.6048, gef. 1128.6030 [M+H]⁺. |
| | Ausbeute: 103 mg (quant.) aus Beispielverbindung 358A (87 mg, 65 µmol) nach Arbeitsvorschrift 2, Rohprodukt ohne Aufreinigung weiter umgesetzt. | |

### Cyclisierung

| Nr | Struktur | |
|---|---|---|
| | Name | Analytik |
| | Ausbeute, Synthesemethode | |
| 383A | 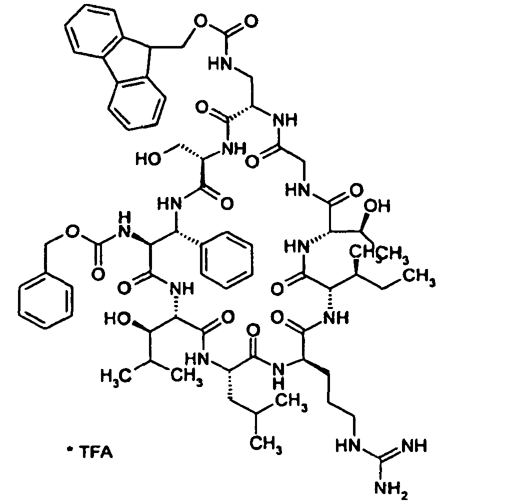 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-allothreonyl-glycyl-[(2S)-2-amino-3-(9H-fluoren-9-ylmethoxycarbonyl)aminobutyryl]-L-serin-*C*^{1.9}-*N*^{3.1}-lactam-trifluoracetat | HPLC (Methode 6): Rₜ = 4.07 min; LC-MS (Methode 19): Rₜ = 2.02 min, MS (ESIpos): *m*/*z* (%) = 681.6 (100) [M+2H]²⁺; HR-TOF-MS: C₆₈H₉₃N₁₄O₁₆ ber. 1361.6889, gef. 1361.6866 [M+H]⁺. |
| | Ausbeute: 590 mg (67% d. Th.) aus Beispielverbindung 359A (1110 mg, 700 µmol) nach Arbeitsvorschrift 11, Reinigung nach Methode 45 und anschließend Methode 34. | |
| 384A | 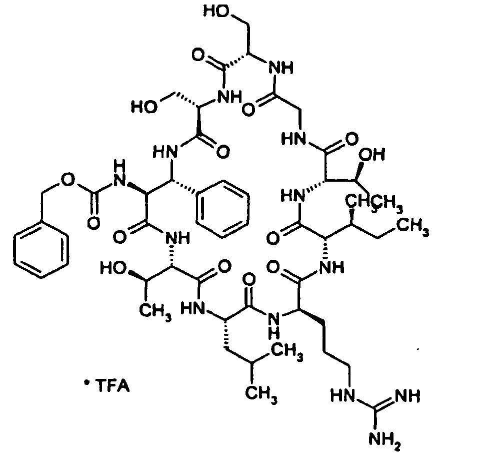 | |
| | [(3*R*)-*N*²-(Benzyloxycarbonyl)-3-amino-L-phenylalanyl]-L-threonyl-L-leucyl-D-arginyl-L-isoleucyl-L-allothreonyl- glycyl-L-seryl-*C^{1.9}-N^{3.1}*-lactam-trifluoracetat | HPLC (Methode 6): Rₜ = 3.45 min; LC-MS (Methode 19): Rₜ = 1.56 min, MS (ESIpos): *m*/*z* (%) = 1112.6 (50) [M+H]⁺, MS (ESIneg) *m*/*z* (%) =1110.7 (100) [M-H]⁻; HR-TOF-MS: C₅₁H₇₈N₁₃O₁₅ ber. 1112.5735, gef. 1112.5710 [M+H]⁺. |
| | aus Ausbeute: 14 mg (73% d. Th.) Beispielverbindung 360A (21 mg, 15 µmol) nach Arbeitsvorschrift 11, Reinigung nach Methode 45. | |
| 385A | 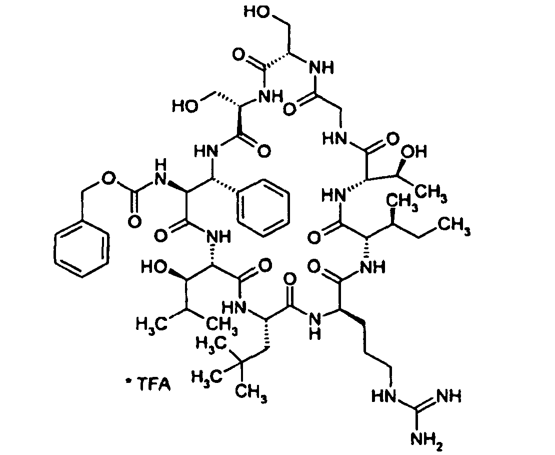 | |
| | [(3*R*)-*N*²-(Benzyloxycarbonyl)-3-amino-L-phenylalanyl]-[(3*R*)-3-hydroxy-L-leucyl]-[3-tertbutyl-L-alanyl]-D-arginyl-L-isoleucyl-L-allothreonyl-glycyl-L-seryl-L-serin-*C^{1.9}-N^{3.1}*-lactam-trifluoracetat | HPLC (Methode 6): Rₜ = 3.62 min; LC-MS (Methode 19): Rₜ = 1.58 min, MS (ESIpos): *m*/*z* (%) = 1154.8 (100) [M+H]⁺, MS (ESIneg): *m*/*z* (%) = 1152.9 (100) [M-H]⁻; HR-TOF-MS: |
| | Ausbeute: 30 mg (90% d. Th.) aus Beispielverbindung 361A (37 mg, 26 µmol) nach Arbeitsvorschrift 11, Reinigung nach Methode 45. | C₅₄H₈₄N₁₃O₁₅ ber. 1154.6205, gef. 1154.6210 [M+H]⁺. |
| 386A | 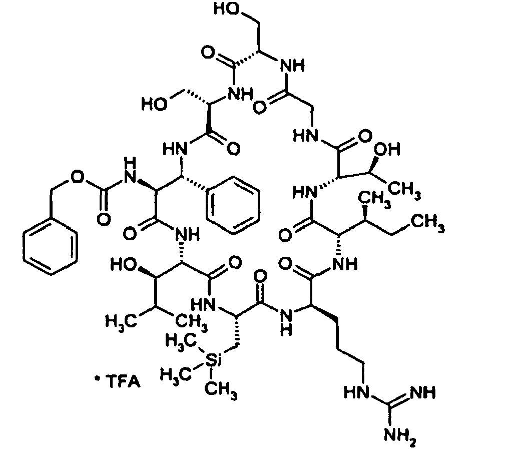 | |
| | [(3*R*)-*N*²-(Benzyloxycarbonyl)-3-amino-L-phenylalanyl]-[(3*R*)-3-hydroxy-L-leucyl]-(3-trimethylsilyl-L-alanyl)-D-arginyl-L-isoleucyl-L-allothreonyl-glycyl-L-seryl-L-serin-*C^{1.9}-N^{3.1}*-lactam-trifluoracetat | HPLC (Methode 6): Rₜ = 3.68 min; LC-MS (Methode 19): Rₜ = 1.62 min, MS (ESIpos): *m*/*z* (%) = 586 (100) [M+2H]²⁺, 1170.8 (70) [M+H]⁺, MS |
| | Ausbeute: 54 mg (98% d. Th.) aus Beispielverbindung 362A (37 mg, 26 µmol) nach Arbeitsvorschrift 11, Reinigung nach Methode 45. | (ESIneg) *m*/*z* (%) = 1168.8 (100) [M-H]⁻; HR-TOF-MS: C₅₃H₈₄N₁₃O₁₅ ber. 1170.5974, gef. 1170.5962 [M+H]⁺. |
| 387A | 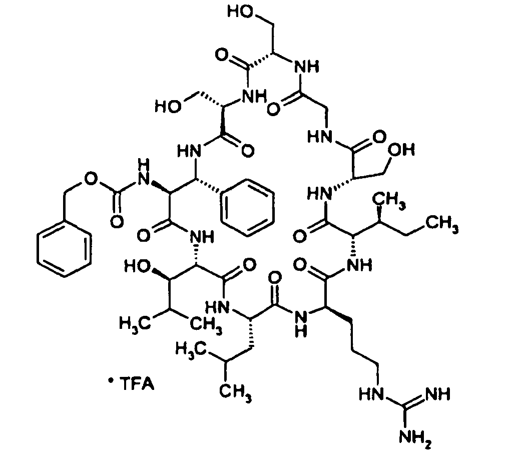 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-seryl-glycyl-L-seryl-L-serin-*C^{1.9}-N^{3.1}*-lactam-trifluoracetat | HPLC (Methode 6): Rₜ = 3.53 min; LC-MS (Methode 19): Rₜ = 1.56 min, MS (ESIpos): *m*/*z* (%) = 1126.6 (100) [M+H]⁺, MS (ESIneg): *m*/*z* (%) = 1124.7 (100) [M-H]⁻. |
| | Ausbeute: 31 mg (81% d. Th.) aus Beispielverbindung 363A (48 mg, 31 µmol) nach Arbeitsvorschrift 11, Reinigung nach Methode 45. | |
| 388A | 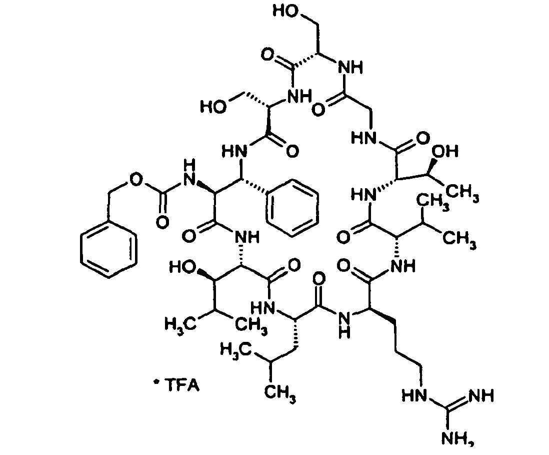 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-valyl-L-allothreonyl-glycyl-L-seryl-L-serin-*C^{1.9}-N^{3.1}*-lactam-trifluoracetat | (Methode 6): Rₜ = 3.48 min; LC-MS (Methode 19): Rₜ = 1.56 min, MS (ESIpos): m/z (%) = 564.0 (90) [M+2H]²⁺, 1126.7 (90) [M+H]⁺, MS (ESIneg): *m*/*z* (%) = 1125.8 (100) [M-H]⁻; HR-TOF-MS: C₅₂H₈₀N₁₃O₁₅ ber. |
| | Ausbeute: 77 mg (41% rein, 91% d. Th.) aus Beispielverbindung 364A (54 mg, 28 µmol) nach Arbeitsvorschrift 11, Reinigung nach Methode 45. | |
| 389A | 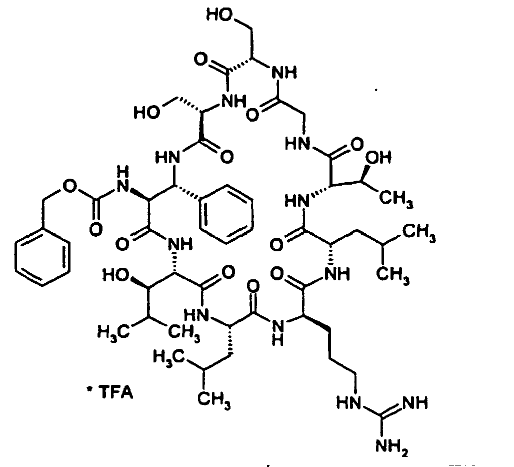 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-leucyl-L-allothreonyl-glycyl-L-seryl-L-serin-*C^{1.9}-N^{3.1}*-lactam-trifluoracetat | HPLC (Methode 6): Rₜ = 3.58 min; LC-MS (Methode 19): Rₜ = 1.63 min, MS (ESIpos): *m*/*z* (%) = 571.6 (30) [M+2H]²⁺, 1140.8 (100) [M+H]⁺, MS (ESIneg): *m*/*z* (%) = 1138.9 (100) [M-H]⁻; HR-TOF-MS: C₅₃H₈₂N₁₃O₁₅ ber. 1140.6048, gef. 1140.6014 [M+H]⁺. |
| | Ausbeute: 78 mg (39% rein, 87% d. Th.) aus Beispielverbindung 365A (55 mg, 28 µmol) nach Arbeitsvorschrift 11, Reinigung nach Methode 45. | |
| 390A | 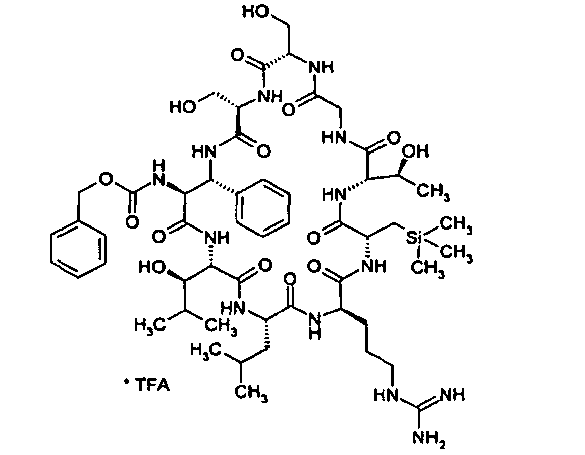 | |
| | [(3*R*)-*N*²-(Benzyloxycarbonyl)-3-amino-L-phenylalanyl]-[(3*R*)-3-hydroxy-L-leucyl]-L-leucyl-D-arginyl-[3-trimethylsilyl-L-alanlyl]-L-allothreonyl-glycyl-L-seryl-L-serin-*C^{1.9}-N^{3.1}*-lactam-trifluoracetat | HPLC (Methode 6): Rₜ = 3.70 min; LC-MS (Methode 19): Rₜ = 1.73 min, MS (ESIpos): *m*/*z* (%) = 586.1 (60) [M+2H]²⁺, 1170.8 (100) [M+H]⁺, MS (ESIneg): *m*/*z* (%) = 1168.7 (100) [M- |
| | Ausbeute: 55 mg (42% rein, 51% d. Th.) aus Beispielverbindung 366A (54 mg, 35 µmol) nach Arbeitsvorschrift 11, Reinigung nach Methode 45. | H] ; HR-TOF-MS: C₅₃H₈₄N₁₃O₁₅Si ber. 1170.5974, gef. 1170.5972 [M+H]⁺. |
| 391A | 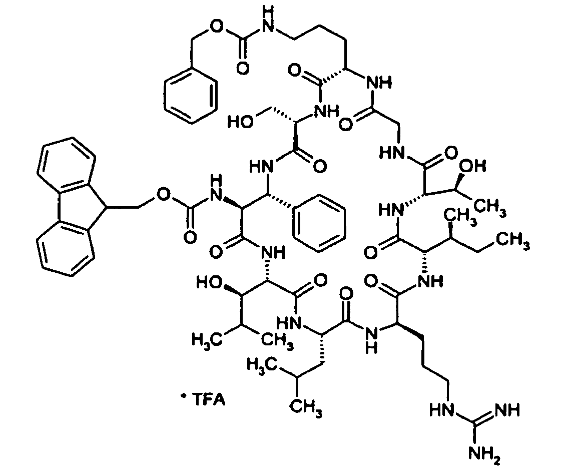 | |
| | {(3*R*)-*N*²-[(9H-fluoren-9-ylmethoxy)carbonyl]-3-amino-L-phenytatanyl}-[(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-*C^{1.9}*-*N^{3.1}*-lactam-trifluoracetat | HPLC (Methode 6): Rₜ = 4.08 min; LC-MS (Methode 19): Rₜ = 2.02 min, MS (ESIpos): *m*/*z* (%) = 695.7 (100) [M+2H]²⁺, 1389.7 (50) [M+H]⁺, MS (ESIneg): *m*/*z* (%) = 1387.8 (80) [M-H]⁻; HR-TOF-MS: C₇₀H₉₇N₁₄O₁₆ ber. 1389.7202, gef. 1389.7228 [M+H]⁺. |
| | Ausbeute: 34 mg (88% d. Th.) aus Beispielverbindung 367A (42 mg, 26 µmol) nach Arbeitsvorschrift 11, Reinigung nach Methode 45. | |
| 392A | 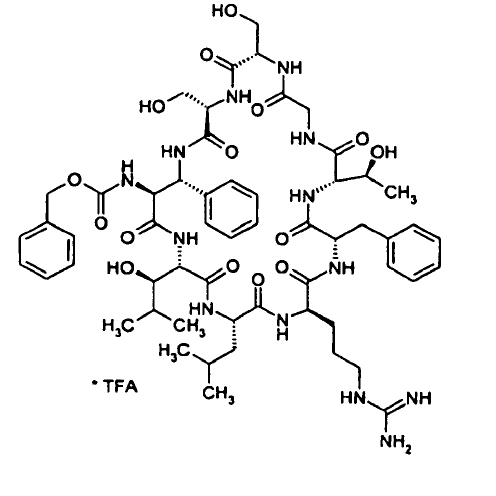 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-phenylalanlyl-L-allothreonyl-glycyl-L-seryl-L-serin-*C^{1.9}-N^{3.1}*-lactam-trifluoracetat | HPLC (Methode 6): Rₜ = 3.61 min; LC-MS (Methode 19): Rₜ = 1.67 min, MS (ESIpos): *m*/*z* (%) = 1174.7 (80) [M+H]⁺, MS (ESInegs): *m*/*z* (%) = 1172.7 (100) [M-H]⁻. |
| | Ausbeute: 23 mg (76% d. Th.) aus Beispielverbindung 368A (33 mg, 23 µmol) nach Arbeitsvorschrift 11, Reinigung nach Methode 45. | |
| 393A | 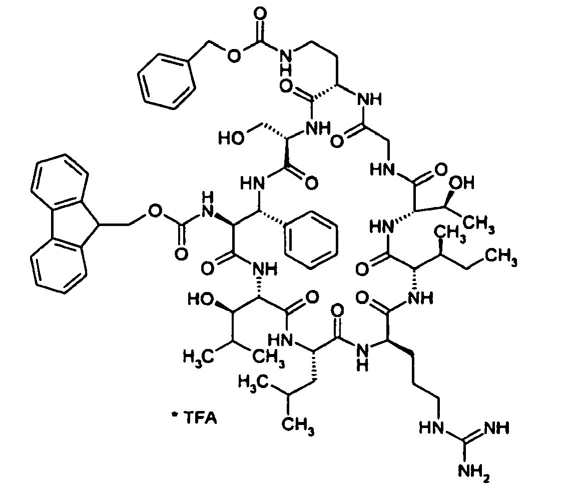 | |
| | {(3*R*)-*N*²-[(9H-fluoren-9-ylmethoxy)carbonyl]-3-amino-L-phenylalanyl}-[(3*R*)-3-hydroxy-L-leucyl]-L-leucyl-D-arginyl-L-isoleucyl-L-allothreonyl-glycyl-[(2*S*)-2-amino-4-benzyloxycarbonylamino-butyryl]-L-serin-*C^{1.9}*-*N^{3.1}*-lactam-trifluoracetat | HPLC (Methode 6): Rₜ = 4.07 min; LC-MS (Methode 19): Rₜ = 2.04 min, MS (ESIpos): *m*/*z* (%) = 688.5 (100) [M+2H]²⁺, 1376.7 (60) [M+H]⁺, MS (ESIneg): *m*/*z* (%) = 1374.7 (60) [M-H]⁻; HR-TOF-MS: C₆₉H₉₅N₁₄O₁₆ ber. 1375.7045, gef. 1375.7006 [M+H]⁺. |
| | Ausbeute: 80 mg (58% rein, 66% d. Th.) aus Beispielverbindung 369A (77 mg, 47 µmol) nach Arbeitsvorschrift 11, Reinigung nach Methode 45. | |
| 394A | 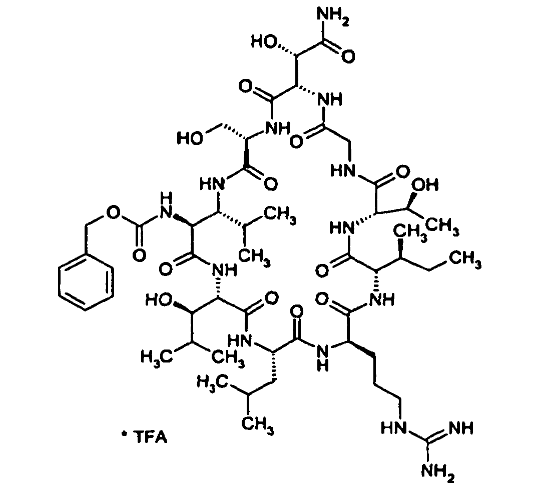 | |
| | [(3*R*)-*N*²-(Benzyloxycarbonyl)-3-amino-L-leucyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-*C^{1.9}*-*N^{3.1}*-lactam-trifluoracetat | HPLC (Methode 6): Rₜ = 3.48 und 3.57 min; LC-MS (Methode 19): Rₜ = 1.56 und 1.60 min, MS (ESIpos): *m*/*z* (%) = 575.7 (80) [M+2H]²⁺, 1149.8 (100) [M+H]⁺; HR-TOF-MS: (100) C₅₁H₈₅N₁₄O₁₆ ber. 1149.6263, gef. 1149.6283 [M+H]⁺. |
| | Ausbeute: 275 mg (2 Isomere, 61% rein, insgesamt 53% d. Th.) aus Beispielverbindung 370A (399 mg, 252 µmol) nach Arbeitsvorschrift 11, Reinigung nach Methode 45. | |
| 395A | 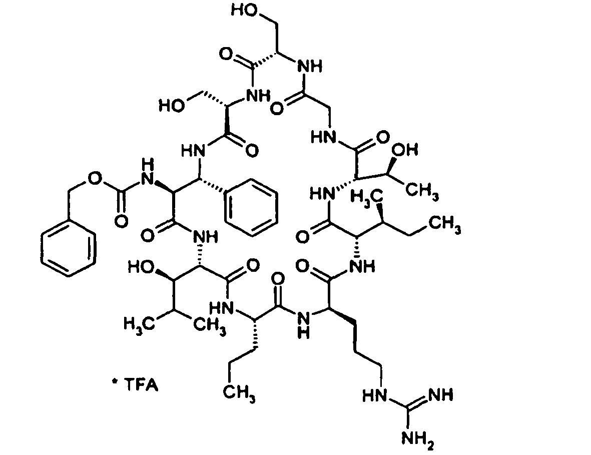 | |
| | [(3*R*)-*N*²-(Benzyloxycarbonyl)-3-amino-L-phenylalanyl]-[(3*R*)-3-hydroxy-L-leucyl]-L-norvalyl-D-arginyl-L-isoleucyl-L-allothreonyl-glycyl-L-seryl-L-serin-*C^{1.9}*-*N^{3.1}*-lactam-trifluoracetat | HPLC (Methode 6): Rₜ = 3.49 min; LC-MS (Methode 19): Rₜ = 1.57 min, MS (ESIpos): *m*/*z* (%) = 1126.6 (100) [M+H]⁺, MS (ESIneg): *m*/*z* (%) = 1124.6 (100) [M-H]⁻; HR-TOF-MS: |
| | Ausbeute: 135 mg (77% d. Th.) aus Beispielverbindung 371A (195 mg, 142 µmol) nach Arbeitsvorschrift 11, Reinigung nach Methode 45. | C₅₂H₈₀N₁₃O₁₅ ber. 1126.5892, gef. 1126.5864 [M+H]⁺. |
| 396A | 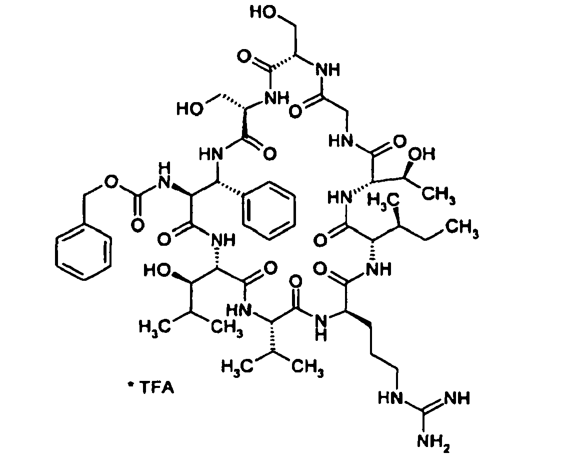 | |
| | [(3*R*)-*N*²-(Benzyloxycarbonyl)-3-amino-L-phenylalanyl]-[(3*R*)-3-hydroxy-L-leucyl]-L-valyl-D-arginyl-L-isoleucyl-L-allothreonyl-glycyl-L-seryl-L-serin-*C^{1.9}*-*N^{3.1}*-lactam-trifluoracetat | HPLC (Methode 6): Rₜ = 3.51 min, LC-MS (Methode 19): Rₜ = 1.58 min, MS (ESIpos): *m*/*z* (%) = 1126.7 (100) [M+H]⁺, MS (ESIneg): *m*/*z* (%) = 1125.7 (100) [M-H]⁻; 1170.8 (40) [M+HCOO⁻]⁻; HR-TOF-MS: C₅₂H₈₀N₁₃O₁₅ ber. 1126.5892, gef. 1126.5880 [M+H]⁺. |
| | Ausbeute: 61 mg (quant.) aus Beispielverbindung 372A (67 mg, 49 µmol) nach Arbeitsvorschrift 11, Reinigung nach Methode 45. | |
| 397A | 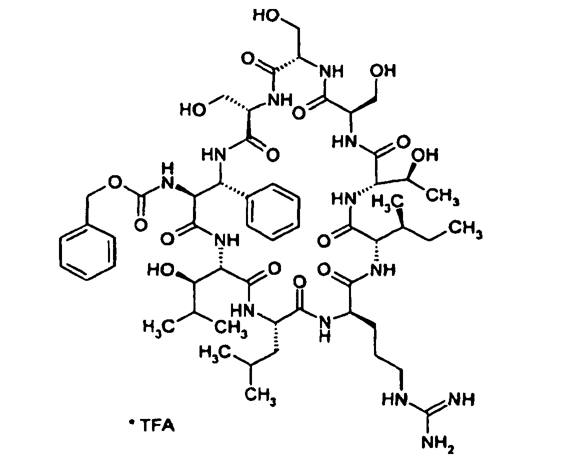 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-allothreonyl-D-seryl-L-seryl-L-serin-*C^{1.9}*-*N^{3.1}*-lactam-trifluoracetat | HPLC (Methode 6): Rₜ = 3.51 min; LC-MS (Methode 19): Rₜ = 1.59 min, MS (ESIpos): *m*/*z* (%) = 586.0 (70) [M+2H]²⁺, 1170.7 (100) [M+H]⁺, MS (ESIneg): *m*/*z* (%) = 1168.7 (100) [M-H]⁻; HR-TOF-MS: C₅₄H₈₄N₁₃O₁₆ ber. 1170.6154, gef. 1170.6132 [M+H]⁺. |
| | Ausbeute: 71 mg (93% d. Th.) aus Beispielverbindung 373A (84 mg, 59 µmol) nach Arbeitsvorschrift 11, Reinigung nach Methode 45. | |
| 398A | 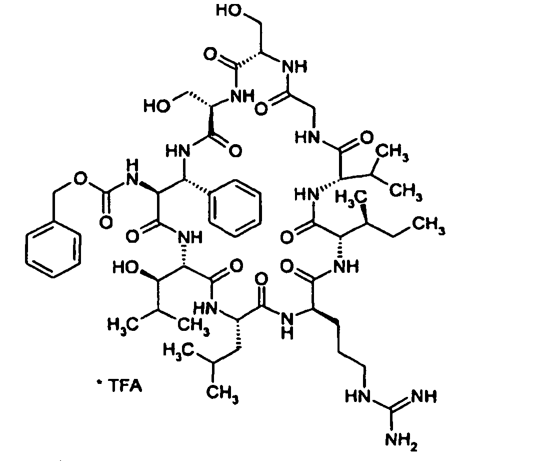 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-valyl-glycyl-L-seryl-L-serin-*C^{1.9}*-*N^{3.1}*-lactam-trifluoracetat | HPLC (Methode 6): Rₜ = 3.62 min; LC-MS (Methode 19): Rₜ = 1.65 min, MS (ESIpos): *m*/*z* (%) = 1138.6 (100) [M+H]⁺, MS (ESIneg): *m*/*z* (%) = 1136.7 (100) [M-H]⁻; HR-TOF-MS: C₅₄H₈₄N₁₃O₁₄ ber. 1138.6256, gef. 1138.6219 [M+H]⁺. |
| | Ausbeute: 31 mg (62% d. Th.) aus Beispielverbindung 374A (55 mg, 40 µmol) nach Arbeitsvorschrift 11, Reinigung nach Methode 45. | |
| 399A | 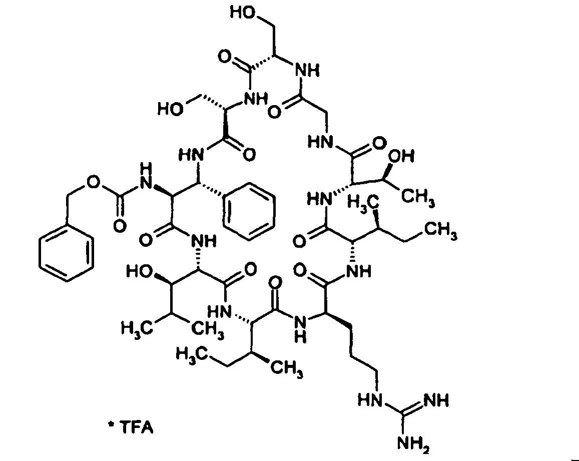 | |
| | [(3*R*)-*N*²-(Benzyloxycarbonyl)-3-amino-L-phenylalanyl]-[(3*R*)-3-hydroxy-L-leucyl]-L-isoleucyl-D-arginyl-L-isoleucyl-L-allothreonyl-glycyl-L-seryl-L-serin-*C^{1.9}*-*N^{3.1}*-lactam-trifluoracetat | HPLC (Methode 6): Rₜ = 3.50 min; LC-MS (Methode 19): Rₜ = 1.62 min, MS (ESIpos): *m*/*z* (%) = 1140.7 (100) [M+H]⁺, MS (ESIneg): *m*/*z* (%) = 1138.7 (100) [M-H]⁻. |
| | Ausbeute: 22 mg (95% rein, 62% d. Th.) aus Beispielverbindung 375A (39 mg, 27 µmol) nach Arbeitsvorschrift 11, Reinigung nach Methode 45. | |
| 400A | 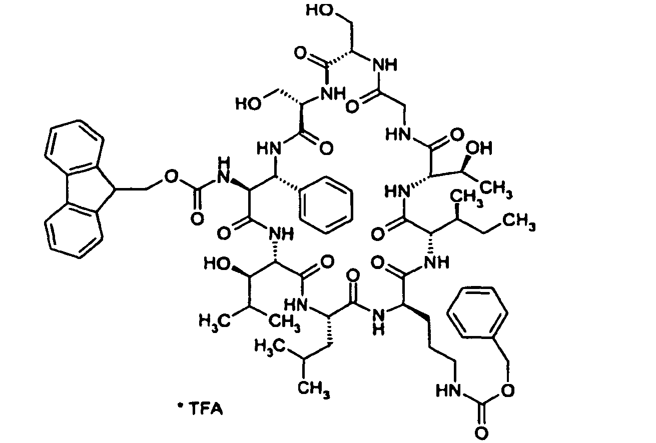 | |
| | {(3*R*)-*N*²-[(9H-fluoren-9-ylmethoxy)carbonyl]-3-amino-L-phenylalanyl]-[(3*R*)-3-hydroxy-L-leucyl]-L-leucyl-(*N*⁵-benzyloxycarbonyl-D-ornithyl)-L-isoleucyl-L-allothreonyl-glycyl-L-seryl-L-serin-*C^{1.9}*-*N^{3.1}*-lactam-trifluoracetat | HPLC (Methode 6): Rₜ = 4.46 min; LC-MS (Methode 19): Rₜ = 2.64 min, MS (ESIpos): *m*/*z* (%) = 1320.7 (100). [M+H]⁺, MS (ESIneg): *m*/*z* (%) = 1319.6 (80) [M-H]⁻; HR-TOF-MS: |
| | Ausbeute: 93 mg (50% rein, 48% d. Th.) aus Beispielverbindung 376A (107 mg, 74 µmol) nach Arbeitsvorschrift 11, Reinigung nach Methode 45. | C₆₇H₉₀N₁₁O₁₇ ber. 1320.6511, gef. 1320.6536 [M+H]⁺. |
| 401A | 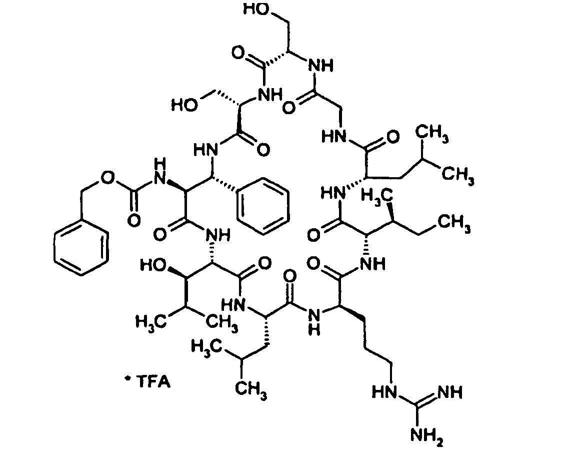 | |
| | [(3*R*)-*N*²-(Benzyioxycarbonyl)-3-amino-L-phenylalanyl]-[(3*R*)-3-hydroxy-L-leucyl]-L-leucyl-D-arginyl-L-isoleucyl-L-leucyl-glycyl-L-seryl-L-serin-*C^{1.9}*-*N^{3.1}*-lactam-trifluoracetat | HPLC (Methode 6): Rₜ = 3.67 min; LC-MS (Methode 19): Rₜ = 1.70 min, MS (ESIpos): *m*/*z* (%) = 1152.7 (100) [M+H]⁺, MS (ESIneg): *m*/*z* (%) = 1150.7 (100) [M-H]⁻; HR-TOF-MS: C₅₅H₈₆N₁₃O₁₄ ber. 1152.6412, gef. 1152.6426 [M+H]⁺. |
| | Ausbeute: 35 mg (97% d. Th.) aus Beispielverbindung 377A (40 mg, 29 µmol) nach Arbeitsvorschrift 11, Reinigung nach Methode 45. | |
| 402A | 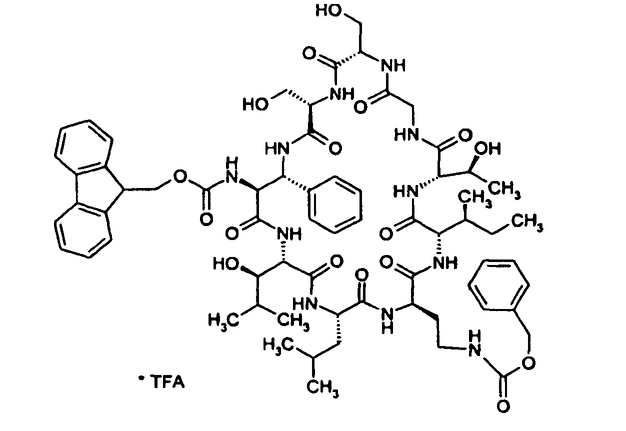 | |
| | ((3*R*)-*N*²-[(9H-fluoren-9-ylmethoxy)carbonyl]-3-amino-L-phenylalanyl)-[(3*R*)-3-hydroxy-L-leucyl]-L-leucyl-[(2*R*)-2-amino-4-benzyloxycarbonylamino-butyryl]-L-isoleucyl-L-allothreonyl-glycyl-L-seryl-L-serin-*C^{1.9}*-*N^{3.1}*-lactam-trifluoracetat | HPLC (Methode 6): Rₜ = 4.45 min; LC-MS (Methode 19): Rₜ = 2.63 min, MS (ESIpos): *m*/*z* (%) = 1306.6 (100) [M+H]⁺, MS (ESIneg): *m*/*z* (%) = 1305.5 (60) [M-H]⁻; HR-TOF-MS: C₆₆H₈₈N₁₁O₁₇ ber. 1306.6355, gef. 1306.6365 [M+H]⁺. |
| | Ausbeute: 96 mg (86% rein, 85% d. Th.) aus Beispielverbindung 378A (107 mg, 74 µmol) nach Arbeitsvorschrift 11, Reinigung nach Methode 45. | |
| 403A | 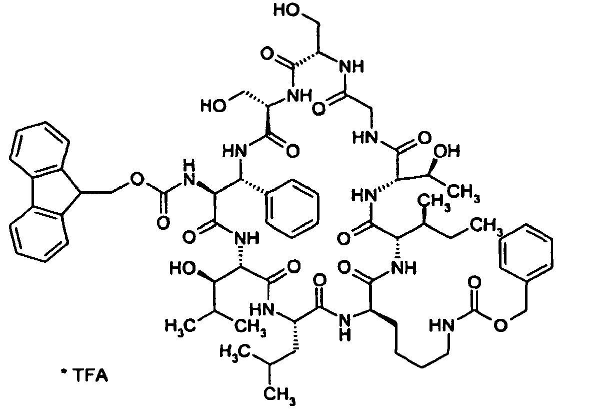 | |
| | {(3*R*)-*N*²-[(9H-fluoren-9-ylmethoxy)carbonyl]-3-amino-L-phenylalanyl}-[(3*R*)-3-hydroxy-L-leucyl]-L-leucyl-(*N*⁶-benzyloxycarbonyl-D-lysyl)-L-isoleucyl-L-allothreonyl-glycyl-L-seryl-L-serin-*C^{1.9}*-*N^{3.1}*-lactam-trifluoracetat | HPLC (Methode 6): Rₜ = 4.44 min; LC-MS (Methode 19): Rₜ = 2.64 min, MS (ESIpos): *m*/*z* (%) = 1335.6 (100) [M+H]⁺, MS (ESIneg): *m*/*z* (%) = 1333.6 (80) [M-H]⁻; HR-TOF-MS: C₆₈H₉₂N₁₁O₁₇ ber. 1334.6668, gef. 1334.6653 [M+H]⁺. |
| | Ausbeute: 46 mg (64% rein, 78% d. Th.) aus Beispielverbindung 379A (48 mg, 26 µmol) nach Arbeitsvorschrift 11, Reinigung nach Methode 45. | |
| 404A | 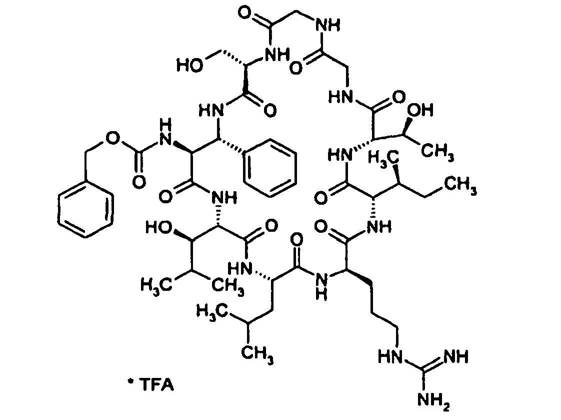 | |
| | [(3*R*)-*N*²-(Benzyloxycarbonyl)-3-amino-L-phenylalanyl]-[(3*R*)-3-hydroxy-L-leucyl]-L-leucyl-D-arginyl-L-isoleucyl-L-allothreonyl-glycyl-glycyl-L-serin-*C^{1.9}*-*N^{3.1}*-lactam-trifluoracetat | HPLC (Methode 6): Rₜ = 3.61 min; LC-MS (Methode 19): Rₜ = 1.62 min, MS (ESIpos): *m*/*z* (%) = 1110.8 (60) [M+H]⁺, MS (ESIneg): *m*/*z* (%) = 1108.8 (100) [M-H]⁻; HR-TOF-MS: C₅₂H₈₀N₁₃O₁₄ ber. 1110.5943, gef. 1110.5898 [M+H]⁺. |
| | Ausbeute: 160 mg (25% rein, 38% d. Th.) aus Beispielverbindung 380A (167 mg, 86 µmol) nach Arbeitsvorschrift 11, Reinigung nach Methode 45. | |
| 405A | 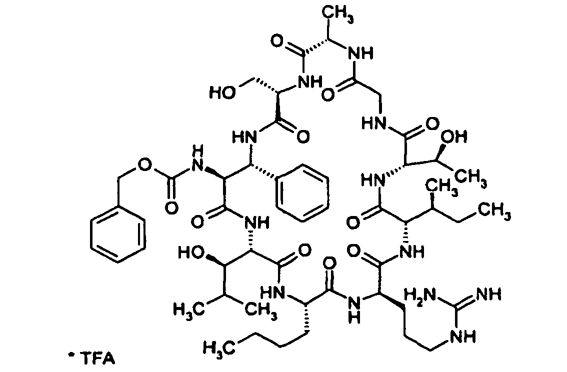 | |
| | [(3*R*)-*N*²-(Benzyloxycarbonyl)-3-amino-L-phenylalanyl]-[(3*R*)-3-hydroxy-L-leucyl]-L-norleucyl-D-arginyl)-L-isoleucyl-L-allothreonyl-glycyl-L-alanyl-L-serin-*C^{1.9}*-*N^{3.1}*-lactam-trifluoracetat | HPLC (Methode 6): Rₜ = 3.65 min; LC-MS (Methode 19): Rₜ = 1.69 min, MS (ESIpos): *m*/*z* (%) = 1124.6 (100) [M+H]⁺, MS (ESIneg): *m*/*z* (%) = 1122.7 (100) [M-H]⁻; HR-TOF-MS: C₅₃H₈₂N₁₃O₁₄ ber. 1124.6099, gef. 1124.6068 [M+H]⁺. |
| | Ausbeute: 74 mg (80% d. Th.) aus Beispielverbindung 381A (103 mg, 75 µmol) nach Arbeitsvorschrift 11, Reinigung nach Methode 45. | |
| 406A | 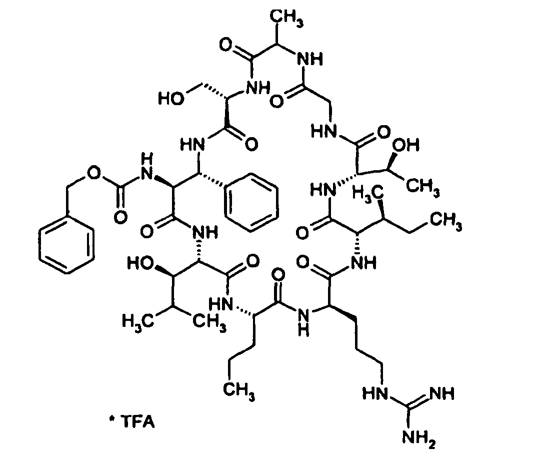 | |
| | [(3*R*)-*N*²-(Benzyloxycarbonyl)-3-amino-L-phenylalanyl]-[(3*R*)-3-hydroxy-L-leucyl]-L-norvalyl-D-arginyl-L-isoleucyl-L-allothreonyl-glycyl-L-alanyl-L-serin-*C^{1.9}*-*N^{3.1}*-lactam-trifluoracetat | HPLC (Methode 6): Rₜ = 3.57 min; LC-MS (Methode 19): Rₜ = 1.62 min, MS (ESIpos): *m*/*z* (%) = 1110.7 (100) [M+H]⁺, MS (ESIneg): *m*/*z* (%) = 1108.6 (100) [M-H]⁻; HR-TOF-MS: C₅₂H₈₀N₁₃O₁₄ ber. 1110.5943, gef. 1110.5936 [M+H]⁺. |
| | Ausbeute: 107 mg (quant.) aus Beispielverbindung 382A (103 mg, 76 µmol) nach Arbeitsvorschrift 11, Reinigung nach Methode 45. | |

### Entschützung der Cyclisierungsprodukte

| **Nr** | **Struktur** | |
|---|---|---|
| | **Name** | **Analytik** |
| | **Ausbeute, Synthesemethode** | |
| 407A | 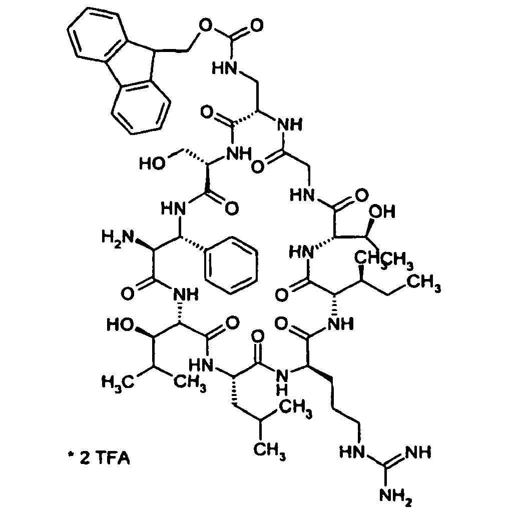 | |
| | [(3*R*)-3-amino-L-phenylalanyl]-[(3*R*)-3-hydroxy-L-leucyl]-L-leucyl-D-arginyl-L-isoleucyl-L-allothreonyl-glycyl-[(2*S*)-2-amino-3-(9H-fluoren-9-ylmethoxycarbonyl)aminobutyryl]-L-serin-*C^{1.9}*-*N^{3.1}*-lactam-bis-hydrochlorid | HPLC (Methode 6): Rₜ = 3.51 min; LC-MS (Methode 19): Rₜ = 1.73 min, MS (ESIpos): *m*/*z* (%) = 614.6 (100) [M+2H]²⁺, 1227.8 (100) [M+H]⁺; HR-TOF-MS: C₆₀H₈₇N₁₄O₁₄ ber. 1227.6521, gef. 1227.6506 [M+H]⁺. |
| | Ausbeute: 368 mg (63% d. Th.) aus Beispielverbindung 383A (590 mg, 400 µmol) nach Arbeitsvorschrift 4, Reinigung nach Methode 34. | |
| 408A | 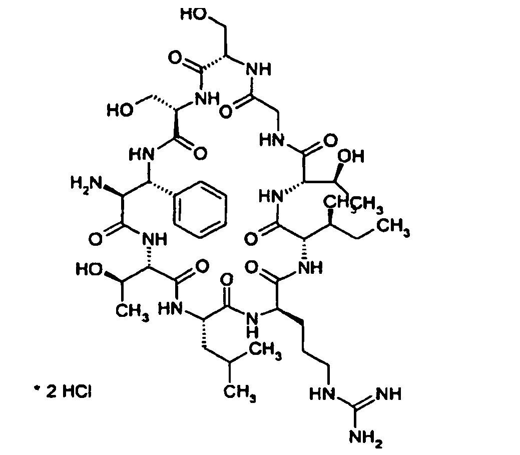 | |
| | [(3*R*)-3-amino-L-phenylalanyl]-L-threonyl-L-leucyl-D-arginyl-L-isoleucyl-L-allothreonyl-glycyl-L-seryl-*C^{1.9}*-*N^{3.1}*-lactam-bis-hydrochlorid | HPLC (Methode 6): Rₜ = 2.87 min; LC-MS (Methode 22): Rₜ = 2.23 min, MS (ESIpos): *m*/*z* (%) = 490 (100) [M+2H]²⁺, MS (ESIneg): *m*/*z* (%) = 977 (100) [M-H]⁻; HR-TOF-MS: C₄₃H₇₂N₁₃O₁₃ ber. 978.5368, gef. 978.5376 [M+H]⁺. |
| | Ausbeute: 10 mg (86% d. Th.) aus Beispielverbindung 384A (13 mg, 11 µmol) nach Arbeitsvorschrift 4. | |
| 409A | 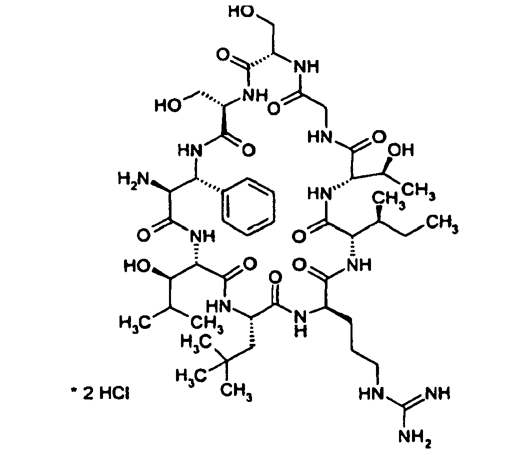 | |
| | [(3*R*)-3-amino-L-phenylalanyl]-[(3*R*)-3-hydroxy-L-leucyl]-[3-*tert*butyl-L-alanyl]-D-arginyl-L-isoleucyl-L-allothreonyl-glycyl-L-seryl-L-serin-*C^{1.9}*-*N^{3.1}*-lactam-bis-hydrochlorid | HPLC (Methode 6): Rₜ = 3.07 min; LC-MS (Methode 19): Rₜ = 0.86 min, MS (ESIpos): *m*/*z* (%) = 511.0 (100) [M+2H]²⁺, MS (ESIneg): *m*/*z* (%) = 1018.8 (100) [M-H]⁻; HR-TOF-MS: C₄₆H₇₈N₁₃O₁₃ ber. 1020.5837, gef. 1020.5822 [M+H]⁺. |
| | Ausbeute: 25 mg (63% d. Th.) aus Beispiel-verbindung 385A (30 mg, 24 µmol) nach Arbeitsvorschrift 4. | |
| 410A | 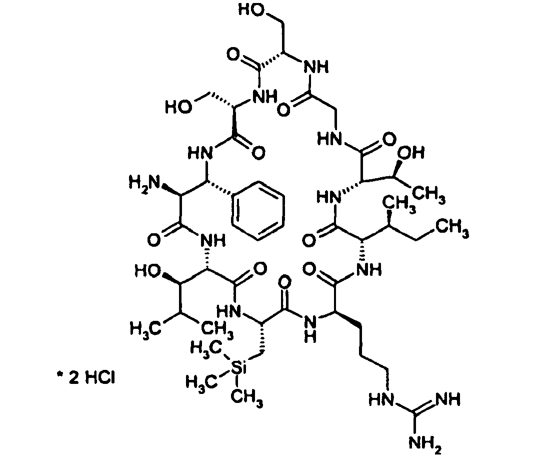 | |
| | [(3*R*)-3-amino-L-phenylalanyl]-[(3*R*)-3-hydroxy-L-leucyl]-(3-trimethylsilyl-L-alanyl)-D-arginyl-L-isoleucyl-L-allothreonyl-glycyl-L-seryl-L-serin-*C^{1.9}*-*N^{3.1}*-lactam-bis-hydrochlorid | HPLC (Methode 6): Rₜ = 3.12 min; LC-MS (Methode 19): Rₜ = 1.04 min, MS (ESIpos): *m*/*z* (%) = 519.2 (100) [M+2H]²⁺, 1036.8 (10) [M+H]⁺, MS (ESIneg): *m*/*z* (%) = 1034.8 (100) [M-H]⁻; HR-TOF-MS: C₄₅H₇₈N₁₃O₁₃Si ber. 1036.5606, gef. 1036.5598 [M+H]⁺. |
| | Ausbeute: 41 mg (90% d. Th.) aus Beispielverbindung 386A (48 mg, 37 µmol) nach Arbeitsvorschrift 4. | |
| 411A | 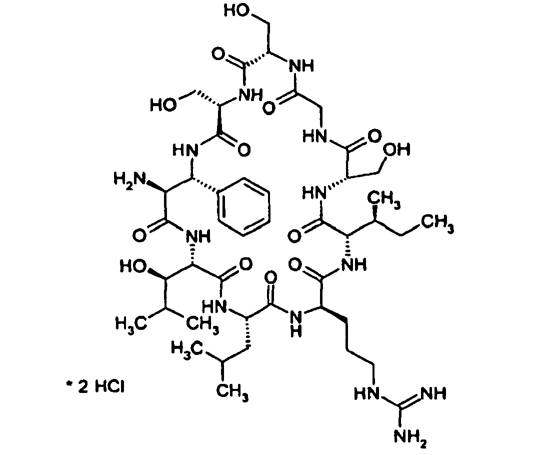 | |
| | [(3*R*)-3-amino-L-phenylalanyl]-[(3*R*)-3-hydroxy-L-leucyl]-L-leucyl-D-arginyl-L-isoleucyl-L-seryl-glycyl-L-seryl-L-serin-*C^{1.9}*-*N^{3.1}*-lactam-bis-hvdrochlorid | HPLC (Methode 6): Rₜ = 2.96 min; LC-MS (Methode 22): Rₜ = 2.29 min, MS (ESIpos): *m*/*z* (%) = 497 (100) [M+2H]²⁺, MS (ESIneg): *m*/*z* (%) = 990.6 (100) [M-H]⁻; HR-TOF-MS: C₄₄H₇₄N₁₃O₁₃ ber. 992.5524, gef. 992.5505 [M+H]⁺. |
| | Ausbeute: 26 mg (98% d. Th.) aus Beispielverbindung 387A (31 mg, 25 µmol) nach Arbeitsvorschrift 4. | |
| 412A | 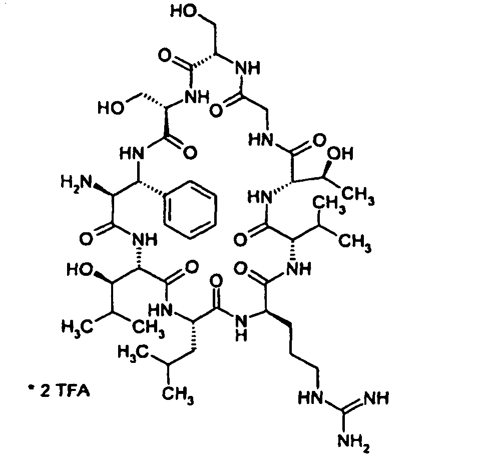 | |
| | [(3*R*)-3-amino-L-phenylalanyl]-[(3*R*)-3-hydroxy-L-leucyl]-L-leucyl-D-arginyl-L-valyl-L-allothreonyl-glycyl-L-seryl-L-serin-*C^{1.9}*-*N^{3.1}*-lactam-bis-trifluoracetat | HPLC (Methode 6): Rₜ = 2.91 min; LC-MS (Methode 19): Rₜ = 0.78 min, MS (ESIpos): *m*/*z* (%) = 496.9 (100) [M+2H]²⁺, 992.6 (10) [M+H]⁺; HR-TOF-MS: C₄₄H₇₄N₁₃O₁₃ ber. 992.5524, gef. 992.5518 [M+H]⁺. |
| | Ausbeute: 57 mg Rohprodukt (-quant.) aus Beispielverbindung 388A (54 mg, 37 µmol) nach Arbeitsvorschrift 4 als gelbliches amorphes Pulver. | |
| 413A | 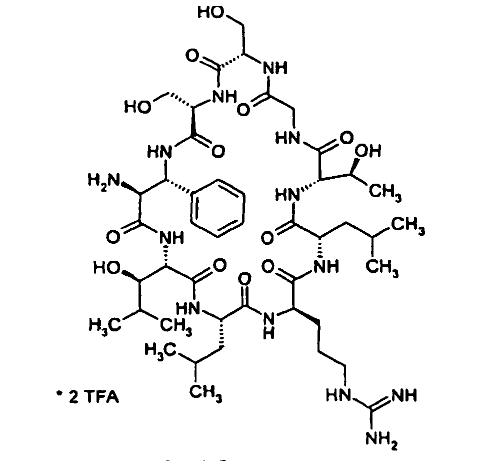 | |
| | [(3*R*)-3-amino-L-phenylalanyl]-[(3*R*)-3-hydroxy-L-leucyl]-L-leucyl-D-arginyl-L-leucyl-L-allothreonyl-glycyl-L-seryl-L-serin-*C^{1.9}*-*N^{3.1}*-lactam-bis-trifluoracetat | HPLC (Methode 6): Rₜ = 2.97 min; LC-MS (Methode 19): Rₜ = 0.95 min, MS (ESIpos): *m*/*z* (%) = 504.0 (100) |
| | Ausbeute: 57 mg (85% rein, 89% d. Th.) aus Beispielverbindung 389A (55 mg, 44 µmol) nach Arbeitsvorschrift 4 als gelbliches amorphes Pulver. | [M+2H]²⁺, MS (ESIneg): *m*/*z* (%) = 1004.7 (10) [M-H]⁻; HR-TOF-MS: C₄₅H₇₅N₁₃O₁₃ ber. 1006.5681, gef. 1006.5715 [M+H]⁺. |
| 414A | 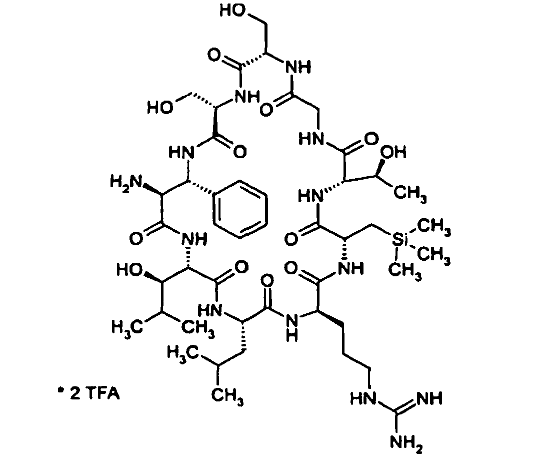 | |
| | [(3*R*)-3-amino-L-phenylalanyl]-[(3*R*)-3-hydroxy-L-leucyl]-L-leucyl-D-arginyl-[3-trimethylsilyl-L-alanlyl]-L-allothreonyl-glycyl-L-seryl-L-serin-*C^{1.9}*-*N^{3.1}*-lactam-bis-trifluoracetat | HPLC (Methode 6): Rₜ = 3.10 min; LC-MS (Methode 19): Rₜ = 1.13 min, MS (ESIpos): *m*/*z* (%) = 519.1 (100) [M+2H]²⁺, 1036.7 (10) [M+H]⁺, MS (ESIneg): *m*/*z* (%) = 1034.6 (100) [M-H]⁻; HR-TOF-MS: C₄₅H₇₈N₁₃O₁₃Si ber. 1036.5606, gef. 1036.5604 [M+H]⁺. |
| | Ausbeute: 74 mg Rohprodukt aus Beispielverbindung 390A (55 mg, 60% rein, 26 µmol) nach Arbeitsvorschrift 4 als gelbliches amorphes Pulver. | |
| 415A | 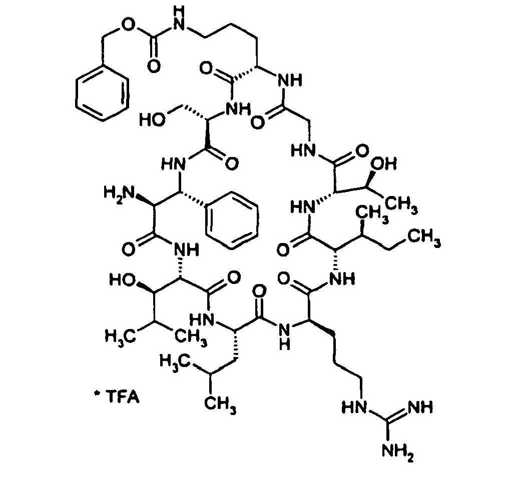 | |
| | (3*R*)-3-amino-L-phenylalanyl)-[(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-*C^{1.9}*-*N^{3.1}*-lactam-bis-hydrochlorid | HPLC (Methode 6): Rₜ = 3.32 min, LC-MS (Methode 19): Rₜ = 1.36 min, MS (ESIpos): *m*/*z* (%) = 584.5 (100) [M+2H]²⁺, 1167.7 (10) [M+H]⁺, MS (ESIneg): *m*/*z* (%) = 1165.7 (100) [M-H]⁻; HR-TOF-MS: C₅₅H₈₇N₁₄O₁₄ ber. 1167.6521, gef. 1167.6541 [M+H]⁺. |
| | Ausbeute: 39 mg (70% rein, 76% d. Th.) aus Beispielverbindung 391A (42 mg, 28 µmol) nach Arbeitsvorschrift 12, Reinigung nach Me-thode 45. | |
| 416A | 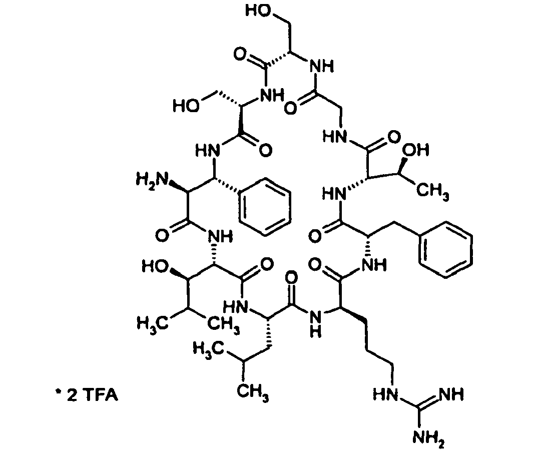 | |
| | [(3*R*)-3-amino-L-phenylalanyl]-[(3*R*)-3-hydroxy-L-leucyl]-L-leucyl-D-arginyl-L-phenylalanlyl-L-allothreonyl-glycyl-L-seryl-L-serin-*C^{1.9}*-*N^{3.1}*-lactam-bis-trifluoracetat | HPLC (Methode 6): Rₜ = 3.01 min; LC-MS (Methode 19): Rₜ = 1.07 min, MS (ESIpos): *m*/*z* (%) = 521.0 (100) [M+2H]²⁺, MS (ESIneg): *m*/*z* (%) = 1038.7 (100) [M-H]⁻; HR-TOF-MS: C₄₈H₇₄N₁₃O₁₃ ber. 1040.5524, gef. 1040.5537 [M+H]⁺. |
| | Ausbeute: 24 mg (71% rein, 77% d. Th.) aus Beispielverbindung 392A (55 mg, 44 µmol) nach Arbeitsvorschrift 4 als gelbliches amorphes Pulver. | |
| 417A | 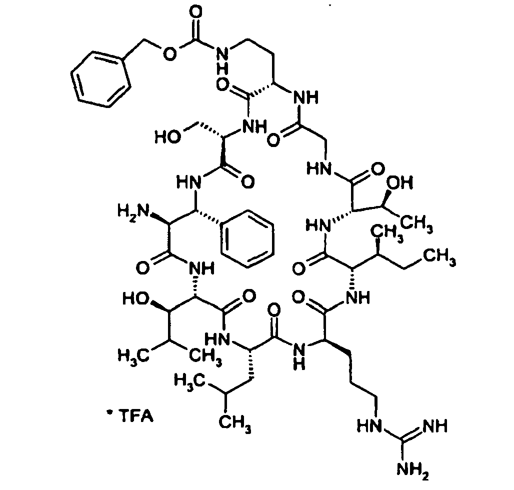 | |
| | {(3*R*)-3-amino-L-phenylalanyl}-[(3*R*)-3-hydroxy-L-leucyl]-L-leucyl-D-arginyl-L-isoleucyl-L-allothreonyl-glycyl-[(2*S*)-2-amino-4-benzyloxycarbonylamino-butyryl]-L-serin-*C^{1.9}*-*N^{3.1}*-lactam-trifluoracetat | HPLC (Methode 6): Rₜ = 3.27 min; LC-MS (Methode 19): Rₜ = 1.34 min, MS (ESIpos): *m*/*z* (%) = 577.6 (100) [M+2H]²⁺; HR-TOF-MS: C₅₄H₈₅N₁₄O₁₄ ber. 1153.6365, gef. 1153.6338 [M+H]⁺. |
| | Ausbeute: 50 mg (76% d. Th.) aus Beispielver-bindung 393A (77 mg, 52 µmol) nach Arbeits-vorschrift 12, Reinigung nach Methode 45. | |
| 418A | 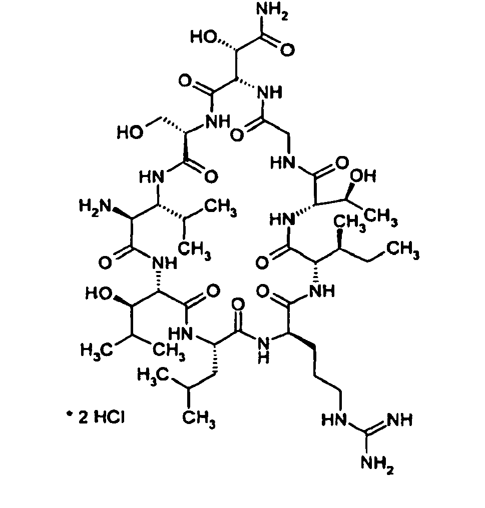 | |
| | [(3*R*)-3-amino-L-leucyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-*C^{1.9}*-*N^{3.1}*-lactam-bis-hydrochlorid | HPLC (Methode 6): Rₜ = 2.90 min; LC-MS (Methode 19): Rₜ = 0.56 min, MS (ESIpos): *m*/*z* (%) = 508.3 (100) [M+2H]²⁺, 1015.6 (20) [M+H]⁺; HR-TOF-MS: C₄₃H₇₉N₁₄O₁₄ ber. 1015.5895, gef. 1015.5869 [M+H]⁺. |
| | Ausbeute: 228 mg (96% d. Th.) aus Beispielverbindung 394A (275 mg, 218 µmol) nach Arbeitsvorschrift 4. | |
| 419A | 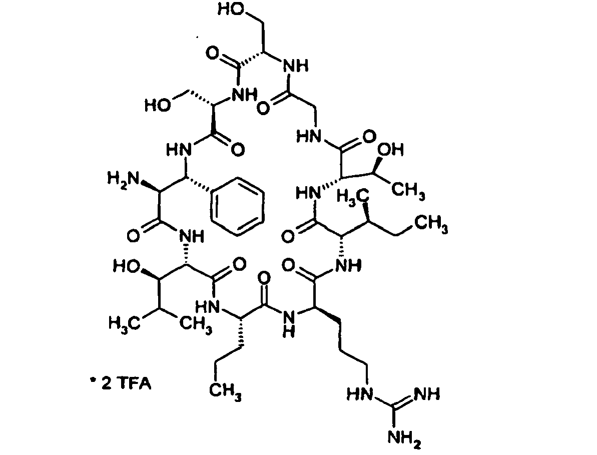 | |
| | [(3*R*)-3-amino-L-phenylalanyl]-[(3*R*)-3-hydroxy-L-leucyl]-L-norvalyl-D-arginyl-L-isoleucyl-L-allothreonyl-glycyl-L-seryl-L-serin-*C^{1.9}*-*N^{3.1}*-lactam-bis-trifluoracetat | HPLC (Methode 6): Rₜ = 2.90 min; LC-MS (Methode 51): Rₜ = 0.95 min, MS (ESIpos): *m*/*z* (%) = 497.4 (100) [M+2H]²⁺, 992.6 (10) [M+H]⁺; HR-TOF-MS: C₄₄H₇₃N₁₃O₁₃ ber. 992.5524, gef. 992.5522 [M+H]⁺. |
| | Ausbeute: 90 mg (68% d. Th.) aus Beispielverbindung 395A (135 mg, 109 µmol) nach Arbeitsvorschrift 4 als gelblicher amorpher Feststoff. | |
| 420A | 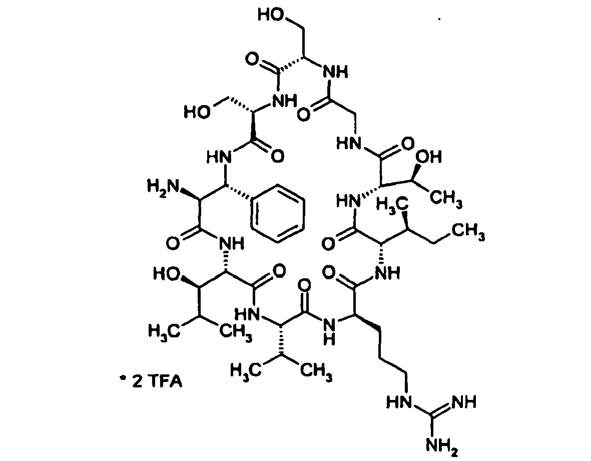 | |
| | [(3*R*)-3-amino-L-phenylalanyl]-[(3*R*)-3-hydroxy-L-leucyl]-L-valyl-D-arginyl-L-isoleucyl-L-allothreonyl-glycyl-L-seryl-L-serin-*C^{1.9}*-*N^{3.1}*-lactam-bis-trifluoracetat | HPLC (Methode 6): Rₜ = 2.90 min; LC-MS (Methode 22): Rₜ = 2.20 min, MS (ESIpos): *m*/*z* (%) = 497.0 (100) [M+2H]²⁺, 992.4 (20) [M+H]⁺, MS (ESIneg): *m*/*z* (%) = 990.5 (100) [M-H] ⁻; HR-TOF-MS: C₄₄H₇₄N₁₃O₁₃ ber. 992.5524, gef. 992.5540 [M+H]⁺. |
| | Ausbeute: 65 mg (Rohprodukt) aus Beispielverbindung 396A (61 mg, 49 µmol) nach Arbeitsvorschrift 4. | |
| 421A | 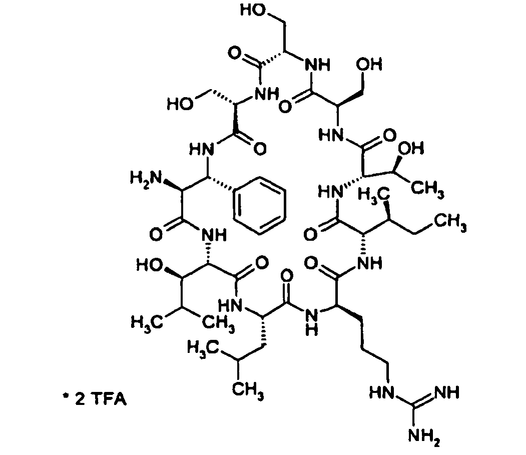 | |
| | [(3*R*)-3-amino-L-phenylalanyl]-[(3*R*)-3-hydroxy-L-leucyl]-L-leucyl-D-arginyl-L-isoleucyl-L-allothreonyl-D-seryl-L-seryl-L-serin-*C^{1.9}*-*N^{3.1}*-lactam-bis-trifluoracetat | HPLC (Methode 6): Rₜ = 2.96 min; LC-MS (Methode 22): Rₜ = 2.39 min, MS (ESIpos): *m*/*z* (%) = 519.1 (100), 1036.6 (10) [M+H]⁺, MS (ESIneg): *m*/*z* (%) = 1034.6 (100) [M-H]⁻; HR-TOF-MS: C₄₆H₇₈N₁₃O₁₄ ber. 1036.5786, gef. 1036.5795 [M+H]⁺. |
| | Ausbeute: 65 mg (93% d. Th.) aus Beispielverbindung 397A (71 mg, 55 µmol) nach Arbeitsvorschrift 4 als gelblicher Feststoff. | |
| 422A | 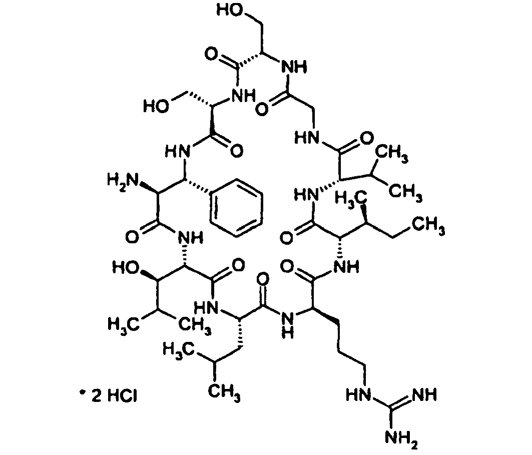 | |
| | [(3*R*)-3-amino-L-phenylalanyl]-[(3*R*)-3-hydroxy-L-leucyl]-L-leucyl-D-arginyl-L-isoleucyl-L-valyl-glycyl-L-seryl-L-serin-*C^{1.9}*-*N^{3.1}*-lactam-bis-hydrochlorid | HPLC (Methode 6): Rₜ = 3.07 min; LC-MS (Methode 51): Rₜ = 1.41 min, MS (ESIpos): *m*/*z* (%) = 503.6 (100) [M+2H]²⁺, MS (ESIneg): *m*/*z* (%) = 1002.8 (100) [M-H]⁻; HR-TOF-MS: C₄₆H₇₈N₁₃O₁₂ ber. 1004.5888, gef. 1004.5897 [M+H]⁺. |
| | Ausbeute: 22 mg (83% d. Th.) aus Beispielverbindung 398A (31 mg, 25 µmol) nach Arbeitsvorschrift 4. | |
| 423A | 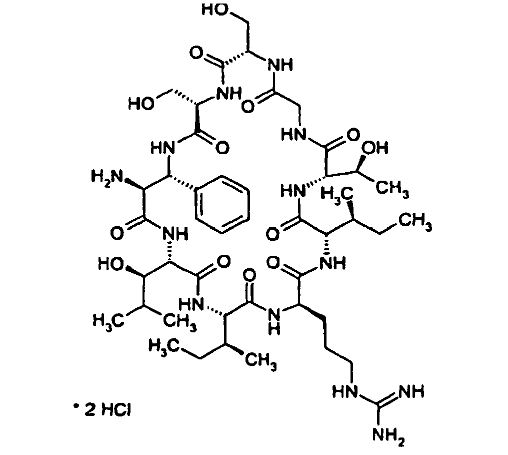 | |
| | [(3*R*)-3-amino-L-phenylalanyl]-[(3*R*)-3-hydroxy-L-leucyl]-L-isoleucyl-D-arginyl-L-isoleucyl-L-allothreonyl-glycyl-L-seryl-L-serin-*C^{1.9}*-*N^{3.1}*-lactam-bis-hydrochlorid | HPLC (Methode 6): Rₜ = 2.93 min; LC-MS (Methode 22): Rₜ = 2.23 min, MS (ESIpos): *m*/*z* (%) = 1006.2 (50) [M+H]⁺, MS (ESIneg): *m*/*z* (%) = 1004.6 (100) [M-H]⁻; HR-TOF-MS: C₄₅H₇₆N₁₃O₁₃ ber. 1006.5681, gef. 1006.5696 [M+H]⁺. |
| | Ausbeute: 19 mg (93% rein, 93% d. Th.) aus Beispielverbindung 399A (22 mg, 18 µmol) nach Arbeitsvorschrift 4. | |
| 424A | 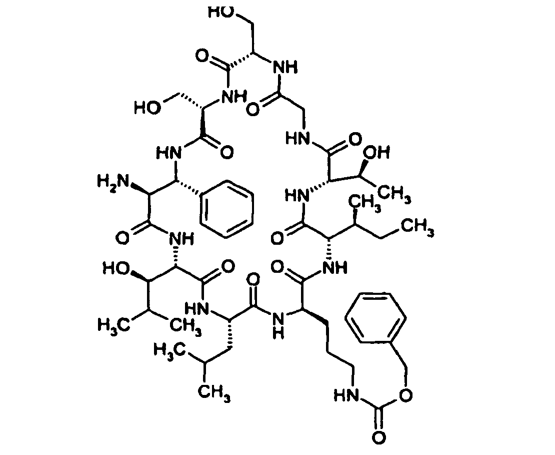 | |
| | {(3*R*)-3-amino-L-phenylalanyl}-[(3*R*)-3-hydroxy-L-leucyl]-L-leucyl-(*N*⁵-benzyloxycarbonyl-D-ornithyl)-L-isoleucyl-L-allothreonyl-glycyl-L-seryl-L-serin-*C^{1.9}*-*N^{3.1}*-lactam | HPLC (Methode 6): Rₜ = 3.63 min; LC-MS (Methode 19): Rₜ = 1.72 min, MS (ESIpos): *m*/*z* (%) = 1098.7 (100) [M+H]⁺, MS (ESIneg): *m*/*z* (%) = 1096.7 (100) [M-H]⁻; HR-TOF-MS: C₅₂H₈₀N₁₁O₁₅ ber. 1098.5830, gef. 1098.5825 [M+H]⁺. |
| | Ausbeute: 95 mg (80% rein, 71% d. Th.) aus Beispielverbindung 400A (130 mg, 98 µmol) nach Arbeitsvorschrift 12. | |
| 425A | 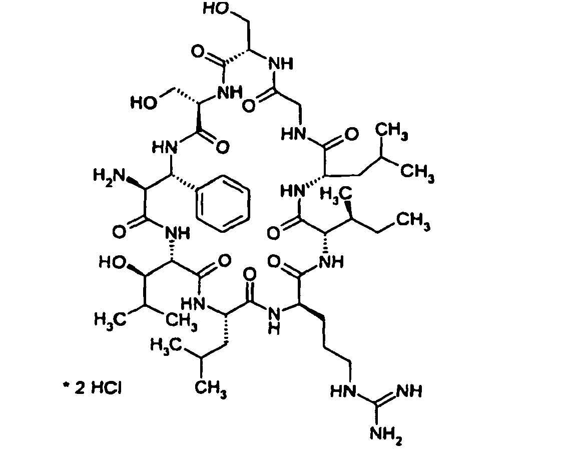 | |
| | [(3*R*)-3-amino-L-phenylalanyl]-[(3*R*)-3-hydroxy-L-leucyl]-L-leucyl-D-arginyl-L-isoleucyl-L-leucyl-glycyl-L-seryl-L-serin-*C^{1.9}*-*N^{3.1}*-lactam-bis-hydrochlorid | HPLC (Methode 6): Rₜ = 3.14 min; LC-MS (Methode 19): Rₜ = 1.25 min, MS (ESIpos): *m*/*z* (%) = 509.9 (100) [M+2H]²⁺, 1018.6 (10) [M+H]⁺, MS (ESIneg): *m*/*z* (%) = 1016.7 (100) [M-H]⁻; HR-TOF-MS: C₄₇H₈₀N₁₃O₁₂ ber. 1018.6044, gef. 1018.6025 [M+H]⁺. |
| | Ausbeute: 35 mg (84% rein, 97% d. Th.) aus Beispielverbindung 401A (35 mg, 28 µmol) nach Arbeitsvorschrift 4. | |
| 426A | 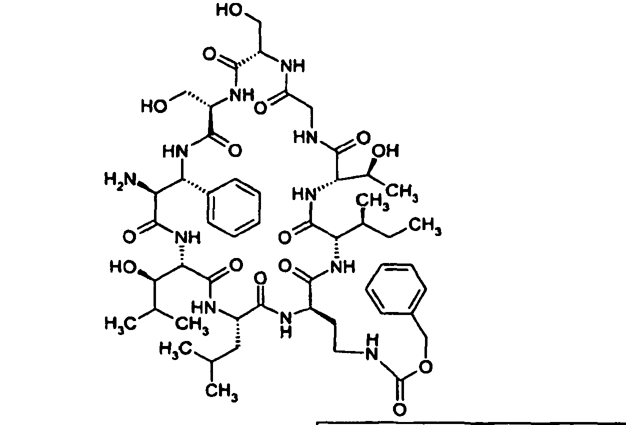 | |
| | {(3*R*)-3-amino-L-phenylalanyl}-[(3*R*)-3-hydroxy-L-leucyl]-L-leucyl-[(2*R*)-2-amino-4-benzyloxycarbonylamino-butyryl]-L-isoleucyl-L-allothreonyl-glycyl-L-seryl-L-serin-*C^{1.9}*-*N^{3.1}*-lactam | HPLC (Methode 6): Rₜ = 3.63 min; LC-MS (Methode 19): Rₜ = 1.70 min, MS (ESIpos): *m*/*z* (%) = 1084.6 (100) [M+H]⁺, MS (ESIneg): *m*/*z* (%) = 1082.6 (100) [M-H]⁻; HR-TOF-MS: C₅₁H₇₈N₁₁O₁₅ ber. 1084.5674, gef. 1084.5679 [M+H]⁺. |
| | Ausbeute: 112 mg (60% rein, 62% d. Th.) aus Beispielverbindung 402A (130 mg, 100 µmol) nach Arbeitsvorschrift 12. | |
| 427A | 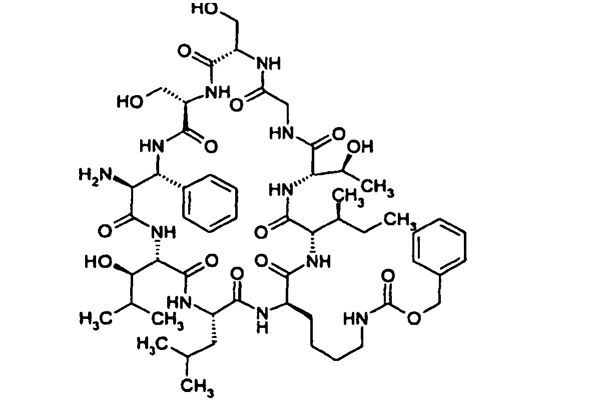 | |
| | {(3*R*)-3-amino-L-phenylalanyl}-(3*R*)-3-hydroxy-L-leucyl]-L-leucyl-(*N*⁶-benzytoxycarbonyl-D-lysyl)-L-isoteucyl-L-allothreonyl-glycyl-L-seryl-L-serin-*C^{1.9}*-*N^{3.1}*-lactam | HPLC (Methode 6): Rₜ = 3.67 min; LC-MS (Methode 19): Rₜ = 1.77 min, MS (ESIpos): *m*/*z* (%) = 1112.6 (100) [M+H]⁺, MS (ESIneg): *m*/*z* (%) = 1110.6 (100) [M-H]⁻; HR-TOF-MS: C₅₃H₈₂N₁₁O₁₅ ber. 1112.5987, gef. 1112.5979 [M+H]⁺. |
| | Ausbeute: 21 mg (92% d. Th.) aus Beispiel-verbindung 403A (46 mg, 20 µmol) nach Ar-beitsvorschrift 12. | |
| 428A | 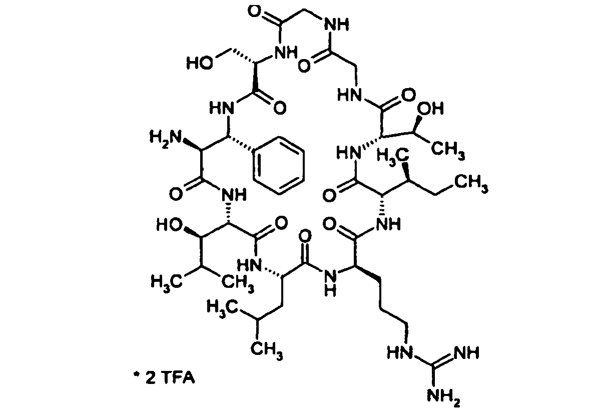 | |
| | [(3*R*)-3-amino-L-phenylalanyl]-[(3*R*)-3-hydroxy-L-leucyl]-L-leucyl-D-arginyl-L-isoleucyl-L-allothreonyl-glycyl-glycyl-L-serin-*C^{1.9}*-*N^{3.1}*-lactam-bis-trifluoracetat | HPLC (Methode 6): Rₜ = 3.00 min; LC-MS (Methode 22): Rₜ = 2.29 min, MS (ESIpos): *m*/*z* (%) = 489 (100) [M+2H]²⁺, MS (ESIneg): *m*/*z* (%) = 975 (100) [M-H]⁻; HR-TOF-MS: C₄₄H₇₄N₁₃O₁₂ ber. 976.5575, gef. 976.5537 [M+H]⁺. |
| | Ausbeute: 160 mg (97% d. Th.) aus Beispielverbindung 404A (167 mg, 136 µmol) nach Arbeitsvorschrift 4 als amorpher Feststoff. | |
| 429A | 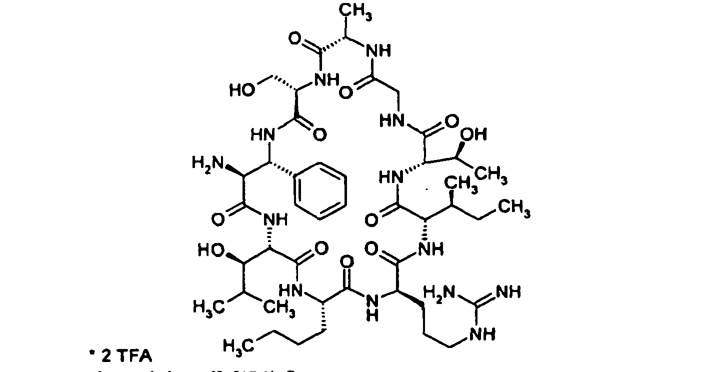 | |
| | [(3*R*)-3-amino-L-phenylalanyl]-[(3*R*)-3-hydroxy-L-leucyl]-L-norleucyl-D-arginyl)-L-isoleucyl-L-allothreonyl-glycyl-L-alanyl-L-serin-*C^{1.9}*-*N^{3.1}*-lactam-bis-trifluoracetat | HPLC (Methode 6): Rₜ = 3.02 min; LC-MS (Methode 19): Rₜ = 1.04 min, MS (ESIpos): *m*/*z* (%) = 990.6 (100) [M+H]⁺, MS (ESIneg): *m*/*z* (%) = 988.6 (100) [M-H]⁻; HR-TOF-MS: C₄₅H₇₆N₁₃O₁₂ ber. 990.5731, gef. 990.5715 [M+H]⁺. |
| | Ausbeute: 77 mg (55% rein, 56% d. Th.) aus Beispielverbindung 405A (74 mg, 60 µmol) nach Arbeitsvorschrift 4, Reinigung nach Me-thode 45. | |
| 430A | 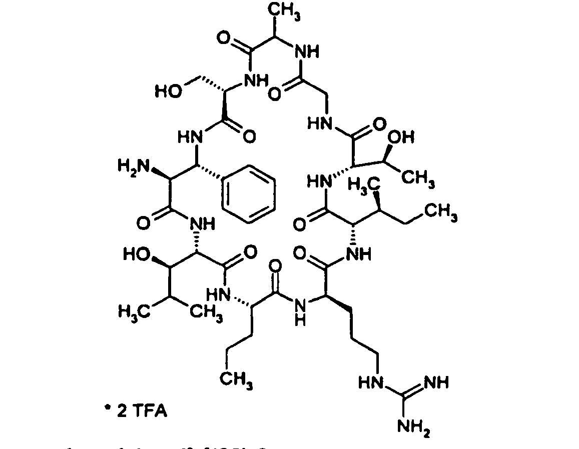 | |
| | [(3*R*)-3-amino-L-phenylalanyl]-[(3*R*)-3-hydroxy-L-leucyl]-L-norvalyl-D-arginyl-L-isoleucyl-L-allothreonyl-glycyl-L-alanyl-L-serin-*C^{1.9}*-*N^{3.1}*-lactam-bis-trifluoracetat | HPLC (Methode 6): Rₜ = 2.96 min; LC-MS (Methode 22): Rₜ = 2.21 min, MS (ESIpos): *m*/*z* (%) = 976.5 (80) [M+H]⁺, MS (ESIneg): *m*/*z* (%) = 975.5 (100) [M-H]⁻; HR-TOF-MS: C₄₄H₇₄N₁₃O₁₂ ber. 976.5575, gef. 976.5552 [M+H]⁺. |
| | Ausbeute: 111 mg (quant.) aus Beispielverbindung 406A (107 mg, 87 µmol) nach Arbeitsvorschrift 4, Reinigung nach Methode 45. | |

### Ankupplung des Nterminalen Dipeptidfragments

| **Beispiel -Nr** | **Struktur** | |
|---|---|---|
| | **Name** | **Analytik** |
| | **Ausbeute, Synthesemethode** | |
| 431A | 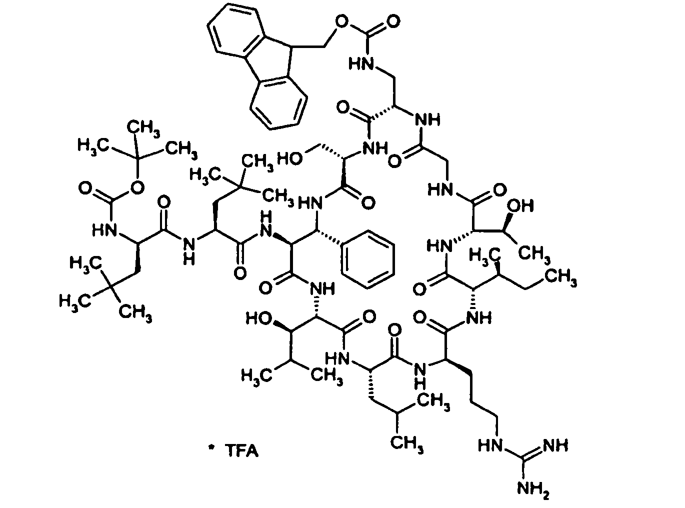 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-[(2*S*)-2-amino-3-(9H-fluoren-9-ylmethoxycarbonyl)aminobutyryl]-L-serin-*C^{1.11}*-*N^{3.3}*-lactam-trifluoracetat | HPLC (Methode 6): Rₜ = 4.89 min; LC-MS (Methode 19): Rₜ = 2.46 min, MS (ESIpos): *m*/*z* (%) = 1582.8 (100) [M+H]⁺; HR-TOF-MS: C₇₉H₁₂₁N₁₆O₁₈ ber. 1581.9040, gef. 1581.9067 [M+H]⁺. |
| | Ausbeute: 310 mg (78% d. Th.) aus Beispiel-verbindung 407A (343 mg, 236 µmol) und Bei-spielverbindung 8A nach Arbeitsvorschrift 13. | |
| 432A | 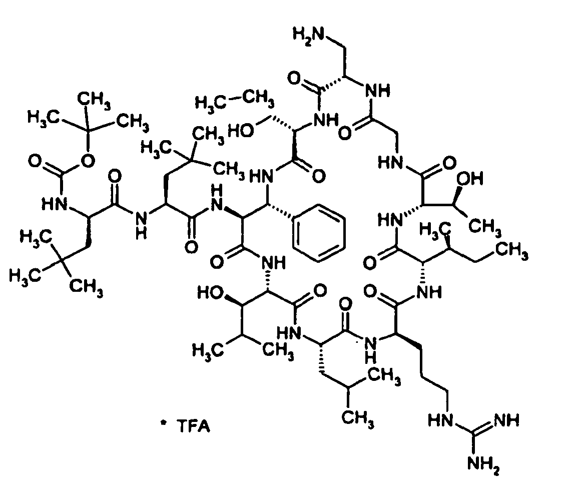 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-[(2*S*)-2,3-diaminobutyryl]-L-serin-*C^{1.11}*-*N^{3.3}*-lactam-trifluoracetat | HPLC (Methode 6): Rₜ= 3.99 min; LC-MS (Methode 19): Rₜ = 2.20 min, MS (ESIpos): *m*/*z* (%) = 680.8 (100) [M+2H]²⁺; HR-TOF-MS: C₆₄H₁₁₁N₁₆O₁₆ ber. 1359.8359, gef. 1359.8328 [M+H]⁺. |
| | Ausbeute: 273 mg (quant.) aus Beispielverbindung 431A (310 mg, 183 µmol) nach Arbeitsvorschrift 12, Reinigung nach Methode 45. | |
| 433A | 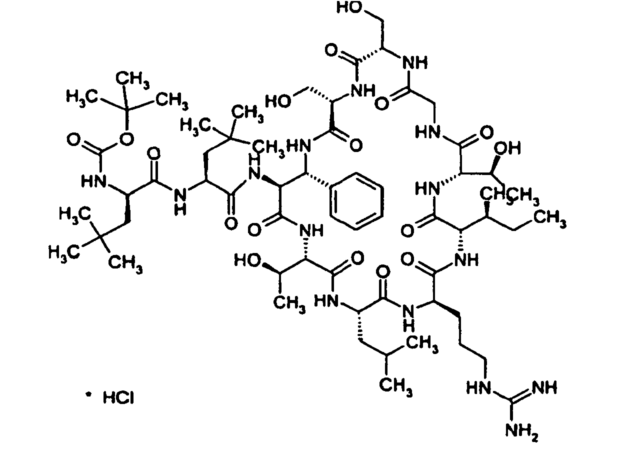 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-L-threonyl-L-leucyl-D-arginyl-L-isoleucyl-L-allothreonyl-glycyl-L-serin-*C^{1.11}*-*N^{3.3}*-lactam-hydrochlorid | HPLC (Methode 6): Rₜ = 4.21 min; LC-MS (Methode 22): Rₜ = 3.59 min, MS (ESIpos): *m*/*z* (%) = 617 (100) [M+2H]²⁺, 1333 (20) [M+H]⁺, MS (ESI-neg): *m*/*z* (%) = 1331 (100) [M-H]⁻; |
| | Ausbeute: 6.5 mg (50% d. Th.) aus Beispielverbindung 408A (10 mg, 10 µmol) und Beispielverbindung 8A nach Arbeitsvorschrift 13. | HR-TOF-MS: C₆₀H₁₀₂N₁₇O₁₇ ber. 1332.7635, gef. 1332.7664 [M+H]⁺. |
| 434A | 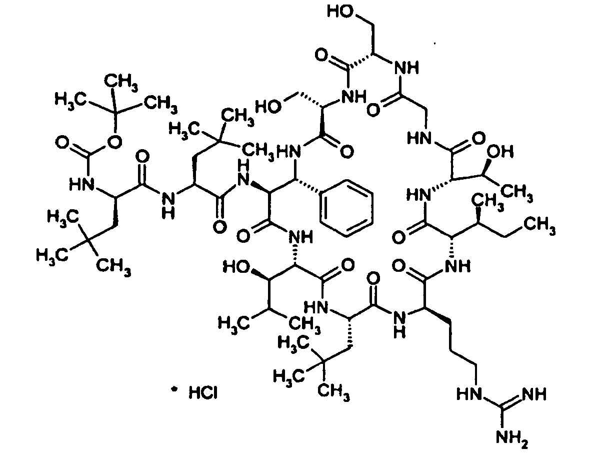 | |
| | [*N*²-(*tert*.-Butoxycarbonyl)-3-*tert* butyl-D-alanyl]-[3-*tert*-butyl-L-alanyl]-[(3*R*)-3-amino-L-phenylalanyl]-[(*3R*)-3-hydroxy-L-leucyl]-[3-*tert*-butyL-L-alanyl]-D-arginyl-L-isoleucyl-L-allothreonyl-glycyl-L-seryl-L-serin-*C^{1.11}*-*N^{3.3}*-lactam-hydrochlorid | HPLC (Methode 6): Rₜ = 4.46 min; LC-MS (Methode 19): Rₜ = 2.15 min, MS (ESIpos): *m*/*z* (%) = 638.2 (100) [M+2H]²⁺, 1375 (40) [M+H]⁺; HR-TOF-MS: C₆₅H₁₁₂N₁₅O₁₇ ber. 1374.8356, gef. 1374.8351 [M+H]⁺. |
| | Ausbeute: 22 mg (68% d. Th.) aus Beispielverbindung 409A (25 mg, 23 µmol) und Beispielverbindung 8A nach Arbeitsvorschrift 13. | |
| 435A | 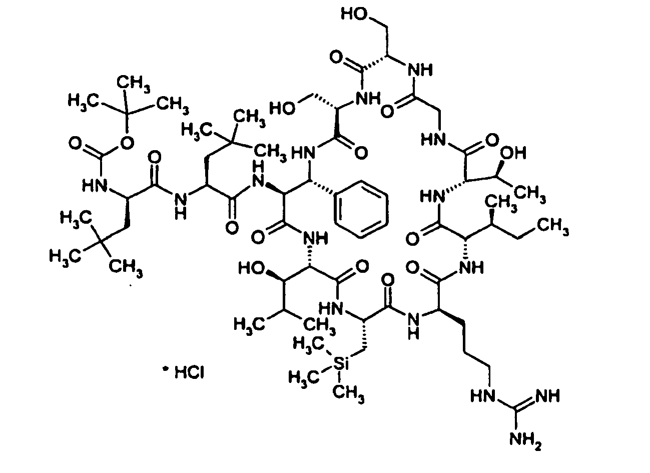 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-[3-trimethylsiyl-L-alanyl]-D-arginyl-L-isoteucyl-L-allothreonyl-glycyl-L-seryl-L-serin-*C^{1.11}*-*N^{3.3}*-lactam-hydrochlorid | HPLC (Methode 6): Rₜ = 4.53 min; LC-MS (Methode 19): Rₜ = 2.19 min, MS (ESIpos): *m*/*z* (%) = 646.3 (100) [M+2H]²⁺, 1392.1 (20) [M+H]⁺, MS (ESIneg): m/z (%) = 1389.3 (50) [M.H] ; HR-TOF-MS: C₆₄H₁₁₂N₁₅O₁₇Si ber. 1390.8125, gef. 1390.8101 [M+H]⁺. |
| | Ausbeute: 29 mg (56% d. Th.) aus Beispielverbindung 410A (40 mg, 36 µmol) und Beispielverbindung 8A nach Arbeitsvorschrift 13. | |
| 436A | 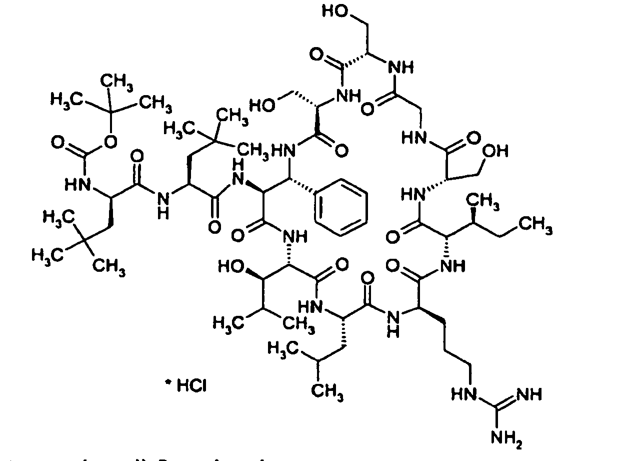 | |
| | [N²-(*tert*.-Butoxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-seryl-glycyl-L-seryl-L-serin-*C^{1.11}*-*N^{3.3}*-lactam-hydrochlorid | HPLC (Methode 6): Rₜ = 4.20 min; LC-MS (Methode 19): Rₜ = 2.08 min, MS (ESIpos): m/z (%) = 1346.9 (40) [M+H]⁺, MS (ESIneg): m/z (%) = 1344.9 (40) [M-H]⁻; HR-TOF-MS: C₆₃H₁₀₈N₁₅O₁₇ ber. 1346.8043, gef. 1346.8036 [M+H]⁺. |
| | Ausbeute: 23 mg (66% d. Th.) aus Beispielver-bindung 411A (26 mg, 24 µmol) und Beispielverbindung 8A nach Arbeitsvorschrift 13. | |
| 437A | 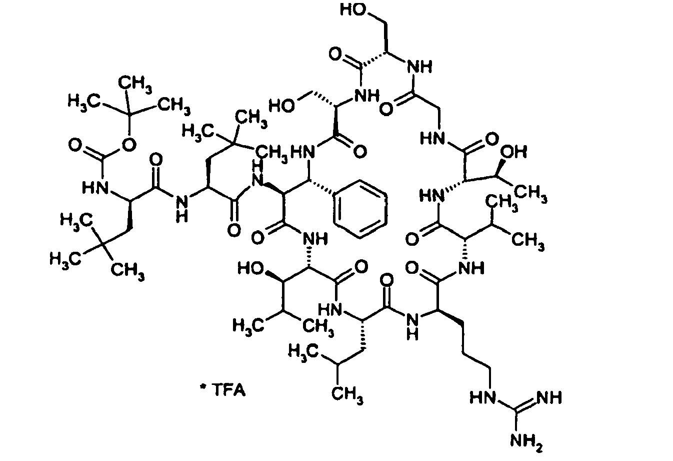 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-valyl-L-allothreonyl-glycyl-L-seryl-L-serin-*C^{1.11}*-*N^{3.3}*-lactam-trifluoracetat | HPLC (Methode 6): Rₜ = 4.23 min; LC-MS (Methode 19): Rₜ = 2.06 min, MS (ESIpos): *m*/*z* (%) = 1346.8 (40) [M+H]⁺, MS (ESIneg): m/Z (%) = 1344.9 (60) [M-H] ; HR-TOF-MS: C₆₃H₁₀₈N₁₅O₁₇ ber. 1346.8043, gef. 1346.8047 [M+H]⁺. |
| | Ausbeute: 75 mg (71% rein, 78% d. Th.) aus Beispielverbindung 412A (57 mg, 47 µmol) und Beispielverbindung 8A nach Arbeitsvorschrift 13. | |
| 438A | 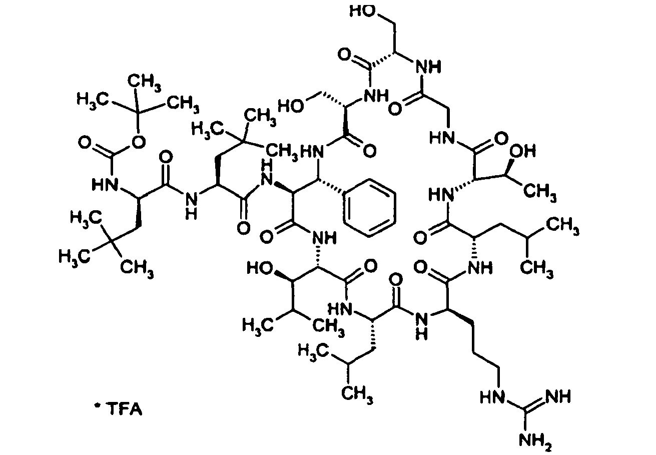 | |
| | [*N²*-(*tert.*-Butoxycarbonyl)-3-*tert*-butyl-D-alanyl]-[3-*tert*-butyl-L-alanyl]-[(3R)-3-amino-L-phenylalanyl]-[(*3R*)-3-hydroxy-L-leucyl]-L-leucyl-D-arginyl-L-leucyl-L-allothreonyl-glycyl-L-seryl-L-serin-*C^{1.11}N^{3.3}*.lactam-trifluoracetat | HPLC (Methode 6): Rₜ = 4.39 min; LC-MS (Methode 19): Rₜ = 2.14 min, MS (ESIpos): *m*/*z* (%) = 1360.0 (40) [M+H]⁺; HR-TOF-MS: C₆₄H₁₁₀N₁₅O₁₇ ber. 1360.8199, gef. 1360.8199 |
| | Ausbeute: 72 mg (quant.) aus Beispielverbindung 413A (60 mg, 49 µmol) und Beispielverbindung 8A nach Arbeitsvorschrift 13. | [M+H]⁺. |
| 439A | 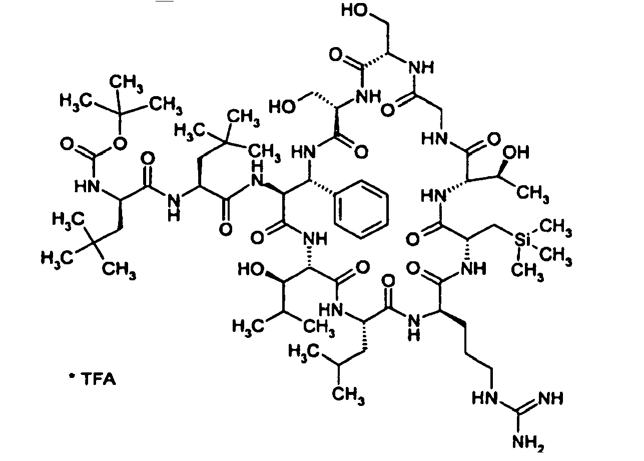 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-[3-trimethylsilyl-L-alanlyl]-L-allothreonyl-glycyl-L-seryl-L-serin-*C^{1.11}-N^{3.3}*-lactam-trifluoracetat | HPLC (Methode 6): Rₜ = 4.53 min; LC-MS (Methode 19): Rₜ = 2.18 min, MS (ESIpos): *m*/*z* (%) = 1391.8 (40) [M+H]⁺, MS (ESIneg): *m*/*z* (%) = 1388.8 (30) [M-H] ; HR-TOF-MS: C₆₄H₁₂N₁₅O₁₇Si ber. 1390.8125, gef. 1390.8092 [M+H]⁺. |
| | Ausbeute: 62 mg (54% rein, 63% d. Th.) aus Beispielverbindung 414A (74 mg, 35 µmol) und Beispielverbindung 8A nach Arbeits-vorschrift 13. | |
| 440A | 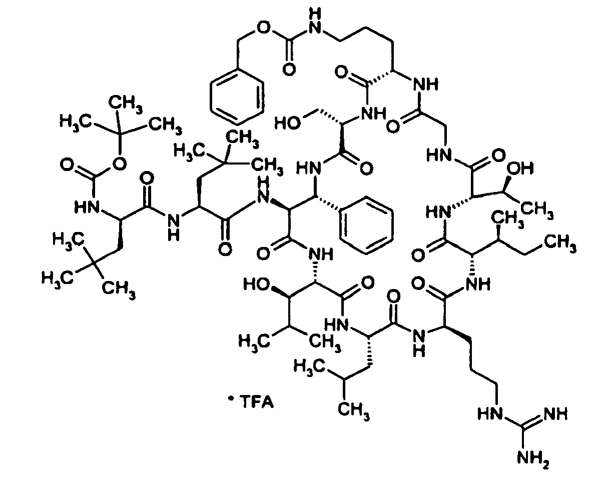 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-*C^{1.11}*-*N^{3.3}*-lactam-trifluoracetat | HPLC (Methode 6): Rₜ = 5.17 min; LC-MS (Methode 19): Rₜ = 2.33 min, MS (ESIpos): *m*/*z* (%) = 1522.9 (30) (M+H]⁺, MS (ESIneg): m/z (%) = 1520.0 (30) [M-H]⁻ ; HR-TOF-MS: 1520.0 (30) [M-H]⁻ ; HR-TOF-MS: C₇₄H₁₂₁N₁₆O₁₈ ber. 1521.9040, gef. 1521.9021 [M+H]⁺. |
| | Ausbeute: 39 mg (79% d. Th.) aus Beispielverbindung 415A (39 mg, 30 µmol) und Beispielverbindung 8A nach Arbeitsvor-schrift 13. | |
| 441A | 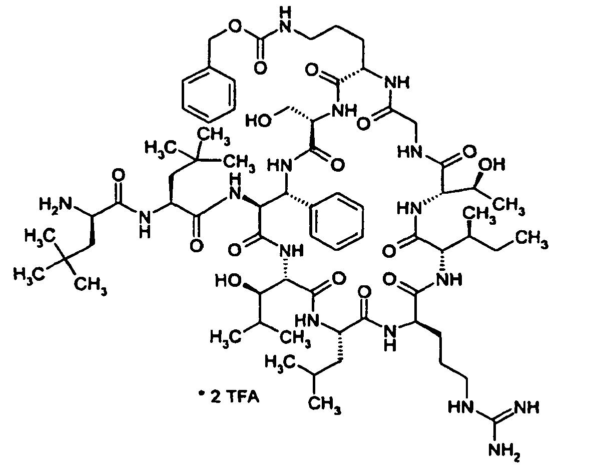 | |
| | [3-*tert*-Butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-(*N*⁵-benzyloxycarbonyl-L-ornithyl-L-serin-*C^{1.11}*-*N^{3.3}*-lactam-bis-trifluoracetat | HPLC (Methode 6): Rₜ = 3.72 min. |
| | Ausbeute: 65 mg Rohprodukt aus Beispielverbindung 440A (39 mg, 24 µmol) nach Arbeitsvorschrift 2. | |
| 442A | 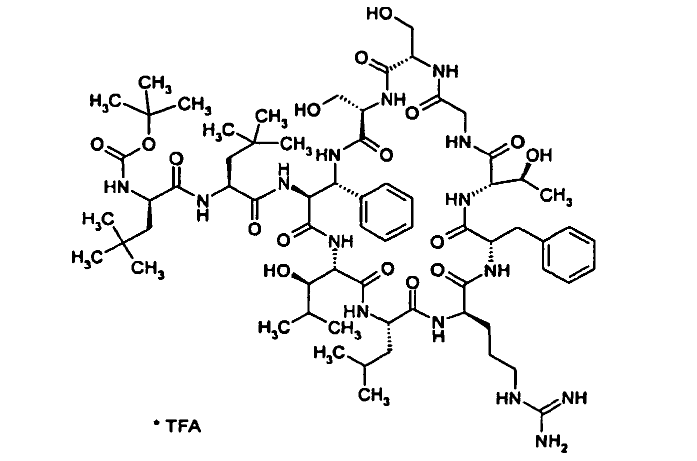 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-phenylalanlyl-L-allothreonyl-glycyl-L-seryl-L-serin-*C^{1.11}*-*N^{3.3}*-lactam-trifluoracetat | HPLC (Methode 6): Rₜ = 4.32 min; LC-MS (Methode 19): Rₜ = 1394.8 (30) [M+H]⁺ min, MS (ESIpos): m/z (%) =, MS (ESIneg): *m*/*z* (%) = 1392.9 [M-H] ; HR-TOF-MS: C₆₇H₁₀₈N₁₅O₁₇ ber. 1394.8043, gef. 1394.8065 [M+H]⁺. |
| | Ausbeute: 12 mg (40% d. Th.) aus Beispielver-bindung 416A (24 mg, 19 µmol) und Beispielverbindung 8A nach Arbeitsvorschrift 13. | |
| 443A | 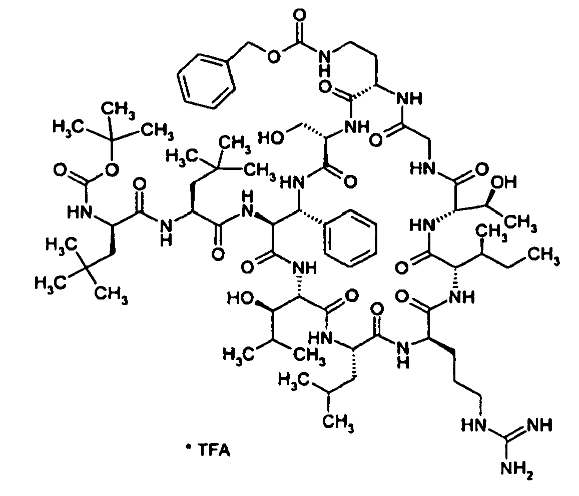 | |
| | [*N*²-(*tert*.-Butoxycarbonyl)-3-*tert* butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-[(2*S*)-2-amino-4-benzyloxycarbonylamino-butyryl]-L-serin-*C^{1.11}*-*N^{3.3}*-lactam-trifluoracetat | HPLC (Methode 6): Rₜ = 4.65 min; LC-MS (Methode 19): Rₜ = 2.32 min, MS (ESIpos): *m*/*z* (%) = 1508.8 (10) [M+H]⁺, MS (ESIneg): *m*/*z* (%) = 1506.7 (60) [M-H] ; HR-TOF-MS: C₇₃H₁₁₉N₁₆O₁₈ ber. 1507.8883, gef. 1507.8895 [M+H]⁺. |
| | Ausbeute: 61 mg (95% d. Th.) aus Beispielverbindung 417A (50 mg, 39 µmol) und Beispielverbindung 8A nach Arbeitsvorschrift 13. | |
| 444A | 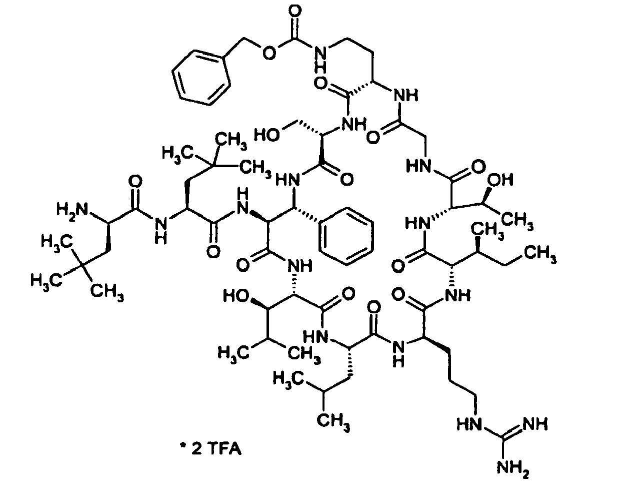 | |
| | [3-*tert*-Butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-allothreonyl-glycyl-[(2*S*)-2-amino-4-benzyloxycarbonylamino-butyryl]-L-serin-*C^{1.11}*-*N^{3.3}*-lactam-bis-trifluoracetat | HPLC (Methode 6): Rₜ = 3.65 min; LC-MS (Methode 19): Rₜ = 1.96 min, MS (ESIpos): *m*/*z* (%) = 704.7 (100) [M+2H]²⁺, 1407 (1) [M+H]⁺, MS (ESI-neg): *m*/*z* (%) = 1405.9 (100) [M-H]⁻. |
| | Ausbeute: 75 mg (Rohprodukt) aus Beispielverbindung 443A (61 mg, 46 µmol) nach Arbeitsvorschrift 2. | |
| 445A | 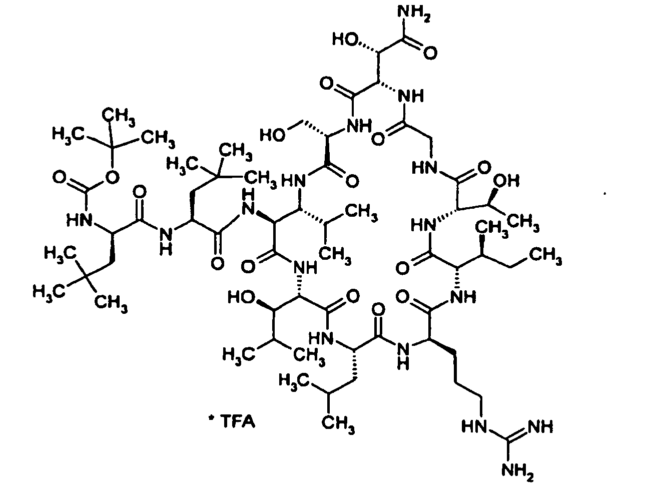 | |
| | [*N*²-(*tert*.-Butoxycarbonyl)-3-*tert*-butyl-D-alanyl]-[3-*tert*-butyl-L-alanyl]-[(3*R*)-3-amino-L-leucyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-*C^{1.11}*-*N^{3.3}*-lactam-trifluoracetat | HPLC (Methode 6): Rₜ = 4.29 min; LC-MS (Methode 19): Rₜ = 2.11 min, MS (ESIpos): *m*/*z* (%) = 1369.8 (50) [M+H]⁺, MS (ESIneg): *m*/*z* (%) = 1367.9 (40) [M-H]⁻ ; HR-TOF-MS: C₆₂H₁₁₃N₁₆O₁₈ ber. 1369.8414, gef. 1369.8396 [M+H]⁺. |
| | Ausbeute: 53 mg (17% d. Th.) aus Beispielverbindung 445A (228 mg, 30 µmol) und Bei-spielverbindung 8A nach Arbeitsvorschrift 13, Abtrennung vom anderen Diastereomer mittels Methode 44. | |
| 446A | 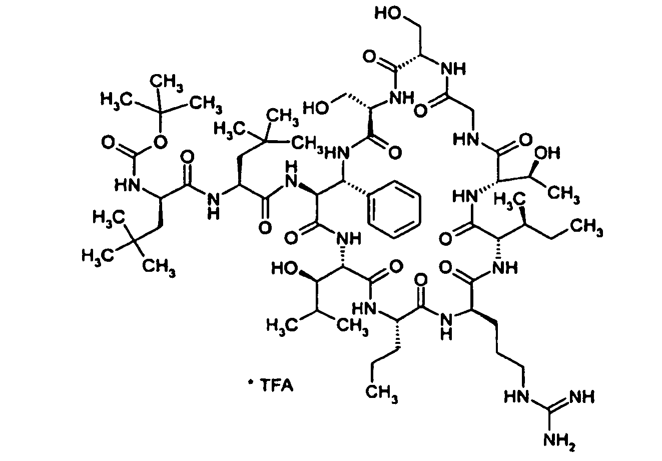 | |
| | [*N*²-(*tert*.-Butoxycarbonyl)-3-*tert* butyl-D-alanyl]-[3-*tert*-butyl-L-alanyl]-[(3*R*)-3-amino-L-phenylalanyl]-[(3*R*)-3-hydroxy-L-leucyl]-L-norvalyl-D-arginyl-L-isoleucyl-L-allothreonyl-glycyl-L-seryl-L-serin-*C^{1.9}-N^{3.1}*-lactam-*C^{1.11}-N^{3.3}*-lactam-trifluoracetat | HPLC (Methode 6): Rₜ = 4.23 min; LC-MS (Methode 19): Rₜ = 2.05 min, MS (ESIpos): *m*/*z* (%) = 1346.9 (60) [M+H]⁺, MS (ESIneg): m/z (%) = 1344.8 (60) [M-H] ; HR-TOF-MS: C₆₃H₁₀₈N₁₅O₁₇ ber. 1346.8043, gef. 1346.8088 [M+H]⁺. |
| | Ausbeute: 54 mg (50% d. Th.) aus Beispielverbindung 419A (90 mg, 74 µmol) und Beispielverbindung 8A nach Arbeitsvorschrift 13. | |
| 447A | 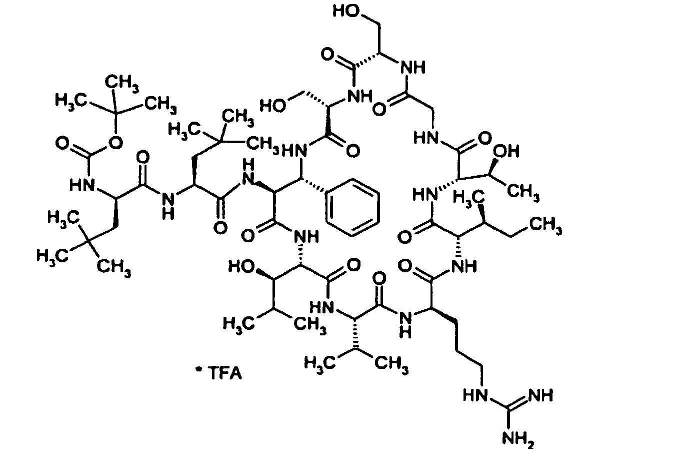 | |
| | [*N²*-(*tert*.-Butoxycarbonyl)-3-tert-butyl-D-alanyl]-[3-*tert*-butyl-L-alanyl]-[(3*R*)-3-amino-L-phenylalanyl]-[(*3R*)-3-hydroxy-L-leucyl]-L-valyl-D-arginyl-L-isoleucyl-L-allothreonyl-glycyl-L-seryl-L-serin-*C^{1.11}*-*N^{3.3}*-lactam-trifluoracetat | HPLC (Methode 6): Rₜ = 4.28 min; LC-MS (Methode 51): Rₜ = 3.32 min, MS (ESIpos): *m*/*z* (%) = 1347.8 (5) [M+H]⁺, MS (ESIneg): *m*/*z* (%) = 1344.7 (1) [M-H] ; HR-TOF-MS: C₆₃H₁₀₈N₁₅O₁₇ ber. 1346.8043, gef. 1346.8046 [M+H]⁺. |
| | Ausbeute: 54 mg (65% d. Th.) aus Beispielverbindung 420A (65 mg, 53 µmol) und Beispielverbindung 8A nach Arbeitsvorschrift 13. | |
| 448A | 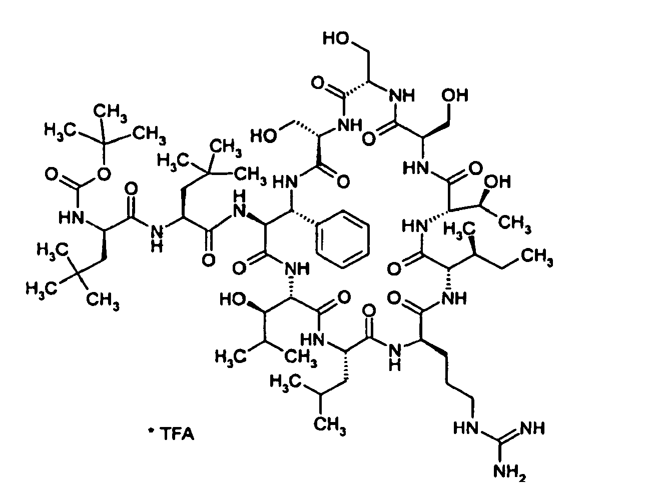 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-D-seryl-L-seryl-L-serin-*C^{1.11}*-*N^{3.3}*-lactam-trifluoracetat | HPLC (Methode 6): Rₜ = 4.23 min; LC-MS (Methode 19): Rₜ = 2.08 min, MS (ESIpos): *m*/*z* (%) = 1390.8 (60) [M+H]⁺, MS (ESIneg): m/z (%) = 1388.8 (60) [M-H] ; HR-TOF-MS: C₆₅H₁₁₂N₁₅O₁₈ ber. 1390.8305, gef. 1390.8306 [M+H]⁺. |
| | Ausbeute: 64 mg (83% d. Th.) aus Beispielverbindung 421A (65 mg, 51 µmol) und Beispielverbindung 8A nach Arbeitsvorschrift 13. | |
| 449A | 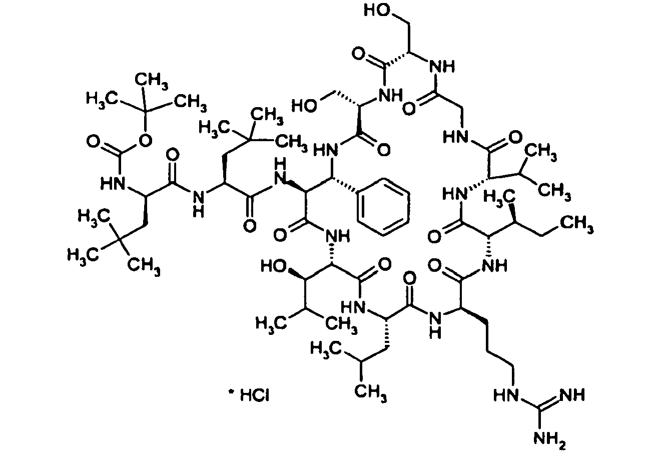 | |
| | [*N*²-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-valyl-glycyl-L-seryl-L,-serin-*C^{1.11}*-*N^{3.3}*-lactam-hydrochlorid | HPLC (Methode 6): Rₜ = 4.29 min; LC-MS (Methode 19): Rₜ = 2.10 min, MS (ESIpos): *m*/*z* (%) = 1358.8 (30) [M+H]⁺, MS (ESIneg): *m*/*z* (%) = 1356.0 (60) [M-H] ⁺[M+H]⁺; HR-TOF- |
| | Ausbeute: 33 mg (75% rein; 89% d. Th.) aus Beispielverbindung 422A (22 mg, 20 µmol) und Beispielverbindung 8A nach Arbeitsvorschrift 13. | MS: C₆₅H₁₁₂N₁₅O₁₆ ber. 1358.8406, gef. 1358.8383 [M+H]⁺. |
| 450A | 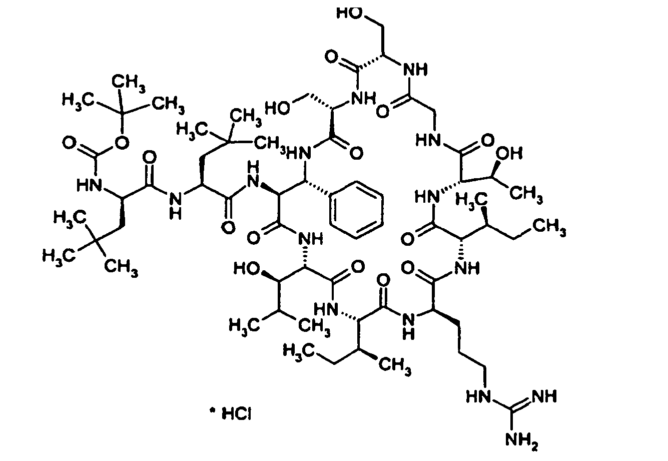 | |
| | [*N*²-(*tert*.-Butoxycarbonyl)-3-tert-butyl-D-alanyl]-[3-*tert*-butyl-L-alanyl]-[(3*R*)-3-amino-L. phenylalanyl]-[(3*R*)-3-hydroxy-L-leucyl]-L-isoleucyl-D-arginyl-L-isoleucyl-L-allothreonyl-glycyl-seryl-L-serin-*C^{1.11}-N^{3.3}*-lactam-hydrochlorid | HPLC (Methode 6): Rₜ = 4.30 min; LC-MS (Methode 19): Rₜ = 2.14 min, MS (ESIpos): *m*/*z* (%) = 1360.8 (50), MS (ESIneg): *m*/*z* (%) = 1358.8 (100) [M- |
| | Ausbeute: 15 mg (78% rein, 50% d. Th.) aus Beispielverbindung 423A (19 mg, 16 µmol) und Beispielverbindung 8A nach Arbeitsvorschrift 13. | H] ⁻; HR-TOF-MS: C₆₄H₁₁₀N₁₅O₁₇ ber. 1360.8199, gef. 1360.8223 [M+H]⁺. |
| 451A | 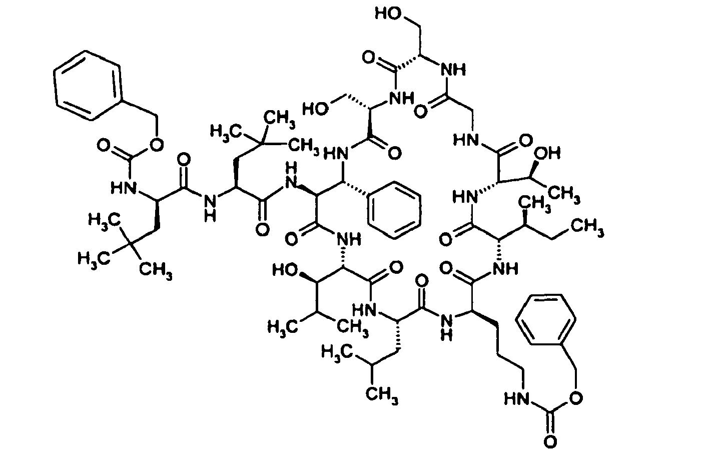 | |
| | [*N*²-(Benzylxycarbonyl)-3-*tert*-butyl-D-alanyl]. [3-*tert*-butyl-L-alanyl]-[(3*R*)-3-amino-L-phenylalanyl]-[(*3R*)-3-hydroxy-L-leucyl]-L-leucyl-(*N⁵*-benzyloxycarbonyl-D-ornithyl)-L-isoleucyl-L-allothreonyl-glycyl-L-seryl-L-serin-*C^{1.11}-N^{3.3}*-lactam | HPLC (Methode 6): Rₜ = 4.97 min; LC-MS (Methode 19): Rₜ = 3.04 min, MS (ESIpos): *m*/*z* (%) = 744.2 (100) [M+2H]²⁺, 1487.8 (90) [M+H]⁺, MS *m*/*z* (%) 1485.9 (100) [M- |
| | Ausbeute: 53 mg (74% rein, 59% d. Th.) aus Beispielverbindung 424A (49 mg, 45 µmol) und Beispielverbindung 10A nach Arbeitsvorschrift 13. | (ESIneg): = H] ; HR-TOF-MS: C₇₄H₁₁₂N₁₃O₁₉ ber. 1486.8192, gef. 1486.8167 [M+H]⁺. |
| | | |
| 452A | 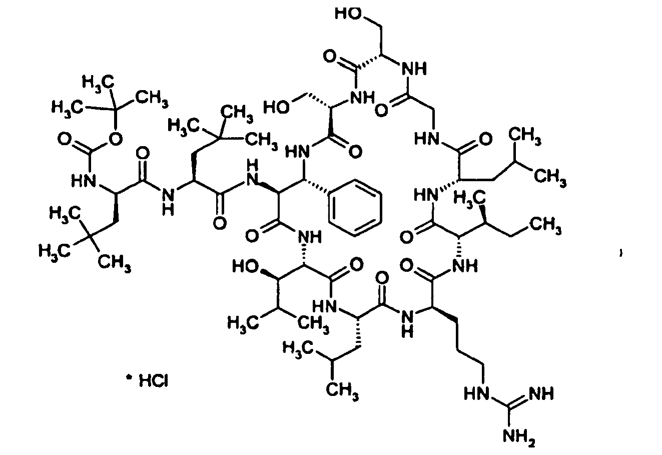 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-leucyl-glycyl-L-seryl-L-serin-*C^{1.11}*-*N^{3.3}*-lactam-hydrochlorid | HPLC (Methode 6): Rₜ = 4.31 min; LC-MS (Methode 19): Rₜ = 2.16 min, MS (ESIpos): *m*/*z* (%) = 1372.9 (50) [M+H]⁺, MS (ESIneg): *m*/*z* (%) = |
| | Ausbeute: 42 mg (75% rein, 80% d. Th.) aus Beispielverbindung 425A (35 mg, 27 µmol) und Beispielverbindung 8A nach Arbeitsvorschrift 13. | 1370.8 (100) [M-H]⁻; HR-TOF-MS: C₆₆H₁₁₄N₁₅O₁₆ ber. 1372.8563, gef. 1372.8544 [M+H]⁺. |
| 453A | 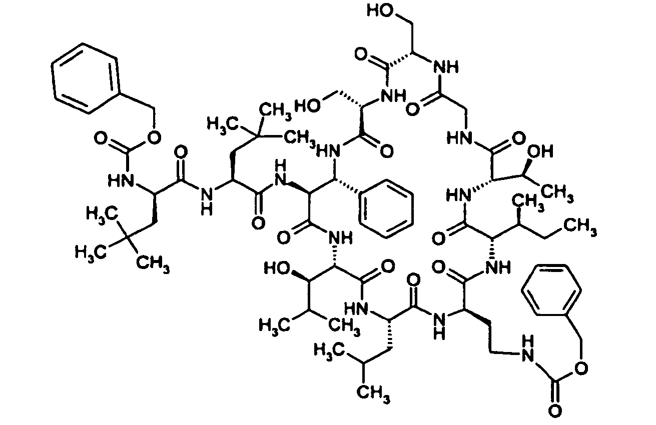 | |
| | [*N*²-(Benzyloxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L. leucyl-[(2*R*)-2-amino-4-benzyloxycarbonylamino-butyryl]-L-isoleucyl-L-allothreonyl-glycyl-L-seryl-L-serin-*C^{1.11}*-*N^{3.3}*-lactam | HPLC (Methode 6): Rₜ = 5.11 min; LC-MS (Methode 19): Rₜ = 3.06 min, MS (ESIpos): *m*/*z* (%) = 737.1 (100) [M+2H]²⁺, 1472.8 (20) [M+H]⁺, MS (ESIneg): *m*/*z* (%) = 1471.8 (100) [M-H]⁻. |
| | Ausbeute: 160 mg (Rohprodukt) aus Beispielverbindung 426A (120 mg Rohprodukt) und Beispielverbindung 10A nach Arbeitsvorschrift 13. | |
| 454A | 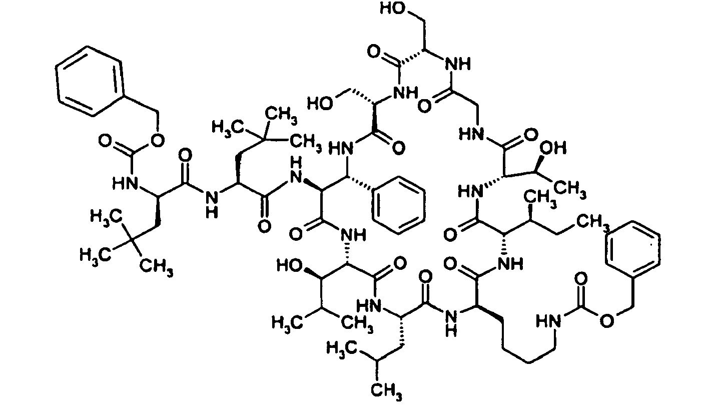 | |
| | [*N*²-(Benzyloxycarbonyl)-3-*tert*-butyl-D-alanyl]-[3-*tert*-butyl-L-alanyl]-[(3*R*)-3-amino-L-phenylalanyl]-[(3*R*)-3-hydroxy-L-leucyl]-L-leucyl-(*N⁶*-benzyloxycarbonyl-D-lysyl)-L-isoleucyl-L-allothreonyl-glycyl-L-seryl-L-serin-*C^{1.11}*-*N^{3.3}*-lactam | HPLC (Methode 6): Rₜ = 5.01 min; LC-MS (Methode 52): Rₜ = 2.48 min, MS (ESIpos): *m*/*z* (%) = 751.3 (100) [M+2H]²⁺, 1502.0 (80) [M+H]⁺, MS (ESIneg): *m*/*z* (%) = 1545.1 (90) [M+HCOO⁻]⁻; HR-TOF-MS: |
| | Ausbeute: 29 mg (80% rein, 82% d. Th.) aus Beispielverbindung 427A (21 mg, 19 µmol) und Beispielverbindung 10A nach Arbeitsvorschrift 13. | C₇₅H₁₁₄N₁₃O₁₉ ber. 1500.8349, gef. 1500.8326 [M+H]⁺. |
| 455A | 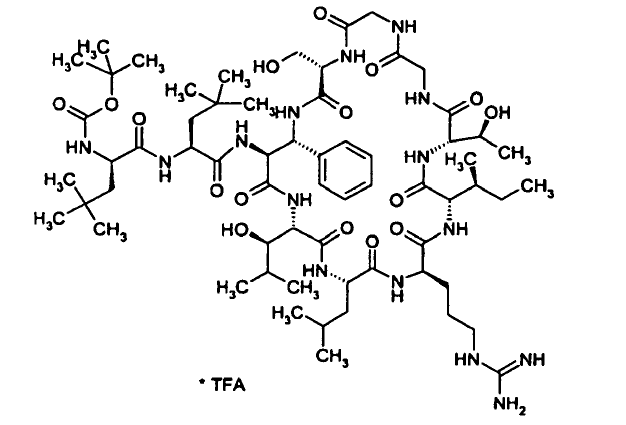 | |
| | [*N²*-(*tert*.-Butoxycarbonyl)-3-*tert*-butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-allothreonyl-glycyl-glycyl-L-serin-*C^{1.11}-N^{3.3}*-lactam-trifluoracetat | HPLC (Methode 6): Rₜ = 4.43 min, LC-MS (Methode 19): Rₜ = 2.14 min, MS (ESIpos): *m*/*z* (%) = 1330.9 (10) [M+H]⁺; HR-TOF-MS: C₆₃H₁₀₈N₁₅O₁₆ ber. 1330.8093, gef. 1330.8109 [M+H]⁺. |
| | Ausbeute: 177 mg (92% d. Th.) aus Beispielverbindung 428A (160 mg, 133 µmol) und Beispielverbindung 8A nach Arbeitsvorschrift 13. | |
| 456A | 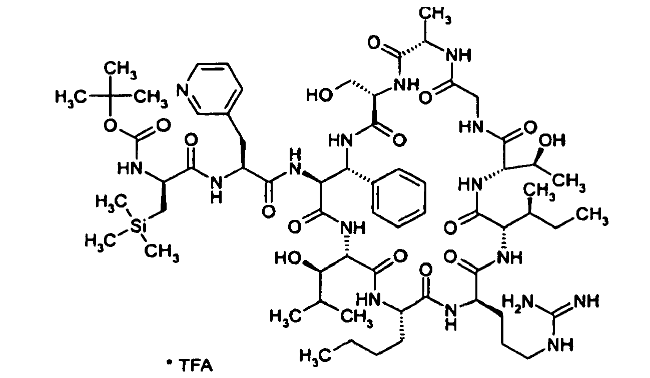 | |
| | [*N*²-(*tert*.-Butoxycarbonyl)-3-trimethylsilyl-D-alanyl]-[3-(3-pyridyl)-L-alanyl]-[(3*R*)-3-amino-L-phenylalanyl]-[(*3R*)-3-hydroxy-L-leucyl]-L-norleucyl-D-arginyl-L-isoleucil-L-allothreonyl-glycyl-L-alanyl-L-serin-*C^{1.11}-N^{3.3}*-lactam-tri-fluoracetat | HPLC (Methode 6): Rₜ = 3.83 min; LC-MS (Methode 19): Rₜ = 1.96 min, MS (ESIpos): *m*/*z* (%) = 691.6 (100) [M+2H]²⁺, 1381.7 (5) [M+H]⁺, MS (E-SIneg): *m*/*z* (%) = 1380.7 (100) [M-H]⁻; HR-TOF-MS: C₆₄H₁₀₅N₁₆O₁₆Si ber. |
| | Ausbeute: 45 mg (60% rein, 52% d. Th.) aus Beispielverbindung 429A (76 mg, 52% rein, 32 µmol) und Beispielverbindung 274A nach Arbeitsvorschrift 13, Reinigung nach Methode 45. | 1381.7659, gef. 1381.7666 [M+H]⁺. |
| 457A | 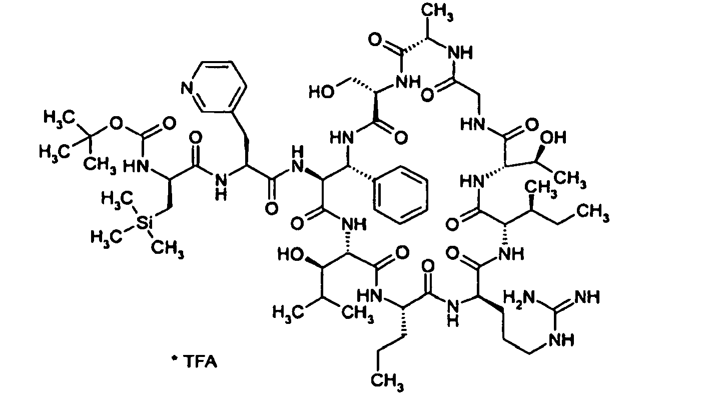 | |
| | [*N*²-(*tert*.-Butoxycarbonyl)-3-trimethylsilyl-D-alanyl]-[3-(3-pyridyl)-L-alanyl]-[(3*R*)-3-amino-L-phenylalanyl]-[(3*R*)-3-hydroxy-L-leucyl]-L-norvalyl-D-arginyl-L-isoleucyl-L-allothreonyl-glycyl-L-alanyl-L-serin-*C^{1.11}-N^{3.3}*-lactam-trifluoracetat | HPLC (Methode 6): Rₜ = 3.73 min; LC-MS (Methode 19): Rₜ = 1.91 min, MS (ESIpos): *m*/*z* (%) = 685.4 (100) [M+2H]²⁺, 1368.8 (30) [M+H]⁺, MS (ESIneg): *m*/*z* (%) = 1367.7 (30) [M-H]⁻; HR-TOF-MS: C₆₃H₁₀₃N₁₆O₁₆Si ber. 1367.7502, gef. 1367.7495 [M+H]⁺. |
| | Ausbeute: 41 mg (52% rein, 32% d. Th.) aus Beispielverbindung 430A (50 mg, 42 µmol) und Beispielverbindung 274A nach Arbeitsvorschrift 13, Reinigung nach Methode 45. | |
| 458A | 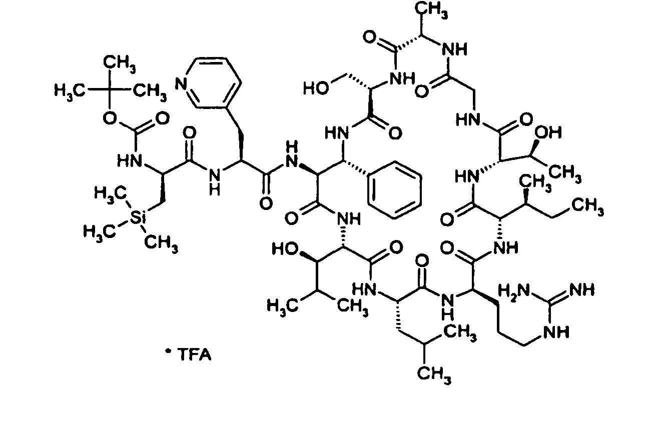 | |
| | [*N²*-(*tert*.-Butoxycarbonyl)-3-trimethylsilyl-D-alanyl]-[3-(3-pyridyl)-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-L-alanyl-L-serin-*C^{1.11}*-*N^{3.3}*-lactam-trifluoracetat | HPLC (Methode 6): Rₜ = 3.83 min; LC-MS (Methode 19): Rₜ = 1.97 min, MS (ESIpos): *m*/*z* (%) = 691.9 (100) [M+2H]²⁺, 1381.7 (20) [M+H]⁺, MS (ESIneg): *m*/*z* (%) = 1379.7 (100) [M-H]⁻; HR-TOF-MS: C₆₄H₁₀₅N₁₆O₁₆Si ber. 1381.7659, gef. 1381.7683 [M+H]⁺. |
| | Ausbeute: 45 mg (60% rein, 36% d. Th.) aus Beispielverbindung 230A (49 mg, 46 µmol) und Beispielverbindung 274A nach Arbeitsvorschrift 13, Reinigung nach Methode 45. | |

### Beispiel 459A:

### tert-Butyl [(phenylsulfonyl)(pyridin-3-yl)methyl]carbamat

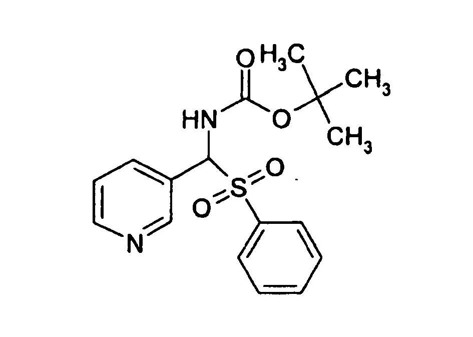

Pyridin-3-carbaldehyd (5.36 g, 50 mmol), *tert*-Butylcarbamat (11.7 g, 100 mmol, 2 Äquivalente), Natriumphenylsulfinat (20.36 g, 124 mmol, 2.48 Äquivalente), Ameisensäure (1.89 mL, 2.30 g, 1 Äquivalent), Methanol (55 mL) und Wasser (111 mL) werden gemischt und bei Raumtemperatur 3 d kräftig gerührt. Das Produkt fällt in Form farbloser Kristalle aus, es wird abfiltriert, mit Wasser und etwas MTBE gewaschen und im Vakuum getrocknet. Ausbeute: 4.10 g (24% d. Th.).
LC-MS (Methode 51): Rₜ = 2.51 min, MS (ESIpos): *m*/*z* (%) = 349.3 (100) [M+H]⁺.

### Beispiel 460A:

### tert-Butyl [(pyridin-3-yl)methylen]carbamat

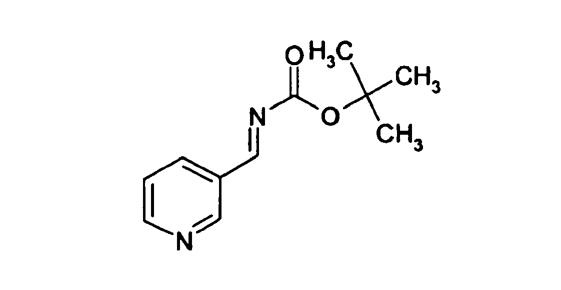

Kaliumcarbonat (4.78 g, 34 mmol, 6 Äquivalente) wird unter Vakuum in der Wärme getrocknet und mit THF (70 mL) überschichtet. Der Ansatz wird über 16 h zum Rückfluss erhitzt, dann lässt man ihn erkalten und filtriert über eine Schicht Celite. Das Filtrat wird eingeengt und der ölige Rückstand (1.33 g Rohprodukt, quant.) ohne Aufreinigung weiter umgesetzt.

### Beispiel 461A:

### Ethyl rac-threo-3-[(tert-Butoxycarbonyl)amino]-N²-(diphenylmethylen)-3-pyridin-3-ylalaninat

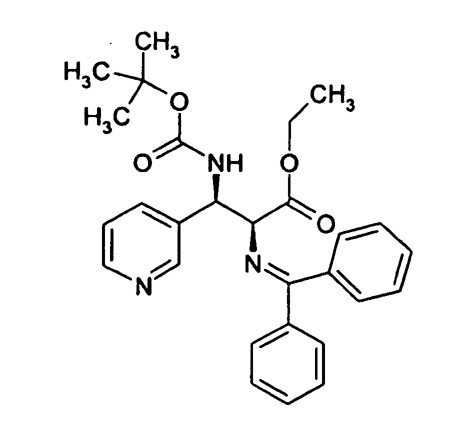

Die Reaktion erfolgt in Anlehnung an eine Methode zur Addition von Kupferenolaten an Sulfonylimine: L. Bernardi, A. Gothelf, R. G. Hazell, K. A. Jørgensen, J. Org. Chem. 2003, 68, 2583-2591. *tert*-Butyl [(1E)-phenylmethylen]carbamat kann nach der Literatur hergestellt werden: A. Klepacz, A. Zwierzak, Tetrahedron Lett. 2002 43; 1079-1080.

Frisch aktiviertes Molekularsieb 3 Å, Tetrakis(acetonitril)kupfer(I) hexafluorphosphat (179 mg, 0.48 mmol, 0,1 Äquivalente) und (*R*)-(+)-2-(2-(Diphenylphosphino)phenyl)-4-phenyl-2-oxazolin (329 mg, 0.53 mmol, 0.11 Äquivalente) werden unter Argon vorgelegt und mit abs. THF (41 mL) versetzt. Dann wird Triethylamin (67 µL, 49 mg, 0.48 mmol, 0.1 Äquivalente) dazupipettiert Die Mischung wird auf -20 °C gekühlt, und, sobald diese Temperatur erreicht ist, werden Ethyl N-(diphenylmethylen)glycinat (1.29 g, 4.81 mmol) und *Beispielverbindung* 459A (1.19 g, 5.77 mmol, 1.2 Äquivalente) dazugegeben. Der Ansatz wird über 16 h gerührt, wobei man ihn langsam auf Raumtemperatur kommen lässt. Danach wird Kieselgel (ca 15 g) dazugegeben und das Lösemittel im Vakuum abdestilliert. Der Rückstand wird mit Cyclohexan-Ethylacetat 9+1 chromatographiert (Biotage 40M mit ZIF-SIM 35). Die Titelverbindung wird in einer Ausbeute von 1.85 g (95% rein, 77% d. Th.) erhalten.
LC-MS (Methode 51): Rₜ = 3.55 min MS (ESIpos): *m*/*z* (%) = 474.3 (100) [M+H]⁺.

### Beispiel 462A:

### Ethyl rac-threo-3-[(tert-Butoxycarbonyl)amino]-3-pyridin-3-yl-alaninat-trifluoracetat

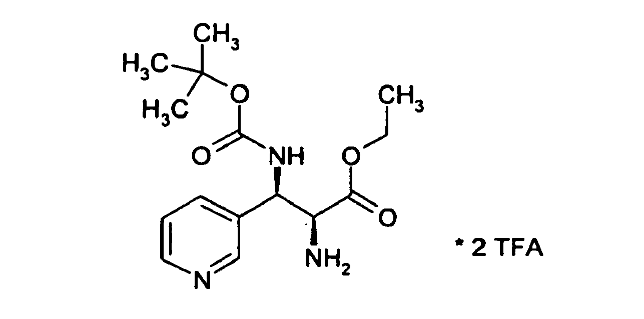

Die Titelverbindung aus Beispiel 461A (350 mg, 0.74 mmol) wird in Acetonitril (9.33 mL) gelöst, 187 µL TFA (2.42 mmol, 3.3 Äquivalente) und Wasser (187 µL, 10.4 µmol, 14 Äquivalente) werden zugegeben und der Ansatz über 16 h bei 4°C stehen gelassen. Alle flüchtigen Bestandteile des Reaktionsgemischs werden im Vakuum abdestilliert und der Rückstand wird chromatographisch gereinigt (Methode 34). Ausbeute: 366 mg (92% d. Th.).
HPLC (Methode 6): Rₜ = 3.03 min.
LC-MS (Methode 22): Rₜ = 2.32 min, MS (ESIpos): *m*/*z* (%) = 310.0 (80) [M+H]⁺.
HR-TOF-MS: C₁₅H₂₄N₃O₄ ber. 310.1762, gef. 310.1767 [M+H]⁺.

### Beispiel 463A:

### Ethyl rac-threo-3-[(tert-Butoxycarbonyl)amino]-N²-(Benzyloxy)carbonylamino-3-pyridin-3-yl-alaninat

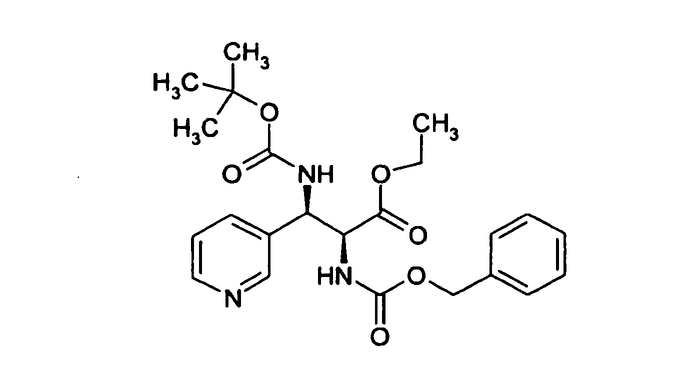

Beispielverbindung 462A (366 mg, 0.68 mmol) und Benzyloxycarbonyl-succinimid (209 mg, 0.82 mmol, 1.2 Äquivalente) werden in 5 mL Dichlormethan-Wasser (4+1) gelöst, der Ansatz wird auf 0 °C gekühlt. Unter kräftigem Rühren werden Natriumhydrogencarbonat (86 mg, 1.03 mmol, 1.5 Äquivalente) und Tetrabutylammoniumbromid (11 mg, 34 µmol, 0.05 Äquivalent) dazugegeben und der Ansatz über 16 h bei Raumtemperatur kräftig gerührt. Die organische Phase wird abgetrennt, mit conc. NaCl gewaschen, über Natriumsulfat getrocknet und eingeengt. Das Rohprodukt wird chromatographiert (Methode 34). Ausbeute: 314 mg (84% rein, 0.60 mmol, 88% d. Th.) der Titelverbindung.
R_{f} = 0.076 (CyHex-EtOAc 9+1).
HPLC (Methode 54): Rₜ = 4.33 min (erythro-Diastereomer) und 4.52 min (threo-Diastereomer).
LC-MS (Methode 19): Rₜ = 2.71 min, MS (ESIpos): *m*/*z* (%) = 409.3 (30) [M+H]⁺, erythro-Isomer und Rₜ = 2.75 min, MS (ESIpos): *m*/*z* (%) = 409.0 (70) [M+H]⁺ threo-Isomer).
¹H NMR (400 MHz, *d₆*-DMSO) δ (ppm) = 0.71-0.86 (m, 6H), 1.16 (t, *J* = 7.1 Hz, 3H), 1.34 (s, 9H), 1.59 (m, 1H), 3.70 (ddd, *J* = 3.4, *J¹ = J²* = 10.3 Hz, 1H), 3.99-4.08 (m, 2H), 4.32 (dd, *J* = 3.4, *J* = 9.5 Hz, 1H), 5.03 (d, *J* = 12.4 Hz, 1H), 5.10 (d, *J* = 12.4 Hz, 1H), 6.62 (d, *J* = 10.5 Hz, 1H), 7.31-7.40 (m, 5H), 7.44 (d, *J* = 9.3 Hz, 1H).
HR-TOF-MS: C₂₁H₃₃N₂O₆ ber. 409.2334, gef. 409.2329 [M+H]⁺.

### Beispiel 464A:

### (3R)-3-[(tert-Butoxycarbonyl)amino]-N²-(Benzyloxy)carbonylamino-3-pyridin-3-yl-L-alanin

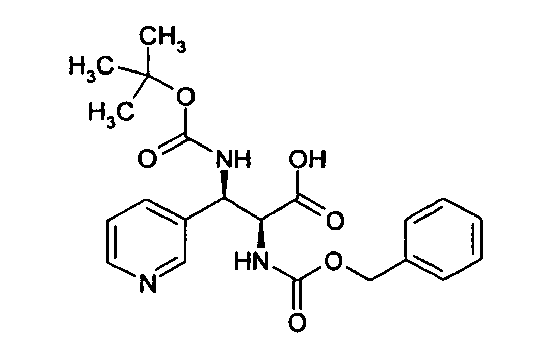

Die Titelverbindung aus Beispiel 463A (314 mg, 0.56 mmol) wird in einem Gemisch aus THF-Wasser (2+1, 17 mL) bei 0 °C gelöst, Lithiumhydroxid-Monohydrat (25 mg, 0.59 mmol, 1.1 Äquivalente) wird dazugegeben und 3 h bei 0 °C gerührt. Dann wird das THF abdestilliert, der wässrige Rückstand wird lyophilisiert und dann chromatographisch gereinigt (Methode 44). Das Produkt wird durch Chromatographie an einer chiralen Phase nach Methode 58 getrennt. Dabei werden die Enantiomeren des threo-Diastereomers und diejenigen des erythro-Minderdiastereomers jeweils einzeln erhalten. Der e.e. des Hauptisomers (Titelverbindung) nach der chiralen Chromatographie wird nach Methode 59 bestimmt, er beträgt 100%. Die Ausbeute der Titelverbindung beträgt 113 mg (38% d. Th. Bezogen auf die Ausgangsverbindung 463A.
HPLC (Methode 6): Rₜ = 3.53 min.
Chirale HPLC (Methode 59): Rₜ = 4.71 min.
LC-MS (Methode 19): Rₜ = 1.85 min, MS (ESIpos): *m*/*z* (%) = 416.2 (100) [M+H]⁺, MS (ESIneg): *m*/*z* (%) = 414.1.2 (100) [M-H]⁻.
HR-TOF-MS: C₂₁H₂₆N₃O₆ ber. 416.1817, gef. 416.1808 [M+H]⁺.

### Beispiel 465A:

### Pentafluorphenyl (3R)-3-[(tert-Butoxycarbonyl)amino]-N²-(Benzyloxy)carbonylamino-3-pyridin-3-yl-L-alaninat

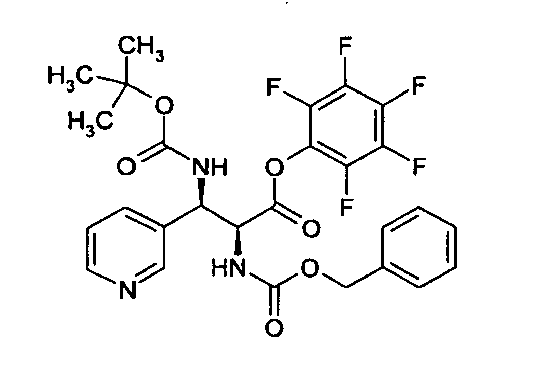

Beispielverbindung 464A (63 mg, 119 µmol) und Pentafluorphenol (24 mg, 131 µmol, 1.1 Äquivalente) werden in Dichlormethan (0.8 mL) bei Raumtemperatur gelöst, dann wird EDCI (25 mg, 131 µmol, 1.1 Äquivalente) hinzugefügt und der Ansatz ca 12 h im Kühlschrank stehen gelassen. Anschließend wird das Lösungsmittel im Vakuum abdestilliert und der Rückstand chromatographisch gereinigt (Methode 44). Die Titelverbindung wird in einer Ausbeute von 47 mg (51% d. Th.) als farbloser Feststoff erhalten.
HPLC (Methode 54): Rₜ = 4.49 min.
LC-MS (Methode 19): Rₜ = 2.74 min, MS (ESIpos): *m*/*z* (%) = 582.2 (40) [M+H]⁺
HR-TOF-MS: C₂₇H₂₅N₃O₆F₅ ber. 582.1659, gef. 582.1647 [M+H]⁺.

Die in der nachfolgenden Tabelle aufgeführten Verbindungen 466A-470A wurden nach den angegebenen Arbeitsvorschriften aus den angegebenen Edukten hergestellt.

| | | |
|---|---|---|
| 466A | 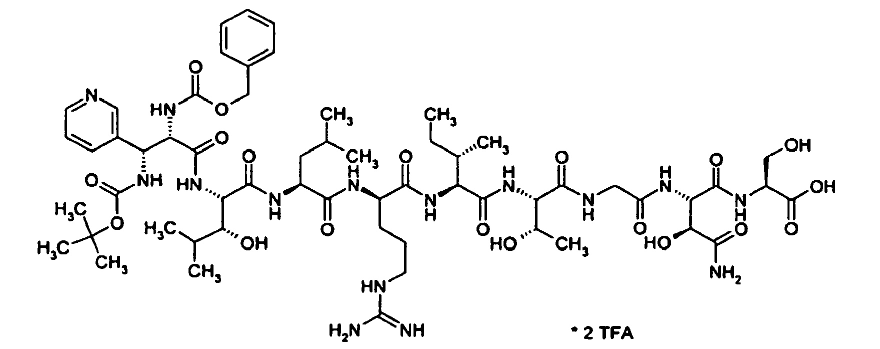 | |
| | [(3*R*)-*N*²-(Benzyloxycarbonyl)-3-{(*tert*-butoxycarbonyl)amino)-3-(pyridin-3-yl)-L-alanyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-bis-trifluoracetat | HPLC (Methode 6): Rₜ = 3.42 min; LC-MS (Methode 19): Rₜ = 1.75 min, MS (ESIpos): *m*/*z* (%) = 652.1 (100) [M+2H]²⁺, 1302.7 (5) [M+H]⁺; MS (ESI-neg) *m*/*z* (%) = 1300.7 (100) [M-H]⁻ ; HR-TOF-MS: C₅₈H₉₂N₁₅O₁₉ ber. 1302.6689, gef. 1302.6661 [N4+H]⁺. |
| | Ausbeute: 51 mg (49% d. Th.) aus Beispielverbindung 3A (76 mg, 68 µmol) und 465A nach Arbeitsvorschrift 10, Reinigung nach Methode 45 | |
| 467A | 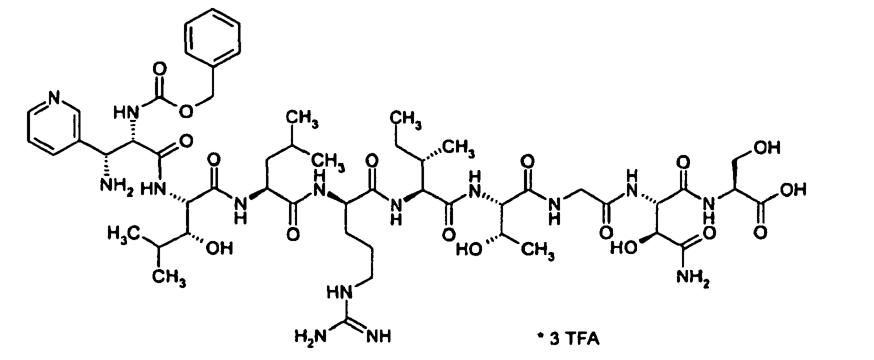 | |
| | [(3*R*)-*N*²-(Benzyloxycarbonyl)-3-amino-3-(pyridin-3-yl)-L-alanyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-bis-trifluoracetat | HPLC (Methode 6): Rₜ = 3.12 min; LC-MS (Methode 52): Rₜ = 0.91 min, MS (ESIpos): *m*/*z* (%) = 602.0 (100) [M+2H]²⁺, 1202.7 (5) [M+H]⁺, MS (ESI-neg): *m*/*z* (%) = 1200.8 (100) [M-H] ; HR-TOF-MS: C₅₃H₈₄N₁₅O₁₇ ber. 1202.6165, gef. 1202.6160 [M+H]⁺. |
| | Ausbeute: 52 mg (quant.) als farbloser Feststoff aus 51 mg der Beispielverbindung 466A nach Arbeitsvorschrift 2, Rohprodukt ohne Aufreinigung weiter umgesetzt. | |
| 468A | 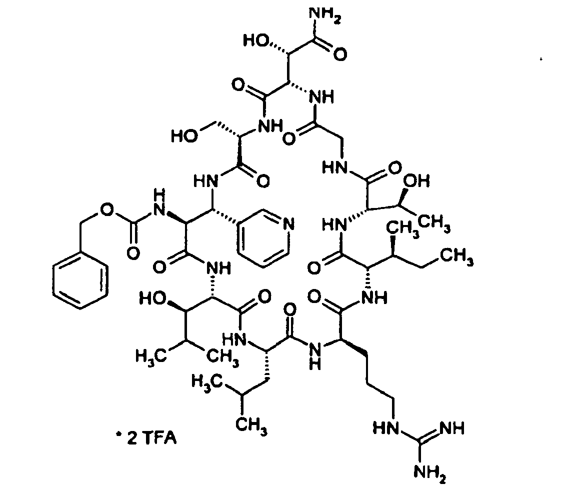 | |
| | [(3*R*)-*N*²-(Benzyloxycarbonyl)-3-amino-3-(pyridin-3-yl)-L-alanyl]-[(3*R*)-3-hydroxy-L-leucyl]-L-leucyl-D-argin yl-L-isoleucyl-L-allothreonyl-glycyl-[(3*R*)-3-hydroxy-L-asparaginyl]-L-serin. *C^{1.9}*-*N^{3.1}*-lactam-bis-trifluoracetat | HPLC (Methode 6): Rₜ = 3.38 min; LC-MS (Methode 19): Rₜ = 1.58 min, MS (ESIpos): *m*/*z* (%) = 592.9 (100) [M+2H]²⁺, 1184.7 (5) [M+H]⁺, MS (ESI-neg): *m*/*z* (%) = 1182.6 (100) [M-H] ⁻; HR-TOF-MS: C₅₃H₈₂N₁₅O₁₆ ber. 1184.6059, gef. 1184.6088 [M+H]⁺. |
| | Ausbeute: 29 mg (61% d. Th.) aus Beispielverbindung 467A (52 mg, 34 µmol) nach Arbeitsvorschrift 11, Reinigung nach Methode 45. | |
| 469A | 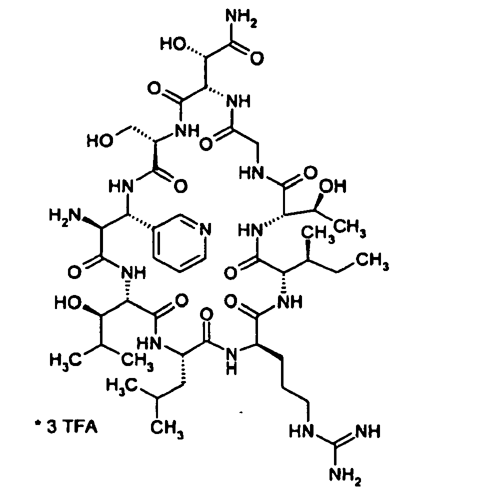 | |
| | [(3*R*)-3-amino-3-(pyridin-3-yl)-L-alanyl]-[(3*R*)-3-hydroxy-L-leucyl]-L-leucyl-D-arginyl-L-iso-leucyl-L-allothreonyl-glycyl-[(3*R*)-3-hydroxy-L-asparaginyl]-L-serin-*C^{1.9}*-*N^{3.1}*-lactam-tris-trifluoracetat | HPLC (Methode 6): Rₜ = 2.84 min; LC-MS (Methode 22): Rₜ = 2.13 min, MS (ESIpos): *m*/*z* (%) = 526.2 (100) [M+2H]²⁺, 1050.6 (30) [M+H]⁺, MS (E-SIneg): *m*/*z* (%) = 1048.6 (100) [M-H]⁻ ; HR-TOF-MS: C₄₅H₇₆N₁₅O₁₄ ber. 1050.5691, gef. 1050.5670 [M+H]⁺. |
| | Ausbeute: 65 mg (96% d. Th.) aus Beispielverbindung 468A (65 mg, 46 µmol) nach Arbeitsvorschrift 4. | |
| 470A | 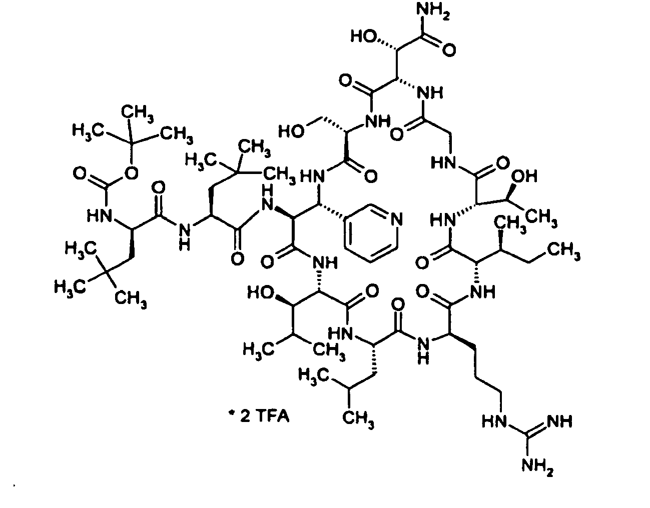 | |
| | [*N²*-(*tert*.-Butoxycarbonyl)-3-*tert*-butyl-D-alanyl]-[3-*tert*-butyl-L-alanyl]-[(3*R*)-3-amino-3. (pyridin-3-yl)-L-alanyl]-[(3*R*)-3-hydroxy-L-leucyl]-L-leucyl-D-arginyl-L-isoleucyl-L-allothreonyl-glycyl-[(3*R*)-3-hydroxy-L-asparaginyl]-L-serin-*C^{1.11}-N^{3.3}*-lactam-bis-trifluoracetat | HPLC (Methode 6): Rₜ = 4.13 min; LC-MS (Methode 19): Rₜ = 2.12 min, MS (ESIpos): *m*/*z* (%) = 703.1 (100) [M+2H]²⁺, 1404.8 (5) [M+H]⁺, MS (ESI-neg): *m*/*z* (%) = 1402.8 (100) [M-H] ; HR-TOF-MS: C₆₄H₁₁₀N₁₇O₁₈ ber. 1404.8210, gef. 1404.821 [M+H]⁺. |
| | Ausbeute: 30 mg (60% d. Th.) aus Beispielverindung 469A (65 mg, 47 µmol) und Beispielverbindung 8A nach Arbeitsvorschrift 13, Chromatographie nach Methode 45. | |

### Ausführungsbeispiele

### Beispiel 1

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-C^{1.11}-N^{3.3}-lactam-bis-trifluoracetat

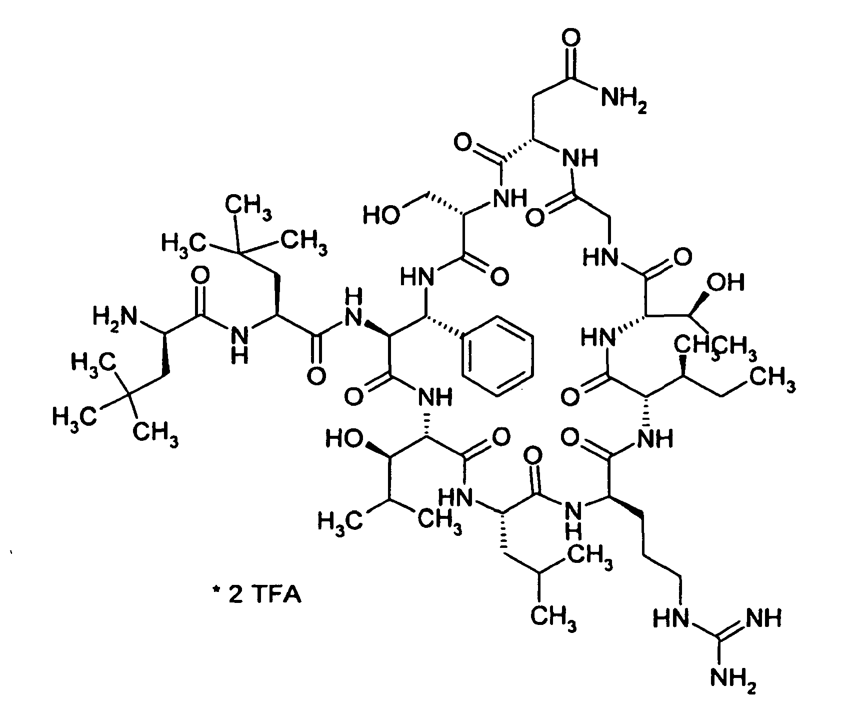

Nach der Arbeitsvorschrift 4 wird von dem Benzyloxycarbonyl-geschützen Peptid (Beispiel 53A, 1.5 mg, 0.89 µmol) in Dioxan und in wässr. 0.1%ige TFA (1 ml, 10 Äqivalente) hydrogenolytisch nach 1 h die Schutzgruppe abgespalten. Das Produkt erhält man nach der Feinreinigung über die präparative HPLC (Methode 26) mit 1.3 mg (96.6% d. Th.).

Alternativ wird aus der Verbindung aus Beispiel 58A (13.0 mg, 8.47 µmol) nach der hydrogenolytischen Esterspaltung (Arbeitsvorschrift 4) in Methanol (5 ml) unter Verwendung von 0.1 N Salzsäure (508 µl, 1.9 mg, 50.79 µmol, 6 Äq.) das Amin (Beispiel 1) freigesetzt. Nach dem Chromatographieren (Methode 26) werden 12.0 mg (93.5% d. Th.) der Titelverbindung isoliert.
HPLC (Methode 9) Rₜ = 14.73 min.
LC-MS (Methode 18): Rₜ = 1.71 min; MS (ESIpos): *m*/*z* (%) = 1288 (3) [M+H]⁺, 644 (100) [M+ 2H]²⁺; MS (ESIneg.): *m*/*z* (%) = 1286 (50) [M - H]⁻, 642 (38) [M - 2H]²⁻, 1332 (100).
HR-TOF-MS (Methode 24): C₆₀H₁₀₃N₁₆O₁₅ [M+H]⁺ gef. 1287.7810, ber. 1287.7784.

### Beispiel 2

### D-Leucyl-L-leucyl-[(3R)-3-amino-L-phenylalanyl]-[(3R)-3-hydroxy-L-leucyl]-L-leucyl. D-arginyl-L-isoleucyl-L-threonyl-glycyl-L-asparaginyl-L-serin-C^{1.11}-N^{3.3}-lactam-bis-trifluoracetat

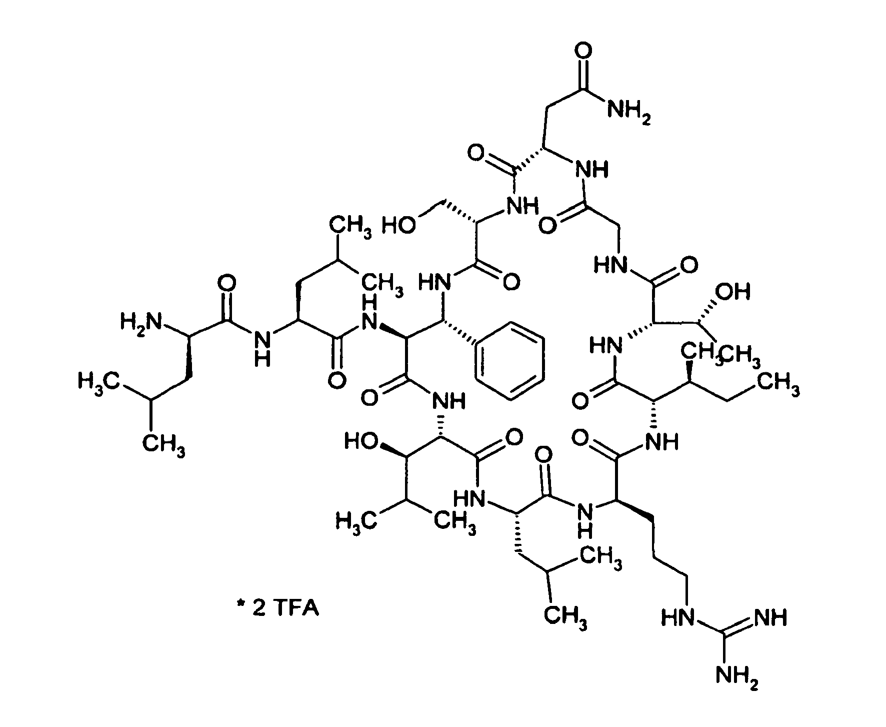

Aus dem Benzyloxycarbonyl-geschützen Peptid (Beispiel 73A, 33.0 mg, 21.89 µmol) wird nach der hydrogenolytischen Esterspaltung (Arbeitsvorschrift 4) in Methanol (10 ml) und 0.1 N wässr. Salzsäure (219 µl, 0.8 mg, 21.89 µmol, 1 Äq.) die Titelverbindung dargestellt. Die Feinreinigung erfolgt über die präparative HPLC (Methode 26) und das Produkt wird mit 18.1 mg (56.1% d. Th.) isoliert.
HPLC (Methode 9) Rₜ = 14.15 min.
LC-MS (Methode 18): Rₜ = 1.52 min; MS (ESIpos): m/z (%) = 1259 (5) [M+H]⁺, 630 (100) [M + 2H]²⁺; MS (ESIneg.): *m*/*z* (%) = 1257 (100) [M - H]⁻, 628 (28) [M - 2H]²⁻.
HR-TOF-MS (Methode 24): C₅₈H₁₀₀N₁₆O₁₅ [M+H]⁺ gef. 1259.7454, ber. 1259.7471.

### Beispiel 3

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-ornityl-L-isoleucyl-L-allothreonyl-glycyl-L-asparaginyl-L-serin-C^{1.11}-N^{3.3}-lactam-bis-trifluoracetat

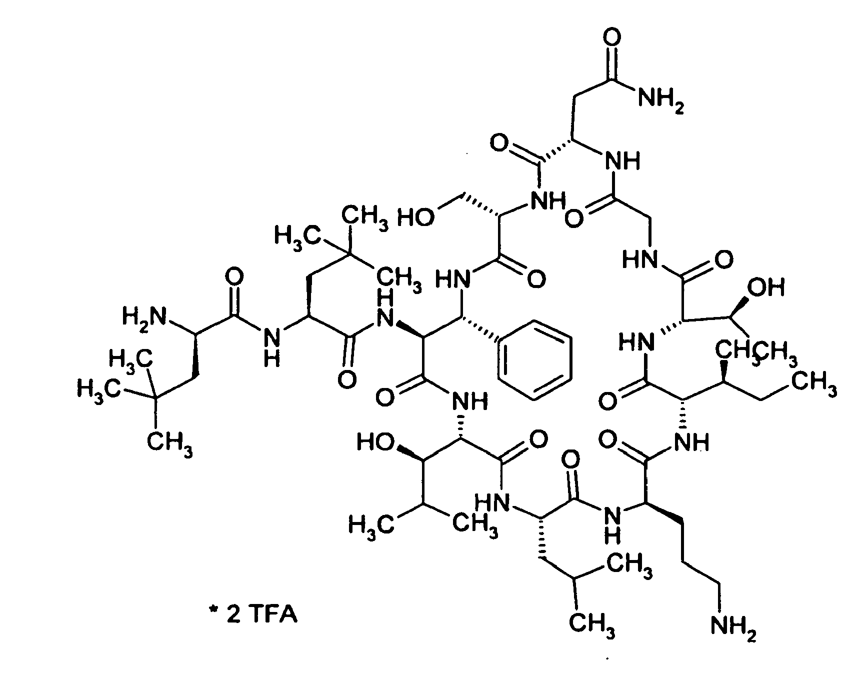

Aus dem Benzyloxycarbonyl-geschützen Peptid (Beispiel 81A, 20.0 mg, 13.21 µmol) wird nach der hydrogenolytischen Esterspaltung (Arbeitsvorschrift 4) in Methanol (10 ml) und 0.1 N wässr. Salzsäure (198 µl, 0.7 mg, 19.8 µmol, 1.5 Äq.) die Titelverbindung dargestellt. Die Feinreinigung erfolgt über die präparative HPLC (Methode 26) und das Produkt wird mit 17.9 mg (91.9% d. Th.) isoliert.
HPLC (Methode 9) Rₜ = 15.51 min.
LC-MS (Methode 18): Rₜ = 1.57 min; MS (ESIpos): *m*/*z* (%) = 1245 (13) [M+H]⁺, 623 (100) [M + 2H]²⁺; MS (ESIneg.): *m*/*z* (%) = 1243 (100) [M - H]⁻.
HR-TOF-MS (Methode 24): C₅₉H₁₀₂N₁₄O₁₅ [M+H]⁺ gef. 1245.7560, ber. 1245.7566.

### Beispiel 4

### [3-tert-Butyl-D-alanyl]-[3-tert-butyl-L-alanyl]-[(3R)-3-amino-O-methyl-L-tyrosyl]-[(3R)-3-hydroxy-L-leucyl]-L-leucyl-D-arginyl-L-isoleucyl-L-allothreonyl-glycyl-[(3S-3-hydroxy-L-asparaginyl]-L-serin-C^{1.11}-N^{3.3}-lactam-bis-trifluoracetat

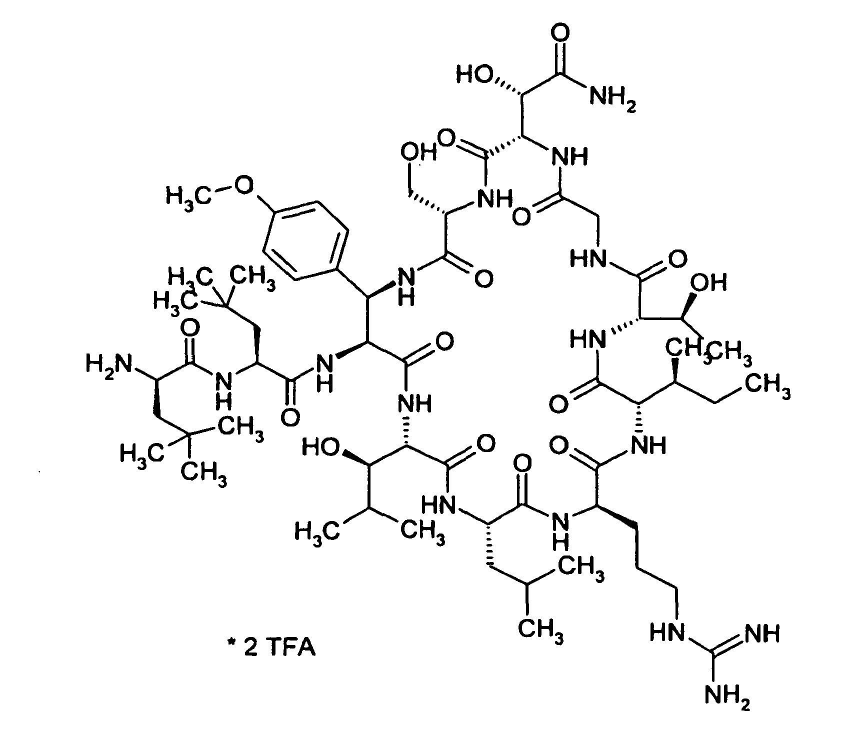

Gemäß der Arbeitsvorschrift 5 wird die Verbindung aus Beispiel 112A (23 mg, 14.5 µmol) umgesetzt. Nach chromatographischer Reinigung mittels präparativer HPLC (Methode 43) erhält man durch Gefriertrocknung 17 mg (75% d. Th.) Produkt.
HPLC/UV-Vis (Methode 5): Rₜ = 3.69 min.
HPLC/UV-Vis (Methode 4): Rₜ = 3.95 min.
LC-MS (Methode 18): Rₜ = 1.71 min; MS (ESIpos.): *m*/*z* (%) = 667 (100) [M + 2H]²⁺, 1333 (2) [M+H]⁺; MS (ESIneg.): *m*/*z* (%) = 1331 (100) [M - H]⁻.

### Beispiel 5

### [3-tert-Butyl-D-alanyl]-[3-tert-butyl-L-alanyl]-[(3R)-3-amino-4-(dimethylaminophenyl)-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{11.1}-N^{3.3}-lactam-tris-trifluoracetat

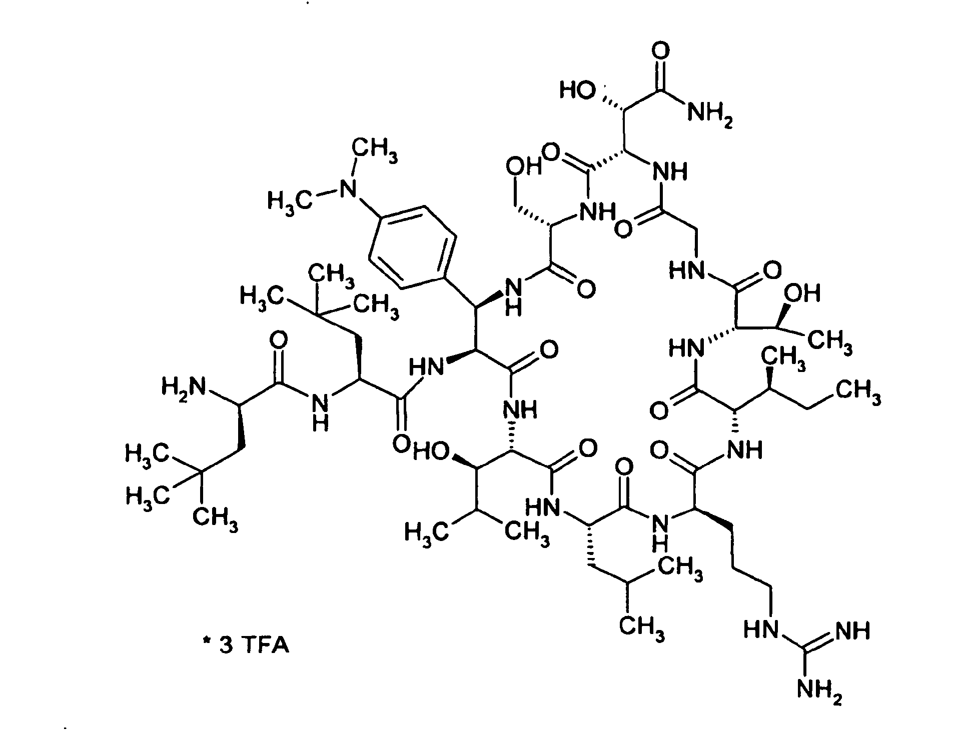

Die Verbindung aus Beispiel 128A (105 mg 66% Reinheit, 41 µmol) wird in 30% TFA in Dichlormethan (4 ml) gelöst und 20 min bei RT gerührt. Dann wird der Ansatz im Vakuum eingeengt und der Rückstand chromatographisch gereinigt (Methode 44 mit modifiziertem Gradienten: 0-2 min 10% B, Rampe, 38 min 60% B). 49 mg (29 µmol, 70% d. Th.) der Titelverbindung werden erhalten.
HPLC (Methode 7): Rₜ = 3.62 min.
LC-MS (Methode 20): Rₜ = 1.72 min, MS (ESIpos.): m/z (%) = 450.0 (100) [M + 3H]³⁺, 674.4 (40) [M + 2H]²⁺.
HR-TOF-MS (Methode 24): C₆₂H₁₀₈N₁₇O₁₆ [M+H]⁺ ber. 1346.8155, gef. 1346.8130.

### Beispiel 6

### [3-Trimethylsilyl-D-alanyl]-[3-tert-butyl-L-alanyl]-[(3R)-3-amino-4-(dimethylaminophenyl)-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam-tris-trifluoracetat

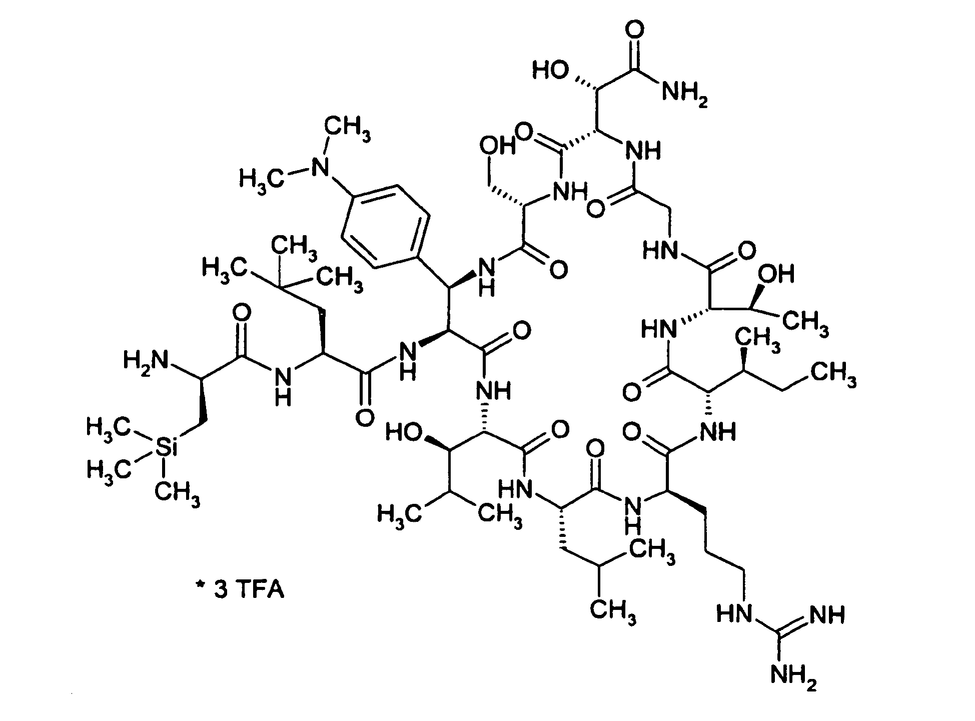

Die Verbindung aus Beispiel 133A (126 mg, 82 µmol) wird in 30% TFA in Dichlormethan (4 ml) gelöst und 20 min bei RT gerührt. Dann wird der Ansatz im Vakuum eingeengt und der Rückstand chromatographisch gereinigt (Methode 44 mit modifiziertem Gradienten: 0-2 min 10% B, Rampe, 38 min 60% B). Man erhält 78 mg (46 µmol, 56% d. Th.) der Titelverbindung.
HPLC (Methode 7): Rₜ = 3.69 min.
LC-MS (Methode 20): Rₜ = 1.66 min, MS (ESIpos.): m/z (%) = 682.3 (100) [M + 2H]²⁺, 1364.1 (2) [M+H]⁺.
HR-TOF-MS (Methode 24): C₆₁H₁₀₈N₁₇O₁₆Si [M+H]⁺ ber. 1362.7924, gef. 1362.7949.

### Beispiel 7

### D-Phenylalanyl-L-phenylalanyl-[(3R)-3-amino-L-alanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-serin-C^{1.11}-N^{3.3}-lactam-bis-trifluoracetat

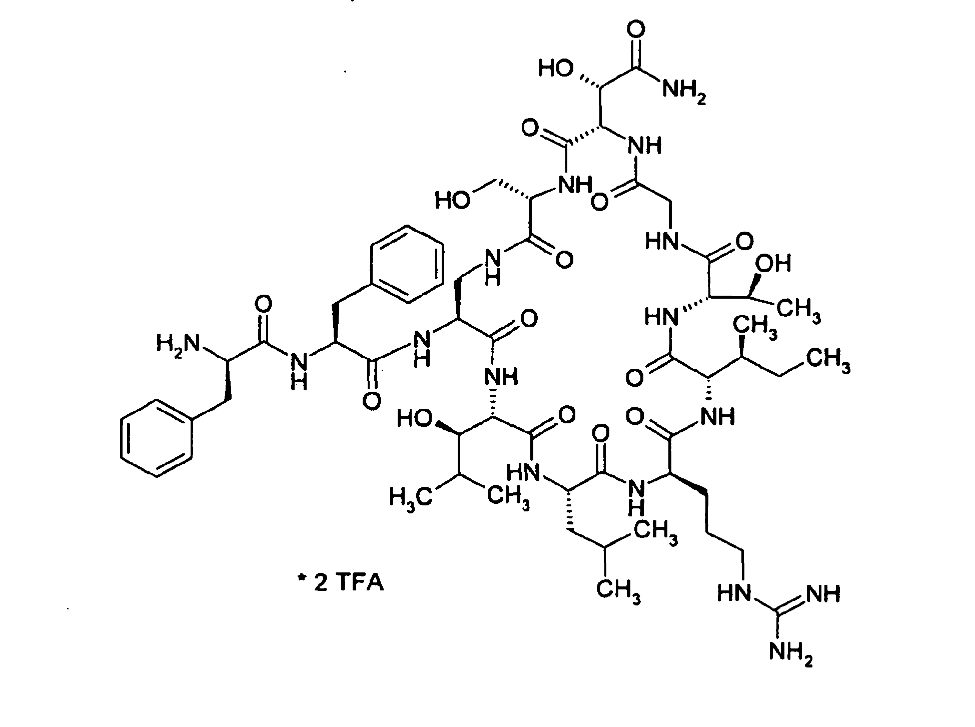

Die Zielverbindung wird, wie in der der Arbeitsvorschrift 1 beschrieben ist, aus der Verbindung aus Beispiel 144A (0.3 mg, 0.20 µmol) dargestellt. Nach der gelchromatographischen Feinreinigung (Methode 45, Eluent: Methanol) wird das Produkt mit 0.1 mg (23.3% d. Th.) isoliert.
HPLC (Methode 12): Rₜ = 5.74 min.
LC-MS (Methode 18): Rₜ = 1.57 min, MS (ESIpos.): m/z (%) = 1268 (2) [M+H]⁺, 634 (100) [M + 2H]²⁺; MS (ESIpos.): m/z (%) = 1266 (50) [M - H]⁻, 632 (22) [M - 2H]²; 1312 (100) [M - H + HCO₂H]⁻.
MALDI-MS (Methode 25): C₅₈H₉₂N₁₆O₁₆ [M+H]⁺ ber. 1267.68, gef. 1267.65.

### Beispiel 8

### [3-tert-Butyl-D-alanyl]-[3-tert-Butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-seryl-L-serin-C^{1.11}-N^{3.3}-lactam bis-trifluoracetat

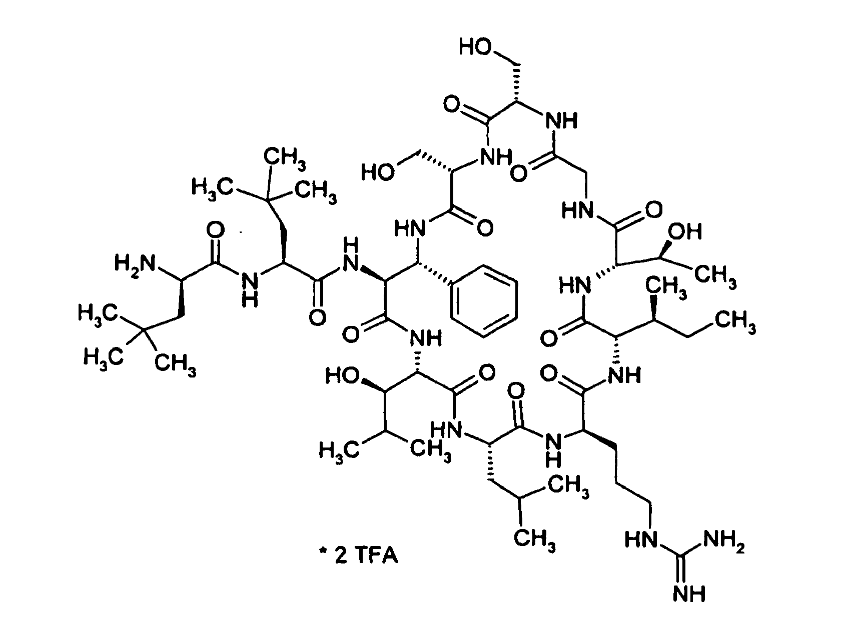

Die Verbindung aus Beispiel 233A (4 mg, 3 µmol) wird in Methanol (1 ml) gelöst. 17 µl 1 M Salzsäure und 10 % Palladium-Kohle (2 mg) werden zugegeben und der Ansatz über 2 h bei Normaldruck und Raumtemperatur hydriert. Die Lösung wird vom Katalysator abfiltriert und eingeengt. Nach chromatographischer Reinigung (Methode 44) erhält man 2.2 mg (1.5 µmol 53% d. Th.) der Titelverbindung.
HPLC (Methode 5): Rₜ = 4.45 min.
LC-MS (Methode 20): Rₜ = 1.45 min, MS (ESIpos): m/z (%) = 631.1 (100) [M+2H]²⁺, 1260.9 (3) [M+H]⁺.
HR-TOF-MS (Methode 24): C₅₉H₁₀₂N₁₅O₁₅ ber. 1260.7675, gef. 1260.7670 [M+H]⁺.

### Beispiel 9

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-[3-(3-pyridyl)-L-alanyl]-L-serin-C^{1.11}-N^{3.3}-lactam tris-trifluoracetat

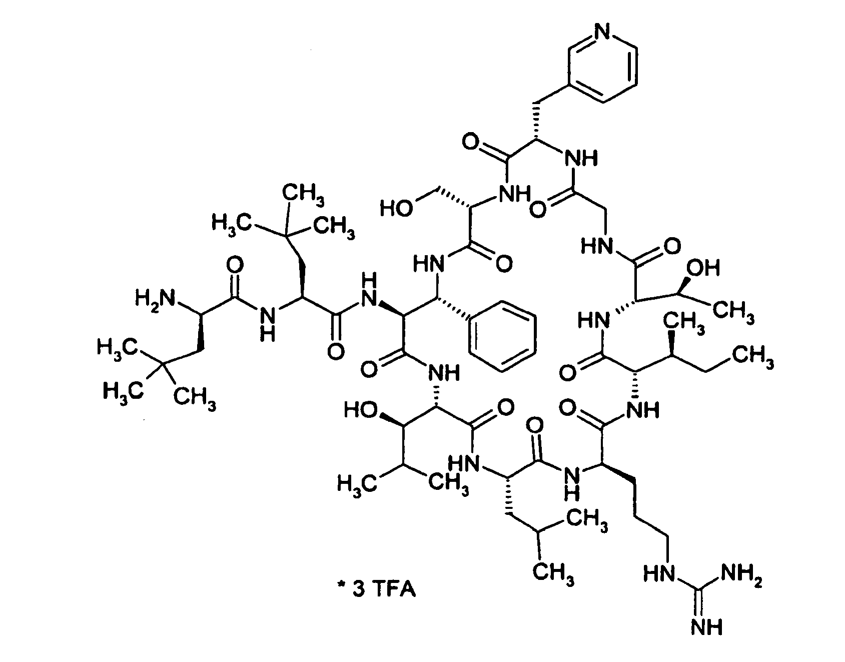

Gemäß der Vorschrift zur Darstellung der Verbindung aus Beispiel 233A wird aus der Verbindung aus Beispiel 226A (12 mg, 13 µmol) die Titelverbindung in einer Ausbeute von 4 mg (32% d. Th.) erhalten.
HPLC (Methode 5): Rₜ = 3.60 min.
LC-MS (Methode 19): Rₜ = 1.43 min, MS (ESIpos): m/z (%) = 661.5 (100) [M+2H]²⁺, 1321.8 (10) [M+H]⁺.
HR-TOF-MS (Methode 24): C₆₄H₁₀₅N₁₆O₁₄ ber. 1321.7991, gef. 1321.7955 [M+H]⁺.

### Beispiel 10

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-phenylalanyl-L-serin-C^{1.11}-N^{3.3}-lactam-bis-trifluoracetat

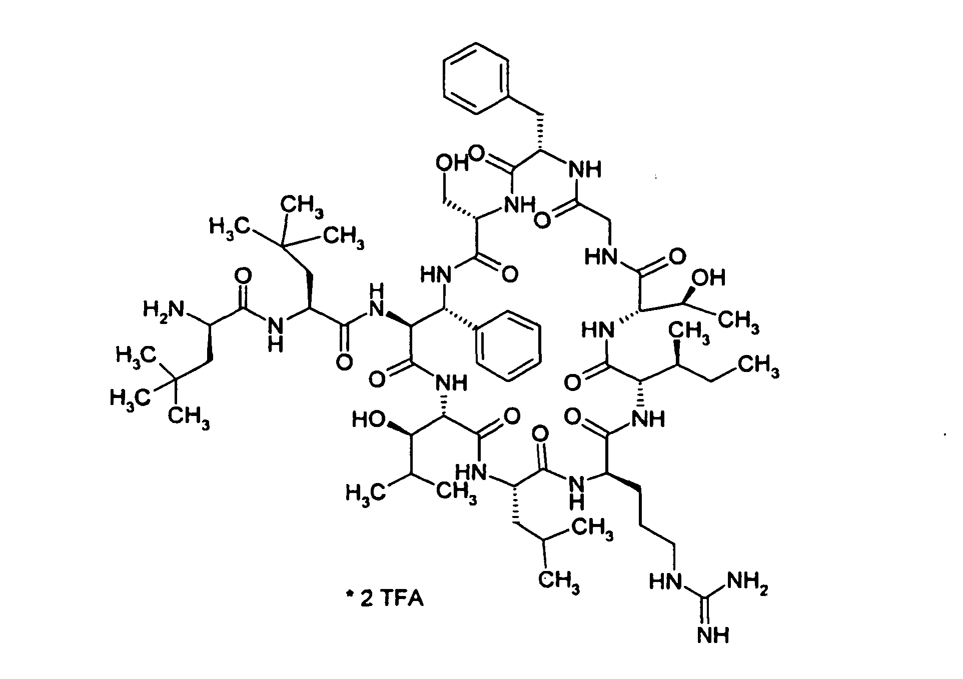

Die Verbindung aus Beispiel 235A wird nach der allgemeinen Arbeitsvorschrift 2 mit 1 ml der Reagenzlösung über 1 h umgesetzt und das Produkt nach der modifizierten Methode 44 (Gradient: 0 min 25 % B, Rampe, 35-38 min 35% B) gereinigt. Man erhält die Titelverbindung in einer Ausbeute von 10 mg (50% d. Th.).
HPLC (Methode 5): Rₜ = 3.41 min.
LC-MS (Methode 19): Rₜ = 1.57 min, MS (ESIpos): m/z (%) = 661.1 (100) [M+2H]²⁺, 1320.8 (5) [M+H]⁺; MS (ESIneg): m/z (%) = 1318.6 (90) [M-H]⁻.
HR-TOF-MS (Methode 24): C₆₅H₁₀₆N₁₅O₁₄ ber. 1320.8039, gef. 1320.8042 [M+H]⁺.

### Beispiel 11

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-attothreonyl-glycyl-[L-threonyl]-L-serin-C^{1.11}-N^{3.3}-lactam-bis-trifluoracetat

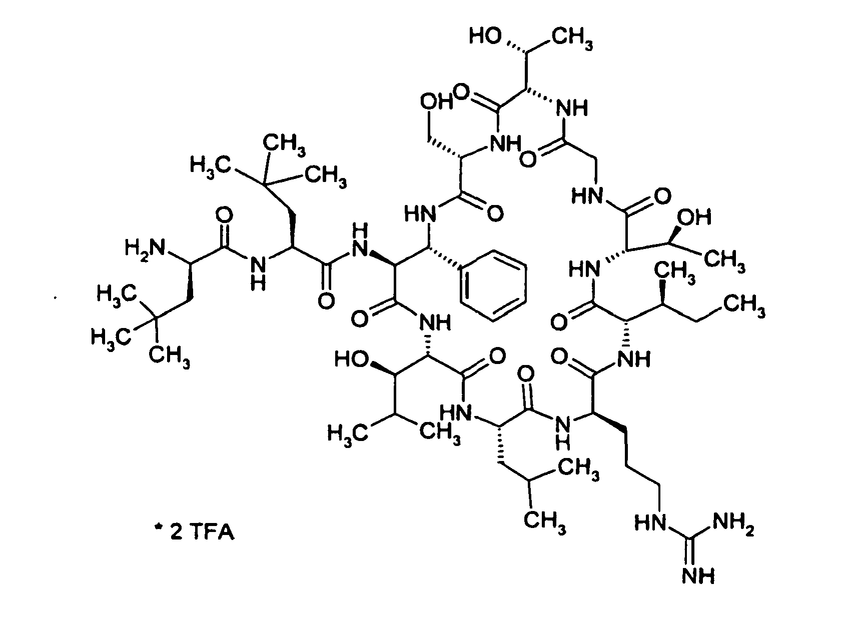

Die Verbindung aus Beispiel 236A wird nach der allgemeinen Arbeitsvorschrift 2 mit 1 ml der Reagenzlösung über 30 min umgesetzt und das Produkt nach Methode 44 gereinigt. Man erhält die Titelverbindung in einer Ausbeute von 3 mg (27% d. Th.).
HPLC (Methode 5): Rₜ = 3.73 min.
LC-MS (Methode 19): Rₜ = 1.53 min, MS (ESIpos): m/z (%) = 638.0 (100) [M+2H]²⁺.
HR-TOF-MS (Methode 24): C₆₀H₁₀₃N₁₅O₁₅ ber. 1274.7831, gef. 1274.7827 [M+H]⁺.

### Beispiel 12

### [3-tert-Butyl-D-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-allothreonyl-L-serin-C^{1.11}-N^{3.3}-lactam-bis-tritluoracetat

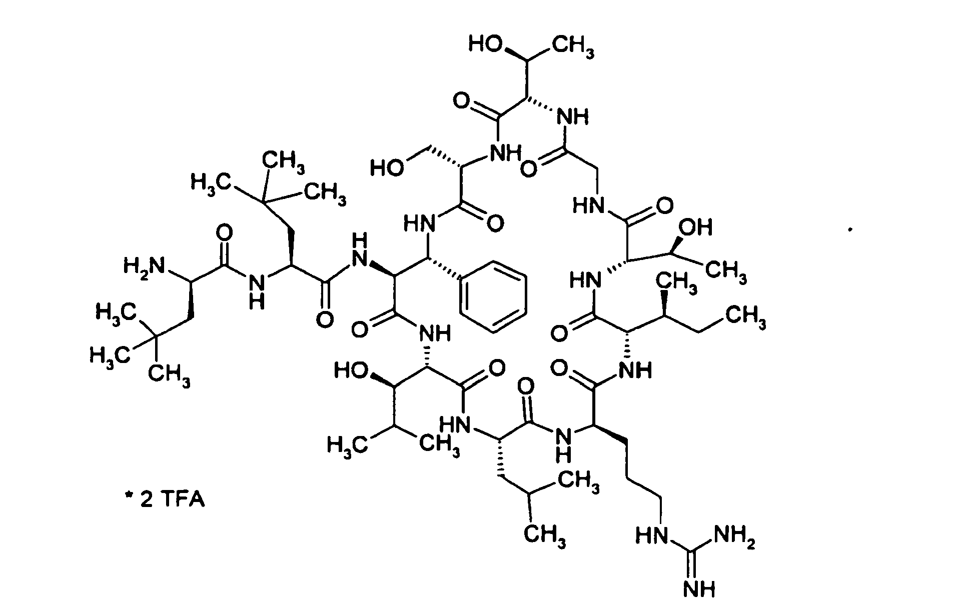

Die Verbindung aus Beispiel 237A (47 mg, 24 µmol) wird nach der allgemeinen Arbeitsvorschrift 2 mit 1 ml der Reagenzlösung über 30 min umgesetzt und das Produkt nach Methode 44 gereinigt. Man erhält die Titelverbindung in einer Ausbeute von 21 mg (57% d. Th.).
HPLC (Methode 6): Rₜ = 3.43 min.
LC-MS (Methode 19): Rₜ = 1.52 min, MS (ESIpos): m/z (%) = 638.1 (100) [M+2H]^{2+;} MS (ESIneg): m/z (%) = 1273.6 [M]⁻.
HR-TOF-MS (Methode 24): C₆₀H₁₀₃N₁₅O₁₅ ber. 1274.7831, gef. 1274.7800 [N4+H]⁺.

### Beispiel 13

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-alanyl-L-serin-C^{1.11}-N^{3.3}-lactam-bis-trifluoracetat

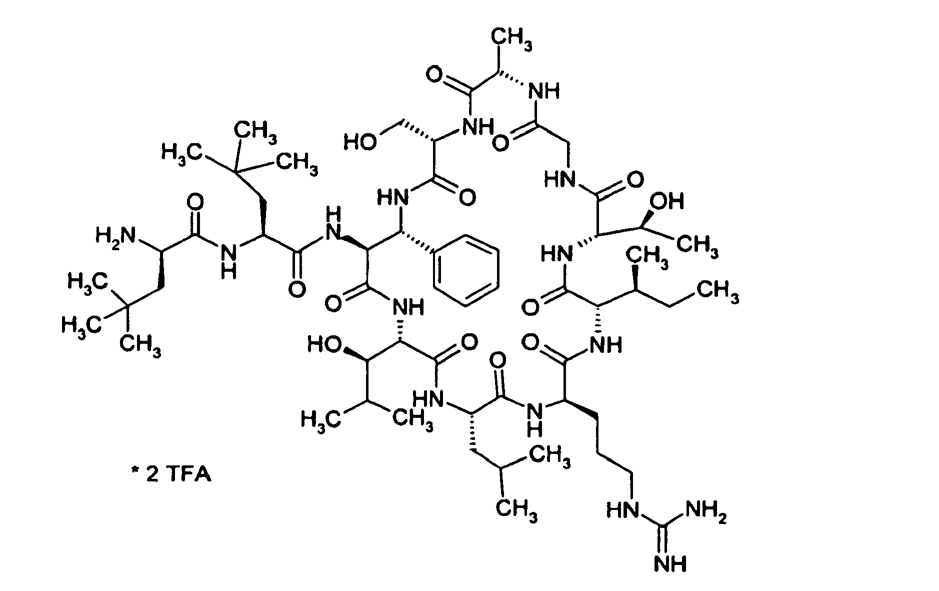

Die Verbindung aus Beispiel 238A wird nach der allgemeinen Arbeitsvorschrift 2 mit 1 ml der Reagenzlösung über 30 min umgesetzt und das Produkt nach Methode 44 gereinigt. Dann wird noch einmal feingereinigt: Säule: Waters Symmetry-Prep™ C-18, 7 µm, 300 mm x 19 mm; Eluens A: Wasser + 0.05% TFA, Eluens B: Acetonitril + 0.05% TFA: Fluss: 10 ml/min; A:B 65/35 isokratisch. Man erhält die Titelverbindung in einer Ausbeute von 8 mg (20% d. Th.).
HPLC (Methode 5): Rₜ = 3.71 min.
LC-MS (Methode 19): Rₜ = 1.49 min, MS (ESIpos): m/z (%) = 623.2 (100) [M+2H]²⁺.
HR-TOF-MS (Methode 24): C₅₉H₁₀₁N₁₅O₁₄ ber. 1244.7726, gef. 1244.7748 [M+H]⁺.

### Beispiel 14

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-seryl-glycyl-[(3S)-3-hydroxy-L-asparaginyl]-L-serin-C^{1.11}-N^{3.3}-lactam-bis-trifluoracetat

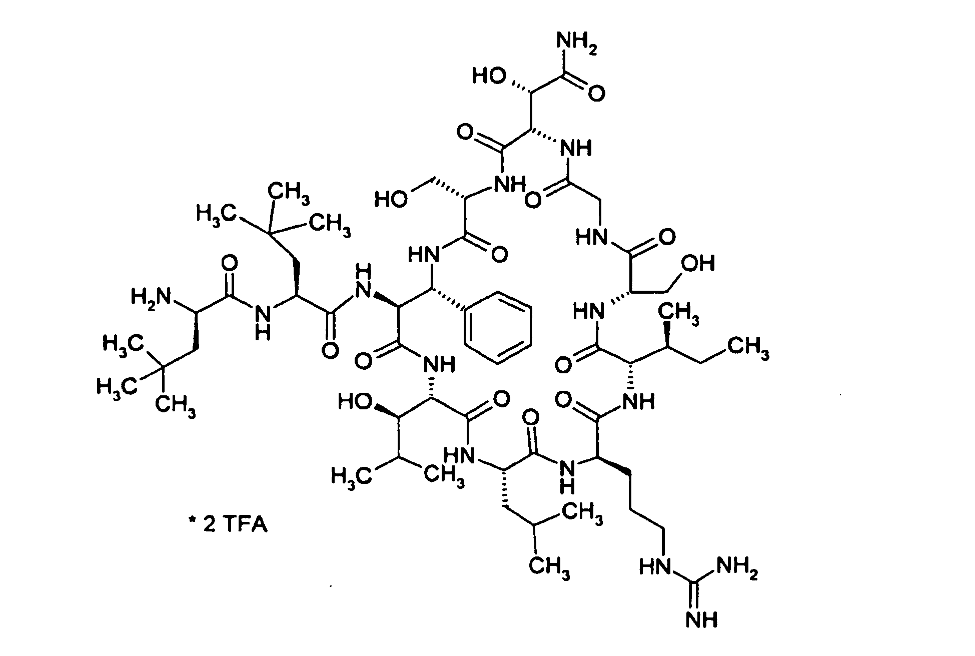

Die Verbindung aus Beispiel 239A wird nach der allgemeinen Arbeitsvorschrift 2 mit 1 ml der Reagenzlösung über 1 h umgesetzt und das Produkt nach Methode 44 gereinigt. Dann wird noch einmal feingereinigt: Säule: Waters Symmetry-Prep^{™} C-18, 7 µm, 300 mm x 19 mm; Eluens A: Wasser + 0.05% TFA, Eluens B: Acetonitril + 0.05% TFA: Fluss: 10 ml/min; A:B 65/35 isokratisch. Man erhält die Titelverbindung in einer Ausbeute von 9 mg (6 µmol, 35% d. Th.).
HPLC (Methode 5): Rₜ = 3.63 min.
LC-MS (Methode 19): Rₜ = 1.43 min, MS (ESIpos): m/z (%) = 643.7 (100) [M+2H]²⁺, 1289.8 (10) [M+H]⁺.
HR-TOF-MS (Methode 24): C₅₉H₁₀₁N₁₆O₁₆ ber. 1289.7576, gef. 1289.7578 [M+H]⁺.

### Beispiel 15

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-L-asparaginyl-L-serin-C^{1.11}-N^{3.3}-lactam-bis-trifluoracetat

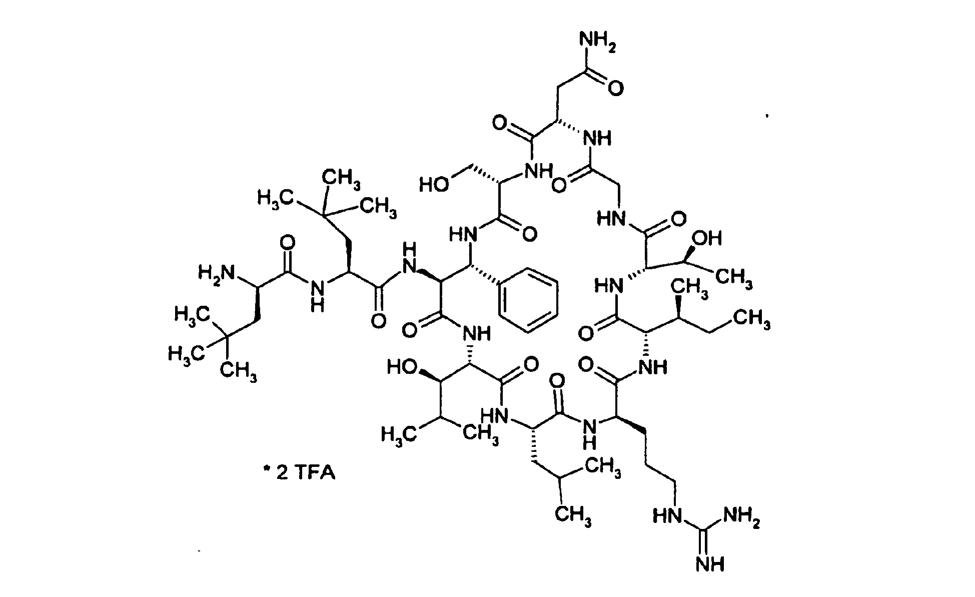

Die Verbindung aus Beispiel 240A wird nach der allgemeinen Arbeitsvorschrift 2 mit 1 ml der Reagenzlösung über 1 h umgesetzt und das Produkt mehrfach nach Methode 44 gereinigt. Man erhält die Titelverbindung in einer Ausbeute von 1.1 mg (6 µmol, 3.5% d. Th.).
HPLC (Methode 5): Rₜ = 3.41 min.
LC-MS (Methode 22): Rₜ = 2.90 min, MS (ESIpos): m/z (Hz) = 645 (100) [M+2H]²⁺, 1288 (10) [M+H]⁺.
HR-TOF-MS (Methode 24): C₆₀H₁₀₃N₁₆O₁₅ ber. 1287.7784, gef. 1287.7786 [M+H]⁺.

### Beispiel 16

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-[(3S)-3-hydroxy-L-asparaginyl]-L-alanin-C^{1.11}-N^{3.3}-lactam-bis-trifluoracetat

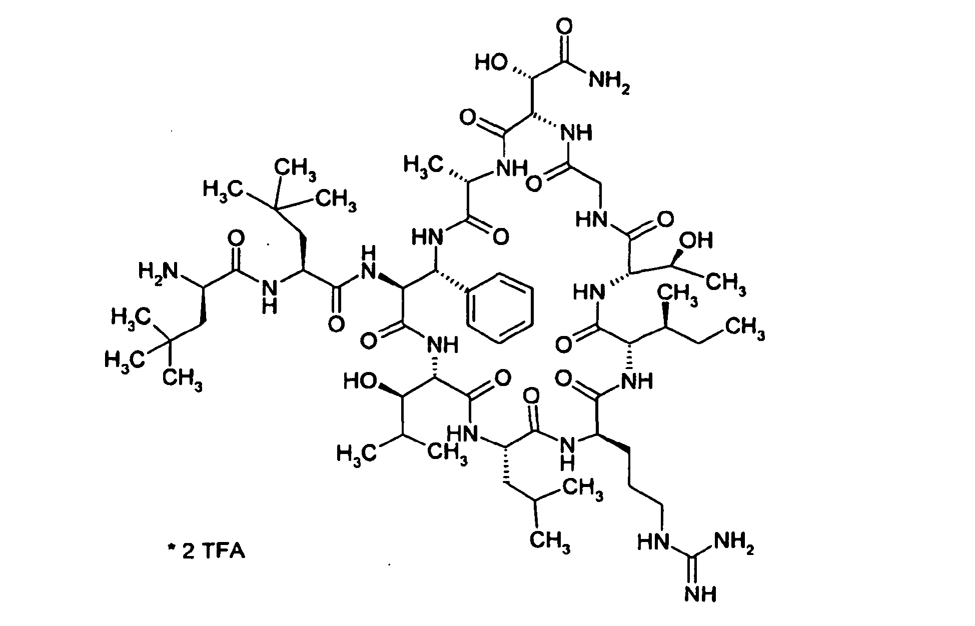

Die Verbindung aus Beispiel 253A wird nach der allgemeinen Arbeitsvorschrift 2 mit 2 ml der Reagenzlösung über 1 h umgesetzt und das Produkt nach Methode 44 gereinigt. Das Produkt wird mit einer Variante der Methode 34 (Verwendung von Eluent A:Eluent B 3:2; isokratisch an Stelle des Gradienten) feingereinigt. Man erhält die Titelverbindung in einer Ausbeute von 22 mg (36% d. Th.).
HPLC (Methode 6): Rₜ = 3.43 min.
LC-MS (Methode 22): Rₜ = 2.90 min, MS (ESIpos): m/z (%) = 645 (100) [M+2H]²⁺, 1288 (60) [M+H]⁺; MS (ESIneg): m/z (%) = 1286 (100) [M-H]⁻.
HR-TOF-MS (Methode 24): C₆₀H₁₀₃N₁₆O₁₅ ber. 1287.7784, gef. 1287.7800 [M+H]⁺.

### Beispiel 17

### [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-D-alanyl-[(3S)-3-hydroxy-L-asparaginyl]-L-serin-C^{1.11}-N^{3.3}-lactam-bis-trifluoracetat

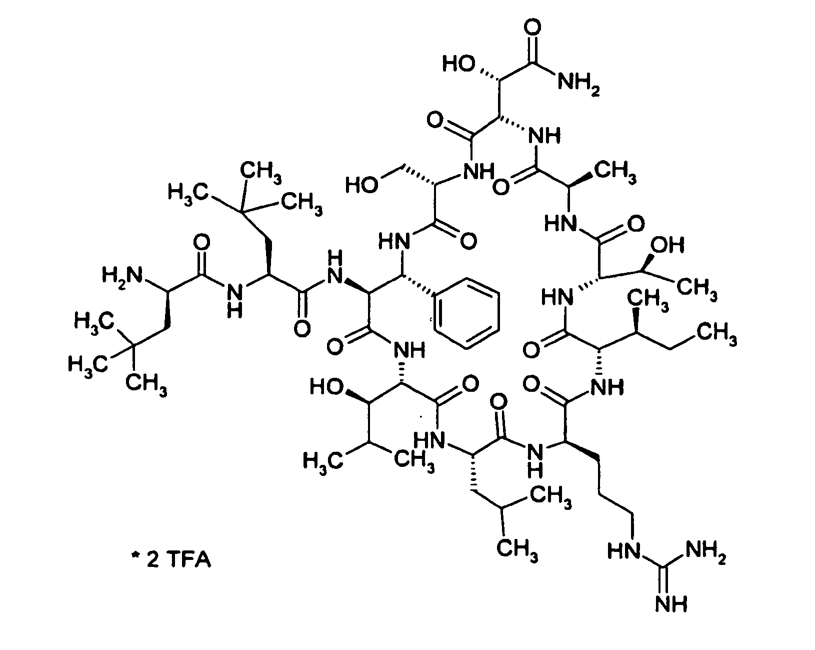

Die Verbindung aus Beispiel 267A (34 mg Rohprodukt, ca 6 µmol) wird nach der allgemeinen Arbeitsvorschrift 2 mit 1 ml der Reagenzlösung über 30 min umgesetzt. Das Produkt wird durch zweimalige Chromatographie nach Methode 33 rein erhalten. Man erhält die Titelverbindung in einer Ausbeute von 6 mg (65% d. Th.).
HPLC (Methode 6): Rₜ = 3.42 min.
LC-MS (Methode 22): Rₜ = 2.90 min, MS (ESIpos): m/z (%) = 660 (100) [M+2H]²⁺, 1317.8 (10) [M+H]⁺; MS (ESIneg): m/z (%) = 1315.8 (100) [M-H]⁻.
HR-TOF-MS (Methode 24): C₆₁H₁₀₅N₁₆O₁₆ ber. 1317.7889, gef. 1317.7892 [M+H]⁺.

Die in der nachfolgenden Tabelle dargestellten Verbindungen 19-44 wurden gemäß den angegebenen Arbeitsvorschriften aus den angegebenen Edukten hergestellt.

| **Ausführungsbeispiel-Nr** | **Struktur** | |
|---|---|---|
| | **Name** | **Analytik** |
| | **Ausbeute, Synthesemethode** | |
| 19 | 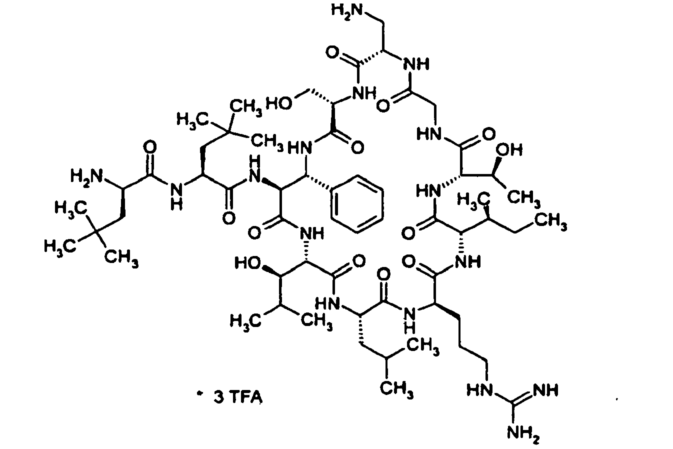 | |
| | [3-*tert*-Butyl-D-alanyl]-[3-*tert*-butyl-L-alanyl]-[(3*R*)-3-amino-L-phenylalanyl]-[(3*R*)-3-hydroxy-L-leucyl]-L-leucyl-n-arginyl-L-isoleucyl-L-allothreonyl-glycyl-[(2*S*)-2,3-diaminopropionyl]-L-serin-*C^{1.11-}N^{3.3}*-lactam-tris-trifluoracetat | HPLC (Methode 6): Rₜ = 3.34 min; LC-MS (Methode 19): Rₜ = 1.38 min, MS (ESIpos): *m*/*z* (%) = 630.5 (100) [M+2H]²⁺, 1259.8 (10) [M+H]⁺, MS (ESIneg): *m*/*z* (%) = 1257.8 (30) [M-H]⁻; 1304.7 (100) (M+HCOO⁻]⁻;HR- |
| | Ausbeute: 120 mg (44% d. Th.) aus Beispielverbindung 432A (273 mg, 172 µmol) nach Arbeitsvorschrift 2, Reinigung nach Methode 44. | TOF-MS: C₅₉H₁₀₃N₁₆O₁₄ ber. 1259.7835, gef. 1259.7811 [M+H]⁺. |
| 20 | 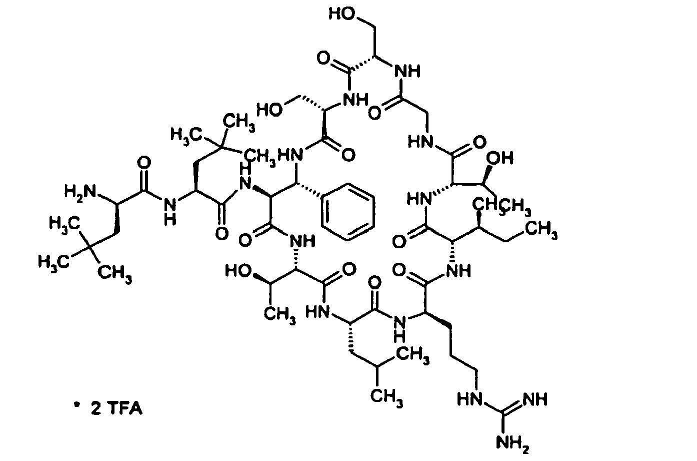 | |
| | [3-*tert*-Butyl-D-alanyl]-[3-*tert*-butyl-L-alanyl]-[(3*R*)-3-amino-L-phenylalanyl]-L-threonyl-L-leucyl-D-arginyl-L-isoleucyl-L-allothreonyl-glycyl-L-serin-*C^{1.11}*-*N^{3.3}*-lactam-bis-trifluoracetat | HPLC (Methode 6): Rₜ = 3.45 min; LC-MS (Methode 19): Rₜ = 1.52 min, MS (ESIpos): m/z (%) = 617.1 (100) [M+2H]²⁺, 1232.9 (10) [M+H]⁺, MS |
| | Ausbeute: 1.1 mg (17% d. Th.) aus Beispielverbindung 433A (6 mg, 4 µmol) nach Arbeitsvorschrift 2, Mehrfache Reinigung nach Methode 33. | (ESIneg): m/z (%) = 1230.9 (100) [M+H]⁺, 1277.0 (80) [M+HCOO⁻]⁻; HR-TOF-MS: C₅₇H₉₈N₁₅O₁₅ ber. 1232.7362, gef. 1232.7371 [M+H]⁺. |
| 21 | 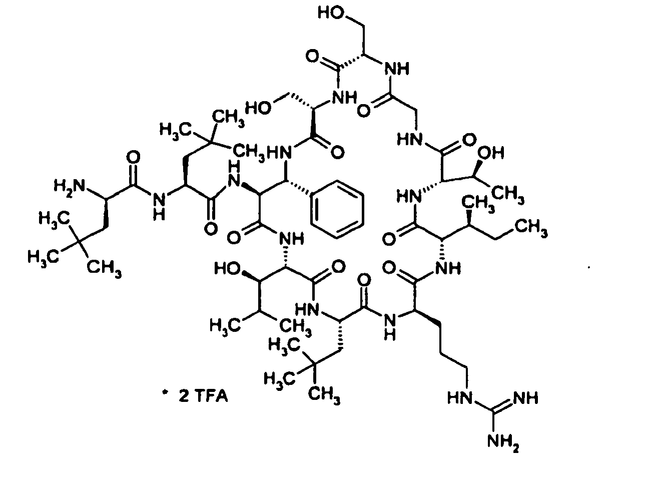 | |
| | [3-*tert*-Butyl-D-alanyl]-[3-tert-butyl-L-alanyl]-[(3*R*)-3-amino-L-phenylalanyl]-[(3*R*)-3-hydroxy-L-leucyl]-[3-*tert*-buryl-L-alanyl]-D-arginyl-L-isoleucyl-L-allothreonyl-glycyl-L-seryl-L-serin-*C^{1.11}-N^{3.3}*-lactam-bis-trifluoracetat | HPLC (Methode 6): Rₜ = 3.52 min; LC-MS (Methode 19): Rₜ = 1.58 min, MS (ESIpos): *m*/*z* (%) = 638.3 (100) [M+2H]²⁺, 1275.2 (5) [M+H]⁺, MS (E-SIneg): *m*/*z* (%) = 1273.0 (100) [M-H] ; HR-TOF-MS: C₆₀H₁₀₄N₁₅O₁₅ ber. 1274.7831, gef. 1274.7804 [M+H]⁺. |
| | Ausbeute: 6.5 mg (25% d. Th.) aus Beispielverbindung 434A (22 mg, 16 µmol) nach Arbeitsvorschrift 2, Reinigung nach Methode 44. | |
| 22 | 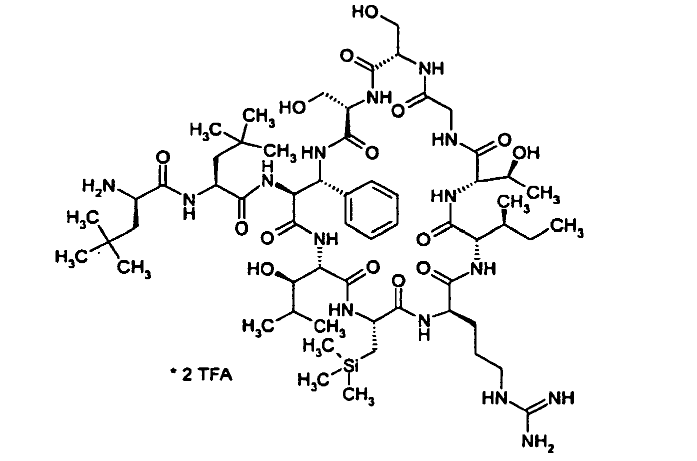 | |
| | [3-*tert*-Butyl-n-alanyl]-[3-*tert*-butyl-L-alanyl]-[(3*R*)-3-amino-L-phenylalanyl]-[(*3R*)-3-hydroxy-L-leucyl]-[3-trimethylsilyl-L-alanyl]-D-arginyl-L-isoleucyl-L-allothreonyl-glycol-L-seryl-L-serin-*C^{1.11}*-*N^{3.3}*-lactam-bis-trifluoracetat | HPLC (Methode 6): Rₜ = 3.58 min; LC-MS (Methode 19): Rₜ = 646.1 (100) [M+2H]²⁺, 1291.1 (5) [M+H]⁺ min, MS (ESIpos): *m*/*z* (%) =, MS (ESIneg): *m*/*z* (%) = 1289.0 (30) m-, 1335.0 (100) [M+HCOO⁻]⁻; HR-TOF-MS: C₅₉H₁₀₄N₁₅O₁₅Si ber. 1290.7601, gef. 1290.7576 [M+H]⁺. |
| | Ausbeute: 13 mg (42% d. Th.) aus Beispielverbindung 435A (29 mg, 20 µmol) nach Arbeitsvorschrift 2, Reinigung nach Methode 44. | |
| 23 | 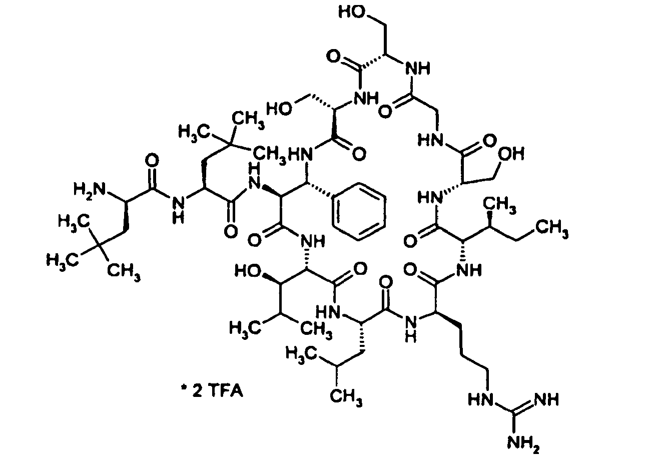 | |
| | [3-*tert-*Butyl-D-alanyl]-[3-*tert*-butyl-L-alanyl]-[(3*R*)-3-amino-L-phenylalanyl]-[(*3R*)-3-hydroxy-L-leucyl]-L-leuCyl-D-arginyl-L-isoleucyl-L-seryl-glycyl-L-seryl-L-serin-*C^{1.11}*-*N^{3.3}*-lactam-bis-trifluoracetat | HPLC (Methode 6): Rₜ = 3.44 min; LC-MS (Methode 19): Rₜ = 1.67 min, MS (ESIpos): *m*/*z* (%) = 624.0 (100) [M+2H]²⁺, 1246.8 (5) [M+H]⁺, MS (E-SIneg): *m*/*z* (%) = 1244.8 (80) [M-H]⁻, 1290.8 (100) [M+HCOO⁻]⁻; HR-TOF-MS: C⁵⁸H₁₀₀N₁₅O₁₅ ber. 1246.7518, gef. 1246.7500 [M+H]⁺. |
| | Ausbeute: 13 mg (54% d. Th.) aus Beispielverbindung 436A (23 mg, 16 µmol) nach Arbeitsvorschrift 2, Reinigung nach variierter Methode 44 (Rampe nur bis 60% Acetonitril). | |
| 24 | 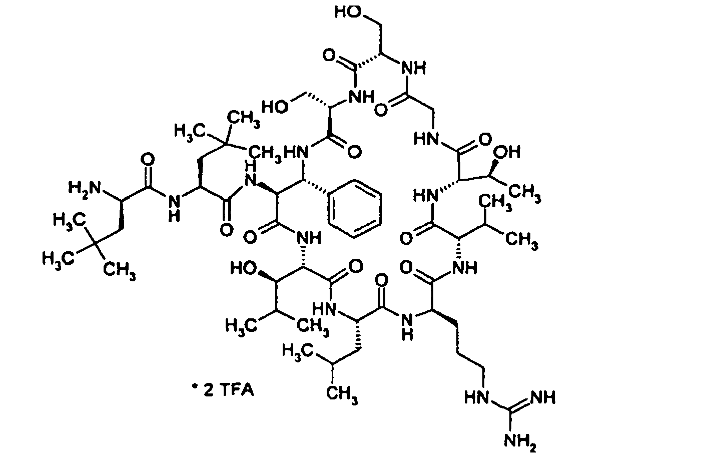 | |
| | [3-*tert*-Butyl-D-alanyl]-[3-*tert*-butyl-L-alanyl]-[(3*R*)-3-amino-L-phenylalanyl]-[(3R)-3-hydroxy-L-leucyl]-L-leucyl-D-arginyl-L-valyl-L-allothreonyl-glycyl-L-seryl-L-serin-*C^{1.11}-N^{3.3}*-lactam-bis-trifluoracetat | HPLC (Methode 6): Rₜ = 3.42 min; LC-MS (Methode 19): Rₜ = 1.47 min, MS (ESIpos): *m*/*z* (%) = 624.1 (100) [M+2H]²⁺, 1246.7 (10) [M+H]⁺, MS (ESIneg): *m*/*z* (%) = 1290.7 (50) |
| | Ausbeute: 13 mg (44% d. Th.) aus Beispielverbindung 437A (73 mg, 20 µmol) nach Arbeitsvorschrift 2, Reinigung nach variierter Methode 44 (Rampe nur bis 60% Acetonitril). | [M+HCOO⁻]⁻; HR-TOF-MS: C₅₈H₁₀₀N₁₅O₁₅ ber. 1246.7518, gef. 1246.7512 [M+H]⁺. |
| 25 | 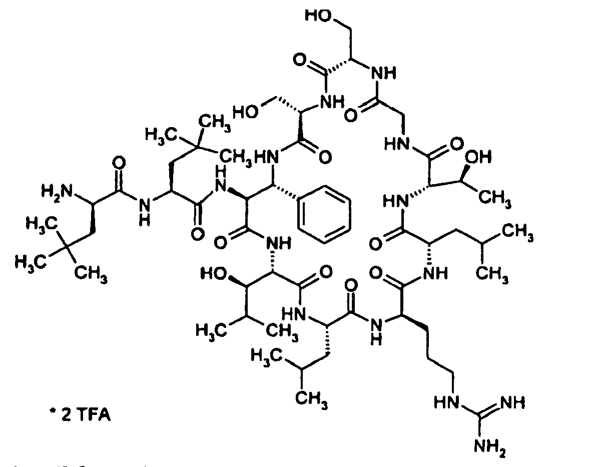 | |
| | [3-*tert*-Butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-leucyl-L-allothreonyl-glycyl-L-seryl-L-serin-*C^{1.11}-N^{3.3}*-lactam-bis-trifluoracetat | HPLC (Methode 6): Rₜ = 3.49 min; LC-MS (Methode 19): Rₜ = 1.62 min, MS (ESIpos): *m*/*z* (%) = 631.2 (100) [M+2H]²⁺, 1260.9 (1) [M+H]⁺, MS (E-SIneg): *m*/*z* (%) = 1259.9 (90) [M-H]⁻, 1305 ([M+HCOO⁻]⁻; HR-TOF-MS: C₅₉H₁₀₀N₁₅O₁₅ ber. 1260.7675, gef. 1260.7642 [M+H]⁺. |
| | Ausbeute: 8 mg (11% d. Th.) aus Beispielverbindung 438A (72 mg, 49 µmol) nach Arbeitsvorschrift 2, mehrfache Reinigung nach variierter Methode 44 (Rampe nur bis 60% Acetonitril). | |
| 26 | 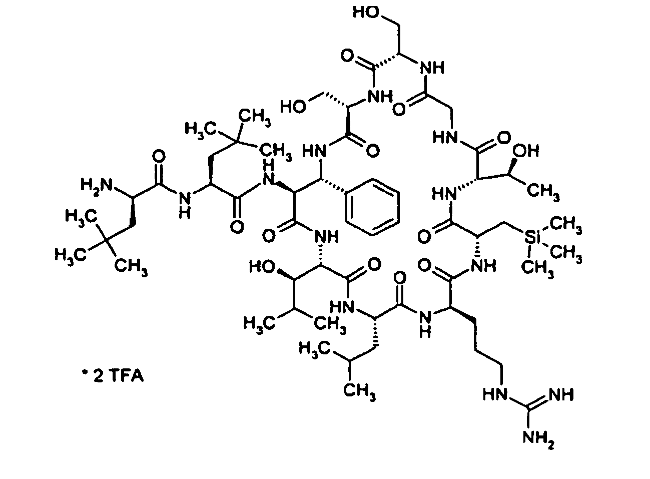 | |
| | [3-*tert*-Butyl-D-alanyl]-[3-tert-butyl-L-alanyl]-[(3*R*)-3-amino-L-phenylalanyl]-[(*3R*)-3-hydroxy-L-leucyl]-L-leucyl-D-arginyl-[3-trimethylsilyl-L-alanlyl]-L-allothreonyl-glycyl-L-seryl-L-serin-*C^{1.11}-N^{3.3}*-lactam-bis-trifluoracetat | HPLC (Methode 6): Rₜ = 3.57 min; LC-MS (Methode 19): Rₜ = 1.69 min, MS (ESIpos): *m*/*z* (%) = 646.1 (100) [M+2H]²⁺, 1290.9 (5) [M+H]⁺, MS (E-SIneg); *m*/*z* (%) = 1288. (90) [M-H]⁻, SIneg): *mlz (%)* = 1288.9 (90) [M-H]⁻, 1336.0 (100) [M+HCOO⁻]⁻; HR-TOF-1336.0 (100) [M+HCOO]; HR-TOF-MS: C₅₉H₁₀₄N₁₅O₁₅Si ber. 1290.7601, gef. 1290.7587 [M+H]⁺. |
| | Ausbeute: 12 mg (26% d. Th.) aus Beispielverbindung 439A (74 mg, 30 µmol) nach Arbeitsvorschrift 2, mehrfache Reinigung nach variierter Methode 44 (Rampe nur bis 60% Acetonitril). | |
| 27 | 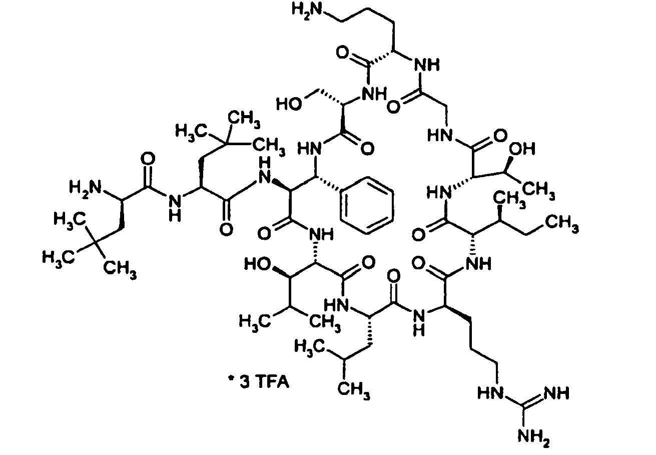 | |
| | [3-*tert*-Butyl-D-alanyl]-[3-*tert*-butyl-L-alanyl]-[(3*R*)-3-amino-L-phenylalanyl]-[(3*R*)-3-hydroxy-L-leucyl]-L-leucyl-D-arginyl-L-isoleucyl-L-allothreonyl-glycyl-L-ornithyl-L-serin-*C^{1.11}-N^{3.3}*-lactam-tris-trifluoracetat | HPLC (Methode 6): Rₜ = 3.31 min; LC-MS (Methode 19): Rₜ = 1.50 min, MS (ESIpos): *m*/*z* (%) = 644.6 (100) [M+2H]²⁺, 1287.9 (5) [M+H]⁺, MS (E-[M+2H]²⁺, 1287.9 (5) [M+H]⁺, MS (E-SIneg): *m*/*z* (%) = 1285.9 (50) [M-H]⁻; HR-TOF-MS: C₆₁H₁₀₇N₁₆O₁₄ ber. 1287.8148, gef. 1287.8126 [M+H]⁺. |
| | Ausbeute: 4 mg (6% d. Th.) aus Beispielverbindung 441A (65 mg, 39 µmol) nach Arbeitsvorschrift 4, mehrfache Reinigung nach variierter Methode 44 (Rampe nur bis 55% Acetonitril). | |
| 28 | 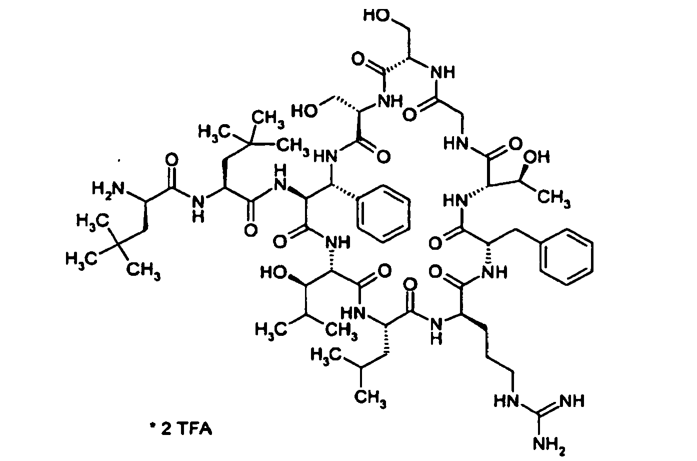 | |
| | [3-*tert*-Butyl-D-alanyl]-3-*tert*-butyl-L-alanyl]-[(3*R*)-3-amino-L-phenylalanyl]-[(*3R*)-3-hydroxy-L-leucyl]-L-leucyl-D-arginyl-L-phenylalanyl-L-allothreonyl-glycyl-L-seryl-L-serin-*C^{1.11}*-*N^{3.3}*-lactam-bis-trifluoracetat | HPLC (Methode 6): Rₜ = 3.45 min; LC-MS (Methode 19): Rₜ = 1.76 min, MS (ESIpos): m/z (%) = 648.1 (100) [M+2H]²⁺, 1294.8 (1) [M+H]⁺, MS (E-SIneg): m/z (%) = 1292.9 (70) [M-H]⁻, 1338.6 (100) [M+HCOO⁻]⁻; HR-TOF-MS: C₆₂H₁₀₀N₁₅O₁₅ ber. 1294.7518, gef. 1294.7520 [M+H]⁺. |
| | Ausbeute: 8 mg (67% d. Th.) aus Beispielverbindung 442A (12 mg, 8 µmol) nach Arbeitsvorschrift 2, Reinigung nach variierter Methode 44 (Rampe nur bis 60% Acetonitril). | |
| 29 | 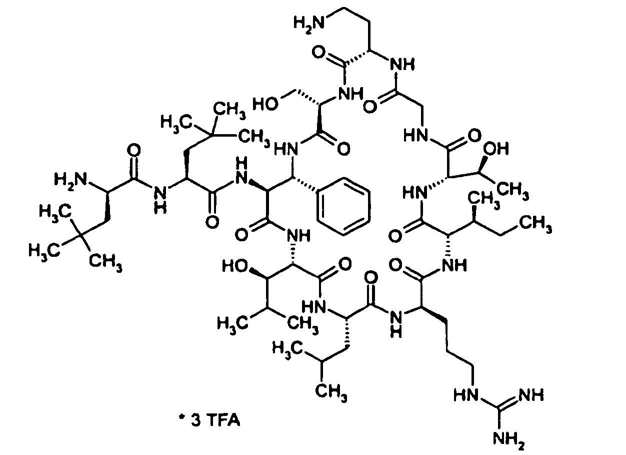 | |
| | [3-*tert*-Butyl-D-alanyl]-[3-*tert*-butyl-L-alanyl]-[(3*R*)-3-amino-L-phenylalanyl]-[(*3R*)-3-hydroxy-L-leucyl]-L-leucyl-D-arginyl-L-isoleucyl-L-allothreonyl-glycyl-[(*2S*)-2,4-diaminobutyryl]-L-serin-*C^{1.11}-N^{3.3}*-lactam-tris-trifluoracetat | HPLC (Methode 6): Rₜ = 3.23 min; LC-MS (Methode 19): Rₜ = 1.45 min, MS (ESIpos): *m*/*z* (%) = 425.4 (100) [M+3H]³⁺, 637.6 (50) [M+2H]²⁺, 1273.9 (5) [M+H]⁺, MS (ESIneg): *m*/*z* (%) = 1273.1 (70) [M-H]⁻ ,1318.1 (100) [M+HCOO⁻]⁻; HR-TOF-MS: C₆₀H₁₀₅N₁₆O₁₄ ber. 1273.7991, gef. 1273.8020 [M+H]⁺. |
| | Ausbeute: 7 mg (10% d. Th.) aus Beispielverbindung 444A (75 mg, 46 µmol) nach Arbeitsvorschrift 4, mehrfache Reinigung nach variierter Methode 44 (Rampe nur bis 55% Acetonitril). | |
| 30 | 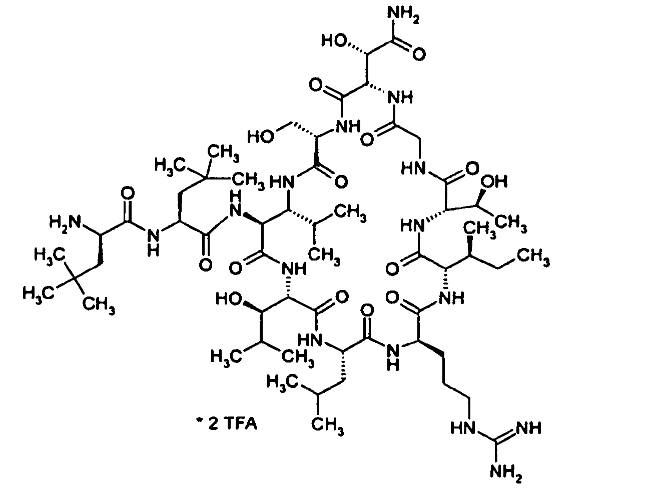 | |
| | [3-*tert-*Butyl-D-alanyl]-[3-tert-butyl-L-alanyl]-[(3*R*)-3-amino-L-leucyl]-[(3*R*)-3-hydroxy-L-leucyl]-L-leucyl-D-arginyl-L-isoleucyl-L-allothreonyl-glycyl-[(3R)-3-hydroxy-L-asparaginyl]-L-serin-*C^{1.11}-N^{3.3}*-lactam-bis-trifluoracetat | HPLC (Methode 6): Rₜ = 3.43 min; LC-MS (Methode 19): Rₜ = 1.52 min, MS (ESIpos): *m*/*z* (%) = 635.6 (100) [M+2H]^{z+}, 1269.8 (10) [M+H]⁺, MS (ESIneg): *m*/*z* (%) = 1267.8 (80) [M.H]⁻, 1313.8 (80) [M+HCOO⁻]⁻; HR-TOF-MS: C₅₇H₁₀₅N₁₆O₁₆ ber. 1269.7889, gef. 1269.7886 [M+H]⁺. |
| | Ausbeute: 29 mg (53% d. Th.) aus Beispielverbindung 445A (53 mg, 36 µmol) nach Arbeitsvorschrift 2, Reinigung nach variierter Methode 44 (Rampe nur bis 60% Acetonitril). | |
| 31 | 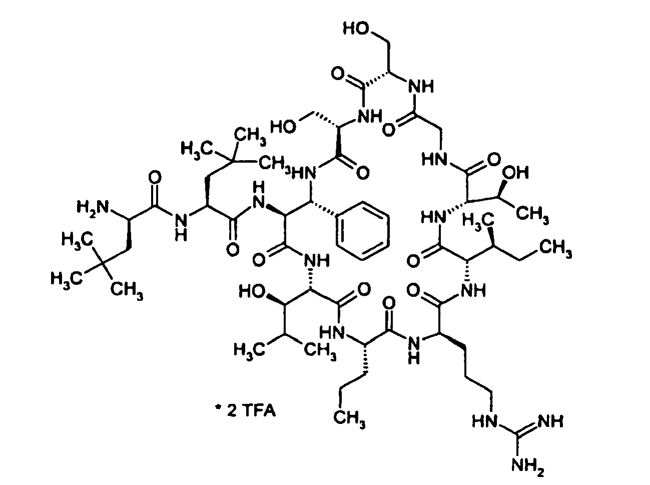 | |
| | [3-*tert* Butyl-D-alanyl]-[3-*tert*-butyl-L-alanyl]-[(3*R*)-3-amino-L-phenylalanyl]-[(3*R*)-3-hydroxy-L-leucyl]-L-norvalyl-D-arginyl-L-isoleucyl-L-allothreonyl-glycyl-L-seryl-L-serin-*C^{1.11}-N^{3.3}*-lactam-bis-trifluoracetat | HPLC (Methode 6): Rₜ = 3.42 min; LC. MS (Methode 51): Rₜ = 2.15 min, MS (ESIpos): *m*/*z* (%) = 624.6 (100) [M+2H]²⁺, 1246.9 (1) [M+H]⁺; HR-TOF-MS: C₅₈H₁₀₀N₁₅O₁₅ ber. 1246.7518, gef. 1246.7549 [M+H]⁺. |
| | Ausbeute: 54 mg (27% d. Th.) aus Beispielverbindung 446A (54 mg, 37 µmol) nach Arbeitsvorschrift 2, Reinigung nach variierter Methode 44 (Rampe nur bis 60% Acetonitril). | |
| 32 | 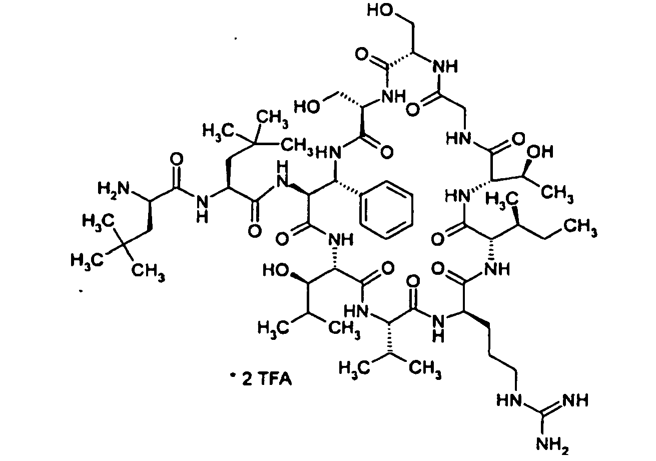 | |
| | [3-*tert*-Butyl-D-alanyl]-[3-tert-butyl-L-alanyl]-[(3*R*)-3-amino-L-phenylalanyl]-[(*3R*)-3-hydroxy-L-leucyl]-L-valyl-D-arginyl-L-isoleucyl-L-allothreonyl-glycyl-L-seryl-L-serin-*C^{1.11}-N^{3.3}*-lactam-bis-trifluoracetat | HPLC (Methode 6): Rₜ = 3.40 min; LC-MS (Methode 19): Rₜ = 1.51 min, MS (ESIpos): *m*/*z* (%) = 624.0 (100) [M+2H]²⁺, 1246.8 (10) [M+H]⁺, MS (EsIneg): *m*/*z* (%) = 1244.8 (90) [M-H]⁻, |
| | Ausbeute: 20 mg (36% d. Th.) aus Beispielverbindung 447A (54 mg, 37 µmol) nach Arbeitsvorschrift 2, Reinigung nach variierter Methode 44 (Rampe nur bis 60% Acetonitril). | 1291.7 (100) [M+HCOO⁻]⁻; HR-TOF-MS: C₅₈H₁₀₀N₁₅O₁₅ ber. 1246.7518, gef. 1246.7494 [M+H]⁺. |
| 33 | 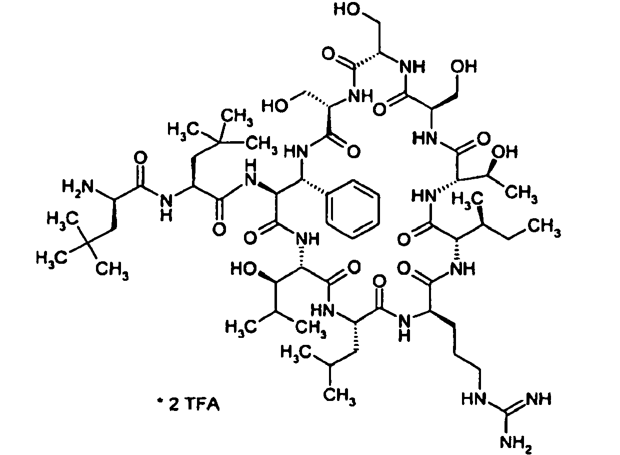 | |
| | [3-*tert*-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-D-seryl-L-seryl-L-serin-*C^{1.11}-N^{3.3}*-lactam-bis-trifluoracetat | HPLC (Methode 6): Rₜ = 3.44 min; LC-MS (Methode 19): Rₜ = 1.54 min, MS (ESIpos): *m*/*z* (%) = 646.2 (100) [M+2H]²⁺, 1290.8 (10) [M+H]⁺, MS |
| | Ausbeute: 21 mg (33% d. Th.) aus Beispielverbindung 448A (64 mg, 43 µmol) nach Arbeitsvorschrift 2, Reinigung nach variierter Methode 44 (Rampe nur bis 50% Acetonitril). | (ESIneg); *m*/*z* (%) = 1289.9 (60) [M-H]⁻, (ESIneg): *m*/*z* (%) = 1289.8 (60) [M-H]⁻, 1334.8 [M+HCOO⁻]⁻; HR-TOF-MS: C₆₀H₁₀₄N₁₅O₁₆ ber. 1290.7780, gef. 1290.7766 [M+H]⁺. |
| 34 | 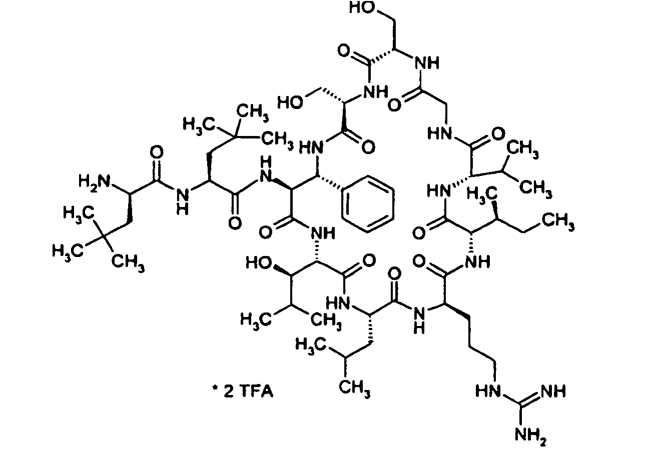 | |
| | [3-tert-Butyl-D-alanyl]-[3-tert-butyl-L-alanyl]-[(3R)-3-amino-L-phenylalanyl]-[(3R)-3-hydroxy-L-leueyl]-L-leucyl-D-arginyl-L-isoleucyl-L-valyl-glycyl-L-seryl-L-serin-*C^{1.11}-N^{3.3}*-lactam-bis-trifluoracetat | HPLC (Methode 6): Rₜ = 3.46 min; LC-MS (Methode 19): Rₜ = 1.66 min, MS (ESIpos): m/z (%) = 630.0 (100) [M+2H]²⁺, 1258.8 (10) [M+H]⁺, MS (ESIneg): m/z (%) = 1257.8 (90) [M-H]⁻, 1303.8 (100) [M+HCOO⁻]⁻; HR-TOF-MS: C₆₀H₁₀₄N₁₅O₁₄ ber. 1258.7882, gef. 1258.7905 [M+H]⁺. |
| | Ausbeute: 3.5 mg (10% d. Th.) aus Beispielverbindung 449A (33 mg, 23 µmol) nach Arbeitsvorschrift 2, Reinigung nach variierter Methode 44 (Rampe nur bis 50% Acetonitril). | |
| 35 | 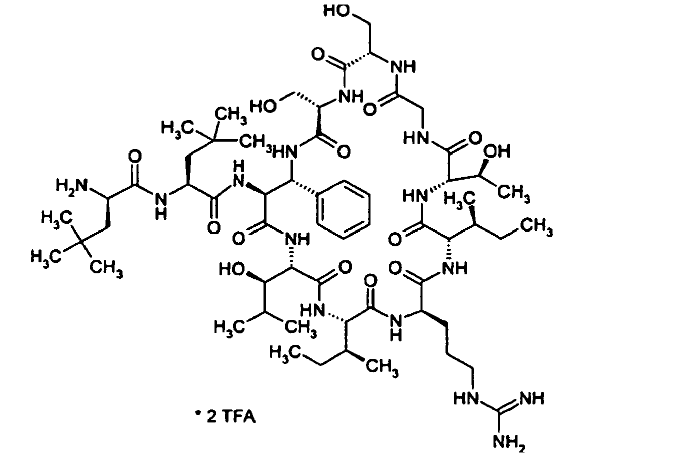 | |
| | *[3-tert-*Butyl-D-alanyl]-[3-tert-butyl-L-alanyl]-[(3R)-3-amino-L-phenylalanyl]-[(3R)-3-hydroxy-L-leucyl]-L-isoleucyl-D-arginyl-L-isoleucyl-L-allothreonyl-glycyl-L-seryl-L-serin-C^{1.11}-N^{3.3}-lactam-bis-trifluoracetat | HPLC (Methode 53): Rₜ = 5.76 min; LC-MS (Methode 19): Rₜ = 1.59 min, MS (ESIpos): m/z (%) = 631.2 (100) [M+2H]²⁺, 1260.8 (5) [M+H]⁺, MS (E-SIneg): m/z (%) = 1258.9 (100) [M-H]⁻; HR-TOF-MS: C₅₉H₁₀₂N₁₅O₁₅ ber. 1260.7675, gef. 1260.7666 [M+H]⁺. |
| | Ausbeute: 12 mg (70% d. Th.) aus Beispiel-verbindung 450A (19 mg, 10 µmol) nach Arbeitsvorschrift 2, Reinigung nach variierter Methode 44 (Rampe nur bis 70% Acetonitril). | |
| 36 | 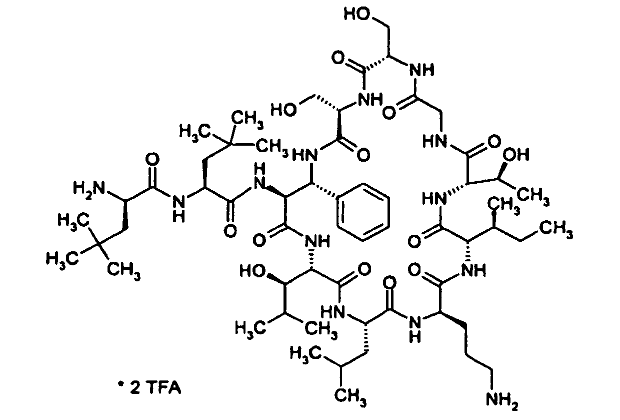 | |
| | [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-ornithyl-L-isoleucyl-L-allothreonyl-glycyl-L-seryl-L-serin-C^{1.11}-N^{3.3}-lactam-bis-trifluoracetat | HPLC (Methode 6): Rₜ = 3.43 min; LC-MS (Methode 19): Rₜ = 1.59 min, MS (ESIpos): m/z (%) = 610.1 (100) [M+2H]²⁺, 1218.8 (10) [M+H]⁺, MS (ESIneg): m/z (%) = 1216.8 (100) [M-H]⁻; HR-TOF-MS: C₅₈H₁₀₀N₁₃O₁₅ ber. 1218.7457, gef. 1218.7477 [M+H]⁺, |
| | Ausbeute: 12 mg (25% d. Th.) aus Beispielverbindung 451A (49 mg, 33 µmol) nach Arbeitsvorschrift 4, Reinigung nach variierter Methode 44 (Rampe nur bis 35% Acetonitril). | |
| 37 | 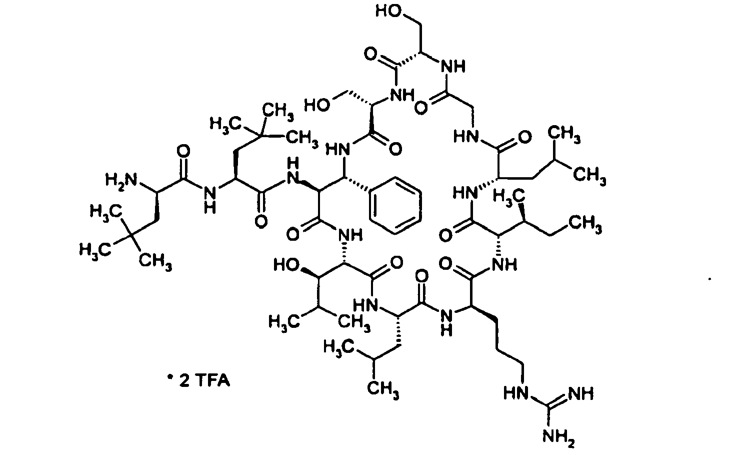 | |
| | [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-leucyl-glycyl-L-seryl-L-serin-C^{1.11}-N^{3.3}lactam-bis-trifluoracetat | HPLC (Methode 6): Rₜ = 3.56 min; LC-MS (Methode 52): Rₜ = 1.22 min, MS (ESIpos): m/z (%) = 637.2 (100) [M+2H]²⁺, 1273.0 (5) [N4+H]⁺, MS (E-SIneg): m/z (%) = 1271.1 (40) [M-H]⁻, |
| | Ausbeute: 15 mg (46% d. Th.) aus Beispielverbindung 452A (42 mg, 22 µmol) nach Arbeitsvorschrift 2, Reinigung nach variierter Methode 33 (Rampe nur bis 70% Acetonitril). | 1317.0 (100) [M+HCOO⁻]⁻; HR-TOF-MS: C₆₁H₁₀₆N₁₅O₁₄ ber. 1272.8039, gef. 1272.8015 [M+H]⁺. |
| 38 | 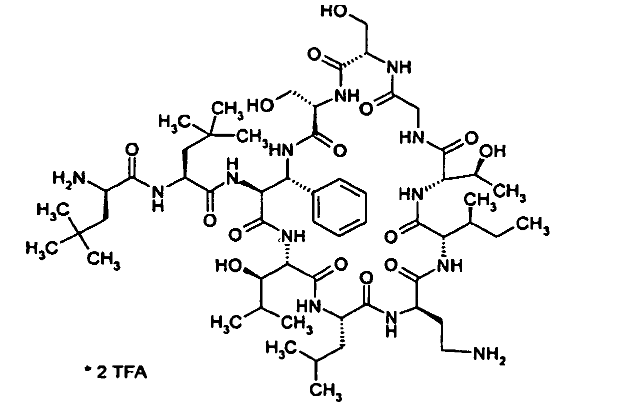 | |
| | [3-tert-Butyl-D-alanyl]-[3-tert-butyl-L-alanyl]-[(3R)-3-amino-L-phenylalanyl]-[(3R)-3-hydroxy-L-leucyl]-L-leucyl-[(2R)-2,4-diaminobutyryl]-L-isoleucyl-L-allothreonyl-glycyl-L-seryl-L-serin-*C^{1.11}-N^{3.3}*-lactam-bis-trifluoracetat | HPLC (Methode 6): Rₜ = 3.42 min; LC-MS (Methode 19): Rₜ = 1.59 min, MS (ESIpos): m/z (%) = 603.0 (100) [M+2H]²⁺; 1204.8 (10) [M+H]⁺, MS (ESIneg): m/z (%) = 1202.8 (100) [M-H]⁻, 1248.8 (50) [M+HCOO⁻]⁻; HR-TOF-MS: C₅₇H₉₈N₁₃O₁₅ ber. 1204.7300, gef. 1204.7329 [M+H]⁺. |
| | Ausbeute: 7 mg (40% d. Th.) aus Beispielverbindung 453A (162 mg, (111% rein, 12 µmol) nach Arbeitsvorschrift 4, Reinigung nach variierter Methode 44 (Rampe nur bis 35% Acetonitril). | |
| 39 | 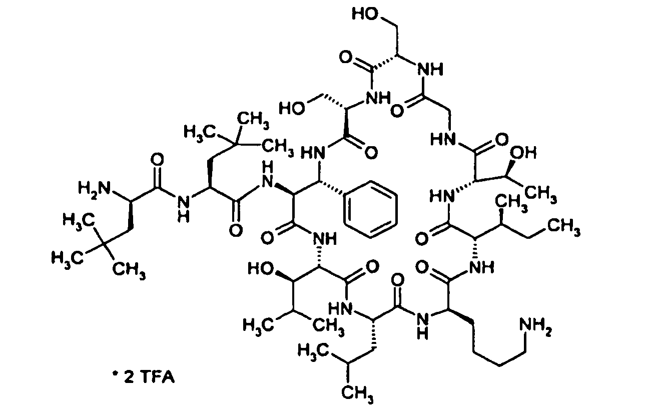 | |
| | [3-*tert*-Butyl-D-alanyl]-[3-*tert*-butyl-L-alanyl]-[(3R)-3-amino-L-phenylalanyl]-[(3R)-3-hydroxy-L-leucyl]-L-leucyl-D-lysyl-L-isoleucyl-L-allothreonyl-glycyl-L-seryl-L-serin-*C^{1.11}-N^{3.3}*-tactam-bis-trifluoracetat | HPLC (Methode 53): Rₜ = 5.66 min; LC-MS (Methode 19): Rₜ = 1.57 min, MS (ESIpos): m/z (%) = 617.1 (100) [M+2H]²⁺, 1232.8 (10) [M+H]⁺, MS (ESIneg): m/z (%) = 1231.8 (100) [M-H]⁻, ,1276.8 (50) [M+HCOO⁻]⁻; HR-TOF-MS: C₅₉H₁₀₂N₁₃O₁₅ ber. 1232.7613, gef. 1232.7598 [M+H]⁺. |
| | Ausbeute: 14 mg (62% d. Th.) aus Beispielverbindung 454A (29 mg, 80% rein, 15 µmol) nach Arbeitsvorschrift 4, Reinigung nach variierter Methode 44 (Rampe nur bis 70% Acetonitril). | |
| 40 | 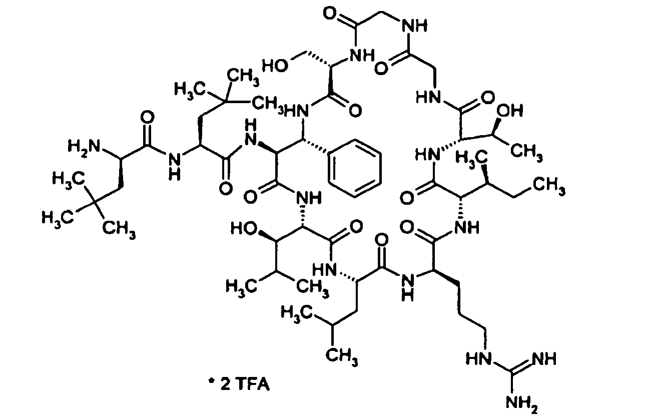 | |
| | [3-tert-Butyl-D-alanyl]-[3-tert-butyl-L-alanyl]-[(3R)-3-amino-L-phenylalanyl]-[(3R)-3-hydroxy-L-leucyl]-L-leucyl-D-arginyl-L-isoleucyl-L-allothreonyl-glycyl-glycyl-L-serin-C^{1.11} -N^{3.3}-lactam-bis-trifluoracetat | HPLC (Methode 6): Rₜ = 3.47 min; LC-MS (Methode 19): Rₜ = 1.54 min, MS (ESIpos): m/z (%) = 616.1 (100) [M+2H]²⁺, 1230.8 (10) [M+H]⁺, MS (ESIneg): m/z (%) = 1228.9 (60) [M-H]⁻, 1274.8 (100) [M+HCOO⁻]⁻; HR-TOF-MS: C₅₈H₁₀₀N₁₅O₁₄ ber. 1230.7569, gef. 1230.7570 [M+H]⁺. |
| | Ausbeute: 13 mg (7% d. Th.) aus Beispielverbindung 455A (177 mg, 123 µmol) nach Arbeitsvorschrift 2, Reinigung nach variierter Methode 33 (Rampe nur bis 60% Acetonitril). | |
| 41 | 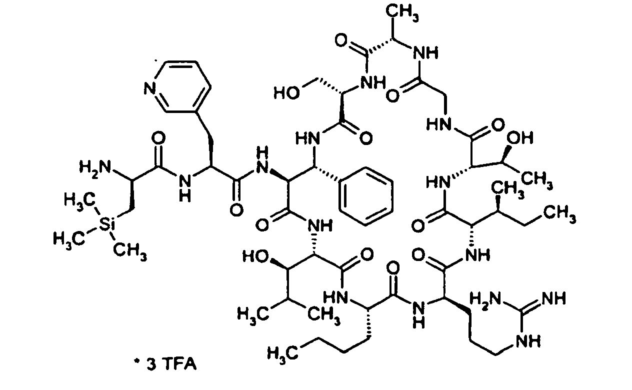 | |
| | [3-Trimethylsilyl-D-alanyl]-[3-(3-pyridyl)-L-alanyl]-[(3*R*)-3-amino-L-phenylalanyl]-[(3*R*)-3-hydroxy-L-leucyl]-L-norleucyl-D-arginyl-L-isoleucyl-L-allothreonyl-glycyl-L-alanyl-L-serin-*C^{1.11}-N^{3.3}*-lactam-tris-trifluoracetat | HPLC (Methode 6): Rₜ = 3.23 min; LC-MS (Methode 19): Rₜ = 1.41 min, MS (ESIpos): *m*/*z* (%) = 641.5 (100) [M+2H]²⁺, MS (ESIneg): *m*/*z* (%) = 1280.8 (100) [M-H]⁻; HR-TOF-MS: C₅₉H₉₇N₁₆O₁₄Si ber. 1281.7134, gef. 1281.7152 [M+H]⁺. |
| | Ausbeute: 13 mg (47% d. Th.) aus Beispielver-bindung 456A (45 mg, 60% rein, 17 µmol) nach Arbeitsvorschrift 2, Reinigung nach variierter Methode 44 (Rampe nur bis 60% Acetonitril). | |
| 42 | 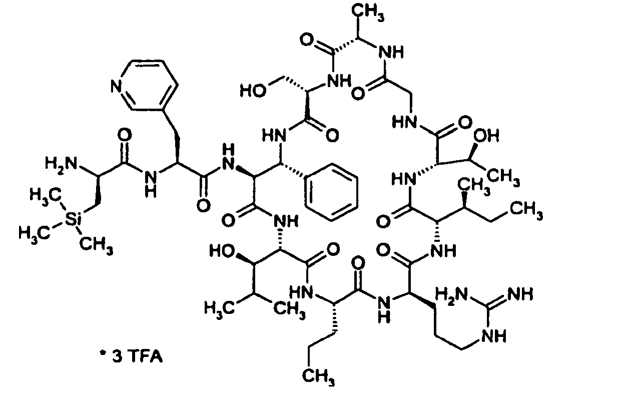 | |
| | [3-Trimethylsilyl-D-alanyl]-[3-(3-pyridyl)-L-alanyl]-[(3*R*)-3-amino-L-phenylalanyl]-[(3R)-3-hydroxy-L-leucyll-L-norvalyl-D-arginyl-L-isoleucyl-L-allothreonyl-glycyl-L-alanyl-L-serin-*C^{1.11}-N^{3.3}*-lactam-tris-trifluoracetat | HPLC (Methode 6): Rₜ = 3.16 min; LC-MS (Methode 19): Rₜ = 1.33 min, MS (ESIpos): *m*/*z* (%) = 634.5 (100) [M+2H]²⁺, 1267.7 (10) [M+H]⁺, MS (ESIneg): *m*/*z* (%) = 1265.8 (100) [M-H]⁻, 1311.8 (40) [M+HCOO⁻]⁻; HR-TOF-MS: C₅₈H₉₅N₁₆O₁₄Si ber. 1267.6978, gef. 1267.6979 [M+H]⁺. |
| | Ausbeute: 15 mg (68% d. Th.) aus Beispielverbindung 457A (41 mg, 51% rein, 13 µmol) und Beispielverbindung 274A nach Arbeitsvorschrift 2, Reinigung nach variierter Methode 44 (Rampe nur bis 55% Acetonitril). | |
| 43 | 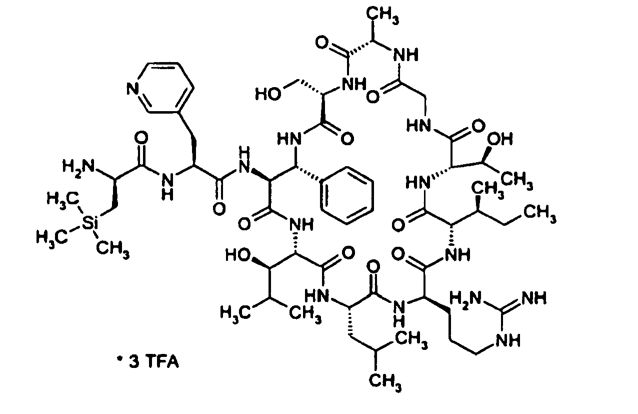 | |
| | [3-Trimethylsilyl-D-alanyl]-[3-(3-pyridyl)-L-alanyl]-[(3*R*)-3-amino-L-phenylalanyl]-[(*3R*)-3-hydroxy-L-leucyl]-L-leucyl-D-arginyl-L-isoleucyl-L-allothreonyl-glycyl-L-alanyl-L-serin-*C^{1.11}-N^{3.3}*-lactam-tris-trifluoracetat | HPLC (Methode 6): Rₜ = 3.22 min; LC-MS (Methode 19): Rₜ = 1.39 min, MS (ESIpos): *m*/*z* (%) = 641.6 (100) [M+2H]²⁺, 1281.7 (10) lM+H]⁺, MS (ESIneg): *m*/*z* (%) = 1279.8 (100) [M-H]⁻; HR-TOF-MS: C₅₉H₉₇N₁₆O₁₄Si ber. 1281.7134, gef. 1281.7128 [M+H]⁺. |
| | Ausbeute: 22 mg (79% d. Th.) aus Beispielverbindung 458A (45 mg, 60% rein, 17 µmol) nach Arbeitsvorschrift 2, Reinigung nach variierter Methode 44 (Rampe nur bis 60% Acetonitril). | |
| 44 | 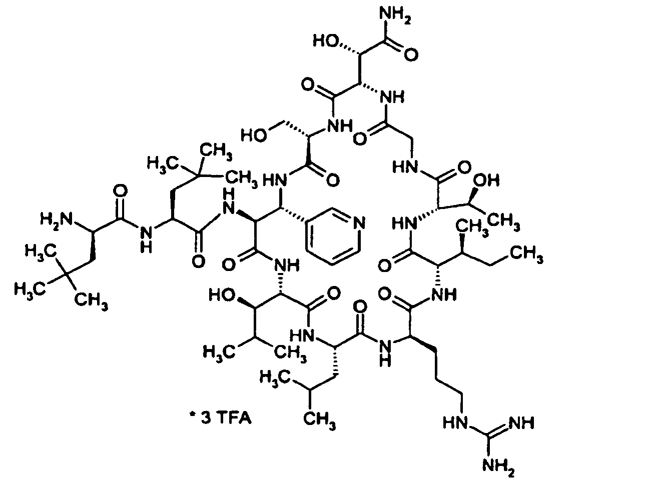 | |
| | [3-*tert*-Butyl-D-alanyl]-[3-*tert*-butyl-L-alanyl]-[(3R)-3-amino-3-(pyridin-3-yl)-L-alanyl]-[(3*R*)-3-hydroxy-L-leucyl]-L-leucyl-D-arginyl-L-isoleucyl-L-allothreonyl-glycyl-[(3*S*)-3-hydroxy-L-asparaginyl]-L-serin-*C^{1.11}-N^{3.3}*-lactam-tris-trifluoracetat | HPLC (Methode 6): Rₜ = 3.34 min; LC-MS (Methode 19): Rₜ = 1.53 min, MS (ESIpos): *m*/*z* (%) = 435.6 (100) [M+3H]³⁺, 653.0 (80, [M+2H]²⁺), 1304.8 (10) [M+H]⁺, MS (ESIneg): *m*/*z* (%) = 1302.8 (100) [M-H] HR-TOF-MS: C₅₉H₁₀₂N₁₇O₁₆ ber. 1304.7685, gef. 1304.7670 [M+H]⁺. |
| | Ausbeute: 10.6 mg (56% d. Th.) aus Beispielverbindung 470A (30 mg, 60% rein, 11 µmol) nach Arbeitsvorschrift 2, Reinigung nach variierter Methode 44 (Rampe nur bis 70% Acetonitril). | |

### B. Bewertung der physiologischen Wirksamkeit

Die *in vitro*-Wirkung der erfindungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Entercococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von S. *pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S*. *pneumoniae* in Gegenwart von 10% CO₂.

Die jeweils niedrigste Substanzkonzentration, bei der kein sichtbares Bakterienwachstum mehr auftritt, wird als MHK definiert. Die MHK-Werte werden in µg/ml angegeben.

Repräsentative in-vitro-Wirkdaten für die erfindungsgemäßen Verbindungen sind in Tabelle A wiedergegeben:

**Tabelle A**

| **Beispiel-Nr.** | **MHK *S. aureus* 133 [µg/ml]** | **MHK *S. pneumoniae G9a* [µg/ml]** | **MHK *E. faecalis ICB 27159* [µg/ml]** |
|---|---|---|---|
| **13** | 0.25 | 0.5 | 0.5 |
| **14** | 0.25 | 0.125 | 0.5 |
| **23** | 0.5 | 0.25 | 1 |
| **35** | 0.25 | 0.5 | 1 |
| **41** | 0.25 | 0.25 | 2 |
| **44** | 0.25 | 0.125 | 0.5 |
| **Lysobactin** | 0.5 | 0.063 | 0.5 |

Die Eignung der erfindungsgemäßen Verbindungen zur Behandlung von bakteriellen Infektionen kann im folgenden Tiermodell gezeigt werden:

### Systemische Infektion mit Staphylococcus aureus 133:

Zellen von *S*. *aureus* 133 werden über Nacht in BHI-Bouillon (Fa. Oxoid, New York/USA) angezüchtet. Die Übernachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden weibliche CFW1-Mäuse verwendet. Das Überleben der Tiere wird über 6 Tage protokolliert.

Die Eigenschaften der erfindungsgemäßen Verbindungen im Hinblick auf die Nierenverträglichkeit können im folgenden Tiermodell gezeigt werden:

### Maus-Modell zur Bestimmung nephrotoxischer Effekte:

Nephrotoxische Nebenwirkungen der Nonadepsipeptide werden durch histopathologische Untersuchungen der Nieren in Mäusen nach mehrfacher Gabe einer bestimmten Dosierung analysiert. Dafür werden 5 - 6 Tiere täglich entweder intravenös (i.v.) oder intraperitoneal (i.p.) mit Substanzen behandelt, die in wässriger Lösung oder unter Zusatz von Solutol gelöst werden. Nierentoxische Effekte werden durch lichtmikroskopische Auswertung Hämatoxilin und Eosin (H&E) gefärbter Paraffinschnitte der Nieren bestimmt. Eine 'Periodic-Acid Schiff' (PAS)-Reaktion wird wahlweise zur besseren Darstellung von Glykoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der durchschnittliche Schweregrad der tubulären Degeneration/Regeneration sowie die Inzidenz (Anzahl der betroffene Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgeführt.

### Ratten-Modell zur Bestimmung nephrotoxischer Effekte:

Nephrotoxische Nebenwirkungen der Nonadepsipeptide werden durch histopathologische Untersuchungen der Nieren in Ratten nach mehrfacher Gabe einer bestimmten Dosierung analysiert. Dafür werden 5 Tiere täglich intravenös (i.v.) mit Substanzen behandelt, die in Saline oder Ringer-Lactat-Lösung gelöst werden. Nierentoxische Effekte werden durch lichtmikroskopische Auswertung Hämatoxilin und Eosin (H&E) gefärbter Paraffinschnitte der Nieren bestimmt. Eine 'Periodic-Acid Schiff (PAS)-Reaktion wird wahlweise zur besseren Darstellung von Glykoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der, durchschnittliche Schweregrad der tubulären Degeneration/Regeneration sowie die Inzidenz (Anzahl der betroffenen Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgeführt.

### Prinzip der Bestimmung der freien Fraktion via Transil:

Die hier beschriebene Methode zur Bestimmung der freien Fraktion (fᵤ) einer Prüfsubstanz gliedert sich in 2 Teile:
a) Bestimmung des Transil^{®}/Puffer-Verteilungsverhältnisses (MA_{buffer}) durch Inkubation der Prüfsubstanz in einer Transil^{®}-Puffer (pH 7.4)-Dispersion und anschließende Bestimmung der Konzentration in der Dispersion und im Puffer-Überstand.
b) Bestimmung des Transil^{®}/Plasma-Verteilungsverhältnisses (MApₗₐₛₘₐ) durch Inkubation der Prüfsubstanz in einer Transil^{®}-Plasma-Dispersion und anschließende Bestimmung der Konzentration in der Dispersion und im Plasma.

Der Quotient der beiden Verteilungsverhältnisse ergibt fᵤ.

Bei hochproteingebundenen Substanzen wird das Plasma in der Regel mit isotonischem Phosphatpuffer (pH 7.4) verdünnt und anschließend mit Transil^{®} suspendiert. Die Bestimmung von fᵤ' (freie Fraktion in verdünntem Plasma) in dieser verdünnten Proteinlösung erfolgt analog zur Bestimmung von fᵤ. Die freie Fraktion in unverdünntem Plasma wird aus fᵤ' und dem Verdünnungsfaktor berechnet.

Vergleich zu dieser Methode auch: Schuhmacher, Joachim; Kohlsdorfer, Christian; Buehner, Klaus; Brandenburger, Tim; Kruk, Renate, "High-throughput determination of the free fraction of drugs strongly bound to plasma proteins." Journal of Pharmaceutical Sciences 2004, 93, 816-830.

### Bestimmung der Membranaffinität einer Prüfsubstanz nach Verteilung zwischen Transil^{®} und Puffer (MA_{buffer}):

Alle Inkubationen werden in geeigneten Glasgefäßen, z. B. Glasvials, Schliffreagenzgläsern, durchgeführt. In der Regel beträgt das Gesamtvolumen 0.5-5 ml, das Transil^{®} Volumen 10-100 µl. Im Falle von hohen erwarteten Membranaffinitäten kann die Transil^{®} Dispersion mit Phosphatpuffer pH 7.4 z.B. Dulbeccos PBS bis zu 20fach verdünnt werden. Phosphatpuffer pH 7.4 wird in die Inkubationsgefäße vorgelegt und das Transil^{®} nach gründlichem Mischen zupipettiert. Die Prüfsubstanz wird in einer Konzentration von z.B. 200 ng/ml, n = 6, zupipettiert. Der Anteil an organischem Lösungsmittel sollte ≤ 2% sein. Die Ansätze werden 30 min bei Raumtemperatur z.B. auf einem Mini-Shaker im Winkel von ca. 45° bei ca. 400 UPM inkubiert. Zur Bestimmung des 100% Wertes wird mindestens ein Aliquot von z.B. 100 µl entnommen, der restliche Ansatz wird ca. 10 min bei ca. 1800 g zentrifugiert. Von jeder Probe werden mindestens 2 Aliquote (z.B. 100 µl) des Überstandes für die Konzentrationsbestimmung entnommen.

### Bestimmung von MAₚₗₐₛₘₐ in unverdünntem oder verdünntem Plasma:

Das Gesamtinkubationsvolumen und das zugesetzte Transil^{®} Volumen hängen von der erwarteten freien Fraktion ab. In der Regel beträgt das Gesamtvolumen 0.5 -1 ml, das Transil^{®} Volumen 10-100 µl. Im Falle von sehr niedrigen freien Fraktionen wird das Plasma der zu untersuchenden Spezies mit isotonischer Pufferlösung, pH 7.4 z.B. 10 -400fach verdünnt und anschließend mit Transil^{®} versetzt. Das weitere Vorgehen erfolgt wie oben für die Bestimmung der MA_{buffer} Werte beschrieben.

### Prinzip der Bestimmung der freien Fraktion via Ultrafiltration:

Das Plasma der zu untersuchenden Spezies wird über eine semipermeable Membran filtriert. Die Substanzkonzentration im Filtrat wird gemessen und daraus die freie Fraktion f_{U} berechnet. Das Centrifree micropartition system von Millipore/Amicon wird verwendet. Die Ultrafiltrationsmembranen haben eine Ausschlussgröße von 30 000 Da. Die Substanz wird zu 1 ml Plasma in einer Konzentration von ca. 1 µg/ml zudotiert. Der Lösungsmittelanteil sollte < 2 % sein. Nach 30 minütiger Inkubation bei Raumtemperatur wird das Plasma in das Ultrafiltrationssystem pipettiert und 10 Minuten bei 1800 g zentrifugiert. Die Substanzkonzentration im Ultrafiltrat (Cᵤ; ungebundene Substanzkonzentration) und im Plasma vor der Zentrifugation (C; Gesamtsubstanzkonzentration) wird gemessen. Die freie Fraktion errechnet sich nach der Formel: fᵤ (%) = Cᵤ/C *100.

### Bestimmung der chemischen Stabilität in wässriger leicht alkalischer Lösung

0,3 mg der Testsubstanz werden in einem Gemisch aus 0,25 mL Acetonitril, 0,25 mL DMSO und 0,5mL Puffer pH 7,8 (bestehend aus 111 mg (1 mmol) Calciumchlorid, 7.91 g (100 mmol) Ammoniumhydrogencarbonat, 100 mL Acetonitril ad 1000 mL) gelöst und bei Raumtemperatur stehen gelassen. Aus der Lösung wird einmal pro Stunde ein Aliquot von 5 µL entnommen und mit HPLC (Methode 57) analysiert Die Konzentration der Testverbindung zum Zeitpunkt 0 h, angegeben durch die UV-Absorption (IE) wird gleich 100% gesetzt und die Abnahme über die Zeit beobachtet. Zugleich wird die Zunahme des ringoffenen Abbauproduktes beobachtet.

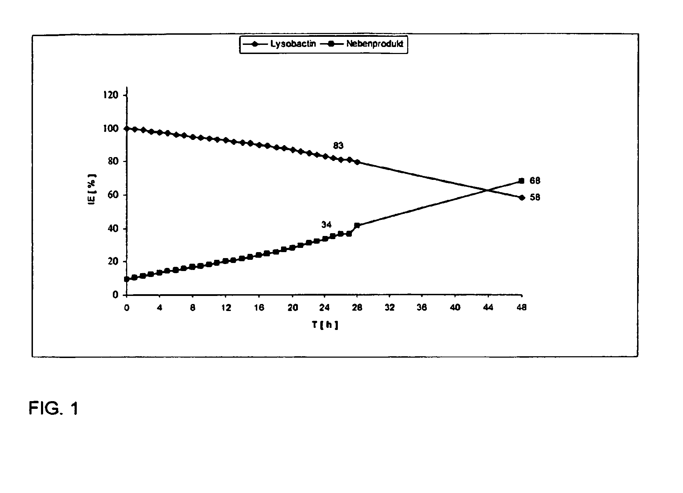

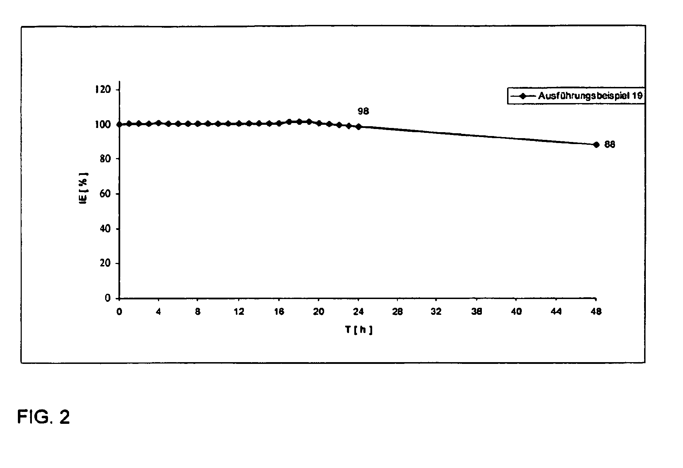

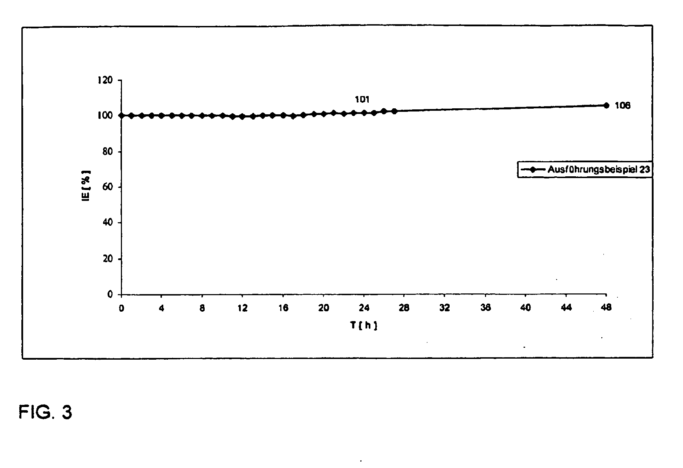

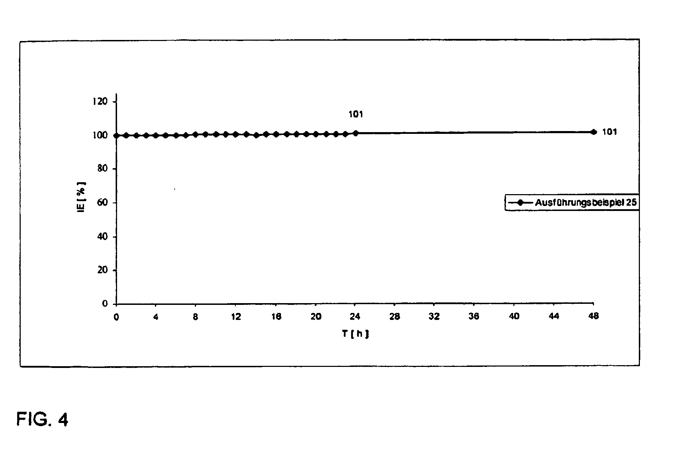

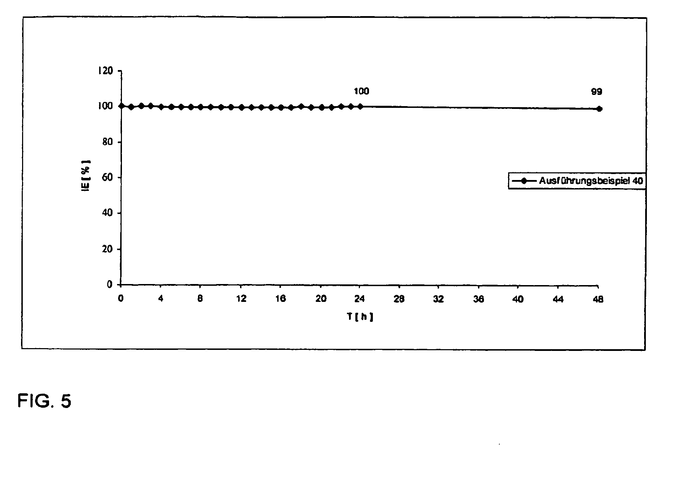

Dieser Assay lieferte die in den Figuren 1-5 dargestellten Ergebnisse. Aus den Kurven geht hervor, dass die Verbindungen der Erfindung in wässriger alkalischer Lösung deutlich stabiler als Lysobactin sind.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-eigen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 um) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 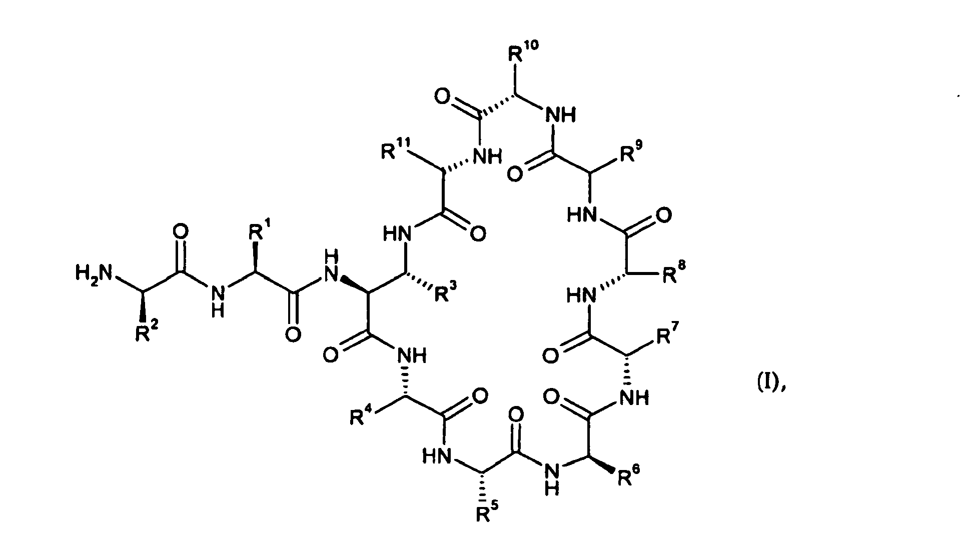 in welcher
R¹ 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, , 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
R² 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thien-ylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
R³ Wasserstoff, C₁-C₄-Alkyl, C₃-C₇-Cycloalkyl, C₃-C₇-Cycloalkylmethyl, Phenyl, Benzyl, 5- oder 6-gliedriges Heteroaryl oder 5- oder 6-gliedriges Heteroarylmethyl bedeutet,
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl, C₁-C₄-Alkylaminocarbonyl und über Stickstoff gebundenes 5- oder 6-gliedriges Heterocyclyl,
R⁴ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁵ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁶ C₁-C₆-Alkyl bedeutet,
wobei Alkyl substituiert ist mit einem Substituenten ausgewählt aus der Gruppe bestehend aus Amino, 1,4,5,6-Tetrahydropyrimidin-2-ylamino, [Amino(imino)-methyl]amino, 2-Pyridyl, 3-Pyridyl und 4-Pyridyl,
R⁷ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl oder Trimethylsilylmethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁸ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁹ Wasserstoff, C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl oder 2-Amino-2-oxoethyl bedeutet,
wobei Alkyl substituiert sein kann mit 1 bis 3 Substituenten unabhängig voneinander ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R¹⁰ Wasserstoff, C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R¹¹ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Ami- no, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
oder eines ihrer Salze, ihrer Solvate oder der Solvate ihrer Salze,
ausgenommen eine Verbindung der Formel 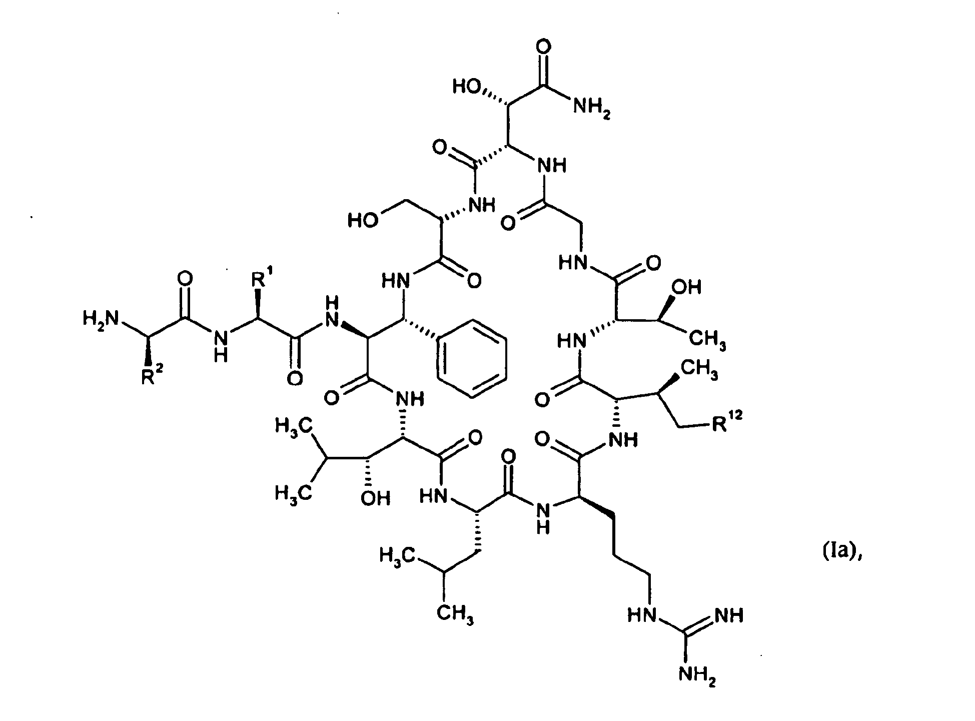 in welcher
R¹ 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
R² 2-Methylprop-1-yl, 2,2-Dimethylprop-1-yl, 2,2-Dimethylbut-1-yl, Trimethylsilylmethyl, Benzyl, 2-Pyridylmethyl, 3-Pyridylmethyl, 2-Thienylmethyl, 3-Thienylmethyl oder 1,3-Thiazol-4-ylmethyl bedeutet,
wobei Benzyl, 2-Pyridylmethyl, 3-Pyridylmethyl, 2-Thienylmethyl, 3-Thienylmethyl und 1,3-Thiazol-4-ylmethyl substituiert sein können mit 1 oder 2 Substituenten unabhängig voneinander ausgewählt aus der Gruppe bestehend aus Halogen, Trifluormethyl, Methyl und Methoxy,
und
R¹² Wasserstoff oder Methyl bedeutet,
oder eines ihrer Salze, ihrer Solvate oder der Solvate ihrer Salze.

2. Verbindung nach Anspruch 1, **dadurch gekennzeichnet, dass**
R¹⁰ C₁-C₆-Alkyl, C₃-C₇-Cycloalkyl, C₃-C₇-Cycloalkylmethyl, Phenyl, Benzyl, 5- oder 6-gliedriges Heteroaryl, 5- oder 6-gliedriges Heteroarylmethyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl, Cycloalkylmethyl, Phenyl, Benzyl, Heteroaryl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
oder eines ihrer Salze, ihrer Solvate oder der Solvate ihrer Salze.

3. Verbindung nach Anspruch 1 oder 2, **dadurch gekennzeichnet, dass**
R¹ 2-Methylprop-1-yl, 2,2-Dimethylprop-1-yl, 2,2-Dimethylbut-1-yl, Trimethylsilylmethyl, Benzyl, 2-Pyridylmethyl oder 3-Pyridylmethyl bedeutet,
wobei Benzyl, 2-Pyridylmethyl und 3-Pyridylmethyl substituiert sein können mit 1 oder 2 Substituenten unabhängig voneinander ausgewählt aus der Gruppe bestehend aus Halogen, Trifluormethyl und Methyl,
R² 2-Methylprop-1-yl, 2,2-Dimethylprop-1-yl, 2,2-Dimethylbut-1-yl, Trimethylsilylmethyl, Benzyl, 2-Pyridylmethyl oder 3-Pyridylmethyl bedeutet,
wobei Benzyl, 2-Pyridylmethyl und 3-Pyridylmethyl substituiert sein können mit 1 oder 2 Substituenten unabhängig voneinander ausgewählt aus der Gruppe bestehend aus Halogen, Trifluormethyl und Methyl,
R³ C₁-C₄-Alkyl, Phenyl, Benzyl, 2-Pyridyl, 3-Pyridyl oder 4-Pyridyl bedeutet,
wobei Phenyl, Benzyl, 2-Pyridyl, 3-Pyridyl und 4-Pyridyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
R⁴ C₁-C₆-Alkyl, C₃-C₇-Cycloalkylmethyl, Benzyl, 5- oder 6-gliedriges Heteroarylmethyl oder Trimethylsilylmethyl bedeutet,
wobei Alkyl substituiert sein kann mit einem Substituenten Hydroxy,
und
wobei Cycloalkylmethyl, Benzyl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁵ C₁-C₆-Alkyl, C₃-C₇-Cycloalkylmethyl, Phenyl, Benzyl, 5- oder 6-gliedriges Heteroarylmethyl oder Trimethylsilylmethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]-amino,
und
wobei Cycloalkylmethyl, Phenyl, Benzyl und Heteroarylmethyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
R⁶ C₁-C₆-Alkyl bedeutet,
wobei Alkyl substituiert ist mit einem Substituenten ausgewählt aus der Gruppe bestehend aus Amino, 1,4,5,6-Tetrahydropyrimidin-2-ylamino, [Amino(imino)-methyl]amino, 2-Pyridyl, 3-Pyridyl und 4-Pyridyl,
R⁷ C₁-C₆-Alkyl, C₃-C₇-Cycloalkylmethyl, Phenyl, Benzyl, 5- oder 6-gliedriges Heteroarylmethyl oder Trimethylsilylmethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [A-mino(imino)methyl]-amino,
und
wobei Cycloalkylmethyl, Phenyl, Benzyl und Heteroarylmethyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
R⁸ C₁-C₆-Alkyl, C₃-C₇-Cycloalkylmethyl, Benzyl, 5- oder 6-gliedriges Heteroarylmethyl oder Trimethylsilylmethyl bedeutet,
wobei Alkyl substituiert sein kann mit einem Substituenten Hydroxy,
und
wobei Cycloalkylmethyl, Benzyl und Heteroarylmethyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy, C₁-C₄-Alkylamino, Hydroxycarbonyl, C₁-C₄-Alkoxycarbonyl, Aminocarbonyl und C₁-C₄-Alkylaminocarbonyl,
R⁹ Wasserstoff, C₁-C₆-Alkyl, C₃-C₇-Cycloalkyl, Phenyl, Trimethylsilylmethyl oder 2-Amino-2-oxoethyl bedeutet,
wobei Methyl substituiert sein kann mit einem Substituenten ausgewählt aus der Gruppe bestehend aus Hydroxy, Amino und Mercapto,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino,
R¹⁰ Wasserstoff, C₁-C₆-Alkyl, C₃-C₇-Cycloalkyl, Phenyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino
R¹¹ Methyl oder Ethyl bedeutet,
wobei Methyl und Ethyl substituiert sein können mit einem Substituenten ausgewählt aus der Gruppe bestehend aus Hydroxy, Amino, Mercapto, 1,4,5,6-Tetrahydropyrimidin-2-ylamino und [Amino(imino)methyl]amino,
oder eines ihrer Salze, ihrer Solvate oder der Solvate ihrer Salze.

4. Verbindung nach Anspruch 3, **dadurch gekennzeichnet, dass**
R¹ 2-Methylprop-1-yl, 2,2-Dimethylprop-1-yl, 2,2-Dimethylbut-1-yl, Trimethylsilylmethyl oder Benzyl bedeutet,
wobei Benzyl substituiert sein kann mit 1 oder 2 Substituenten unabhängig voneinander ausgewählt aus der Gruppe bestehend aus Halogen, Trifluormethyl und Methyl,
oder eines ihrer Salze, ihrer Solvate oder der Solvate ihrer Salze.

5. Verbindung nach Anspruch 3 oder 4, **dadurch gekennzeichnet, dass**
R³ Phenyl, Benzyl, 2-Pyridyl, 3-Pyridyl oder 4-Pyridyl bedeutet,
wobei Phenyl, Benzyl, 2-Pyridyl, 3-Pyridyl und 4-Pyridyl substituiert sein können mit 1 bis 4 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Alkylamino,
oder eines ihrer Salze, ihrer Solvate oder der Solvate ihrer Salze.

6. Verbindung nach einem der Ansprüche 3 bis 5, **dadurch gekennzeichnet, dass**
R¹⁰ C₁-C₆-Alkyl, C₃-C₇-Cycloalkyl, Phenyl, Trimethylsilylmethyl, 2-Amino-2-oxoethyl, 2-Amino-1-hydroxy-2-oxoethyl, (Aminosulfonyl)(hydroxy)methyl, 2-(C₁-C₄-Alkylamino)-2-oxoethyl oder 2-(C₁-C₄-Alkylamino)-1-hydroxy-2-oxoethyl bedeutet,
wobei Alkyl substituiert sein kann mit einem Substituenten ausgewählt aus der Gruppe bestehend aus Halogen, Hydroxy, Amino, Mercapto, 1,4,5,6-Tetrahydropyrimidin-2-ylamino und [Amino(imino)methyl]amino,
und
wobei Cycloalkyl und Phenyl substituiert sein können mit 1 bis 4 Substituenten unabhängig voneinander ausgewählt aus der Gruppe bestehend aus Halogen, Cyano, Hydroxy, Amino, Trifluormethyl, Trifluormethoxy, C₁-C₄-Alkyl, C₁-C₄-Alkoxy und C₁-C₄-Alkylamino,
oder eines ihrer Salze, ihrer Solvate oder der Solvate ihrer Salze.

7. Verbindung nach einem der Ansprüche 1 bis 6, **dadurch gekennzeichnet, dass**
R¹ 2,2-Dimethylprop-1-yl, 2,2-Dimethylbut-1-yl, Trimethylsilylmethyl oder 3-Pyridylmethyl bedeutet,
R² 2,2-Dimethylprop-1-yl, 2,2-Dimethylbut-1-yl, Trimethylsilylmethyl oder 3-Pyridylmethyl bedeutet,
R³ C₁-C₄-Alkyl, 3-Pyridyl oder Phenyl bedeutet,
wobei 3-Pyridyl oder Phenyl substituiert sein können mit einem Substituenten ausgewählt aus der Gruppe bestehend aus Halogen, Cyano, Methyl, Methoxy, Dimethylamino und Diethylamino,
R⁴ -CH(OH)-C₁-C₅-Alkyl oder -CH(OH)Phenyl bedeutet,
wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino,
R⁵ C₁-C₆-Alkyl, C₃-C₇-Cycloalkylmethyl, Benzyl oder Trimethylsilylmethyl bedeutet,
R⁶ linerares C₂-C₄-Alkyl bedeutet,
wobei Alkyl substituiert ist mit einem Substituenten ausgewählt aus der Gruppe bestehend aus Amino, 1,4,5,6-Tetrahydropyrimidin-2-ylamino und [Amino(imino)-methyl]amino,
R⁷ C₁-C₆-Alkyl, C₃-C₇-Cycloalkylmethyl, Benzyl oder Trimethylsilylmethyl bedeutet,
R⁸ C₁-C₄-Alkyl, -CH₂-OH, -CH(OH)-C₁-C₅-Alkyl oder -CH(OH)Phenyl bedeutet,
wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino, und
R⁹ Wasserstoff, C₁-C₆-Alkyl, Hydroxymethyl oder 2-Amino-2-oxoethyl bedeutet,
R¹⁰ Wasserstoff, C₁-C₄-Alkyl, 2-Amino-2-oxoethyl oder 2-Amino-1-hydroxy-2-oxoethyl bedeutet,
wobei C₁-C₄-Alkyl substituiert sein kann mit einem Substituenten ausgewählt aus der Gruppe bestehend aus Amino und Hydroxy, mit der Ausnahme, dass R¹⁰ nicht 4-Aminobut-1-yl bedeutet,
R¹¹ Methyl bedeutet,
wobei Methyl substituiert ist mit einem Substituenten ausgewählt aus der Gruppe bestehend aus Hydroxy und Amino,
oder eines ihrer Salze, ihrer Solvate oder der Solvate ihrer Salze.

8. Verbindung nach Anspruch 7, **dadurch gekennzeichnet, dass**
R¹ 2,2-Dimethylprop-1-yl, 2,2-Dimethylbut-1-yl oder Trimethylsilylmethyl bedeutet,
oder eines ihrer Salze, ihrer Solvate oder der Solvate ihrer Salze.

9. Verbindung nach Anspruch 7 oder 8, **dadurch gekennzeichnet, dass**
R² 2,2-Dimethylprop-1-yl oder 3-Pyridylmethyl bedeutet,
oder eines ihrer Salze, ihrer Solvate oder der Solvate ihrer Salze.

10. Verbindung nach einem der Ansprüche 7 bis 9, **dadurch gekennzeichnet, dass**
R³ Phenyl bedeutet,
wobei Phenyl substituiert sein kann mit einem Substituenten ausgewählt aus der Gruppe bestehend aus Halogen, Cyano, Methyl, Methoxy, Dimethylamino und Diethylamino,
oder eines ihrer Salze, ihrer Solvate oder der Solvate ihrer Salze.

11. Verbindung nach einem der Ansprüche 7 bis 10, **dadurch gekennzeichnet, dass**
R⁸ -CH(OH)-C₁-C₅-Alkyl oder -CH(OH)Phenyl bedeutet,
wobei -CH(OH)Phenyl substituiert sein kann mit 1 bis 3 Substituenten unabhängig voneinander ausgewählt aus der Gruppe bestehend aus Halogen, Cyano, Trifluormethyl, Trifluormethoxy, C₁-C₄-Alkyl, C₁-C₄-Alkoxy und C₁-C₄-Dialkylamino, und
oder eines ihrer Salze, ihrer Solvate oder der Solvate ihrer Salze.

12. Verbindung nach einem der Ansprüche 7 bis 11, **dadurch gekennzeichnet, dass**
R¹⁰ Methyl, Ethyl, 2-Amino-2-oxoethyl oder 2-Amino-1-hydroxy-2-oxo-ethyl bedeutet,
wobei Methyl und Ethyl substituiert sein können mit einem Substituenten Hydroxy,
oder eines ihrer Salze, ihrer Solvate oder der Solvate ihrer Salze.

13. Verfahren zur Herstellung einer Verbindung der Formel (I) nach Anspruch 1, **dadurch gekennzeichnet, dass**
[A] eine Verbindung der Formel 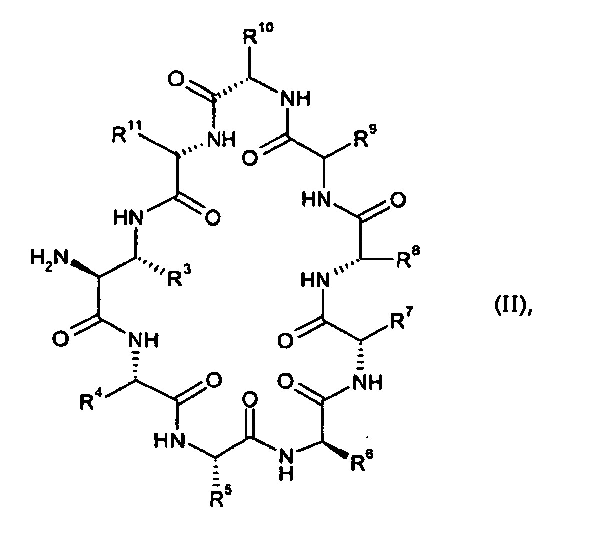 in welcher
R³, R⁴, R⁵, R⁶, R⁷, R⁸, R⁹, R¹⁰ und R¹¹ die in Anspruch 1 angegebene Bedeutung haben,
zunächst mit einer Verbindung der Formel 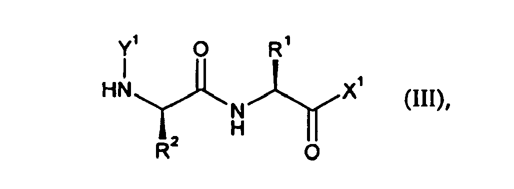 in welcher
R¹ und R² die in Anspruch 1 angegebene Bedeutung haben,
Y¹ tert-Butoxycarbonyl oder Benzyloxycarbonyl bedeutet, und
X¹ Halogen, bevorzugt Brom, Chlor oder Fluor, oder Hydroxy bedeutet,
und anschließend mit einer Säure und/oder durch Hydrogenolyse umgesetzt wird,
oder
[B] eine Verbindung der Formel 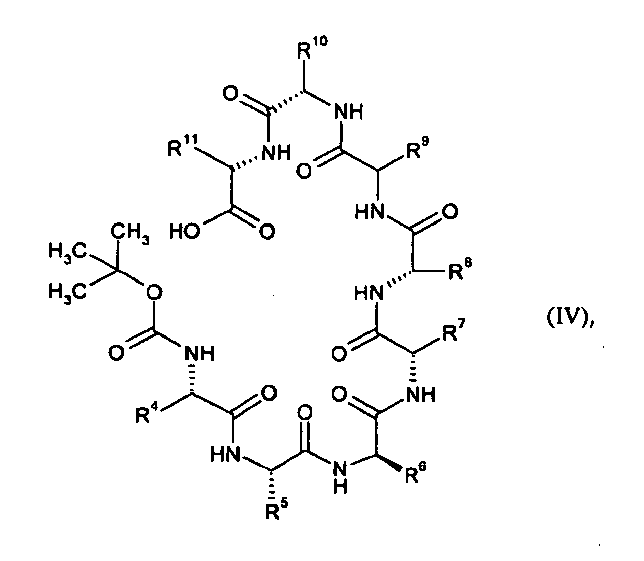 in welcher
R¹ und R² die in Anspruch 1 angegebene Bedeutung haben, 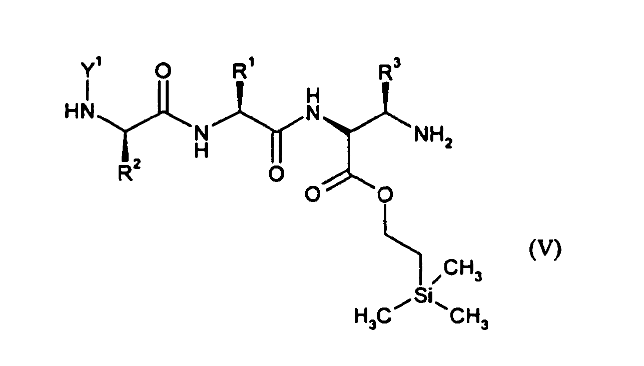
in welcher
R¹, R² und R³ die in Anspruch 1 angegebene Bedeutung haben, und
Y¹ *tert*-Butoxycarbonyl oder Benzyloxycarbonyl bedeutet,
und anschließend in einer 4-stufigen Synthese
a) mit einem Fluorid-Reagenz wie Tetrabutylammoniumfluorid,
b) mit einer Säure,
c) mit einem Dehydratisierungsreagenz, gegebenenfalls in Gegenwart einer Base,
und
d) durch Hydrogenolyse
umgesetzt wird.

14. Verfahren nach Anspruch 13, **dadurch gekennzeichnet, dass** die Verbindung der Formel (II) hergestellt wird durch Umsetzung einer Verbindung der Formel 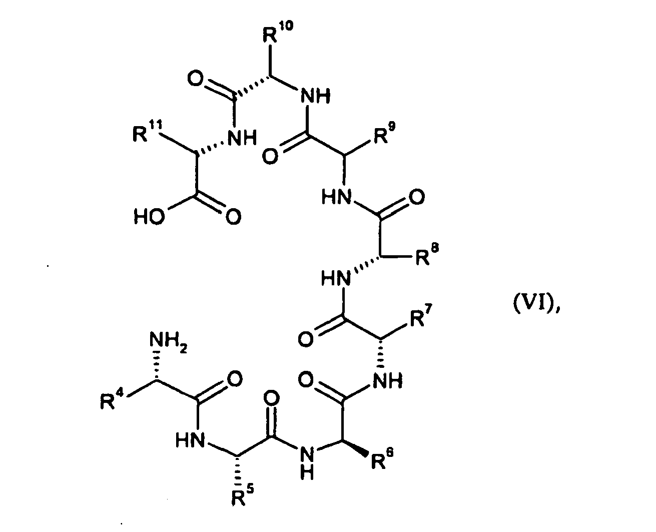 in welcher
R⁴, R⁵, R⁶, R⁷, R⁸, R⁹, R¹⁰ und R¹¹ die in Anspruch 1 angegebene Bedeutung haben,
zunächst mit einer Verbindung der Formel 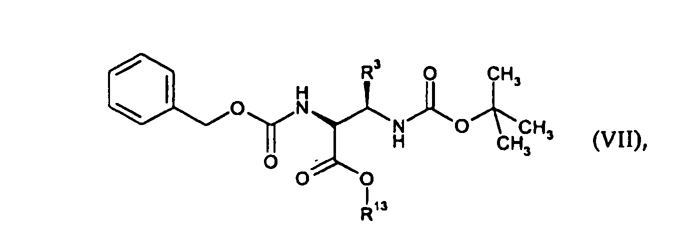 in welcher
R³ die in Anspruch 1 angegebene Bedeutung hat, und
R¹³ Cyanomethyl, p-Nitrophenyl, o-Nitrophenyl, 2,4-Dinitrophenyl, 2,4,5-Trichlorphenyl, Pentachlorphenyl, Pentafluorphenyl (Pfp), N-Hydroxyphtalimidyl, N-Hydroxy-succinimidyl (O-Su), 1-Hydroxypiperidinyl oder 5-Chlor-8-hydroxychinolinyl bedeutet,
und anschließend in einer 3-stufigen Synthese
a) mit einer Säure,
b) mit einem Dehydratisierungsreagenz, gegebenenfalls in Gegenwart einer Base,
und
c) durch Hydrogenolyse.

15. Verbindung nach einem der Ansprüche 1 bis 12 zur Verwendung in einem Verfahren zur Behandlung und/oder Prophylaxe von Krankheiten.

16. Verwendung einer Verbindung nach einem der Ansprüche 1 bis 12 zur Herstellung eines Arzneimittels zur Behandlung und/oder Prophylaxe von Krankheiten.

17. Verwendung einer Verbindung nach einem der Ansprüche 1 bis 12 zur Herstellung eines Arzneimittels zur Behandlung und/oder Prophylaxe von bakteriellen Infektionen.

18. Arzneimittel enthaltend eine Verbindung nach einem der Ansprüche 1 bis 12 in Kombination mit einem inerten, nichttoxischen, pharmazeutisch geeigneten Hilfsstoff.

19. Arzneimittel nach Anspruch 18 zur Behandlung und/oder Prophylaxe von bakteriellen Infektionen.

20. Eine antibakteriell wirksame Menge mindestens einer Verbindung nach einem der Ansprüche 1 bis 12, eines Arzneimittels nach Anspruch 18 oder eines nach Anspruch 16 oder 17 erhaltenen Arzneimittels zur Verwendung in einem Verfahren zur Bekämpfung von bakteriellen Infektionen in Menschen und Tieren.

## Claims

1. Compound of formula 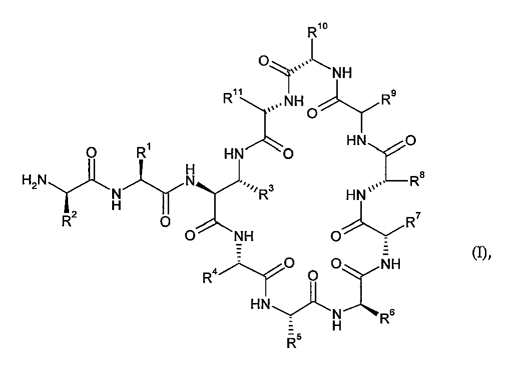 in which
R¹ represents 2-methylprop-1-yl, 2,2-dimethylprop-1-yl, 2,2-dimethylbut-1-yl, trimethylsilylmethyl, benzyl, 2-pyridylmethyl, 3-pyridylmethyl, 2-thienylmethyl, 3-thienylmethyl or 1,3-thiazol-4-ylmethyl,
whereby benzyl, 2-pyridylmethyl, 3-pyridylmethyl, 2-thienylmethyl, 3-thienylmethyl and 1,3-thiazol-4-ylmethyl may be substituted with 1 or 2 substituents selected independently of one another from the group consisting of halogen, trifluoromethyl, methyl and methoxy,
R² represents 2-methylprop-1-yl, 2,2-dimethylprop-1-yl, 2,2-dimethylbut-1-yl, trimethylsilylmethyl, benzyl, 2-pyridylmethyl, 3-pyridylmethyl, 2-thienylmethyl, 3-thienylmethyl or 1,3-thiazol-4-ylmethyl,
whereby benzyl, 2-pyridylmethyl, 3-pyridylmethyl, 2-thienylmethyl, 3-thienylmethyl and 1,3-thiazol-4-ylmethyl may be substituted with 1 or 2 substituents selected independently of one another from the group consisting of halogen, trifluoromethyl, methyl and methoxy,
R³ represents hydrogen, C₁-C₄-alkyl, C₃-C₇-cycloalkyl, C₃-C₇-cycloalkylmethyl, phenyl, benzyl, 5- or 6-membered heteroaryl or 5- or 6-membered heteroarylmethyl,
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl, C₁-C₄-alkylaminocarbonyl and 5- or 6-membered heterocyclyl which is bonded via nitrogen,
R⁴ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl or 2-amino-2-oxoethyl,
whereby alkyl may be substituted with 1 to 3 substituents selected independently of one another from the group consisting of halogen, hydroxy, amino, mercapto, 1,4,5,6-tetrahydropyrimidin-2-ylamino and [amino(imino)methyl]amino, and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁C₄-alkoxycarbonyl, aminocarbonyl and C₁-C₄-alkylaminocarbonyl,
R⁵ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl or 2-amino-2-oxoethyl,
whereby alkyl may be substituted with 1 to 3 substituents selected independently of one another from the group consisting of halogen, hydroxy, amino, mercapto, 1,4,5,6-tetrahydropyrimidin-2-ylamino and [amino(imino)methyl]amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁C₄-alkylaminocarbonyl,
R⁶ represents C₁-C₆-alkyl,
whereby alkyl is substituted with a substituent selected from the group consisting of amino, 1,4,5,6-tetrahydropyrimidin-2-ylamino, [amino-(imino)methyl]amino, 2-pyridyl, 3-pyridyl and 4-pyridyl,
R⁷ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl or trimethylsilylmethyl,
whereby alkyl may be substituted with 1 to 3 substituents selected independently of one another from the group consisting of halogen, hydroxy, amino, mercapto, 1,4,5,6-tetrahydropyrimidin-2-ylamino and [amino(imino)methyl]amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R⁸ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl or 2-amino-2-oxoethyl,
whereby alkyl may be substituted with 1 to 3 substituents selected independently of one another from the group consisting of halogen, hydroxy, amino, mercapto, 1,4,5,6-tetrahydropyrimidin-2-ylamino and [amino(imino)methyl]amino, and whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R⁹ represents hydrogen, C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl or 2-amino-2-oxoethyl,
whereby alkyl may be substituted with 1 to 3 substituents selected independently of one another from the group consisting of halogen, hydroxy, amino, mercapto, 1,4,5,6-tetrahydropyrimidin-2-ylamino and [amino(imino)methyl]amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R¹⁰ represents hydrogen, C₁-C₆-alkyl, C₃-C₇-cydoalkyl, C₃-C₇-cycloalkylmethyl, phenyl, benzyl, 5- or 6-membered heteroaryl, 5- or 6-membered heteroarylmethyl, trimethylsilylmethyl, 2-amino-2-oxoethyl, 2-amino-1-hydroxy-2-oxoethyl, (aminosulfonyl)(hydroxy)methyl, 2-(C₁-C₄-alkylamino)-2-oxoethyl or 2-(C₁-C₄-alkylamino)-1-hydroxy-2-oxoethyl, whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R¹¹ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl, 2-amino-2-oxoethyl, 2-amino-1-hydroxy2-oxoethyl, (aminosulfonyl)(hydroxy)methyl, 2-(C₁-C₄-alkylamino)-2-oxoethyl or 2-(C₁-C₄-alkylamino)-1-hydroxy-2-oxoethyl,
whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
or one of the salts thereof, the solvates thereof or the solvates of the salts thereof,
except a compound of formula 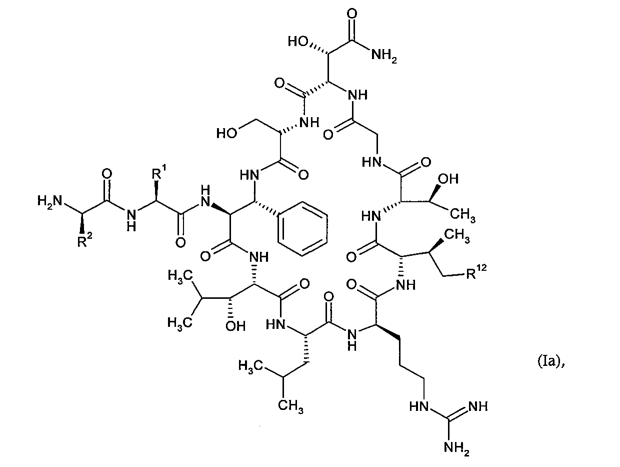 in which
R¹ represents 2-methylprop-1-yl, 2,2-dimethylprop-1-yl, 2,2-dimethylbut-1-yl, trimethylsilylmethyl, benzyl, 2-pyridylmethyl, 3-pyridylmethyl, 2-thienylmethyl, 3-thienylmethyl or 1,3-thiazol-4-ylmethyl,
whereby benzyl, 2-pyridylmethyl, 3-pyridylmethyl, 2-thienylmethyl, 3-thienylmethyl and 1,3-thiazol-4-ylmethyl may be substituted with 1 or 2 substituents selected independently of one another from the group consisting of halogen, trifluoromethyl, methyl and methoxy,
R² represents 2-methylprop-1-yl, 2,2-dimethylprop-1-yl, 2,2-dimethylbut-1-yl, trimethylsilylmethyl, benzyl, 2-pyridylmethyl, 3-pyridylmethyl, 2-thienylmethyl, 3-thienylmethyl or 1,3-thiazol-4-ylmethyl,
whereby benzyl, 2-pyridylmethyl, 3-pyridylmethyl, 2-thienylmethyl, 3-thienylmethyl and 1,3-thiazol-4-ylmethyl may be substituted with 1 or 2 substituents selected independently of one another from the group consisting of halogen, trifluoromethyl, methyl and methoxy,
and
R¹² represents hydrogen or methyl,
or one of the salts thereof, the solvates thereof or the solvates of the salts thereof.

2. Compound according to claim 1, **characterized in that**
R¹⁰ represents C₁-C₆-alkyl, C₃-C₇-cycloalkyl, C₃-C₇-cycloalkylmethyl, phenyl, benzyl, 5- or 6-membered heteroaryl, 5- or 6-membered heteroarylmethyl, trimethylsilylmethyl, 2-amino-2-oxoethyl, 2-amino-1-hydroxy-2-oxoethyl, (aminosulfonyl)(hydroxy)methyl, 2-(C₁-C₄-alkylamino)-2-oxoethyl or 2-(C₁-C₄-alkylamino)-1-hydroxy-2-oxoethyl,
whereby alkyl may be substituted with a substituent selected from the group consisting of halogen, hydroxy, amino, mercapto, 1,4,5,6-tetrahydropyrimidin-2-ylamino and [amino(imino)methyl] amino,
and
whereby cycloalkyl, cycloalkylmethyl, phenyl, benzyl, heteroaryl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
or one of the salts thereof, the solvates thereof or the solvates of the salts thereof.

3. Compound according to claim 1 or 2, **characterized in that**
R¹ represents 2-methylprop-1-yl, 2,2-dimethylprop-1-yl, 2,2-dimethylbut-1-yl, trimethylsilylmethyl, benzyl, 2-pyridylmethyl or 3-pyridylmethyl,
whereby benzyl, 2-pyridylmethyl and 3-pyridylmethyl may be substituted with 1 or 2 substituents selected independently of one another from the group consisting of halogen, trifluoromethyl and methyl,
R² represents 2-methylprop-1-yl, 2,2-dimethylprop-1-yl, 2,2-dimethylbut-1-yl, trimethylsilylmethyl, benzyl, 2-pyridylmethyl or 3-pyridylmethyl,
whereby benzyl, 2-pyridylmethyl and 3-pyridylmethyl may be substituted with 1 or 2 substituents selected independently of one another from the group consisting of halogen, trifluoromethyl and methyl,
R³ represents C₁-C₄-alkyl, phenyl, benzyl, 2-pyridyl, 3-pyridyl or 4-pyridyl,
whereby phenyl, benzyl, 2-pyridyl, 3-pyridyl and 4-pyridyl may be substituted with 1 to 4 substituents selected independently of one another from the group consisting of halogen, cyano, trifluoromethyl, trifluoromethoxy, C₁-C₄-alkyl, C₁-C₄-alkoxy and C₁-C₄-alkylamino,
R⁴ represents C₁-C₆-alkyl, C₃-C₇-cycloalkylmethyl, benzyl, 5- or 6-membered heteroarylmethyl or trimethylsilylmethyl,
whereby alkyl may be substituted with a hydroxy substituent,
and
whereby cycloalkylmethyl, benzyl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R⁵ represents C₁-C₆-alkyl, C₃-C₇-cycloalkylmethyl, phenyl, benzyl, 5- or 6-membered heteroarylmethyl or trimethylsilylmethyl,
whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkylmethyl, phenyl, benzyl and heteroarylmethyl may be substituted with 1 to 4 substituents selected independently of one another from the group consisting of halogen, cyano, trifluoromethyl, trifluoromethoxy, C₁-C₄-alkyl, C₁-C₄-alkoxy and C₁-C₄-alkylamino,
R⁶ represents C₁-C₆-alkyl,
whereby alkyl is substituted with a substituent selected from the group consisting of amino, 1,4,5,6-tetrahydropyrimidin-2-ylamino, [amino-(imino)methyl]amino, 2-pyridyl, 3-pyridyl and 4-pyridyl,
R⁷ represents C₁-C₆-alkyl, C₃-C₇-cycloalkylmethyl, phenyl, benzyl, 5- or 6-membered heteroarylmethyl or trimethylsilylmethyl,
whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkylmethyl, phenyl, benzyl and heteroarylmethyl may be substituted with 1 to 4 substituents selected independently of one another from the group consisting of halogen, cyano, trifluoromethyl, trifluoromethoxy, C₁-C₄-alkyl, C₁-C₄-alkoxy and C₁-C₄-alkylamino,
R⁸ represents C₁-C₆-alkyl, C₃-C₇-cycloalkylmethyl, benzyl, 5- or 6-membered heteroarylmethyl or trimethylsilylmethyl,
whereby alkyl may be substituted with a hydroxy substituent,
and
whereby cycloalkylmethyl, benzyl and heteroarylmethyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy, C₁-C₄-alkylamino, hydroxycarbonyl, C₁-C₄-alkoxycarbonyl, aminocarbonyl and C₁-C₄-alkylaminocarbonyl,
R⁹ represents hydrogen, C₁-C₆-alkyl, C₃-C₇-cycloalkyl, phenyl, trimethylsilylmethyl or 2-amino-2-oxoethyl,
whereby methyl may be substituted with a substituent selected from the group consisting of hydroxy, amino and mercapto,
and
whereby cycloalkyl and phenyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy and C₁-C₄-alkylamino,
R¹⁰ represents hydrogen, C₁-C₆-alkyl, C₃-C₇-cycloalkyl, phenyl, trimethyl-silylmethyl, 2-amino-2-oxoethyl, 2-amino-1-hydroxy-2-oxoethyl, (aminosulfonyl)(hydroxy)methyl, 2-(C₁-C₄-alkylamino)-2-oxoethyl or 2-(C₁-C₄-alkylamino)-1-hydroxy-2-oxoethyl,
whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkyl and phenyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy and C₁-C₄-alkylamino,
R¹¹ represents methyl or ethyl,
whereby methyl and ethyl may be substituted with a substituent selected from the group consisting of hydroxy, amino, mercapto, 1,4,5,6-tetrahydropyrimidin-2-ylamino and [amino(imino)methyl]amino,
or one of the salts thereof, the solvates thereof, or the solvates of the salts thereof.

4. Compound according to claim 3, **characterized in that**
R¹ represents 2-methylprop-1-yl, 2,2-dimethylprop-1-yl, 2,2-dimethylbut-1-yl, trimethylsilylmethyl or benzyl,
whereby benzyl may be substituted with 1 or 2 substituents selected independently of one another from the group consisting of halogen, trifluoromethyl and methyl,
or one of the salts thereof, the solvates thereof or the solvates of the salts thereof.

5. Compound according to claim 3 or 4, **characterized in that**
R³ represents phenyl, benzyl, 2-pyridyl, 3-pyridyl or 4-pyridyl,
whereby phenyl, benzyl, 2-pyridyl, 3-pyridyl and 4-pyridyl may be substituted with 1 to 4 substituents selected independently of one another from the group consisting of halogen, cyano, trifluoromethyl, trifluoromethoxy, C₁-C₄-alkyl, C₁-C₄-alkoxy and C₁-C₄-alkylamino,
or one of the salts thereof, the solvates thereof or the solvates of the salts thereof.

6. Compound according to any one of claims 3 to 5, **characterized in that**
R¹⁰ represents C₁-C₆-alkyl, C₃-C₇-cycloalkyl, phenyl, trimethylsilylmethyl, 2-amino-2-oxoethyl, 2-amino-1-hydroxy-2-oxoethyl, (aminosulfonyl)-(hydroxy)methyl, 2-(C₁-C₄-alkylamino)-2-oxoethyl or 2-(C₁-C₄-alkylamino)-1-hydroxy-2-oxoethyl, whereby alkyl may be substituted with a substituent selected from the group consisting of halogen, hydroxy, amino, mercapto, 1,4,5,6-tetrahydropyrimidin-2-ylamino and [amino(imino)methyl]amino,
and
whereby cycloalkyl and phenyl may be substituted with 1 to 4 substituents selected independently of one another from the group consisting of halogen, cyano, hydroxy, amino, trifluoromethyl, trifluoromethoxy, C₁-C₄-alkyl, C₁-C₄-alkoxy and C₁-C₄-alkylamino,
or one of the salts thereof, the solvates thereof or the solvates of the salts thereof.

7. Compound according to any one of claims 1 to 6, **characterized in that**
R¹ represents 2,2-dimethylprop-1-yl, 2,2-dimethylbut-1-yl, trimethylsilylmethyl or 3-pyridylmethyl,
R² represents 2,2-dimethylprop-1-yl, 2,2-dimethylbut-1-yl, trimethylsilylmethyl or 3-pyridylmethyl,
R³ represents C₁-C₄-alkyl, 3-pyridyl or phenyl,
whereby 3-pyridyl or phenyl may be substituted with a substituent selected from the group consisting of halogen, cyano, methyl, methoxy, dimethylamino and diethylamino,
R⁴ represents -CH(OH)-C₁-C₅-alkyl or -CH(OH)phenyl,
whereby -CH(OH)phenyl may be substituted with 1 to 3 substituents selected independently of one another from the group consisting of halogen, cyano, trifluoromethyl, trifluoromethoxy, C₁-C₄-alkyl, C₁-C₄-alkoxy and C₁-C₄-dialkylamino,
R⁵ represents C₁-C₆-alkyl, C₃-C₇-cycloalkylmethyl, benzyl or trimethylsilylmethyl,
R⁶ represents linear C₂-C₄-alkyl,
whereby alkyl is substituted with a substituent selected from the group consisting of amino, 1,4,5,6-tetrahydropyrimidin-2-ylamino and [amino(imino)methyl]amino,
R⁷ represents C₁-C₆-alkyl, C₃-C₇-cycloalkylmethyl, benzyl or trimethylsilylmethyl,
R⁸ represents C₁-C₄-alkyl, -CH₂-OH, -CH(OH)-C₁-C₅-alkyl or -CH(OH)-phenyl,
whereby -CH(OH)phenyl may be substituted with 1 to 3 substituents selected independently of one another from the group consisting of halogen, cyano, trifluoromethyl, trifluoromethoxy, C₁-C₄-alkyl, C₁-C₄-alkoxy and C₁-C₄-dialkylamino, and
R⁹ represents hydrogen, C₁-C₆-alkyl, hydroxymethyl or 2-amino-2-oxoethyl,
R¹⁰ represents hydrogen, C₁-C₄-alkyl, 2-amino-2-oxoethyl or 2-amino-1-hydroxy-2-oxoethyl,
whereby C₁-C₄-alkyl may be substituted with a substituent selected from the group consisting of amino and hydroxy, with the exception that R¹⁰ is not 4-aminobut-1-yl,
R¹¹ represents methyl,
whereby methyl is substituted with a substituent selected from the group consisting of hydroxy and amino,
or one of the salts thereof, the solvates thereof or the solvates of the salts thereof.

8. Compound according to claim 7, **characterized in that**
R¹ represents 2,2-dimethylprop-1-yl, 2,2-dimethylbut-1-yl or trimethylsilylmethyl,
or one of the salts thereof, the solvates thereof or the solvates of the salts thereof.

9. Compound according to claim 7 or 8, **characterized in that**
R² represents 2,2-dimethylprop-1-yl or 3-pyridylmethyl,
or one of the salts thereof, the solvates thereof or the solvates of the salts thereof.

10. Compound according to any one of claims 7 to 9, **characterized in that**
R³ represents phenyl,
whereby phenyl may be substituted with a substituent selected from the group consisting of halogen, cyano, methyl, methoxy, dimethylamino and diethylamino,
or one of the salts thereof, the solvates thereof or the solvates of the salts thereof.

11. Compound according to any one of claims 7 to 10, **characterized in that**
R⁸ represents -CH(OH)-C₁-C₅-alkyl or -CH(OH)phenyl,
whereby -CH(OH)phenyl may be substituted with 1 to 3 substituents selected independently of one another from the group consisting of halogen, cyano, trifluoromethyl, trifluoromethoxy, C₁-C₄-alkyl, C₁-C₄-alkoxy and C₁-C₄-dialkylamino, and
or one of the salts thereof, the solvates thereof or the solvates of the salts thereof.

12. Compound according to any one of claims 7 to 11, **characterized in that**
R¹⁰ represents methyl, ethyl, 2-amino-2-oxoethyl or 2-amino-1-hydroxy-2-oxoethyl,
whereby methyl and ethyl may be substituted with a hydroxy substituent,
or one of the salts thereof, the solvates thereof or the solvates of the salts thereof.

13. Method for preparing a compound of formula (I) according to claim 1, **characterized in that**
[A] a compound of formula 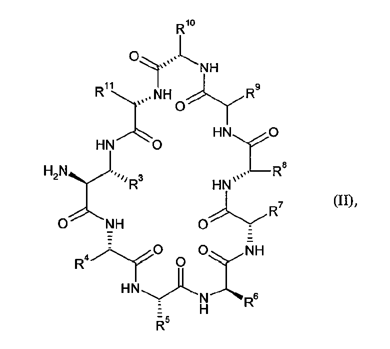 in which
R³, R⁴, R⁵, R⁶, R⁷, R⁸, R⁹, R¹⁰ and R¹¹ have the meaning indicated in claim 1,
is first reacted with a compound of formula 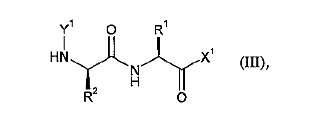 in which
R¹ and R² have the meaning indicated in claim 1,
Y¹ represents *tert*-butoxycarbonyl or benzyloxycarbonyl, and
X¹ represents halogen, preferably bromine, chlorine or fluorine, or hydroxy,
and subsequently with an acid and/or by hydrogenolysis,
or
[B] a compound of formula 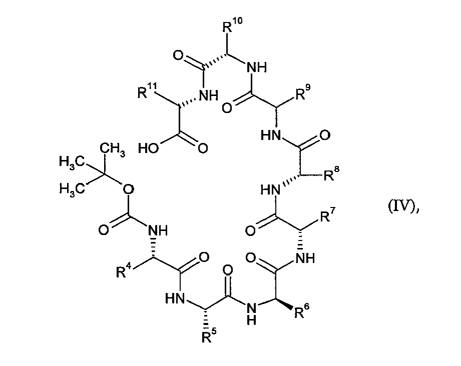 in which
R⁴, R⁵, R⁶, R⁷, R⁸, R⁹, R¹⁰ and R¹¹ have the meaning indicated in claim 1,
is first reacted with a compound of formula 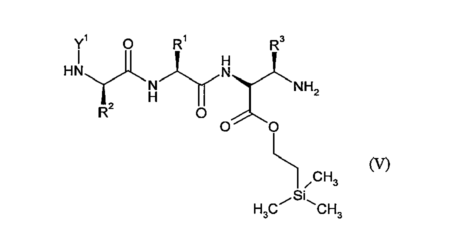 in which
R¹, R² and R³ have the meaning indicated in claim 1, and
Y¹ represents *ter*t-butoxycarbonyl or benzyloxycarbonyl,
and subsequently in a 4-stage synthesis
a) with a fluoride reagent such as tetrabutylammonium fluoride,
b) with an add,
c) with a dehydrating reagent, where appropriate in the presence of a base,
and
d) by hydrogenolysis.

14. Method according to claim 13, **characterized in that** the compound of formula (II) is prepared by reacting a compound of formula 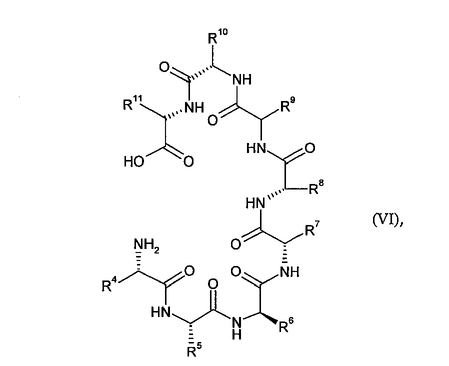 in which
R⁴, R⁵, R⁶, R⁷, R⁸, R⁹, R¹⁰ and R¹¹ have the meaning indicated in claim 1,
first with a compound of formula 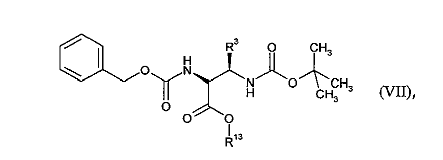 in which
R³ has the meaning indicated in claim 1, and
R¹³ represents cyanomethyl, p-nitrophenyl, o-nitrophenyl, 2,4-dinitrophenyl, 2,4,5-trichlorophenyl, pentachlorophenyl, pentafluorophenyl (Pfp), N-hydroxyphthalimidyl, N-hydroxysuccinimidyl (O-Su), 1-hydroxypiperidinyl or 5-chloro-8-hydroxyquinolinyl,
and subsequently in a 3-stage synthesis
a) with an acid,
b) with a dehydrating reagent, where appropriate in the presence of a base,
and
c) by hydrogenolysis.

15. Compound according to any one of claims 1 to 12 for use in a method of treating and/or preventing diseases.

16. Use of a compound according to any one of claims 1 to 12 for the manufacture of a medicament for the treatment and/or prophylaxis of diseases.

17. Use of a compound according to any one of claims 1 to 12 for the manufacture of a medicament for the treatment and/or prophylaxis of bacterial infections.

18. Medicament comprising a compound according to any one of claims 1 to 12 in combination with an inert, nontoxic, pharmaceutically acceptable excipient.

19. Medicament according to claim 18 for the treatment and/or prophylaxis of bacterial infections.

20. An antibacterially effective amount of at least one compound according to any one of claims 1 to 12, of a medicament according to claim 18 or of a medicament obtained according to claim 16 or 17 for use in a method for controlling bacterial infections in humans and animals.

## Revendications

1. Composé de formule 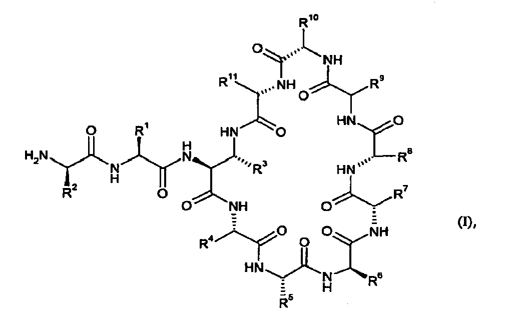 dans laquelle
R¹ représente un radical 2-méthylprop-1-yle, 2,2-diméthylprop-1-yle, 2,2-diméthylbut-1-yle, triméthylsilylméthyle, benzyle, 2-pyridylméthyle, 3-pyridylméthyle, 2-thiénylméthyle, 3-thiénylméthyle ou 1,3-thiazol-4-ylméthyle,
où les radicaux benzyle, 2-pyridylméthyle, 3-pyridylméthyle, 2-thiénylméthyle, 3-thiénylméthyle et 1,3-thiazol-4-ylméthyle peuvent être substitués par 1 ou 2 substituants choisis, indépendamment les uns des autres, dans le groupe constitué par un atome d'halogène, un groupe trifluorométhyle, méthyle et méthoxy,
R² représente un radical 2-méthylprop-1-yle, 2,2-diméthylprop-1-yle, 2,2-diméthylbut-1-yle, triméthylsilylméthyle, benzyle, 2-pyridylméthyle, 3-pyridylméthyle, 2-thiénylméthyle, 3-thiénylméthyle ou 1,3-thiazol-4-ylméthyle,
où les radicaux benzyle, 2-pyridylméthyle, 3-pyridylméthyle, 2-thiénylméthyle, 3-thiénylméthyle et 1,3-thiazol-4-ylméthyle peuvent être substitués par 1 ou 2 substituants choisis, indépendamment les uns des autres, dans le groupe constitué par un atome d'halogène, un groupe trifluorométhyle, méthyle et méthoxy,
R³ représente un atome d'hydrogène, un groupe alkyle en C₁-C₄, cycloalkyle en C₃-C₇, cycloalkyl(C₃-C₇)méthyle, phényle, benzyle, hétéroaryle de 5 ou 6 membres ou hétéroarylméthyle de 5 ou 6 membres,
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle, alkyl(C₁-C₄)aminocarbonyle, hétérocyclyle de 5 ou 6 membres reliés par un atome d'azote,
R⁴ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle ou 2-amino-2-oxoéthyle,
où le radical alkyle peut être substitué par 1 à 3 substituants choisis, indépendamment les uns des autres, dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁵ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle ou 2-amino-2-oxoéthyle,
où le radical alkyle peut être substitué par 1 à 3 substituants choisis, indépendamment les uns des autres, dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁶ représente un groupe alkyle en C₁-C₆,
où le radical alkyle est substitué par un substituant choisi dans le groupe constitué par un groupe amino, 1,4,5,6-tétrahydropyrimidin-2-ylamino, [amino(imino)méthyl]-amino, 2-pyridyle, 3-pyridyle et 4-pyridyle,
R⁷ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres ou triméthylsilylméthyle,
où le radical alkyle peut être substitué par 1 à 3 substituants choisis, indépendamment les uns des autres, dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁸ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle ou 2-amino-2-oxoéthyle,
où le radical alkyle peut être substitué par 1 à 3 substituants choisis, indépendamment les uns des autres, dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁹ représente un atome d'hydrogène, un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle ou 2-amino-2-oxoéthyle,
où le radical alkyle peut être substitué par 1 à 3 substituants choisis, indépendamment les uns des autres, dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R¹⁰ représente un atome d'hydrogène, un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle, 2-amino-2-oxoéthyle, 2-amino-1-hydroxy-2-oxoéthyle, (aminosulfonyl)(hydroxy)méthyle, 2-(alkyl(C₁-C₄)amino)-2-oxoéthyle ou 2-(alkyl(C₁-C₄)amino)-1-hydroxy-2-oxoéthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R¹¹ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle, 2-amino-2-oxoéthyle, 2-amino-1-hydroxy-2-oxoéthyle, (aminosulfonyl)(hydroxy)-méthyle, 2-(alkyl(C₁-C₄)amino)-2-oxoéthyle ou 2-(alkyl(C₁-C₄)amino)-1-hydroxy-2-oxoéthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
ou un de ses sels, un de ses solvates ou solvates de ses sels, excepté un composé de formule 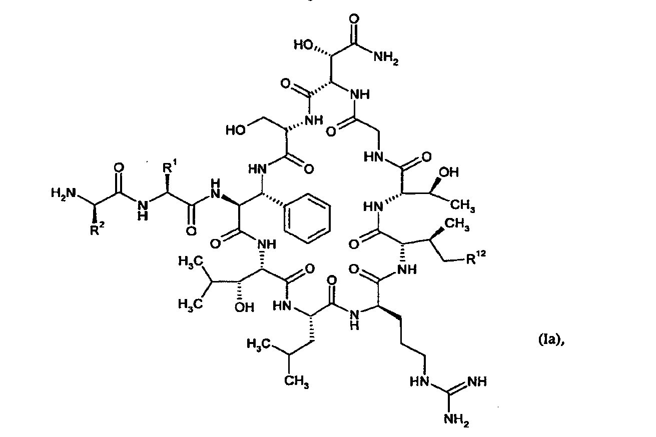 dans laquelle
R¹ représente un radical 2-méthylprop-1-yle, 2,2-diméthylprop-1-yle, 2,2-diméthylbut-1-yle, triméthylsilylméthyle, benzyle, 2-pyridylméthyle, 3-pyridylméthyle, 2-thiénylméthyle, 3-thiénylméthyle ou 1,3-thiazol-4-ylméthyle,
où les radicaux benzyle, 2-pyridylméthyle, 3-pyridylméthyle, 2-thiénylméthyle, 3-thiénylméthyle et 1,3-thiazol-4-ylméthyle peuvent être substitués par 1 ou 2 substituants choisis, indépendamment les uns des autres, dans le groupe constitué par un atome d'halogène, un groupe trifluorométhyle, méthyle et méthoxy,
R² représente un radical 2-méthylprop-1-yle, 2,2-diméthylprop-1-yle, 2,2-diméthylbut-1-yle, triméthylsilylméthyle, benzyle, 2-pyridylméthyle, 3-pyridylméthyle, 2-thiénylméthyle, 3-thiénylméthyle ou 1,3-thiazol-4-ylméthyle,
où les radicaux benzyle, 2-pyridylméthyle, 3-pyridylméthyle, 2-thiénylméthyle, 3-thiénylméthyle et 1,3-thiazol-4-ylméthyle peuvent être substitués par 1 ou 2 substituants choisis, indépendamment les uns des autres, dans le groupe constitué par un atome d'halogène, un groupe trifluorométhyle, méthyle et méthoxy,
et
R¹² représente un atome d'hydrogène ou un groupe méthyle,
ou un de ses sels, un de ses solvates ou les solvates de ses sels.

2. Composé selon la revendication 1, **caractérisé en ce que**
R¹⁰ représente un groupe alkyle en C₁-C₆, cycloalkyle en C₃-C₇, cycloalkyl(C₃-C₇)méthyle, phényle, benzyle, hétéroaryle de 5 ou 6 membres, hétéroarylméthyle de 5 ou 6 membres, triméthylsilylméthyle, 2-amino-2-oxoéthyle, 2-amino-1-hydroxy-2-oxoéthyle, (aminosulfonyl)(hydroxy)-méthyle, 2-(alkyl(C₁-C₄)amino)-2-oxoéthyle ou 2-(alkyl(C₁-C₄)amino)-1-hydroxy-2-oxoéthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle, cycloalkylméthyle, phényle, benzyle, hétéroaryle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
ou un de ses sels, un de ses solvates ou solvates de ses sels.

3. Composé selon la revendication 1 ou 2, **caractérisé en ce que**
R¹ représente un radical 2-méthylprop-1-yle, 2,2-diméthylprop-1-yle, 2,2-diméthylbut-1-yle, triméthylsilylméthyle, benzyle, 2-pyridylméthyle ou 3-pyridylméthyle,
où les radicaux benzyle, 2-pyridylméthyle et 3-pyridylméthyle peuvent être substitués par 1 ou 2 substituants choisis, indépendamment les uns des autres, dans le groupe constitué par un atome d'halogène, un groupe trifluorométhyle et méthyle,
R² représente un radical 2-méthylprop-1-yle, 2,2-diméthylprop-1-yle, 2,2-diméthylbut-1-yle, triméthylsilylméthyle, benzyle, 2-pyridylméthyle ou 3-pyridylméthyle,
où les radicaux benzyle, 2-pyridylméthyle et 3-pyridylméthyle peuvent être substitués par 1 ou 2 substituants choisis, indépendamment les uns des autres, dans le groupe constitué par un atome d'halogène, un groupe trifluorométhyle et méthyle,
R³ représente un groupe alkyle en C₁-C₄, phényle, benzyle, 2-pyridyle, 3-pyridyle ou 4-pyridyle,
où les radicaux phényle, benzyle, 2-pyridyle, 3-pyridyle et 4-pyridyle peuvent être substitués par 1 à 4 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et alkyl(C₁-C₄)amino,
R⁴ représente un groupe alkyle en C₁-C₆, cycloalkyl-(C₃-C₇)méthyle, benzyle, hétéroarylméthyle de 5 ou 6 membres ou triméthylsilylméthyle,
où le radical alkyle peut être substitué par un substituant hydroxy,
et
où les radicaux cycloalkylméthyle, benzyle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁵ représente un groupe alkyle en C₁-C₆, cycloalkyl-(C₃-C₇)méthyle, phényle, benzyle, hétéroarylméthyle de 5 ou 6 membres ou triméthylsilylméthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkylméthyle, phényle, benzyle et hétéroarylméthyle peuvent être substitués par 1 à 4 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄, alkyl(C₁-C₄)amino,
R⁶ représente un groupe alkyle en C₁-C₆,
où le radical alkyle est substitué par un substituant choisi dans le groupe constitué par un groupe amino, 1,4,5,6-tétrahydropyrimidin-2-ylamino, [amino(imino)méthyl]-amino, 2-pyridyle, 3-pyridyle et 4-pyridyle,
R⁷ représente un groupe alkyle en C₁-C₆, cycloalkyl-(C₃-C₇)méthyle, phényle, benzyle, hétéroarylméthyle de 5 ou 6 membres ou triméthylsilylméthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et les radicaux cycloalkylméthyle, phényle, benzoyle et où les radicaux phényle, benzyle et hétéroarylméthyle peuvent être substitués par 1 à 4 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et alkyl(C₁-C₄)amino,
R⁸ représente un alkyle en C₁-C₆, cycloalkyl-(C₃-C₇)méthyle, benzyle, hétéroarylméthyle de 5 ou 6 membres ou triméthylsilylméthyle,
où le radical alkyle peut être substitué par un substituant hydroxy,
et
où les radicaux cycloalkylméthyle, benzyle et hétéroarylméthyle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄, alkyl(C₁-C₄)amino, hydroxycarbonyle, alcoxy(C₁-C₄)carbonyle, aminocarbonyle et alkyl(C₁-C₄)aminocarbonyle,
R⁹ représente un atome d'hydrogène, un groupe alkyle en C₁-C₆, cycloalkyle en C₃-C₇, phényle, triméthylsilylméthyle ou 2-amino-2-oxoéthyle,
où le radical méthyle peut être substitué par un substituant choisi dans le groupe constitué par un groupe hydroxy, amino et mercapto,
et
où les radicaux cycloalkyle et phényle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄ et alkyl(C₁-C₄)amino,
R¹⁰ représente un atome d'hydrogène, un groupe alkyle en C₁-C₆, cycloalkyle en C₃-C₇, phényle, triméthylsilylméthyle, 2-amino-2-oxoéthyle, 2-amino-1-hydroxy-2-oxoéthyle, (aminosulfonyl)(hydroxy)méthyle, 2-(alkyl(C₁-C₄)amino)-2-oxoéthyle ou 2-(alkyl(C₁-C₄)amino)-1-hydroxy-2-oxoéthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle et phényle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄ et alkyl(C₁-C₄)amino,
R¹¹ représente un groupe méthyle ou éthyle,
où les radicaux méthyle et éthyle peuvent être substitués par un substituant choisi dans le groupe constitué par un groupe hydroxy, amino, mercapto, 1,4,5,6-tétrahydropyrimidin-2-ylamino et [amino(imino)méthyl]-amino,
ou un de ses sels, un de ses solvates ou solvates de ses sels.

4. Composé selon la revendication 3, **caractérisé en ce que**
R¹ représente un radical 2-méthylprop-1-yle, 2,2-diméthylprop-1-yle, 2,2-diméthylbut-1-yle, triméthylsilylméthyle ou benzyle,
où le radical benzyle peut être substitué par 1 ou 2 substituants choisis, indépendamment les uns des autres, dans le groupe constitué par un atome d'halogène, un groupe trifluorométhyle et méthyle,
ou un de ses sels, un de ses solvates ou solvates de ses sels.

5. Composé selon la revendication 3 ou 4, **caractérisé en ce que**
R³ représente un groupe phényle, benzyle, 2-pyridyle, 3-pyridyle ou 4-pyridyle,
où les radicaux phényle, benzyle, 2-pyridyle, 3-pyridyle et 4-pyridyle peuvent être substitués par 1 à 4 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et alkyl(C₁-C₄)amino,
ou un de ses sels, un de ses solvates ou solvates de ses sels.

6. Composé selon l'une des revendications 3 à 5, **caractérisé en ce que**
R¹⁰ représente un groupe alkyle en C₁-C₆, cycloalkyle en C₃-C₇, phényle, triméthylsilylméthyle, 2-amino-2-oxoéthyle, 2-amino-1-hydroxy-2-oxoéthyle, (aminosulfonyl)(hydroxy)méthyle, 2-(alkyl(C₁-C₄)amino)-2-oxoéthyle ou 2-(alkyl(C₁-C₄)amino)-1-hydroxy-2-oxoéthyle,
où le radical alkyle peut être substitué par un substituant choisi dans le groupe constitué par un atome d'halogène, un groupe hydroxy, amino, mercapto, 1,4,5,6-tétrahydropyrimidin-2-ylamino et [amino(imino)méthyl]-amino,
et
où les radicaux cycloalkyle et phényle peuvent être substitués par 1 à 4 substituants choisis, indépendamment les uns des autres, dans le groupe constitué par un atome d'halogène, un groupe cyano, hydroxy, amino, trifluorométhyle, trifluorométhoxy, alkyle en C₁-C₄, alcoxy en C₁-C₄ et alkyl(C₁-C₄)amino,
ou un de ses sels, un de ses solvates ou solvates de ses sels.

7. Composé selon l'une des revendications 1 à 6, **caractérisé en ce que**
R¹ représente un radical 2,2-diméthylprop-1-yle, 2,2-diméthylbut-1-yle, triméthylsilylméthyle ou 3-pyridylméthyle,
R² représente un radical 2,2-diméthylprop-1-yle, 2,2-diméthylbut-1-yle, triméthylsilylméthyle ou 3-pyridylméthyle,
R³ représente un groupe alkyle en C₁-C₄, 3-pyridyle ou phényle,
où les radicaux 3-pyridyle ou phényle peuvent être substitués par un substituant choisi dans le groupe constitué par un atome d'halogène, un groupe cyano, méthyle, méthoxy, diméthylamino et diéthylamino,
R⁴ représente un groupe -CH(OH)-alkyle en C₁-C₅ ou -CH(OH)-phényle,
où le radical -CH(OH)-phényle peut être substitué par 1 à 3 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et dialkyl(C₁-C₄)amino,
R⁵ représente un groupe alkyle en C₁-C₆, cycloalkyl-(C₃-C₇)méthyle, benzyle ou triméthylsilylméthyle,
R⁶ représente un groupe alkyle en C₂-C₄ linéaire,
où le radical alkyle est substitué par un substituant choisi dans le groupe constitué par un groupe amino, 1,4,5,6-tétrahydropyrimidin-2-ylamino et [amino(imino)méthyl]-amino,
R⁷ représente un groupe alkyle en C₁-C₆, cycloalkyl-(C₃-C₇)méthyle, benzyle ou triméthylsilylméthyle,
R⁸ représente un groupe alkyle en C₁-C₄, -CH₂-OH, - CH(OH)-alkyle en C₁-C₅ ou -CH(OH)-phényle,
où le radical -CH(OH)-phényle peut être substitué par 1 à 3 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et dialkyl(C₁-C₄)amino, et
R⁹ représente un atome d'hydrogène, un groupe alkyle en C₁-C₆, hydroxyméthyle ou 2-amino-2-oxoéthyle,
R¹⁰ représente un atome d'hydrogène, un groupe alkyle en C₁-C₄, 2-amino-2-oxoéthyle ou 2-amino-1-hydroxy-2-oxoéthyle,
où le radical alkyle en C₁-C₄ peut être substitué par un substituant choisi dans le groupe constitué par un groupe amino et hydroxy, sauf que R¹⁰ ne doit pas représenter un groupe 4-aminobut-1-yle,
R¹¹ représente un groupe méthyle,
où le radical méthyle est substitué par un substituant choisi dans le groupe constitué par un groupe hydroxy et amino,
ou un de ses sels, un de ses solvates ou solvates de ses sels.

8. Composé selon la revendication 7, **caractérisé en ce que**
R¹ représente un radical 2,2-diméthylprop-1-yle, 2,2-diméthylbut-1-yle ou triméthylsilylméthyle,
ou un de ses sels, un de ses solvates ou solvates de ses sels.

9. Composé selon la revendication 7 ou 8, **caractérisé en ce que** R² représente un radical 2,2₋diméthylprop-1-yle ou 3-pyridylméthyle,
ou un de ses sels, un de ses solvates ou solvates de ses sels.

10. Composé selon l'une des revendications 7 à 9, **caractérisé en ce que**
R³ représente un groupe phényle,
où le radical phényle peut être substitué par un substituant choisi dans le groupe constitué par un atome d'halogène, un groupe cyano, méthyle, méthoxy, diméthylamino et diéthylamino,
ou un de ses sels, un de ses solvates ou solvates de ses sels.

11. Composé selon l'une des revendications 7 à 10, **caractérisé en ce que**
R⁸ représente un groupe -CH(OH)-alkyle en C₁-C₅ ou -CH(OH)-phényle,
où le radical -CH(OH)-phényle peut être substitué par 1 à 3 substituants choisis, indépendamment les uns des autres, dans le groupe constitué par un atome d'halogène, un groupe cyano, trifluorométhyle, trifluorométhoxy, alkyle en C₁-C₄, alcoxy en C₁-C₄ et dialkyl(C₁-C₄)aMino,
ou un de ses sels, un de ses solvates ou solvates de ses sels.

12. Composé selon l'une des revendications 7 à 11, **caractérisé en ce que**
R¹⁰ représente un groupe méthyle, éthyle, 2-amino-2-oxoéthyle ou 2-amino-1-hydroxy-2-oxoéthyle,
où les radicaux méthyle et éthyle peuvent être substitués par un substituant hydroxy,
ou un de ses sels, un de ses solvates ou solvates de ses sels.

13. Procédé de préparation d'un composé de formule (I) selon la revendication 1, **caractérisé en ce que** l'on fait réagir
[A] un composé de formule 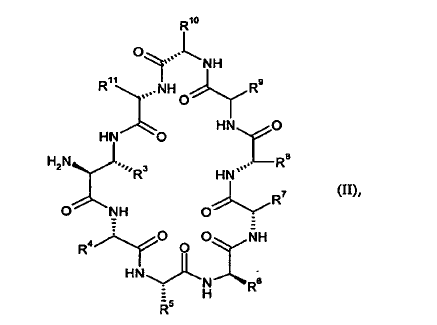 dans laquelle
R³, R⁴, R⁵, R⁶, R⁷, R⁸, R⁹, R¹⁰ et R¹¹ ont la signification indiquée dans la revendication 1,
d'abord avec un composé de formule 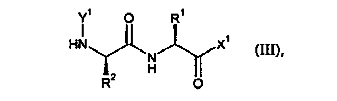 dans laquelle
R¹ et R² ont la signification indiquée dans la revendication 1,
Y¹ représente un radical tert-butoxycarbonyle ou benzyloxy-carbonyle et
X¹ représente un atome d'halogène, de préférence de brome, de chlore ou de fluor, ou un groupe hydroxy,
et puis avec un acide et/ou par hydrogénolyse,
ou
[B] un composé de formule 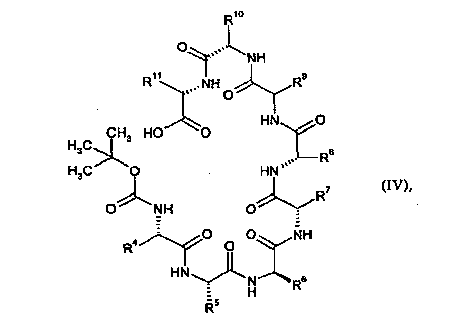 dans laquelle
R⁴, R⁵, R⁶, R⁷, R⁸, R⁹, R¹⁰ et R¹¹ ont la signification indiquée dans la revendication 1,
d'abord avec un composé de formule 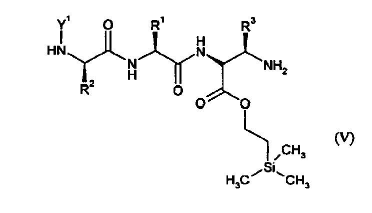 dans laquelle
R¹, R² et R³ ont la signification indiquée dans la revendication 1, et
Y¹ représente un radical tert-butoxycarbonyle ou benzyloxy-carbonyle,
et puis, dans une synthèse en 4 étapes
a) avec un réactif fluorure comme le fluorure de tétrabutyl-ammonium,
b) avec un acide,
c) avec un réactif de déshydratation, le cas échéant en présence d'une base,
et
d) par hydrogénolyse.

14. Procédé selon la revendication 13, **caractérisé en ce que** le composé de formule (II) est préparé en faisant réagir un composé de formule 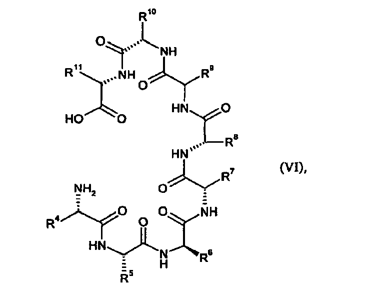 dans laquelle
R⁴, R⁵, R⁶, R⁷, R⁸, R⁹, R¹⁰ et R¹¹ ont la signification indiquée dans la revendication 1,
d'abord avec un composé de formule 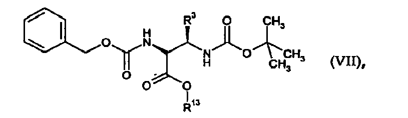 dans laquelle
R³ a la signification indiquée dans la revendication 1, et
R¹³ représente un groupe cyanométhyle, p-nitrophényle, o-nitrophényle, 2,4-dinitrophényle, 2,4,5-trichlorophényle, pentachlorophényle, pentafluorophényle (Pfp), N-hydroxyphtalimidyle, N-hydroxy-succinimidyle (O-Su), 1-hydroxypipéridinyle ou 5-chloro-8-hydroxyquinolinyle,
et puis, dans une synthèse en 3 étapes
a) avec un acide,
b) avec un réactif de déshydratation, le cas échéant en présence d'une base,
et
c) par hydrogénolyse.

15. Composé selon une des revendications 1 à 12, destiné à l'utilisation dans un procédé de traitement et/ou de prophylaxie des maladies.

16. Utilisation d'un composé selon une des revendications 1 à 12 pour la préparation d'un médicament destiné au traitement et/ou à la prophylaxie des maladies.

17. Utilisation d'un composé selon une des revendications 1 à 12 pour la préparation d'un médicament destiné au traitement et/ou à la prophylaxie des infections bactériennes.

18. Médicament comprenant un composé selon une des revendications 1 à 12, en combinaison avec un auxiliaire inerte, non toxique, pharmaceutiquement acceptable.

19. Médicament selon la revendication 18, destiné au traitement et/ou à la prophylaxie des infections bactériennes.

20. Quantité efficace sur le plan antibactérien d'au moins un composé selon l'une des revendications 1 à 12, d'un médicament selon la revendication 18 ou d'un médicament obtenu selon la revendication 16 ou 17 pour l'utilisation dans un procédé de lutte contre les infections bactériennes chez l'homme et chez l'animal.
